(19) Europäisches Patentamt
European Patent Office
Office européen des brevets

(11) EP 1 968 957 B1

(12) # EUROPÄISCHE PATENTSCHRIFT

(45) Veröffentlichungstag und Bekanntmachung des Hinweises auf die Patenterteilung:
**20.01.2010 Patentblatt 2010/03**

(21) Anmeldenummer: **06829855.3**

(22) Anmeldetag: **22.12.2006**

(51) Int Cl.:
***C07D 277/42*** *(2006.01)* ***C07D 277/44*** *(2006.01)*
***C07D 417/04*** *(2006.01)* ***A61K 31/427*** *(2006.01)*

(86) Internationale Anmeldenummer:
**PCT/EP2006/012482**

(87) Internationale Veröffentlichungsnummer:
**WO 2007/079960 (19.07.2007 Gazette 2007/29)**

(54) **SUBSTITUIERTE THIAZOLE UND IHRE VERWENDUNG ZUR HERSTELLUNG VON ARZNEIMITTELN**

SUBSTITUTED THIAZOLES AND THEIR USE FOR PRODUCING DRUGS

THIAZOLES SUBSTITUES ET LEUR UTILISATION POUR PRODUIRE DES MEDICAMENTS

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **28.12.2005 DE 102005062991**

(43) Veröffentlichungstag der Anmeldung:
**17.09.2008 Patentblatt 2008/38**

(73) Patentinhaber: **Grünenthal GmbH**
**52078 Aachen (DE)**

(72) Erfinder:
- **HAURAND, Michael**
**52078 Aachen (DE)**
- **SCHIENE, Klaus**
**41363 Jüchen (DE)**
- **KÜHNERT, Sven**
**52355 Düren (DE)**
- **REICH, Melanie**
**52072 Aachen (DE)**

(74) Vertreter: **Brosch, Oliver et al**
**Kutzenberger & Wolff**
**Theodor-Heuss-Ring 23**
**50668 Köln (DE)**

(56) Entgegenhaltungen:
**WO-A-03/093236**
**WO-A-2005/007641**
**WO-A-2006/002981**

EP 1 968 957 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft substituierte Thiazole, Verfahren zu ihrer Herstellung, Arzneimittel enthaltend diese Verbindungen sowie deren Verwendung zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufrieden stellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nozizeption in letzter Zeit erschienen sind.

Klassische Opioide, wie beispielsweise Morphin, sind bei der Therapie starker bis sehr starker Schmerzen wirksam, führen jedoch oftmals zu unerwünschten Begleiterscheinungen wie beispielsweise Atemdepression, Erbrechen, Sedierung, Obstipation oder Toleranzentwicklung. Des Weiteren sind sie bei neuropathischen Schmerzen, unter denen insbesondere Tumorpatienten leiden, häufig nicht ausreichend wirksam.

Eine Aufgabe der vorliegenden Erfindung bestand daher darin, neue Verbindungen zur Verfügung zu stellen, die sich insbesondere als pharmazeutische Wirkstoffe in Arzneimitteln eignen, bevorzugt in Arzneimitteln zur Behandlung von Schmerzen.

Es wurde nun überraschenderweise gefunden, dass sich die substituierten Thiazole der nachstehend angegebenen allgemeinen Formel I zur mGluR5-Rezeptor-Regulation eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Ein Gegenstand der vorliegende Erfindung sind daher substituierte Thiazole der allgemeinen Formel I,

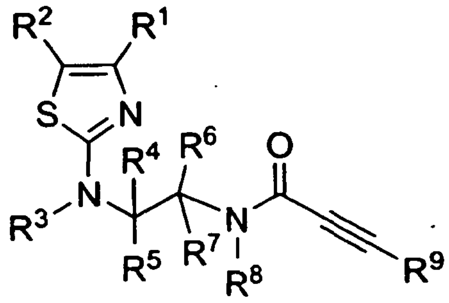

I,

worin

$R^1$ und $R^2$, unabhängig voneinander, jeweils für H; F; Cl; Br; I; $-NO_2$; -CN; $-NH_2$; -OH;-SH; -C(=O)-OH; -C(=O)-H; -NH-C(=O-H; $-NH-R^{55}$; $-NR^{56}R^{57}$; $-C(=O)-R^{58}$; $-C(=O)-O-R^{59}$; $-O-C(=O)-R^{60}$; $-NH-C(=O)-R^{61}$; $-NR^{62}-C(=O)-R^{63}$; $-C(=O)-NH_2$; $-C(=O)-NH-R^{64}$; $-C(=O)-NR^{65}R^{66}$; $-O-R^{67}$; $-S-R^{68}$; $-S(=O)-R^{69}$; $-S(=O)_2-R^{70}$; $-NH-C(=O)-NH-R^{71}$; $-NH-C(=S)-NH-R^{72}$ ; $-NH-S(=O)_2-R^{73}$; $-NR^{74}-S(=O)_2-R^{75}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl,-(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder wenigstens einfach substituiertes -(Heteroalkylen)-Cycloalkyl, -(Heteroalkenylen)-Cycloalkyl,-(Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, -(Alkylen)-Heterocycloalkenyl,-(Alkenylen)-Heterocycloalkenyl oder -(Alkinylen)-Heterocycloalkenyl; unsubstituiertes oder wenigstens einfach substituiertes -(Heteroalkylen)-Heterocycloalkyl,-(Heteroalkenylen)-Heterocycloalkyl,- (Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, -(Heteroalkylen)-Aryl oder -(Heteroalkenylen)-Aryl; oder unsubstituiertes oder wenigstens einfach substituiertes -(Alkylen)-Heteroaryl, - (Alkenylen)-Heteroaryl, -(Alkinylen)-Heteroaryl, -(Heteroalkylen)-Heteroaryl oder - (Heteroalkenylen)-Heteroaryl stehen;

oder $R^1$ und $R^2$ zusammen mit den sie verbindenden Kohlenstoffatomen einen unsubstituierten oder wenigstens einfach substituierten Phenylen-Rest bilden;

$R^3$, $R^8$ und $R^{54}$, unabhängig voneinander, jeweils für H; -C(=O)-$R^{58}$; -C(=O)-O-$R^{59}$; - C(=O)-$NH_2$; -C(=O)-NH-$R^{64}$; -C(=O)-N$R^{65}R^{66}$; -S(=O)-$R^{69}$: -$S(=O)_2$-$R^{70}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl,-(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl, unsubstituiertes oder substituiertes - (Heteroalkylen)-Cycloalkyl, -(Heteroalkenyleny)-Cycloalkyl, -(Heteroalkylen)-Cycloalkenyl oder -(Heteroalkenylen)-Cycloalkenyl, unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenylen)-Heterocycloalkyl, -(Heteroalkylen-Heterocycloalkenyl oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, -(Heteroalkylen)-Aryl oder -(Heteroalkenylen)-Aryl; oder unsubstituiertes oder substituiertes -(Alkylen)-Heteroaryl, -(Alkenylen)- Heteroaryl, -(Alkinylen)-Heteroaryl, -(Heteroalkylen)-Heteroaryl oder -(Heteroalkenylen)-Heteroaryl stehen;

$R^4$, $R^5$, $R^6$, $R^7$, $R^{10}$, $R^{11}$, $R^{12}$ , $R^{13}$ , $R^{14}$, $R^{15}$, $R^{16}$, $R^{17}$, $R^{18}$, $R^{19}$, $R^{20}$, $R^{21}$, $R^{22}$ , $R^{23}$, $R^{24}$, $R^{25}$, $R^{26}$, $R^{27}$ , $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45,}$ $R^{46}$ $R^{47}$, $R^{48}$ $R^{49}$, $R^{50}$, $R^{51}$ $R^{52}$ und $R^{53}$, unabhängig voneinander, jeweils für H; F; Cl; Br; I; -$NO_2$; -CN; -$NH_2$; -OH; -SH; -C(=O)-OH; -C(=O)-H; -NH-C(=O)-H; -NH-$R^{55}$;-N$R^{56}R^{57}$ ; -C(=O)-$R^{58}$; -C(=O-O-$R^{59}$; -O-C(=O)-$R^{60}$, -NH-C(=O)-$R^{61}$; -N$R^{62}$-C(=O)-$R^{63}$; C(=O)-$NH_2$; -C(=O)-NH-$R^{64}$; -C(=O)-N$R^{65}R^{66}$; -O-$R^{67}$; -S-$R^{68}$; -S(=O)-$R^{69}$; -$S(=O)_2$-$R^{70}$;-NH-C(=O)-NH-$R^{71}$; -NH-C(=S)-NH-$R^{72}$; -NH-$S(=O)_2$-$R^{73}$; -N$R^{74}$-$S(=O)_2$-$R^{75}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, -(Alkinylen)-Cycloalkyl, -(Alkylen)-Cycloalkenyl, - (Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl; -(Alkylen)-Heterocycloalkenyl, -(Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenylen)-Heterocycloalkyl, - (Heteroalkylen)-Heterocycloalkenyl oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, - (Heteroalkylen)-Heteroaryl oder -(Heteroalkenylen)-Heteroaryl stehen;

oder $R^4$ und $R^5$ oder $R^6$ und $R^7$ oder $R^{10}$ und $R^{11}$ oder $R^{12}$ und $R^{13}$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$ oder $R^{28}$ und $R^{29}$ oder $R^{30}$ und $R^{31}$ oder $R^{32}$ und $R^{33}$ oder $R^{34}$ und $R^{35}$ oder $R^{36}$ und $R^{37}$ oder $R^{38}$ und $R^{39}$ oder $R^{40}$ und $R^{41}$ oder $R^{42}$ und $R^{43}$ oder $R^{44}$ und $R^{45}$ oder $R^{46}$ und $R^{41}$ oder $R^{48}$ und $R^{49}$ oder $R^{50}$ und $R^{51}$ oder $R^{52}$ und $R^{53}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^3$ und $R^4$ zusammen mit der sie verbindenden -N-C$R^5$-Gruppe einen Rest der allgemeinen Formel A,

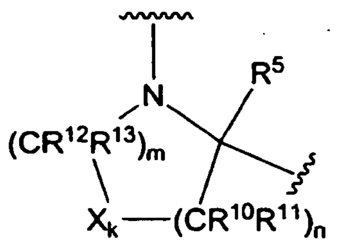

A,

bilden;
oder $R^6$ und $R^8$ zusammen mit der sie verbindenden -N-$CR^7$-Gruppe einen Rest der allgemeinen Formel B,

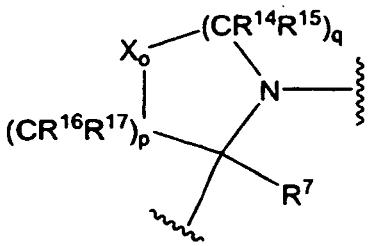

B,

bilden;
m und q jeweils für 1, 2, 3, 4 oder 5 stehen;
n und p jeweils für 0, 1, 2, 3 oder 4 stehen;
k und o jeweils für 0 oder 1 stehen;
wobei die Summe aus m, n und k oder die Summe aus p, q und o jeweils gleich 1, 2, 3, 4, 5 oder 6 ist;
oder $R^3$ und $R^6$ zusammen mit der sie verbindenden -N-$CR^4R^5$-$CR^7$-Gruppe einen Rest der allgemeinen Formel C,

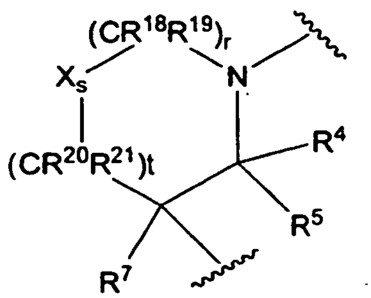

C,

bilden;
oder $R^4$und $R^8$ zusammen mit der sie verbindenden -$CR^5$-$CR^6R^7$-N-Gruppe einen Rest der allgemeinen Formel D,

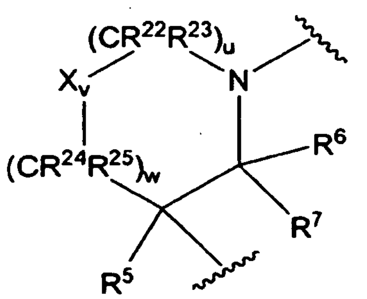

D,

bilden;
r und u jeweils für 1, 2, 3 oder 4 stehen;
t und w jeweils für 0, 1, 2 oder 3 stehen;

s und v jeweils für 0 oder 1 stehen;
wobei die Summe aus r, s und t oder die Summe aus u, v und w jeweils gleich 1, 2, 3, 4 oder 5 ist;
oder $R^3$ und $R^8$ zusammen mit der sie verbindenden -N-$CR^4R^5$-$CR^6R^7$-N-Gruppe einen Rest der allgemeinen Formel E,

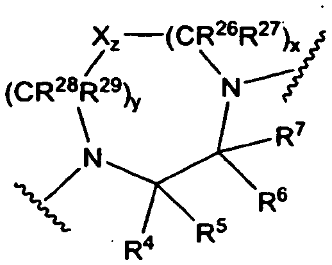

E,

bilden;
x und y jeweils für 1 oder 2 stehen;
z für 0 oder 1 steht;
wobei die Summe aus x, y und z gleich 3 oder 4 ist;
oder $R^4$ und $R^6$ zusammen mit der sie verbindenden -$CR^5$-$CR^7$-Gruppe einen Rest der allgemeinen Formel F,

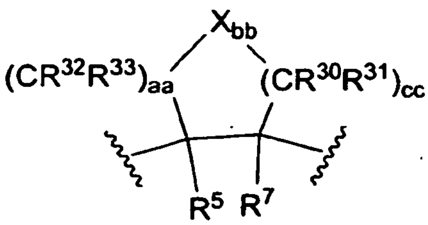

F,

bilden;
aa und cc, unabhängig voneinander, jeweils für 1, 2, 3, 4 oder 5 stehen;
bb für 0 oder 1 steht;
wobei die Summe aus aa, bb und cc gleich 1, 2, 3, 4, 5 oder 6 ist;
oder $R^3$ und $R^4$ zusammen mit der sie verbindenden -N-$CR^5$-Gruppe und $R^6$ und $R^8$ zusammen mit der sie verbindenden -N-$CR^7$-Gruppe einen Rest der allgemeinen Formel G,

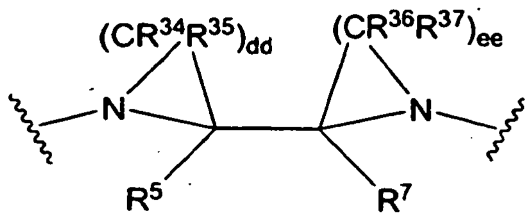

G,

bilden;
dd und ee, unabhängig voneinander, jeweils für 1, 2, 3 oder 4 stehen;
oder $R^3$ und $R^6$ zusammen mit der sie verbindenden -N-$CR^4CR^5$-$CR^7$-Gruppe und $R^4$ und $R^8$ zusammen mit der sie verbindenden -$CR^5$-$CR^6R^7$-N-Gruppe einen Rest der allgemeinen Formel H,

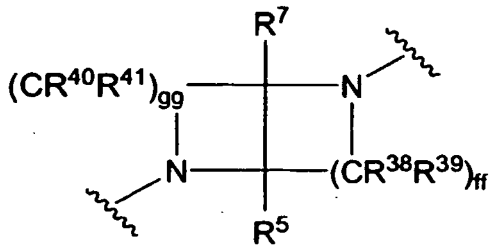

H,

bilden,
ff und gg, unabhängig voneinander, jeweils für 1, 2 oder 3 stehen;
oder $R^3$ und $R^3$ zusammen mit der sie verbindenden $-N-CR^4R^5-CR^6R^7-N-$Gruppe und $R^4$ und $R^6$ zusammen mit der sie verbindenden $-CR^5-CR^7-$Gruppe einen Rest der allgemeinen Formel K,

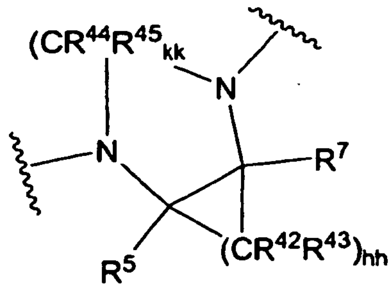

K,

bilden;
hh für 1, 2, 3 oder 4 steht;
kk für 1, 2, 3, 4, 5 oder 6 steht;
oder $R^3$ und $R^8$ zusammen mit der sie verbindenden $-N-CR^4R^5-CR^6R^7-N-$Gruppe einen bizyklischen Rest der allgemeinen Formel L,

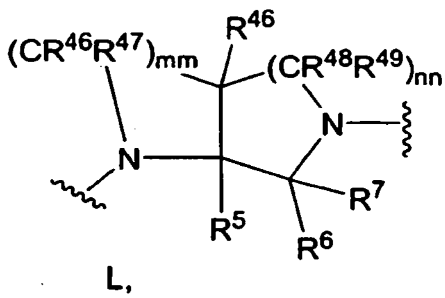

L,

bilden;
mm für 1, 2 oder 3 steht;
nn für 1 oder 2 steht;
oder $R^3$ und $R^8$ zusammen mit der sie verbindenden $-N-CR^4R^5-CR^6R^7-N-$Gruppe einen bizyklischen Rest der allgemeinen Formel M,

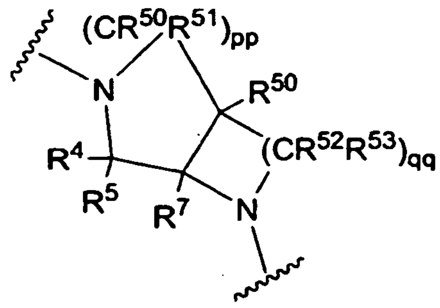

M,

bilden;
pp für 1 oder 2 steht;
qq für 1, 2 oder 3 steht;
X für O, S oder N-$R^{54}$ steht;
$R^9$ für H; F; Cl; Br; I; -CN; -C(=O)-OH; -C(=O)-H; -C(=O)-$R^{58}$; -C(=O)-O-$R^{59}$; -C(=O)$NH_2$; -C(=O)-NH-$R^{64}$; -C (=O)-$NR^{65}R^{66}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, - (Alkinylen)-Cycloalkyl, -(Alkylen)-Cycloalkenyl, -(Alkenylen)- Cycloalkenyl oder - (Alkinylen)-Cycloalkenyl; unsubstituiertes oder substituiertes -(Heteroalkylen)-Cycloalkyl, -(Heteroalkenylen)-Cycloalkyl, -(Heteroalkylen)-Cycloalkenyl oder -(Heteroalkenylen)-Cycloalkenyl; unsubstituiertes oder substituiertes -(Alkylen)-Heterocycloalkyl,-(Alkenylen)-Heterocycloalkyl, -(Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenylen)-Heterocycloalkyl, -(Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl; -(Alkinylen)-Aryl, -(Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl steht;
und $R^{55}$, $R^{56}$, $R^{57}$, $R^{58}$, $R^{59}$, $R^{60}$, $R^{61}$, $R^{62}$ $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$ und $R^{75}$, unabhängig voneinander, jeweils für unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl,-(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes - (Heteroalkylen)-Cycloalkyl, -(Heteroalkenylen)-Cycloalkyl, -(Heteroalkylen)-Cycloalkenyl oder -(Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl,- (Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenyten)-Heterocycloalkyl, -(Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl. -(Alkenylen)-Aryl, -(Alkinylen)-Aryl, -(Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Der Begriff "Alkyl" umfaßt im Sinne der vorliegenden Erfindung azyklische gesättigte Kohlenwasserstoffreste, die verzweigt oder geradkettig sowie unsubstituiert oder wenigstens einfach substituiert sein können mit wie im Fall von $C_{1-12}$-Alkyl 1 bis 12 (d. h. 1, 2, 3, 4, 5, 6, 7, 8, 9, 10, 11 oder 12) C-Atomen bzw, mit wie im Fall von $C_{1-6}$-Alkyl 1 bis 6 (d. h. 1, 2, 3, 4, 5 oder 6) C-Atomen. Sofern einer oder mehrere der Substituenten für einen Alkyl-Rest stehen oder einen Alkyl-Rest aufweisen, der einfach oder mehrfach substituiert ist, kann dieser bevorzugt mit ggf. 1, 2, 3. 4 oder 5, besonders bevorzugt mit 1, 2 oder 3, Substituenten unabhängig voneinander ausgewählt aus der Gruppe bestehend

aus F, Cl. Br, I, $-NO_2$, -CN, -OH, -SH, $-NH_2$, $-N(C_{1-5}\text{-Alkyl})_2$, $-N(C_{1-5}\text{-Alkyl})(\text{Phenyl})$, $-N(C_{1-5}\text{-Alkyl})(CH_2\text{-Phenyl})$, $-N(C_{1-5}\text{-Alkyl})(CH_2\text{-}CH_2\text{-Phenyl})$, -C(=O)-H, - $C(=O)\text{-}C_{1-5}$-Alkyl, -C(=O)-Phenyl, $-C(=S)\text{-}C_{1-5}$-Alkyl, -C(=S)-Phenyl, -C(=O)-OH, $-C(=O)\text{-O-}C_{1-5}$-Alkyl, -C(=O)-O-Phenyl, $-C(=O)\text{-}NH_2$, $-C(=O)\text{-NH-}C_{1-5}$-Alkyl, $-C(=O)\text{-N}(C_{1-5}\text{-Alkyl})_2$, -S(=O)-$C_{1-5}$-Alkyl, -S(=O)-Phenyl, $-S(=O)_2\text{-}C_{1-5}$-Alkyl, $-S(=O)_2$-Phenyl, $-S(=O)_2\text{-}NH_2$ und - $SO_3H$ substituiert sein, wobei die vorstehend genannten $C_{1-5}$-Alkyl-Reste jeweils linear oder verzweigt sein können und die vorstehend genannten Phenyl-Reste bevorzugt mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O\text{-}CF_3$, -SH, $-O\text{-}CH_3$, $-O\text{-}C_2H_5$, $-0\text{-}C_3H_7$, Methyl, Ethyl, n-Propyl, Isopropyl, n-Butyl, 2-Butyl, Isobutyl und tert-Butyl substituiert sein können. Besonders bevorzugte Substituenten können unabhängig voneinander ausgewählt werden aus der Gruppe bestehend aus F, Cl, Br, I, $-NO_2$, -CN, -OH, -SH, $-NH_2$, $-N(CH_3)_2$, $-N(C_2H_5)_2$ und $-N(CH_3)(C_2H_5)$.

Als geeignete $C_{1-12}$-Alkyl-Reste, die unsubstituiert oder einfach oder mehrfach substituiert sein können, seien beispielsweise Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, 2-Hexyl, 3-Hexyl, n-Heptyl, n-Octyl, $-C(H)(C_2H_5)_2$, $-C(H)(n\text{-}C_3H_7)_2$ und $-CH_2\text{-}CH_2\text{-}C(H)(CH_3)\text{-}(CH_2)_3\text{-}CH_3$ genannt. Als geeignete $C_{1-6}$)Alkyl-Reste seien beispielsweise Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, 2-Hexyl und 3-Hexyl genannt.

Unter mehrfach substituierten Alkyl-Resten sind solche Alkyl-Reste zu verstehen, die entweder an verschiedenen oder an gleichen C-Atomen mehrfach, bevorzugt zwei- oder dreifach, substituiert sind, beispielsweise dreifach am gleichen C-Atom wie im Fall von - $CF_3$ oder an verschiedenen Stellen wie im Fall von $-(CHCl)\text{-}(CH_2F)$. Die Mehrfachsubstitution kann mit dem gleichen oder mit verschiedenen Substituenten erfolgen. Als geeignete substituierte Alkyl-Reste seien beispielsweise $-CF_3$, $-CF_2H$, - $CFH_2$, $-(CH_2)\text{-OH}$, $-(CH_2)\text{-}NH_2$, $-(CH_2)\text{-CN}$, $-(CH_2)\text{-}(CF_3)$, $-(CH_2)\text{-}(CHF_2)$, $-(CH_2)\text{-}(CH_2F)$. - $(CH_2)\text{-}(CH_2)\text{-OH}$, $-(CH_2)\text{-}(CH_2)\text{-}NH_2$. $-(CH_2)\text{-}(CH_2)\text{-CN}$, $-(CF_2)\text{-}(CF_3)$, $-(CH_2)\text{-}(CH_2)\text{-}(CF_3)$ und $-(CH_2)\text{-}(CH_2)\text{-}(CH_2)\text{-OH}$ genannt.

Der Begriff "Alkenyl" umfaßt im Sinne der vorliegenden Erfindung azyklische ungesättigte Kohlenwasserstoffreste, die verzweigt oder geradkettig sowie unsubstituiert oder wenigstens einfach substituiert sein können und wenigstens eine Doppelbindung, bevorzugt 1, 2 oder 3 Doppelbindungen, aufweisen, mit wie im Fall von $C_{2-12}$-Alkenyl 2 bis 12 (d. h. 2, 3, 4, 5, 6, 7, 8, 9, 10, 11 oder 12) C-Atomen bzw. mit wie im Fall von $C_{2-6}$-Alkenyl 2 bis 6 (d. h. 2, 3, 4, 5 oder 6) C-Atomen. Sofern einer oder mehrere der Substituenten für einen Alkenyl-Rest stehen oder einen Alkenyl-Rest aufweisen, der einfach oder mehrfach substituiert ist, kann dieser bevorzugt mit ggf. 1, 2, 3, 4 oder 5, besonders bevorzugt mit 1, 2 oder 3, Substituenten unabhängig voneinander ausgewählt aus der Gruppe bestehend aus F, Cl, Br, I, $-NO_2$. -CN, -OH, -SH, $-NH_2$,$-N(C_{1-5}\text{-Alkyl})_2$,$-N(C_{1-5}\text{-Alkyl})(\text{Phenyl})$, $-N(C_{1-5}\text{-Alkyl})(CH_2\text{-Phenyl})$, $-N(C_{1-5}\text{-Alkyl})(CH_2\text{-}CH_2\text{-Phenyl})$, -C(=O)-H, $-C(=O)\text{-}C_{1-5}$-Alkyl, -C(=O)-Phenyl, $-C(=S)\text{-}C_{1-5}$-Alkyl, -C(=S)-Phenyl, - C(=O)-OH, $-C(=O)\text{-O-}C_{1-5}$-Alkyl, -C(=O)O-Phenyl, $-C(=O)\text{-}NH_2$, $-C(=O)\text{-NH-}C_{1-5}$-Alkyl, - $C(=O)\text{-N}(C_{1-5}\text{-Alkyl})_2$, $-S(=O)\text{-}C_{1-5}$-Alkyl, -S(=O)-Phenyl, $-S(=O)_2\text{-}C_{1-5}$-Alkyl, $-S(=O)_2$-Phenyl, $-S(=O)_2\text{-}NH_2$ und $-SO_3H$ substituiert sein, wobei die vorstehend genannten $C_{1-5}$-Alkyl-Reste jeweils linear oder verzweigt sein können und die vorstehend genannten Phenyl-Reste bevorzugt mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O\text{-}CF_3$,-SH, $-O\text{-}CH_3$, $-O\text{-}C_2H_5$, $-O\text{-}C_3H_7$, Methyl, Ethyl, n-Propyl, Isopropyl, n-Butyl, 2-Butyl, Isobutyl und tert-Butyl substituiert sein können. Besonders bevorzugte Substituenten können unabhängig voneinander ausgewählt werden aus der Gruppe bestehend aus F, Cl, Br, I, $-NO_2$, -CN, -OH, -SH, $-NH_2$, $-N(CH_3)_2$, $-N(C_2H_5)_2$ und $-N(CH_3)(C_2H_5)$.

Als geeignete $C_{2-12}$-Alkenyl-Reste seien beispielsweise Ethenyl, 1-Propenyl, 2-Propenyl, 1-Butenyl, 2-Butenyl, 3-Butenyl, 1-Pentenyl, 2-Pentenyl, 3-Pentenyl, 4-Pentenyl, Hexenyl, $-CH{=}CH\text{-}CH{=}CH\text{-}CH_3$ und $-CH_2\text{-}CH_2\text{-}CH{=}CH_2$ genannt.

Unter mehrfach substituierten Alkenyl-Resten sind solche Alkenyl-Reste zu verstehen, die entweder an verschiedenen oder an gleichen C-Atomen mehrfach, bevorzugt zweifach, substituiert sind, beispielsweise zweifach am gleichen C-Atom wie im Fall von $-CH{=}CCl_2$ oder an verschiedenen Stellen wie im Fall von $-CCl{=}CH\text{-}(CH_2)\text{-}NH_2$. Die Mehrfachsubstitution kann mit dem gleichen oder mit verschiedenen Substituenten erfolgen. Als geeignete substituierte Alkenyl-Reste seien beispielsweise $-CH{=}CH\text{-}(CH_2)\text{-OH}$, $-CH{=}CH\text{-}(CH_2)\text{-}NH_2$ und -CH=CH-CN genannt.

Der Begriff "Alkinyl" umfaßt im Sinne der vorliegenden Erfindung azyklische ungesättigte Kohlenwasserstoffreste, die verzweigt oder geradkettig sowie unsubstituiert oder wenigstens einfach substituiert sein können und wenigstens eine Dreifachbindung, bevorzugt 1 oder 2 Dreifachbindungen, aufweisen, mit wie im Fall von $C_{2-12}$-Alkinyl 2 bis 12 (d. h. 2, 3, 4, 5, 6, 7, 8, 9, 10, 11 oder 12) C-Atomen bzw. mit wie im Fall von $C_{2-6}$-Alkinyl 2 bis 6 (d. h. 2, 3, 4, 5 oder 6) C-Atomen. Sofern einer oder mehrere der Substituenten für einen Alkinyl-Rest stehen oder einen Alkinyl-Rest aufweisen, der einfach oder mehrfach substituiert ist, kann dieser bevorzugt mit ggf. 1, 2, 3, 4 oder 5, besonders bevorzugt mit ggf. 1 oder 2, Substituenten unabhängig voneinander ausgewählt aus der Gruppe bestehend aus F, Cl, Br, I, $-NO_2$, -CN, -OH, -SH, $-NH_2$, - $N(C_{1-5}\text{-Alkyl})_2$,$-N(C_{1-5}\text{-Alkyl})(\text{Phenyl})$, $-N(C_{1-5}\text{-Alkyl})(CH_2\text{-Phenyl})$, $-N(C_{1-5}\text{-Alkyl})(CH_2\text{-}CH_2\text{-Phenyl})$, -C(=O)-H, $-C(=O)\text{-}C_{1-5}$-Alkyl, -C(=O)-Phenyl, $-C(=S)\text{-}C_{1-5}$-Alkyl, -C(=S)-Pheny), - C(=O)-OH, $-C(=O)\text{-O-}C_{1-5}$-Alkyl, -C(=O)-O-Phenyl, -C(=O)-NH2, $-C(=O)\text{-NH-}C_{1-5}$-Alkyl, - $C(=O)\text{-N}(C_{1-5}\text{-Alkyl})_2$, $-S(=O)C_{1-5}$-Alkyl, -S(=O)-Pheny), $-S(=O)_2\text{-}C_{1-5}$-Alkyl, $-S(=O)_2$-Phenyl, $-S(=O)_2\text{-}NH_2$ und $-SO_3H$ substituiert sein, wobei die vorstehend genannten $C_{1-5}$-Alkyl-

Reste jeweils linear oder verzweigt sein können und die vorstehend genannten Phenyl-Reste bevorzugt mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, - SH, $-O-CH_3$, $-O-C_2H_5$, $-O-C_3H_7$, Methyl, Ethyl, n-Propyl, Isopropyl, n-Butyl, 2-Butyl, Isobutyl und tert-Butyl substituiert sein können. Besonders bevorzugte Substituenten können unabhängig voneinander ausgewählt werden aus der Gruppe bestehend aus F, Cl, Br, I, $-NO_2$, -CN, -OH, -SH, $-NH_2$, $-N(CH_3)_2$, $-N(C_2H_5)_2$ und $-N(CH_3)(C_2H_5)$.

Als geeignete $C_{2-12}$-Alkinyl-Reste seien beispielsweise Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl und Hexinyl genannt.

Unter mehrfach substituierten Alkinyl-Resten sind solche Alkinyl-Reste zu verstehen, die entweder an verschiedenen C-Atomen mehrfach substituiert sind, beispielsweise zweifach an verschiedenen C-Atomen wie im Fall von -CHCl-C≡CCl. Als geeignete substituierte Alkinyl-Reste seien beispielsweise -C≡C-F, -C≡C-Cl und -C≡C-I genannt.

Der Begriff "Heteroalkyl" bezeichnet einen wie vorstehend beschriebenen Alkyl-Rest, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkyl-Reste können bevorzugt 1, 2 oder 3 Heteroatom(e) unabhängig voneinander ausgewählt aus der Gruppe bestehend aus Sauerstoff, Schwefel und Stickstoff (NH) als Kettenglied(er) aufweisen. Heteroalkyl-Reste können bevorzugt 2- bis 12-gliedrig, besonders bevorzugt 2- bis 6-gliedrig, sein.

Als geeignete Heteroalkyl-Reste, die unsubstituiert oder einfach oder mehrfach substituiert sein können, seien beispielsweise $-CH_2-O-CH_3$, $-CH_2-O-C_2H_5$, $-CH_2-O-CH(CH_3)_2$, $-CH_2-O-C(CH_3)_3$, $-CH_2-S-CH_3$, $-CH_2-S-C_2H_5$, $-CH_2-S-CH(CH_3)_2$, $-CH_2-S-C(CH_3)_3$, $-CH_2-NH-CH_3$, $-CH_2-NH-C_2H_5$, $-CH_2-NH-CH(CH_3)_2$, $-CH_2-NH-C(CH_3)_3$, $-CH_2-CH_2-O-CH_3$, $-CH_2-CH_2-O-C_2H_5$, $-CH_2-CH_2-O-CH(CH_3)_2$, $-CH_2-CH_2-O-C(CH_3)_3$, $-CH_2-CH_2-S-CH_3$, $-CH_2-CH_2-S-C_2H_5$, $-CH_2-CH_2-S-CH(CH_3)_2$, $-CH_2-CH_2-S-C(CH_3)_3$, $-CH_2-CH_2-NH-CH_3$, $-CH_2-CH_2-NH-C_2H_5$, $-CH_2-CH_2-NH-CH(CH_3)_2$, $-CH_2-CH_2-NH-C(CH_3)_3$, $-CH_2-S-CH_2-O-CH_3$, $-CH_2-O-CH_2-O-C_2H_5$, $-CH_2-O-CH_2-O-CH(CH_3)_2$, $-CH_2-S-CH_2-O-C(CH_3)_3$, - $CH_2-O-CH_2-S-CH_3$, $-CH_2-O-CH_2-S-C_2H_5$, $-CH_2-O-CH_2-S-CH(CH_3)_2$, $-CH_2-NH-CH_2-S-C(CH_3)_3$, $-CH_2-O-CH_2-NH-CH_3$, $-CH_2-O-CH_2-NH-C_2H_5$, $-CH_2-O-CH_2-NH-CH(CH_3)_2$, $-CH_2-S-CH_2-NH-C(CH_3)_3$ und $-CH_2-CH_2-C(H)(CH_3)-(CH_2)_3-CH_3$ genannt.

Als geeignete substituierte Heteroalkyl-Reste seien beispielsweise $-(CH_2)-O-(CF_3)$, - $(CH_2)-O-(CHF_2)$, $-(CH_2)-O-(CH_2F)$, $-(CH_2)-S-(CF_3)$, $-(CH_2)-S-(CHF_2)$. $-(CH_2)-S-(CH_2F)$, - $(CH_2)-(CH_2)-O-(CF_3)$, $-(CF_2)-O-(CF_3)$, $-(CH_2)-(CH_2)-S-(CF_3)$ und $-(CH_2)-(CH_2)-(CH_2)-O-(CF_3)$ genannt.

Der Begriff "Heteroalkenyl" bezeichnet einen wie vorstehend beschriebenen Alkenyl-Rest, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkenyl-Reste können bevorzugt 1, 2 oder 3 Heteroatom (e) unabhängig voneinander ausgewählt aus der Gruppe bestehend aus Sauerstoff, Schwefel und Stickstoff (NH) als Kettenglied(er) aufweisen. Heteroalkenyl-Reste können bevorzugt 2- bis 12-gliedrig, besonders bevorzugt 2- bis 6-gliedrig, sein.

Als geeignete Heteroalkenyl-Reste seien beispielsweise $-CH_2-O-CH=CH_2$, $-CH=CH-O-CH=CH-CH_3$, $-CH_2-CH_2-O-CH=CH_2$, $-CH_2-S-CH=CH_2$, $-CH=CH-S-CH=CH-CH_3$, $-CH_2-CH_2-S-CH=CH_2$, $-CH_2-NH-CH=CH_2$, $-CH=CH-NH-CH=CH-CH_3$ und $-CH_2-CH_2-NH-CH=CH_2$ genannt.

Als geeignete substituierte Heteroalkenyl-Reste seien beispielsweise **-CH2-O- CH=CH-**$(CH_2)$-OH, $-CH_2-S-CH=CH-(CH_2)-NH_2$ und $-CH_2-NH-CH=CH-CN$ genannt.

Der Begriff "Heteroalkinyl" bezeichnet einen wie vorstehend beschriebenen Alkinyl-Rest, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkinyl-Reste können bevorzugt 1, 2 oder 3 Heteroatom(e) unabhängig voneinander ausgewählt aus der Gruppe bestehend aus Sauerstoff, Schwefel und Stickstoff (NH) als Kettenglied(er) aufweisen. Heteroalkinyl-Reste können bevorzugt 2- bis 12-gliedrig, besonders bevorzugt 2- bis 6-gliedrig, sein.

Als geeignete Heteroalkinyl-Reste seien beispielsweise $-CH_2-O-C\equiv CH$, $-CH_2-CH_2-O-C=CH$, $-CH_2-O-C\equiv C-CH_3$, $-CH_2-CH_2-O-C\equiv C-CH_3$. $-CH_2-S-C\equiv CH$, $-CH_2-CH_2-S-C\equiv CH$, - $CH_2-S-C\equiv C-CH_3$, $-CH_2-CH_2-S-C\equiv C-CH_3$ genannt.

Als geeignete substituierte Heteroalkinyl-Reste seien beispielsweise $-CH_2-O-C\equiv C-Cl$, - $CH_2-CH_2-O-C\equiv C-I$, $-CHF-O-C\equiv C-CH_3$, -CHF-CH2-O-C≡C-$CH_3$, $-CH_2-S-C\equiv C-Cl$, $-CH_2-CH_2-S-C\equiv C-Cl$, $-CHF-S-C\equiv C-CH_3$, $-CHF-CH_2-S-C\equiv C-CH_3$ genannt.

Der Begriff "cycloalkyl" bedeutet im Sinne der vorliegenden Erfindung einen zyklischen gesättigten Kohlenwasserstoff-Rest mit bevorzugt 3, 4, 5, 6, 7, 8 oder 9 C-Atomen, besonders bevorzugt mit 3, 4, 5, 6 oder 7 C-Atomen, ganz besonders bevorzugt mit 5 oder 6 C-Atomen, wobei der Rest unsubstituiert oder einfach substituiert oder mehrfach gleich oder verschieden substituiert sein kann.

Als geeignete $C_{3-9}$-Cycloalkyl-Reste, die unsubstituiert oder einfach oder mehrfach substituiert sein können, seien beispielsweise Cyclopropyl, Cyclobutyl, Cyclopentyl, Cyclohexyl, Cycloheptyl, Cyclooctyl und Cyclononyl genannt. Als geeignete $C_{3-7}$-Cycloalkyl-Reste seien Cyclopropyl, Cyclobutyl, Cyclopentyl, Cyclohexyl und Cycloheptyl genannt.

Der Begriff "Cycloalkenyl" bedeutet im Sinne der vorliegenden Erfindung einen zyklischen ungesättigten Kohlenwasserstoff-Rest mit bevorzugt 3, 4, 5, 6, 7, 8 oder 9 C-Atomen, besonders bevorzugt mit 3, 4, 5, 6 oder 7 C-Atomen,

ganz besonders bevorzugt mit 5 oder 6 C-Atomen, der wenigstens eine Doppelbindung, vorzugsweise eine Doppelbindung, aufweist und unsubstituiert oder einfach substituiert oder mehrfach gleich oder verschieden substituiert sein kann.

Als geeignete $C_{3-9}$-Cycloalkenyl-Reste, die unsubstituiert oder einfach oder mehrfach substituiert sein können, seien Cyclobutenyl, Cyclopentenyl, Cyclohexenyl, Cycloheptenyl, Cyclononenyl und Cyclooctenyl genannt. Als geeignete $C_{5-6}$-Cycloalkenyl-Reste seien Cyclopentenyl und Cyclohexenyl genannt.

Der Begriff "Heterocycloalkyl" bedeutet im Sinne der vorliegenden Erfindung einen zyklischen gesättigten Kohlenwasserstoff-Rest mit bevorzugt 3, 4, 5, 6, 7, 8 oder 9 C-Atomen, besonders bevorzugt mit 3, 4, 5, 6 oder 7 C-Atomen, ganz besonders bevorzugt mit 5 oder 6 C-Atomen, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heterocycloalkyl-Reste können bevorzugt 1, 2 oder 3 Heteroatom(e) unabhängig voneinander ausgewählt aus der Gruppe bestehend aus Sauerstoff, Schwefel und Stickstoff (NH) als Ringglied(er) aufweisen. Ein Heterocycloalkyl-Rest kann unsubstituiert oder einfach substituiert oder mehrfach gleich oder verschieden substituiert sein. Heterocycloalkyl-Reste können bevorzugt 3- bis 9-gliedrig, besonders bevorzugt 3- bis 7-gliedrig, ganz besonders bevorzugt 5- bis 7-gliedrig, sein.

Als geeignete 3- bis 9-gliedrige Heterocycloalkyl-Reste, die unsubstituiert oder einfach oder mehrfach substituiert sein können, seien beispielsweise Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Oxetanyl, Azepanyl, Azocanyl, Diazepanyl, Dithiolanyl, (1,3)-Dioxolan-2-yl, Isoxazolidinyl, Isothioazolidinyl, Pyrazolidinyl, Oxazolidinyl, (1,2,4)-Oxadiazolidinyl, (1,2,4)-Thiadiazolidinyl, (1,2,4)-Triazolidin-3-yl, (1,3,4)-Thiadiazolidin-2-yl, (1,3,4)-Triazolidin-1-yl, (1,3,4)-Triazoldidin-2-yl, Tetrahydropyridazinyl, Tetrahydropyrimidinyl, Tetrahydropyrazinyl, (1,3,5)-Tetrahydrotriazinyl, (1,2,4)-Tetrahydrotriazin-1-yl, (1,3)-Dithian-2-yl und (1,3)-Thiazolidinyl genannt. Als geeignete 5- bis 7-gliedrige Heterocycloalkyl-Reste seien beispielsweise Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Oxetanyl, Azepanyl, Diazepanyl und (1,3)-Dioxolan-2-yl genannt.

Der Begriff "Heterocycloalkenyl" bedeutet im Sinne der vorliegenden Erfindung einen zyklischen ungesättigten Kohlenwasserstoff-Rest mit bevorzugt 4, 5, 6, 7, 8 oder 9 C-Atomen, besonders bevorzugt mit 4, 5, 6 oder 7 C-Atomen, ganz besonders bevorzugt mit 5 oder 6 C-Atomen, der wenigstens eine Doppelbindung, vorzugsweise eine Doppelbindung, aufweist und in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heterocycloalkenyl-Reste können bevorzugt 1, 2 oder 3 Heteroatom(e) unabhängig voneinander ausgewählt aus der Gruppe bestehend aus Sauerstoff, Schwefel und Stickstoff (NH) als Ringglied(er) aufweisen. Ein Heterocycloalkenyl-Rest kann unsubstituiert oder einfach substituiert oder mehrfach gleich oder verschieden substituiert sein. Heterocycloalkenyl-Reste können bevorzugt 4- bis 9-gliedrig, besonders bevorzugt 4- bis 7-gliedrig, ganz besonders bevorzugt 5- bis 7-gliedrig, sein.

Als geeignete Heterocycloalkenyl-Reste bzw. als geeignete 5- bis 7-gliedrige Heterocycloalkenyl-Reste, die unsubstituiert oder einfach oder mehrfach substituiert sein können, seien beispielsweise (2,3)-Dihydrofuranyl, (2,5)-Dihydrofuranyl, (2,3)-Dihydrothienyl, (2,5)-Dihydrothienyl, (2,3)-Dihydropyrrolyl, (2,5)-Dihydropyrrolyl, (2,3)-Dihydroisoxazolyl, (4,5)-Dihydroisoxazolyl, (2,5)-Dihydroisothiazolyl, (2,3)-Dihydropyrazolyl, (4,5)-Dihydropyrazolyl, (2,5)-Dihydropyrazolyl, (2,3)-Dihydrooxazolyl, (4,5)-Dihydrooxazolyl, (2,5)-Dihydrooxazolyl, (2,3)-Dihydrothiazolyl, (4,5)-Dihydrothiazolyl, (2,5)-Dihydrothiazolyl, (2,3)-Dihydroimidazolyl, (4,5)-Dihydroimidazolyl, (2,5)-Dihydroimidazolyl, (3,4,5,6)-Tetrahydropyridin-2-yl, (1,2,5,6)-Tetrahydropyridin-1-yl, (1,2)-Dihydropyridin-1-yl, (1,4)-Dihydropyridin-1-yl, Dihydropyranyl und (1,2,3,4)-Tetrahydropyridin-1-yl genannt.

Die Cycloalkyl-Reste, Heterocycloalkyl-Reste, Cycloalkenyl-Reste oder Heterocycloalkenyl-Reste können im Sinne der vorliegenden Erfindung mit einem unsubstituierten oder wenigstens einfach substituierten mono- bzw. bizyklischem Ringsystem kondensiert (anneliert) sein. Unter einem mono- bzw. bizyklischem Ringsystem werden im Sinne der vorliegenden Erfindung mono- bzw. bizyklische Kohlenwasserstoffreste verstanden, die gesättigt, ungesättigt oder aromatisch sein und ggf. eines oder mehrere Heteroatome als Ringglieder aufweisen können. Vorzugsweise sind die Ringe der vorstehend genannten mono- oder bizyklischen Ringsysteme jeweils 4-, 5- oder 6-gliedrig und können jeweils bevorzugt ggf. 0, 1, 2, 3, 4 oder 5 Heteroatom(e), besonders bevorzugt ggf. 0, 1 oder 2 Heteroatom(e) als Ringglied(er) aufweisen, die unabhängig voneinander aus der Gruppe bestehend aus Sauerstoff, Stickstoff und Schwefel ausgewählt sind. Sofern ein bizyklisches Ringsystem vorliegt, können die verschiedenen Ringe, jeweils unabhängig voneinander, einen unterschiedlichen Sättigungsgrad aufweisen, d.h. gesättigt, ungesättigt oder aromatisch sein.

Sofern einer oder mehrere der Substituenten ein monozyklisches oder bizyklischem Ringsystem aufweisen, das einfach oder mehrfach substituiert ist, kann dieses bevorzugt mit ggf. 1, 2, 3, 4 oder 5, besonders bevorzugt mit ggf. 1, 2 oder 3, Substituenten substituiert sein, die unabhängig voneinander ausgewählt werden können aus der Gruppe bestehend aus F, Cl, Br, I, -CN, $-NO_2$, -OH, -SH, $-NH_2$, Oxo (=O), Thioxo (=S), -C(=O)-OH, $C_{1-5}$-Alkyl, $-C_{2-5}$-Alkenyl, $-C_{2-5}$-Alkinyl, $-C{=}C-Si(CH_3)_3$, $-C{=}C-Si(C_2H_5)_3$, $-(CH_2)-O-C_{1-5}$-Alkyl, $-S-C_{1-5}$-Alkyl, -S-Phenyl, $-S-CH_2$-Phenyl, $-O-C_{1-5}$-Alkyl, - O-Phenyl, $-O-CH_2$-Phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, $-S(=O)_2-C_{1-5}$-Alkyl), $-S(=O)-C_{1-5}$-Alkyl, $-NH-C_{1-5}$--Alkyl, $N(C_{1-5}$Alkyl)($C_{1-5}$-Alkyl), $-C(=O)-O-C_{1-5}$-Alkyl, -C(=O)-H, $-C(=O)-C_{1-5}$-Alkyl, - $CH_2$-O-C(=O)-Phenyl, -O-C(=O)-Phenyl, $-NH-S(=O)_2-C_{1-5}$-Alkyl, -NH-C

(=O)-$C_{1-5}$-Alkyl, - C(=O)-$NH_2$, -C(=O)NH-$C_{1-5}$-Alkyl, -C(=O)-N($C_{1-5}$-Alkyl)$_2$, Pyrazolyl, Phenyl, Furyl (Furanyl), Thiadiazolyl, Thiophenyl (Thienyl) und Benzyl, wobei die vorstehend genannten $C_{1-5}$-Alkyl-Reste jeweils linear oder verzweigt sein können und die zyklischen Substituenten bzw. die zyklischen Reste dieser Substituenten selbst jeweils mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, -$CF_3$, -OH, -$NH_2$, -O-$CF_3$, - SH, -O-$C_{1-5}$-Alkyl, -O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-Si($CH_3$)$_3$, -C=C-Si ($C_2H_5$)$_3$, -C(=O)-O-$C_{1-5}$-Alkyl) und -C(=O)-$CF_3$ substituiert sein können.

Besonders bevorzugt können die Substituenten, jeweils unabhängig voneinander, ausgewählt werden aus der Gruppe bestehend aus F, Cl, Br, I, -CN, -$NO_2$ Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C=C-Si($CH_3$)$_3$, -C=C-Si($C_2H_5$)$_3$, -C≡C-Si($CH_3$)$_3$, -C≡C-Si($C_2H_5$)$_3$, - $CH_2$-O-$CH_3$, -$CH_2$-O-$C_2H_5$, -OH, -SH, -$NH_2$, Oxo (=O), -C(=O)-OH, -S-$CH_3$, -S-$C_2H_5$, - S(=O)-$CH_3$, -S(=O)$_2$-$CH_3$, -S (=O)-$C_2H_5$, -S(=O)$_2$-$C_2H_5$, -O-$CH_3$, -O-$C_2H_5$, -O-$C_3H_7$, -0-C($CH_3$)$_3$, -$CF_3$, -$CHF_2$, -$CH_2$F, -O-$CF_3$, -O-$CHF_2$, -O-$CH_2$F, -C(=O)-$CF_3$, -S-$CF_3$, -S-$CHF_2$, -S-$CH_2$F, -S(=O)$_2$-Phenyl, Pyrazolyl, Phenyl, -N($CH_3$)$_2$, -N($C_2H_5$)$_2$, -NH-$CH_3$, -NH-$C_2H_5$, -$CH_2$-O-C(=O)-Phenyl, -NH-S(=O)$_2$-$CH_3$, -C(=O)-O-$CH_3$, -C(=O)-O-$C_2H_5$, -C(=O)-O-C($CH_3$)$_3$, -C(=O)-H, -C(=O)-$CH_3$, -C(=O)-$C_2H_5$, -NH-C(=O)-$CH_3$, -NH-C(=O)-$C_2H_5$, -0-C(=O)-Phenyl, -C(=O)-$NH_2$, -C(=O)-NH-$CH_3$, -C(=O)-N($CH_3$)$_2$, Phenyl, Furyl (Furanyl), Thiadiazolyl, Thiophenyl (Thienyl) und Benzyl, wobei die zyklischen Substituenten bzw. die zyklischen Reste dieser Substituenten selbst mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, -$CF_3$, -OH, -$NH_2$, -O-$CF_3$, -SH, -O-$CH_3$, -O-$C_2H_5$, -0-$C_3H_7$, Methyl, Ethyl, n-Propyl, Isopropyl, n-Butyl, 2-Butyl, Isobutyl, tert-Butyl, Ethenyl, Allyl, Ethinyl, Propinyl, -C=C-Si($CH_3$)$_3$, -C≡C-Si($C_2H_5$)$_3$, -C(=O)-O-$C_{1-5}$-Alkyl und -C(=O)-$CF_3$ substituiert sein können.

Als geeignete Cycloalkyl-Reste, Heterocycloalkyl-Reste, Cycloalkenyl-Reste oder Heterocyclalkenyl-Reste, die unsubstituiert oder einfach oder mehrfach substituiert sein können, und mit einem mono- bzw. bizyklischem Ringsystem kondensiert sind, seien beispielhaft (1,2,3,4)-Tetrahydrochinolinyl, (1,2,3,4)-Tetrahydroisochinolinyl, (2,3)-Dihydro-1H-isoindolyl, (1,2,3,4)-Tetrahydronaphthyl, (2,3)-Dihydro-benzo[1.4]dioxinyl, Benzo[1.3]dioxolyl, (3,4)-Dihydro-2H-benzo[1.4]oxazinyl und Octahydro-pyrrolo[3,4-c]pyrrolyl genannt.

Sofern einer oder mehrere der Substituenten für einen Cycloalkyl-Rest, Heterocycloalkyl-Rest, Cycloalkenyl-Rest oder Heterocyclalkenyl-Rest stehen oder einen solchen Rest aufweisen, der einfach oder mehrfach substituiert ist, kann dieser bevorzugt mit ggf. 1, 2, 3, 4 oder 5, besonders bevorzugt mit ggf. 1, 2 oder 3, Substituenten unabhängig voneinander ausgewählt aus der Gruppe bestehend aus F, Cl, Br, I, -CN, -$CF_3$, -OH, -$NH_2$, -O-$CF_3$, -SH, -O-$C_{1-5}$-Alkyl, -O-Phenyl, -O-$CH_2$-Phenyl,-($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-Si(CH3)3, -C≡C-Si($C_2H_5$)$_3$, -C(=O)-O-$C_{1-5}$-Alkyl, -C(=O)-$CF_3$, -S(=O)$_2$-$C_{1-5}$-Alkyl, -S(=O)-$C_{1-5}$-Alkyl, -S(=O)$_2$-Phenyl, Oxo (=O), Thioxo (=S), -N($C_{1-5}$-Alkyl)$_2$, - N(H)($C_{1-5}$-Alkyl), -$NO_2$, -S-$CF_3$, -C(=O)-OH, -NH-S (=O)$_2$-$C_{1-5}$-Alkyl, -NH-C(=O)-$C_{1-5}$-Alkyl, -C(=O)-H, -C(=O)-$C_{1-5}$-Alkyl, -C(=O)-$NH_2$, -C(=O)-N($C_{1-5}$-Alkyl)$_2$, -C(=O)-N(H) ($C_{1-5}$-Alkyl) und Phenyl substituiert sein, wobei die vorstehend genannten $C_{1-5}$-Alkyl-Reste jeweils linear oder verzweigt sein können und die Phenyl-Reste jeweils unsubstituiert oder mit 1, 2, 3, 4 oder 5, bevorzugt mit 1, 2, 3 oder 4, Substituenten unabhängig voneinander ausgewählt aus der Gruppe bestehend aus F, Cl, Br, I, -CN, -$CF_3$, -OH, -$NH_2$, -O-$CF_3$, - SH, -O-$C_1$-$_5$-Alkyl, -O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-Si($CH_3$)$_3$, -C=C-Si($C_2H_5$)$_3$, -C(=O)-O-$C_{1-5}$-Alkyl und -C(=O)-$CF_3$ substituiert sein können.

Besonders bevorzugt können die Substituenten, jeweils unabhängig voneinander, ausgewählt werden aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, - C≡C-Si($CH_3$)$_3$, -C≡C-Si($C_2H_5$)$_3$, -OH, Oxo, Thioxo, -O-$CH_3$, -O-$C_2H_5$, -O-$C_3H_7$, -($CH_2$)-O-$CH_3$, -($CH_2$)-O-$C_2H_5$, -$NH_2$, -N($CH_3$)$_2$, -N($C_2H_5$)$_2$, -NH-$CH_3$, -NH-$C_2H_5$, -$NO_2$, -$CF_3$, -O-$CF_3$, -S-$CF_3$, -SH, -S-$CH_3$, -S-$C_2H_5$, -S(=OFCH$_3$, -S(=O)$_2$-$CH_3$, -S(=O)$C_2H_5$, -S(=O)$_2$-$C_2H_5$, -NH-S(=O)$_2$-$CH_3$, -C(=O)-OH, -C(=O)-H: -C(=O)-$CH_3$, -C (=O)-$C_2H_5$, -C(=O)-N($CH_3$)$_2$, -C(=O)-NH-$CH_3$, -C(=O)-$NH_2$, -NH-C(=O)-$CH_3$. -NH-C(=O)-$C_2H_5$, -C(=O)-O-$CH_3$, -C (=O)-O-$C_2H_5$, -C(=O)-O-C($CH_3$)$_3$ und Phenyl, wobei der Phenyl-Rest mit 1, 2, 3, 4 oder 5, bevorzugt 1, 2 oder 3, Substituenten unabhängig voneinander ausgewählt aus der Gruppe bestehend aus F, Cl, Br, I, -CN, -$CF_3$, -OH, -$NH_2$, -O-$CF_3$, -SH, -O-$CH_3$, -O-$C_2H_5$, -O-$C_3H_7$, Methyl, Ethyl, n-Propyl, Isopropyl, n-Butyl, 2-Butyl, Isobutyl, tert-Butyl, Ethenyl, Allyl, Ethinyl, Propinyl, -C≡C-Si($CH_3$)$_3$, -C≡C-Si($C_2H_5$)$_3$, -C(=O)-O-$C_{1-5}$-Alkyl und -C(=O)-$CF_3$ substituiert sein kann.

Der Begriff "Phenylen" bezeichnet einen zweiwertigen 6-gliedrigen aromatischen Kohlenwasserstoffrest der folgenden Struktur.

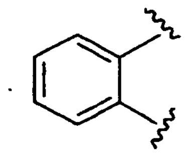

Sofern $R^1$ und $R^2$ zusammen mit den sie verbindenden Kohlenstoffatomen einen unsubstituierten oder wenigstens einfach substituierten Phenylen-Rest bilden ergibt sich zusammen mit dem Thiazolyl-Rest der allgemeinen Formel I ein unsubstituierter oder wenigstens einfach substituierter Benzothiazolyl-Rest der folgenden Struktur:

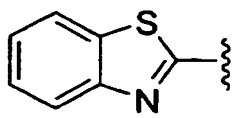

Der Begriff "Aryl" bedeutet im Sinne der vorliegenden Erfindungen einen mono- oder polyzyklischen, bevorzugt einen mono- oder bizyklischen, aromatischen Kohlenwasserstoff-Rest mit bevorzugt 6, 10 oder 14 C-Atomen. Ein Aryl-Rest kann unsubstituiert oder einfach substituiert oder mehrfach gleich oder verschieden substituiert sein. Als geeignete Aryl-Reste seien beispielsweise Phenyl-, 1-Naphthyl, 2-Naphthyl und Anthracenyl genannt. Besonders bevorzugt ist ein Aryl-Rest ein Phenyl-Rest.

Der Begriff "Heteroaryl" bedeutet im Sinne der vorliegende Erfindung einen monozyklischen oder polyzyklischen, bevorzugt einen mono-, bi- oder trizyklischen, aromatischen Kohlenwasserstoff-Rest mit bevorzugt 5, 6, 7, 8, 9, 10, 11, 12, 13 oder 14 C-Atomen, besonders bevorzugt mit 5, 6, 9, 10; 13 oder 14 C-Atomen, ganz besonders bevorzugt mit 5 oder 6 C-Atomen, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroaryl-Reste können bevorzugt 1, 2, 3, 4 oder 5, besonders bevorzugt 1, 2 oder 3, Heteroatom(e) unabhängig voneinander ausgewählt aus der Gruppe bestehend aus Sauerstoff, Schwefel und Stickstoff (NH) als Ringglied(er) aufweisen. Ein Heteroaryl-Rest kann unsubstituiert oder einfach substituiert oder mehrfach gleich oder verschieden substituiert sein.

Als geeignete Heteroaryl-Reste seien beispielsweise Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Pyrimidinyl, Indazolyl, Chinoxalinyl, Chinazolinyl, Chinolinyl, Naphthridinyl und Isochinolinyl genannt.

Aryl- oder Heteroaryl-Reste können im Sinne der vorliegenden Erfindung mit einem mono- bzw. bizyklischem Ringsystem kondensiert (anneliert) sein.

Beispielhaft für Aryl-Reste, die mit einem mono- bzw. bizyklischen Ringsystem kondensiert sind, seien (1,2,3,4)-Tetrahydrochinolinyl, (1,2,3,4)-Tetrahydroisochinolinyl, (2,3)-Dihydro-1H-isoindolyl, (1,2,3,4)-Tetrahydronaphthyl, (2,3)-Dihydrobenzo[1.4]dioxinyl, Benzo[1.3]dioxolyl und (3,4)-Dihydro-2H-benzo[1.4]oxazinyl genannt.

Sofern einer oder mehrere der Substituenten für einen Phenylen-, Aryl- oder Heteroaryl-Rest stehen oder einen Aryl- oder Heteroaryl-Rest aufweisen, der einfach oder mehrfach substituiert ist, kann dieser bevorzugt mit ggf. 1, 2, 3, 4 oder 5, besonders bevorzugt mit ggf. 1, 2 oder 3, Substituenten unabhängig voneinander ausgewählt aus der Gruppe bestehend aus F, Cl, Br, I, -CN, $-NO_2$, -OH, -SH, $-NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl, $-C{\equiv}C-Si(CH_3)_3$, $-C{\equiv}C-Si(C_2H_5)_3$, - $S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, - $CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, - S(=O)-Phenyl, $-S(=O)_2-C_{1-5}$-Alkyl, $-S(=O)-C_{1-5}$-Alkyl, $-NH-C_{1-5}$-Alkyl, $N(C_{1-5}Alkyl)_2$, - $C(=O)-O-C_{1-5}$-Alkyl, -C(=O)-H; $-C(=O)-C_{1-5}$-Alkyl, $-CH_2-O-C(=O)$-Phenyl, -O-C(=O)-Phenyl, $-NH-S(=O)_2-C_{1-5}$-Alkyl, $-NH-C(=O)-C_{1-5}$-Alkyl, $-C(=O)-NH_2$, $-C(=O)-NH-C_{1-5}$-Alkyl, $-C(=O)-N(C_{1-5}\text{-Alkyl})_2$, Pyrazolyl, Phenyl, Furyl (Furanyl), Thiazolyl, Thiadiazolyl, Thiophenyl (Thienyl) , Benzyl und Phenethyl substituiert sein, wobei die vorstehend genannten $C_{1-5}$-Alkyl-Reste jeweils linear oder verzweigt sein können und die zyklischen Substituenten bzw. die zyklischen Reste dieser Substituenten selbst mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, $-NO_2$, -OH, -SH, $-NH_2$,-C(=O)-OH, $-C_{1-5}$-Alkyl, $-(CH_2)-O-C_{1-5}$-Alkyl, $-C_{2-5}$-Alkenyl, $-C_{2-5}$-Alkirlyl, $-C{\equiv}C-Si(CH_3)_3$, - $C{\equiv}C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-0-CH_2$-Phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein können.

Besonders bevorzugt können die Substituenten, jeweils unabhängig voneinander ausgewählt werden aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl,

n-Pentyl, neo-Pentyl, Ethenyl, Allyl, Ethinyl; Propinyl, -C≡C-Si($CH_3$)$_3$. -C≡C-Si($C_2H_5$)$_3$; -$CH_2$-O-$CH_3$, -$CH_2$-O-$C_2H_5$, -OH, -SH, -$NH_2$, -C(=O)-OH, -S-$CH_3$, -S-$C_2H_5$, -S(=O)-$CH_3$, -S(=O)$_2$-$CH_3$, -S(=O)-$C_2H_5$, - S(=O)$_2$-$C_2H_5$, -O-$CH_3$, -O-$C_2H_5$, -O-$C_3H_7$, -O-C($CH_3$)$_3$, -$CF_3$, -$CHF_2$, -$CH_2$F, -O-$CF_3$, -0-$CHF_2$, -O-$CH_2$F, -C(=O)-$CF_3$, -S-$CF_3$, -S-$CHF_2$, -S-$CH_2$F, -S (=O)$_2$-Phenyl, Pyrazolyl, Phenyl, -N($CH_3$)$_2$, -N($C_2H_5$)$_2$, -NH-$CH_3$, -NH-$C_2H_5$, -$CH_2$-O-C(=O)-Phenyl, -NH-S(=O)$_2$-$CH_3$, -C(=O)-O-$CH_3$, -C(=O)-O-$C_2H_5$, -C(=O)-O-C($CH_3$)$_3$, -C(=O)-H, -C(=O)-$CH_3$, -C(=O)-$C_2H_5$, -NH-C(=O)-$CH_3$, -NH-C (=O)-$C_2H_5$, -O-C(=O)-Phenyl, -C(=O)-$NH_2$, -C(=O)-NH-$CH_3$, -C(=O)-N($CH_3$)$_2$, Phenyl, Furyl (Furanyl), Thiadiazolyl, Thiophenyl (Thienyl) und Benzyl, wobei die zyklischen Substituenten bzw. die zyklischen Reste dieser Substituenten selbst jeweils mit ggf. 1, 2, 3, 4, oder 5, bevorzugt mit ggf. 1, 2, 3 oder 4, Substituenten unabhängig voneinander ausgewählt aus der Gruppe bestehend aus F, Cl, Br, I, -CN, -$NO_2$, -OH, -SH, -$NH_2$, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, - C≡C-Si($CH_3$)$_3$, -C≡C-Si($C_2H_5$)$_3$, -$CH_2$-O-$CH_3$, -$CH_2$-O-$C_2H_5$, -S-$CH_3$, -S-$C_2H_5$, -S(=O)-$CH_3$, -S(=O)$_2$$CH_3$, -S(=O)-$C_2H_5$, -S(=O)$_2$-$C_2H_5$, -O-$CH_3$, -O-$C_2H_5$, -O-$C_3H_7$, -O-C($CH_3$)$_3$, - $CF_3$, -$CHF_2$, -$CH_2$F, -O-$CF_3$, -O-$CHF_2$, -O-$CH_2$F, -C(=O)-$CF_3$, -S-$CF_3$, -S-$CHF_2$ und -S-$CH_2$F substituiert sein können.

Ganz besonders bevorzugt kann ein substituierter Aryl-Rest aus der Gruppe bestehend aus 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxy-phenyl, 3-Hydroxy-phenyl, 4-Hydroxy-phenyl, 2-Amino-phenyl, 3-Amino-phenyl, 4-Amino-phenyl, 2-Dimethylamino-phenyl; 3-Dimethylamino-phenyl, 4-Dimethylamino-phenyl, 2-Methylamino-phenyl, 3-Methylamino-phenyl, 4-Methylamino-phenyl, 2-Acetyl-phenyl, 3-Acetyl-phenyl, 4-Acetyl-phenyl, 2-Methylsulfinyl-phenyl, 3-Methylsulfinyl-phenyl, 4-Methylsulfinyl-phenyl, 2-Methylsulfonyl-phenyl, 3-Methylsulfonyl-phenyl, 4-Methylsulfonyl-phenyl; 2-Methoxy-phenyl, 3-Methoxy-phenyl, 4-Methoxy-phenyl, 2-Chlor-phenyl, 3-Chlor-phenyl, 4-Chlor-phenyl, 2-Ethoxy-phenyl, 3-Ethoxy-phenyl, 4-Ethoxyphenyl, 2-Trifluormethyl-phenyl, 3-Trifluormethyl-phenyl, 4-Trifluormethyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 2-Nitro-phenyl, 3-Nitro-phenyl, 4-Nitro-phenyl, 2-Ethyl-phenyl, 3-Ethyl-phenyl, 4-Ethyl-phenyl, 2-Propyl-phenyl, 3-Propyl-phenyl, 4-Propyl-phenyl, 2-Isopropyl-phenyl, 3-Isopropyl-phenyl, 4-Isopropyl-phenyl, 2-tert-Butyl-phenyl, 3-tert-Butyl-phenyl, 4-tert-Butyl-phenyl, 2-Carboxyphenyl, 3-Carboxyphenyl, 4-Carboxyphenyl, 2-Ethenyl-phenyl, 3-Ethenyl-phenyl, 4-Ethenyl-phenyl, 2-Ethinyl-phenyl, 3-Ethinyl-phenyl, 4-Ethinyl-phenyl, 2-Allyl-phenyl, 3-Allyl-phenyl, 4-Allyl-phenyl, 2-Trimethylsilarlylethinyl-phenyl, 3-Trimethylsilanylethinyl-phenyl, 4-Trirnethylsilanylethinyl-phenyl, 2-Formyl-phenyl, 3-Formyl-phenyl, 4-Formyl-phenyl, 2-Acetamino-phenyl, 3-Acetamino-phenyl, 4-Acetamino-phenyl, 2-Dimethylaminocarbonyl-phenyl, 3-Dimethylaminocarbonyl-phenyl, 4-Dimethylaminocarbonyl-phenyl, 2-Methoxymethyl-phenyl, 3-Methoxymethyl-phenyl, 4-Methoxymethyl-phenyl, 2-Ethoxymethyl-phenyl, 3-Ethoxymethyl-phenyl, 4-Ethoxymethyl-phenyl, 2-Aminocarbonyl-phenyl, 3-Aminocarbonyl-phenyl, 4-Aminocarbonyl-phenyl, 2-Methylaminocarbonyl-phenyl, 3-Methylaminocarbonyl-phenyl, 4-Methylaminocarbonyl-phenyl, 2-Carboxymethylester-phenyl, 3-Carboxymethylester-phenyl, 4-Carboxymethylester-phenyl, 2-Carboxyethylester-phenyl, 3-Carboxyethylester-phenyl, 4-Carboxyethylester-phenyl, 2-Carboxy-tert-butylester-phenyl, 3-Carboxy-tert-butylester-phenyl, 4-Carboxy-tert-butylester-phenyl, 2-Methylmercapto-phenyl, 3-Methylmercapto-phenyl, 4-Methylmercapto-phenyl, 2-Ethylmercapto-phenyl, 3-Ethylmercapto-phenyl, 4-Ethylmercaptophenyl, 2-Biphenyl, 3-Biphenyl, 4-Biphenyl, 2-Bromphenyl, 3-Bromphenyl, 4-Bromphenyl, 2-Iod-phenyl, 3-Iodphenyl, 4-Iodphenyl, 2-Trifluormethoxy-phenyl, 3-Trifluormethoxy-phenyl, 4-Trifluormethoxy-phenyl, 2-Fluor-3-trifluormethylphenyl, 2-Fluor-4-methyl-phenyl, (2,3)-Difluorphenyl, (2,3)-Dimethyl-phenyl, (2,3)-Dichlorphenyl, 3-Fluor-2-trifluormethylphenyl, (2,4)-Dichlor-phenyl, (2,4)-Difluorphenyl, 4-Fluor-2-trifluormethyl-phenyl, (2,4)-Dimethoxyphenyl, 2-Chlor-4-fluor-phenyl, 2-Chlor-4-nitro-phenyl, 2-Chlor-4-methyl-phenyl, 2-Chlor-5-trifluormethyl-phenyl, 2-Chlor-5-methoxy-phenyl, 2-Brom-5-trifluormethyl-phenyl, 2-Brom-5-methoxy-phenyl, (2,4)-Dibrom-phenyl, (2,4)-Dimethyl-phenyl, 2-Fluor-4-trifluormethyl-phenyl, (2,5)-Difluor-phenyl, 2-Fluor-5-trifluormethyl-phenyl, 5-Fluor-2-trifluormethyl-phenyl, 5-Chlor-2-trifluormethyl-phenyl, 5-Brom-2-trifluormethyl-phenyl, (2,5)-Dimethoxy-phenyl, (2,5)-Bis-trifluormethyl-phenyl, (2,5)-Dichlor-phenyl, (2,5)-Dibrom-phenyl, 2-Methoxy-5-nitro-phenyl, 2-Fluor-6-trifluormethyl-phenyl, (2,6)-Dimethoxy-phenyl, (2,6)-Dimethyl-phenyl, (2,6)-Dichlor-phenyl, 2-Chlor-6-fluor-phenyl, 2-Brom-6-chlor-phenyl, 2-Brom-6-fluor-phenyl, (2,6)-Difluor-phenyl, (2,6)-Difluor-3-methyl-phenyl, (2,6)-Dibrom-phenyl, (2,6)-Dichlorphenyl, 3-Chlor-2-fluor-phenyl, 3-Chlor-5-methyl-phenyl, (3,4)-Dichlorphenyl, (3,4)-Dimethyl-phenyl, 3-Methyl-4-methoxy-phenyl, 4-Chlor-3-nitro-phenyl, (3,4)-Dimethoxy-phenyl, 4-Fluor-3-trifluormethylphenyl, 3-Fluor-4-trifluormethyl-phenyl, (3,4)-Difluor-phenyl, 3-Cyano-4-fluor-phenyl, 3-Cyano-4-methyl-phenyl, 3-Cyano-4-methoxy-phenyl, 3-Brom-4-fluor-phenyl, 3-Brom-4-methyl-phenyl, 3-Brom-4-methoxy-phenyl, 4-Chlor-2-fluor-phenyl, 4-Chlor-3-trifluormethyl, 4-Brom-3-methyl-phenyl, 4-Brom-5-methyl-phenyl, 3-Chlor-4-fluor-phenyl, 4-Fluor-3-nitro-phenyl, 4-Brom-3-nitro-phenyl, (3,4)-Dibrom-phenyl, 4-Chlor-3-methyl-phenyl, 4-Brom-3-methyl-phenyl, 4-Fluor-3-methyl-phenyl, 3-Fluor-4-methyl-phenyl, 3-Fluor-5-methyl-phenyl, 2-Fluor-3-methyl-phenyl, 4-Methyl-3-nitro-phenyl, (3,5)-Dimethoxy-phenyl, (3,5)-Dimethyl-phenyl, (3,5)-Bis-trifluormethyl-phenyl, (3,5)-Difluor-phenyl, (3,5)-Dinitro-phenyl, (3,5)-Dichlor-phenyl, 3-Fluor-5-trifluormethyl-phenyl, 5-Fluor-3-trifluormethyl-phenyl, (3,5)-Dibrom-phenyl, 5-Chlor-4-fluor-phenyl, 5-Chlor-4-fluor-phenyl, 5-Brom-4-methyl-phenyl, (2,3,4)-Trifluorphenyl, (2,3,4)-Trichlorphenyl, (2,3,6)-Trifluor-phenyl, 5-Chlor-2-methoxy-phenyl, (2,3)-Difluor-4-methyl, (2,4,5)-Trifluor-phenyl, (2,4,5)-Trichlor-phenyl, (2,4)-Dichlor-5-fluor-phenyl, (2,4,6)-Trichlor-phenyl, (2,4,6)-Trimethylphenyl, (2,4,6)-Trifluor-phenyl, (2,4,6)-Trimethoxy-phenyl, (3,4,5)-Trimethoxy-phenyl, (2,3,4,5)-Tetrafluor-phenyl, 4-Methoxy-(2,3,6)-trimethyl-phenyl, 4-Methoxy-(2,3,6)-trimethyl-phenyl, 4-Chlor-2,5-dimethyl-phenyl, 2-Chlor-6-fluor-3-methyl-phenyl, 6-Chlor-2-fluor-3-methyl, (2,4,6)-Trimethylphenyl und (2,3,4,5,6)-Pentafluor-phenyl ausgewählt werden.

Ganz besonders bevorzugt kann ein substituierter Heteroaryl-Rest aus der Gruppe bestehend aus 3-Methyl-pyrid-2-yl, 4-Methyl-pyrid-2-yl, 5-Methyl-pyrid-2-yl, 6-Methyl-pyrid-2-yl, 2-Methyl-pyrid-3-yl, 4-Methyl-pyrid-3-yl, 5-Methyl-pyrid-3-yl, 6-Methyl-pyrid-3-yl, 2-Methyl-pyrid-4-yl, 3-Methyl-pyrid-4-yl, 3-Fluor-pyrid-2-yl, 4-Fluor-pyrid-2-yl, 5-Fluor-pyrid-2-yl, 6-Fluor-pyrid-2-yl, 3-Chlor-pyrid-2-yl, 4-Chlor-pyrid-2-yl, 5-Chlor-pyrid-2-yl, 6-Chlor-pyrid-2-yl, 3-Trifluormethyl-pyrid-2-yl, 4-Trifluormethyl-pyrid-2-yl, 5-Trifluormethyl-pyrid-2-yl, 6-Trifluormethyl-pyrid-2-yl, 3-Methoxy-pyrid-2-yl, 4-Methoxy-pyrid-2-yl, 5-Methoxy-pyrid-2-yl, 6-Methoxy-pyrid-2-yl, 4-Methyl-thiazol-2-yl, 5-Methyl-thiazol-2-yl, 4-Trifluormethyl-thiazol-2-yl, 5-Trifluormethyl-thiazol-2-yl, 4-Chlor-thiazol-2-yl, 5-Chlor-thiazol-2-yl, 4-Brom-thiazol-2-yl, 5-Brom-thiazol-2-yl, 4-Fluor-thiazol-2-yl, 5-Fluor-thiazol-2-yl, 4-Cyano-thiazol-2-yl, 5-Cyano-thiazol-2-yl, 4-Methoxy-thiazol-2-yl, 5-Methoxy-thiazol-2-yl, 4-Methyl-oxazol-2-yl, 5-Methyl-oxazol-2-yl; 4-Trifluornethyl-oxazol-2-yl, 5-Trifluormethyl-oxazol-2-yl, 4-Chlor-oxazol-2-yl, 5-Chlor-oxazol-2-yl, 4-Brom-oxazol-2-yl; 5-Brom-oxazol-2-yl, 4-Fluor-oxazol-2-y1, 5-Fluor-oxazol-2-yl, 4-Cyano-oxazol-2-yl, 5-Cyano-oxazol-2-yl, 4-Methoxy-oxazol-2-yl, 5-Methoxy-oxazol-2-yl, 2-Methyl-(1,2,4)-thiadiazol-5-yl, 2-Trifluormethyl-(1,2,4)-thiadiazol-5-yl, 2-Chlor-(1,2,4)-thiadiazol-5-yl, 2-Fluor-(1,2,4)-thiadiazol-5-yl, 2-Methoxy-(1,2,4)-thiadiazol-5-yl, 2-Cyano-(1,2,4)-thiadiazol-5-yl, 2-Methyl-(1,2,4)-oxadiazol-5-yl, 2-Trifluormethyl-(1,2,4)-oxadiazol-5-yl, 2-Chlor-(1,2,4)-oxadiazol-5-yl, 2-Fluor-(1,2,4)-oxadiazol-5-yl, 2-Methoxy-(1,2,4)-oxadiazol-5-yl und 2-Cyano-(1,2,4)-oxadiazol-5-yl ausgewählt werden.

Der Begriff "Alkylen" umfaßt im Sinne der vorliegenden Erfindung acyclische gesättigte Kohlenwasserstoffketten, die einen Aryl-, Heteroaryl-, Cycloalkyl-, Heterocycloalkyl-, Cycloalkenyl- oder Heterocycloalkenyl-Rest mit dem substituierten Thiazol der allgemeinen Formel I bzw. mit einem anderen Substituenten verbinden. Alkylen-Ketten können verzweigt oder geradkettig sowie unsubstituiert oder wenigstens einfach substituiert sein mit wie im Fall von $C_{1-12}$-Alkylen 1 bis 12 (d. h. 1, 2, 3, 4, 5, 6, 7, 8, 9, 10, 11 oder 12) C-Atomen, mit wie im Fall von $C_{1-6}$-Alkylen 1 bis 6 (d. h. 1, 2, 3, 4, 5 oder 6) C-Atomen bzw. mit wie im Fall von $C_{1-3}$-Alkylen 1 bis 3 (d. h. 1, 2 oder 3) C-Atomen. Beispielhaft seien $C_{1-6}$-Alkylen-Gruppen wie $-(CH_2)-$, $-(CH_2)_2-$, $-C(H)(CH_3)-$, - $(CH_2)_3-$, $-(CH_2)_4-$, $-(CH_2)_5-$, $-C(CH_3)_2-$, $-C(H)(CH_3)-$, $-C(H)(C(H)(CH_3)_2)-$ und $C(C_2H_5)(H)$-genannt. Als geeignete $C_{1-3}$-Alkylen-Gruppe seien beispielhaft $-(CH_2)-$, $-(CH_2)_2-$ und -$(CH_2)_3$-genannt.

Der Begriff "Alkenylen" umfaßt im Sinne der vorliegenden Erfindung azyklische ungesättigte Kohlenwasserstoffketten, die einen Aryl-, Heteroaryl-, Cycloalkyl-, Heterocyloalkyl-, Cycloalkenyl- oder Heterocycloalkenyl-Rest mit dem substituierten Thiazol der allgemeinen Formel I bzw. mit einem anderen Substituenten verbinden. Alkenylen-Ketten weisen wenigstens eine Doppelbindung, bevorzugt 1, 2 oder 3 Doppelbindungen, auf und können verzweigt oder geradkettig sowie unsubstituiert oder wenigstens einfach substituiert sein mit wie im Fall von $C_{2-12}$-Alkenylen 2 bis 12 (d: h. 2, 3, 4, 5, 6, 7, 8, 9, 10, 11 oder 12) C-Atomen, mit wie im Fall von $C_{2-6}$-Alkenylen 2 bis 6 (d. h. 2, 3, 4, 5 oder 6) C-Atomen bzw. mit wie im Fall von $C_{2-3}$-Alkenylen 2 bis 3 (d. h. 2 oder 3) C-Atomen. Beispielhaft seien $C_{2-3}$-Alkenylen-Gruppen wie -CH=CH- und $-CH_2-CH=CH-$ genannt.

Der Begriff "Alkinylen" umfaßt im Sinne der vorliegenden Erfindung azyklische ungesättigte Kohlenwasserstoffketten, die einen Aryl-, Heteroaryl-, Cycloalkyl-, Heterocyloalkyl-, Cycloalkenyl- oder Heterocycloalkenyl-Rest mit dem substituierten Thiazol der allgemeinen Formel I bzw. mit einem anderen Substituenten verbinden. Alkinylen-Ketten weisen wenigstens eine Dreifachbindung, bevorzugt 1 oder 2 Dreifachbindungen, auf und können verzweigt oder geradkettig sowie unsubstituiert oder wenigstens einfach substituiert sein mit wie im Fall von $C_{2-12}$-Alkinylen 2 bis 12 (d. h. 2, 3, 4, 5, 6, 7, 8, 9, 10, 11, oder 12) C-Atomen, mit wie im Fall von $C_{2-6}$-Alkinylen 2 bis 6 (d. h. 2, 3, 4, 5 oder 6) C-Atomen bzw. mit wie im Fall von $C_{2-3}$-Alkinylen 2 bis 3 (d. h. 2 oder 3) C-Atomen. Beispielhaft seien $C_{2-3}$-Alkinylen-Gruppen wie -C≡C- und $-CH_2-C≡C-$ genannt.

Der Begriff "Heteroalkylen" bezeichnet eine wie vorstehend beschriebenen AlkylenKette, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkylen-Gruppen können bevorzugt 1, 2 oder 3 Heteroatom (e), besonders bevorzugt ein Heteroatom, ausgewählt aus der Gruppe bestehend aus Sauerstoff, Schwefel und Stickstoff (NH) als Kettenglied(er) aufweisen. Heteroalkylen-Gruppen können bevorzugt 2- bis 12-gliedrig, besonders bevorzugt 2- bis 6-gliedrig, ganz besonders bevorzugt 2- oder 3-gliedrig, sein.

Beispielhaft seien Heteroalkylen-Gruppen wie $-(CH_2)-O-$, $-(CH_2)_2-O-$, $-(CH_2)_3-O-$, - $(CH_2)_4-O-$, $-O-(CH_2)-$, $-O-(CH_2)_2-$, $-O-(CH_2)_3-$, $-O-(CH_2)_4-$, $-C(C_2H_5)(H)-O-$, $-0-C(C_2H_5)(H)-$, $-CH_2-O-CH_2-$, $-CH_2-S-CH_2-$, $-CH_2-NH-CH_2-$, $-CH_2-NH-$ und $-CH_2-CH_2-NH-CH_2-CH_2$ genannt.

Der Begriff "Heteroalkenylen" bezeichnet eine wie vorstehend beschriebene Alkenyle-Kette, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkenylen-Gruppen können bevorzugt 1, 2 oder 3 Heteroatom(e), besonders bevorzugt 1 Heteroatom, ausgewählt aus der Gruppe bestehend aus Sauerstoff, Schwefel und Stickstoff (NH) als Kettenglied(er) aufweisen. Heteroalkenylen-Gruppen können bevorzugt 2- bis 12-gliedrig, besonders bevorzugt 2-bis 6-gliedrig, ganz besonders bevorzugt 2- oder 3-gliedrig, sein. Beispielhaft seien Heteroalkenylen-Gruppen wie -CH=CH-NH-, -CH=CH-O- und -CH=CH-S- genannt.

Sofern einer oder mehrere der Substituenten für eine Alkylen-, Alkenylen-, Alkinylen-, Heteroalkylen- oder Heteroalkenylen-Gruppe stehen oder eine solche Gruppe aufweisen, die einfach oder mehrfach substituiert ist, kann diese bevorzugt mit ggf. 1, 2, 3, 4 oder 5, besonders bevorzugt mit ggf. 1, 2 oder 3, Substituenten unabhängig voneinander ausgewählt aus der Gruppe bestehend aus Phenyl, F, Cl, Br, I, $-NO_2$, -CN, -OH, -O-Phenyl, $-O-CH_2$-Phenyl, -SH, -S-Phenyl, $-S-CH_2$-Phenyl, $-NH_2$, $-N(C_{1-5}\text{-Alkyl})_2$,-NH-Phenyl, (Phenyl)-$N(C_{1-5}$-Alkyl)(Phenyl), $-N(C_{1-5}$-Alkyl)($CH_2$-Phenyl), $-N(C_{1-5}$-Alkyl)($CH_2$-$CH_2$-Phenyl), -C(=O)-H, -C(=O)-$C_{1-5}$-Alkyl, -C(=O)-Phenyl, -C(=S)-$C_{1-5}$-Alkyl,-C(=S)-Phenyl,- C (=O)-OH, -C(=O)-O-$C_{1-5}$-Alkyl, -C(=O)-O-Phenyl, -C(=O)-$NH_2$, -C(=O)-NH-$C_{1-5}$-Alkyl, -C(=O)-$N(C_{1-5}\text{-Alkyl})_2$, -S (=O)-$C_{1-5}$-Alkyl, -S(=O)-Phenyl, $-S(=O)_2-C_{1-5}$-Alkyl, $-S(=O)_2$-Phenyl, $-S(=O)_2-NH_2$ und $-SO_3H$ substituiert sein, wobei die vorstehend genannten $C_{1-5}$-Alkyl-Reste jeweils linear oder verzweigt sein können und die vorstehend genannten Phenyl-Reste mit 1, 2, 3, 4 oder 5, bevorzugt mit 1, 2, 3 oder 4, Substituenten unabhängig voneinander ausgewählt aus der Gruppe bestehend aus F, Cl, Br, I, -CN, $-NO_2$, -OH, -SH, $-NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl, $-C{\equiv}C-Si(CH_3)_3$, $-C{\equiv}C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein können.

Besonders bevorzugt können Alkylen-, Alkenylen-, Alkinylen-, Heteroalkylen- oder Heteroalkenylen- Gruppen mit 1, 2 oder 3 Substituenten unabhängig voneinander ausgewählt werden aus der Gruppe bestehend aus Phenyl, F, Cl, Br, I, $-NO_2$, -CN, -OH, -O-Phenyl, -SH, -S-Phenyl, $-NH_2$, $-N(CH_3)_2$, $-N(C_2H_5)_2$ und $-N(CH_3)(C_2H_5)$ substituierte sein, wobei der Phenyl-Rest mit 1, 2, 3, 4 oder 5 Substituenten unabhängig voneinander ausgewählt aus der Gruppe bestehend aus F, Cl, Br, I, -OH. -SH, $-NO_2$, -CN, $-O-CH_3$,$-O-CF_3$ und $-O-C_2H_5$ substituiert sein kann.

Sofern Verbindungen der allgemeinen Formel I mehrere Substituenten ausgewählt aus der Gruppe bestehend aus $R^{10}$, $R^{11}$, $R^{12}$ , $R^{13}$, $R^{14}$, $R^{15}$, $R^{16}$ , $R^{11}$, $R^{18}$, $R^{19}$, $R^{20}$, $R^{21}$ , $R^{22}$, $R^{23}$ $R^{24}$, $R^{25}$, $R^{26}$ , $R^{27}$ , $R^{28}$ , $R^{29}$ , $R^{30}$ , $R^{31}$, $R^{32}$, $R^{33}$, $R^{34}$, $R^{35}$ , $R^{36}$, $R^{37}$, $R^{38}$, $R^{39}$, $R^{40}$, $R^{41}$ $R^{42}$, $R^{43}$, $R^{44}$, $R^{45}$, $R^{46}$, $R^{47}$, $R^{48}$, $R^{49}$, $R^{50}$, $R^{51}$, $R^{52}$ und $R^{53}$ mit der gleichen Bezeichnung aufweisen, kann jeder dieser Substituenten jeweils unabhängig von anderen Substituenten mit der gleichen Bezeichnung des Substituenten ausgewählt werden. Beispielhaft kann der folgende Rest,

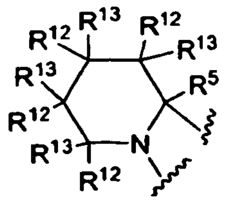

nach Auswahl der entsprechenden Substituenten für diesen Rest stehen

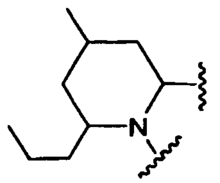

Bevorzugt sind substituierte Thiazole der vorstehend angegebenen allgemeinen Formel I, worin

$R^1$ und $R^2$, unabhängig voneinander, jeweils für H; F; Cl; Br; I; $-NO_2$; -CN; $-NH_2$; -OH;-SH; -C(=O)-OH; -C(=O)-H; -NH-C(=O)-H; -NH-$R^{55}$; -$NR^{56}R^{57}$; -C(=O)-$R^{58}$; -C(=O)-O-$R^{59}$; -O-C(=O)-$R^{60}$; -NH-C(=O)-$R^{61}$; -$NR^{62}$-C(=O)-$R^{63}$; -C(=O)-$NH_2$; -C(=O)-NH-$R^{64}$; -C(=O)-$NR^{65}R^{66}$; -O-$R^{67}$; -S-$R^{68}$; -S(=O)-$R^{69}$; $-S(=O)_2-R^{70}$; -NH-C(=O)-NH-$R^{71}$; -NH-C(=S)-NH-$R^{72}$ ; $-NH-S(=O)_2-R^{73}$; $-NR^{74}-S(=O)_2-R^{75}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, - (Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder wenigstens einfach substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, -(Heteroalkylen)-Aryl oder -(Heteroalkenylen)-Aryl; oder unsubstituiertes oder wenigstens einfach substituiertes -(Alkylen)-Heteroaryl, - (Alkenylen)-Heteroaryl, -(Alkinylen)-Heteroaryl, -(Heteroalkylen)-Heteroaryl oder - (Heteroalkenylen)-Heteroaryl stehen;

oder $R^1$ und $R^2$ zusammen mit den sie verbindenden Kohlenstoffatomen einen unsubstituierten oder wenigstens einfach substituierten Phenylen-Rest bilden;

$R^3$, $R^8$ und $R^{54}$, unabhängig voneinander, jeweils für H; -C(=O)-$R^{58}$; -C(=O)-O-$R^{59}$;-C(=O)-$NH_2$; -C(=O)-NH-$R^{64}$; -C(=O)-N$R^{65}R^{66}$; -S(=O)-$R^{69}$; -$S(=O)_2$-$R^{70}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, - (Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes-(Heteroalkylen)-Cycloalkyl, -(Heteroalkenylen)-Cycloalkyl, -(Heteroalkylen)-Cycloalkenyl oder -(Heteroalkenylen)- Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenylen)-Heterocycloalkyl, -(Heteroalkylen)-Heterocycloalkenyl oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, -(Heteroalkylen)-Aryl oder -(Heteroalkenylen)-Aryl; oder unsubstituiertes oder substituiertes -(Alkylen)-Heteroaryl, -(Alkenylen)- Heteroaryl, -(Alkinylen)-Heteroaryl, -(Heteroalkylen)-Heteroaryl oder -(Heteroalkenylen)-Heteroaryl stehen;

$R^4$, $R^5$, $R^6$, $R^7$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ , $R^{27}$ $R^{28}$ $R^{29}$ $R^{30}$ , $R^{31}$, $R^{32}$, $R^{33}$ , $R^{34}$ , $R^{35}$ , $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$ , $R^{51}$ $R^{52}$ und $R^{53}$, unabhängig voneinander, jeweils für H; F; Cl; Br, I; -$NO_2$; -CN; -$NH_2$; -OH; -SH; -C(=O)-OH; -C(=O)-H; -NH-C(=O)-H; -NH-$R^{55}$; - N$R^{56}R^{57}$ ; -C(=O)-$R^{58}$; -C(=O)-O-$R^{59}$; -O-C(=O)-$R^{60}$; -NH-C(=O)-$R^{61}$; -N$R^{62}$-C(=O)-$R^{63}$; C(=O)-$NH_2$; -C(=O)-NH-$R^{64}$; -C(=O)-N$R^{65}R^{66}$; -O-$R^{67}$; -S-$R^{68}$; -S(=O)-$R^{69}$; -$S(=O)_2$-$R^{70}$;-NH-C(=O)-NH-$R^{71}$; -NH-C(=S)-NH-$R^{72}$; -NH-$S(=O)_2$-$R^{73}$; -N$R^{74}$-$S(=O)_2$-$R^{75}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, -(Alkinylen)-Cycloalkyl, -(Alkylen)-Cycloalkenyl, - (Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenylen)-Heterocycloalkyl, - (Heteroalkylen)-Heterocycloalkenyl oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, - (Heteroalkylen)-Aryl oder -(Heteroalkenylen)-Aryl, oder unsubstituiertes oder substituiertes -(Alkylen)-Heteroaryl, -(Alkenylen)-Heteroaryl, -(Alkinylen)-Heteroaryl,-(Heteroalkylen)-Heteroaryl oder -(Heteroalkenylen)-Heteroaryl stehen;

oder $R^4$ und $R^5$ oder $R^6$ und $R^7$ oder $R^{10}$ und $R^{11}$ oder $R^{12}$ und $R^{13}$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$ oder $R^{28}$ und $R^{29}$ oder $R^{30}$ und $R^{31}$ oder $R^{32}$ und $R^{33}$ oder $R^{34}$ und $R^{35}$ oder $R^{36}$ und $R^{37}$ oder $R^{38}$ und $R^{39}$ oder $R^{40}$ und $R^{41}$ oder $R^{42}$ und $R^{43}$ oder $R^{44}$ und $R^{45}$ oder $R^{46}$ und $R^{47}$ oder $R^{48}$ und $R^{49}$ oder $R^{50}$ und $R^{51}$ oder $R^{52}$ und $R^{53}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^3$ und $R^4$ zusammen mit der sie verbindenden -N-C$R^5$-Gruppe einen Rest der allgemeinen Formel A,

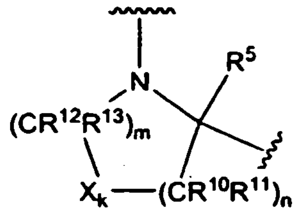

A,

bilden;

oder $R^6$ und $R^8$ zusammen mit der sie verbindenden -N-$CR^7$-Gruppe einen Rest der allgemeinen Formel B,

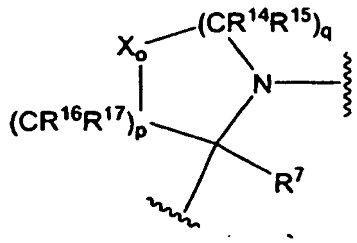

B,

bilden;
m und q jeweils für 1, 2, 3, 4 oder 5 stehen;
n und p jeweils für 0, 1, 2, 3 oder 4 stehen;
k und o jeweils für 0 oder 1 stehen; wobei die Summe aus m, n und k oder die Summe aus p, q und o jeweils gleich 1, 2, 3, 4, 5 oder 6 ist;

oder $R^3$ und $R^6$ zusammen mit der sie verbindenden -N-$CR^4R^5$-$CR^7$-Gruppe einen Rest der allgemeinen Formel C,

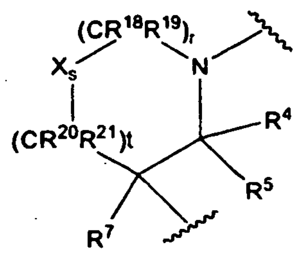

C,

bilden;

oder $R^4$ und $R^8$ zusammen mit der sie verbindenden -$CR^5$-$CR^6R^7$-N-Gruppe einen Rest der allgemeinen Formel D,

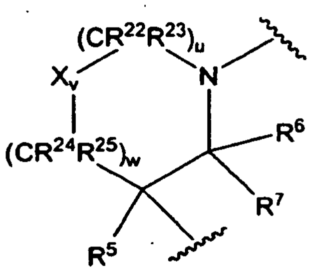

D,

bilden;
r und u jeweils für 1, 2, 3 oder 4 stehen;
t und w jeweils für 0, 1, 2 oder 3 stehen;
s und v jeweils für 0 oder 1 stehen;
wobei die Summe aus r, s und t oder die Summe aus u, v und w jeweils gleich 1, 2, 3, 4 oder 5 ist;

oder $R^3$ und $R^8$ zusammen mit der sie verbindenden -N-$CR^4R^5$-$CR^6R^7$-N-Gruppe einen Rest der allgemeinen Formel E,

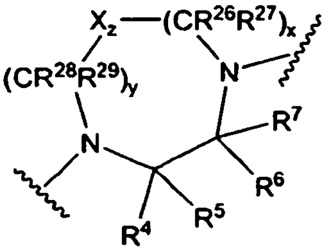

E,

bilden;
x und y jeweils für 1 oder 2 stehen;
z für 0 oder 1 steht;
wobei die Summe aus x, y und z gleich 3 oder 4 ist;

oder $R^4$ und $R^6$ zusammen mit der sie verbindenden -$CR^5$-$CR^7$-Gruppe einen Rest der allgemeinen Formel F,

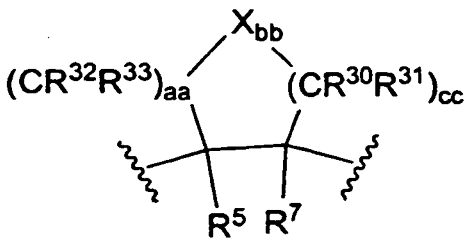

F,

bilden;
aa und cc, unabhängig voneinander, jeweils für 1, 2, 3, 4 oder 5 stehen;
bb für 0 oder 1 steht;
wobei die Summe aus aa, bb und cc gleich 1, 2, 3, 4, 5 oder 6 ist;

oder $R^3$ und $R^4$ zusammen mit der sie verbindenden -N-$CR^5$-Gruppe und $R^6$ und $R^8$ zusammen mit der sie verbindenden -N-$CR^7$-Gruppe einen Rest der allgemeinen Formel G,

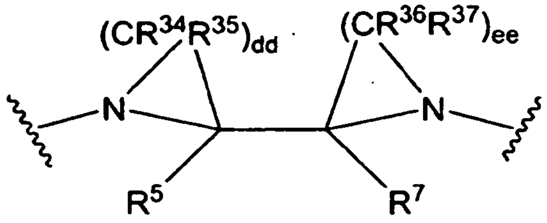

G,

bilden;
dd und ee, unabhängig voneinander, jeweils für 1, 2, 3 oder 4 stehen;

oder $R^3$ und $R^6$ zusammen mit der sie verbindenden -N-$CR^4CR^5$-$CR^7$-Gruppe und $R^4$ und $R^8$ zusammen mit der sie verbindenden -$CR^5$-$CR^6R^7$-N-Gruppe einen Rest der allgemeinen Formel H,

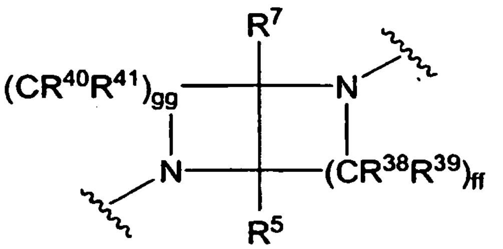

H,

bilden,
ff und gg, unabhängig voneinander, jeweils für 1, 2 oder 3 stehen;

oder $R^3$ und $R^8$ zusammen mit der sie verbindenden -N-$CR^4R^5$-$CR^6R^7$-N-Gruppe und $R^4$ und $R^6$ zusammen mit der sie verbindenden -$CR^5$-$CR^7$-Gruppe einen Rest der allgemeinen Formel K,

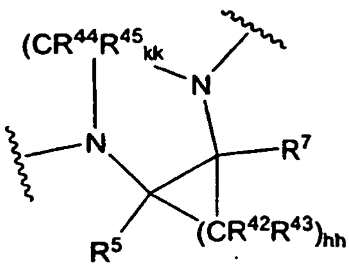

K,

bilden;
hh für 1, 2, 3 oder 4 steht;
kk für 1, 2, 3, 4, 5 oder 6 steht;

oder $R^3$ und $R^8$ zusammen mit der sie verbindenden -N-$CR^4R^5$-$CR^6R^7$-N-Gruppe einen bizyklischen Rest der allgemeinen Formel L,

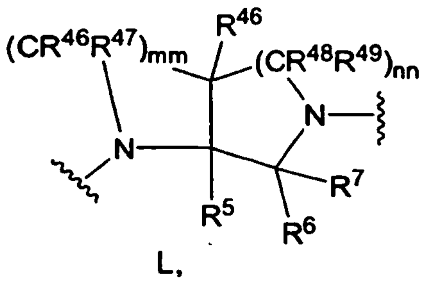

L,

bilden;
mm für 1, 2 oder 3 steht;
nn für 1 oder 2 steht;
oder $R^3$ und $R^8$ zusammen mit der sie verbindenden -N-$CR^4R^5$-$CR^6R^7$-N-Gruppe einen bizyklischen Rest der allgemeinen Formel M,

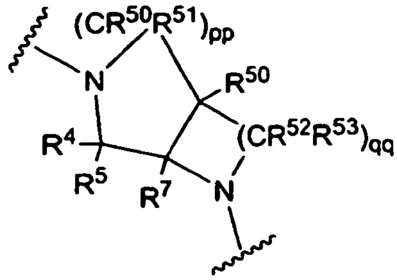

M,

bilden;
pp für 1 oder 2 steht;
qq für 1, 2 oder 3 steht;

X für O, S oder N-$R^{54}$ steht;

$R^9$ für H; F; Cl; Br; I; -CN; -C(=O)-OH; -C(=O)-H: -C(=O)-$R^{58}$; -C(=O)-O-$R^{59}$; -C(=O)-$NH_2$; -C(=O)-NH-$R^{64}$; -C (=O)-$NR^{65}R^{66}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, - (Alkinylen)-Cycloalkyl, -(Alkylen)-Cycloalkenyl, -(Alkenylen)- Cycloalkenyl oder - (Alkinylen)-Cycloalkenyl; unsubstituiertes oder substituiertes -(Heteroalkylen)-Cycloalkyl, -(Heteroalkenylen)-Cycloalkyl, -(Heteroalkylen)-Cycloalkenyl oder -(Heteroalkenylen)-Cycloalkenyl; unsubstituiertes oder substituiertes -(Alkylen)-Heterocycloalkyl, - (Alkenylen)-Heterocycloalkyl, -(Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenylen)-Heterocycloalkyl, -(Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, -(Heteroalkylen)-Aryl oder -(Heteroalkenylen)-Aryl; oder unsubstituiertes oder substituiertes -(Alkylen)-Heteroaryl,- (Alkenylen)-Heteroaryl, -(Alkinylen)-Heteroaryl, -(Heteroalkylen)-Heteroaryl oder -(Heteroalkenylen)-Heteroaryl steht;

und $R^{55}$, $R^{56}$, $R^{57}$, $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$ und $R^{75}$, unabhängig voneinander, jeweils für unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes

oder substituiertes -(Alkylen)-Cycloalkyl, - (Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes - (Heteroalkylen)-Cycloalkyl, -(Heteroalkenylen)-Cycloalkyl, -(Heteroalkylen)-Cycloalkenyl oder -(Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenylen)-Heterocycloalkyl, -(Heteroalkylen-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, -(Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl stehen;

wobei

die vorstehend genannten Alkyl-Reste jeweils verzweigt oder geradkettig sind und 1, 2, 3, 4, 5, 6, 7, 8, 9, 10, 11 oder 12 Kohlenstoffatome als Kettenglieder aufweisen;

die vorstehend genannten Alkenyl-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Alkinyl-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Heteroalkyl-Reste, Heteroalkenyl-Reste und Heteroalkinyl-Reste jeweils 2-, 3-, 4-, 5-, 6-, 7-, 8-, 9-, 10-, 11- oder 12-gliedrig sind;

die vorstehend genannten Heteroalkyl-Reste, Heteroalkenyl-Reste und Heteroalkinyl-Reste jeweils ggf. 1, 2 oder 3 Heteroatom(e) unabhängig voneinander ausgewählt aus der Gruppe bestehend aus Sauerstoff, Schwefel und Stickstoff als Kettenglied(er) aufweisen;

die vorstehend genannten Alkyl-Reste, Alkenyl-Reste, Alkinyl-Reste, Heteroalkyl-Reste, Heteroalkenyl-Reste und Heteroalkinyl-Reste jeweils mit ggf. 1, 2, 3, 4 oder 5 Substituenten unabhängig voneinander ausgewählt aus der Gruppe bestehend aus F, Cl, Br, I, $-NO_2$, -CN, -OH, -SH, $-NH_2$, $-N(C_{1-5}$-Alkyl$)_2$, $-N(C_{1-5}$-Alkyl)(Phenyl), $-N(C_{1-5}$-Alkyl)($CH_2$-Phenyl), $-N(C_{1-5}$-Alkyl)($CH_2$-$CH_2$-Phenyl), -C(=O)-H, $-C(=O)-C_{1-5}$-Alkyl, - C(=O)-Phenyl, $-C(=S)-C_{1-5}$-Alkyl, -C(=S)-Phenyl, -C(=O)-OH, $-C(=O)-O-C_{1-5}$-Alkyl, - C(=O)-O-Phenyl, $-C(=O)-NH_2$, $-C(=O)-NH-C_{1-5}$-Alkyl, -C(=O)-N($C_{1-5}$-Alkyl$)_2$, $-S(=O)-C_{1-5}$-Alkyl, -S(=O)-Phenyl, $-S(=O)_2-C_{1-5}$-Alkyl, $-S(=O)_2$-Phenyl, $-S(=O)_2-NH_2$ und $-SO_3H$ substituiert sein können, wobei die Phenyl-Reste mit 1, 2, 3, 4 oder 5 Substituenten unabhängig voneinander ausgewählt aus der Gruppe bestehend aus F, Cl, Br, I, -CN,$-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl und tert-Butyl substituiert sein können;

die vorstehend genannten Cycloalkyl-Reste jeweils 3, 4, 5, 6, 7, 8 oder 9 Kohlenstoffatome als Ringglieder aufweisen;

die vorstehend genannten Cycloalkenyl-Reste jeweils 3, 4, 5, 6, 7, 8 oder 9 Kohlenstoffatome als Ringglieder aufweisen;

die vorstehend genannten Heterocycloalkyl-Reste jeweils 3-, 4-, 5-. 6-, 7-, 8- oder 9-gliedrig sind;

die vorstehend genannten Heterocycloalkenyl-Reste jeweils 4-, 5-, 6-, 7-, 8- oder 9-gliedrig sind;

die vorstehend genannten Heterocycloalkyl-Reste und Heterocycloalkenyl-Resten jeweils ggf. 1, 2 oder 3 Heteroatom(e) unabhängig voneinander ausgewählt aus der Gruppe bestehend aus Sauerstoff, Schwefel und Stickstoff (NH) als Ringglied(er) aufweisen;

die vorstehend genannten Cycloalkyl-Reste, Heterocycloalkyl-Reste, Cycloalkenyl-Reste oder Heterocycloalkenyl-Reste jeweils mit ggf. 1, 2, 3, 4- oder 5 Substituenten unabhängig voneinander ausgewählt aus der Gruppe bestehend aus F, Cl, Br, I, -CN,$-CF_3$, -OH, $-NH_2$, $-O-CF_3$, -SH, $-O-C_{1-5}$-Alkyl, -O-Phenyl, $-O-CH_2$-Phenyl, $-(CH_2)-O-C_{1-5}$-Alkyl, $-S-C_{1-5}$-Alkyl, -S-Phenyl, $-S-CH_2$-Phenyl, $-C_{1-5}$-Alkyl, $-C_{2-5}$-Alkenyl, $-C_{2-5}$-Alkinyl, - $C\equiv C-Si(CH_3)_3$, $-C\equiv C-Si(C_2H_5)_3$; $-C(=O)-O-C_{1-5}$-Alkyl, $-C(=O)-CF_3$, $-S(=O)_2-C_{1-5}$-Alkyl, - $S(=O)-C_{1-5}$-Alkyl, -S(=O)2-Phenyl, Oxo (=O), Thioxo (=S),

-N($C_{1-5}$-Alkyl)$_2$, -N(H)($C_{1-5}$-Alkyl), -$NO_2$. -S-$CF_3$, -C(=O)-OH, -NH-S(=O)$_2$-$C_{1-5}$-Alkyl, -NH-C(=O)-$C_{1-5}$-Alkyl, -C(=O)-H, -C(=O)-$C_{1-5}$-Alkyl, -C(=O)-$NH_2$, -C(=O)-N($C_{1-5}$-Alkyl)$_2$, -C(=O)-N(H)($C_{1-5}$-Alkyl) und Phenyl substituiert sein können, wobei die Phenyl-Reste jeweils unsubstituiert oder mit 1, 2, 3, 4 oder 5 Substituenten unabhängig voneinander ausgewählt aus der Gruppe bestehend aus F, Cl, Br, I, -CN, -$CF_3$, -OH, -$NH_2$, -O-$CF_3$, -SH, -O-$C_{1-5}$-Alkyl, -O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-Si($CH_3$)$_3$, -C≡C-Si($C_2H_5$)$_3$, -C(=O)-O-$C_{1-5}$-Alkyl und - C(=O)-$CF_3$ substituiert sein können, wobei die vorstehend genannten Phenyl-Reste bevorzugt mit 1, 2, 3, 4 oder 5 Substituenten unabhängig voneinander ausgewählt aus der Gruppe bestehend aus F, Cl, Br, I, -CN, -$CF_3$, -OH, -$NH_2$, -O-$CF_3$, -SH, -O-$CH_3$, -O-$C_2H_5$, -O-$C_3H_7$, Methyl, Ethyl, n-Propyl, Isopropyl, n-Butyl, 2-Butyl, Isobutyl und tert-Butyl substituiert sein können;

die vorstehend genannten Alkylen-Reste jeweils verzweigt oder geradkettig sind und 1, 2, 3, 4, 5, 6, 7, 8, 9, 10, 11 oder 12 Kohlenstoffatome als Kettenglieder aufweisen;

die vorstehend genannten Alkenylen-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Alkinylen-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Heteroalkylen-, Heteroalkenylen-Reste und Heteroalkinylen-Reste jeweils 2-, 3-, 4-, 5-, 6-, 7-, 8-, 9-, 10-, 11- oder 12-gliedrig sind;

die vorstehend genannten Heteroalkylen-, Heteroalkenylen- und Heteroalkinylen-Gruppen jeweils ggf. 1, 2 oder 3 Heteroatom(e) unabhängig voneinander ausgewählt aus der Gruppe bestehend aus Sauerstoff, Schwefel und Stickstoff (NH) als Kettenglied(er) aufweisen;

die vorstehend genannten Alkylen-, Alkenylen-, Alkinylen-, Heteroalkylen-Heteroalkenylen- und Heteroalkinylen-Gruppen jeweils unsubstituiert oder mit ggf. 1, 2, 3, 4 oder 5 Substituenten unabhängig voneinander ausgewählt aus der Gruppe bestehend aus Phenyl, F, Cl, Br, I, -$NO_2$, -CN, -OH, -O-Phenyl, -O-$CH_2$-Phenyl, -SH, -S-Phenyl, -S-$CH_2$-Phenyl, $NH_2$, -N($C_{1-5}$-Alkyl)$_2$, -NH-Phenyl, -N($C_{1-5}$-Alkyl)(Phenyl), -N($C_{1-5}$-Alkyl)($CH_2$-Phenyl), -N($C_{1-5}$-Alkyl)($CH_2$-$CH_2$-Phenyl), -C(=O)-H, -C(=O)-$C_{1-5}$-Alkyl, - C(=O)-Phenyl, -C(=S)-$C_{1-5}$-Alkyl, -C(=S)Phenyl, -C(=O)-OH, -C(=O)-O-$C_{1-5}$-Alkyl, - C(=O)-O-Phenyl, -C(=O)-$NH_2$, -C(=O)-NH-$C_{1-5}$-Alkyl, -C(=O)-N($C_{1-5}$-Alkyl)$_2$, -S(=O)-$C_{1-5}$-Alkyl, -S(=O)-Phenyl, -S(=O)$_2$-$C_{1-5}$-Alkyl, -S(=O)$_2$Phenyl, -S(=O)$_2$-$NH_2$ und -$SO_3H$ substituiert sein können, wobei die Phenyl-Reste mit 1, 2, 3, 4 oder 5 Substituenten unabhängig voneinander ausgewählt aus der Gruppe bestehend aus F, Cl, Br, I, -CN,-$NO_2$, -OH, -SH, -$NH_2$, -C(=O)-OH, -$C_{1-5}$-Alkyl, -($CH_2$)O-$C_{1-5}$-Alkyl, -$C_{2-5}$-Alkenyl, -$C_{2-5}$-Alkinyl, -CΞC-Si($CH_3$)$_3$, -C=C-Si($C_2H_5$)$_3$, -S-$C_{1-5}$-Alkyl, -S-Phenyl, -S-$CH_2$-Phenyl, -O-$C_{1-5}$-Alkyl, -O-Phenyl, -O-$CH_2$-Phenyl, -$CF_3$, -$CHF_2$. -$CH_2F$, -O-$CF_3$, -O-$CHF_2$, -O-$CH_2F$,-C(=O)-$CF_3$, -S-$CF_3$, -S-$CHF_2$ und -S-$CH_2F$ substituiert sein können;

die vorstehend genannten Aryl-Reste mono- oder bizyklisch sind und 6, 10 oder 14-Kohlenstoffatome aufweisen;

die vorstehend genannten Heteroaryl-Reste mono-, bi- oder trizyklisch und 5-, 6-, 7-, 8-, 9-, 10-, 11-, 12-, 13- oder 14-gliedrig sind;

die vorstehend genannten 5- bis 14-gliedriges Heteroaryl-Reste ggf. 1, 2, 3, 4 oder 5 Heteroatom(e) unabhängig voneinander ausgewählt aus der Gruppe bestehend aus Sauerstoff, Schwefel und Stickstoff (NH) als Ringglied(er) aufweisen;

und die vorstehend genannten Phenylen-Reste, Aryl-Reste und Heteroaryl-Reste jeweils mit ggf. 1, 2, 3, 4 oder 5 Substituenten unabhängig voneinander ausgewählt aus der Gruppe bestehend aus F, Cl, Br, I, -CN, -$NO_2$, -OH, -SH, -$NH_2$, -C(=O)-OH, -$C_{1-5}$-Alkyl,-($CH_2$)-O-$C_{1-5}$-Alkyl, -$C_{2-5}$-Alkenyl, -$C_{2-5}$-Alkinyl, -C≡C-Si($CH_3$)$_3$, -C≡C-Si($C_2H_5$)$_3$, -S-$C_{1-5}$-Alkyl, -S-Phenyl, -S-$CH_2$-Phenyl, -O-$C_{1-5}$-Alkyl, -O-Phenyl, -O-$CH_2$-Phenyl, -$CF_3$, -$CHF_2$, -$CH_2F$, -O-$CF_3$, -O-$CHF_2$, -O-$CH_2F$, -C(=O)-$CF_3$, -S-$CF_3$, -S-$CHF_2$, -S-$CH_2F$, -S(=O)$_2$-Phenyl, -S(=O)$_2$-$C_{1-5}$-Alkyl, -S(=O)-$C_{1-5}$-Alkyl, -NH-$C_{1-5}$-Alkyl, N($C_{1-5}$Alkyl)$_2$, -C(=O)-O-$C_{1-5}$-Alkyl, -C(=O)-H, -C(=O)-$C_{1-5}$-Alkyl, -$CH_2$-O-C(=O)-Phenyl, -O-C(=O)-Phenyl, -NH-S(=O)$_2$-$C_{1-5}$-Alkyl, -NH-C(=O)-$C_{1-5}$-Alkyl, -C(=O)-$NH_2$, -C(=O)-NH-$C_{1-5}$-Alkyl, -C(=O)-N($C_{1-5}$-Alkyl)$_2$, Pyrazolyl, Phenyl, Furyl (Furanyl), Thiazolyl, Thiadiazolyl, Thiophenyl (Thienyl) , Benzyl und Phenethyl substituiert sein können, wobei die zyklischen Substituenten bzw. die zyklischen Reste dieser Substituenten selbst mit ggf. 1, 2, 3, 4 oder 5, Substituenten unabhängig voneinander ausgewählt aus der Gruppe bestehend

aus F, Cl, Br, I, -CN, $-NO_2$, -OH, -SH, $-NH_2$, -C(=O)-OH, $-C_{1-5}$-Alkyl, $-(CH_2)-O-C_{1-5}$-Alkyl,$-C_{2-5}$-Alkenyl, $-C_{2-5}$-Alkinyl, $-C\equiv C-Si(CH_3)_3$, $-C\equiv C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-0-CHF_2$, $-O-CH_2F$, $-C(=O)-CF_3$, $-S-CF_3$, $-S-CHF_2$ und $-S-CH_2F$ substituiert sein können;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Weiterhin bevorzugt sind substituierte Thiazole der vorstehend angegebenen allgemeinen Formel I, worin $R^1$ für H; F; Cl; Br; I; $-NO_2$; -CN; $-NH_2$; -OH; -SH; -C(=O)-OH; -C(=O)-H; -NH-C(=O)-H; $-NH-R^{55}$; $-NR^{56}R^{57}$; $-C(=O)-R^{58}$; $-C(=O)-O-R^{59}$; $-O-C(=O)-R^{60}$; $-NH-C(=O)-R^{61}$; $-NR^{62}-C(=O)-R^{83}$; $-C(=O)-NH_2$; $-C(=O)-NH-R^{64}$; $-C(=O)-NR^{65}R^{68}$:$-O-R^{67}$; $-S-R^{68}$; $-S(=O)-R^{69}$; $-S(=O)_2-R^{70}$; $-NH-C(=O)-NH-R^{71}$; $-NH-C(=S)-NH-R^{72}$; $-NH-S(=O)_2-R^{73}$; $-NR^{74}-S(=O)_z-R^{75}$; $C_{1-8}$-Alkyl, das unsubstituiert oder mit ggf. 1, 2, 3, 4 oder 5 Substituenten unabhängig voneinander ausgewählt aus der Gruppe bestehend aus F, Cl. Br, I, $-NO_2$, -CN, -OH, -SH und $-NH_2$ substituiert ist;
$C_{3-7}$-Cycloalkyl, $C_{5-6}$Cycloalkenyl, 5- bis 7-gliedriges Heterocycloalkyl oder 5- bis 7-gliedriges Heterocycloalkenyl, das jeweils über eine $C_{1-3}$-Alkylen-. $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$; $-NO_2$, $-CF_3$, $-O-CF_3$, $-S-CF_3$, -SH, $-S-CH_3$ und$-S-C_2H_5$ substituiert ist; oder für einen Rest ausgewählt aus der Gruppe bestehend aus Phenyl, Naphthyl, Anthracenyl, Thienyl, Furyl, Pyridinyl, Thiazolyl, Thiadiazolyl, Oxazolyl, Oxadiazolyl und Isoxazolyl steht, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl,-OH, -SH, $-NH_2$, -C(=O)-OH, $-S-CH_3$, $-S-C_2H_5$, $-S(=O)-CH_3$, $-S(=O)_2-CH_3$, $-S(=O)-C_2H_5$,$-S(=O)-C_2H_5$, $-O-CH_3$, $-O-C_2H_5$, $-O-C_3H_7$, $-CF_3$, $-CHF_2$, $-CH_2F$ und $-O-CF_3$ substituiert ist;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Ebenfalls bevorzugt sind substituierte Thiazole der vorstehend angegebenen allgemeinen Formel I, worin $R^2$ für H; F: Cl; Br; I, $-NO_2$; -CN; $-NH_2$; -OH; -SH; -C(=O)-OH; $-NH-R^{55}$; $-NR^{56}R^{57}$; $-C(=O)-R^{58}$; $-C(=O)-O-R^{59}$; $-C(=O)-NH_2$: $-C(=O)-NH-R^{64}$; $-C(=O)-NR^{65}R^{66}$. $-O-R^{67}$; $-S-R^{68}$; $-S(=O)-R^{69}$; $-S(=O)_2-R^{70}$; $-NH-C(=O)-NH-R^{71}$; $-NH-C(=O)-NH-R^{72}$ $-NH-S(=O)_2-R^{73}$; $-NR^{74}-S(=O)_2-R^{75}$; $C_{1-6}$Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl. Br, I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH. $-S-CH_3$ und$-S-C_2H_5$ substituiert ist; oder für einen Rest ausgewählt aus der Gruppe bestehend aus Phenyl, Naphthyl, Anthracenyl, Thienyl, Furyl, Pyridinyl, Thiazolyl, Thiadiazolyl, Oxazolyl, Oxadiazolyl und Isoxazolyl steht, der jeweils über eine $C_{1-3}$Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl,-OH, -SH, $-NH_2$, -C(=O)-OH, $-S-CH_3$, $-S-C_2H_5$, $-S(=O)-CH_3$, $-S(=O)_2-CH_3$, $-S(=O)-C_2H_5$, - $S(=O)_2-C_2H_5$, $-O-CH_3$, $-O-C_2H_5$, $-O-C_3H_7$, $-CF_3$, $-CHF_2$, $-CH_2F$ und $-O-CF_3$ substituiert ist;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, Insbesondere der Enantiomeren und/oder Diastereomeren, in einem beliebigen Mischungsverhältnis, oder jeweils in Form entsprechender Salze.

Weiterhin bevorzugt sind substituierte Thiazole der vorstehend angegebenen allgemeinen Formel I, worin $R^1$ und $R^2$ zusammen mit den sie verbindenden Kohlenstoffatomen einen Phenylen-Rest bilden, der unsubstituiert oder mit 1, 2, 3 oder 4 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, -OH, -SH, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-CF_3$, $-CHF_2$, $-CH_2F$ und - $O-CF_3$ substituiert ist;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von

Stereoisomeren, insbesondere der Enantiomeren und/oder Diastereomeren, in einem beliebigen Mischungsverhältnis, oder jeweils in Form entsprechender Salze.

Ebenfalls bevorzugt sind substituierte Thiazole der vorstehend angegebenen allgemeinen Formel I, worin $R^3$. $R^8$ und $R^{54}$, unabhängig voneinander, jeweils für H; - C(=O)-$R^{67}$; -C(=O)-O-$R^{59}$; -C(=O)-$NH_2$; -C(=O)-NH-$R^{64}$; -C(=O)-$NR^{65}R^{66}$; -S(=O)-$R^{69}$; - $S(=O)_2R^{70}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, -$NO_2$,-CN, -OH, -SH und -$NH_2$ substituiert ist; $C_{3-8}$-Cycloalkyl, das unsubstituiert oder mit ggf. 1, 2, 3, 4 oder 5 Substituenten unabhängig voneinander ausgewählt aus der Gruppe bestehend aus F, Cl, Br, I, -$NO_2$, -CN, -OH, -SH und -$NH_2$ substituiert ist; oder für einen Phenyl-Rest stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl,-OH, -O-$CH_3$, -O-$C_2H_5$ und -O-$C_3H_7$ substituiert ist;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Weiterhin bevorzugt sind substituierte Thiazole der vorstehend angegebenen allgemeinen Formel 1, worin $R^4$, $R^5$, $R^6$, $R^7$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{26}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{48}$, $R^{47}$, $R^{46}$, $R^{49}$, $R^{50}$, $R^{51}$, $R^{52}$ und $R^{53}$, unabhängig voneinander, jeweils für H; F: Cl; Br; I; -$NO_2$; -CN; -$NH_2$; -OH; -SH; -C(=O)-OH; -NH-$R^{55}$; -$NR^{58}R^{57}$; -C(=O)$R^{58}$; -C(=O)-O-$R^{59}$; -O-$R^{67}$; -S-$R^{68}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, -$NO_2$, -CN, -OH, -SH und -$NH_2$ substituiert ist; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-$CH_3$, -O-$C_2H_5$, -O-$C_3H_7$, -$NH_2$, -$N(CH_3)_2$, -$N(C_2H_5)_2$, -NH-$CH_3$, -NH-$C_2H_5$, -$NO_2$, -$CF_3$, -O-$CF_3$, -S-$CF_3$, -SH, -S-$CH_3$ und-S-$C_2H_5$ substituiert ist; oder für einen Rest ausgewählt aus der Gruppe bestehend aus Phenyl, Naphthyl, Anthracenyl, Pyrrolyl, Indolyl, Furanyl, Benzo[b]furanyl, Thiophenyl, Benz[b]thiophenyl. Benzo[d]thiazolyl, Pyrazolyl, Imidazolyl, Thiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Chinolinyl, Isochinolinyl und Chinazolinyl stehen, der Jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist; oder $R^4$ und $R^5$ oder $R^6$ und $R^7$ oder $R^{10}$ und $R^{11}$ oder $R^{12}$ und $R^{13}$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$ oder $R^{28}$ und $R^{29}$ oder $R^{30}$ und $R^{31}$ oder $R^{32}$ und $R^{33}$ oder $R^{34}$ und $R^{35}$ oder $R^{36}$ und $R^{37}$ oder $R^{38}$ und $R^{39}$ oder $R^{40}$ und $R^{41}$ oder $R^{42}$ und $R^{43}$ oder $R^{44}$ und $R^{45}$ oder $R^{46}$ und $R^{47}$ oder $R^{48}$ und $R^{49}$ oder $R^{50}$ und $R^{51}$ oder $R^{52}$ und $R^{53}$, unabhängig voneinander, zusammen jeweils für einen Rest ausgewählt aus der Gruppe bestehend aus einer Oxo-Gruppe (=O) und einer Thioxo-Gruppe (=S) stehen;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Ebenfalls bevorzugt sind substituierte Thiazole der vorstehend angegebenen allgemeinen Formel I, worin $R^3$ und $R^4$ zusammen mit der sie verbindenden -N-C$R^5$-Gruppe einen Rest ausgewählt aus der Gruppe bestehend aus

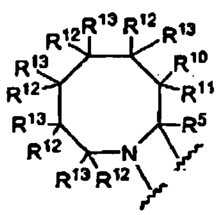
,
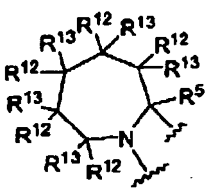
,
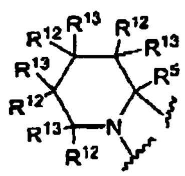
,
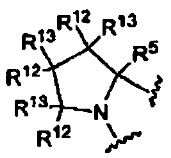
,

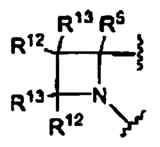, 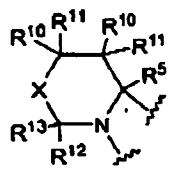, 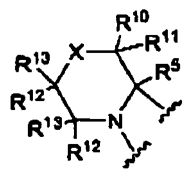, 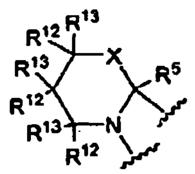,

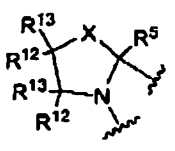, 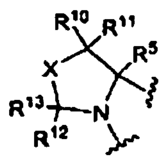, 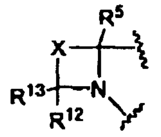, 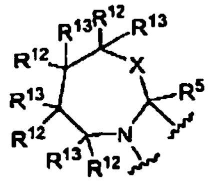,

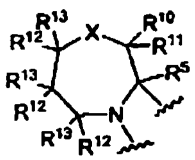, 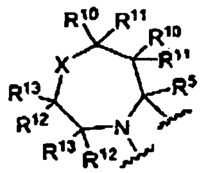, 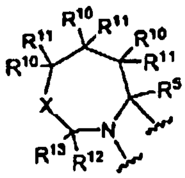, 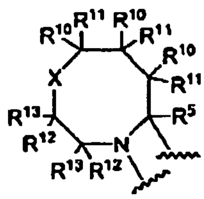,

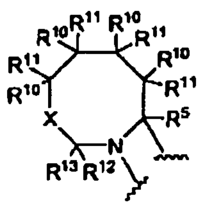, 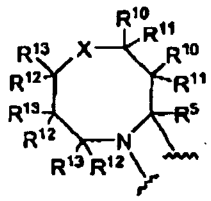, 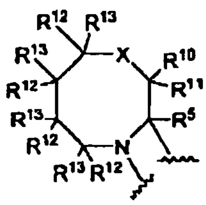 und 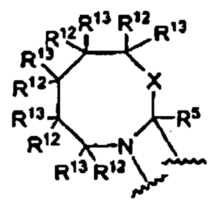

bilden;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Weiterhin bevorzugt sind substituierte Thiazole der vorstehend angegebenen allgemeinen Formel I, worin $R^6$ und $R^8$ zusammen mit der sie verbindenden -N-$CR^7$-Gruppe einen Rest ausgewählt aus der Gruppe bestehend aus

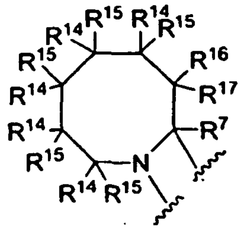, 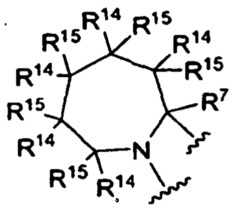, 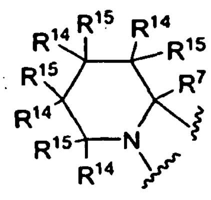, 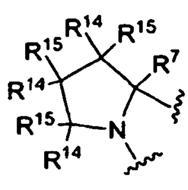,

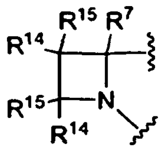 , 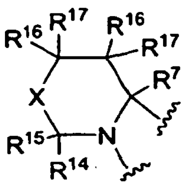 , 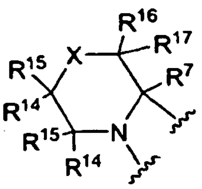 , 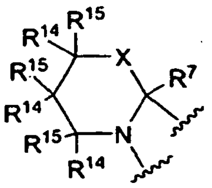 ,

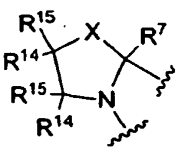 , 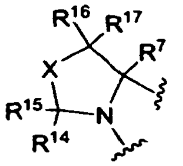 , 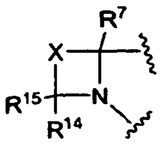 ; 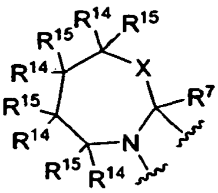 ,

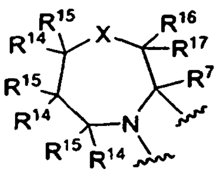 , 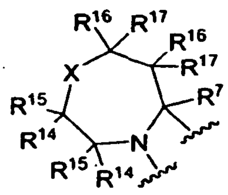 , 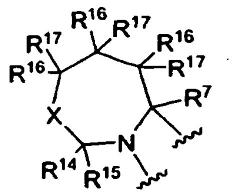 , 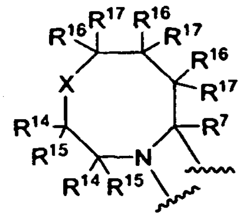 ,

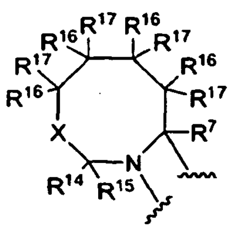 , 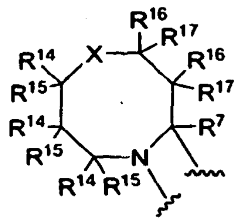 , 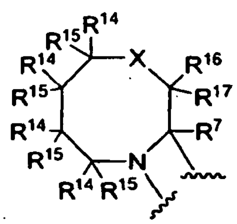 und 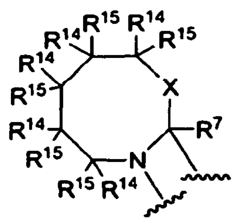

bilden;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Weiterhin bevorzugt sind substituierte Thiazole der vorstehend angegebenen allgemeinen Formel I, worin $R^3$ und $R^6$ zusammen mit der sie verbindenden -N-$CR^4R^5$-$CR^7$-Gruppe einen Rest ausgewählt aus der Gruppe bestehend aus

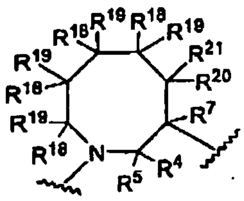 , 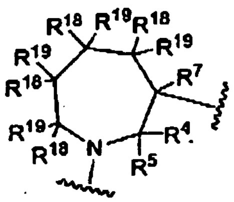 ; 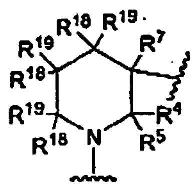 , 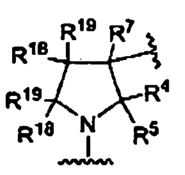 ,

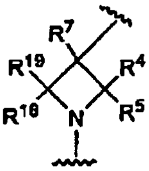, 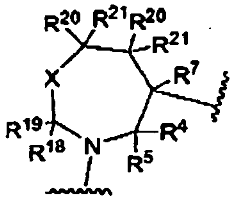, 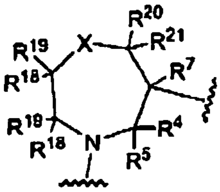, 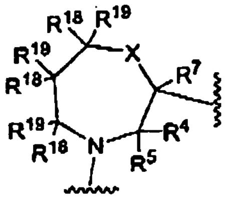,

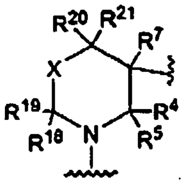, 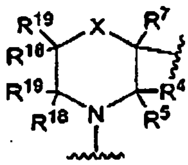 und 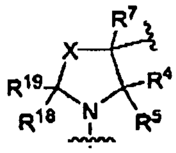

bilden:

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Ebenfalls bevorzugt sind substituierte Thiazole der vorstehend angegebenen allgemeinen Formel I. worin gekennzeichnet, dass $R^4$ und $R^8$ zusammen mit der sie verbindenden $-CR^5-CR^6CR^7-N$-Gruppe einen Rest ausgewählt aus der Gruppe bestehend aus

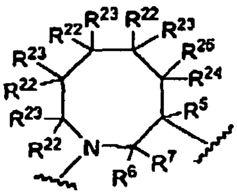, 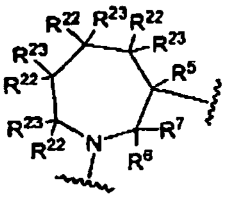, 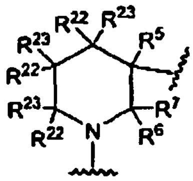, 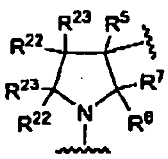,

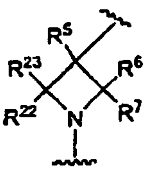, 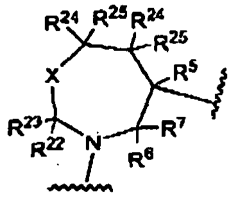, 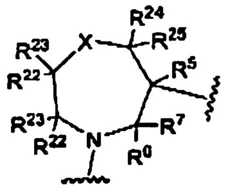, 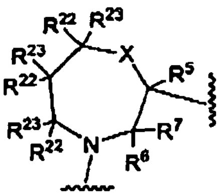,

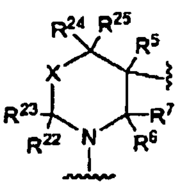, 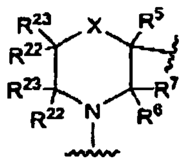 und 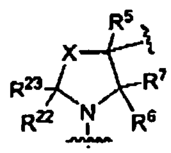

bilden:

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von

Stereoisomeren, insbesondere der Enantiomeren und/oder Diastereomeren, in einem beliebigen Mischungsverhältnis, oder jeweils in Form entsprechender Salze.

Weiterhin bevorzugt sind substituierte Thiazole der vorstehend angegebenen allgemeinen Formel I, worin $R^3$ und $R^8$ zusammen mit der sie verbindenden -N-$CR^4R^5$-$CR^6CR^7$-N -Gruppe einen Rest ausgewählt aus der Gruppe bestehend aus

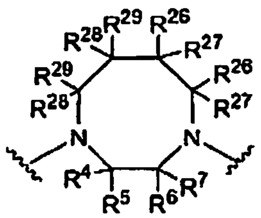 , 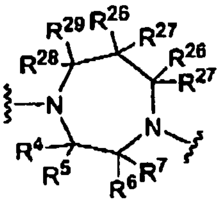 , 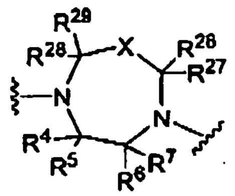 ,

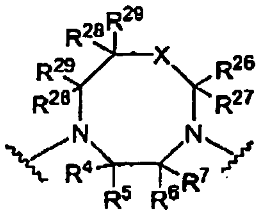 und 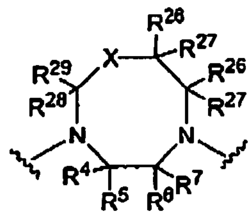

bilden;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Ebenfalls bevorzugt sind substituierte Thiazole der vorstehend angegebenen allgemeinen Formel I, worin $R^4$ und $R^6$ zusammen mit der sie verbindenden -$CR^5$-$CR^7$-Gruppe einen Rest ausgewählt aus der Gruppe bestehend aus

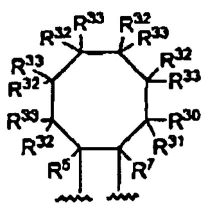 , 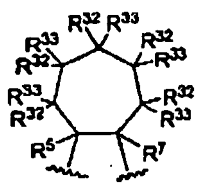 , 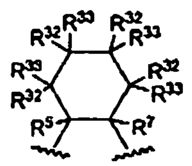 , 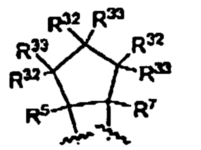 ,

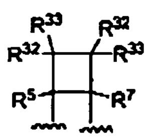 , 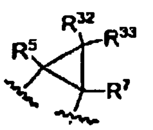 , 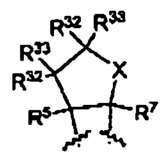 , 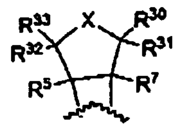 ,

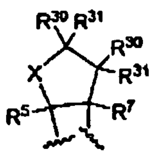 , 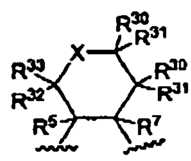 , 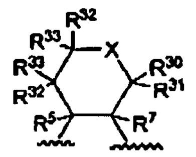 , 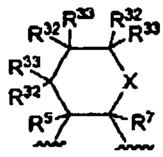 ,

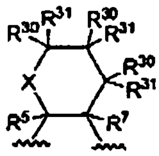 , 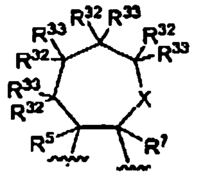 , 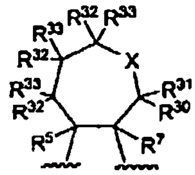 , 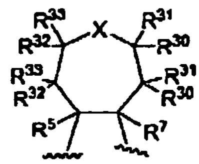 ,

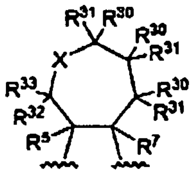 und 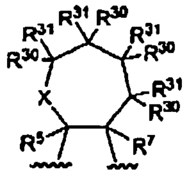

bilden;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Weiterhin bevorzugt sind substituierte Thiazole der vorstehend angegebenen allgemeinen Formel I, worin $R^3$ und $R^4$ zusammen mit der sie verbindenden -N-$CR^5$ Gruppe und $R^6$ und $R^8$ zusammen mit der sie verbindenden -N-$CR^7$-Gruppe einen Rest ausgewählt aus der Gruppe bestehend aus

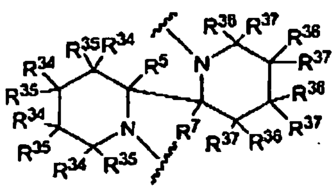 , 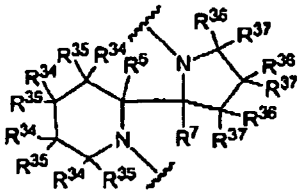 , 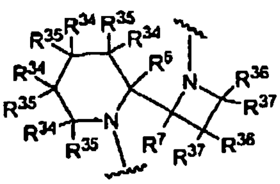 ,

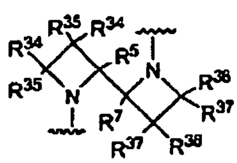 , 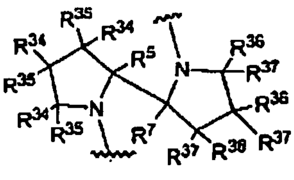 , 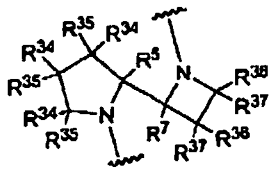 ,

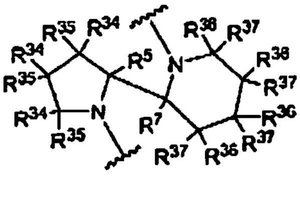 , 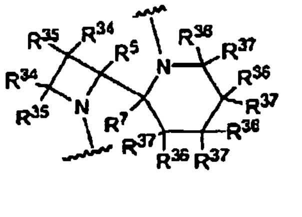 und 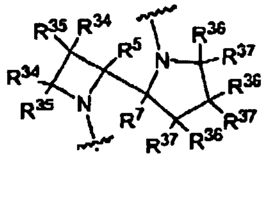

bilden;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Ebenfalls bevorzugt sind substituierte Thiazole der vorstehend angegebenen allgemeinen Formel I, worin $R^3$ und $R^6$ zusammen mit der sie verbindenden -N-$CR^4CR^5$-$CR^7$-Gruppe und $R^4$ und $R^8$ zusammen mit der sie verbindenden -$CR^5$-$CR^6R^7$-N-Gruppe einen Rest ausgewählt aus der Gruppe bestehend aus

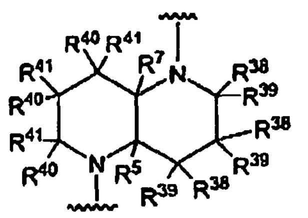 ,

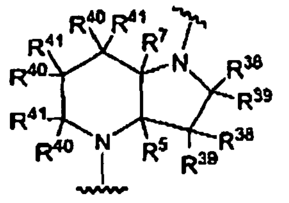 ,

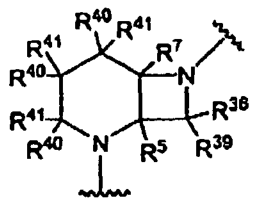 .

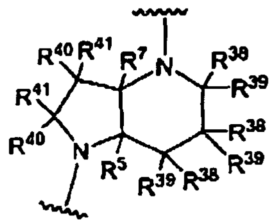 ,

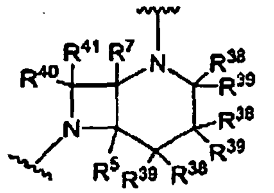 ,

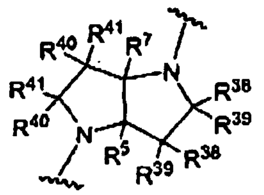 .

und

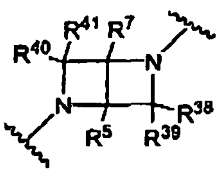

bilden;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Ebenfalls bevorzugt sind substituierte Thiazole der vorstehend angegebenen allgemeinen Formel I, worin $R^3$ und $R^8$ zusammen mit der sie verbindenden -N-$CR^4R^5$-$CR^6R^7$-N-Gruppe und $R^4$ und $R^6$ zusammen mit der sie verbindenden -$CR^5$-$CR^7$-Gruppe einen Rest ausgewählt aus der Gruppe bestehend aus

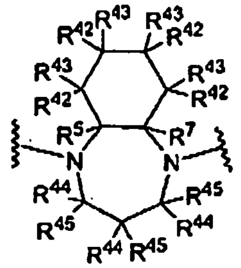 ,

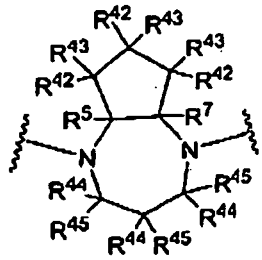 ,

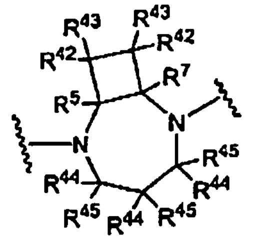 .

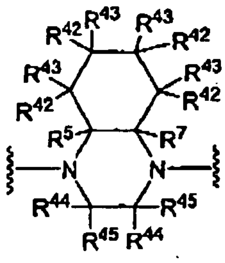 , 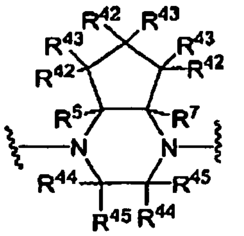 , 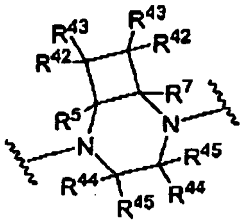 ,

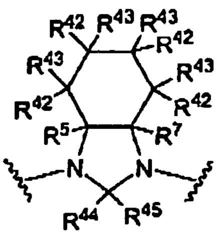 , 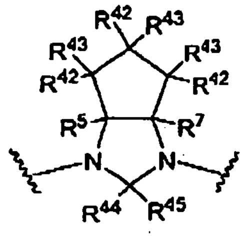 und 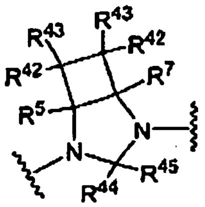

bilden;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride.

Ebenfalls bevorzugt sind substituierte Thiazole der vorstehend angegebenen allgemeinen Formel I, worin $R^3$ und $R^6$ zusammen mit der sie verbindenden -N-$CR^4R^5$-$CR^6R^7$-N-Gruppe einen bizyklischen Rest ausgewählt aus der Gruppe bestehend aus

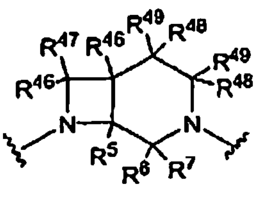 , 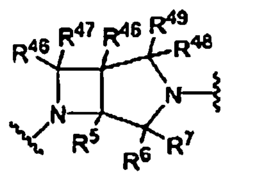 ,

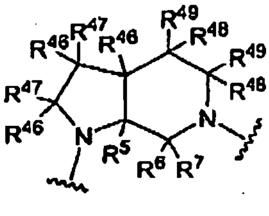 und 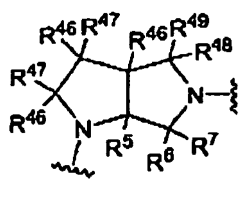

bilden:

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Weiterhin bevorzugt sind substituierte Thiazole der vorstehend angegebenen allgemeinen Formel I, worin $R^3$ und $R^8$ zusammen mit der sie verbindenden -N-$CR^4R^5$-$CR^6R^7$-N-Gruppe einen bizyklischen Rest ausgewählt aus der Gruppe

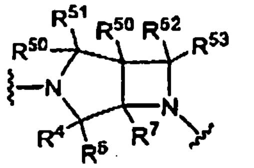, 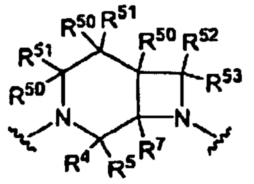,

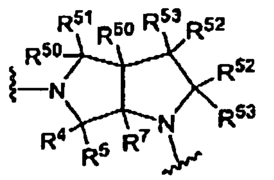 und 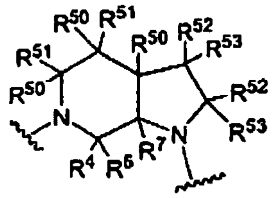

bilden;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Ebenfalls bevorzugt sind substituierte Thiazole der vorstehend angegebenen allgemeinen Formel I, worin $R^9$ für H; $-C(=O)NH_2$; $-C(=O)-NH-R^{64}$; $-C(=O)-NR^{66}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$. -CN, -OH,-SH und $-NH_2$ substituiert ist; $C_{3-7}$Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN. Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-S-CH_3$ und-S-$C_2H_5$ substituiert ist; oder für einen Rest ausgewählt aus der Gruppe bestehend aus Phenyl, Naphthyl, Anthracenyl, Furyl, Thienyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl, Imidazolyl, Indolyl, Benz[b]thiophenyl, Benzo[d]thiazolyl. Benzo[b]furanyl, Chinolinyl, Isochinolinyl und Chinazolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl. Isopropyl, n-Butyl, 2-Butyl, Isobutyl, tert-Butyl, Ethenyl, Allyl, Ethinyl, Propinyl, $-C{\equiv}C-Si(CH_3)_3$, $-C{\equiv}C-Si(C_2H_5)_3$, $-CH_2-O-CH_3$, $-CH_2-O-C_2H_5$, -OH, $-O-CH_3$, $-O-C_2H_5$, $-O-C_3H_7-S-CH_3$, $-S-C_2H_5$, $-S(=O)-CH_3$, $-S(=O)_2-CH_3$, $-S(=O)-C_2H_5$, $-S(=O)_2-C_2H_5$, $-NH_2$, -N $(CH_3)_2$, $-N(C_2H_5)_2$, $-NH-CH_3$, $-NH-C_2H_5$, $-NO_2$, $-CF_3$, $-CH_2F$, $-CHF_2$, $-O-CF_3$, $-S-CF_3$, -SH, $-NH-S(=O)_2-CH_3$, -C(=O)-OH, -C(=O)-H; $-C(=O)-CH_3$, $-C(=O)-C_2H_5$, $-C(=O)-NH_2$, $-C(=O)-N(CH_3)_2$, $-C(=O)-NH-CH_3$, $-NH-C(=O)-CH_3$, -NH-C $(=O)-C_2H_5$. $-C(=O)-O-CH_3$, $-C(=O)-O-C_2H_5$, $-C(=O)-O-C(CH_3)_3$ und Phenyl substituiert ist;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Weiterhin bevorzugt sind substituierte Thiazole der vorstehend angegebenen allgemeinen Formel I, worin $R^{55}$, $R^{56}$, $R^{57}$, $R^{58}$, $R^{59}$, $R^{60}$ $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$ und $R^{75}$, unabhängig voneinander, jeweils für $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH,-SH und $-NH_2$ substituiert ist: $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$Alkinylen-Gruppe gebunden sein kann und/oder unsubstituiert oder mit ggf.1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH.- $S-CH_3$ und-S-$C_2H_5$ substituiert ist; oder für einen Rest ausgewählt aus der Gruppe bestehend aus Phenyl, Naphthyl, Anthracenyl, Pyrrolyl, Indolyl, Furanyl, Benzo[b]furanyl, Thiophenyl, Benz[b]thiophenyl, Benzo[d]thiazolyl, Pyrazolyl, Imidazolyl, Thiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Chinolinyl, Isochinolinyl und Chinazolinyl stehen, der jeweils über eine $C_{1-3}$-Alkylen-, $C_{2-3}$-Alkenylen- oder $C_{2-3}$-Alkinylen-Gruppe

gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$. $-S-CF_3$,-SH,$-NH-S(=O)_2-CH_3$,-C(=O)-OH, $-C(=O)-CH_3$, $-C(=O)-C_2H_5$, $-C(=O)-N(CH_3)_2$, - $C(=O)-NH-CH_3$, $-NH-C(=O)-CH_3$, $-NH-C(=O)-C_2H_5$, $-C(=O)-O-CH_3$ und $-C(=O)-O-C_2H_5$ substituiert ist;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Weiterhin bevorzugt sind substituierte Thiazole der vorstehend angegebenen allgemeinen Formel I, worin

$R^1$ für H; F; Cl, Br; I; $-CF_3$; $-NO_2$; -CN; $-NH_2$; -OH: -SH; -C(=O)-OH; -C(=O)-H; -NH-C(=O)-H; $-NH-R^{55}$; $-NR^{58}R^{57}$; $-C(=O)-R^{58}$; $-C(=O)-O-R^{59}$; $-C(=O)-NH_2$; $-C(=O)-NH-R^{64}$; - $C(=O)-NR^{65}R^{66}$; $-O-R^{67}$; $-S-R^{68}$; $-S(=O)-R^{69}$; $-S(=O)_2-R^{70}$;
unsubstituiertes $C_{1-6}$-Alkyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist, steht;

$R^2$ für H; F; Cl; Br; I; $-CF_3$; $-NO_2$; -CN; $-NH_2$; -OH; -SH; -C(=O)-OH; -C(=O)-H; -NH-C(=O)-H; $-NH-R^{55}$; $-NR^{56}R^{57}$; $-C(=O)-R^{58}$; $-C(=O)-O-R^{59}$; $-C(=O)-NH_2$; $-C(=O)-NH-R^{64}$; - $C(=O)-NR^{65}R^{66}$; $-O-R^{67}$; $-S-R^{68}$; $-S(=O)-R^{69}$; $-S(=O)_2-R^{70}$;
unsubstituiertes $C_{1-6}$Alkyl oder für einen Rest ausgewählt aus der Gruppe bestehend aus (1,3)Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist, steht;

oder $R^1$ und $R^2$ zusammen mit den sie verbindenden Kohlenstoffatomen einen Phenylen-Rest bilden, der unsubstituiert oder mit 1, 2, 3 oder 4 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, -OH, -SH, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-CF_3$, $-CHF_2$, $-CH_2F$ und - $O-CF_3$ substituiert ist;

$R^3$ und $R^8$, unabhängig voneinander, jeweils für H; $-C(=O)-R^{58}$; $-C(=O)-O-R^{59}$; $-C(=O)-NH_2$; $-C(=O)-NH-R^{64}$; $-C(=O)-NR^{65}R^{66}$; $-S(=O)-R^{69}$; $-S(=O)_2-R^{70}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist;
$C_{3-6}$-Cycloalkyl, das unsubstituiert oder mit ggf. 1, 2, 3, 4 oder 5 Substituenten unabhängig voneinander ausgewählt aus der Gruppe bestehend aus F, Cl, Br, I, $-NO_2$,-CN, -OH, -SH und $-NH_2$ substituiert ist;
oder für einen Phenyl-Rest stehen, der jeweils über eine $C_{1-3}$-Alkylen- $C_{2-3}$-Alkenylen- oder $C_{2-3}$-Alkinylen-Gruppe gebunden sein kann und/oder unsubstituiert oder mit 1; 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist;

$R^4$, $R^5$, $R^6$, $R^7$, $R^{10}$,$R^{11}$ $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{32}$, $R^{33}$, $R^{34}$, $R^{35}$, $R^{36}$, $R^{37}$, $R^{38}$, $R^{39}$, $R^{40}$, $R^{41}$ $R^{42}$, $R^{43}$, $R^{44}$ und $R^{45}$, unabhängig voneinander, jeweils für H; F; Cl; Br; I; $-NO_2$; -CN; $-NH_2$; -OH; -SH; -C(=O)-OH; $-NH-R^{55}$; $-NR^{56}R^{57}$; $-C(=O)-R^{58}$; $-C(=O)-O-R^{59}$; $-O-R^{67}$; $-S-R^{68}$;
unsubstituiertes $C_{1-6}$-Alkyl; oder für einen Rest ausgewählt aus der Gruppe bestehend aus Phenyl, Benzyl und Phenethyl stehen, der unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, - OH, $-O-CH_3$, $-O-C_2H_5$ und $-O-C_3H_7$ substituiert ist;

oder $R^4$ und $R^5$ oder $R^6$ und $R^7$ oder $R^{10}$ und $R^{11}$ oder $R^{12}$ und $R^{13}$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$ oder $R^{28}$ und $R^{29}$ oder $R^{32}$ und $R^{33}$ oder $R^{34}$ und $R^{35}$ oder $R^{36}$ und $R^{37}$ oder $R^{38}$ und $R^{39}$ oder $R^{40}$ und $R^{41}$ oder $R^{42}$ und $R^{43}$ oder $R^{44}$ und $R^{45}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^3$ und $R^4$ zusammen mit der sie verbindenden -N-$CR^5$-Gruppe einen Rest ausgewählt aus der Gruppe bestehend aus

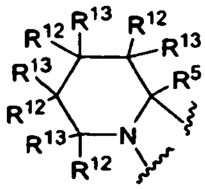 , 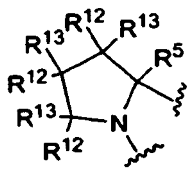 , 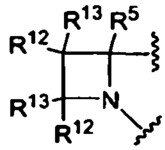 , 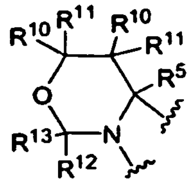 ,

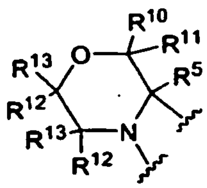 , 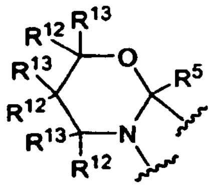 , 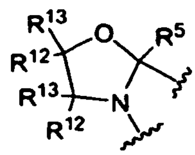 und 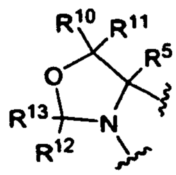

bilden;

oder $R^6$ und $R^8$ zusammen mit der sie verbindenden -N-$CR^7$-Gruppe einen Rest ausgewählt aus der Gruppe bestehend aus

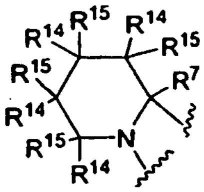 , 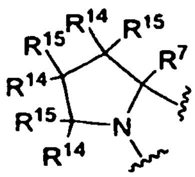 , 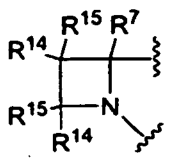 , 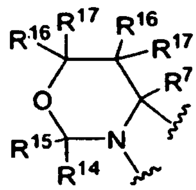 ,

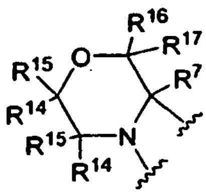 , 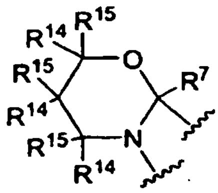 , 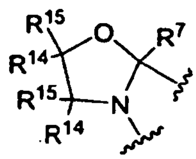 und 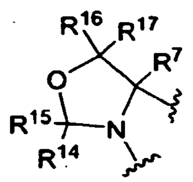 ,

bilden;

oder $R^3$ und $R^6$ zusammen mit der sie verbindenden -N-$CR^4R^5$-$CR^7$-Gruppe einen Rest ausgewählt aus der Gruppe bestehend aus

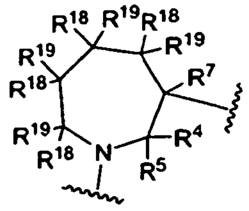 , 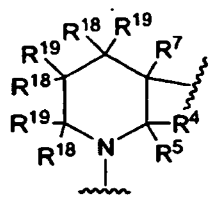 , 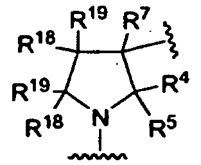 ,

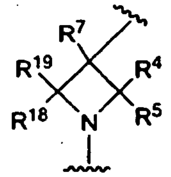 , 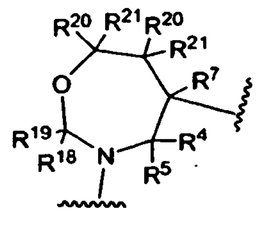 , 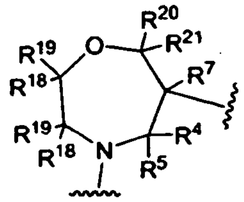 , 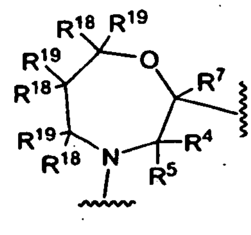 ,

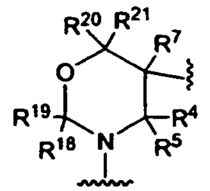 , 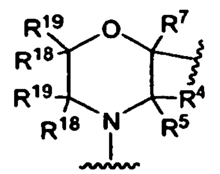 und 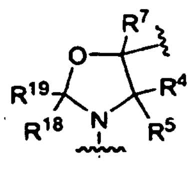

bilden;

oder $R^4$ und $R^8$ zusammen mit der sie verbindenden $-CR^5-CR^6CR^7-N-$Gruppe einen Rest ausgewählt aus der Gruppe bestehend aus

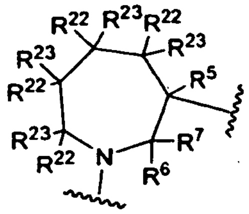 , 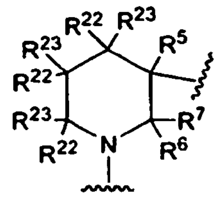 , 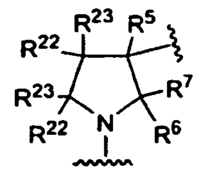 ,

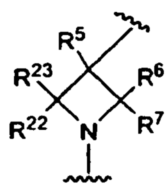 , 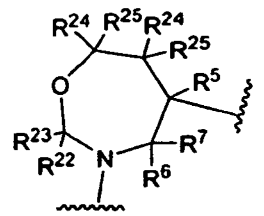 , 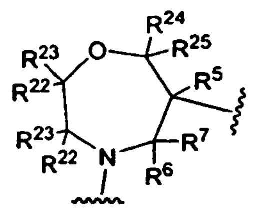 , 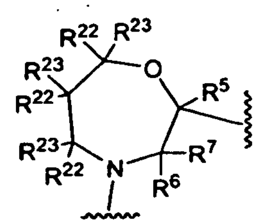 .

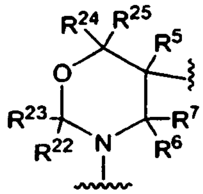, 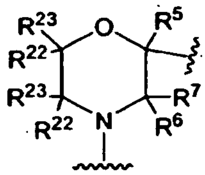 und 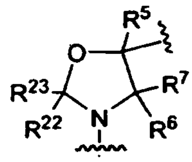

bilden;

oder $R^3$ und $R^8$ zusammen mit der sie verbindenden -N-$CR^4R^5$-$CR^6CR^7$-N-Gruppe einen Rest ausgewählt aus der Gruppe bestehend aus

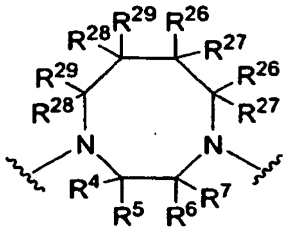 und 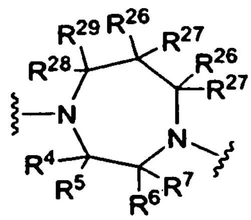

bilden;

oder $R^4$ und $R^6$ zusammen mit der sie verbindenden -$CR^5$-$CR^7$-Gruppe einen Rest ausgewählt aus der Gruppe bestehend aus

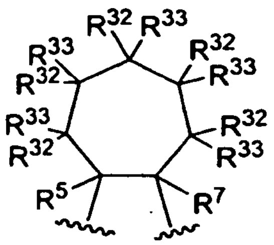, 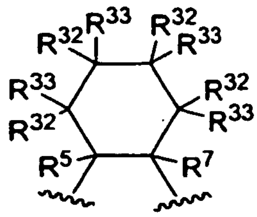 und 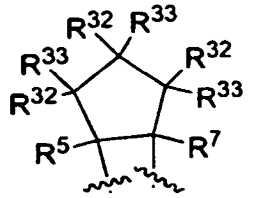,

bilden;

oder $R^3$ und $R^4$ zusammen mit der sie verbindenden -N-$CR^5$-Gruppe und $R^6$ und $R^8$ zusammen mit der sie verbindenden -N-$CR^7$-Gruppe einen Rest ausgewählt aus der Gruppe bestehend aus

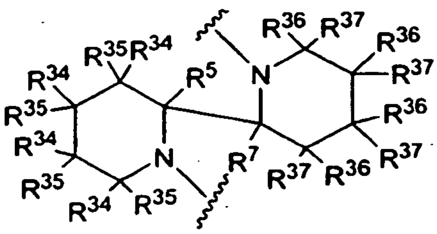, 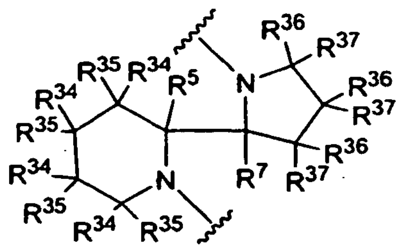,

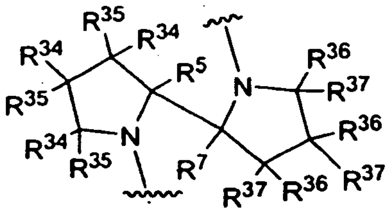 und 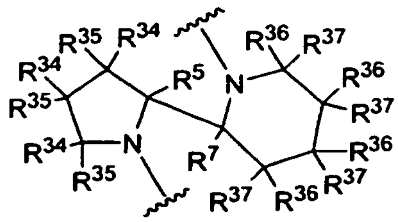

bilden;

oder $R^3$ und $R^6$ zusammen mit der sie verbindenden -N-$CR^4CR^5$-$CR^7$-Gruppe und $R^4$ und $R^8$ zusammen mit der sie verbindenden -$CR^5$-$CR^6R^7$-N-Gruppe einen Rest ausgewählt aus der Gruppe bestehend aus

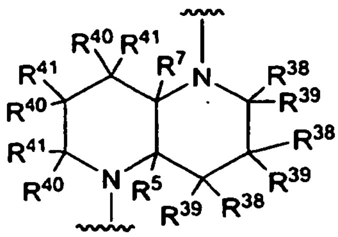, 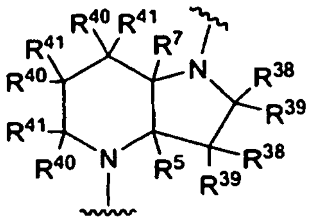,

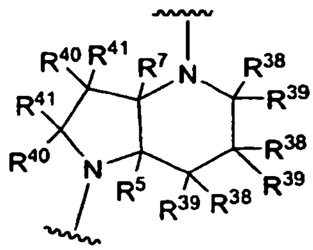 und 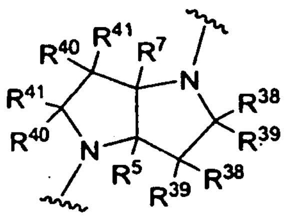

bilden;

oder $R^3$ und $R^8$ zusammen mit der sie verbindenden -N-$CR^4R^5$-$CR^6R^7$-N-Gruppe und $R^4$ and $R^6$ zusammen mit der sie verbindenden -$CR^5$-$CR^7$-Gruppe einen Rest ausgewählt aus der Gruppe bestehend aus

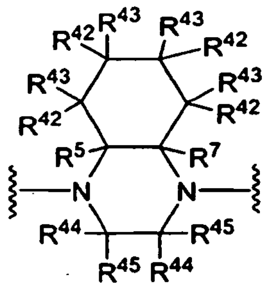, 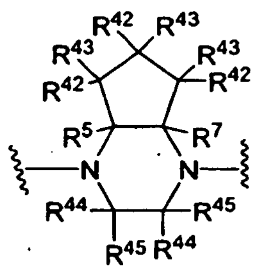,

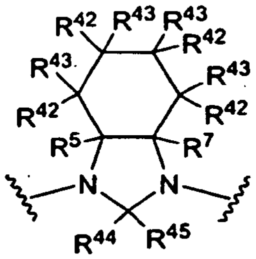

und

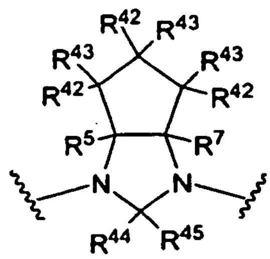

bilden;

$R^9$ für H; -C(=O)-$NH_2$; -C(=O)-NH-$R^{64}$; -C(=O)-$NR^{65}R^{66}$; unsubstituiertes $C_{1-6}$-Alkyl; unsubstituiertes $C_{3-7}$-Cycloalkyl; unsubstituiertes $C_{5-6}$-Cycloalkenyl; unsubstituiertes 5- bis 7-gliedriges Heterocycloalkyl und unsubstituiertes 5- bis 7-gliedriges Heterocycloalkenyl;
oder für einen Rest ausgewählt aus der Gruppe bestehend aus Phenyl, Naphthyl, Anthracenyl, Furyl, Thienyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl, Imidazolyl, Indolyl, Benz[b]thiophenyl, Benzo[d]thiazolyl, Benzo[b]furanyl, Chinolinyl, Isochinolinyl und Chinazolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, -C≡C-Si$(CH_3)_3$, -C≡C-Si$(C_2H_5)_3$, -$CH_2$-O-$CH_3$, -$CH_2$-O-$C_2H_5$, -OH, -O-$CH_3$, -O-$C_2H_5$, -O-$C_3H_7$, -S-$CH_3$, -S-$C_2H_5$, -S(=O)-$CH_3$, -S$(=O)_2$-$CH_3$, - S(=O)-$C_2H_5$, -S(=O)$C_2H_5$, -$NH_2$, -N$(CH_3)_2$, -N$(C_2H_5)_2$, -NH-$CH_3$, -NH-$C_2H_5$, -$NO_2$, -$CF_3$, -$CH_2F$, -$CHF_2$, -O-$CF_3$, -S-$CF_3$, -SH, -NH-S$(=O)_2$-$CH_3$, -C (=O)-OH, -C(=O)-H; -C(=O)-$CH_3$, -C(=O)-$C_2H_5$, -C(=O)-$NH_2$, -C(=O)-N$(CH_3)_2$, -C(=O)NH-$CH_3$, -NH-C(=O)-$CH_3$, -NH-C(=O)-$C_2H_5$, -C(=O)-O-$CH_3$ , -C(=O)-O-C2H5, -C(=O)-O-C$(CH_3)_3$ und Phenyl substituiert ist;

und $R^{55}$, $R^{56}$, $R^{57}$, $R^{58}$, $R^{59}$, $R^{64}$, $R^{65}$, $R^{66}$, $R^{67}$, $R^{68}$, $R^{69}$ und $R^{70}$, unabhängig voneinander, jeweils für unsubstituiertes $C_{1-6}$-Alkyl; unsubstituiertes $C_{3-7}$-Cycloalkyl; unsubstituiertes $C_{5-6}$-Cycloalkenyl; unsubstituiertes 5- bis 7-gliedriges Heterocycloalkyl und unsubstituiertes 5- bis 7-gliedriges Heterocycloalkenyl; oder für einen Rest ausgewählt aus der Gruppe bestehend aus Phenyl, Benzyl, Naphthyl, Anthracenyl, Pyrrolyl, Indolyl, Furanyl, Benzo[b] furanyl, Thiophenyl, Benz[b]thiophenyl, Benzo[d]thiazolyl, Pyrazolyl, Imidazolyl. Thiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Chinolinyl, Isochinolinyl und Chinazolinyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, - OH, -O-$CH_3$, -O-$C_2H_5$, -O-$C_3H_7$, -$NH_2$, -N$(CH_3)_2$, -N$(C_2H_5)_2$, -NH-$CH_3$, -NH-$C_2H_5$, -$NO_2$, - $CF_3$, -O-$CF_3$, -S-$CF_3$, -SH, -NH-S$(=O)_2$-$CH_3$, -C(=O)-OH, -C(=O)-$CH_3$, -C(=OyC$_2H_5$, - C(=O)-N$(CH_3)_2$, -C (=O)-NH-$CH_3$, -NH-C(=O)-$CH_3$, -NH-C(=O)-$C_2H_5$, -C(=O)-O-$CH_3$ und - C(=O)-O-$C_2H_5$ substituiert ist;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Besonders bevorzugt sind substituierte Thiazole der vorstehend angegebenen allgemeinen Formel I, worin

$R^1$ für H; F; Cl; Br; I; -$CF_3$; -$NO_2$; -CN; -C(=O)-OH: -C(=O)-O-$R^{59}$; -C(=O)-$NH_2$; -C(=O)-NH-$R^{64}$; -C(=O)-$NR^{65}R^{66}$; -O-$R^{67}$; -S-$R^{68}$; -S(=O)-$R^{69}$; -S$(=O)_2$-$R^{70}$;
einen Alkyl-Rest ausgewählt aus der Gruppe bestehend aus Methyl, Ethyl, n-Propyl, Isopropyl, n-Butyl, 2-Butyl. Isobutyl und tert-Butyl; oder für einen Rest ausgewählt aus der Gruppe bestehend aus (1,3)-Dioxolan-2-yl, Phenyl, Benzyl, Phenethyl, Oxadiazolyl, 2-Pyridyl, 3-pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F. Cl, Br, Methyl, Ethyl, n-Propyl, Isopropyl, n-Butyl, 2-Butyl, Isobutyl, tert-Butyl, -OH, -O-$CH_3$, -O-$C_2N_5$ und -O-$C_3H_7$ substituiert ist, steht;

$R^2$ für H; F; Cl, Br; I; -$CF_3$; -$NO_2$; -CN; -C(=O)-OH; -C(=O)-O-$R^{59}$; -C(=O)-$NH_2$; -C(=O)-NH-$R^{64}$; -C(=O)-$NR^{65}R^{66}$; -O-$R^{67}$; -S-$R^{68}$; -S(=O)-$R^{69}$; -S$(=O)_2$-$R^{70}$;
einen Alkyl-Rest ausgewählt aus der Gruppe bestehend aus Methyl, Ethyl, n-Propyl, Isopropyl, n-Butyl, 2-Butyl, Isobutyl und tert-Butyl; oder für einen Rest ausgewählt aus der Gruppe bestehend aus (1,3)-Dioxolan-2-yl, Phenyl,

Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist, steht;

oder $R^1$ und $R^2$ zusammen mit den sie verbindenden Kohlenstoffatomen einen Phenylen-Rest bilden, der unsubstituiert oder mit 1, 2, 3 oder 4 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, $-O-CH_3$, $-O-C_2H_5$, $-O-C_3H_7$, $-CF_3$, $-CHF_2$, $-CH_2F$ und $-O-CF_3$ substituiert ist;

$R^3$ und $R^8$, unabhängig voneinander, jeweils für H; $-C(=O)-R^{58}$; $-C(=O)-O-R^{58}$; $-S(=O)-R^{69}$; $-S(=O)_2-R^{70}$; einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; für einen Cycloalkylrest ausgewählt aus der Gruppe bestehend aus Cyclopropyl, Cyclobutyl, Cyclopentyl und Cyclohexyl; oder für einen Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-0-C_3H_7$ substituiert ist;

$R^4$, $R^5$, $R^6$, $R^7$, $R^{12}$, $R^{13}$, $R^{14}$, $R^{15}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{32}$, $R^{33}$, $R^{38}$, $R^{39}$, $R^{40}$, $R^{41}$, $R^{42}$, $R^{43}$, $R^{44}$ und $R^{44}$, unabhängig voneinander, jeweils für H; F; Cl; Br; I; $-NO_2$; -CN; $-NH_2$; -OH; -SH; $-NH-R^{55}$; $-NR^{56}R^{57}$; $-O-R^{67}$; $-S-R^{68}$; oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl stehen;

oder $R^4$ und $R^5$ oder $R^6$ und $R^7$ oder $R^{12}$ und $R^{13}$ oder $R^{14}$ und $R^{15}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{21}$ oder $R^{28}$ und $R^{29}$ oder $R^{32}$ und $R^{33}$ oder oder $R^{38}$ und $R^{39}$ oder $R^{40}$ und $R^{41}$ oder $R^{42}$ und $R^{43}$ oder $R^{44}$ und $R^{453}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^3$ und $R^4$ zusammen mit der sie verbindenden $-N-CR^5$-Gruppe einen Rest ausgewählt aus der Gruppe bestehend aus

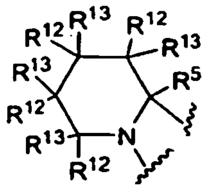

und

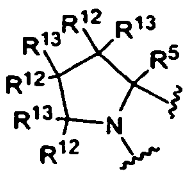

bilden;

oder $R^6$ und $R^8$ zusammen mit der sie verbindenden $-N-CR^7$-Gruppe einen Rest ausgewählt aus der Gruppe bestehend aus

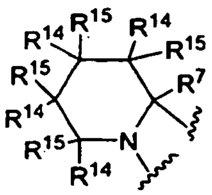

und

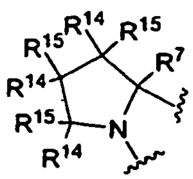

bilden;

oder $R^3$ und $R^6$ zusammen mit der sie verbindenden $-N-CR^4R^5-CR^7$-Gruppe einen Rest ausgewählt aus der Gruppe bestehend aus

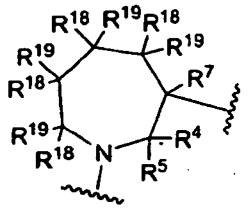, 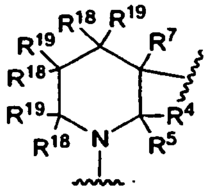, 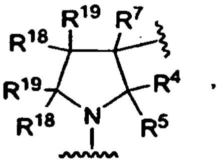,

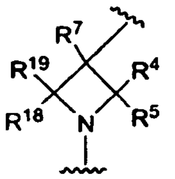, 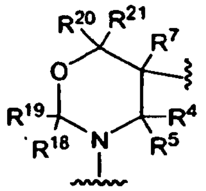, 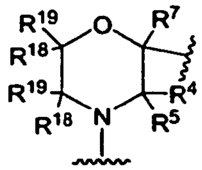 und 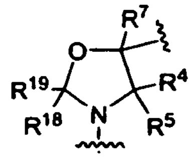

bilden;

oder $R^4$ und $R^8$ zusammen mit der sie verbindenden $-CR^5-CR^6CR^7-N-$Gruppe einen Rest ausgewählt aus der Gruppe bestehend aus

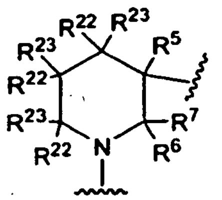, 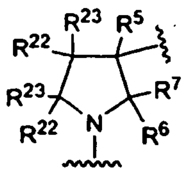, 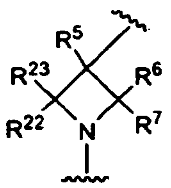,

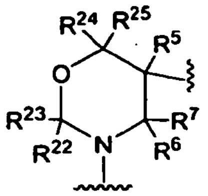, 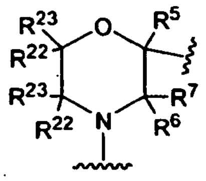 und 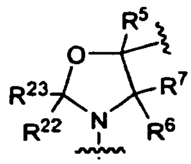

bilden;

oder $R^3$ und $R^8$ zusammen mit der sie verbindenden $-N-CR^4R^5-CR^6CR^7-N-$Gruppe den folgenden Rest

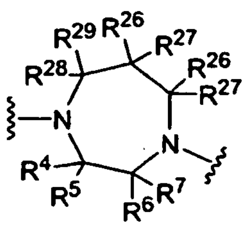

bilden;

oder $R^4$ und $R^6$ zusammen mit der sie verbindenden $-CR^s-CR^7-$Gruppe den folgenden Rest

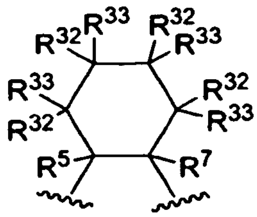

bilden;

oder $R^3$ und $R^6$ zusammen mit der sie verbindenden $-N-CR^4CR^5-CR^7$-Gruppe und $R^4$ und $R^8$ zusammen mit der sie verbindenden $-CR^5-CR^6R^7-N$-Gruppe den folgenden Rest

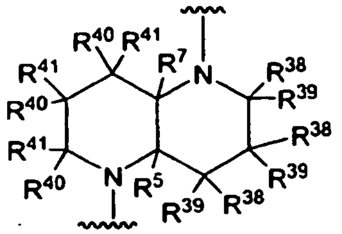

bilden;

oder $R^3$ und $R^8$ zusammen mit der sie verbindenden $-N-CR^4R^5-CR^6R^7-N$-Gruppe und $R^4$ und $R^6$ zusammen mit der sie verbindenden $-CR^5-CR^7$-Gruppe den folgenden Rest

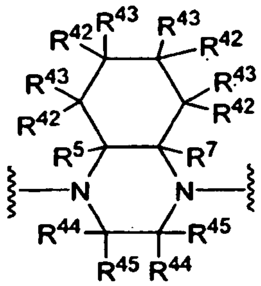

bilden;

$R^9$ für $-C(=O)-NH-R^{64}$; $-C(=O)-NR^{65}R^{66}$;
oder für einen Rest ausgewählt aus der Gruppe bestehend aus Phenyl, Naphthyl, Anthracenyl, Furyl, Thienyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl, Imidazolyl, Indolyl, Benz[b]thiophenyl, Benzo[d]thiazolyl, Benzo[b]furanyl, Chinolinyl, Isochinolinyl und Chinazolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, $-C{\equiv}C-Si(CH_3)_3$, $-C{\equiv}C-Si(C_2H_5)_3$, $-CH_2-O-CH_3$, $-CH_2-O-C_2H_5$, -OH, $-O-CH_3$, $-O-C_2H_5$, $-O-C_3H_7$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-NH_2$, $-N(CH_3)_2$, $-N(C_2H_5)_2$, $-NH-CH_3$, $-NH-C_2H_5$, $-NO_2$, $-CF_3$, $-CH_2F$, $-CHF_2$, $-O-CF_3$, $-S-CF_3$, -SH, $-NH-S(=O)_2-CH_3$, -C(=O)-OH, -C(=O)-H; $-C(=O)-CH_3$, $-C(=O)-C_2H_5$, $-C(=O)-NH_2$, $-C(=O)-N(CH_3)_2$, $-C(=O)-NH-CH_3$, $-NH-C(=O)-CH_3$, $-NH-C(=O)-C_2H_5$, $-C(=O)-O-CH_3$ , $-C(=O)-O-C_2H_5$, $-C(=O)-O-C(CH_3)_3$ und Phenyl substituiert ist;

und $R^{55}$, $R^{56}$, $R^{57}$, $R^{58}$, $R^{59}$, $R^{64}$, $R^{65}$, $R^{66}$, $R^{67}$, $R^{68}$, $R^{69}$ und $R^{70}$, unabhängig voneinander, jeweils für einen Alkyl-Rest ausgewählt aus der Gruppe bestehend aus Methyl, Ethyl, n-Propyl, Isopropyl, n-Butyl. 2-Butyl. Isobutyl, tert-Butyl, n-Pentyl und n-Hexyt; oder für einen Phenyl-, Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl,-OH, $-O-CH_3$, $-O-C_2H_5$ und $-O-C_3H_7$ substituiert ist;

jeweils ggf. in Form eines ihrer reinen Stereoisomeren, insbesondere Enantiomeren oder Diastereomeren, ihrer Racemate oder in Form einer Mischung von Stereoisomeren, Insbesondere der Enantiomeren und/oder Diastereomeren, in einem beliebigen Mischungsverhältnis, oder jeweils in Form entsprechender Salze.

Ganz besonders bevorzugt sind substituierte Thiazole der vorstehend angegebenen allgemeinen Formel , worin

$R^1$ für H; F; Cl; Br, I; $-CF_3$; $-NO_2$; -CN; -C(=O)-OH; $-C(=O)-O-R^{59}$; $-C(=O)-NH_2$; $-C(=O)-NH-R^{64}$; $-C(=O)-NR^{65}R^{66}$ $-O-R^{67}$; $-S-R^{68}$; $-S(=O)-R^{69}$; $-S(=O)_2-R^{70}$;
einen Alkyl-Rest ausgewählt aus der Gruppe bestehend aus Methyl, Ethyl, n-Propyl, Isopropyl, n-Butyl, 2-Butyl, Isobutyl und tert-Butyl; oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, Methyl. Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist, steht;

$R^2$ für H; F; Cl; Br,1; $-CF_3$; $-NO_2$; -CN; -C(=O)-OH; $-C(=O)-O-R^{59}$; $-C(=O)-NH_2$; $-C(=O)-NH-R^{84}$; $-C(=O)-NR^{65}R^{66}$; $-O-R^{67}$; $-S-R^{68}$; $-S(=O)-R^{69}$; $-S(=O)_2-R^{70}$:
einen Alkyl-Rest ausgewählt aus der Gruppe bestehend aus Methyl, Ethyl, n-Propyl, Isopropyl, n-Butyl, 2-Butyl, Isobutyl und tert-Butyl: oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist, steht;

oder $R^1$ und $R^2$ zusammen mit den sie verbindenden Kohlenstoffatomen einen Phenylen-Rest bilden, der unsubstituiert oder mit 1, 2, 3 oder 4
Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, $-O-CH_3$, $-O-C_2H_5$, $-O-C_3H_7$, $-CF_3$, $-CHF_2$, $-CH_2F$ und $-O-CF_3$ substituiert ist;

$R^3$ und $R^8$, unabhängig voneinander, jeweils für H; $-C(=O)-R^{58}$; $-C(=O)-O-R^{59}$; $-S(=O)-R^{69}$;$-S(=O)_2-R^{70}$; einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; für einen Cycloalkylrest ausgewählt aus der Gruppe bestehend aus Cyclopropyl, Cyclobutyl, Cyclopentyl und Cyclohexyl; oder für einen Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-0-C_3H_7$ substituiert ist;

$R^4$, $R^5$, $R^6$ und $R^7$, unabhängig voneinander, jeweils für H; F; Cl; Br; I; $-NO_2$; -CN; $-NH_2$; - OH; -SH; $-NH-R^{55}$; $-NR^{56}R^{s7}$; $-O-R^{67}$; $-S-R^{68}$; oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl stehen;

oder $R^4$ und $R^5$ oder $R^6$ und $R^7$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^3$ und $A^4$ zusammen mit der sie verbindenden $-N-CR^5$-Gruppe einen Rest ausgewählt aus der Gruppe bestehend aus

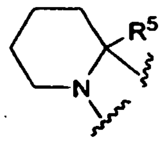 und 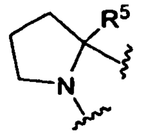

bilden;

oder $R^6$ und $R^8$ zusammen mit der sie verbindenden $-N-CR^7$-Gruppe einen Rest ausgewählt aus der Gruppe bestehend aus

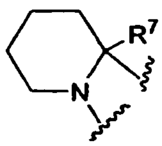 und 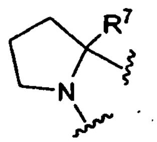.

bilden;

der $R^3$ und $R^6$ zusammen mit der sie verbindenden $-N-CR^4R^5-CR^7$-Gruppe einen Rest ausgewählt aus der Gruppe bestehend aus

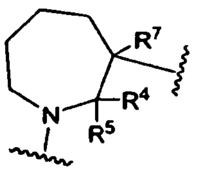, 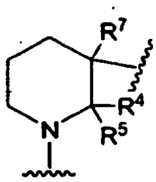, 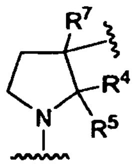,

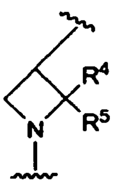, 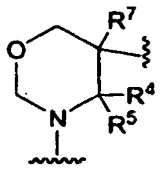, 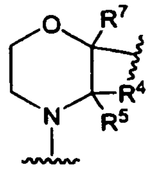 und 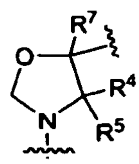

bilden;

oder $R^4$ und $R^8$ zusammen mit der sie verbindenden $-CR^5-CR^6CR^7-N$-Gruppe einen Rest ausgewählt aus der Gruppe bestehend aus

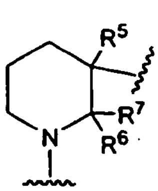, 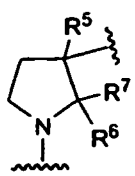, 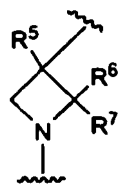.

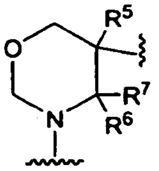 , 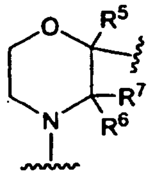 und 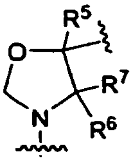

bilden;

oder $R^3$ und $R^8$ zusammen mit der sie verbindenden -N-$CR^4$Rs-$CR^6CR^7$-N-Gruppe den folgenden Rest

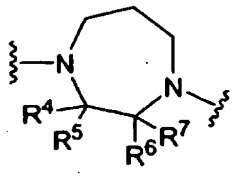

bilden;

oder $R^4$ und $R^6$ zusammen mit der sie verbindenden -$CR^5$-$CR^7$-Gruppe den folgenden Rest

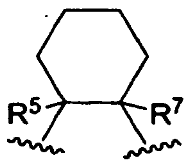

bilden;

oder $R^3$ und $R^6$ zusammen mit der sie verbindenden -N-$CR^4CR^5$-$CR^7$-Gruppe und $R^4$ und $R^8$ zusammen mit der sie verbindenden -$CR^5$-$CR^6R^7$-N-Gruppe den folgenden Rest

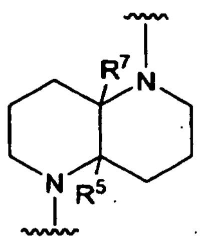

bilden;

oder $R^3$ und $R^8$ zusammen mit der sie verbindenden -N-$CR^4R^5$-$CR^6R^7$-N-Gruppe und $R^4$ und $R^6$ zusammen mit der sie verbindenden -$CR^5$-$CR^7$-Gruppe den folgenden Rest

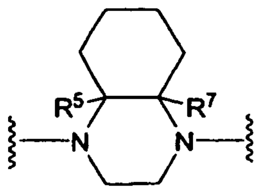

bilden;

$R^9$ für -C(=O)-NH-$R^{64}$; -C(=O)-$NR^{65}R^{66}$; oder für einen Rest ausgewählt aus der Gruppe bestehend aus Phenyl, Naphthyl, Furyl, Thienyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl, Imidazolyl, Indolyl, Benz[b]thiophenyl, Benzo[d]thiazolyl, Benzo[b]furanyl, Chinolinyl, Isochinolinyl und Chinazolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, -O-$CH_3$, -O-$C_2H_5$, -$NO_2$, -$CF_3$, -$CH_2F$, -$CHF_2$, -O-$CF_3$ und -S-$CF_3$ substituiert ist;

und $R^{55}$, $R^{56}$, $R^{57}$, $R^{58}$, $R^{59}$ , $R^{64}$, $R^{65}$, $R^{66}$, $R^{67}$ , $R^{68}$, $R^{69}$ und $R^{70}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; oder für einen Phenyl-, Benzyl- oder Phenethyl-Rest stehen, der unsubstituiert oder mit 1, 2, 3, 4 oder 5 Substituenten unabhängig voneinander ausgewählt aus der Gruppe bestehend aus F. Cl. Br, Methyl, Ethyl, n-Propyl, Isopropyl, n-Butyl, 2-Butyl, Isobutyl, tert-Butyl, - OH, -O-$CH_3$, -O-$C_2H$ 5und -O-$C_3H_7$ substituiert ist;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Ganz besonders bevorzugt sind substituierte Thiazole der vorstehend angegebenen allgemeinen Formel 1, worin

$R^1$ für H; F; Cl; Br, I; -CN; -$CF_3$; -$NO_2$; -C(=O)-O-$R^{59}$; einen Alkyl-Rest ausgewählt aus der Gruppe bestehend aus Methyl, Ethyl, n-Propyl, Isopropyl, n-Butyl, 2-Butyl, Isobutyl und tert-Butyl; oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl steht;

$R^2$ für H; F; Cl; Br, I; -CN; -$CF_3$; -$NO_2$; -C(=O)-O-$R^{59}$; einen Alkyl-Rest ausgewählt aus der Gruppe bestehend aus Methyl, Ethyl, n-Propyl, Isopropyl, n-Butyl, 2-Butyl, Isobutyl und tert-Butyl; oder für einen Rest ausgewählt aus der Gruppe bestehend aus (1,3)-Dioxolan-2-yl, Phenyl, Benzyl, Phenethyl, Oxadiazolyl, 2-Pyridyl. 3-Pyridyl, 4-Pyridyl, 2-Thienyl, 3-Thienyl, 2-Furyl und 3-Furyl steht;

oder $R^1$ und $R^2$ zusammen mit den sie verbindenden Kohlenstoffatomen einen Phenylen-Rest bilden;

$R^3$ und $R^8$, unabhängig voneinander, jeweils für H; -C(=O}-$R^{58}$; -C(=O)-D-$R^{59}$; -einen Alkyl-Rest ausgewählt aus der Gruppe bestehend aus Methyl, Ethyl, n-Propyl, Isopropyl. n-Butyl, 2-Butyl, Isobutyl, tert-Butyl, n-Pentyl und n-Hexyl; einen Cycloalkylrest ausgewählt aus der Gruppe bestehend aus Cyclopropyl, Cyclobutyl, Cyclopentyl und Cyclohexyl; oder für einen Benzyl- oder Phenethyl-Rest stehen, der unsubstituiert oder mit 1,2,3,4 oder 5 Substituenten unabhängig voneinander ausgewählt aus der Gruppe bestehend aus F, Cl, Br, Methyl, Ethyl, n-Propyl, Isopropyl, n-Butyl, 2-.Butyl, Isobutyl, tert-Butyl, -OH, -O-$CH_3$, -O-$C_2H_5$ und -O-$C_3H_7$ substituiert ist;

$R^4$, $R^5$, $R^6$ und $R^7$, unabhängig voneinander, jeweils für H; F; Cl; Br; I; -$NO_2$; -CN; -$NH_2$;-OH; -SH; -NH-$R^{55}$; -$NR^{56}R^{57}$; -O-$R^{67}$; -S-$R^{68}$; oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl stehen;

oder $R^4$ und $R^5$ oder $R^6$ und $R^7$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^3$ und $R^6$ zusammen mit der sie verbindenden -N-$CR^4R^5$-$CR^7$-Gruppe einen Rest ausgewählt aus der Gruppe bestehend aus

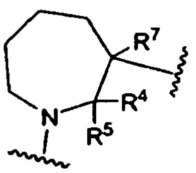 , 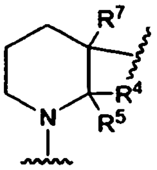 , 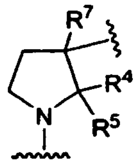 ,

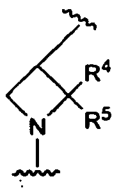 , 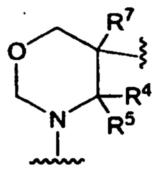 , 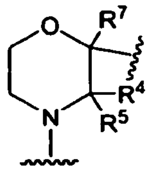 und 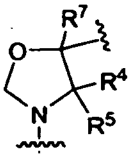

bilden;

oder $R^4$ und $R^8$ zusammen mit der sie verbindenden -$CR^5$-$CR^6CR^7$-N-Gruppe einen Rest ausgewählt aus der Gruppe bestehend aus

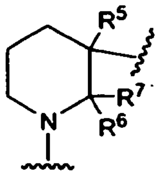 , 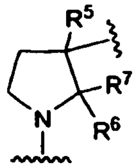 , 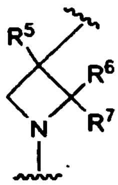 ,

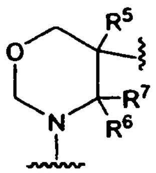 , 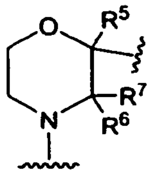 und 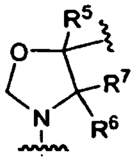

bilden;

oder $R^3$ und $R^8$ zusammen mit der sie verbindenden -N-$CR^4R^5$-$CR^6CR^7$-N-Gruppe den folgenden Rest

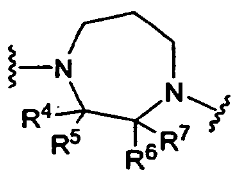

bilden;

oder $R^4$ und $R^6$ zusammen mit der sie verbindenden -$CR^5$-$CR^7$-Gruppe den folgenden Rest

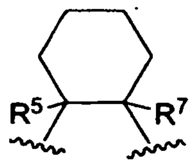

bilden;

$R^9$ für einen Rest ausgewählt aus der Gruppe bestehend aus Phenyl, Furyl, Thienyl, Pyrazolyl, Pyrazinyl, Pyridazinyl, Pyrimidinyl, Pyridinyl, Pyrrolyl, Oxazolyl, Isoxazolyl,-Thiazolyl, Thiadiazolyl, Oxadiazolyl, Triazolyl und Imidazolyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, $-O-CH_3$, - $O-C_2H_5$, $-NO_2$, $-CF_3$, $-CH_2F$, $-CHF_2$, $-O-CF_3$ und $-S-CF_3$ substituiert ist;

und $R^{55}$, $R^{56}$, $R^{57}$, $R^{58}$, $R^{59}$, $R^{67}$ und $R^{68}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl, tert-Butyt, n-Pentyl und n-Hexyl stehen;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Noch weiter bevorzugt sind substituierte Thiazole der vorstehend angegebenen allgemeinen Formel 1, worin

$R^1$ für H, F, Cl, Br, -CN, Methyl, Ethyl, n-Propyl, Isobutyl, tert-Butyl, n-Butyl. $-C(=O)-O-CH_3$, $-C(=O)-O-C_2H_5$ oder $-C(=O)-O-C(CH_3)_3$ steht;

$R^2$ für H, F, Cl, Br, -CN, Methyl, Ethyl, n-Propyl, Isobutyl, tert-Butyl, n-Butyl, $-C(=O)-O-CH_3$. $-C(=O)-O-C_2H_5$ oder $-C(=O)-O-C(CH_3)_3$ steht;

oder $R^1$ und $R^2$ zusammen mit den sie verbindenden Kohlenstoffatomen einen Phenylen-Rest bilden;

$R^3$ und $R^8$, unabhängig voneinander, jeweils für H; für einen Alkyl-Rest ausgewählt aus der Gruppe bestehend aus Methyl, Ethyl, n-Propyl, Isopropyl, n-Butyl, 2-Butyl, Isobutyl und tert-Butyl; oder für Cyclopropyl; stehen;

$R^4$. $R^5$ und $R^7$, jeweils für H stehen;

$R^6$ für H oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl steht;

oder $R^4$ und $R^5$ oder $R^6$ und $R^7$, unabhängig voneinander, zusammen jeweils für eine Oxo-Gruppe (=O) stehen;

oder $R^3$ und $R^6$ zusammen mit der sie verbindenden $-N-CH_2-CH-$Gruppe einen Rest ausgewählt aus der Gruppe bestehend aus

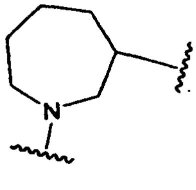 ,

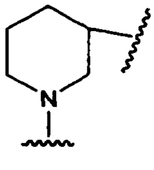 ,

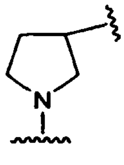 ,

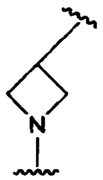 , 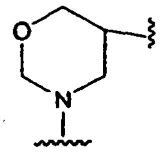 , 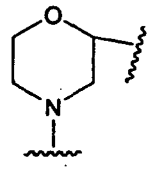 und 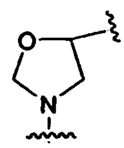

bilden,

oder $R^4$ und $R^8$ zusammen mit der sie verbindenden -CH-$CH_2$-N-Gruppe einen Rest ausgewählt aus der Gruppe bestehend aus

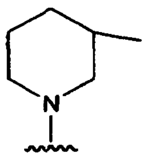 , 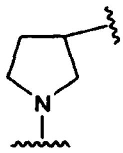 , 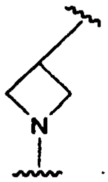 ,

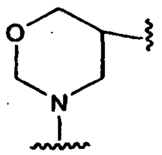 , 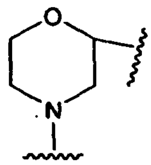 und 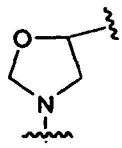

bilden;

oder $R^3$ und $R^8$ zusammen mit der sie verbindenden -N-$CH_2$-$CH_2$-N-Gruppe den folgenden Rest

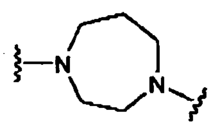

bilden;

oder $R^4$ und $R^6$ zusammen mit der sie verbindenden -CH-CH-Gruppe den folgenden Rest

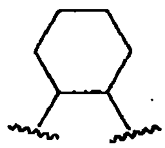

bilden;

und $R^9$ für einen Rest ausgewählt aus der Gruppe bestehend aus Pyrid-2-yl, Pyrid-3-yl, Pyrid-4-yl, Phenyl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Chlor-phenyl, 3-Chlor-phenyl, 3-Chlor-phenyl, 2-Trifluormethyl-phenyl, 3-Trifluormethyl-phenyl, 4-Trifluormethylphenyl, 2-Methoxy-phenyl, 3-Methoxy-phenyl, 4-Methoxy-phenyl, (2,4)-Difluor-phenyl, (2,4)-Dichlor-phenyl, (3,5)-Dichlor-phenyl, (3,5)-Ditluor-phenyl, 2-Thiophenyl, 2-Chfor-5-trifluormethyl-phenyl, 3-Fluor-4-methyl-phenyl, 2-Fluor-3-methyl-phenyl, 2-Difluormethyl-phenyl, 3-Difiuomletltyl-phenyl, 4-Difluormethyl-phenyl, 2-Fluormethyl-phenyl, 3-Fluormethyl-phenyl, 4-Fluormethyl-phenyl, 3-Nitro-phenyl, 3-Ethenyl-phenyl, 3-Ethinyl-phenyl, 3-Alfyl-phenyl, 3-Bromphenyl, 2-Trifluormethoxy-phenyl, 3-Trifuormethoxy-phenyl und 4-Trifluormethoxy-phenyl steht;

jeweils ggf. in Form eines ihrer reinen Stereoisomeren, insbesondere Enantiomeren oder Diastereomeren, ihrer Racemate oder in Form einer Mischung von Stereoisomeren, Insbesondere der Enantiomeren und/oder Diastereomeren, in einem beliebigen Mischungsvefiältnis, oder jeweils in Form entsprechender Salze.

Noch weiter bevorzugt sind substituierte Thiazole der allgemeinen Formel Ia,

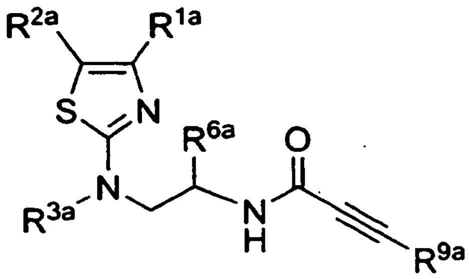

**Ia,**

worin

$R^{1a}$ für H, F, Cl, Br, -CN, Methyl, Ethyl, n-Propyl, Isobutyl, tert-Butyl, n-Butyl, $-C(=O)-O-CH_3$, $-C(=O)-O-C_2H_5$ oder $-C(=O)-O-C(CH_3)_3$ steht;

$R^{2a}$ für H, F, Cl, Br, -CN, Methyl, Ethyl, n-Propyl, Isobutyl, tert-Butyl, n-Butyl, $-C(=O)-O-CH_3$, $-C(=O)-O-C_2H_5$ oder $-C(=O)-O-C(CH_3)_3$ steht;

oder $R^{1a}$ und $R^{2a}$ zusammen mit den sie verbindenden Kohlenstoffatomen einen Phenylen-Rest bilden;

$R^{3a}$ für H; für einen Alkyl-Rest ausgewählt aus der Gruppe bestehend aus Methyl, Ethyl, n-Propyl, Isopropyl, n-Butyl, 2-Butyl, Isobutyl und tert-Butyl; oder für Cyclopropyl steht;

$R^{6a}$ für H oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl steht;

und $R^{9a}$ für einen Rest ausgewählt aus der Gruppe bestehend aus Pyrid-2-yl, Pyrid-3-yl, Pyrid-4-yl, Phenyl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Chlor-phenyl, 3-Chlor-phenyl, 3-Chlor-phenyl, 2-Trifluormethyl-phenyl, 3-Trifluormethyl-phenyl, 4-Trifluormethylphenyl, 2-Methoxy-phenyl, 3-Methoxy-phenyl, 4-Methoxy-phenyl, (2,4)-Difluor-phenyl, (2.4)-Dichlor-phenyl, (3,5)-Dichlor-phenyl, (3,5)-Difluor-phenyl, 2-Thiophenyl, 2-Chtor-5-trifluormethyl-phenyl, 3-Ftuor-4-methyl-phenyl, 2-Fluor-3-methyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 3-Nitro-phenyl, 3-Ethenyl-phenyl, 3-Ethinyl-phenyl, 3-Allyl-phenyl, 3-Bromphenyl, 2-Trifluormethoxylphenyl, 3-Trifuormethoxy-phenyl und 4-Trifluormethoxy-phenyl steht;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Ebenfalls besonders bevorzugt sind Thiazole der vorstehend angegebenen allgemeinen Formel I, die nach 60

Minuten Inkubation in 450 μg Protein aus Schweinehimhomogenat bei einer Temperatur zwischen 20 °C und 25°C in einer Konzentration kleiner 2000 nM, bevorzugt kleiner 1000 nM, besonders bevorzugt kleiner 700 nM, ganz besonders bevorzugt kleiner 100 nM, noch weiter bevorzugt keiner 30 nM, eine 50-prozentige Verdrängung von [$^3$H]-2-Methyl-6-(3-methoxyphenyl)-ethinylpyridin bewirken, dass In einer Konzentration von 5 nM vorliegt.

Die Bestimmung der Verdrängung von [$^3$H]-2-Methyl-6-(3-methoxyphenyl)-ethinylpyridin erfolgt dabei wie im Abschnitt Pharmakologische Methoden, 1. Methode zur Bestimmung der Hermmung der [$^3$H]-MPEP-Bindung im mGluR5-Rezeptor-Bindungsassay beschrieben.

Noch weiter bevorzugt sind substituierte Thiazole der vorstehend angegebenen allgemeinen Formel I ausgewählt aus der Gruppe bestehend aus

[1] 3-Phenyl-N-(1-(thiazol-2-yl)pyrrolidin-3-yl)propiolamid,
[2] N-Methyl-3-phenyl-N-(1-(thiazol-2-yl)pyrrolidin-3-yl)propiolamid Hydrochlorid,
[3] 1-Thiazol-2-yl-4g-(3-phenyl-propiolyl)-1,4-diazepan,
[4] N-Methyl-N-(2-(methyl(thiazol-2-yl)amino)ethyl)-3-phenylpropiolamid,
[5] N-(2-((Thiazol-2-yl)amino)ethyl)-3-phenylpropiolamid,
[6] 3-Phenyl-N-(2-(thiazol-2-ylamino)cyclohexyl)propiolamid,
[7] N-Methyl-N-(2-(methyl(thiazol-2-yl)amino)ethyl)-3-(3-methyl-phenyl)-propiolamid,
[8] 3-Phenyl-N-(1-(thiazol-2-yl)azetidin-3-yl)propiolamid,
[9] N-(2-(Methyl(thiazol-2-yl)amino)ethyl)-3-phenylpropiolamid,
[10] N-Methyl-N-(2-(thiazol-2-yl)amino)ethyl-3-phenylpropiotamid,
[11] 3-(Thiazol-2-yl-amino)-1-(3-phenyl-propiolyl)pyrrolidin,
[12] N-Methyl-N-(2-(methyl(thiazol-2-yl)amino)cyclohexyl)-3-phenylpropiolamid,
[13] N-Methyl-N-(2-(methyl(thiazol-2-yl)amino)ethyl)-3-(3-cyano-phenyl)-propiolamid,
[14] N-Methyl-N-(2-(methyl(thiazol-2-yl)amino)-2-oxoethyl)-3-phenylpropiolamid,
[15] 3-Phenyl-N-(1-(thiazol-2-yl)piperidin-3-yl)propiolamid,
[16] N-Methyl-N-(1 -(thiazol-2-yl)piperidin-3-yl)-3-phenyl-propiolamid,
[17] N-Methyl-N-(2-(methyl(thiazol-2-yl)amino)ethyl)-3-(pyrid-2-yl)-propiolamid,
[18] N-Methyl-N-(2-(methyl(thiazol-2-yl)amino)ethyl)-3-(3-chlor-phenyl)-propiolamid,
[19] N-(2-(Methyl(thiazol-2-yl)amino)ethyl)-3-(3-chlor-phenyl)-propiolamid,
[20] N-Methyl-N-(1-(thiazol-2-yl)azetidin-3-yl)-3-phenyl-propiolamid,
[21] N-(2-(Methyl(thiazol-2-yl)amino)ethyl)-3-(tol-3-yl)-propiolamid,
[22] 3-Methyl(thiazol-2-yl)amino)-1-(3-phenyl-propiolyl)pyrrolidin,
[23] N-(2-(Methyl(thiazol-2-yl)-amino)ethyl)-3-(3-chlor-phenyl)-propiolamid Hydrochlorid,
[24] N-(2-(Benzo[d]thiazol-2-y1(methyl)amino)ethyl)-3-(3-chlor-phenyl)propiolamid,
[25] N-(2-Methyl(5-ethoxycarbonyl-thiazol-2-yl)amino)ethylr3-(3-chlor-phenyl) propiolamid,
[26] N-(2-Methyl(4-ethoxycarbonyl-thiazol-2-yl)amino)ethyl)-3-(3-chlor-phenyl)-propiolamid,
[27] 3-(Thiazol-2-yl-amino)-1-(3-phenyl-propiolyl)piperidin,
[28] 3-(Methyl-(thiazol-2-yl)-amino)-1-(3-phenyl-propiolyl)piperidin
[29] N-(2-(Methyl-(4-methyl-thiazol-2-yl)amino)ethyl)-3-(3-chlor-phenyl)-propiolamid
[30] 3-(3-Chlorphenyl)-N-(2-(methyl(5-methylthiazol-2-yl)amino)ethyl)propiolamid
[31] N-(2-((5-Bromthiazol-2-yl)(methyl)amino)ethyl)-3-(3-chlorphenyl)propiolamid,
[32] 3-(3-Chlorphenyl)-N-(1-(thiazol-2-yl)piperidin-3-yl)propiolamid,
[33] N-(2-((4-Bromthiazol-2-yl)(methyl)amino)ethyl)-3-(3-chlorphenyl)propiolamid,
[34] Methyl2-((2-(3-(3-chlorphenyl)propiolamido)ethyl)(methyl)amino)thiazol-5-carboxylat,
[35] 1-(3-(3-Chlorphenyl)propiolyl)-3-(methyl(thiazol-2-yl)amino)azetidin,
[36] 3-(3-Chlorphenyl)-N-methyl-N-(1-(thiazol-2-yl)piperidin-3-yl)propiolamid,
[37] 3-(3-Chlorphenyl)-N-(2-((4-chlorthiazol-2-yl)(methyl)amino)ethyl)propiolamid,
[38] 3-(3-Chlorphenyl)-N-(2-((5-chlorthiazol-2-yl)(methyl)amino)ethyl)propiolamid,
[39] 3-(3-Chlorphenyl)-N-(thiazol-2-yl)azetidin-3-yl)propiolamid,
[40] 3-(3-Chlorphenyl)-N-methyl-N-(1-(thiazol-2-yl)azetidin-3-yl)propiolamid,
[41] 3-(3-Chlorphenyl)-N-(2-(ethyl(thiazol-2-yl)amino)ethyl)propiolamid,
[42] 3-(3-Chlorphenyl)-N-(2-((5-cyanothiazol-2-yl)(methyl)amino)ethyl)propiolamid,
[43] 1-(3-(3-Chlorphenyl)propiolyl)-3-(methyl(5-fluor-thiazol-2-yl)amino)azetidin,
[44] 1-(3-(3-Chlorphenyl)propiolyl)-3-(methyl(5-fluor-thiazol-2-yl)amino)pyrrolidin,
[45] N-(1-(4-Methylthiazol-2-yl)pyrrolidin-3-yl)-3-phenylpropiolamid,
[46] 3-(3-Methoxyphenyl)-N-(1-(4-methylthiazol-2-yl)pyrrolidin-3-yl)propiolamid,
[47] 3-(2-Methoxyphenyl)-N-(1-(4-methylthiazol-2-yl)pyrrolidin-3-yl)propiolamid,
[48] 3-(4-Methoxyphenyl)-N-(1-(4-methylthiazol-2-yl)pyrrolidin-3-yl)propiolamid,

[49] 3-(4-Fluorphenyl)-N-(1-(4-methylthiazol-2-yl)pyrrolidin-3-yl)propiolamid,
[50] N-(1-(4-Methylthiazol-2-yl)pyrrolidin-3-yl)-3-p-tolylpropiolamid,
[51] N-(1-(4,5-Dimethylthiazol-2-yl)pyrrolidin-3-yl)-3-phenylpropiolamid,
[52] N-(1-(4,5-Dimethylthiazol-2-yl)pyrrolidin-3-yl)-3-(4-methoxyphenyl)propiolamid,
[53] N-(1-(4,5-Dimethylthiazol-2-yl)pyrrolidin-3-yl)-3-(4-fluorphenyl)propiolamid,
[54] N-(1-(4,5-Dimethylthiazol-2-yl)pyrrolidin-3-yl)-3-(2-methoxyphenyl)propiolamid,
[55] N-(1-(4-tert-Butylthiazol-2-yl)pyrrolidin-3-yl)-3-(4-methoxyphenyl)propiolamid,
[56] N-(1-(4-tert-Butylthiazol-2-yl)pyrrolidin-3-yl)-3-p-tolylpropiofamid,
[57] N-(1-(4-tert-Butylthiazol-2-yl)pyrrolidin-3-yl)-3-(4-fluorphenyl)propiolamid,
[58] N-(1-(4-tert-Butylthiazol-2-yl)pyrrolidin-3-yl)-3-o-tolylpropiolamid,
[59] N-(1-(4-tert-Butylthiazol-2-yl)pyrrolidin-3-yl)-3-(3-fluorphenyl)propiolamid,
[60] N-(1-(4-tert-Butylthiazol-2-yl)pyrrotidin-3-yl)-3-phenylpropiolamid,
[61] N-(1-(4-tert-Butylthiazol-2-yl)pyrrolidin-3-yl)-3-(2-fluorphenyl)propiolamid,
[62] N-(1-(4-tert-Butylthiazol-2-yl)pyrrolidin-3-yl)-3-(3-methoxyphenyl)propiolamid,
[63] N-(1-(4-tert-Butylthiazol-2-yl)pyrrolidin-3-yl)-3-(3-fluor-4-methylphenyl)propiolamid,
[64] N-(1-(4-tert-Butylthiazol-2-yl)pyrrolidin-3-yl)-3-(2-methoxyphenyl)propiolamid,
[65] N-(1-(4-Methylthiazol-2-yl)pyrrolidin-3-yl)-3-(4-(trifluomnethyl)phenyl)propiolamid,
[66] N-(1-(4-Methylthiazol-2-yt)pyrrolidin-3-yl)-3-o-tolylpropiolamid,
[67] 3-(3-Fluorphenyl)-N-(1-(4-methytthiazol-2-yl)pyrrolidin-3-yl)propiolamid,
[68] 3-(3-Fluor-4-methylphenyl)-N-(1-(4-methylthiazol-2-yl)pyrrolidin-3-yl)propiolamid,
[69] 3-(2,4-Difluorphenyl)-N-(1-(4-methylthiazol-2-yl)pyrrolidin-3-yl)propiolamid,
[70] 3-(2-Fluorphenyl)-N-(1-(4-methylthiazoi-2-yl)pyrtolidin-3-yl)propiolamid,
[71] N-(1-(4-Methylthiazol-2-yl)pyrrolidin-3-yl)-3-(3-(trifluormethyl)phenyl)propiolamid,
[72] N-(1-(4-Methylthiazol-2-yl)pyrrolidin-3-yl)-3-m-tolylpropiolamid,
[73] N-(1-(5-Methylthiazol-2-yl)pyrrolidin-3-yl)-3-p-tolylpropiolamid,
[74] N-(1-(5-Methylthiazol-2-yl)pyrrolidin-3-yl)-3-m-tolyipropiolamid,
[75] 3-(2-Methoxyphenyl)-N-(1-(5-methylthiazol-2-yl)pyn-olidin-3-yl)propiolamid,
[76] N-(1-(5-Methylthiazol-2-yl)pyrrolidin-3-yl)-3-phenylpropiolamid,
[77] 3-(2-Fluorphenyl)-N-(1-(5-methylthiazol-2-yl)pyrrolidin-3-yl)propiolamid,
[78] 3-(3-Methoxyphenyl)-N-(1-(5-methylthiazol-2-yl)pyrroüdin-3-yl)propiolamid,
[79] N-(1-(4,5-Dimethylthiazol-2-yl)pyrrolidin-3-yl)-3-(3-fluor-4-methylphenyl)propiolamid,
[80] N-(1-(4,5-Dimethylthiazol-2-yl)pyrrolidin-3-yl)-3-o-tolytpropiolamid,
[81] 3-(3-Fluorphenyl)-N-(1-(5-methylthiazol-2-yl)pyrrolidin-3-yl)propiolamid,
[82] N-(1-(4,5-Dimethylthiazol-2-yl)pyrrolidin-3-yl)-3-m-tolypropiolamid,
[83] N-(1-(5-Methylthiazol-2-yl)pyrrolidin-3-yl)-3-o-tolylpropiolamid,
[84] N-(1-(4,5-Dimethylthiazol-2-yl)pyrrolidin-3-yl)-3-p-tolylpropiolamid,
[85] N-(1-(4,5-Dimethylthiazol-2-yl)pyrrolidin-3-yl)-3-(3-(trifluormethyl)phenyl)propiolamid,
[86] 3-(4-Methoxyphenyl)-N-(1-(5-methylthiazol-2-yl)pyrrolidin-3-yl)propiolamid,
[87] N-(1-(5-Methylthiazol-2-yl)pyrrolidin-3-yl)-3-(3-(trifluormethyl)phenyl)propiolamid,
[88] N-(1-(4,5-Dimethylthiazol-2-yl)pyrrolidin-3-yl)-3-(4-(trifluormethyl)phenyl)propiolamid,
[89] 3-(4-FluorphenylrN-(1-(5-methylthiazot-2-yl)pyrrolidin-3-yl)propiolamid,
[90] N-(1-(4,5-Dimethylthiazol-2-yl)pyrrolidin-3-y1)-3-(3-methoxyphenyl)propiolamid,
[91] 3-(2,4-Difluorphenyl)-N-(1-(4,5-dimethylthiazol-2-yl)pyrrolidin-3-yl)propiolamid,
[92] N-(1-(4,5-Dimethylthiazol-2-yl)pyrrolidin-3-yl)-3-(2-fluorphenyl)propiolamid,
[93] N-(1-(5-Methytthiazo)-2-yl)pyrrolidn-3-yl)-3-(4-(trifluormethyl)phenyl)propiolamid,
[94] 3-(3-Fluor-4-methylphenyl)-N-(1-(5-methylthiazol-2-yl)pyrrolidin-3-yl)propiolamid,
[95] 3-(2,4-Difluorphenyl)-N-(1-(5-methylthiazol-2-y)pyrrolidin-3-y)propiolamid,
[96] N-(1-(4-tert-Butylthiazol-2-yl)pyrrolidin-3-yl)-3-(2,4-difluorphenyl)propiolamid,
[97] N-(1-(4-tert-Butylthiazol-2-yl)pyrrolidin-3-yl)-3-(4-(trifluormethyl)phenyl)propiolamid,
[98] N-(1-(4-tert-Butylthiazol-2-yl)pyrrolidin-3-yl)-3-(3-(trifluormethyl)phenyl)propiolamid,
[99] N-(1-(4-tert-Butylthiazol-2-yl)pyrrolidin-3-yl)-3-m-tolylpropiolamid,
[100] N-(1-(4-tert-Butylthiazol-2-yl)pyrrolidin-3-yl)-3-(3-chlorphenyl)propiolamid,
[101] N-(1-(4-tert-Butylthiazol-2-yl)pyrrolidin-3-yl)-3-(thiophen-2-yl)propiolamid,
[102] N-(1-(4-tert-Butylthiazol-2-yl)pyrrolidin-3-yl)-3-(2,4-dichlorphenyl)propiolamid,
[103] N-(1-(4-tert-Butylthiazol-2-yl)pyrrolidin-3-yl)-3-(2-chlor-5-(trifluormethyl)phenyl)propiolamid,
[104] N-(1-(4-tert-Butylthiazol-2-yl)pyrrolidin-3-yl)-3-(3,5-dichlorphenyl)propiolamid,
[105] 3-(3-Chlorphenyl)-N-(1-(4-methylthiazol-2-yl)pyrrolidin-3-yl)propiolamid,
[106] N-(1-(4-Methylthiazol-2-yl)pyrrolidin-3-yl)-3-(thiophen-2-yl)propiolamid,

[107] 3-(2,4-Dichlorphenyl)-N-(1-(4-methylthiazol-2-yl)pyrrolidin-3-yl)propiolamid,
[108] 3-(2-Chlor-5-(trifluorrnethyl)phenyl)-N-(1-(4-methylthiazol-2-yl)pyrrolidin-3-yl)propiolamid,
[109] 3-(3,5-Dichlorphenyl)-N-(1-(4-methylthiazol-2-yl)pyrrolidin-3-yl)propiotamid,
[110] 3-(3-Chlorphenyl)-N-(1-(4,5-dimethylthiazol-2-yl)pyrrolidin-3-yl)propiolamid,
[111] 3-(2,4-Dichlorphenyl)-N-(1-(4,5-dimethylthiazol-2-yl)pyrrolidin-3-yl)propiolamid,
[112] 3-(3-Chlorphenyl)-N-(1-(5-methylthiazol-2-yl)pyrrolidin-3-yl)propiolamid,
[113] N-(1-(5-Methytthiazol-2-yl)pyrrolidin-3-yl)-3-(thiophen-2-yl)propiolamid,
[114] 3-(2,4-Dichlorphenyl)-N-(1-(5-methylthiazol-2-yl)pyrrolidin-3-yl)propiolamid,
[115] 3-(2-Chlor-5-(trifluormethyl)phenyl)-N-(1-(5-methylthiazol-2-yl)pyrrolidin-3-yl)propiolamid,
[116] 3-(3,5-Dichlorphenyl)-N-(1-(5-methylthiazol-2-yl)pyrrolidin-3-yl)propiolamid,
[117] N-(1-(4,5-Dimethylthiazol-2-yl)pyrrolidin-3-yl)-3-(thiophen-2-yl)propiolamid,
[118] 3-(2-Chlor-5-(trifluormethyl)phenyl)-N-(1-(4,5-dimethylthiazol-2-yl)pyrrolidin-3-yl)propiolamid,
[119] N-(1-(5-Methylthiazol-2-yl)pyrrolidin-3-yl)-3-(pyridin-2-yl)propiolamid,
[120] N-(1-(5-Methylthiazol-2-yl)pyrrolidin-3-yl)-3-(pyridin-3-yl)propiolamid,
[121] N-(1-(5-Methylthiazol-2-yl)pyrrolidin-3-yl)-3-(pyridin-4-yl)propiolamid,
[122] N-(1-(4-tert-Butylthiazol-2-yl)pyrrolidin-3-yl)-3-(pyridin-2-yl)propiolamid,
[123] N-(1-(4-tert-Butylthiazol-2-yl)pyrrolidin-3-yl)-3-(pyridin-3-yl)propiolamid und
[126] 3-(3-Chlorphenyl)-N-(2-((5-fluorthiazol-2-yl)(methyl)amino)ethyl)propiolamid;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

Ein weiterer Gegenstand der vorliegenden Erfindung ist ein Verfahren zur Herstellung von Verbindungen der vorstehend angegebenen allgemeinen Formel I gemäß dem wenigstens eine Verbindung der allgemeinen Formel II,

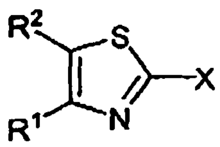

II,

worin die Reste $R^1$ und $R^2$ die vorstehend genannte Bedeutung haben und X für eine Abgangsgruppe, vorzugsweise für einen Halogen-Rest oder einen Sulfonsäureester, besonders bevorzugt für einen Chlor- oder Brom-Rest, steht, mit wenigstens einer Verbindung der allgemeinen Formel III,

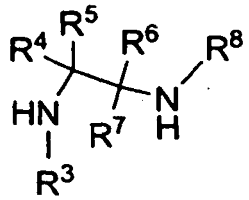

III,

worin $R^3$ $R^4$, $R^5$, $R^6$, $R^7$ und $R^8$ die vorstehend genannte Bedeutung haben, ggf. in einem Reaktionsmedium, ggf. in Gegenwart wenigstens einer Base und/oder wenigstens einer metallorganischen Verbindung und/oder wenigstens eines Metallhydrid-Reagenzes oder in Gegenwart wenigstens eines Kupfersalzes und ggf. in Gegenwart wenigstens eines Metalls, vorzugsweise in Gegenwart von Kupfer, vorzugsweise bei einer Temperatur von - 70 °C bis 300 °C, besonders bevorzugt von - 70 °C bis 150 °C, in wenigstens eine entsprechende Verbindung der allgemeinen Formel IV, ggf. in Form eines entsprechenden Salzes,

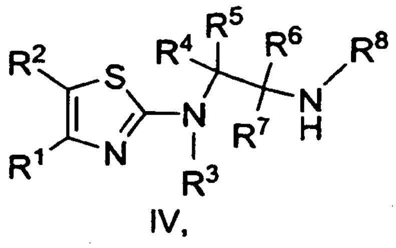

IV,

worin $R^1$, $R^2$, $R^3$, $R^4$, $R^5$, $R^6$, $R^7$ und $R^8$ die vorstehend genannte Bedeutung haben, überführt wird und diese ggf. gereinigt und/oder isoliert wird;
oder wenigstens eine Verbindung der allgemeinen Formel II mit wenigstens einer Verbindung der allgemeinen Formel V,

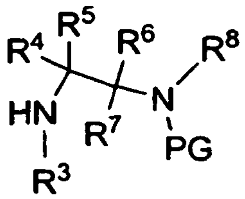

V,

worin $R^3$, $R^4$, $R^5$, $R^6$, $R^7$ und $R^8$ die vorstehend genannte Bedeutung haben und PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht,
ggf. in einem Reaktionsmedium, ggf. in Gegenwart wenigstens einer Base und/oder wenigstens einer metallorganischen Verbindung und/oder wenigstens eines Metallhydrid-Reagenzes, vorzugsweise bei einer Temperatur von - 70 °C bis 300 °C in wenigstens eine entsprechende Verbindung der allgemeinen Formel VI,

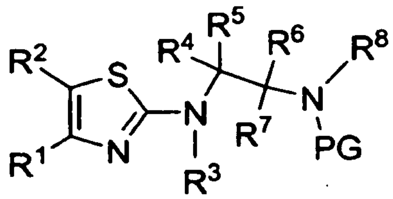

VI,

worin $R^1$, $R^2$, $R^3$, $R^4$, $R^5$, $R^6$, $R^7$, $R^8$ und PG die vorstehend genannte Bedeutung haben, überführt wird und diese ggf. gereinigt und/oder isoliert wird;
und ggf. wenigstens eine Verbindung der allgemeinen Formel VI, worin $R^1$, $R^2$, $R^4$, $R^5$, $R^6$, $R^7$, $R^8$ und PG die vorstehend genannte Bedeutung haben und $R^3$ für Wasserstoff steht, mit wenigstens einer Verbindung $R^3$-X, worin $R^3$ die vorstehend genannte Bedeutung mit Ausnahme von Wasserstoff hat und X für eine Abgangsgruppe, vorzugsweise für einen Halogen-Rest steht, ggf. in einem Reaktionsmedium, ggf. in Gegenwart wenigstens einer Base, bevorzugt in Gegenwart wenigstens eines Metallhydrid-Reagenzes, besonders bevorzugt in Gegenwart von Natriumhydrid, vorzugsweise bei einer Temperatur von - 70 °C bis 300 °C in wenigstens eine entsprechende Verbindung der allgemeinen Formel VIa, wenigstens eine entsprechende Verbindung der allgemeinen Formel IV überführt wird und diese ggf. gereinigt und/oder isoliert wird;
oder wenigstens eine Verbindung der allgemeinen Formel VII,

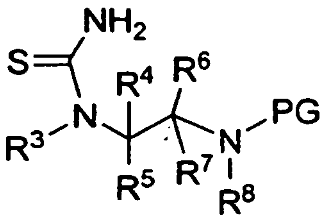

VII,

worin $R^3$, $R^4$, $R^5$, $R^6$, $R^7$ und $R^8$ die vorstehend genannte Bedeutung haben und PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, durch Umsetzung mit wenigstens einer Verbindung der allgemeinen Formel $R^1$-C(=O)-$CH_2$-X oder ($C_{1-5}$-Alkyl-O)$_2$-CH-$CH_2$-X, worin $R^1$ die vorstehend genannte Bedeutung hat und X für eine Abgangsgruppe, vorzugsweise für einen Halogen-Rest, besonders bevorzugt für ein Brom-Atom steht, in einem Reaktionsmedium, ggf. in Gegenwart wenigstens einer organischen Base oder in Gegenwart wenigstens einer Säure, vorzugsweise in Gegenwart wenigstens einer Base ausgewählt aus der Gruppe bestehend aus Triethylamin, Diisopropylethylamin, N-Methylmorpholin, Dimethylaminopyridin und Pyridin oder in Gegenwart wenigstens einer Säure ausgewählt aus der Gruppe bestehend aus Essigsäure, Trifluoressigsäure und Salzsäure, vorzugsweise bei einer Temperatur zwischen - 70 °C bis 300 °C in wenigstens eine entsprechende Verbindung der allgemeinen Formel VI, ggf. in Form eines entsprechenden Salzes überführt wird, und diese ggf. gereinigt und/oder isoliert wird;
und wenigstens eine Verbindung der allgemeinen Formel VI oder der allgemeinen Formel VIa für den Fall, dass PG für eine tert-Butoxycarbonyl- oder 9-Fluorenylmethyloxycarbonyl-Gruppe steht, in einem Reaktionsmedium, in Gegenwart wenigstens einer Säure, vorzugsweise in Gegenwart wenigstens einer Säure

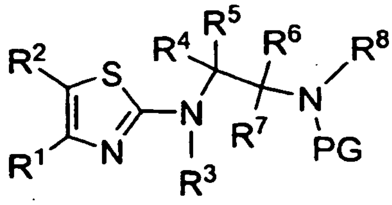

VIa,

worin $R^1$, $R^2$, $R^3$, $R^4$, $R^5$, $R^6$, $R^7$, $R^8$ und PG die vorstehend genannte Bedeutung haben und $R^3$ nicht für ein Wasserstoffatom steht, überführt wird und diese ggf. gereinigt und/oder isoliert wird;
oder wenigstens eine Verbindung der allgemeinen Formel XIII,

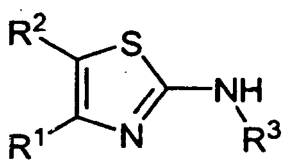

XIII,

worin $R^1$, $R^2$ und $R^3$ die vorstehend genannte Bedeutung haben, mit wenigstens einer Verbindung der allgemeinen Formel XIV,

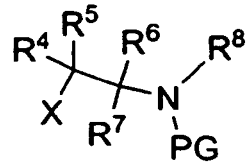

XIV,

worin $R^4$, $R^5$, $R^6$, $R^7$, $R^8$ und PG die vorstehend genannte Bedeutung haben und X für eine Abgangsgruppe, vorzugsweise für einen Halogen-Rest oder für einen Sulfonsäureester, besonders bevorzugt für einen Chlor- oder Brom-Rest steht, ggf. in einem Reaktionsmedium, ggf. in Gegenwart wenigstens einer Base, vorzugsweise in Gegenwart wenigstens einer Base ausgewählt aus der Gruppe bestehend aus Kalium-tert-butylat, Natriumhydroxid, Kaliumhydroxid, Dimethylamin und Triethylamin, besonders bevorzugt in Gegenwart von Diethylamin, oder ggf. in Gegenwart wenigstens einer metallorganischen Verbindung, bevorzugt in Gegenwart wenigstens einer metallorganischen Verbindung ausgewählt aus der Gruppe bestehend aus Methyllithium und Butyllithium oder ggf. in Gegenwart wenigstens einer Metallhydridverbindung, besonders bevorzugt in Gegenwart von Natriumhydrid, vorzugsweise bei einer Temperatur von - 70°C bis 300 °C, besonders bevorzugt von - 70 °C bis 150°C, in wenigstens eine entsprechende Verbindung der allgemeinen Formel VI überführt wird und diese ggf. gereinigt und/oder isoliert wird;
oder wenigstens eine Verbindung der allgemeinen Formel XIII,

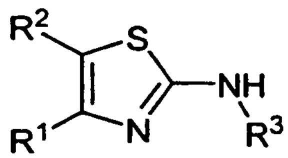

XIII,

worin $R^1$, $R^2$ und $R^3$ die Bedeutung gemäß einem oder mehreren der Ansprüche 1 bis 27 haben, mit wenigstens einer Verbindung der allgemeinen Formel XIVa,

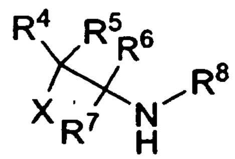

XIVa,

worin $R^4$, $R^5$, $R^6$, $R^7$ und $R^8$ die vorstehend genannte Bedeutung haben und X für eine Abgangsgruppe, vorzugsweise für einen Halogen-Rest oder für einen Sulfonsäureester, besonders bevorzugt für einen Chlor- oder Brom-Rest steht, ggf. in einem Reaktionsmedium, ggf. in Gegenwart wenigstens einer Base, vorzugsweise in Gegenwart wenigstens einer Base ausgewählt aus der Gruppe bestehend aus Kalium-tert-butylat, Natriumhydroxid, Kaliumhydroxid, Dimethylamin und Triethylamin, besonders bevorzugt in Gegenwart von Diethylamin, oder ggf. in Gegenwart wenigstens einer metallorganischen Verbindung, bevorzugt in Gegenwart wenigstens einer metallorganischen Verbindung ausgewählt aus der Gruppe bestehend aus Methyllithium und Butyllithium oder ggf. in Gegenwart wenigstens einer Metallhydridverbindung, besonders bevorzugt in Gegenwart von Natriumhydrid, vorzugsweise bei einer Temperatur von - 70°C bis 300 °C, besonders bevorzugt von - 70 °C bis 150 °C, in ausgewählt aus der Gruppe bestehend aus Salzsäure und Trifluoressigsäure, vorzugsweise bei einer Temperatur zwischen -70 °C bis 100 °C oder für den Fall, dass PG für eine Benzyl-Gruppe oder Benzyloxycarbonyl-Gruppe steht, in einem Reaktionsmedium, in Gegenwart von Wasserstoff und in Gegenwart wenigstens eines Katalysators, vorzugsweise in Gegenwart von Palladium auf Kohle, vorzugsweise bei

einer Temperatur zwischen -70 °C bis 100 °C in wenigstens eine entsprechende Verbindung der allgemeinen Formel IV, ggf. in Form eines entsprechenden Salzes überführt wird, und diese ggf. gereinigt und/oder isoliert wird;
und wenigstens eine Verbindung der allgemeinen Formel IV durch Umsetzung mit wenigstens einer Verbindung der allgemeinen Formel $R^9$-C≡C-C(=O)-OH, worin $R^9$ die vorstehend genannte Bedeutung hat, in einem Reaktionsmedium, ggf. in Gegenwart wenigstens eines geeigneten Kopplungsmittels, das polymergebunden sein kann, ggf. in Gegenwart wenigstens einer Base, vorzugsweise bei einer Temperatur von - 70 °C bis 100 °C, oder durch Umsetzung mit wenigstens einer Verbindung der allgemeinen Formel $R^9$-C≡C-C(=O)-X, worin $R^9$ die vorstehend genannte Bedeutung hat und X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel I, ggf. in Form eines entsprechenden Salzes überführt wird,

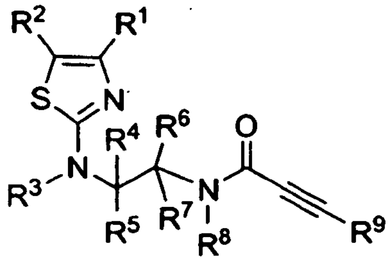

I,

worin $R^1$, $R^2$, $R^3$, $R^4$, $R^5$, $R^6$, $R^7$, $R^8$ und $R^9$ die vorstehend genannte Bedeutung haben, und diese ggf. gereinigt und/oder isoliert wird;
oder wenigstens eine Verbindung der allgemeinen Formel IV durch Umsetzung mit Propiotsäure [HC≡C-C(=O)-OH] in einem Reaktionsmedium, ggf. in Gegenwart wenigstens eines geeigneten Kopplungsmittels, das polymergebunden sein kann, ggf. in Gegenwart wenigstens einer Base, vorzugsweise bei einer Temperatur von - 70 °C bis 100 °C, oder durch Umsetzung mit wenigstens einer Verbindung der allgemeinen Formel HC≡C-C(=O)-X, worin X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel VIII, ggf. in Form eines entsprechenden Salzes überführt wird,

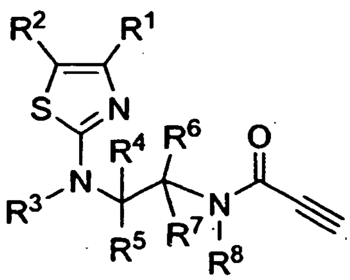

VIII,

worin $R^1$, $R^2$, $R^3$, $R^4$, $R^5$, $R^6$, $R^7$ und $R^8$ die vorstehend genannte Bedeutung haben, und diese ggf. gereinigt und/oder isoliert wird,
und wenigstens eine Verbindung der allgemeinen Formel VIII durch Umsetzung mit wenigstens einer Verbindung der allgemeinen Formel $R^9$-X, worin $R^9$ die vorstehend genannte Bedeutung mit Ausnahme von Wasserstoff hat und X für eine Abgangsgruppe, vorzugsweise für einen Halogen-Rest oder einen Sulfonsäureester, besonders bevorzugt für Iod, Brom oder Triflat, steht, in einem Reaktionsmedium, ggf. in Gegenwart wenigstens eines Katalysators, bevorzugt in Gegenwart wenigstens eines Palladium-Katalysators ausgewählt aus der Gruppe bestehend aus Palladiumchlorid [$PdCl_2$], Palladiumacetat [$Pd(OAc)_2$], Tetrakistriphenylphosphinpalladium [$Pd(PPh_3)_4$], Bistriphenylphosphinpalladiumdichlorid [$Pd(PPh_3)_2Cl_2$] und Bistriphenylphosphinpalladiumacetat [$Pd(PPh_3)_2(OAc)_2$], ggf. in Gegenwart wenigstens eines Liganden, bevorzugt in Gegenwart wenigstens eines Liganden ausgewählt aus der Gruppe bestehend aus Triphenylphosphin, Triphenylarsin und Tri-2-furyl-phosphin, ggf. in Gegenwart wenigstens eines anorganischen Salzes,

bevorzugt in Gegenwart wenigstens eines anorganischen Salzes ausgewählt aus der Gruppe bestehend aus Lithiumchlorid und Zinkchlorid, ggf. in Gegenwart wenigstens eines Kupfersalzes, bevorzugt in Gegenwart von Kupferiodid, ggf. in Gegenwart wenigstens einer organischen oder anorganischen Base, vorzugsweise in Gegenwart wenigstens einer Base ausgewählt aus der Gruppe bestehend aus Triethylamin, [1,4]-Diazabicyclo-[2.2.2]-octan, Diisopropylamin, Diisopropylethylamin, Kaliumcarbonat und Natriumhydrogencarbonat, vorzugsweise bei einer Temperatur zwischen -70 °C bis 300 °C in wenigstens eine entsprechende Verbindung der allgemeinen Formel I, ggf. in Form eines entsprechenden Salzes überführt wird, und diese ggf. gereinigt und/oder isoliert wird.

Ebenfalls ein Gegenstand der vorliegenden Erfindung ist ein Verfahren zur Herstellung von Verbindungen der allgemeinen Formel I gemäß dem wenigstens eine Verbindung der allgemeinen Formel III,

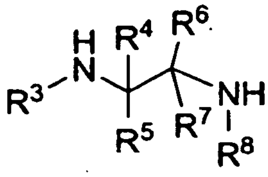

III,

worin $R^3$, $R^4$, $R^5$, $R^6$, $R^7$ und $R^8$ die vorstehend genannte Bedeutung haben,
durch Umsetzung mit wenigstens einer Verbindung der allgemeinen Formel $R^9$-C≡C-C(=O)-OH, worin $R^9$ die vorstehend genannte Bedeutung hat, in einem Reaktionsmedium, ggf. in Gegenwart wenigstens eines geeigneten Kopplungsmittels, das polymergebunden sein kann, ggf. in Gegenwart wenigstens einer Base, vorzugsweise bei einer Temperatur von - 70 °C bis 100 °C, oder durch Umsetzung mit wenigstens einer Verbindung der allgemeinen Formel $R^9$-C≡C-C(=O)-X, worin $R^9$ die vorstehend genannte Bedeutung hat und X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel IX, ggf. in Form eines entsprechenden Salzes,

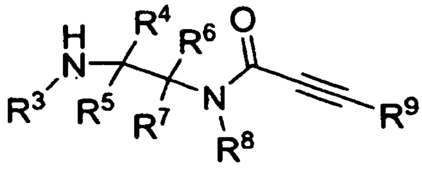

IX,

worin $R^3$, $R^4$, $R^5$, $R^6$, $R^7$, $R^8$ und $R^9$ die vorstehend genannte Bedeutung haben, überführt wird und diese ggf. gereinigt und/oder isoliert wird;
oder wenigstens eine Verbindung der allgemeinen Formel V,

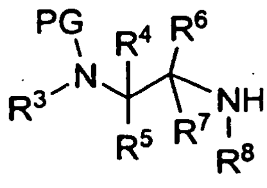

V,

worin $R^3$, $R^4$, $R^5$ , $R^6$, $R^7$ und $R^8$ die vorstehend genannte Bedeutung haben und PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht,
durch Umsetzung mit wenigstens einer Verbindung der allgemeinen Formel $R^9$-C≡C-C(=O)-OH, worin $R^9$ die vorstehend genannte Bedeutung hat, in einem Reaktionsmedium, ggf. in Gegenwart wenigstens eines geeigneten Kopplungsmittels, das polymergebunden sein kann, ggf. in Gegenwart wenigstens einer Base, vorzugsweise bei einer Temperatur von - 70 °C bis 100 °C, oder durch Umsetzung mit wenigstens einer Verbindung der allgemeinen Formel $R^9$-C≡C-C(=O)-X, worin $R^9$ die vorstehend genannte Bedeutung hat und X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel XI, ggf. in Form eines entsprechenden Salzes,

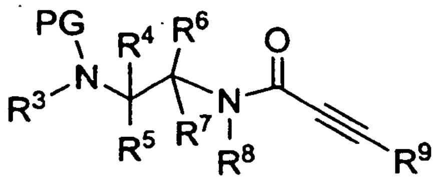

XI,

worin $R^3$, $R^4$, $R^5$, $R^6$, $R^7$, $R^8$, $R^9$ und PG die vorstehend genannte Bedeutung haben, überführt wird und diese ggf. gereinigt und/oder isoliert wird;
und wenigstens eine Verbindung der allgemeinen Formel XI für den Fall, dass PG für eine tert-Butoxycarbonyl- oder 9-Fluorenylmethyloxycarbonyl-Gruppe steht, in einem Reaktionsmedium, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100 °C oder für den Fall, dass PG für eine Benzyl- oder Benzyloxycarbonyl-Gruppe steht, in einem Reaktionsmedium, in Gegenwart von Wasserstoff und in Gegenwart wenigstens eines Katalysators, vorzugsweise in Gegenwart von Palladium auf Kohle, vorzugsweise bei einer Temperatur zwischen -70 °C bis 100 °C in wenigstens eine entsprechende Verbindung der allgemeinen Formel IX, ggf. in Form eines entsprechenden Salzes überführt wird, und diese ggf. gereinigt und/oder isoliert wird;
und wenigstens eine Verbindung der allgemeinen Formel IX durch Umsetzung mit wenigstens einer Verbindung der allgemeinen Formel II,

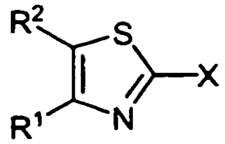

II,

worin die Reste $R^1$ und $R^2$ die vorstehend genannte Bedeutung haben und X für eine Abgangsgruppe, vorzugsweise für einen Halogen-Rest oder einen Sulfonsäureester, besonders bevorzugt für einen Chlor- oder Brom-Rest, steht, in einem Reaktionsmedium, ggf. in Gegenwart wenigstens einer Base und/oder wenigstens einer metallorganischen Verbindung und/oder wenigstens eines Metallhydrid-Reagenzes, vorzugsweise bei einer Temperatur von - 70 °C bis 300 °C in wenigstens eine entsprechende Verbindung der allgemeinen Formel I, ggf. in Form eines entsprechenden Salzes überführt wird, worin $R^1$, $R^2$, $R^3$, $R^4$, $R^5$, $R^6$, $R^7$, $R^8$ und $R^9$ die vorstehend genannte Bedeutung haben, und diese ggf. gereinigt und/oder isoliert wird;
oder ggf. wenigstens eine Verbindung der allgemeinen Formel IX durch Umsetzung mit Kaliumthiocyanat und Ethylchloroformiat oder Ammoniumthiocyanat oder Trimethylsilylisothiocyanat oder Thiophosgen und Ammoniak oder Bromcyan und Schwefelwasserstoff in einem Reaktionsmedium, ggf. in Gegenwart wenigstens einer Säure, vorzugsweise in Gegenwart wenigstens einer Säure ausgewählt aus der Gruppe bestehend aus Salzsäure, Schwefelsäure, Essigsäure und Trifluoressigsäure, besonders bevorzugt in Gegenwart von Salzsäure, oder ggf. in Gegenwart wenigstens einer

Base, vorzugsweise in Gegenwart wenigstens einer Base ausgewählt aus der Gruppe bestehend aus Kalium-tert-butylat, Natriumhydroxid, Kaliumhydroxid, Dimethylamin und Triethylamin, besonders bevorzugt in Gegenwart von Diethylamin, oder ggf. in Gegenwart wenigstens einer metallorganischen Verbindung, bevorzugt in Gegenwart wenigstens einer metallorganischen Verbindung ausgewählt aus der Gruppe bestehend aus Methyllithium und Butyllithium oder ggf. in Gegenwart wenigstens einer Metallhydridverbindung, besonders bevorzugt in Gegenwart von Natriumhydrid, vorzugsweise bei einer Temperatur von - 70 °C bis 250 °C, in wenigstens eine entsprechende Verbindung der allgemeinen Formel XII, ggf. in Form eines entsprechenden Salzes,

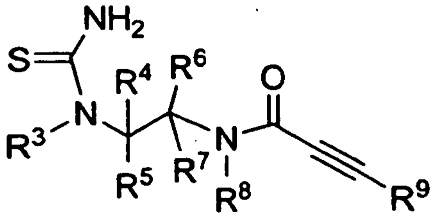

XII,

worin $R^3$, $R^4$, $R^5$, $R^6$, $R^7$, $R^8$ und $R^9$ die vorstehend genannte Bedeutung haben, überführt wird und diese ggf. gereinigt und/oder isoliert wird;

und wenigstens eine Verbindung der allgemeinen Formel XII durch Umsetzung mit wenigstens einer Verbindung der allgemeinen Formel $R^1$-C(=O)-$CH_2$-X oder ($C_{1-5}$-Alkyl-O)$_2$-CH-$CH_2$-X, worin $R^1$ die vorstehend genannte Bedeutung hat und X für eine Abgangsgruppe, vorzugsweise für einen Halogen-Rest, besonders bevorzugt für ein Brom-Atom steht, in einem Reaktionsmedium, ggf. in Gegenwart wenigstens einer organischen Base oder in Gegenwart wenigstens einer Säure, vorzugsweise in Gegenwart wenigstens einer Base ausgewählt aus der Gruppe bestehend aus Triethylamin, Diisopropylethylamin, N-Methylmorpholin, Dimethylaminopyridin und Pyridin oder in Gegenwart wenigstens einer Säure ausgewählt aus der Gruppe bestehend aus Essigsäure, Trifluoressigsäure und Salzsäure, vorzugsweise bei einer Temperatur zwischen - 70 °C bis 300 °C in wenigstens eine entsprechende Verbindung der allgemeinen Formel I, ggf. in Form eines entsprechenden Salzes überführt wird, und diese ggf. gereinigt und/oder isoliert wird.

Ein erfindungsgemäßes Verfahren zur Herstellung substituierter Thiazole der vorstehend angegebenen allgemeinen Formel I ist auch im folgenden Schema 1 angegeben.

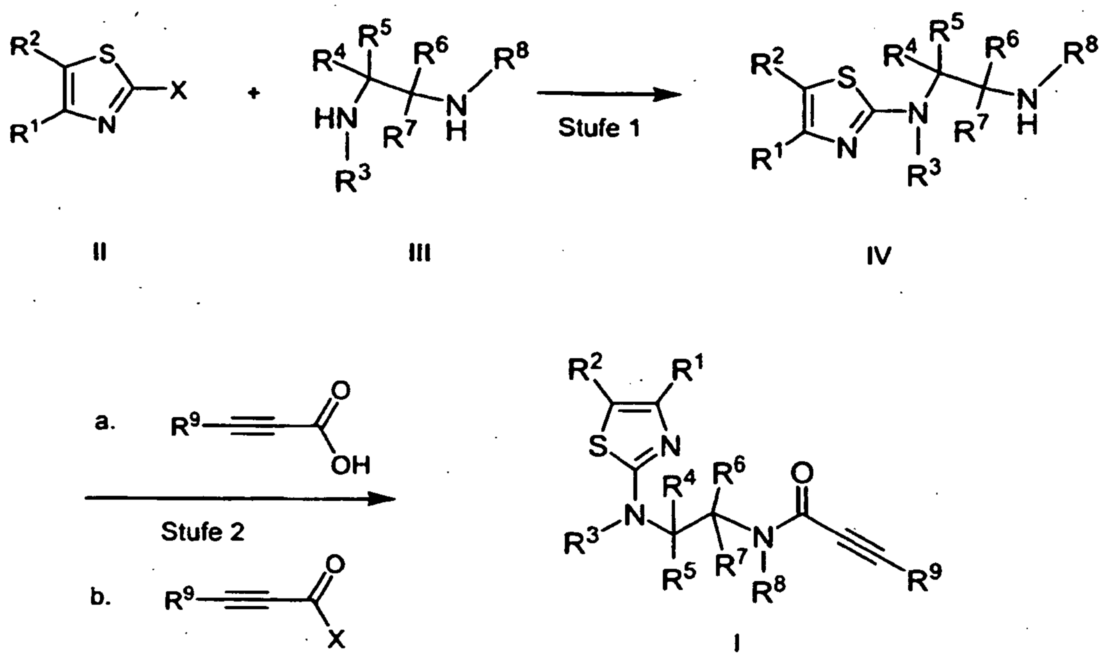


II III IV

I

Schema 1.

In Stufe 1 werden Thiazole der vorstehend angegebenen allgemeinen Formel II, worin X für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor oder Bromatom steht, mit Verbindungen der vorstehend angegebenen allgemeinen Formel III, ggf. in einem Reaktionsmedium, vorzugsweise ausgewählt aus der Gruppe bestehend aus Methanol, Ethanol, Isopropanol, n-Butanol, Diethylether, Tetrahydrofuran, Dichlormethan, Chloroform, Dimethylformamid, Acetonitril, Pyridin, Dioxan, Ethylacetat, Dimethylsulfoxid, Toluol und entsprechenden Mischungen, besonders bevorzugt in einem Reaktionsmedium ausgewählt aus der Gruppe bestehend aus Methanol, Ethanol und n-Butanol, ggf. in Gegenwart wenigstens einer organischen oder anorganischen Base, vorzugsweise ausgewählt aus der Gruppe bestehend aus Triethylamin, Natriumhydrogencarbonat, Dimethylaminopyridin, Kaliumcarbonat und Natriumhydroxid, und/oder ggf. in Gegenwart wenigstens eines Metallsalzes, vorzugsweise eines Kupfersalzes, besonders bevorzugt Kupfer(I)-jodid und/oder Kupfer(I)chlorid, und ggf. in Gegenwart wenigstens eines Metalls, bevorzugt in Gegenwart von Kupfer, und/oder ggf. in Gegenwart wenigstens einer metallorganischen Verbindung oder eines Metallhydrid-Reagenzes, vorzugsweise ausgewählt aus der Gruppe bestehend aus n-Butyllithium, Phenyllithium, Natriumhydrid, Kaliumhydrid und Natriumamid, vorzugsweise bei Temperaturen von -70 °C bis 300 °C, besonders bevorzugt bei Temperaturen von -70 °C bis 150 °C, zu Verbindungen der allgemeinen Formel IV umgesetzt.

In Stufe 2 werden Verbindungen der vorstehend angegebenen allgemeinen Formel IV mit Carbonsäuren der vorstehend angegebenen allgemeinen Formel $R^9$-C≡C-(C=O)-OH in einem Reaktionsmedium, vorzugsweise ausgewählt aus der Gruppe bestehend aus Diethylether, Tetrahydrofuran, Acetonitril, Methanol, Ethanol, (1,2)-Dichlorethan, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), Diisoproylcarbodiimid, 1,1'-Carbonyldiimidazol (CDI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU), O-(Benzotriazol-1-yl)-N,N,N',N'-tetramethyluronium-tetrafluoroborat (TBTU) und 1-Hydroxy-7-azabenzotriazol (HOAt), bevorzugt in Gegenwart von TBTU als Kopplungsreagenz, ggf. in Gegenwart wenigstens einer organischen Base, vorzugsweise ausgewählt aus der Gruppe bestehend aus Triethylamin, Pyridin, Dimethylaminopyridin, N-Methylmorpholin und Diisopropylethylamin, bevorzugt in Gegenwart von Diisopropylethylamin, vorzugsweise bei Temperaturen von -70°C bis 100°C zu Verbindungen der allgemeinen Formel I umgesetzt.

Alternativ werden Verbindungen der vorstehend angegebenen allgemeinen Formel IV mit Carbonsäurederivaten der vorstehend angegebenen allgemeinen Formel $R^9$-C≡C-(C=O)-X, worin X für eine Abgangsgruppe, bevorzugt einen Halogen-Rest, besonders bevorzugt Chlor oder Brom, steht, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens einer organischen oder anorganischen Base, vorzugsweise ausgewählt aus der Gruppe bestehend aus Triethylamin, Dimethylaminopyridin, Pyridin und Diisopropylamin, bei Temperaturen von -70°C bis 100°C zu Verbindungen der allgemeinen Formel I umgesetzt.

Ein weiteres erfindungsgemäßes Verfahren zur Herstellung substituierter Thiazole der vorstehend angegebenen allgemeinen Formel I ist auch im folgenden Schema 2 angegeben.

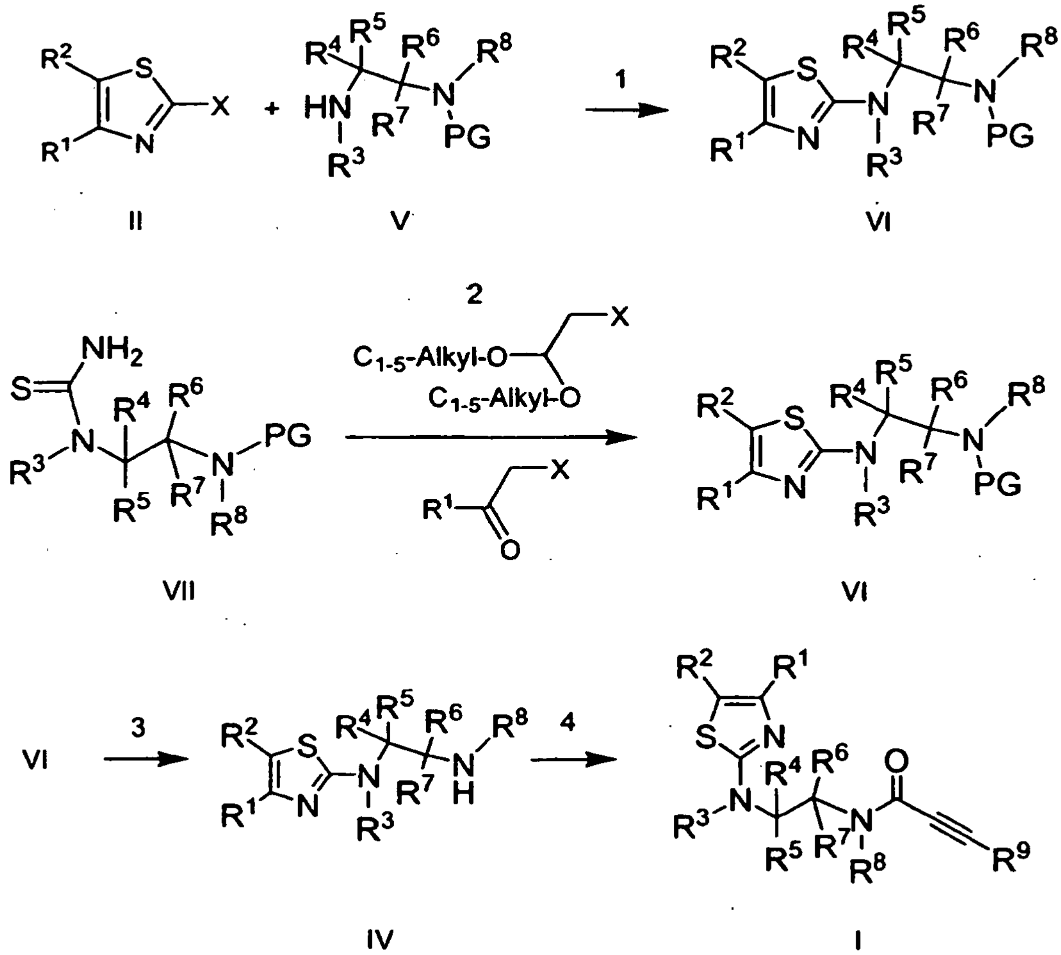


II V VI

VII VI

IV I

Schema 2.

In Stufe 1 werden Thiazole der vorstehend angegebenen allgemeinen Formel II, worin X für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor- oder Bromatom steht, mit Verbindungen der vorstehend angegebenen allgemeinen Formel V, worin PG für eine Schutzgruppe, bevorzugt für eine Schutzgruppe ausgewählt aus der Gruppe bestehend aus tert-Butyloxy-carbonyl, Benzyloxycarbonyl, Benzyl und 9-Fluorenylmethyloxycarbonyl steht zu Verbindungen der allgemeinen Formel VI umgesetzt.

Genaue Bedingungen lassen sich auch der Veröffentlichung Journal of Medicinal Chemistry 1972, 15(3), Seiten 295 bis 301 entnehmen. Die entsprechenden Teile der Veröffentlichung gelten hiermit als Teil der Offenbarung.

In Stufe 2 werden Verbindungen der vorstehend angegebenen allgemeinen Formel VII, worin PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, mit wenigstens einer Verbindung der allgemeinen Formel $R^1$-C(=O)-$CH_2$-X oder ($C_{1-5}$-Alkyl-O)$_2$-CH-$CH_2$-X, bevorzugt mit wenigstens einer Verbindung der allgemeinen Formel $R^1$-C(=O)-$CH_2$-X oder ($C_2H_5$-O)$_2$-CH-$CH_2$-X, worin X für eine Abgangsgruppe, vorzugsweise für einen Halogen-Rest, besonders bevorzugt für ein Brom-Atom steht, in einem Reaktionsmedium, bevorzugt in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Toluol und entsprechenden Mischungen, besonders bevorzugt in Ethanol und/oder Dioxan, ggf. in Gegenwart wenigstens einer organischen Base oder in Gegenwart wenigstens einer Säure, vorzugsweise in Gegenwart wenigstens einer Base ausgewählt aus der Gruppe bestehend aus Triethylamin, Diisopropylethylamin, N-Methylmorpholin, Dimethylaminopyridin und Pyridin oder in Gegenwart wenigstens einer Säure ausgewählt aus der Gruppe bestehend aus Essigsäure, Trifluoressigsäure und Salzsäure, vorzugsweise bei einer Temperatur zwischen - 70 °C bis 300 °C in eine entsprechende Verbindung der allgemeinen Formel VI umgesetzt.

Genaue Bedingungen lassen sich auch der Veröffentlichung Journal of Medicinal Chemistry 1998, 41 (25), Seiten 5027 bis 5054 entnehmen. Die entsprechenden Teile der Veröffentlichung gelten hiermit als Teil der Offenbarung.

In Stufe 3 werden Verbindungen der allgemeinen Formel VI für den Fall, dass PG für eine tert-Butoxycarbonyl- oder 9-Fluorenylmethyloxycarbonyl-Gruppe steht, in einem Reaktionsmedium, vorzugsweise in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Toluol und entsprechenden Mischungen, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100 °C oder für den Fall, dass PG für eine Benzyl-Gruppe oder Benzyloxycarbonyl-Gruppe steht, in einem Reaktionsmedium, vorzugsweise in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Toluol und entsprechenden Mischungen, in Gegenwart von Wasserstoff und in Gegenwart wenigstens eines Katalysators, vorzugsweise in Gegenwart von Palladium auf Kohle, vorzugsweise bei einer Temperatur zwischen -70 °C bis 100 °C in eine entsprechende Verbindung der allgemeinen Formel IV überführt.

Geeignete Methoden zum Entfernen der vorstehend genannten Schutzgruppen lassen sich auch den Monografien Protective Groups in Organic Synthesis, T. W. Greene et al., 3rd edition, 1999, Wiley, New York und Protecting Groups, P. J. Kocienski, 3rd edition, 2004, Georg Thieme Verlag, Stuttgart 2004 entnehmen. Die entsprechenden Teile der Referenzen gelten hiermit als Teil der Offenbarung.

In Stufe 4 werden Verbindungen der vorstehend angegebenen allgemeinen Formel IV mit Carbonsäuren der vorstehend angegebenen allgemeinen Formel $R^9$-C≡C-(C=O)-OH oder mit Carbonsäurederivaten der vorstehend angegebenen allgemeinen Formel $R^9$-C≡C-(C=O)-X wie in Schema 1, Stufe 2, beschrieben zu Verbindungen der allgemeinen Formel I umgesetzt.

Ein weiteres erfindungsgemäßes Verfahren zur Herstellung substituierter Thiazole der vorstehend angegebenen allgemeinen Formel I ist auch im folgenden Schema 3 angegeben.

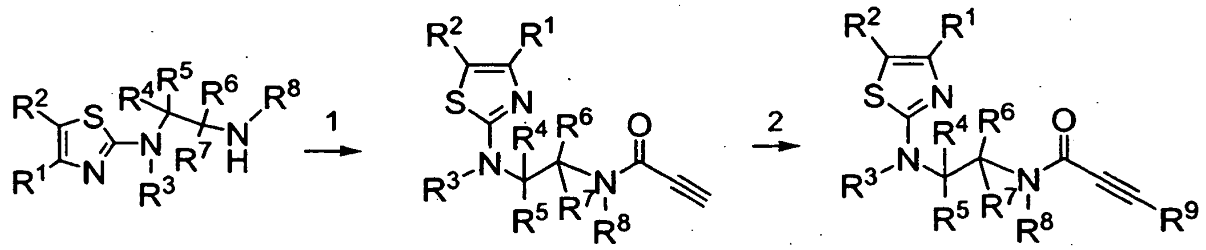


IV VIII I

Schema 3.

In Stufe 1 werden Verbindungen der vorstehend angegebenen allgemeinen Formel IV mit Propiolsäure H-C≡C-(C=O)-OH oder mit Carbonsäurederivaten der allgemeinen Formel H-C≡C-(C=O)-X, worin X für eine Abgangsgruppe, bevorzugt einen Halogen-Rest, besonders bevorzugt Chlor oder Brom, steht, wie in Schema 1, Stufe 2, beschrieben, zu Verbindungen der allgemeinen Formel VIII umgesetzt.

In Stufe 2 werden Verbindungen der vorstehend angegebenen allgemeinen Formel VIII mit Verbindungen der allgemeinen Formel $R^9$-X, worin $R^9$ die vorstehend genannte Bedeutung mit Ausnahme von Wasserstoff hat und X für eine Abgangsgruppe, vorzugsweise für einen Halogen-Rest oder einen Sulfonsäureester, besonders bevorzugt für Iod, Brom oder Triflat, steht, in einem Reaktionsmedium, bevorzugt in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Wasser, Toluol und entsprechenden Mischungen, bevorzugt in Dimethylformamid, Wasser, Ethylacetat, Tetrahydrofuran und entsprechenden Mischungen, ggf. in Gegenwart wenigstens eines Katalysators, bevorzugt in Gegenwart wenigstens eines Palladium-Katalysators ausgewählt aus der Gruppe bestehend aus Palladiumchlorid [$PdCl_2$], Palladiumacetat [$Pd(OAc)_2$], Tetrakistriphenylphosphinpalladium [$Pd(PPh_3)_4$], Bistriphenylphosphinpalladium-dichlorid [$Pd(PPh_3)_2Cl_2$] und Bistriphenylphosphinpalladiumacetat [$Pd(PPh_3)_2(OAc)_2$], bevorzugt in Gegenwart von $Pd(PPh_3)_4$, $Pd(PPh_3)_2Cl_2$ und $Pd(PPh_3)_2(OAc)_2$, ggf. in Gegenwart wenigstens eines Liganden, bevorzugt in Gegenwart wenigstens eines Liganden ausgewählt aus der Gruppe bestehend aus Triphenylphosphin, Triphenylarsin und Tri-2-furyl-phosphin, bevorzugt in Gegenwart von Triphenylphosphin, ggf. in Gegenwart wenigstens eines anorganischen Salzes, bevorzugt in Gegenwart wenigstens eines anorganischen Salzes ausgewählt aus der Gruppe bestehend aus Lithiumchlorid und Zinkchlorid, ggf. in Gegenwart wenigstens eines Kupfersalzes, bevorzugt in Gegenwart von Kupferiodid, ggf. in Gegenwart wenigstens einer organischen oder anorganischen Base, vorzugsweise in Gegenwart wenigstens einer Base ausgewählt aus der Gruppe bestehend aus Triethylamin, [1,4]-Diazabicyclo-[2.2.2]-octan, Diisopropylamin, Diisopropylethylamin, Kaliumcarbonat und Natriumhydrogencarbonat, vorzugsweise bei einer Temperatur zwischen -70 °C bis 300 °C in eine Verbindung der allgemeinen Formel I umgesetzt. Besonders bevorzugt werden Verbindungen der allgemeinen Formel $R^9$-I oder $R^9$-Br mit Verbindungen der allgemeinen Formel VIII in Dimethylformamid in Gegenwart von $Pd(PPh_3)_2Cl_2$, Kupfer(I)iodid und Diisopropylamin oder Triethylamin umgesetzt.

Ein weiteres erfindungsgemäßes Verfahren zur Herstellung substituierter Thiazole der vorstehend angegebenen allgemeinen Formel I ist auch im folgenden Schema 4 angegeben

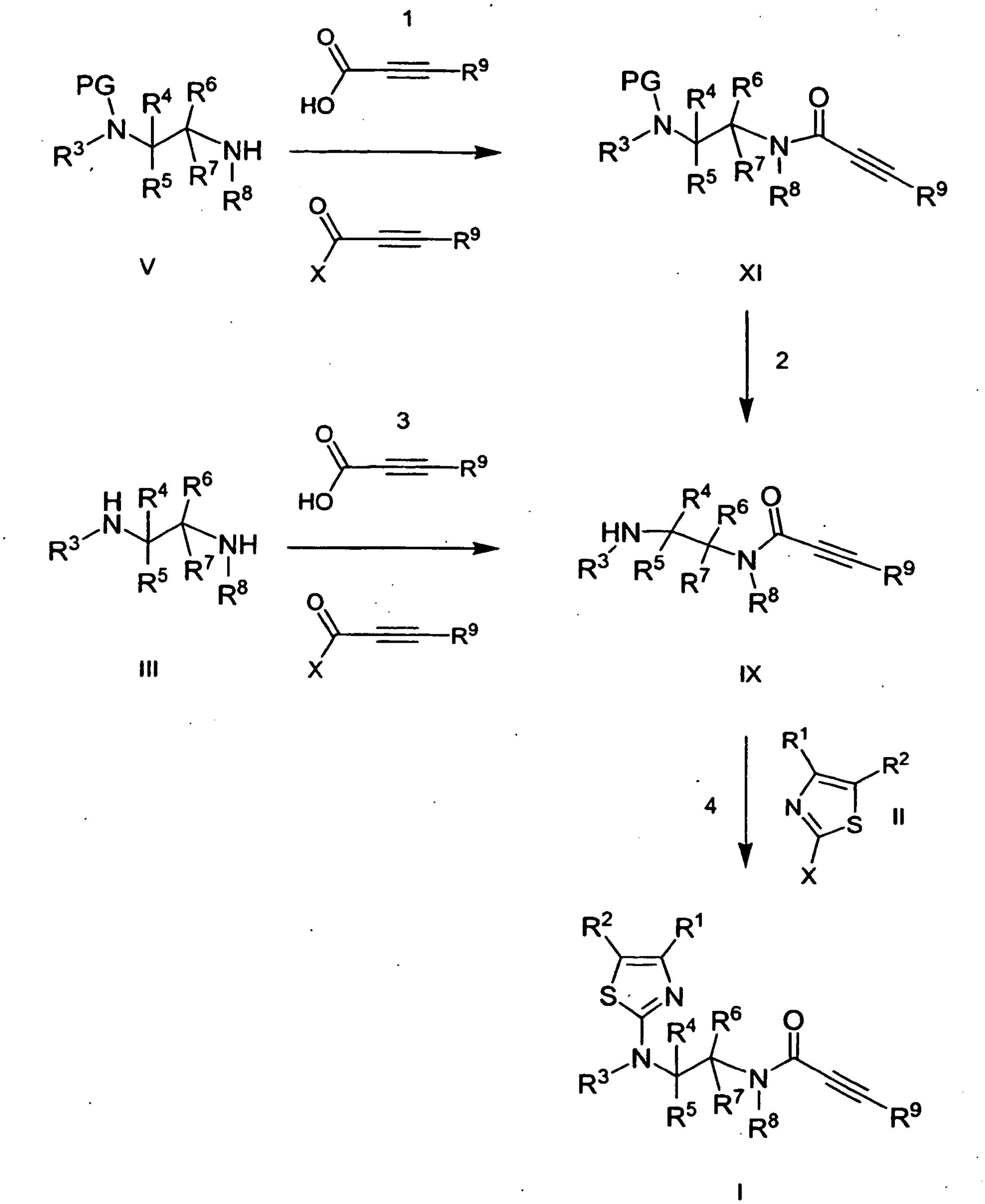


**Schema 4.**

In Stufe 1 werden Verbindungen der allgemeinen Formel V, worin PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, mit Verbindungen der allgemeinen Formel $R^9$-C≡C-(C=O)-OH oder $R^9$-C≡C (C=O)-X, worin X für eine Abgangsgruppe, bevorzugt einen Halogen-Rest, besonders bevorzugt Chlor oder Brom, steht, wie in Schema 1, Stufe 2, zu Verbindungen der allgemeinen Formel XI umgesetzt.

In Stufe 2 werden Verbindungen der allgemeinen Formel XI, worin PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, wie in Schema 2, Stufe 3, beschrieben zu Verbindungen der allgemeinen Formel IX umgesetzt.

In Stufe 3 werden Verbindungen der vorstehend angegebenen allgemeinen Formel III mit Propiolsäure H-C≡C-(C=O)-OH oder mit Carbonsäurederivaten der vorstehend angegebenen allgemeinen Formel H-C≡C-(C=O)-X, worin X für eine Abgangsgruppe, bevorzugt einen Halogen-Rest, besonders bevorzugt Chlor oder Brom, steht, wie in Schema 1, Stufe 2, beschrieben, zu Verbindungen der allgemeinen Formel IX umgesetzt.

In Stufe 4 werden Verbindungen der allgemeinen Formel IX mit Verbindungen der allgemeinen Formel II wie in Schema 1, Stufe 1, zu Verbindungen der allgemeinen Formel I umgesetzt.

Verbindungen der allgemeinen Formel I, worin $R^3$ und $R^8$ jeweils für einen Wasserstoff-Rest stehen, im Folgenden als Verbindungen der allgemeinen Formel XV bezeichnet, lassen sich in Verbindungen der allgemeinen Formel I, worin $R^3$ und $R^8$ mit der sie verbindenden -N-$CR^4R^5$-$CR^6R^7$-N-Gruppe einen zyklischen Rest bilden, im Folgenden als Verbindungen der allgemeinen Formel XVI bezeichnet, überführen.

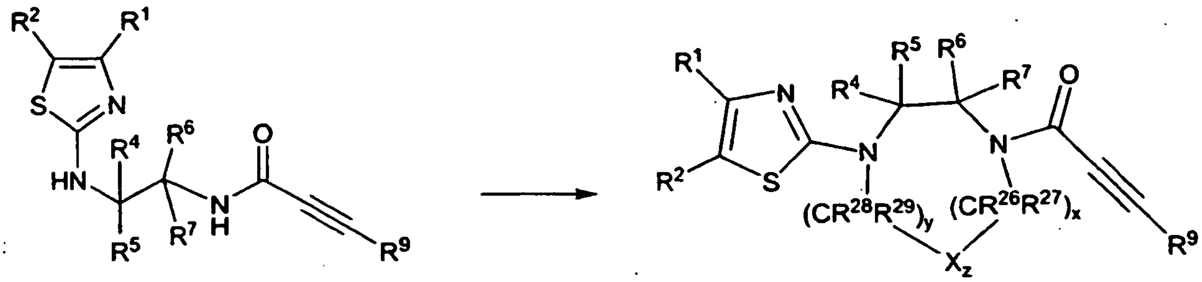

XV XVI

Verbindungen der allgemeinen Formel XV lassen sich mit Verbindungen der allgemeinen Formel Y-$(CR^{28}R^{29})_y$-$X_z$-$(CR^{26}R^{27})_x$-W, worin $R^{28}$, $R^{29}$, $R^{26}$, $R^{27}$, X, y, z und x die vorstehend genannte Bedeutung haben und Y und W unabhängig voneinander jeweils für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor-, oder Bromatom stehen, ggf. in einem Reaktionsmedium, vorzugsweise ausgewählt aus der Gruppe bestehend aus Methanol, Ethanol, Isopropanol, n-Butanol, Diethylether, Tetrahydrofuran, Dichlormethan, Chloroform, Dimethylformamid, Acetonitril, Pyridin, Dioxan, Ethylacetat, Dimethylsulfoxid, Toluol und entsprechenden Mischungen, besonders bevorzugt in einem Reaktionsmedium ausgewählt aus der Gruppe bestehend aus Acetonitril, Dichlorethan, Chloroform, Dimethylformamid, Tetrahydrofuran und Diethylether, ggf. in Gegenwart wenigstens einer organischen oder anorganischen Base, vorzugsweise ausgewählt aus der Gruppe bestehend aus Triethylamin, Natriumhydrogencarbonat, Dimethylaminopyridin, Kaliumcarbonat und Natriumhydroxid, und/oder ggf. in Gegenwart wenigstens einer metallorganischen Verbindung oder eines Metallhydrid-Reagenzes, vorzugsweise ausgewählt aus der Gruppe bestehend aus n-Butyllithium, Phenyllithium, Natriumhydrid, Kalium-tert.-Butanolat, Kaliumhydrid und Natriumamid, vorzugsweise bei Temperaturen von -70 °C bis 300 °C, besonders bevorzugt bei Temperaturen von -70 °C bis 150 °C, zu Verbindungen der allgemeinen Formel XVI, umsetzen.

Verbindungen der allgemeinen Formel XV lassen sich ebenfalls in Verbindungen der allgemeinen Formel I, worin wenigstens einer der Reste $R^3$ und $R^8$ nicht für einen Wasserstoff-Rest stehen, überführen.

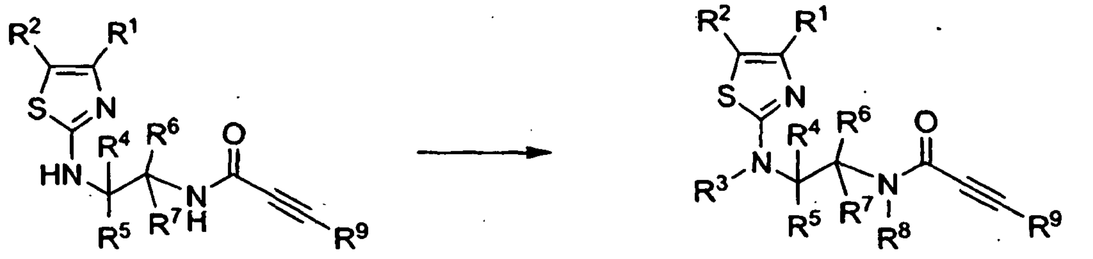

XV

I, $R^3$ und/oder $R^8$ ungleich H

Verbindungen der allgemeinen Formel XV lassen sich mit Verbindungen der allgemeinen Formel $R^3$-X oder $R^8$-X, worin $R^3$ und $R^8$ die vorstehend genannte Bedeutung haben und X für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor-, oder Bromatom steht, ggf. in einem Reaktionsmedium, vorzugsweise ausgewählt aus der Gruppe bestehend aus Methanol, Ethanol, Isopropanol, n-Butanol, Diethylether, Tetrahydrofuran, Dichlormethan, Chloroform, Dimethylformamid, Acetonitril, Pyridin, Dioxan, Ethylacetat, Dimethylsulfoxid, Toluol und entsprechenden Mischungen, besonders bevorzugt in einem Reaktionsmedium ausgewählt aus der Gruppe bestehend aus Acetontiril, Dichlorethan, Chloroform, Dimethylformamid, Tetrahydrofuran und Diethylether, ggf. in Gegenwart wenigstens einer organischen oder anorganischen Base, vorzugsweise ausgewählt aus der Gruppe bestehend aus Triethylamin, Natriumhydrogencarbonat, Dimethylaminopyridin, Kaliumcarbonat und Natriumhydroxid, und/oder ggf. in Gegenwart wenigstens einer metallorganischen Verbindung oder eines Metallhydrid-Reagenzes, vorzugsweise ausgewählt aus der Gruppe bestehend aus n-Butyllithium, Phenyllithium, Natriumhydrid, Kalium-tert.-Butanolat, Kaliumhydrid und Natriumamid, vorzugsweise bei Temperaturen von -70 °C bis 300 °C, besonders bevorzugt bei Temperaturen von -70 °C bis 150 °C, zu Verbindungen der allgemeinen Formel 1, worin wenigstens einer der Reste $R^3$ und $R^8$ nicht für einen Wasserstoff-Rest steht, umsetzen.

Die Verbindungen der vorstehend angegebenen Formeln II, III, V, VII, XIII, XIV, sowie der allgemeinen Formeln $R^9$-X, Y-$(CR^{28}R^{29})_y$-$X_z$-$(CR^{26}R^{27})_x$-W, $R^3$-X, $R^8$-X, $R^9$-C≡C-(C=O)-OH, $R^9$-C≡C-(C=O)-X und H-C≡C-C(=O)-X sind jeweils am Markt käuflich erhältlich und/oder können nach den üblichen, dem Fachmann bekannten Verfahren hergestellt werden.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die gemäß den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Sofern die erfindungsgemäßen substituierten Thiazole der vorstehend angegebenen allgemeinen Formel I bezeichnet, nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Thiazole der vorstehend angegebenen allgemeinen Formel I sowie ggf. jeweils entsprechende Stereoisomere können nach üblichen, dem Fachmann bekannten Verfahren in Form entsprechender Salze, bevorzugt in Form entsprechender Hydrochloride, insbesondere in Form entsprechender physiologisch verträglicher Salze erhalten werden, erhalten werden, wobei das erfindungsgemäße Arzneimittel eines oder mehrere

Salze einer oder mehrerer dieser Verbindungen aufweisen kann.

Die jeweiligen Salze der erfindungsgemäßen substituierten Thiazole der vorstehend angegebenen allgemeinen Formel I sowie entsprechender Stereoisomeren können beispielsweise durch Umsetzung mit einer oder mehreren anorganischen Säuren und/oder einer oder mehreren organischen Säuren erhalten werden. Geeignete Säuren können bevorzugt ausgewählt werden aus der Gruppe bestehend aus Perchlorsäure, Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Sacharinsäure, Cyclohexansulfamidsäure, Aspartam, Monomethylsebacinsäure, 5-Oxo-prolin, Hexan-1-sulfonsäure, Nicotinsäure, 2-Aminobenzoesäure, 3-Aminobenzoesäure oder 4-Aminobenzoesäure, 2,4,6-Trimethylbenzoesäure, $\alpha$-Liponsäure, Acetylglycin, Hippursäure, Phosphorsäure, Maleinsäure, Malonsäure und Asparaginsäure.

Die erfindungsgemäßen substituierten Thiazole der vorstehend angegebenen allgemeinen Formel I sowie ggf. entsprechende Stereoisomere und jeweils deren physiologisch verträgliche Salze können nach üblichen, dem Fachmann bekannten Verfahren auch in Form ihrer Solvate, insbesondere in Form ihrer Hydrate erhalten werden.

Es wurde überraschenderweise gefunden, dass sich die erfindungsgemäßen substituierten Thiazole der vorstehend angegebenen allgemeinen Formel I zur mGluR5-Rezeptor-Regulation eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Die erfindungsgemäßen substituierten Thiazole der vorstehend angegebenen allgemeinen Formel I und ggf. entsprechende Stereoisomere sowie jeweils die entsprechenden Salze erscheinen toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens ein erfindungsgemäßes substituiertes Thiazol der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, sowie ggf. einen oder mehrere pharmazeutisch verträgliche Hilfsstoffe.

Das erfindungsgemäße Arzneimittel eignet sich zur mGluR5-Rezeptor-Regulation, insbesondere zur Inhibierung des mGluR5-Rezeptors.

Bevorzugt eignet sich das erfindungsgemäße Arzneimittel zur Prophylaxe und/oder Behandlung von Störungen und/oder Krankheiten, die zumindest teilweise durch mGluR5-Rezeptoren vermittelt werden.

Besonders bevorzugt eignet sich das erfindungsgemäße Arzneimittel daher zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz: Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt des Aufmerksamkeit-Defizit-Syndroms (ADS); Angstzuständen; Panikattacken; Epilepsie; Husten; Harninkontinenz; Diarrhöe; Pruritus; Schizophrenie; cerebralen Ischämien; Muskelspasmen; Krämpfen; Lungenkrankheiten, vorzugsweise ausgewählt aus der Gruppe bestehend aus Asthma und Pseudokrupp; Regurgitation (Erbrechen); Schlaganfall; Dyskinesie; Retinopathie; Antriebslosigkeit; Kehlkopfentzündung (Laryngitis); Störungen der Nahrungsaufnahme, vorzugsweise ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-)abhängigkeit; Toleranzentwicklung gegenüber Medikamenten, vorzugsweise gegenüber natürlichen oder synthetischen Opioiden; Magen-Ösaphagus-Reflux-Syndrom; gastroösophageale Refluxkrankheit; Reizdarmsyndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

Ganz besonders bevorzugt eignet sich das erfindungsgemäße Arzneimittel zur Prophylaxe von Schmerz, vorzugsweise von Schmerz ausgewählt aus der Gruppe bestehend aus akutem Schmerz, chronischem Schmerz, neuropathischem Schmerz und visceralem Schmerz; Angstzuständen; Panikattacken; Alkoholabhängigkeit; Medikamentenabhängigkeit; Störungen der Nahrungsaufnahme, vorzugsweise ausgewählt aus der Gruppe bestehend aus Bulimie, Kachexie, Anorexie und Fettleibigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-)abhängigkeit; Toleranzentwicklung gegenüber Medikamenten und/oder Drogen, insbesondere gegenüber natürlichen oder synthetischen Opioiden; Magen-Ösaphagus-Reflux-Syndrom, gastroösophageale Refluxkrankheit und Reizdarmsyndrom.

Noch weiter bevorzugt eignet sich das erfindungsgemäße Arzneimittel zur Prophylaxe und/oder Behandlung

von Schmerz, vorzugsweise von Schmerz ausgewählt aus der Gruppe bestehend aus akutem Schmerz, chronischem Schmerz, neuropathischem Schmerz und visceralem Schmerz, Angstzuständen und Panikattacken.

Am weitesten bevorzugt eignet sich das erfindungsgemäße Arzneimittel zur Prophylaxe und/oder Behandlung von Schmerzen, vorzugsweise von akuten Schmerzen, chronischen Schmerzen, neuropathischen Schmerzen oder visceralen Schmerzen.

Ein weiterer Gegenstand der vorliegenden Erfindung ist die Verwendung wenigstens eines erfindungsgemäßen substituierten Thiazols der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, sowie ggf. eines oder mehrerer pharmazeutisch verträglicher Hilfsstoffe zur Herstellung eines Arzneimittels zur mGluR5-Rezeptor-Regulation, vorzugsweise zur Inhibierung des mGluR5-Rezeptors.

Bevorzugt ist die Verwendung wenigstens eines erfindungsgemäßen substituierten Thiazols der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, sowie ggf. eines oder mehrerer pharmazeutisch verträglicher Hilfsstoffe zur Herstellung eines Arzneimittels zur Prophylaxe und/oder Behandlung von Störungen und/oder Krankheiten, die zumindest teilweise durch mGluR5-Razeptoren vermittelt werden.

Besonders bevorzugt ist die Verwendung wenigstens eines erfindungsgemäßen substituierten Thiazols der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz; Migräne: Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt des Aufmerksamkeit-Defizit-Syndroms (ADS); Angstzuständen; Panikattacken; Epilepsie; Husten; Harninkontinenz; Diarrhöe; Pruritus; Schizophrenie; cerebralen Ischämien; Muskelspasmen: Krämpfen; Lungenkrankheiten, vorzugsweise ausgewählt aus der Gruppe bestehend aus Asthma und Pseudokrupp; Regurgitation (Erbrechen): Schlaganfall; Dyskinesie; Retinopathie; Antriebslosigkeit; Kehlkopfentzündung (Laryngitis); Störungen der Nahrungsaufnahme, vorzugsweise ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-)abhängigkeit; Toleranzentwicklung gegenüber Medikamenten, insbesondere gegenüber natürlichen oder synthetischen Opioiden; Magen-Ösaphagus-Reflux-Syndrom; gastroösophageale Refluxkrankheit; Reizdarmsyndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

Ganz besonders bevorzugt ist die Verwendung wenigstens eines erfindungsgemäßen substituierten Thiazols der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz, und visceralem Schmerz; Angstzuständen; Panikattacken; Alkoholabhängigkeit; Medikamentenabhängigkeit; Störungen der Nahrungsaufnahme, vorzugsweise ausgewählt aus der Gruppe bestehend aus Bulimie, Kachexie, Anorexie und Fettleibigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-) abhängigkeit; Toleranzentwicklung gegenüber Medikamenten und/oder Drogen, insbesondere gegenüber natürlichen oder synthetischen Opioiden; Magen-Ösaphagus-Reflux-Syndrom, gastroösophageale Refluxkrankheit und Reizdarmsyndrom.

Noch weiter bevorzugt ist die Verwendung wenigstens eines erfindungsgemäßen substituierten Thiazols der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz, Angstzuständen und Panikattacken.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf, zu Tabletten verpreßt, in Kapseln abgefüllt oder in einer Flüssigkeit suspendiert, vorliegen und als solche auch verabreicht werden.

Neben wenigstens einem erfindungsgemäßen substituierten Thiazol der vorstehend angegebenen allgemeinen Formel I, ggf. in Form seiner reinen Stereoisomeren, insbesondere Enantiomeren oder Diastereomeren, seines Racemates oder in Form von Mischungen der Stereoisomeren, insbesondere der Enantiomeren oder Diastereomeren, in einem beliebigen Mischungsverhältnis, oder ggf. in Form eines entsprechenden Salzes, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln. Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Injektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten Thiazol der vorstehend angegebenen allgemeinen Formel I sind in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten Thiazole der vorstehend angegebenen allgemeinen Formel I auch verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge des jeweiligen substituierten Thiazols der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,05 bis 100 mg/kg, vorzugsweise 0,05 bis 10 mg/kg, Körpergewicht des Patienten wenigstens einer solchen Verbindung appliziert.

**Pharmakologische Methoden:**

**I. Methode zur Bestimmung der Hemmung der [$^3$H]-MPEP-Bindung im mGluR5-Rezeptor-Bindungsassay**

Schweinehimhomogenat wird durch Homogenisieren (Polytron PT 3000, Kinematica AG, 10.000 Umdrehungen pro Minute für 90 Sekunden) von Schweinehimhälften ohne Medulla, Cerebellum und Pons in Puffer pH 8.0 (30mM Hepes, Sigma, Bestellnr.H3375 + 1 Tablette Complete auf 100ml, Roche Diagnostics, Bestellnr. 1836145) im Verhältnis 1:20 (Hirngewicht/Volumen) und Differentialzentrifugation bei 900 x g und 40.000 x g hergestellt. In 250 µl Inkubationsansätzen in 96 Well Mikrotiterplatten werden jeweils 450 µg Protein aus Himhomogenat mit 5nM $^3$[H]-MPEP (Tocris, Bestellnr. R1212) (MPEP = 2-Methyl-6-(3-methoxyphenyl)-ethinylpyridin) und die zu untersuchenden Verbindungen (10 µM im Test) in Puffer (wie oben) bei Raumtemperatur für 60 min inkubiert.

Danach werden die Ansätze mit Hilfe eines Brandel Cell Harvesters (Brandel, TYP Robotic 9600) auf Unfilterplates mit Glasfaserfiltermatten (Perkin Elmer, Bestellnr. 6005177) filtriert und anschließend mit Puffer (wie oben) 3 mal mit je 250 µl pro Probe nach gewaschen. Die Filterplatten werden anschließend für 60 min bei 55 °C getrocknet. Anschließend werden pro Well 30 µL Ultima Gold™ Szintillator (Packard BioScience, Bestellnr.6013159) zugegeben und nach 3 Stunden werden die Proben am β-Counter (Mikrobeta, Perkin Elmer) gemessen. Die unspezifische Bindung wird durch Zugabe von 10 µM MPEP (Tocris, Bestellnr.1212) bestimmt.

**II. Methode zur Bestimmung des $Ca^{2+}$-Influx im mGluR5-Rezeptor-Assay**

Eine agonistische und/oder antagonistische Wirkung von Substanzen kann am mGluR5-Rezeptor der Spezies Ratte mit folgendem Assay bestimmt werden. Gemäß diesem Assay wird die intrazelluläre $Ca^{2+}$-Freisetzung nach Aktivierung des mGluR5-Rezeptors mit Hilfe eines $Ca^{2+}$-sensitiven Farbstoffs (Typ Fluo-4, Molecular Probes Europe BV, Leiden Niederlande) in der FlexStation (Molecular Devices, Sunnyvale, USA) quantifiziert.

**Präparation kortikaler Neurone:**

Kortikale Neuronen werden unter sterilen Bedingungen aus postnatalen Ratten (P2-6) präpariert. Hierzu wird der Kortex entnommen und direkt in Kollagenase-Lösung (PAA Laboratories GmbH, Cölbe, Deutschland) überführt und 45 Minuten im Heizschüttler (37°C, 300 Umdrehungen pro Minute) inkubiert. Anschließend wird die Kollagenase-Lösung entnommen und das Gewebe mit Kulturmedium versetzt.

<u>Kulturmedium (100 ml):</u>

Neurobasalmedium (Gibco Invitrogen GmbH, Karlsruhe, Deutschland)
2 mM L-Glutamin (Sigma, Taufkirchen, Deutschland)
1 Vol-% Antibiotika/Antimycotika-Lösung (PAA Laboratories GmbH, Cölbe, Deutschland)
15 ng/ml NGF (Gibco Invitrogen GmbH, Karlsruhe, Deutschland)
1 ml B27 Supplement (Gibco Invitrogen GmbH, Karlsruhe, Deutschland)
1 ml ITS Supplement (Sigma, Taufkirchen, Deutschland)

Die Zellen werden durch Resuspendieren vereinzelt und nach Zugabe von 15 ml Neurobasalmedium durch einen 70 μm Filtereinsatz (BD Biosciences, Heidelberg, Deutschland) zentrifugiert. Das resultierende Zellpellet wird in Kulturmedium aufgenommen. Anschließend werden die Zellen auf Poly-D-Lysin-beschichtete, schwarze 96-Loch-Platten mit klarem Boden (BD Biosciences, Heidelberg, Deutschland), die zuvor zusätzlich mit Laminin (2 $\mu g/cm^2$, Gibco Invitrogen GmbH, Karlsruhe, Deutschland) beschichtet wurden, ausplattiert. Die Zelldichte beträgt 15.000 Zellen/Loch. Die Zellen werden bei 37°C und 5 % $CO_2$ inkubiert und am 2. oder 3. Tag nach Präparation erfolgt ein Mediumwechsel. Je nach Zellwachstum kann die funktionelle Untersuchung am 3.-7. Tag nach Präparation durchgeführt werden.

**Beschreibung des funktionellen $Ca^{2+}$-Influx assay**

20.000 CHO-hmGluR5 cells/ well (Euroscreen,Gosselies, Belgium) werden in 96 well Platten (BD Biosciences, Heidelberg, Deutschland, Ref 356640, clear bottom, 96 well, Poly-D-Lysine) auspipettiert und über Nacht in HBSS-Puffer (Gibco Nr. 14025-050) mit folgenden Zusätzen: 10% FCS (GIBCO, 10270-106) und Doxycyclin (BD Biosciences Clontech 631311 600ng/ml) inkubiert.

Zur funktionellen Untersuchung wurden die Zellen mit 2 μM Fluo-4 und 0,01 Vol% Pluronic F127 (Molecular Probes Europe BV, Leiden Niederlande) in HBSS-Puffer (Hank's buffered saline solution, Gibco Invitrogen GmbH, Karlsruhe, Deutschland) mit Probenicid (Sigma P8761, 0.69 mg/ml) für 30 min bei 37 °C beladen.

Die Zellen werden dann 3 mal mit Waschpuffer (HBSS-Puffer, Gibco Nr. 14025-050, mit Probenicid (Sigma P8761, 0.69 mg/ml) gewaschen und anschließend mit dem gleichen Puffer ad 100 μl aufgenommen. Nach 15 min. werden die Platten für die Bestimmung von $Ca^{2+}$-Messungen in Gegenwart von DHPG ((S)-3,5-Dihydroxyphenylglycine, Tocris Biotrend Chemikalien GmbH, Köln, Deutschland, finale DHPG-Konzentration: 10 μM) sowie in Gegenwart oder Abwesenheit von Testsubstanzen in einen Fuorometric Imaging Plate Reader (FLIPR, Molecular Devices, Sunnyvale, CA) transferiert.

Die $Ca^{2+}$-abhängige Fluoreszenz wird dabei vor und nach Zugabe von Testsubstanzen gemessen. Die Quantifizierung erfolgt durch die Messung der höchsten Fluoreszenzintensität über die Zeit.

Nach der Aufnahme einer Fluoreszenzbasislinie für 10 sec. werden 50 μl Testsubstanzlösung (verschiedene Testsubstanzkonzentrationen in HBSS-Puffer mit 1 % DMSO und 0.02% Tween 20, Sigma) zugegeben und das Fluoreszenzsignal wird für 6 min gemessen. Anschließend werden 50 μl DHPG-Lösung ((S)-3,5-Dihydroxyphenylglycine, Tocris Biotrend Chemikalien GmbH, Köln, Deutschland, finale DHPG-Konzentration: 10 μM) zugegeben und der Einstrom von $Ca^{2+}$ wird simultan für 60 sec gemessen. Die finale DMSO-Konzentration beträgt 0.25% und der finale Tween 20 Gehalt beträgt 0.005%. Die Daten werden mit Microsoft Excel und GraphPad Prism analysiert. Die Dosis-Wirkungskurven werden mit nicht-linearer Regression berechnet und $IC_{50}$-Werte ermittelt. Jeder Datenpunkt wird 3-fach bestimmt und $IC_{50}$-Werte werden aus minimal 2 unabhängigen Messungen gemittelt.

Ki-Werte werden nach folgender Formel berechnet: $Ki = IC50/(1 + (AG_{konz}/EC50))$.

$AG_{konz.}$ = 10 μM; EC50 entspricht der DHPG-Konzentration, die für den halbmaximalen Einstrom von $Ca^{2+}$ notwendig ist.

**III. Formalintest an der Ratte:**

Der Formalintest (Dubuisson, D. and Dennis, S.G., 1977, Pain, 4, 161 - 174) stellt ein Modell für den akuten sowie den chronischen Schmerz dar. Durch eine einmalige Formalin-Injektion in die dorsale Seite einer Hinterpfote wird bei freibeweglichen Versuchstieren eine biphasische nozizeptive Reaktion induziert, die durch Beobachtung von drei deutlich voneinander unterscheidbaren Verhaltensmustern erfaßt wird. Die Reaktion ist zweiphasisch: Phase 1 = Sofortreaktion (Dauer bis 10 min; Pfotenschütteln, Lecken), Phase 2 = Spätreaktion (nach einer Ruhephase; ebenfalls Pfotenschütteln, Lecken; Dauer bis 60 min). Die 1. Phase reflektiert eine direkte Stimulation der peripheren Nozisensoren mit hohem spinalen nozizeptiven Input bzw. Glutamatfreisetzung (akute Schmerzphase); die 2. Phase reflektiert eine spinale und periphere Hypersensibilisierung (chronische Schmerzphase). In den hier vorgestellten Untersuchungen wurde die chronische Schmerzkomponente (Phase 2) ausgewertet.

Formalin wird mit dem Volumen von 50μl und einer Konzentration von 5 % subkutan in die dorsale Seite der rechten Hinterpfote jedes Tieres appliziert. Die zu testenden Substanzen werden 30 min vor der Formalininjektion oral (p.o.), intravenös (i.v.) oder intraperitoneal (i.p.)appliziert. Die spezifischen Verhaltensänderungen, wie Anheben und Schütteln der Pfote, Gewichtsverlagerungen des Tieres sowie Beiß- und Leckreaktionen werden im Beobachtungszeitraum von 21 bis 27 min nach Formalininjektion beobachtet und registriert. Die Zusammenfassung der verschiedenen Verhaltensweisen erfolgt in der sogenannten Pain-Rate (PR), die, auf die Teilintervalle von 3 min bezogen, die Berechnung einer mittleren Nozizeptionsreaktion darstellt. Die Berechnung der PR erfolgt aufgrund einer numerischen Gewichtung (= jeweils Faktor 1, 2, 3) der beobachteten Verhaltensweisen (entsprechend Verhaltensscore 1, 2, 3) und wird mit folgender Formel berechnet:

$$PR = [(T_0 \times 0) + (T_1 \times 1) + (T_2 \times 2) + (T_3 \times 3)] / 180$$

wobei $T_0$, $T_1$, $T_2$, und $T_3$ jeweils der Zeit in Sekunden entspricht, in der das Tier die Verhaltensweisen 0, 1, 2 oder 3 zeigt. Die Gruppengröße beträgt 10 Tiere (n=10).

Die folgenden Beispiele dienen nur zur Erläuterung der Erfindung und schränken den allgemeinen Erfindungsgedanken nicht ein.

**Beispiele:**

Die Ausbeuten der hergestellten Verbindungen wurden nicht optimiert.

Alle Temperaturen sind unkorrigiert.

Die Angabe "Äquivalente" bedeutet Stoffmengenäquivalente, "RT" Raumtemperatur, "konz." konzentriert, "d" Tage, "min" Minuten, "h" Stunden, "M" ist eine Konzentrationsangabe in mol/l, "aq." wäßrig, "ges." gesättigt, "Lsg." Lösung, "SC" Säulenchromatographie

Weitere Abkürzungen:

| | |
|---|---|
| BOC | tert-Butoxy-carbonyl |
| Brine | gesättigte wäßrige Natriumchloridlösung |
| BuLi | Butyllithium |
| CDI | 1,1'-Carbonyl-diimidazol |
| DCC | Dicyclohexylcarbodiimid |
| DCE | Dichlorethan |
| DCM | Dichlormethan |
| DIC | N,N'-Diisopropylcarbodiimid |
| DMF | N,N-Dimethylformamid |
| DIPE | Diisopropylether |
| DIPEA | Diisopropylethylamin |
| EDCI | N-Ethyl-N'-(3-dimethylaminopropyl)-carbodiimid Hydrochlorid |
| EE | Essigsäureethylester |
| EtOH | Ethanol |
| $H_2O$ | Wasser |
| HOBt | 1-Hydroxy-benzotriazol |

| | |
|---|---|
| Lsg. | Lösung |
| M | Molar |
| MeCN | Acetonitril |
| MeOH | Methanol |
| PS- | Carbodiimid ein polymergebundenes Carbodiimid mit folgender Struktur: |

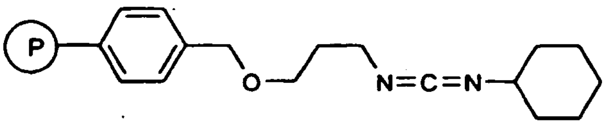

Beladung: 0.9-1.4 mmol/g
Partikelgröße: 75-150 μm

| | |
|---|---|
| TBTU | O-(Benzotriazol-1-yl)-N,N,N',N'-tetramethyluronium-tetrafluoroborat |
| TFA | Trifluoressigsäure |
| THF | Tetrahydrofuran |
| TMSCI | Trimethylchlorsilan |

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den üblichen dem Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik erfolgte durch Massenspektroskopie und/oder NMR.

**Synthese von Beispielverbindung 1:**

**3-Phenyl-N-(1-(thiazol-2-yl)pyrrolidin-3-yl)propiolamid**

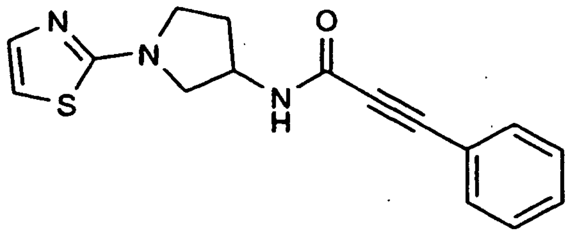

**a) Synthese von tert-Butyl-1-(thiazol-2-yl)pyrrolidin-3-ylcarbamat**

Eine Lösung von 815 μl (9.1 mmol) 2-Bromthiazol und 1.7 g (9.1 mmol) tert-Butyl-pyrrolidin-3-ylcarbamat in n-Butanol (30 ml) wurde 3 h unter Rückfluß erhitzt. Anschließend wurde das Lösungsmittel, im Vakuum entfernt und der Rückstand in Chloroform aufgenommen. Diese Lösung wurde nacheinander mit Wasser und ges. aq. NaCl-Lsg. gewaschen, über $MgSO_4$ getrocknet, filtriert und im Vakuum eingeengt. Mit dem Rückstand wurde eine Säulenchromatographie ($SiO_2$, EE/Hexan 1:1) durchgeführt, wobei 900 mg (3.3 mmol, 37 %) tert-Butyl-1-(thiazol-2-yl)pyrrolidin-3-ylcarbamat erhalten wurden.

**b) Synthese von 1-(Thiazol-2-yl)pyrrolidin-3-amin Hydrochlorid**

Zu einer Lösung von 890 mg (3.3 mmol) tert-Butyl-1-(thiazol-2-yl)pyrrolidin-3-ylcarbamat in MeOH (20 ml)

wurde aq. konz. Salzsäure (2 ml) gegeben. Die Reaktionslösung wurde 16 h bei RT gerührt und anschließend im Vakuum eingeengt. Durch Kristallisation des Rückstands aus einem Ethanol/Ether-Gemisch wurden 653 mg (3.2 mmol, 97%) 1-(Thiazol-2-yl)pyrrolidin-3-amin Hydrochlorid erhalten.

**c) Synthese von 3-Phenyl-N-(1-(thiazol-2-yl)pyrrolidin-3-yl)propiolamid**

Zu einer Lösung von 206 mg (1.4 mmol) Phenylpropiolsäure in DMF (20 ml) wurden unter Kühlung (Eisbad) 323 mg (1.7 mmol) EDCI, 228 mg (1.7 mmol) HOBt und 287 µl (1.7 mmol) Diisopropylethylamin gegeben. Nach 30 min Rühren bei RT wurde unter Kühlung (Eisbad) eine Lösung von 319 mg (1.6 mmol) 1-(Thiazol-2-yl)pyrrolidin-3-amin Hydrochlorid hinzu gegeben. Anschließend wurde die Reaktionslösung 4 h bei 55 °C gerührt. Nach Entfernen des Lösungsmittels im Vakuum wurde der Rückstand in EE aufgenommen, mit einer ges. aq. $NaHCO_3$-Lsg. gewaschen und über $MgSO_4$ getrocknet. Nach Filtrieren und Einengen im Vakuum wurde mit dem Rückstand eine Säulenchromatographie ($SiO_2$, EE/Hexan 1:1) durchgeführt, wobei 180 mg (0.6 mmol, 43 %) 3-Phenyl-N-(1-(thiazol-2-yl)pyrrolidin-3-yl) propiolamid erhalten wurden. MS [MH+] 298.1

**Synthese von Beispielverbindung 2:**

**N-Methyl-3-phenyl-N-(1-(thiazol-2-yl)pyrrolidin-3-yl)propiolamid**

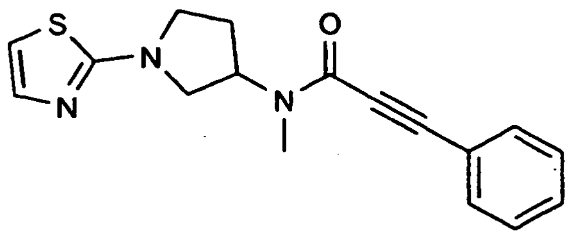

Zu einer Lösung von 71 mg (3.0 mmol) Natriumhydrid in DMF (5 ml) wurde unter Kühlung (Eisbad) eine Lösung von 180 mg (0.6 mmol) 3-Phenyl-N-(1-(thiazol-2-yl)pyrrolidin-3-yl)propiolamid (Beispiel 1) in DMF (5 ml) zugetropft. Anschließen wurde 60 min bei RT gerührt und nach Abkühlen auf 0 °C (Eisbad) wurden 106 µl (1.7 mmol) Iodmethan zugegeben. Die Reaktionslösung wurde 16 h bei RT gerührt und danach mit EE und einer ges. aq. $NaHCO_3$-Lsg. verdünnt. Die Phasen wurden getrennt und die wässrige Phase wurde mehrmals mit EE extrahiert. Die vereinigten organischen Phasen wurden über $MgSO_4$ getrocknet, filtriert und im Vakuum eingeengt. Mit dem Rückstand wurde eine Säulenchromatographie ($SiO_2$, EE/Hexan 1:1) durchgeführt, wobei 170 mg (0.55 mmol, 91 %) N-Methyl-3-phenyl-N-(1-(thiazol-2-yl)pyrrolidin-3-yl)propiolamid erhalten wurden. MS [MH+] 312.1

Aus dieser Verbindung wurde das entsprechende Hydrochlorid erhalten.

**Synthese von Beispielverbindung 3:**

**1-Thiazol-2-yl-4-(3-phenyl-propiolyl)-1,4,-diazepan**

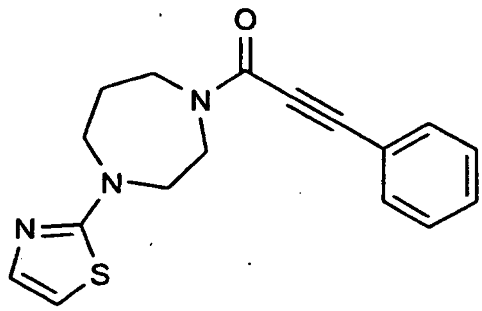

**a) Synthese von 2-(1,4-Diazepan-1-yl)thiazol**

11.0 g (110 mmol) Homopiperazin und 980 µl (11 mmol) 2-Bromthiazol wurden zusammen 10 min auf 140 °C erhitzt. The Reaktionslösung wurde in Wasser gegossen und diese Lösung wurde mit NaCl gesättigt und mit EE extrahiert. Die organische Phase wurde mit Wasser und ges. aq. NaCl-Lsg. gewaschen, über $MgSO_4$ getrocknet und im Vakuum eingeengt. Durch Kristallisation des Rückstands aus Ether wurden 1.54 (8.4 mmol, 76 %) 2-(1,4-Diazepan-1-yl)thiazol erhalten.

**b) Synthese von 1-Thiazol-2-yl-4-(3-phenyl-propiolyl)-1,4-diazepan**

Zu einer Lösung von 292 mg (2.0 mmol) Phenylpropiolsäure in DCE (10 ml) wurden 340 mg (2.1 mmol) CDI gegeben und die Reaktionsmischung wurde 100 min bei RT gerührt.

Nach Abkühlen auf 10 °C wurden 366 mg (2.0 mmol) 2-(1,4-Diazepan-1-yl)thiazol zugegeben und anschließend wurde weitere 4 h bei RT gerührt. Dann wurde die Lösung mit Wasser und ges. aq. NaCl-Lsg. gewaschen, über $MgSO_4$ getrocknet und im Vakuum eingeengt. Mit dem Rückstand wurde eine Säulenchromatographie ($SiO_2$, DCE/EtOH 40:1) durchgeführt, wobei 113 mg (0.36 mmol, 18 %) 1-Thiazol-2-yl-4-(3-phenylpropiolyl)-1,4-diazepan erhalten wurden. MS [MH+] 312.1

**Synthese von Beispielverbindung 4:**

**N-Methyl-N-(2-(methyl(thiazol-2-yl)amino)ethyl)-3-phenylpropiolamid**

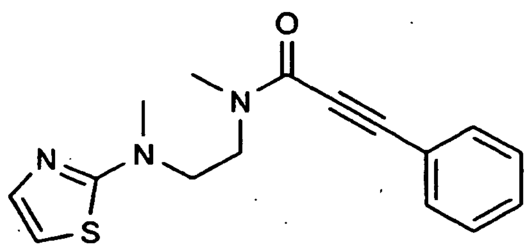

**a) Synthese von $N^1$,$N^2$-Dimethyl-$N^1$-(thiazol-2-yl)ethan-1,2-diamin**

0.54 ml (6.0 mmol) 2-Bromthiazol wurden mit 3.84 ml (36.0 mmol) $N^1$,$N^2$-Dimethylethan-1,2-diamin versetzt und 3 h auf 80 °C erhitzt. Mit dem Rückstand wurde eine Säulenchromatographie ($SiO_2$, DCE/EtOH 5:1) durchgeführt, wobei 371 mg (2.2 mmol, 36 %) $N^1$,$N^2$-Dimethyl-$N^1$-(thiazol-2-yl)ethan-1,2-diamin erhalten wurden.

**b) Synthese von N-Methyl-N-(2-(methyl(thiazol-2-yl)amino)ethyl)-3-phenylpropiolamid**

Die Synthese wurde wie vorstehend nach dem bei Beispiel 3b) beschriebenen Verfahren durchgeführt. MS [MH+] 300.1

**Synthese von Beispielverbindung 8:**

**3-Phenyl-N-(1-(thiazol-2-yl)azetidin-3-yl)propiolamid**

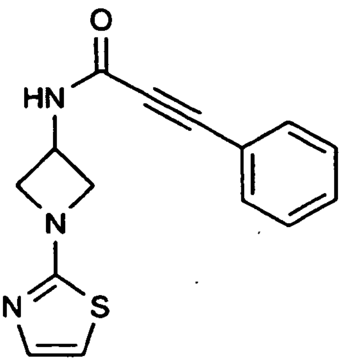

**a) Synthese von tert-Butyl-1-(thiazol-2-yl)azetidin-3-ylcarbamat**

Die Synthese wurde wie vorstehend nach dem bei Beispiel 1a) beschriebenen Verfahren durchgeführt.

**b) Synthese von 1-(Thiazol-2-yl)azetidin-3-amin Hydrochlorid**

467 mg (1.83 mmol) tert-Butyl-1-(thiazol-2-yl)azetidin-3-ylcarbamat wurden in DCE (10 ml) gelöst und mit einer etherischen HCl-Lsg. versetzt. Nach 1 h Rühren bei RT wurde der entstandene Niederschlag abfiltriert und mit Ether nach gewaschen. Dabei wurden 300 mg (1.56 mmol, 86 %) 1-(Thiazol-2-yl)azetidin-3-amin Hydrochlorid erhalten.

**c) Synthese von 3-Phenyl-N-(1-(thiazol-2-yl)azetidin-3-y1)propiolamid**

Zu einer Lösung von 290 mg (1.51 mmol) 1-(Thiazol-2-yl)azetidin-3-amin Hydrochlorid, 221 mg (1.51 mmol) Phenylpropiolsäure und 210 μl (1.51 mmol) $NEt_3$ in DMF (15 ml) wurden 2.32 g (entspricht 3.02 mmol) PS-Carbodiimid resin (Polystrol-Carbodimid Harz) gegeben. Die Reaktionslösung wurde 3 d bei RT geschüttelt. Anschließend wurde filtriert und mit DCM und Ethanol nach gewaschen. Die vereinten Filtrate wurden im Vakuum eingeengt und mit dem Rückstand wurde eine Säulenchromatographie ($SiO_2$, DCE/EtOH 20:1) durchgeführt. Dabei wurden 60 mg (0.21 mmol, 14%) 3-Phenyl-N-(1-(thiazol-2-yl)azetidin-3-yl)propiolamid erhalten. MS [MH+] 284.1

**Synthese von Beispielverbindung 11:**

**3-(Thiazol-2-yl-amino)-1-(3-phenyl-propiolyl)pyrrolidin**

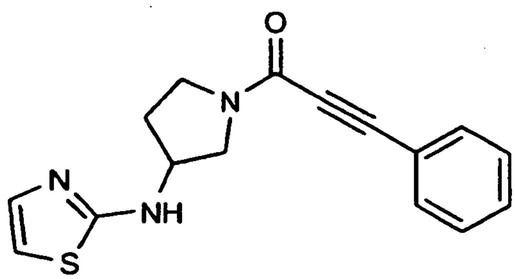

**a) Synthese von tert-Butyl-3-(thiazol-2-ylamino)pyrrolidin-1-carboxylat**

Eine Mischung aus 501 mg (5.0 mmol) 2-Aminothiazol, 926 mg (5.0 mmol) tert-Butyl 3-aminopyrrolidin-1-carboxylat, 2.1 g (10.0 mmol) Natriumtriacetoxyborhydrid und 0.57 ml (10.0 mmol) Essigsäure in DCE (10 ml) wurde 16 h bei RT gerührt. Anschließend wurde mit DCE (50 ml) verdünnt und mit einer ges. aq. $NaHCO_3$-Lsg., Wasser und ges. aq. NaCl-Lsg. gewaschen. Die organische Phase wurde über $MgSO_4$ getrocknet, filtriert und im Vakuum eingeengt. Mit dem Rückstand wurde eine Säulenchromatographie ($SiO_2$, Chlorofom/EtOH 10:1) durchgeführt, wobei 317 mg (1.2 mmol, 24 %) tert-Butyl-3-(thiazol-2-ylamino)pyrrolidin-1-carboxylat erhalten wurden.

**b) Synthese von N-(Pyrrolidin-3-yl)thiazol-2-amin**

317 mg (1.18 mmol) tert-Butyl-3-(thiazol-2-ylamino)pyrrolidin-1-carboxylat wurden in Ether (15 ml) gelöst und mit einer etherischen HCl-Lsg. (10 ml) versetzt. Nach 1 h Rühren bei RT wurde im Vakuum eingeengt. Der Rückstand wurde mit Wasser aufgenommen und mit einer 10%-igen NaOH-Lsg auf pH-11 eingestellt. Danach wurde mit Chloroform extrahiert. Die organische Phase wurde mit Wasser und ges. aq. NaCl-Lsg. gewaschen, über $MgSO_4$ getrocknet, filtriert und im Vakuum eingeengt. Dabei wurden 97 mg (0.57 mmol, 48 %) N-(Pyrrolidin-3-yl)thiazol-2-amin erhalten.

**c) Synthese von 3-(Thiazol-2-yl-amino)-1-(3-phenyl-propiolyl)pyrrolidin**

Zu einer Lösung von 97 mg (0.57 mmol) N-(Pyrrolidin-3-yl)thiazol-2-amin und 85 mg (0.57 mmol) Phenylpropiolsäure in MeCN (5 ml) wurden 100 µl (0.57 mmol) DIPEA und 183 mg (0.57 mmol) TBTU gegeben. Die Reaktionslösung wurde 16 h bei RT gerührt. Anschließend wurde im Vakuum eingeengt und der Rückstand wurde in Chlorform aufgenommen. Dann wurde die org. Lösung mit Wasser und ges. aq. NaCl-Lsg. gewaschen, über $MgSO_4$ getrocknet, filtriert und im Vakuum eingeengt. Mit dem Rückstand wurde eine Säulenchromatographie ($SiO_2$, DCE/EtOH 10:1) durchgeführt. Dabei wurden 72 mg (0.24 mmol, 42%) 3-(Thiazol-2-yl-amino)-1-(3-phenylpropiolyl)pyrrolidin erhalten. MS [MH+] 298.1

**Synthese von Beispielverbindung 12:**

**N-Methyl-N-(2-(methyl(thiazo!-2-yl)amino)cyclohexyl)-3-phenylpropiolamid**

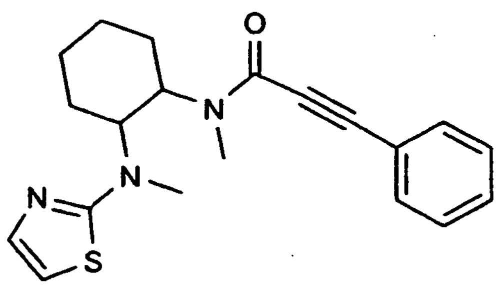

Zu einer Lösung von 150 mg (0.46 mmol) 3-Phenyl-N-(2-(thiazol-2-ylamino)-cyclohexyl)propiolamid (Beispielverbindung 6) in MeCN (10 ml) wurden nacheinander 46 mg (1.15 mmol, 60% in Mineralöl) Natriumhydrid und 1144 µl (2.31 mmol) Iodmethan gegeben. Die Reaktionslösung wurde 4 h bei RT gerührt. Diese Prozedur wurde noch zweimal wiederholt und anschließend wurde im Vakuum eingeengt. Der Rückstand wurde mit DCM aufgenommen, mit Wasser gewaschen, über $MgSO_4$ getrocknet, filtriert und im Vakuum eingeengt. Dabei wurden 131 mg (0.37 mmol, 81 %) N-Methyl-N-(2-(methyl(thiazol-2-yl)amino)cyclohexyl)-3-phenylpropiolamid erhalten. MS [MH+] 354.2

**Synthese von Beispielverbindung 14:**

**N-Methyl-N-(2-(methyl(thiazol-2-yl)amino)-2-oxoethyl)-3-phenylpropiolamid**

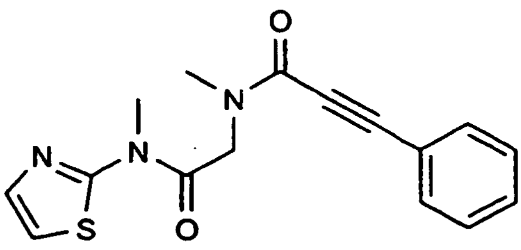

**a) Synthese von N-Methylthiazol-2-amin**

900 µl (10 mmol) 2-Bromthiazol wurden zusammen mit einer 33%-igen ethanolischen Methylamin-Lsg. (70 ml) in einem Autoklaven so hoch erhitzt (ca. 120 °C) bis sich ein Druck von 10 bar aufgebaut hatte. Bei diesen Bedingungen wurde 2 h gerührt. Anschließend wurde die Reaktionsmischung im Vakuum eingeengt und der Rückstand wurde in einer 3%-igen aq. HCl-Lsg. aufgenommen und mit Ether gewaschen. Danach wurde mit einer 20%-igen aq. NaOH-Lsg. auf pH ~12 eingestellt. Die Lösung wurde mit NaCl gesättigt und mit Chloroform extrahiert. Die organische Phase wurde über $MgSO_4$ getrocknet, filtriert und im Vakuum eingeengt. Dabei wurden 761 mg (6.7 mmol, 67%) N-Methylthiazol-2-amin erhalten.

**b) Synthese von tert-Butyl-methyl(2-(methyl(thiazol-2-yl)amino)-2-oxoethyl)carbamat**

Zu einer Lösung von 946 mg (5.0 mmol) Boc-Sarcosin in MeCN (11 ml) wurden 871 µl (5. 0 mmol) Diisopropylethylamin, 571 mg (5.0 mmol) N-Methylthiazol-2-amin und 1.60 g (5.0 mmol) TBTU gegeben und die Reaktionsmischung wurde 16 h bei RT gerührt. Danach wurden nochmals 284 mg (1.5 mmol) Boc-Sarcosin, 216 µl (1.5 mmol) Diisopropylethylamin, 5 ml MeCN und 1.60 g (5.0 mmol) TBTU hinzu gegeben und die Reaktionsmischung wurde weitere 16 h bei RT gerührt. Nach Entfernen des Lösungsmittels im Vakuum wurde der Rückstand in Chloroform aufgenommen, mit Wasser und ges. aq. NaCl-Lsg. gewaschen und über $MgSO_4$ getrocknet. Nach Filtrieren und Einengen im Vakuum wurde mit dem Rückstand eine Säulenchromatographie ($SiO_2$, Chloroform) durchgeführt, wobei 300 mg (1.05 mmol, 21 %) tert-Butyl-methyl(2-(methyl(thiazol-2-yl)amino)-2-oxoethyl)carbamat erhalten wurden.

**c) Synthese von N-Methyl-2-(methylamino)-N-(thiazol-2-yl)acetamid**

300 mg (1.05 mmol) tert-Butyl-methyl(2-(methyl(thiazol-2-yl)amino)-2-oxoethyl)carbamat wurden in DCM (5 ml) gelöst und mit einer etherischen HCl-Lsg. (5 ml) versetzt. Nach 16 h Rühren bei RT wurde die Lösung im Vakuum eingeengt. Der Rückstand wurde in Wasser gelöst und mit Ether gewaschen. Anschließend wurde die Reaktionsmischung mit einer 20%-igen aq. $Na_2CO_3$ basisch gestellt und mit Chloroform extrahiert. Die organische Phase wurde über $MgSO_4$ getrocknet, filtriert und im Vakuum eingeengt.

Dabei wurden 185 mg (1.0 mmol, 95%) N-Methyl-2-(methylamino)-N-(thiazol-2-yl)acetamid erhalten.

**d) Synthese von N-Methyl-N-(2-(methyl(thiazol-2-yl)amino)-2-oxoethyl)-3-phenylpropiolamid**

Zu einer Lösung von 185 mg (1.0 mmol) N-Methyl-2-(methylamino)-N-(thiazol-2-yl)acetamid und 146 mg (1.0 mmol) Phenylpropiolsäure in DCM (40 ml) wurden 1.54 g (entspricht 2.0 mmol) PS-Carbodiimid resin (Polystyrol-Carbodiimid Harz) gegeben. Die Reaktionslösung wurde 16 h bei RT geschüttelt. Anschließend wurde filtriert und mit DCM und Ethanol nach gewaschen. Die vereinten Filtrate wurden mit einer 5%-igen aq. Kaliumcarbonat-Lsg., Wasser und ges. aq. NaCl-Lsg. gewaschen, über $MgSO_4$ getrocknet, filtriert und im Vakuum eingeengt. Mit dem Rückstand wurde eine Säulenchromatographie ($SiO_2$, DCE/EtOH 10:1) durchgeführt. Dabei wurden 99 mg (0.32 mmol, 32 %) N-Methyl-N-(2-(methyl(thiazol-2-yl)amino)-2-oxoethyl)-3-phenylpropiolamid erhalten. MS [MH+] 314.1

**Synthese von Beispielverbindung 15:**

**3-Phenyl-N-(1-(thiazol-2-yl)piperidin-3-yl)propiolamid**

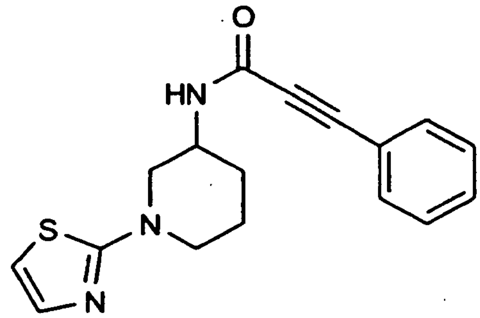

**a) Synthese von 1-(Thiazol-2-yljpiperidin-3-ol**

2.02 g (20 mmol) 3-Hydroxypiperidin und 1.8 ml (20 mmol) 2-Bromthiazol wurden zusammen in n-Butanol (29 ml) gelöst und 6 h bei 110 °C gerührt. Anschließend wurde im Vakuum eingeengt und mit dem Rückstand wurde eine Säulenchromatographie ($SiO_2$, DCM/EtOH 10:1) durchgeführt, wobei 1.54 g (8.4 mmol, 42 %) 1-(Thiazol-2-yl)piperidin-3-ol erhalten wurden.

**b) Synthese von 1-(Thiazol-2-yl)piperidin-3-yl-methansulfonat**

Zu einer Lösung von 500 mg (2.7 mmol) 1-(Thiazol-2-yl)piperidin-3-ol in DCE (15 ml) wurden 1.5 ml (10.8 mmol) $NEt_3$ und eine katalytische Menge 4-Dimethylaminopyridin gegeben. Dann wurde unter Kühlung (Eisbad) eine Lösung von 376 µl (4.86 mmol) Methansulfonsäurechlorid in DCE (2.5 ml) hinzu getropft und anschließend wurde 2 h bei RT gerührt. Dann wurde mit Wasser und ges. aq. NaCl-Lsg. gewaschen und über $MgSO_4$ getrocknet. Nach Filtrieren und Einengen im Vakuum wurde mit dem Rückstand eine Säulenchromatographie ($SiO_2$, Chloroform/EE 10:1) durchgeführt, wobei 502 mg (1.9 mmol, 71%) 1-(Thiazol-2-yl)piperidin-3-yl-methansulfonat erhalten wurden

**c) Synthese von 1-(Thiazol-2-yl)piperidin-3-amin**

Eine Lösung von 836 mg (3.18 mmol) 1-(Thiazol-2-yl)piperidin-3-yl-methansulfonat in einer bei 0° C gesättigten ethanolischen Ammoniak-Lsg. (25 ml) wurde 10 h im Autoklaven bei 100 °C gerührt. Anschließend wurde in Vakuum eingeengt und mit dem Rückstand eine Säulenchromatographie (DCE/EtOH/aq.$NH_3$ 250:50:3) durchgeführt, wobei 406 mg (2.2 mmol, 70 %) 1-(Thiazol-2-yl)piperidin-3-amin erhalten wurden.

**d) Synthese von 3-Phenyl-N-(1-(thiazol-2-yl)piperidin-3-yl)propiolamid**

Zu einer Lösung von 206 mg (1.12 mmol) 1-(Thiazol-2-yl)piperidin-3-amin in DCE (10 ml) wurden 234 µl $NEt_3$ gegeben und die Lösung wurde auf -20 °C abgekühlt. Bei dieser Temperatur wurden 221 mg (1.34 mmol) Phenylpropiolsäurechlorid gelöst in DCE (2 ml) zugegeben und die Reaktionsmischung wurde 2 h bei RT gerührt. Anschließend wurde mit Wasser und ges. aq. NaCl-Lsg. gewaschen. Die organische Phase wurde über $MgSO_4$ getrocknet, filtriert und im Vakuum eingeengt. Mit dem Rückstand wurde eine Säulenchromatographie ($SiO_2$, DCE/EtOH 10:1, dann Hexan/EE 1:1) durchgeführt, wobei 37 mg (0.12 mmol, 11 %) 3-Phenyl-N-(1-(thiazol-2-yl)piperidin-3-yl)propiolamid erhalten wurden. MS [MH+] 312.1

**Synthese von Beispielverbindung 17:**

**N-Methyl-N-(2-(methyl(thiazol-2-yl)amino)ethyl)-3-(pyrid-2-yl)-propiolamid**

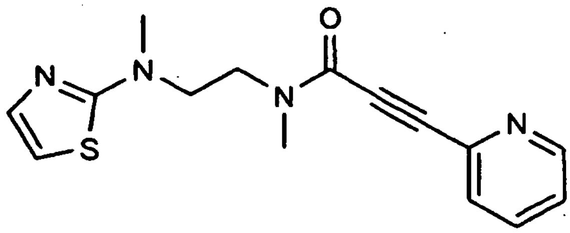

**a) Synthese von N-Methyl-N-(2-(methyl(thiazol-2-yl)amino)ethyl)propiolamid**

Zu einer Lösung von 514 mg (3.0 mmol) $N^1$,$N^2$-Dimethyl-$N^1$-(thiazol-2-yl)ethan-1,2-diamin (siehe Beispiel 4a)) und 185 µl (3.0 mmol) Propiolsäure in THF (20 ml) wurden 525 µl (3.9 mmol) Diisopropylethylamin und 963 mg (3.0 mmol) TBTU gegeben und die Reaktionsmischung wurde 1 h bei RT gerührt. Anschließend wurde im Vakuum eingeengt und der Rückstand wurde mit DCM aufgenommen, mit Wasser und ges. aq. NaCl-Lsg. gewaschen und über $MgSO_4$ getrocknet. Nach Filtrieren und Einengen im Vakuum wurde mit dem Rückstand eine Säulenchromatographie ($SiO_2$, Hexan/EE 4:1) durchgeführt, wobei 484 mg (2.2 mmol, 72 %) N-Methyl-N-(2-(methyl(thiazol-2-yl)amino)ethyl)propiolamid erhalten wurden.

**b) Synthese von N-Methyl-N-(2-(methyl(thiazol-2-yl)amino)ethyl)-3-(pyrid-2-yl)-propiolamid**

484 mg (2.17 mmol) N-Methyl-N-(2-(methyl(thiazol-2-yl)amino)ethyl)propiolamid wurden zusammen mit 444 mg (2.17 mmol) 2-Iodpyridin, 28 mg $Pd(PPh_3)_2Cl_2$, 15 mg Kupfer(I)iodid und 600 µl $NEt_3$ in DMF (10 ml) gelöst und 1 h bei 60 °C gerührt. Die Reaktionslösung wurde im Vakuum weitgehend eingeengt und anschließend mit Wasser verdünnt. Dann wurde mit DCM extrahiert und die organische Phase wurde mit Wasser gewaschen und über $MgSO_4$ getrocknet. Nach Filtrieren und Entfernen des Lösungsmittels im Vakuum wurde mit dem Rückstand eine Säulenchromatographie ($SiO_2$, DCM/EtOH 10:1) durchgeführt, wobei 134 mg (0.45 mmol, 21 %) N-Methyl-N-(2-(methyl(thiazol-2-yl)amino)ethyl)-3-(pyrid-2-yl)-propiolamid erhalten wurden. MS [MH+] 301.1

**Synthese von Beispielverbindung 18:**

**N-Methyl-N-(2-(methyl(thiazol-2-yl)amino)ethyl)-3-(3-chlor-phenyl)-propiolamid**

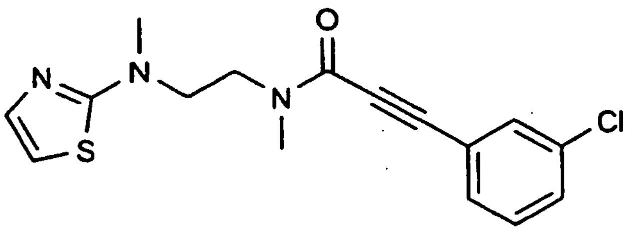

Eine Lösung von 514 mg (3.0 mmol) $N^1$,$N^2$-Dimethyl-$N^1$-(thiazol-2-yl)ethan-1,2-diamin (siehe Beispiel 4 a)) und 542 mg (3.0 mmol) (3-Chlorphenyl)propiolsäure in THF (15 ml) wurde mit 546 µl (3.6 mmol) DIC versetzt und 1 h bei RT gerührt. Anschließend wurde im Vakuum eingeengt und der Rückstand wurde mit DCM aufgenommen, mit Wasser und ges. aq. NaCl-Lsg. gewaschen und über $MgSO_4$ getrocknet. Nach Filtrieren und Entfernen des Lösungsmittels im Vakuum wurde mit dem Rückstand eine Säulenchromatographie (DCM/EtOH 10:1) durchgeführt, wobei 300 mg (0.90 mmol, 30%) N-Methyl-N-(2-(methyl(thiazol-2-yl)amino)ethyl)-3-(3-chlor-phenyl)-propiolamid erhalten wurden. MS [MH+] 334.1

**Synthese von Beispielverbindung 19:**

**N-(2-(Methyl(thiazo)-2-yl)amino)ethyl)-3-(3-chlor-phenyl)-propiolamid**

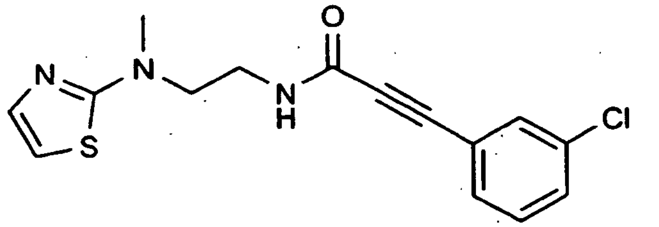

**a) Synthese von N1-Methyl-N1-(thiazol-2-yl)ethan-1,2-diamin**

Zu einer Lösung von 4.5 ml (50 mmol) 2-Bromthiazol und 26.4 ml (300 mmol) N-Methylethylen-1,2-diamin in Isopropanol (25 ml) wurden 159 mg (2.5 mmol) gepulvertes Kupfer und 642 mg (7.5 mmol) Kupfer(I)chlorid gegeben. Die Reaktionsmischung wurde 2 h bei RT gerührt und anschließend im Vakuum eingeengt. Mit dem Rückstand wurde eine Säulenchromatographie ($SiO_2$, DCE/EtOH 5:1) durchgeführt, wobei 576 mg (3.6 mmol, 7 %) N1-Methyl-N1-(thiazol-2-yl)ethan-1,2-diamin erhalten wurden.

**b) Synthese von N-(2-(Methyl(thiazol-2-yl)amino)ethyl)-3-(3-chlor-phenyl)-propiolamid**

314 mg (2.0 mmol) N1-Methyl-N1-(thiazol-2-yl)ethan-1,2-diamin wurden zusammen mit 361 mg (2.0 mmol) (3-Chlorphenyl)propiolsäure, 348 μl (2.0 mmol) Diisopropylethylamin und 642 mg (2.0 mmol) TBTU in MeCN (30 ml) gelöst und 16 h bei RT gerührt. Die Reaktionsmischung wurde im Vakuum eingeengt und der Rückstand wurde mit DCM aufgenommen, mit Wasser gewaschen, über $MgSO_4$ getrocknet und im Vakuum eingeengt. Mit dem Rückstand wurde eine Säulenchromatographie ($SiO_2$, Chloroform/EtOH 40:1) durchgeführt wobei 153 mg (0.48 mmol, 24 %) N-(2-(Methyl (thiazol-2-yl)amino)ethyl)-3-(3-chlor-phenyl)-propiolamid erhalten wurden. MS [MH+] 320.1

**Synthese von Beispielverbindung 22:**

**3-(Methyl(thiazol-2-yl)amino)-1-(3-phenyl-propiolyl)pyrrolidin**

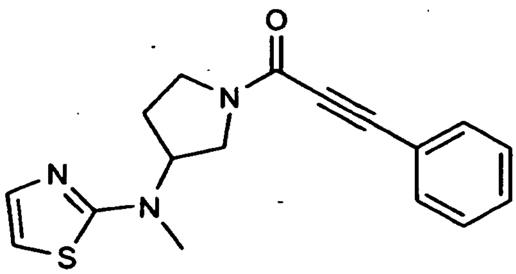

Zu einer Lösung von 334 mg (1.12 mmol) 3-(Thiazol-2-yl-amino)-1-(3-phenylpropiolyl)pyrrolidin (Beispiel11) in MeCN (15 ml) wurden 67 mg (1.68 mmol) Natriumhydrid (60% Suspension in Mineralöl) gegeben und die Lösung wurde 1 h bei RT gerührt. Anschließend wurden 140 μl (2.24 mmol) Methyljodid zugegeben und es wurde wieder 1 h bei RT gerührt. Dann wurde mit einer 25% aq.. Ammoniak-Lsg. (2 ml) versetzt und im Vakuum konzentriert. Der Rückstand wurde mit Chloroform extrahiert. Die organische Phase wurde mit Wasser und ges. aq. NaCl-Lsg. gewaschen, über $MgSO_4$ getrocknet, filtriert und im Vakuum eingeengt. Mit dem Rückstand wurde eine Säulenchromatographie ($SiO_2$, Chloroform) durchgeführt, wobei 99 mg (0.32 mmol, 28%) 3-(Methyl-(thiazol-2-yl)amino)-1-(3-phenyl-propiolyl)pyrrolidin

erhalten wurden. MS [MH+] 312.1

**Synthese von Beispielverbindung 23:**

**N-(2-(Methyl(thiazol-2-yl)amino)ethyl)-3-(3-chlor-phenyl)-propiolamid Hydrochlorid**

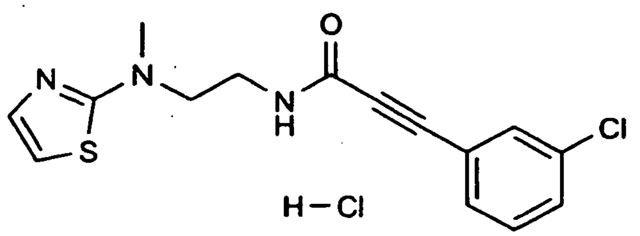

Zu einer Lösung von 500 mg (1.56 mmol) N-(2-(Methyl-(thiazol-2-yl)amino)ethyl)-3-(3-chlor-phenyl)-propiolamid (Beispiel 19) in Ether (50 ml) wurde eine ges. etherische HCl-Lsg. gegeben (10 ml) und es wurde 1 h bei RT gerührt, wobei ein Niederschlag entstand. Dieser wurde abgesaugt und mit Ether nachgewaschen. Dabei wurden 507 mg (1.42 mmol, 91 %) N-(2-(Methyl-(thiazol-2-yl)amino)ethyl)-3-(3-chlor-phenyl)-propiolamid Hydrochlorid erhalten. MS [MH+] 320.1

Die Synthese von Beispielverbindung 5 N-(2-((Thiazol-2-yl)amino)ethyl)-3-phenylpropiolamid (MS [MH+] 272.1) erfolgte nach den vorstehend bei den Beispielverbindungen 19a) und 3b) beschriebenen Verfahren.

Die Beispielverbindungen **6** 3-Phenyl-N-(2-(thiazol-2-ylamino)cyclohexyl)propiolamid (MS [MH+] 326.1),
**9** N-(2-(Methyl(thiazol-2-yl)amino)ethyl)-3-phenylpropiolamid (MS [MH+] 286.1),
**10** N-Methyl-N-(2-(thiazol-2-yl)amino)ethyl-3-phenylpropiolamid (MS [MH+] 286.1).
**21** N-(2-(Methyl(thiazol-2-yl)amino)ethyl)-3-(tol-3-yl)-propiolamid (MS [MH+] 300.1),
**24** N-(2-(Benzo[d]thiazol-2-yl(methyl)amino)ethyl)-3-(3-chlor-phenyl)propiolamid (MS [MH+] 370.1), **25** N-(2-(Methyl-(5-ethoxycarbonyl-thiazol-2-yl)amino)ethyl)-3-(3-chlor-phenyl)-propiolamid (MS [MH+] 392.1), **26** N-(2-(Methyl-(4-ethoxycarbonyl-thiazol-2-yl)amino)ethyl)-3-(3-chlor-phenyl)-propiolamid (MS [MH+] 392.1) und **29** N-(2-(Methyl-(4-methyl-thiazol-2-yl)amino)ethyl)-3-(3-chlor-phenyl)-propiolamid (MS [MH+] 334.1) wurden nach dem vorstehend für Beispielverbindung 19 beschriebenen Verfahren hergestellt.

Die Synthese der Beispielverbindurigen **7** N-Methyl-N-(2-(methyl(thiazol-2-yl)amino)ethyl)-3-(3-methyl-phenyl)-propiolamid (MS [MH+] 314.1)
und 13 N-Methyl-N-(2-(methyl(thiazol-2-yl)amino)ethyl)-3-(3-cyano-phenyl)-propiolamid (MS [MH+] 325.1) erfolgte analog der vorstehend bei den Beispielverbindungen 4a) und 19b) beschriebenen Verfahren.

Die Beispielverbindungen **16** N-Methyl-N-(1-(thiazol-2-yl)piperidin-3-yl)-3-phenyl-propiolamid (MS [MH+] 326.1)
und 20 N-Methyl-N-(1-(thiazol-2-yl)azetidin-3-yl)-3-phenyl-propiolamid (MS [MH+] 298.1) wurden aus wie vorstehend nach dem bei Beispielverbindung 2 beschriebenen Verfahren hergestellt.

Die Beispielverbindung **27** 3-(Thiazol-2-yl-amino)-1-(3-phenyl-propiolyl)piperidin (MS [MH+] 312.1) wurden nach dem vorstehend für Beispielverbindung 11 beschriebenen Verfahren hergestellt.

Die Beispielverbindungen **28** 3-(Methyl-(Thiazol-2-yl)-amino)-1-(3-pheny)-propiolyl)piperidin (MS [MH+] 326.1) wurden aus wie vorstehend nach dem bei Beispielverbindung 22 beschriebenen Verfahren hergestellt.

**Synthese von Beispielverbindung 30:**

**3-(3-Chlorphenyl)-N-(2-(methyl(5-methylthiazol-2-yl)amino)ethyl)propiolamid**

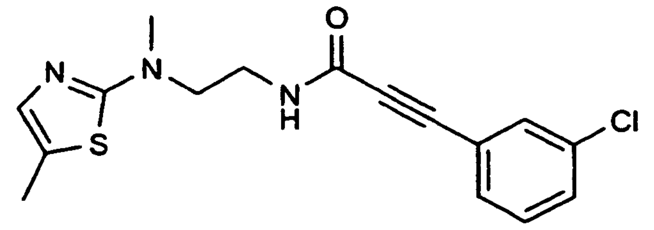

a) **Synthese von N,5-Dimethylthiazol-2-amin**

Eine Mischung aus 21.5 g (188.0 mmol) 2-Amino-5-thiazol, 16.8 ml (225.0 mmol) 37%-ige aq. Formaldehyd-Lösung, 22.43 g (188.0 mmol) Benzotriazol und Ethanol (150 ml) wurde 2 h unter Rückfluß erhitzt und anschließend 16 h bei RT gerührt. Der entstandene Niederschlag wurde abfiltriert und mit kaltem Ethanol gewaschen. Anschließend wurde der Rückstand mit MeOH (1000 ml) aufgenommen. Unter Kühlen (Eisbad) wurden 3.48 g (92 mmol) Natriumborhydrid portionsweise zugegeben. Anschließend wurde im Vakuum eingeengt. Dieser Rückstand wurde mit Chloroform aufgenommen, mit Wasser und Brine gewaschen, über $MgSO_4$ getrocknet und im Vakuum eingeengt. Durch SC (Hexan/EE 1:1) mit dem Rückstand wurden 4.0 g (31.3 mmol, 17%) N,5-Dimethylthiazol-2-amin erhalten.

**b) Synthese von $N^1$-Methyl-$N^1$-(5-methylthiazol-2-yl)ethan-1,2-diamin**

Eine Mischung aus 512 mg (4.0 mmol) N,5-Dimethylthiazol-2-amin, 352 mg (8.8 mmol, 60%ig in Mineralöl) Natriumhydrid und 2-Bromethylamine Hydrobromid mit DMF (10 ml) wurde 2 h bei RT gerührt. Anschließend wurde in Wasser gegossen und mit DCM extrahiert. Die organische Phase wurde über $MgSO_4$ getrocknet, filtriert und im Vakuum eingeengt. Durch SC (DCM/EtOH/konz. aq. $NH_4OH$-Lsg. 5:1:0.06) mit dem Rückstand wurden 232 mg (1.4 mmol, 34 %) $N^1$-Methyl-$N^1$-(5-methylthiazol-2-yl)ethan-1,2-diamin erhalten.

**c) Synthese von 3-(3-Chlorphenyl)-N-(2-(methyl(5-methylthiazol-2-yl)amino)ethyl)propiolamid**

Zu einer Lösung von 232 mg (1.35 mmol) $N^1$-Methyl-$N^1$-(5-methylthiazol-2-yl)ethan-1,2-diamin und 367 mg (2.03 mmol) (3-Chlorphenyl)propiolsäure in DCM (40 ml) wurden 2.19 g (- 2.6 mmol) PS-Carbodiimid gegeben. Anschließend wurde 16 h bei RT geschüttelt. Danach wurde das Harz abfiltriert und mit DCM und EtOH nachgespült. Das Filtrat wurde im Vakuum eingeengt und mit dem Rückstand wurde eine SC (DCE/EtOH 5:1) durchgeführt, wobei 169 mg (0.51 mmol, 37%) N-(2-(Methyl(thiazol-2-yl)amino)ethyl)-3-(3-chlor-phenyl)-propiolamid erhalten wurden. MS [MH+] 334.1

**Synthese von Beispielverbindung 31:**

**N-(2-((5-Bromthiazol-2-yl)(methyl)amino)ethyl)-3-(3-chlorphenyl)propiolamid**

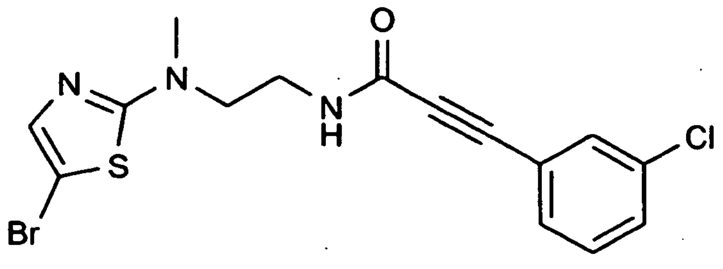

**a) Synthese von $N^1$-(5-Bromthiazol-2-yl)-$N^1$-methylethan-1,2-diamin**

Zu einer Lösung aus 1.0 g (4.1 mmol) 2,5-Dibromthiazol und 2.2 ml (24.7 mmol) N-Methyl-ethylen-diamin in Isopropanol (2.3 ml) wurden 15 mg (0.24 mmol) Kupfer und 68 mg (0.69 mmol) Kupfer(I)-chlorid gegeben. Anschließend

wurde 1 h auf 70 °C erhitzt. Nach Abkühlen auf RT wurde durch Kieselgel filtriert und das Filtrat wurde im Vakuum eingeengt. Durch SC (DCE/EtOH/ konz. aq. $NH_4OH$-Lsg. 5:1:0.06) mit dem Rückstand wurden 348 mg (1.5 mmol, 36%) $N^1$-(5-Bromthiazol-2-yl)-$N^1$-methylethan-1,2-diamin erhalten.

**b) Synthese von N-(2-((5-Bromthiazol-2-yl)(methyl)amino)ethyl)-3-(3-chlorphenyl)propiolamid**

Zu einer Lösung von 348 mg (1.47 mmol) $N^1$-(5-Bromthiazol-2-yl)-$N^1$-methylethan-1,2-diamin, 205 µl (1.47 mmol) Triethylamin und 399 mg (2.21 mmol) (3-Chlorphenyl)propiolsäure in DCM (40 ml) wurden 2.39 g (~ 2.9 mmol) PS-Carbodiimid gegeben. Anschließend wurde 16 h bei RT geschüttelt. Danach wurde das Harz abfiltriert und mit DCM und EtOH nachgespült. Das Filtrat wurde im Vakuum eingeengt und mit dem Rückstand wurde eine SC (DCE/EtOH 10: 1) durchgeführt, wobei 119 mg (0.30 mmol, 20%) N-(2-((5-Bromthiazol-2-yl)(methyl)amino)ethyl)-3-(3-chlorphenyl)propiolamid erhalten wurden. MS [MH+] 398.0

**Synthese von Beispielverbindung 35:**

**1-(3-(3-Chlorphenyl)propiolyl)-3-(methyl(thiazol-2-yl)amino)azetidin**

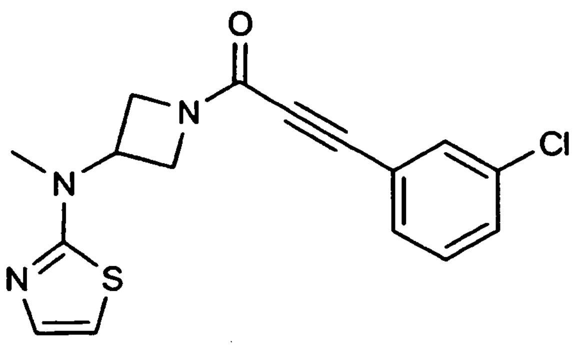

**a) Synthese von 1-Boc-3-(thiazol-2-ylamino)azetidin**

Eine Mischung aus 5.0 g (29.1 mmol) 1-Boc-3-aminoazetidin und 2.62 ml (29.1 mmol) 2-Bromthiazol wurde unter Rühren 2 h auf 140 °C. Nach Abkühlen auf RT wurden aus der Mischung 800 mg (3.14 mmol, 11 %) 1-Boc-3-(thiazol-2-ylamino)azetidin durch SC ($SiO_2$, 1. DCE 2. Chloroform) erhalten.

**b) Synthese von 1-Boc-3-(methyl(thiazol-2-yl)amino)azetidin**

Eine Suspension von 270 mg (1.05 mmol) 1-Boc-3-(thiazol-2-ylamino)azetidin in MeCN (5 ml) wurde mit 68 mg (1.2 mmol, 60%ig in Mineralöl) Natriumhydrid versetzt und 30 min bei RT gerührt. Anschließend wurden 151 µl (1.73 mmol) Iodmethan zugegeben und es wurde weitere 16 h bei RT geührt. Nach Zugabe von MeOH (200 µl) wurde im Vakuum eingeengt. Durch SC (Hexan/EE 3:2) mit dem Rückstand wurden 166 mg (0.62 mmol, 59%) 1-Boc-3-(methyl (thiazol-2-yl)amino)azetidin erhalten.

**c) Synthese von 3-(Methyl(thiazol-2-yl)amino)azetidin**

Eine Lösung von 166 mg (0.62 mmol) 1-Boc-3-(methyl(thiazol-2-yl)amino)azetidin in DCM (5 ml) wurde mit 2.5 ml TFA versetzt und 3 h bei RT gerührt. Anschließend wurde im Vakuum eingeengt. Der Rückstand wurde mit Wasser aufgenommen und mit Ether gewaschen. Die wäßrige Phase wurde mit einer 20%-igen aq. NaOH-Lsg auf pH 11 eingestellt und mit Kochsalz gesättigt. Anschließend wurde mit Chloroform extrahiert und die organische Phase wurde über $MgSO_4$ getrocknet und filtriert. Nach Entfernen der Lösungsmittel im Vakuum wurden 102 mg (0.60 mmol, 97%) 3-(Methyl(thiazol-2-yl)amino)azetidin erhalten.

**d) Synthese von 1-(3-(3-Chlorphenyl)propiolyl)-3-(methyl(thiazol-2-yl)amino)azetidin**

Zu einer Lösung von 100 mg (0.59 mmol) 3-(Methyl(thiazol-2-yl)amino)azetidin und 160 mg (0.89 mmol) (3-Chlorphenyl)propiolsäure in DCM (15 ml) wurden 0.78 g (- 0.9 mmol) PS-Carbodiimid gegeben. Anschließend wurde 16 h bei RT geschüttelt. Danach wurde das Harz abfiltriert und mit DCM und MeOH nachgespült. Das Filtrat wurde im Vakuum eingeengt und mit dem Rückstand wurde eine SC (Chloroform) durchgeführt, wobei 148 mg (0.45mmol, 76%) 1-(3-(3-Chlorphenyl)propiolyl)-3-(methyl(thiazol-2-yl)amino)azetidin erhalten wurden. MS [MH+] 332.1

**Synthese von Beispielverbindung 36:**

**3-(3-Chlorphenyl)-N-methyl-N-(1-(thiazol-2-yl)piperidin-3-yl)propiolamid**

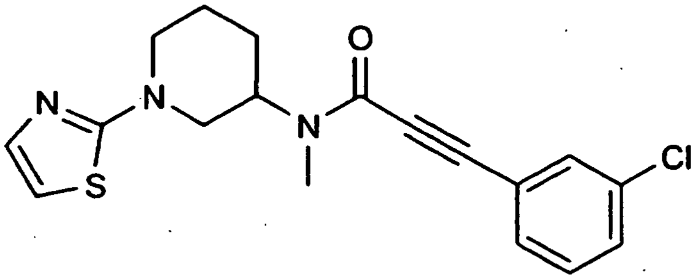

**a) Synthese von N-Methyl-1-(thiazol-2-yl)piperidin-3-amin**

Eine Mischung aus 502 mg (1.92 mmol) 1-(Thiazol-2-yl)piperidin-3-yl-methansulfonat (Synthese ist unter Beispiel 15 beschrieben) in einer 33%-igen ethanolischen Methylamin-Lsg wurde in einem Druckgefäß in der Mikrowelle (CEM Explorer, max. 300 W) für 10 min auf 140 °C erhitzt. Anschließend wurde im Vakuum eingeengt. Der Rückstand wurde mit DCM aufgenommen, mit Wasser und Brine gewaschen, über $MgSO_4$ getrocknet, filtriert und erneut im Vakuum eingeengt. Durch SC (DCE/EtOH/aq.$NH_4OH$ 5:1:0.06) dieses Rückstands wurden 276 mg (1.40 mmol, 73%) N-Methyl-1-(thiazol-2-yl)piperidin-3-amin erhalten.

**b) Synthese von 3-(3-Chlorphenyl)-N-methyl-N-(1-(thiazol-2-yl)piperidin-3-yl)propiolamid**

Zu einer Lösung von 258 mg (1.31 mmol) N-Methyl-1-(thiazol-2-yl)piperidin-3-amin und 354 mg (1.96 mmol) (3-Chlorphenyl)propiolsäure in DCM (40 ml) wurden 2.1 g (- 2.52 mmol) PS-Carbodiimid gegeben. Anschließend wurde 16 h bei RT geschüttelt. Danach wurde das Harz abfiltriert und mit DCM und MeOH nachgespült. Das Filtrat wurde im Vakuum eingeengt und mit dem Rückstand wurde eine SC (Hexan/EE 1:3) durchgeführt, wobei 243 mg (0.67mmol, 52%) 3-(3-Chlorphenyl)-N-methyl-N-(1-(thiazol-2-yl)piperidin-3-yl)propiolamid erhalten wurden. MS [MH+] 360.1

**Synthese von Beispielverbindung 41:**

**3-(3-Chlorphenyl)-N-(2-(ethyl(thiazol-2-yl)amino)ethyl)propiolamid**

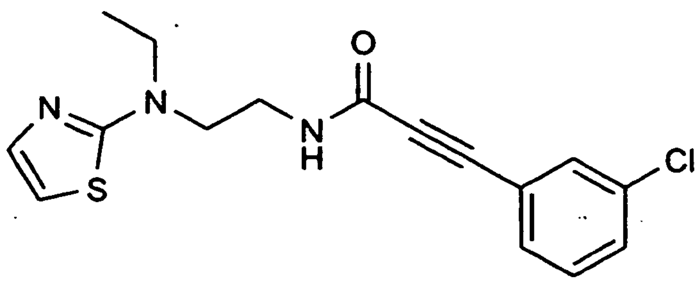

**a) Synthese von $N^1$-Ethyl-$N^1$-(thiazol-2-yl)ethan-1,2-diamin**

Zu einer Lösung aus 1.35 g (15.0 mmol) 2-Bromthiazol und 9.48 ml (90.0 mmol) N-Ethyl-ethylen-diamin in Isopropanol (7.5 ml) wurden 47 mg (0.74 mmol) Kupfer und 225 mg (2.27 mmol) Kupfer(I)-chlorid gegeben. Anschließend wurde 1 h auf 70 °C erhitzt. Nach Abkühlen auf RT wurde durch Reaktionslösung in Wasser gegossen und mit EE extrahiert. Die organische Phase wurde über $MgSO_4$ getrocknet, filtriert und im Vakuum eingeengt. Durch zweimalige SC (i. DCE/EtOH 5:1; ii. DCE/EtOH/ konz. aq. $NH_4OH$-Lsg. 5:1:0.06) mit dem Rückstand wurden 200 mg (1.2 mmol, 8%) $N^1$-Ethyl-$N^1$-(thiazol-2-yl)ethan-1,2-diamin erhalten.

**b) Synthese von 3-(3-Chlorphenyl)-N-(2-(ethyl(thiazol-2-yl)amino)ethyl)-propiolamid**

Zu einer Lösung von 200 mg (1.17 mmol) $N^1$-Ethyl-$N^1$-(thiazol-2-yl)ethan-1,2-diamin und 316 mg (1.75 mmol) (3-Chlorphenyl)propiolsäure in DCM (30 ml) wurden 1.9 g (~ 2.28 mmol) PS-Carbodiimid gegeben. Anschließend wurde 1 h bei RT geschüttelt. Danach wurde das Harz abfiltriert und mit DCM und MeOH nachgespült. Das Filtrat wurde im Vakuum eingeengt und mit dem Rückstand wurde eine SC (Chloroform) durchgeführt, wobei 181 mg (0.54 mmol, 46%) 3-(3-Chlorphenyl)-N-(2-(ethyl(thiazol-2-yl)amino)ethyl)-propiolamid erhalten wurden. MS [MH+] 334.1

**Synthese von Beispielverbindung 43:**

**1-(3-(3-Chlorphenyl)propiolyl)-3-(methyl(5-fluor-thiazol-2-yl)amino)azetidin**

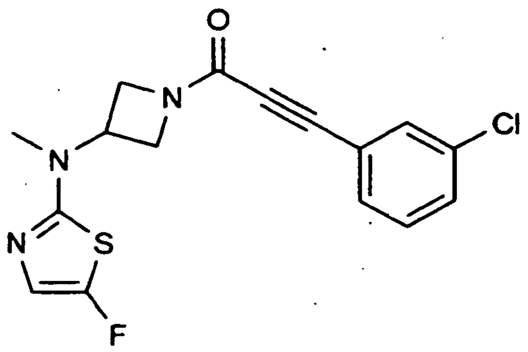

**a) Synthese von 1-Boc-3-(methyl(5-fluor-thiazol-2-yl)amino)azetidin**

Zu einer Lösung von 1.72 g (6.4 mmol) 1-Boc-3-(methyl(thiazol-2-yl)amino)azetidin (Synthese ist unter Beispiel 35 beschrieben) in THF (24 ml) wurden bei -50 °C 7.5 ml (12.8 mmol, 1.7 M in Hexan) tert.-Butyllithium innerhalb 30 min zugetropft. Nach 30 min Rühren bei -50 °C wurde eine Lösung von 2.1 g (6.7 mmol) N-Fluorbenzol-sulfonimid in THF (6 ml) innerhalb 30 min zu getropft. Nachdem sich die Reaktionslösung auf 10 °C erwärmt hatte, wurde eine 10%-ige aq. Ammoniumchorid-Lsg. zugegeben und weitere 15 min bei RT gerührt. Anschließend wurden die Phasen getrennt, und die wäßrige Phase wurde mehrmals mit Ether extrahiert. Die gesammelten organischen Phasen wurden über $MgSO_4$ getrocknet, filtriert und im Vakuum eingeengt. Mit dem Rückstand wurde eine SC (Hexan/EE 3:2) durchgeführt, wobei 170 mg (0.59 mmol, 9%) 1-Boc-3-(methyl(5-fluor thiazol-2-yl)amino)azetidin erhalten wurden.

**b) Synthese von 3-(Methyl(5-fluor-thiazol-2-yl)amino)azetidin Trifluoracetat**

Eine Lösung von 170 mg (0.59 mmol) 1-Boc-3-(methyl(5-fluor-thiazol-2-yl)amino)-azetidin in DCM (5 ml) wurde mit 2.5 ml TFA versetzt und 1 h bei RT gerührt. Anschließend wurde im Vakuum eingeengt, wobei 145 mg (0.48 mmol, 82%) 3-(Methyl(5-fluor-thiazol-2-yl)amino)azetidin Trifluoracetat erhalten wurde, die ohne Aufreinigung weiter umgesetzt wurden.

**c) Synthese von 1-(3-(3-Chlorphenyl)propiolyl)-3-(methyl(5-fluor-thiazol-2-yl)amino)azetidin**

Zu einer Lösung von 135 mg (0.45 mmol) 3-(Methyl(5-fluor-thiazol-2-yl)amino)azetidin Trifluoracetat, 69 µl (0.5 mmol) Triethylamin und 121 mg (0.67 mmol) (3-Chlorphenyl)propiolsäure in DCM (15 ml) wurden 0.76 g (~ 0.9 mmol) PS-Carbodiimid gegeben. Anschließend wurde 1 h bei RT geschüttelt. Danach wurde das Harz abfiltriert und mit DCM und MeOH nachgespült. Das Filtrat wurde im Vakuum eingeengt und mit dem Rückstand wurde eine SC (Hexan/EE 3: 2) durchgeführt, wobei 60 mg (0.17 mmol, 38%) 1-(3-(3-Chlorphenyl)propiolyl)-3-(methyl(5-fluor-thiazol-2-yl)amino)azetidin erhalten wurden. MS [MH+] 350.0

**Synthese von Beispielverbindung 44:**

**1-(3-(3-Chlorphenyl)propiolyl)-3-(methyl(5-fluor-thiazol-2-yl)amino)pyrrolidin**

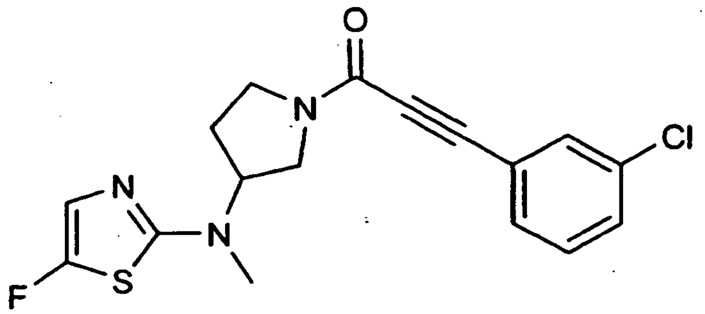

**a) Synthese von tert-Butyl-3-(methyl(thiazol-2-yi)amino)pyrrolidin-1-carboxylat**

Eine Lösung von 8.73 g (32.4 mmol) tert-Butyl-3-(thiazol-2-ylamino)pyrrolidin-1-carboxylat (Synthese ist unter Beispiel 11 beschrieben) in MeCN (120 ml) wurde mit 1.56 g (38.9 mmol, 60%ig in Mineralöl) Natriumhydrid versetzt und 30 min bei RT gerührt. Anschließend wurden 4.04 ml (64.8 mmol) lodmethan zugegeben und es wurde weitere 3 h bei RT gerührt. Nach Zugabe einer konz. aq. $NH_4OH$-Lsg (30 ml) wurde die Phasen getrennt und die wäßrige Phase wurde mit EE extrahiert. Die gesammelten organischen Phasen wurden über $MgSO_4$ getrocknet, filtriert und im Vakuum eingeengt. Durch SC (Chloroform/EE 20:1) mit dem Rückstand wurden 3.16 g (11.2 mmol, 34%) tert-Butyl-3-(methyl (thiazol-2-yl)amino)pyrrolidin-1-carboxylat erhalten.

**b) Synthese von tert-Butyl 3-((5-fluorthiazol-2-yl)(methyl)amino)pyrrolidin-1-carboxylat**

Zu einer Lösung von 1.70 g (6.0 mmol) tert-Butyl-3-(methyl(thiazol-2-yl)amino)-pyrrolidin-1-carboxylat in THF (24 ml) wurden bei -50 °C 5.3 ml (7.8 mmol, 1.7 M in Hexan) tert.-Butyllithium innerhalb 30 min zu getropft. Nach 30 min Rühren bei -50 °C wurde eine Lösung von 2.0 g (6.3 mmol) N-Fluorbenzol-sulfonimid in THF (6 ml) innerhalb 20 min zu getropft. Nachdem sich die Reaktionslösung auf 10 °C erwärmt hatte, wurde eine 10%-igen aq. Ammoniumchorid-Lsg zugegeben und weitere 15 min bei RT gerührt. Anschließend wurden die Phasen getrennt, und die wäßrige Phase wurde mehrmals mit Ether extrahiert. Die gesammelten organischen Phasen wurden über $MgSO_4$ getrocknet, filtriert und im Vakuum eingeengt. Mit dem Rückstand wurde eine SC (Hexan/EE 4:1) durchgeführt, wobei 590 mg (2.0 mmol, 33%) tert-Butyl-3-((5-fluorthiazol-2-yl)(methyl)amino)pyrrolidin-1-carboxylat erhalten wurden.

**c) Synthese von 5-Fluor-N-methyl-N-(pyrrolidin-3-yl)thiazol-2-amin Trifluoracetat**

Eine Lösung von 590 mg (1.96 mmol) tert-Butyl-3-((5-fluorthiazol-2-yl)(methyl)amino)-pyrrolidin-1-carboxylat in DCM (10 ml) wurde mit 5 ml TFA versetzt und 1 h bei RT gerührt. Anschließend wurde im Vakuum eingeengt, mit MeOH aufgenommen und erneut eingeengt, wobei 611 mg (1.94 mmol, 99 %) 5-Fluor-N-methyl-N-(pyrrolidin-3-yl)thiazol-2-amin Trifluoracetat erhalten wurde, die ohne Aufreinigung weiter umgesetzt wurden.

**d) Synthese von 1-(3-(3-Chlorphenyl)propiolyl)-3-(methyl(5-fluor-thiazol-2-yl)amino)pyrrolidin**

Eine Lösung von 611 mg (1.94 mmol) 5-Fluor-N-methyl-N-(pyrrolidin-3-yl)thiazol-2-amin Trifluoracetat in DCM (50 ml) wurde mit Brine (10 ml) und einer 5%-igen aq. NaOH-Lsg versetzt und 1 h bei RT gerührt. Anschließend wurden

die Phasen getrennt und die organische Phase wurde über $MgSO_4$ getrocknet und filtriert. Zum Filtrat wurden 530 mg (2.94 mmol) (3-Chlorphenyl)propiolsäure und 3.39 g (~ 4.1 mmol) PS-Carbodiimid gegeben. Anschließend wurde 2 h bei RT geschüttelt. Danach wurde das Harz abfiltriert und mit DCM und MeOH nachgespült. Das Filtrat wurde im Vakuum eingeengt und mit dem Rückstand wurde eine SC (Chloroform/EE 3:2) durchgeführt, wobei 148 mg (0.41 mmol, 21%) 1-(3-(3-Chlorphenyl)propiolyl)-3-(methyl(5-tluor-thiazol-2-yl)amino)pyrrolidin erhalten wurden. MS [MH+] 364.1

**Synthese von Beispielverbindung 126:**

**3-(3-Chlorphenyl)-N-(2-((5-fluorthiazol-2-yl)(methyl)amino)ethyl)propiolamid**

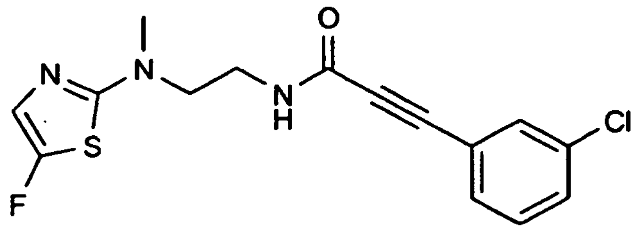

Zu einer Lösung von 540 mg (3.4 mmol) $N^1$-Methyl-$N^1$-(thiazol-2-yl)ethan-1,2-diamin (Synthese ist unter Beispiel 19 Abschnitt a) beschrieben) in THF (20 ml) wurden 2.9 ml (7.3 mmol, 2.5 M in Hexan) n-BuLi bei -78 °C zugetropft. Anschließend wurde 15 min bei -40 °C nachgerührt und danach erneut auf -78 °C abgekühlt. Bei dieser Temperatur wurde eine Lösung von 754 mg (3.5 mmol) 1,2-Bis-(chlordimethylsilyl)ethan in THF (12 ml) langsam zugegeben. Nach Erwärmen auf RT wurde die Reaktionslösung noch 45 min gerührt, anschließend auf -50 °C abgekühlt und tropfenweise mit 3.0 ml (5.1 mmol, 1.7 M in Hexan) t-BuLi versetzt. Nach weiteren 30 min Rühren bei -50 °C wurde bei dieser Temperatur eine Lösung von 1.1 g (3.5 mmol) N-Fluorbenzol-sulfonimid in THF (12 ml) zugetropft. Nach vollendeter Zugabe wurde auf RT erwärmt, 60 min nachgerührt und mit einer ges. Ammoniumchlorid-Lsg (20 ml) gequencht. Anschließend wurde mit einer 6N Salzsäure pH 2 eingestellt und 20 min bei RT gerührt. Die Phasen wurden getrennt und die wässrige Phase wurde mit einer aq. $NH_3$-Lsg basich gestellt und mit DCM extrahiert.

Diese organische Phase wurde über $MgSO_4$ getrocknet und filtriert. Zum Filtrat wurden 921 mg (5.1 mmol) (3-Chlorphenyl)propiolsäure und 5.54 g PS-Carbodiimid zugegeben und die Reaktionslösung wurde 60 min bei RT gerührt. Danach wurde abfiltriert und das Filtrat wurde im Vakuum eingeengt. Mit dem Rückstand wurde eine SC (DCE/EtOH 100: 1) durchgeführt, wobei 108 mg 3-(3-Chlorphenyl)-N-(2-((5-fluorthiazol-2-yl)(methyl)amino)ethyl)propiolamid erhalten wurden. MS [MH+] 338.0.

Die Synthese der **Beispielverbindungen 32** 3-(3-Chlorphenyl)-N-(1-(thiazol-2-yl)piperidin-3-yl)propiolamid MS [MH+] 346.1, **33** N-(2-((4-Bromthiazol-2-yl)(methyl)amino)ethyl)-3-(3-chlorphenyl)propiolamid MS [MH+] 398.0, **34** Methyl 2-((2-(3-(3-chlorphenyl)propiolamido)ethyl)(methyl)amino)thiazol-5-carboxylat MS [MH+] 378.1, **37** 3-(3-Chlorphenyl)-N-(2-((4-chlorthiazol-2-yl)(methyl)amino)ethyl)propiolamid MS [MH+] 354.0, **38** 3-(3-Chlorphenyl)-N-(2-((5-chlorthiazol-2-yl)(methyl)amino)ethyl)propiolamid MS [MH+] 354.0 und **42** 3-(3-Chlorphenyl)-N-(2-((5-cyanothiazol-2-yl)(methyl)amino)ethyl)propiolamid MS [MH+] 345.0 erfolgte nach dem vorstehend bei der Beispielverbindung 31 beschriebenen Verfahren.

Die Synthese der **Beispielverbindung 39** 3-(3-Chlorphenyl)-N-(1-(thiazol-2-yl)azetidin-3-yl)propiolamid MS [MH+] 318.0 erfolgte nach dem vorstehend bei der Beispielverbindung 1 beschriebenen Verfahren.

Die **Beispielverbindung 40** 3-(3-Chlorphenyl)-N-methyl-N-(1-(thiazol-2-yl)azetidin-3-yl)propiolamid MS [MH+] 332.1 wurde aus wie vorstehend nach dem bei Beispielverbindung 2 beschriebenen Verfahren hergestellt.

**Allgemeine Synthesevorschrift für die Umsetzung von primären und sekundären Aminen der allgemeinen Formel IV mit aromatisch substituierten Propiolsäuren der allgemeinen Formel $R^9$-C≡C-C(=O)-OH**

Zu einer Lösung der jeweiligen aromatisch substituierten Propiolsäure der allgemeinen Formel $R^9$-C≡C-C(=O)-OH (100 μmol) in DCM (2 ml) wurde eine Lösung von CDI (105μ mol, 1.05 Äquivalente) in DCM (1.05 ml) gegeben. Die Reaktionslösung wurde 1 h bei 20 °C gerührt und anschließend mit einer Lösung des jeweiligen primären oder sekundären Amins der allgemeinen Formel IV (100 μmol, 1.0 Äquivalente) in DCM (1 ml) versetzt. Danach wurde weitere 16 h bei RT gerührt. Im Anschluß wurde die Reaktionsmischung mit Wasser (3 ml) versetzt und die Phasen wurden getrennt. Die organische Phase wurde mit Wasser (3 ml) und mit Brine (3 ml) gewaschen, über $MgSO_4$ getrocknet und filtriert. Nach Entfernen des Lösungsmittels im Vakuum wurde die jeweilige Zielverbindung aus dem Rückstand mittels

präparativer HPLC isoliert.

Die Synthese der **Beispiele 45 bis 123** (Tabelle 1) erfolgte nach dem beschriebenen allgemeinen Verfahren zur Umsetzung von primären und sekundären Aminen mit aromatisch substituierten Propiolsäuren. Die Analytik erfolgte über HPLC-MS, wobei die Reinheit in allen Fällen bei > 85% lag. Dem Fachmann ist dabei ersichtlich, welche Ausgangsverbindungen und Zwischenprodukte jeweils eingesetzt wurden.

Tabelle 1.

| Bsp. | Name | [MH+] |
|---|---|---|
| [45] | N-(1-(4-Methylthiazol-2-yl)pyrrolidin-3-yl)-3-phenylpropiolamid | 312,1 |
| [46] | 3-(3-Methoxyphenyl)-N-(1-(4-methylthiazol-2-yl)pyrrolidin-3-yl)propiolamid | 342,1 |
| [47] | 3-(2-Methoxyphenyl)-N-(4-methylthiazol-2-yl)pyrrolidin-3-yl)propiolamid | 342,1 |
| [48] | 3-(4-Methoxyphenyl)-N-(1-(4-methylthiazol-2-yl)pyrrolidin-3-yl)propiolamid | 342,1 |
| [49] | 3-(4-Fluorphenyl)-N-(1-(4-methylthiazol-2-yl)pyrrolidin-3-yl)propiolamid | 330,1 |
| [50] | N-(1-(4-Methylthiazol-2-yl)pyrrolidin-3-yl)-3-p-tolylpropiolamid | 326,1 |
| [51] | N-(1-(4,5-Dimethylthiazol-2-yl)pyrrolidin-3-yl)-3-phenylpropiolamid | 326,1 |
| [52] | N-(1-(4,5-Dimethylthiazol-2-yl)pyrrolidin-3-yl)-3-(4-methoxyphenyl)propiolamid | 356,1 |
| [53] | N-(1-(4,5-Dimethylthiazol-2-yl)pyrrolidin-3-yl)-3-(4-fluorphenyl)propiolamid | 344,1 |
| [54] | N-(1-(4,5-Dimethylthiazol-2-yl)pyrrolidin-3-yl)-3-(2-methoxyphenyl)propiolamid | 356,1 |
| [55] | N-(1-(4-tert-Butylthiazol-2-yl)pyrrolidin-3-yl)-3-(4-methoxyphenyl)propiolamid | 384,2 |
| [56] | N-(1-(4-tert-Butylthiazol-2-yl)pyrrolidin-3-yl)-3-p-tolylpropiolamid | 368,2 |
| [57] | N-(1-(4-tert-Butylthiazol-2-yl)pyrrolidin-3-yl)-3-(4-fluorphenyl)propiolamid | 372,1 |
| [58] | N-(1-(4-tert-Butylthiazol-2-yl)pyrrolidin-3-yl)-3-o-tolylpropiolamid | 368,2 |
| [59] | N-(1-(4-tert-Butylthiazol-2-yl)pyrrolidin-3-yl)-3-(3-fluorphenyl)propiolamid | 372,1 |
| [60] | N-(1-(4-tert-Butylthiazol-2-yl)pyrrolidin-3-yl)-3-phenylpropiolamid | 354,2 |
| [61] | N-(1-(4-tert-Butylthiazol-2-yl)pyrrolidin-3-yl)-3-(2-fluorphenyl)propiolamid | 372,1 |
| [62] | N-(1-(4-tert-Butylthiazol-2-yl)pyrrolidin-3-yl)-3-(3-methoxyphenyl)propiolamid | 384,2 |
| [63] | N-(1-(4-tert-Butylthiazol-2-yl)pyrrolidin-3-yl)-3-(3-fluor-4-methylphenyl)propiolamid | 386,2 |
| [64] | N-(1-(4-tert-Butylthiazol-2-yl)pyrrolidin-3-yl)-3-(2-methoxyphenyl)propiolamid | 384,2 |
| [65] | N-(1-(4-Methylthiazol-2-yl)pyrrolidin-3-yl)-3-(4-(trifluormethyl)phenyl)propiolamid | 380,1 |
| [66] | N-(1-(4-Methylthiazol-2-yl)pyrrolidin-3-yl)-3-o-tolylpropiolamid | 326,1 |
| [67] | 3-(3-Fluorphenyl)-N-(1-(4-methylthiazol-2-yl)pyrrolidin-3-yl)propiolamid | 330,1 |
| [68] | 3-(3-Fluor-4-methylphenyl)-N-(1-(4-methylthiazol-2-yl)pyrrolidin-3-yl)propiolamid | 344,1 |
| [69] | 3-(2,4-Difluorphenyl)-N-(1-(4-methylthiazol-2-yl)pyrrolidin-3-yl)propiolamid | 348,1 |
| [70] | 3-(2-Fluorphenyl)-N-(1-(4-methylthiazol-2-yl)pyrrolidin-3-yl)propiolamid | 330,1 |
| [71] | N-(1-(4-Methylthiazol-2-yl)pyrrolidin-3-yl)-3-(3-(trifluormethyl)phenyl)propiolamid | 380,1 |
| [72] | N-(1-(4-Methylthiazol-2-yl)pyrrolidin-3-yl)-3-m-tolylpropiolamid | 326,1 |
| [73] | N-(1-(5-Methylthiazol-2-yl)pyrrolidin-3-yl)-3-p-totylpropiolamid | 326,1 |
| [74] | N-(1-(5-Methylthiazol-2-yl)pyrrolidin-3-yl)-3-m-tolylpropiolamid | 326,1 |
| [75] | 3-(2-Methoxyphenyl)-N-(1-(5-methylthiazol-2-yl)pyrrolidin-3-yl)propiolamid | 342,1 |
| [76] | N-(1-(5-Methylthiazol-2-yl)pyrrolidin-3-yl)-3-phenylpropiolamid | 312,1 |
| [77] | 3-(2-Fluorphenyl)-N-(1-(5-methylthiazol-2-yl)pyrrolidin-3-yl)propiolamid | 330,1 |
| [78] | 3-(3-Methoxyphenyl)-N-(1(5-methylthiazol-2-yl)pyrrolidin-3-yl)propiolamid | 342,1 |

(fortgesetzt)

| Bsp. | Name | [MH+] |
|---|---|---|
| [79] | N-(1-(4,5-Dimethylthiazol-2-yl)pyrrolidin-3-yl)-3-(3-fluor-4-methylphenyl)propiolamid | 358,1 |
| [80] | N-(1-(4,5-Dimethylthiazol-2-yl)pyrrolidin-3-yl)-3-o-tolylpropiolamid | 340,1 |
| [81] | 3-(3-Fluorphenyl)-N-(1-(5-methylthiazol-2-yl)pyrrolidin-3-yl)propiolamid | 330,1 |
| [82] | N-(1-(4,5-Dimethylthiazol-2-yl)pyrrolidin-3-yl)-3-m-tolylpropiolamid | 340,1 |
| [83] | N-(1-(5-Methylthiazol-2-yl)pynrolidin-3-yl)-3-o-tolylpropiolamid | 326,1 |
| [84] | N-(1-(4,5-Dimethylthiazol-2-yl)pyrrolidin-3-yl)-3-p-tolylpropiolamid | 340,1 |
| [85] | N-(1-(4,5-Dimethylthiazol-2-yl)pyrrolidin-3-yl)-3-(3-(trifluormethyl)phenyl)propiolamid | 394,1 |
| [86] | 3-(4-Methoxyphenyl)-N-(1-(5-methylthiazol-2-yl)pyrrolidin-3-yl)propiolamid | 342,1 |
| [87] | N-(1-(5-Methylthiazol-2-yl)pyrrolidin-3-yl)-3-(3-(trifluormethyl)phenyl)propiolamid, | 380,1 |
| [88] | N-(1-(4,5-Dimethylthiazol-2-yl)pyrrolidin-3-yl)-3-(4-(trifluormethyl)phenyl)propiolamid | 394,1 |
| [89] | 3-(4-Fluorphenyl)-N-(1-(5-methylthiazol-2-yl)pyrrolidin-3-yl)propiolamid | 330,1 |
| [90] | N-(1-(4,5-Dimethylthiazol-2-yl)pyrrolidin-3-yl)-3-(3-methoxyphenyl)propiolamid | 356,1 |
| [91] | 3-(2,4-Difluorphenyl)-N-(1-(4,5-dimethylthiazol-2-yl)pyrrolidin-3-yl)propiolamid | 362,1 |
| [92] | N-(1-(4,5-Dimethylthiazol-2-yl)pyrrolidin-3-yl)-3-(2-fluorphenyl)propiolamid | 344,1 |
| [93] | N-(1-(5-Methylthiazol-2-yl)pyrrolidin-3-yl)-3-(4-(trifluomnethyl)phenyl)propiolamid | 380,1 |
| [94] | 3-(3-Fluor-4-methylphenyl)-N-(1-(5-methylthiazol-2-yl)pyrrolidin-3-yl)propiolamid | 344,1 |
| [95] | 3-(2,4-Difluorphenyl)-N-(1-(5-methylthiazol-2-yl)pyrrolidin-3-yl)propiolamid | 348,1 |
| [96] | N-(1-(4-tert-Butylthiazol-2-yl)pyrrolidin-3-yl)-3-(2,4-difluorphenyl)propiolamid | 390,1 |
| [97] | N-(1-(4-tert-Butylthiazol-2-yl)pyrrolidin-3-yl)-3-(4-(trifluormethyl)phenyl)propiolamid | 422,1 |
| [98] | N-(1-(4-tert-Butylthiazol-2-yl)pyrrolidin-3-yl)-3-(3-(trifluormethyl)phenyl)propiolamid | 422,1 |
| [99] | N-(1-(4-tert-Butylthiazo)-2-yl)pyrrolidin-3-yl)-3-m-tolylpropiolamid | 368,2 |
| [100] | N-(1-(4-tert-Butylthiazol-2-yl)pyrrolidin-3-yl)-3-(3-chlorphenyl)propiolamid | 388,1 |
| [101] | N-(1-(4-tert-Butylthiazol-2-yl)pyrrolidin-3-yl)-3-(thiophen-2-yl)propiolamid | 360,1 |
| [102] | N-(1-(4-tert-Butylthiazol-2-yl)pyrrolidin-3-yl)-3-(2,4-dichlorphenyl)propiolamid, | 422,1 |
| [103] | N-(1-(4-tert-Butylthiazol-2-yl)pyrrolidin-3-yl)-3-(2-chlor-5-(trifluormethyl)phenyl)propiolamid | 456,1 |
| [104] | N-(1-(4-tert-Butylthiazol-2-yl)pyrrolidin-3-yl)-3-(3,5-dichlorphenyl)propiolamid | 422,1 |
| [105] | 3-(3-Chlorphenyl)-N-(4-methylthiazol-2-yl)pyrrolidin-3-yl)propiolamid | 346,1 |
| [106] | N-(1-(4-Methylthiazol-2-yl)pyrrolidin-3-yl)-3-(thiophen-2-yl)propiolamid | 318,1 |
| [107] | 3-(2,4-Dichlorphenyl)-N-(1-(4-methylthiazol-2-yl)pyrrolidin-3-yl)propiolamid | 380,0 |
| [108] | 3-(2-Chlor-5-(trifluormethyl)phenyl)-N-(1-(4-methylthiazol-2-yl)pyrrolidin-3-yl)propiolamid | 414,1 |
| [109] | 3-(3,5-Dichlorphenyl)-N-(1-(4-methylthiazol-2-yl)pyrrolidin-3-yl)propiolamid | 380,0 |
| [110] | 3-(3-Chlorphenyl)-N-(1-(4,5-dimethylthiazol-2-yl)pyrrolidin-3-yl)propiolamid | 360,1 |
| [111] | 3-(2,4-Dichlorphenyl)-N-(1-(4,5-dimethylthiazol-2-y1)pyrrolidin-3-yl)propiolamid | 394,0 |
| [112] | 3-(3-Chlorphenyl)-N-(1-(5-methylthiazol-2-yl)pyrrolidin-3-yl)propiolamid | 346,1 |
| [113] | N-(1-(5-Methylthiazot-2-yt)pyrrolidin-3-yl)-3-(thiophen-2-yl)propiolamid | 318,1 |
| [114] | 3-(2,4-Dichlorphenyl)-N-(1-(5-methylthiazol-2-yl)pyrrolidin-3-yl)propiolamid | 380,0 |
| [115] | 3-(2-Chlor-5-(trifluormethyl)phenyl)-N-(1-(5-methylthiazol-2-yl)pyrrolidin-3-yl)propiolamid | 414,1 |
| [116] | 3-(3,5-Dichlorphenyl)-N-(1-(5-methylthiazol-2-yl)pyrrolidin-3-yl)propiolamid | 380,0 |

(fortgesetzt)

| Bsp. | Name | [MH+] |
|---|---|---|
| [117] | N-(1-(4,5-Dimethylthiazol-2-yl)pyrrolidin-3-yl)-3-(thiophen-2-yl)propiolamid | 332,1 |
| [118] | 3-(2-Chlor-5-(trifluormethyl)phenyl)-N-(1-(4,5-dimethylthiazol-2-yl)pyrrolidin-3-yl)propiolamid | 428,1 |
| [119] | N-(1-(5-Methylthiazol-2-yl)pyrrolidin-3-yl)-3-(pyridin-2-yl)propiolamid | 313.1 |
| [120] | N-(1 -(5-Methylthiazol-2-yl)pyrrolidin-3-yl)-3-(pyridin-3-yl)propiolamid | 313,1 |
| [121] | N-(1-(5-Methylthiazol-2-yl)pyrrolidin-3-yl)-3-(pyridin-4-yl)propiolamid | 313,1 |
| [122] | N-(1-(4-tert-Butylthiazol-2-yl)pyrrolidin-3-yl)-3-(pyridin-2-yl)propiolamid | 355,2 |
| [123] | N-(1-(4-tert-Butylthiazol-2-yl)pyrrolidin-3-yl)-3-(pyridin-3-yl)propiolamid | 355,2 |
| [126] | 3-(3-Chlorphenyl)-N-(2-((5-fluorthiazol-2-yl)(methyl)amino)ethyl)propiolamid | 338,0 |

**Pharmakologische Daten:**

1. Die Affinität der erfindungsgemäßen substituierten Thiazole der allgemeinen Formel I für den mGluR5-Rezeptor wurde wie vorstehend bei den pharmakologischen Methoden I. und II. beschrieben bestimmt.

Die erfindungsgemäßen substituierten Thiazole zeigen eine ausgezeichnete Affinität für den mGluR5-Rezeptor.

In der nachfolgenden Tabelle 2 sind die pharmakologischen Daten für die substituierten Thiazole wiedergegeben:

Tabelle 2

| Bsp. | %-Hemmung mGluR5 Rezeptor (Schwein) [$^3$H]-MPEP-Bindung bei 1 μM | %-Hemmung mGluR5 Rezeptor (Schwein) [$^3$H]-MPEP-Bindung bei 10 μM | $IC_{50}$ mGluR5 Rezeptor (Schwein) [$^3$H]-MPEP-Bindung [μM] | $K_i$ mGluR5 Rezeptor (Mensch) $Ca^{2+}$-Influx [nm] |
|---|---|---|---|---|
| **1** | | 66 | | |
| **2** | | | 1,3200 | |
| **3** | | | 0,5900 | |
| **4** | | | 0,0570 | 5,2 |
| **5** | | | 1,5800 | |
| **6** | | 57 | | |
| **7** | | | 0,0260 | 4,8 |
| **8** | | | 10,2100 | |
| **9** | | | 0,0380 | 1,0 |
| **10** | | | 1,8600 | |
| **11** | | | 2,1800 | |
| **12** | | 41 | | |
| **13** | | | 0,0870 | 20,8 |
| **14** | | | 4,9700 | |
| **15** | | | 0,0860 | 14,4 |
| **16** | | | 0,1400 | |
| **17** | | | 1,2500 | |
| **18** | | | 0,0200 | 4,9 |
| **19** | | | 0,0019 | 0,4 |
| **20** | | | 0,2800 | |

(fortgesetzt)

| Bsp. | %-Hemmung mGluR5 Rezeptor (Schwein) [$^3$H]-MPEP-Bindung bei 1 μM | %-Hemmung mGluR5 Rezeptor (Schwein) [$^3$H]-MPEP-Bindung bei 10 μM | $IC_{50}$ mGluR5 Rezeptor (Schwein) [$^3$H]-MPEP-Bindung [μM] | $K_i$ mGluR5 Rezeptor (Mensch) $Ca^{2+}$-Influx [nm] |
|---|---|---|---|---|
| **21** | | | 0,0083 | 0,8 |
| **22** | | | 0,3500 | |
| **23** | | | 0,0056 | |
| **24** | | | 2,7800 | |
| **25** | | | 0,1500 | |
| **26** | | | 0,0860 | 3,6 |
| **27** | | 19 | | |
| **28** | | | 1,1500 | |
| **29** | | | 0,0270 | |
| **30** | | | 0,0770 | |
| **3** | | | 0,0130 | |
| **32** | | | 0,0050 | 4,0 |
| **33** | | | 0,0130 | 10,0 |
| **34** | | | 0,0200 | |
| **35** | | | 0,0022 | 6,5 |
| **36** | | | 0,0220 | |
| **37** | | | 0,0095 | |
| **38** | | | 0,1600 | |
| **39** | | | 0,3900 | |
| **40** | | | 0,0170 | |
| **41** | | | 0,0041 | |
| **42** | | | 0,2200 | |
| **43** | | | 0,0740 | |
| **44** | | | 0,0710 | |
| **45** | 59 | | | |
| **46** | 48 | | | |
| **51** | 48 | | | |
| **55** | 26 | | | |
| **56** | 37 | | | |
| **57** | 26 | | | |
| **59** | 34 | | | |
| **60** | 30 | | | |
| **61** | 28 | | | |
| **67** | 76 | | | |
| **70** | 50 | | | |
| **71** | 59 | | | |

(fortgesetzt)

| Bsp. | %-Hemmung mGluR5 Rezeptor (Schwein) [$^3$H]-MPEP-Bindung bei 1 µM | %-Hemmung mGluR5 Rezeptor (Schwein) [$^3$H]-MPEP-Bindung bei 10 µM | $IC_{50}$ mGluR5 Rezeptor (Schwein) [$^3$H]-MPEP-Bindung [µM] | $K_i$ mGluR5 Rezeptor (Mensch) $Ca^{2+}$-Influx [nm] |
|---|---|---|---|---|
| **72** | 87 | | | |
| **73** | 18 | | | |
| **74** | 89 | | | |
| **76** | 56 | | | |
| **77** | 21 | | | |
| **78** | 26 | | | |
| **81** | 57 | | | |
| **82** | 81 | | | |
| **85** | 41 | | | |
| **87** | 23 | | | |
| **90** | 34 | | | |
| **99** | 46 | | | |
| **100** | 38 | | | |
| **105** | | 83 | | |
| **106** | | 38 | | |
| **110** | | 63 | | |
| **112** | 24 | | | |
| **113** | | 22 | | |
| **119** | | 20 | | |
| **123** | 20 | | | |
| **126** | | | 0,0160 | |

2. Die erfindungsgemäßen substituierten Thiazole der allgemeinen Formel I zeigen ebenfalls eine ausgezeichnete Wirkung im Formalin-Test an der Ratte, wie an Beispiel 23 N-(2-Methyl(thiazol-2-yl)amino)ethyl)-3-(3-chlor-phenyl)-propiolamid Hydrochlorid mit einem $ED_{50}$-Wert von 1.0 mg/kg nach i.v. Applikation, gezeigt. Bei einer p.o. Applikation von 10 mg/kg zeigt diese Verbindung im Formalin-Test eine Hemmung von 38 %.

## Patentansprüche

**1.** Substituierte Thiazole der allgemeinen Formel I,

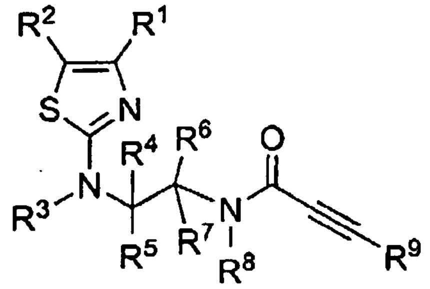

I,

worin

$R^1$ und $R^2$, unabhängig voneinander, jeweils für H; F; Cl; Br; I; $-NO_2$; -CN;$-NH_2$; -OH; -SH; -C(=O)-OH; -C(=O)-H; -NH-C(=O)-H; $-NH-R^{55}$; $-NR^{56}R^{57}$; - $C(=O)-R^{58}$; $-C(=O)-O-R^{59}$; $-O-C(=O)-R^{60}$; $-NH-C(=O)-R^{61}$; $-NR^{62}-C(=O)-R^{63}$; - $C(=O)-NH_2$; $-C(=O)-NH-R^{64}$; $-C(=O)-NR^{65}R^{66}$; $-O-R^{67}$; $-S-R^{68}$; $-S(=O)-R^{89}$;$-S(=O)_2-R^{70}$; $-NH-C(=O)-NH-R^{71}$; $-NH-C(=S)-NH-R^{72}$; $-NH-S(=O)_2-R^{73}$; $-NR^{74}-S(=O)_2-R^{75}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl;
unsubstituiertes oder wenigstens einfach substituiertes Heterocycloalkyl oder Heterocycloalkenyl; unsubstituiertes oder wenigstens einfach substituiertes - (Alkylen)-Cycloalkyl, -(Alkenylen)-Cycloalkyl, -(Alkinylen)-Cycloalkyl, - (Alkylen)-Cycloalkenyl, -(Alkenylen)-Cycloalkenyl oder -(Alkinylen)-Cycloalkenyl; unsubstituiertes oder wenigstens einfach substituiertes - (Heteroalkylen)-Cycloalkyl, -(Heteroalkenylen)-Cycloalkyl, -(Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl;
unsubstituiertes oder wenigstens einfach substituiertes -(Heteroalkylen)-Heterocycloalkyl, -(Heteroalkenylen)-Heterocycloalkyl, -(Heteroalkylen)-Heterocycloalkenyl oder -(Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, -(Heteroalkylen)-Aryl oder -(Heteroalkenylen)-Aryl; oder unsubstituiertes oder wenigstens einfach substituiertes -(Alkylen)-Heteroaryl, - (Alkenylen)-Heteroaryl, -(Alkinylen)-Heteroaryl, -(Heteroalkylen)-Heteroaryl oder -(Heteroalkenylen)-Heteroaryl stehen;
oder $R^1$ und $R^2$ zusammen mit den sie verbindenden Kohlenstoffatomen einen unsubstituierten oder wenigstens einfach substituierten Phenylen-Rest bilden;
$R^3$, $R^8$ und $R^{54}$, unabhängig voneinander, jeweils für H; $-C(=O)-R^{58}$; $-C(=O)-O-R^{59}$; $-C(=O)-NH_2$; $-C(=O)-NH-R^{64}$; $-C(=O)-NR^{65}R^{66}$; $-S(=O)-R^{69}$; $-S(=O)_2-R^{70}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, - (Heteroalkylen)-Cycloalkenyl oder -(Heteroalkenylen)- Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl; unsubstituiertes oder substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alklnylen)-Aryl, - (Heteroalkylen)-Aryl oder -(Heteroalkenylen)-Aryl; oder unsubstituiertes oder substituiertes -(Alkylen)-Heteroaryl, -(Alkenylen)- Heteroaryl, -(Alkinylen)-Heteroaryl, -(Heteroalkylen)-Heteroaryl oder -(Heteroalkenylen)-Heteroaryl stehen;
$R^4$, $R^5$, $R^6$, $R^7$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$ $R^{30}$, $R^{31}$, $R^{32}$, $R^{33}$, $R^{34}$, $R^{35}$, $R^{36}$, $R^{37}$, $R^{36}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$ und $R^{53}$, unabhängig voneinander, jeweils für H; F; Cl; Br; I; $-NO_2$; -CN; $-NH_2$; -OH; -SH; -C(=O)-OH; -C(=O)-H; -NH-C(=O)-H; -NH-$R^{55}$; -$NR^{56}R^{57}$; -C(=O)-$R^{58}$; -C(=O)-O-$R^{59}$; -O-C(=O)-$R^{60}$; -NH-C(=O)-$R^{61}$; -$NR^{62}$-C(=O)-$R^{63}$; -C(=O)$NH_2$; -C(=O)-NH-$R^{64}$; - C(=O)-$NR^{65}R^{66}$; -O-$R^{67}$; -S-$R^{68}$; -S(=O)-$R^{69}$; -$S(=O)_2$-$R^{70}$; -NH-C(=O)-NH-$R^{71}$; - NH-C(=S)-NH-$R^{72}$; -NH-$S(=O)_2$-$R^{73}$; -$NR^{14}$-$S(=O)_2$-$R^{75}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes-(Alkylen)-Cycloalkyl, -(Alkenylen)-Cycloalkyl, -(Alkinylen)-Cycloalkyl, - (Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, - (Heteroalkenylen)-Cycloalkyl, -(Heteroalkylen)-Cycloalkenyl oder - (Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, - (Alkylen)-Heterocycloalkenyl, -(Alkenylen)-Heterocycloalkenyl oder - (Alkinylen)-Heterocycloalkenyl; unsubstituiertes oder substituiertes - (Heteroalkylen)-Heterocycloalkyl, -(Heteroalkenylen)-Heterocycloalkyl,-(Heteroalkylen)-Heterocycloalkenyl oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, -(Heteroalkylen)-Aryl oder-(Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl stehen;

oder $R^4$ und $R^5$ oder $R^6$ und $R^7$ oder $R^{10}$ und $R^{11}$ oder $R^{12}$ und $R^{13}$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$ oder $R^{28}$ und $R^{29}$ oder $R^{30}$ und $R^{31}$ oder $R^{32}$ und $R^{33}$ oder $R^{34}$ und $R^{35}$ oder $R^{36}$ und $R^{37}$ oder $R^{38}$ und $R^{39}$ oder $R^{40}$ und $R^{41}$ oder $R^{42}$ und $R^{43}$ oder $R^{44}$ und $R^{45}$ oder $R^{46}$ und $R^{47}$ oder $R^{48}$ und $R^{49}$ oder $R^{50}$ und $R^{51}$ oder $R^{52}$ und $R^{53}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^3$ und $R^4$ zusammen mit der sie verbindenden -N-$CR^5$-Gruppe einen Rest der allgemeinen Formel A,

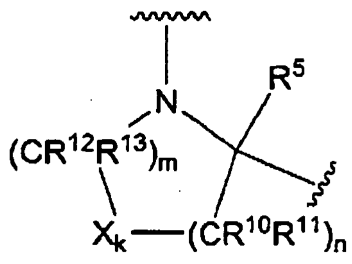

A,

bilden;

oder $R^6$ und $R^8$ zusammen mit der sie verbindenden -N-$CR^7$-Gruppe einen Rest der allgemeinen Formel B,

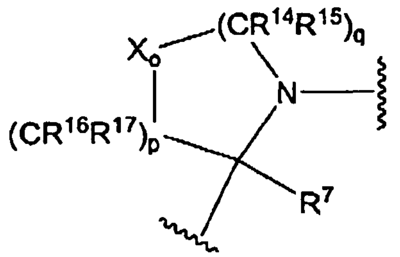

B,

bilden;

m und q jeweils für 1, 2, 3, 4 oder 5 stehen;

n und p jeweils für 0, 1, 2, 3 oder 4 stehen;

k und o jeweils für 0 oder 1 stehen;

wobei die Summe aus m, n und k oder die Summe aus p, q und o jeweils gleich 1, 2, 3, 4, 5 oder 6 ist;
oder $R^3$ und $R^6$ zusammen mit der sie verbindenden -N-$CR^4R^5$-$CR^7$-Gruppe einen Rest der allgemeinen Formel C,

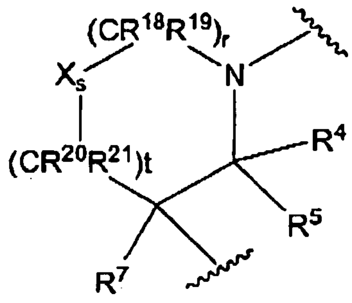

C,

bilden;
oder $R^4$ und $R^8$ zusammen mit der sie verbindenden -$CR^5$-$CR^6R^7$-N-Gruppe einen Rest der allgemeinen Formel D,

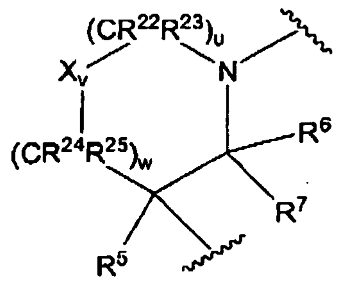

D,

bilden;
r und u jeweils für 1, 2, 3 oder 4 stehen;
t und w jeweils für 0, 1, 2 oder 3 stehen;
s und v jeweils für 0 oder 1 stehen;
wobei die Summe aus r, s und t oder die Summe aus u, v und w jeweils gleich 1, 2, 3, 4 oder 5 ist;
oder $R^3$ und $R^8$ zusammen mit der sie verbindenden -N-$CR^4R^5$-$CR^6R^7$-N-Gruppe einen Rest der allgemeinen Formel E,

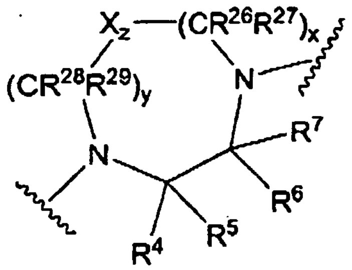

E,

bilden;
x und y jeweils für 1 oder 2 stehen;

z für 0 oder 1 steht;
wobei die Summe aus x, y und z gleich 3 oder 4 ist;
oder $R^4$ und $R^6$ zusammen mit der sie verbindenden $-CR^5-CR^7$-Gruppe einen Rest der allgemeinen Formel F,

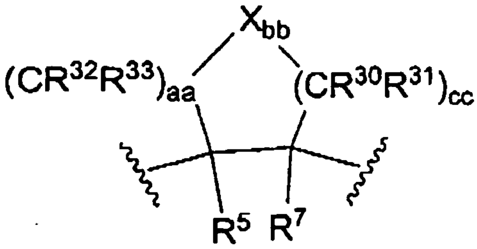

F,

bilden;
aa und cc, unabhängig voneinander, jeweils für 1, 2, 3, 4 oder 5 stehen;
bb für 0 oder 1 steht;
wobei die Summe aus aa, bb und cc gleich 1, 2, 3, 4, 5 oder 6 ist;
oder $R^3$ und $R^4$ zusammen mit der sie verbindenden $-N-CR^5$-Gruppe und $R^6$ und $R^8$ zusammen mit der sie verbindenden $-N-CR^7$-Gruppe einen Rest der allgemeinen Formel G,

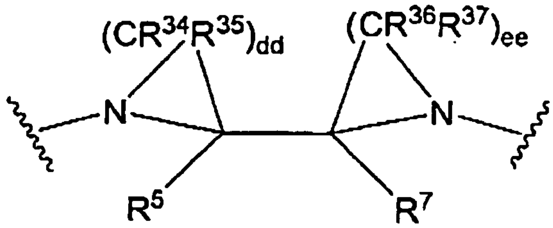

G,

bilden;
dd und ee, unabhängig voneinander, jeweils für 1, 2, 3 oder 4 stehen;
oder $R^3$ und $R^6$ zusammen mit der sie verbindenden $-N-CR^4CR^5-CR^7$-Gruppe und $R^4$ und $R^8$ zusammen mit der sie verbindenden $-CR^5-CR^6R^7-N$-Gruppe einen Rest der allgemeinen Formel H,

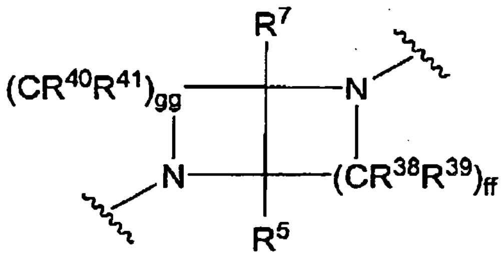

H,

bilden,
ff und gg, unabhängig voneinander, jeweils für 1, 2 oder 3 stehen;
oder $R^3$ und $R^8$ zusammen mit der sie verbindenden $-N-CR^4R^5-CR^6R^7-N$-Gruppe und $R^4$ und $R^6$ zusammen mit der sie verbindenden $-CR^5-CR^7$-Gruppe einen Rest der allgemeinen Formel K,

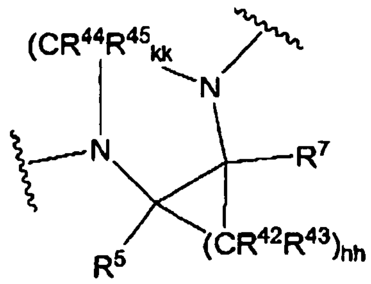

K,

bilden;
hh für 1, 2, 3 oder 4 steht;
kk für 1, 2, 3, 4, 5 oder 6 steht;
oder $R^3$ und $R^6$ zusammen mit der sie verbindenden -N-$CR^4R^5$-$CR^6R^7$-N-Gruppe einen bizyklischen Rest der allgemeinen Formel L,

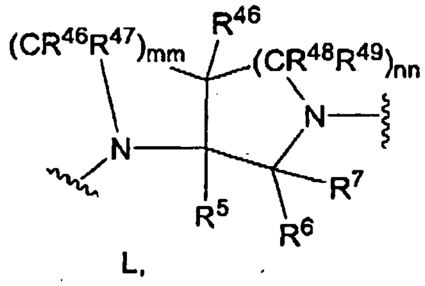

L,

bilden;
mm für 1, 2 oder 3 steht;
nn für 1 oder 2 steht;
oder $R^3$ und $R^8$ zusammen mit der sie verbindenden -N-$CR^4R^5$-$CR^6R^7$-N-Gruppe einen bizyklischen Rest der allgemeinen Formel M,

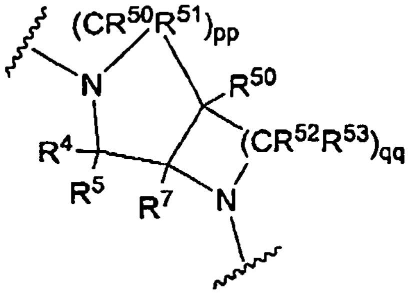

M,

bilden;
pp für 1 oder 2 steht;
qq für 1, 2 oder 3 steht;
X für O, S oder N-$R^{54}$ steht;
$R^9$ für H; F; Cl; Br; I; -CN; -C(=O)-OH; -C(=O)-H; -C(=O)-$R^{58}$; -C(=O)-O-$R^{59}$;-C(=O)-$NH_2$; -C(=O)-NH-$R^{64}$; -C

(=O)-$NR^{65}R^{66}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes - (Alkylen)-Cycloalkyl, -(Alkenylen)-Cycloalkyl, -(Alkinylen)-Cycloalkyl, - (Alkylen)-Cycloalkenyl, -(Alkenylen)- Cycloalkenyl oder -(Alkinylen)-Cycloalkenyl; unsubstituiertes oder substituiertes -(Heteroalkylen)-Cycloalkyl, - (Heteroalkenylen)-Cycloalkyl, -(Heteroalkylen)-Cycloalkenyl oder - (Heteroalkenylen)-Cycloalkenyl ; unsubstituiertes oder substituiertes -(Alkylen)-Heterocycloalkyl, -(Alkenylen)-Heterocycloalkyl, -(Alkinylen)-Heterocycloalkyl, - (Alkylen)-Heterocycloalkenyl, -(Alkenylen)-Heterocycloalkenyl oder - (Alkinylen)-Heterocycloalkenyl; unsubstituiertes oder substituiertes - (Heteroalkylen)-Heterocycloalkyl, -(Heteroalkenylen)-Heterocycloalkyl, - (Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl,- (Alkenylen)-Aryl, -(Alkinylen)-Aryl, -(Heteroalkylen)-Aryl oder - (Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl steht;
und $R^{55}$, $R^{56}$, $R^{57}$, $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$ und $R^{75}$, unabhängig voneinander, jeweils für unsubstituiertes oder substituiertes Alkyl, Alkenyl oder Alkinyl; unsubstituiertes oder substituiertes Heteroalkyl, Heteroalkenyl oder Heteroalkinyl;
unsubstituiertes oder substituiertes Cycloalkyl oder Cycloalkenyl;

unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl;
unsubstituiertes oder substituiertes -(Heteroalkylen)-Heterocycloalkyl, - (Heteroalkenylen)-Heterocycloalkyl, -(Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl,-(Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

**2.** Verbindungen gemäß Anspruch 1, **dadurch gekennzeichnet, dass**

$R^1$ und $R^2$, unabhängig voneinander, jeweils für H; F; Cl; Br; I; -$NO_2$; -CN;-$NH_2$; -OH; -SH; -C(=O)-OH; -C(=O)-H; -NH-C(=O)-H; -NH-$R^{55}$; -$NR^{56}R^{57}$; - C(=O)-$R^{58}$; -C(=O)-O-$R^{59}$; -O-C(=O)-$R^{60}$; -NH-C(=O)-$R^{61}$; -$NR^{62}$-C(=O)-$R^{63}$; - C(=O)-$NH_2$; -C(=O)-NH-$R^{64}$; -C(=O)-$NR^{65}R^{66}$; -O-$R^{67}$; -S-$R^{68}$; -S(=O)-$R^{69}$; - $S(=O)_2$-$R^{70}$; -NH-C(=O)-NH-$R^{71}$; -NH-C(=S)-NH-$R^{72}$; -NH-$S(=O)_2$-$R^{73}$; -$NR^{74}$-$S(=O)_2$-$R^{75}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl;
unsubstituiertes oder wenigstens einfach substituiertes Heterocycloalkyl oder Heterocycloalkenyl; unsubstituiertes oder wenigstens einfach substituiertes - (Alkylen)-Cycloalkyl, -(Alkenylen)-Cycloalkyl, -(Alkinylen)-Cycloalkyl, - (Alkylen)-Cycloalkenyl, -(Alkenylen)-Cycloalkenyl oder -(Alkinylen)-Cycloalkenyl; unsubstituiertes oder wenigstens einfach substituiertes - (Heteroalkylen)-Cycloalkyl, -(Heteroalkenylen)-Cycloalkyl, -(Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl;
unsubstituiertes oder wenigstens einfach substituiertes -(Heteroalkylen)-Heterocycloalkyl, -(Heteroalkenylen)-Heteracycloalkyl, -(Heteroalkylen)-Heterocycloalkenyl oder -(Heteroalkenylen)-Heterocycloalkenyl;
unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, -(Heteroalkylen)-Aryl oder -(Heteroalkenylen)-Aryl; oder
unsubstituiertes oder wenigstens einfach substituiertes -(Alkylen)-Heteroaryl, - (Alkenylen)-Heteroaryl, -(Alkinylen)-Heteroaryl, -(Heteroalkylen)-Heteroaryl oder -(Heteroalkenylen)-Heteroaryl stehen;

oder $R^1$ und $R^2$ zusammen mit den sie verbindenden Kohlenstoffatomen einen unsubstituierten oder wenigstens einfach substituierten Phenylen-Rest bilden;

$R^3$, $R^8$ und $R^{54}$, unabhängig voneinander, jeweils für H; $-C(=O)-R^{58}$; $-C(=O)-O-R^{59}$; $-C(=O)-NH_2$; $-C(=O)-NH-R^{64}$, $-C(=O)-NR^{65}R^{66}$; $-S(=O)R^{69}$; $-S(=O)_2-R^{70}$; unsubstituiertes oder substituiertes Alkyl, Alkenyl oder Alkinyl; unsubstituiertes oder substituiertes Heteroalkyl, Heteroalkenyl oder Heteroalkinyl;

unsubstituiertes oder substituiertes Cycloalkyl oder Cycloalkenyl;

unsubstituiertes oder substituiertes Heterocycloalkyl oder Heterocycloalkenyl;

unsubstituiertes oder substituiertes -(Alkylen)-Cycloalkyl, -(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, - (Heteroalkylen)-Cycloalkenyl oder -(Heteroalkenylen)- Cycloalkenyl;

unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl;

unsubstituiertes oder substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder substituiertes -(Alkylen)-Heteroaryl, -(Alkenylen)- Heteroaryl, -(Alkinylen)-Heteroaryl, -(Heteroalkylen)-Heteroaryl oder -(Heteroalkenylen)-Heteroaryl stehen;

$R^4$, $R^5$, $R^6$, $R^7$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$ , $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$ und $R^{53}$, unabhängig voneinander, jeweils für H; F; Cl; Br; I; $-NO_2$; -CN; $-NH_2$; -OH; -SH; -C(=O)-OH: -C(=O)-H; -NH-C(=O)-H; $-NH-R^{55}$; $-NR^{56}R^{57}$; $-C(=O)-R^{58}$; $-C(=O)-O-R^{59}$; $-O-C(=O)-R^{60}$; $-NH-C(=O)-R^{61}$-, $-NR^{62}-C(=O)-R^{63}$; $-C(=O)-NH_2$; $-C(=O)-NH-R^{64}$; - $C(=O)-NR^{61}R^{66}$; $-O-R^{67}$; $-S-R^{68}$; $-S(=O)-R^{69}$; $-S(=O)_2-R^{70}$; $-NH-C(=O)-NH-R^{71}$; - $NH-C(=S)-NH-R^{72}$; $-NH-S(=O)_2-R^{73}$; $-NR^{74}-S(=O)_2-R^{75}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes - (Alkylen)-Cycloalkyl, -(Alkenylen)-Cycloalkyl, -(Alkinylen)-Cycloalkyl, - (Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, - (Heteroalkenylen)-Cycloalkyl, -(Heteroalkylen)-Cycloalkenyl oder - (Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, - (Alkylen)Heterocycloalkenyl, -(Alkenylen)-Heterocycloalkenyl oder - (Alkinylen)-Heterocycloalkenyl; unsubstituiertes oder substituiertes - (Heteroalkylen)-Heterocycloalkyl, -(Heteroalkenylen)-Heterocycloalkyl, - (Heteroalkylen)-Heterocycloalkenyl oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, -(Heteroalkylen)-Aryl oder - (Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl stehen;

oder $R^4$ und $R^5$ oder $R^6$ und $R^7$ oder $R^{10}$ und $R^{11}$ oder $R^{12}$ und $R^{13}$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{28}$ und $R^{27}$ oder $R^{28}$ und $R^{29}$ oder $R^{30}$ und $R^{31}$ oder $R^{32}$ und $R^{33}$ oder $R^{34}$ und $R^{35}$ oder $R^{38}$ und $R^{37}$ oder $R^{38}$ und $R^{39}$ oder $R^{40}$ und $R^{41}$ oder $R^{42}$ und $R^{43}$ oder $R^{44}$ und $R^{45}$ oder $R^{46}$ und $R^{47}$ oder $R^{48}$ und $R^{49}$ oder $R^{50}$ und $R^{51}$ oder $R^{52}$ und $R^{53}$, unabhängig voneinander, zusammen jeweils für einen Rest ausgewählt aus der Gruppe bestehend aus einer Oxo-Gruppe (=O) und einer Thioxo-Gruppe (=S) stehen; oder $R^3$ und $R^4$ zusammen mit der sie verbindenden $-N-CR^5$-Gruppe einen Rest der allgemeinen Formel A,

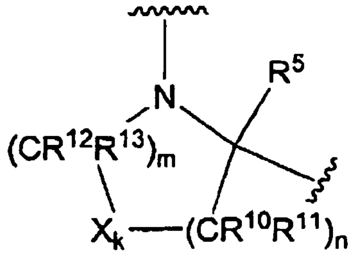

A,

bilden;
oder $R^6$ und $R^8$ zusammen mit der sie verbindenden -N-$CR^7$-Gruppe einen Rest der allgemeinen Formel B,

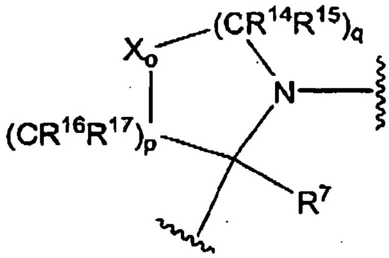

B,

bilden;
m und q jeweils für 1, 2, 3, 4 oder 5 stehen;
n und p jeweils für 0, 1, 2, 3 oder 4 stehen;
k und o jeweils für 0 oder 1 stehen;
wobei die Summe aus m, n und k oder die Summe aus p, q und o jeweils gleich 1, 2, 3, 4, 5 oder 6 ist;
oder $R^3$ und $R^6$ zusammen mit der sie verbindenden -N-$CR^4R^5$-$CR^7$-Gruppe einen Rest der allgemeinen Formel C,

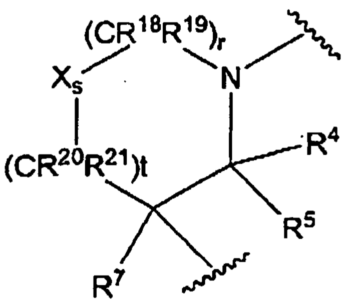

C,

bilden;
oder $R^4$ und $R^8$ zusammen mit der sie verbindenden -$CR^5$-$CR^6R^7$-N-Gruppe einen Rest der allgemeinen Formel D,

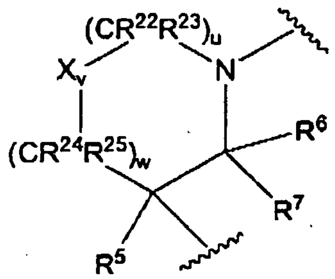

D,

bilden;
r und u jeweils für 1, 2, 3 oder 4 stehen;
t und w jeweils für 0, 1, 2 oder 3 stehen;
s und v jeweils für 0 oder 1 stehen;
wobei die Summe aus r, s und t oder die Summe aus u, v und w jeweils gleich 1, 2, 3, 4 oder 5 ist;
oder $R^3$ und $R^8$ zusammen mit der sie verbindenden $-N-CR^4R^5-CR^6R^1-N-$Gruppe einen Rest der allgemeinen Formel E,

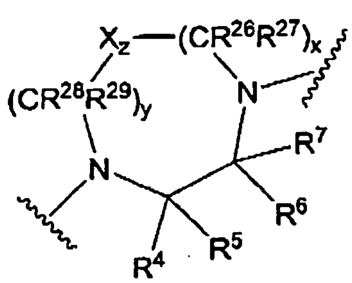

E,

bilden;
x und y jeweils für 1 oder 2 stehen;
z für 0 oder 1 steht;
wobei die Summe aus x, y und z gleich 3 oder 4 ist;
oder $R^4$ und $R^6$ zusammen mit der sie verbindenden $-CR^5-CR^7-$Gruppe einen Rest der allgemeinen Formel F,

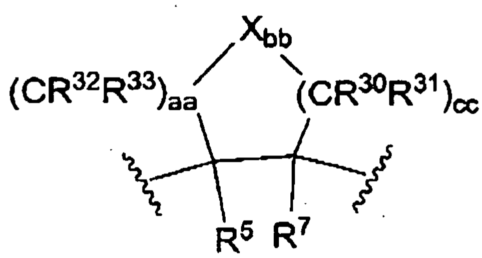

F,

bilden;
aa und cc, unabhängig voneinander, jeweils für 1, 2, 3, 4 oder 5 stehen; bb für 0 oder 1 steht;
wobei die Summe aus aa, bb und cc gleich 1, 2, 3, 4, 5 oder 6 ist;
oder $R^3$ und $R^4$ zusammen mit der sie verbindenden $-N-CR^5-$Gruppe und $R^6$ und $R^8$ zusammen mit der sie verbindenden $-N-CR^7-$Gruppe einen Rest der allgemeinen Formel G,

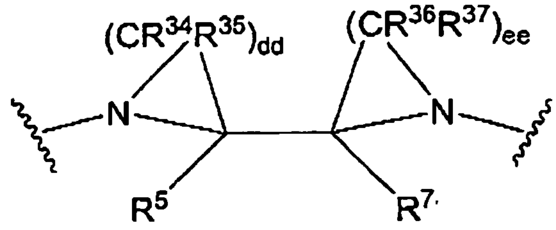

G,

bilden;
dd und ee, unabhängig voneinander, jeweils für 1, 2, 3 oder 4 stehen;
oder $R^3$ und $R^6$ zusammen mit der sie verbindenden -N-$CR^4CR^5$-$CR^7$-Gruppe und $R^4$ und $R^8$ zusammen mit der sie verbindenden -$CR^5$-$CR^6R^7$-N-Gruppe einen Rest der allgemeinen Formel H,

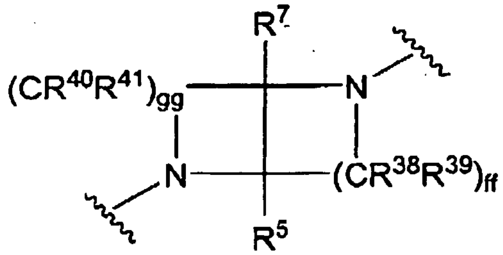

H,

bilden,
ff und gg, unabhängig voneinander, jeweils für 1, 2 oder 3 stehen;
oder $R^3$ und $R^8$ zusammen mit der sie verbindenden -N-$CR^4R^5$-$CR^6R^7$-N-Gruppe und $R^4$ und $R^6$ zusammen mit der sie verbindenden -$CR^5$-$CR^7$-Gruppe einen Rest der allgemeinen Formel K,

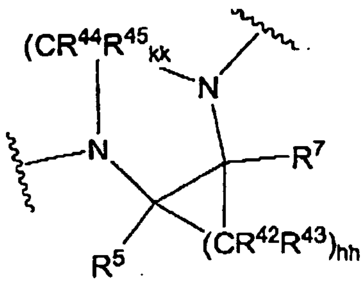

K,

bilden;
hh für 1, 2, 3 oder 4 steht;
kk für 1, 2, 3, 4, 5 oder 6 steht;
oder $R^3$ und $R^8$ zusammen mit der sie verbindenden -N-$CR^4R^5$-$CR^6R^7$-N-Gruppe einen bizyklischen Rest der allgemeinen Formel L,

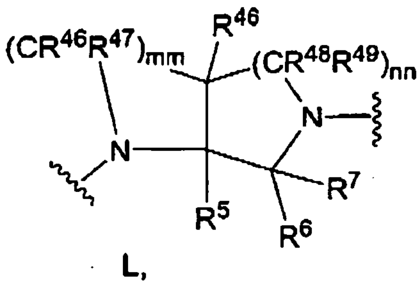

L,

bilden;
mm für 1, 2 oder 3 steht;
nn für 1 oder 2 steht;
oder $R^3$ und $R^8$ zusammen mit der sie verbindenden $-N-CR^4R^5-CR^6R^7-N-$Gruppe einen bizyklischen Rest der allgemeinen Formel M,

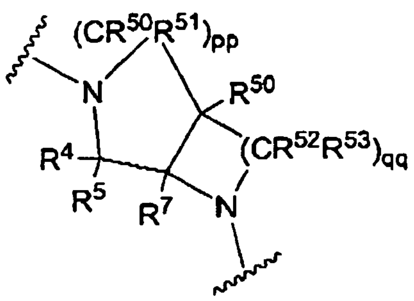

M,

bilden;
pp für 1 oder 2 steht;
qq für 1, 2 oder 3 steht;
X für O, S oder $N-R^{54}$ steht;
$R^9$ für H; F; Cl; Br; I; -CN; -C(=O)-OH; -C(=O)-H; $-C(=O)-R^{58}$; $-C(=O)-O-R^{59}$;$-C(=O)-NH_2$; $-C(=O)-NH-R^{64}$; $-C(=O)-NR^{65}R^{66}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes - (Alkylen)-Cycloalkyl, -(Alkenylen)-Cycloalkyl, -(Alkinylen)-Cycloalkyl, - (Alkylen)-Cycloalkenyl, -(Alkenylen)- Cycloalkenyl oder -(Alkinylen)-Cycloalkenyl; unsubstituiertes oder substituiertes -(Heteroalkylen)-Cycloalkyl, - (Heteroalkenylen)-Cycloalkyl, -(Heteroalkylen)-Cycloalkenyl oder - (Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, - (Alkylen)-Heterocycloalkenyl, -(Alkenylen)-Heterocycloalkenyl oder - (Alkinylen)-Heterocycloalkenyl; unsubstituiertes oder substituiertes - (Heteroalkylen)-Heterocycloalkyl, -(Heteroalkenylen)-Heterocycloalkyl, - (Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, -(Heteroalkylen)-Aryl oder - (Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl steht;
und $R^{55}$, $R^{55}$, $R^{57}$ $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$ und $R^{75}$, unabhängig voneinander, jeweils für unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenylen)-Heterocycloalkyl, -(Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl stehen;

wobei

die vorstehend genannten Alkyl-Reste jeweils verzweigt oder geradkettig sind und 1, 2, 3, 4, 5, 6, 7, 8, 9, 10, 11 oder 12 Kohlenstoffatome als Kettenglieder aufweisen;

die vorstehend genannten Alkenyl-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Alkinyl-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Heteroalkyl-Reste, Heteroalkenyl-Reste und Heteroalkinyl-Reste jeweils 2-, 3-, 4-, 5-, 6-, 7-, 8-, 9-, 10-, 11- oder 12-gliedrig sind;

die vorstehend genannten Heteroalkyl-Reste, Heteroalkenyl-Reste und Heteroalkinyl-Reste jeweils ggf. 1, 2 oder 3 Heteroatom(e) unabhängig voneinander ausgewählt aus der Gruppe bestehend aus Sauerstoff, Schwefel und Stickstoff als Kettenglied(er) aufweisen;

die vorstehend genannten Alkyl-Reste, Alkenyl-Reste, Alkinyl-Reste, Heteroalkyl-Reste, Heteroalkenyl-Reste und Heteroalkinyl-Reste jeweils mit ggf. 1, 2, 3, 4 oder 5 Substituenten unabhängig voneinander ausgewählt aus der Gruppe bestehend aus F, Cl, Br, I, $-NO_2$, -CN, -OH, -SH, $-NH_2$, -N($C_{1-5}$-Alkyl, -N($C_{1-5}$-Alkyl)(Phenyl), -N($C_{1-5}$-Alkyl)($CH_2$-Phenyl), -N($C_{1-5}$-Alkyl)($CH_2$-$CH_2$-Phenyl), -C(=O)-H, -C(=O)-$C_{1-5}$ Alkyl, -C(=O)-Phenyl, -C(=S)-$C_{1-5}$-Alky), - C(=S)-Phenyl, -C(=O)-OH, -C(=O)-O-$C_{1-5}$-Alkyl, -C(=O)-O-Phenyl, -C(=O)-$NH_2$, -C(=O)-NH-$C_{1-5}$-Alkyl, -C(=O)-N($C_{1-5}$-Alkyl)$_2$, -S(=O)-$C_{1-5}$-Alkyl, -S(=O)-Phenyl, -S(=O)$_2$-$C_{1-5}$-Alkyl, -S(=O)$_2$-Phenyl, -S(=O)$_2$-$NH_2$ und -$SO_3H$ substituiert sein können, wobei die Phenyl-Reste mit 1, 2, 3, 4 oder 5 Substituenten unabhängig voneinander ausgewählt aus der Gruppe bestehend aus F, Cl, Br, I, -CN, -$CF_3$, -OH, -$NH_2$, -O-$CF_3$, -SH, -O-$CH_3$, -O-$C_2H_5$, -O-$C_3H_7$, Methyl, Ethyl, n-Propyl, Isopropyl, n-Butyl, 2-Butyl, Isobutyl und tert-Butyl substituiert sein können;

die vorstehend genannten Cycloalkyl-Reste jeweils 3, 4, 5, 6, 7, 8 oder 9 Kohlenstoffatome als Ringglieder aufweisen;

die vorstehend genannten Cycloalkenyl-Reste jeweils 3, 4, 5, 6, 7, 8 oder 9 Kohlenstoffatome als Ringglieder aufweisen;

die vorstehend genannten Heterocycloalkyl-Reste jeweils 3-, 4-, 5-, 6-, 7-, 8- oder 9-gliedrig sind;

die vorstehend genannten Heterocycloalkenyl-Reste jeweils 4-, 5-, 6-, 7-, 8- oder 9-gliedrig sind;

die vorstehend genannten Heterocycloalkyl-Reste und Heterocycloalkenyl-Resten jeweils ggf. 1, 2 oder 3 Heteroatom(e) unabhängig voneinander ausgewählt aus der Gruppe bestehend aus Sauerstoff, Schwefel und Stickstoff (NH) als Ringglied(er) aufweisen;

die vorstehend genannten Cycloalkyl-Reste, Heterocycloalkyl-Reste, Cycloalkenyl-Reste oder Heterocycloalkenyl-Reste jeweils mit ggf. 1, 2, 3, 4- oder 5 Substituenten unabhängig voneinander ausgewählt aus der Gruppe bestehend aus F, Cl, Br, I, -CN, -$CF_3$, -OH, -$NH_2$, -O-$CF_3$, -SH, -O-$C_1$-$_5$-Alkyl,-O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-Si($CH_3$)$_3$, -C≡C-Si($C_2H_5$)$_3$, -C(=O)-O-$C_{1-5}$-Alkyl, -C(=O)-$CF_3$, -S(=O)$_2$-$C_{1-5}$-Alkyl, -S(=O)-$C_{1-5}$-Alkyl, -S(=O)$_2$-Phenyl, Oxo (=O), Thioxo (=S), -N($C_{1-5}$-Alkyl)$_2$, -N(H)($C_{1-5}$-Alkyl), -$NO_2$, -S-$CF_3$, -C(=O)-OH, -NH-S(=O)$_2$-$C_{1-5}$-Alkyl, -NH-C(=O)-$C_{1-5}$-Alkyl, - C(=O)-H, -C(=O)-$C_{1-5}$-Alkyl, -C(=O)-$NH_2$, -C(=O)-N($C_{1-5}$-Alkyl)$_2$, -C(=O)-N(H)($C_{1-5}$-Alkyl) und Phenyl substituiert sein können, wobei die Phenyl-Reste jeweils unsubstituiert oder mit 1, 2, 3, 4 oder 5 Substituenten unabhängig voneinander ausgewählt aus der Gruppe bestehend aus F, Cl, Br, I, -CN,-$CF_3$, -OH, -$NH_2$, -O-$CF_3$, -SH, -O-$C_{1-5}$-Alkyl, -O-Phenyl, -O-$CH_2$-Phenyl, - ($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-Si($CH_3$)$_3$, -C≡C-Si($C_2H_5$)$_3$, -C(=O)-O-$C_{1-5}$-Alkyl und -C(=O)-$CF_3$ substituiert sein können, wobei die vorstehend genannten Phenyl-Reste bevorzugt mit 1, 2, 3, 4 oder 5 Substituenten unabhängig voneinander ausgewählt aus der Gruppe bestehend aus F, Cl, Br, I, -CN, -$CF_3$, -OH, -$NH_2$,-O-$CF_3$, -SH, -O-$CH_3$, -O-$C_2H_5$, -O-$C_3H_7$, Methyl, Ethyl, n-Propyl, Isopropyl, n-Butyl, 2-Butyl, Isobutyl und tert-Butyl substituiert sein können;

die vorstehend genannten Alkylen-Reste jeweils verzweigt oder geradkettig sind und 1, 2, 3, 4, 5, 6, 7, 8, 9, 10, 11 oder 12 Kohlenstoffatome als Kettenglieder aufweisen;

die vorstehend genannten Alkenylen-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Alkinylen-Reste jeweils verzweigt oder geradkettig sind und 2, 3, 4, 5, 6, 7, 8, 9, 10,

11 oder 12 Kohlenstoffatome als Kettenglieder aufweisen;
die vorstehend genannten Heteroalkylen-, Heteroalkenylen-Reste und Heteroalkinylen-Reste jeweils 2-, 3-, 4-, 5-, 6-, 7-, 8-, 9-, 10-, 11- oder 12-gliedrig sind;
die vorstehend genannten Heteroalkylen-, Heteroalkenylen- und Heteroalkinylen-Gruppen jeweils ggf. 1, 2 oder 3 Heteroatom(e) unabhängig voneinander ausgewählt aus der Gruppe bestehend aus Sauerstoff, Schwefel und Stickstoff (NH) als Kettenglied(er) aufweisen;
die vorstehend genannten Alkylen-, Alkenylen-, Alkinylen-, Heteroalkylen-, Heteroalkenylen- und Heteroalkinylen-Gruppen jeweils unsubstituiert oder mit ggf. 1, 2, 3, 4 oder 5 Substituenten unabhängig voneinander ausgewählt aus der Gruppe bestehend aus Phenyl, F, Cl, Br, I, $-NO_2$, -CN, -OH, -O-Phenyl, $-O-CH_2$-Phenyl,- SH, -S-Phenyl, $-S-CH_2$-Phenyl, $NH_2$, $-N(C_{1-5}\text{-Alkyl})_2$, -NH-Phenyl, $-N(C_{1-5}$-Alkyl)(Phenyl), $-N(C_{1-5}\text{-Alkyl})(CH_2$-Phenyl), $-N(C_{1-5}\text{-Alkyl})(CH_2-CH_2$-Phenyl), -C(=O)-H, $-C(=O)-C_{1-5}$-Alkyl, -C(=O)-Phenyl, $-C(=S)-C_{1-5}$-Alkyl, - C (=S)-Phenyl, -C(=O)-OH, $-C(=O)-O-C_{1-5}$-Alkyl, -C(=O)-O-Phenyl, $-C(=O)-NH_2$, $-C(=O)-NH-C_{1-5}$-Alkyl, $-C(=O)-N(C_{1-5}\text{-Alkyl})_2$, $-S(=O)-C_{1-5}$-Alkyl, -S(=O)-Phenyl, $-S(=O)_2-C_{1-5}$-Alkyl, $-S(=O)_2$Phenyl, $-S(=O)_2-NH_2$ und $-SO_3H$ substituiert sein können, wobei die Phenyl-Reste mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl, $-C=C-Si(CH_3)_3$, $-C=C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$, -C (=O)-$CF_3$, $-S-CF_3$, $-S=CHF_2$ und $-S-CH_2F$ substituiert sein können;
die vorstehend genannten Aryl-Reste mono- oder bizyklisch sind und 6, 10 oder 14-Kohlenstoffatome aufweisen;
die vorstehend genannten Heteroaryl-Reste mono-, bi- oder trizyklisch und 5-, 6-, 7-, 8-, 9-, 10-, 91-, 12-, 13- oder 14-gliedrig sind;
die vorstehend genannten 5- bis 14-gliedriges Heteroaryl-Reste ggf. 1, 2, 3, 4 oder 5 Heteroatom(e) unabhängig voneinander ausgewählt aus der Gruppe bestehend aus Sauerstoff, Schwefel und Stickstoff (NH) als Ringglied (er) aufweisen;
und die vorstehend genannten Phenylen-Reste, Aryl-Reste und Heteroaryl-Reste jeweils mit ggf. 1, 2, 3, 4 oder 5 Substituenten unabhängig voneinander ausgewählt aus der Gruppe bestehend aus F, Cl, Br, I, -CN, $-NO_2$, -OH, -SH, - $NH_2$, -C(=O)-OH, $-C_{1-5}$-Alkyl, $-(CH_2)-O-C_{1-5}$-Alkyl, $-C_{2-5}$-Alkenyl, $-C_{2-6}$-Alkinyl, - $C\equiv C-Si(CH_3)_3$, $-C=C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, -S $(=O)_2-C_{1-5}$-Alkyl, $-S(=O)-C_{1-5}$-Alkyl, $-NH-C_{1-5}$-Alkyl, $N(C_{1-5}\text{-Alkyl})_2$, $-C(=O)-O-C_{1-5}$-Alkyl, - C(=O)-H, -C (=O)-$C_{1-5}$-Alkyl, $-CH_2-O-C(=O)$-Phenyl, -O-C(=O)-Phenyl, $-NH-S(=O)_2-C_{1-5}$-Alkyl, $-NH-C(=O)-C_{1-5}$-Alkyl, -C (=O)-$NH_2$, $-C(=O)-NH-C_{1-5}$-Alkyl, - $C(=O)-N(C_{1-5}\text{-Alkyl})_2$, Pyrazolyl, Phenyl, Furyl (Furanyl), Thiazolyl, Thiadiazolyl, Thiophenyl (Thienyl) , Benzyl und Phenethyl substituiert sein können, wobei die zyklischen Substituenten bzw. die zyklischen Reste dieser Substituenten selbst mit ggf. 1, 2, 3, 4 oder 5, Substituenten unabhängig voneinander ausgewählt aus der Gruppe bestehend aus F, Cl, Br, I, -CN,-$NO_2$, -OH, -SH, $-NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl, $-C\equiv C-Si(CH_3)_3$, $-C\equiv C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

**3.** Verbindungen gemäß Anspruch 1 oder 2, **dadurch gekennzeichnet, dass**

$R^1$ für H: F; Ci; Br; I; $-CF_3$; $-NO_2$; -CN; $-NH_2$; -OH; -SH; -C(=O)-OH; -C(=O)-H;-NH-C(=O)-H; $-NH-R^{55}$; $-NR^{56}R^{57}$; $-C(=O)-R^{58}$; $-C(=O)-O-R^{59}$; $-C(=O)-NH_2$; - $C(=O)-NH-R^{64}$; $-C(=O)-NR^{65}R^{66}$; $-O-R^{67}$; $-S-R^{68}$; $-S(=O)-R^{69}$; -S $(=O)_2-R^{70}$; unsubstituiertes $C_{1-6}$-Alkyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, - $O-CH_3$, $-O-C_2H_5$ und $-O-C_3H_7$ substituiert ist, steht;
$R^2$ für H; F; Cl; Br; I; $-CF_3$; $-NO_2$; -CN; $-NH_2$; -OH; -SH; -C(=O)-OH; -C(=O)-H; - NH-C(=O)-H; $-NH-R^{55}$; $-NR^{56}R^{57}$; $-C(=O)-R^{58}$; $-C(=O)-O-R^{59}$; $-C(=O)-NH_2$; - $C(=O)-NH-R^{64}$; $-C(=O)-NR^{65}R^{66}$; $-O-R^{67}$; $-S-R^{68}$; $-S(=O)-R^{69}$; -S $(=O)_2-R^{70}$; unsubstituiertes $C_{1-6}$-Alkyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, - $O-CH_3$, $-O-C_2H_5$ und $-O-C_3H_7$ substituiert ist, steht;

oder $R^1$ und $R^2$ zusammen mit den sie verbindenden Kohlenstoffatomen einen Phenylen-Rest bilden, der unsubstituiert oder mit 1, 2, 3 oder 4 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, -OH, -SH, $-NH_2$, -C(=O)-OH, $-S-CH_3$, $-S-C_2H_5$, - $S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-CF_3$, $-CHF_2$, $-CH_2F$ und $-O-CF_3$ substituiert ist;

$R^3$ und $R^8$, unabhängig voneinander, jeweils für H; $-C(=O)-R^{58}$; $-C(=O)-O-R^{59}$; - $C(=O)-NH_2$; $-C(=O)-NH-R^{64}$; $-C(=O)-NR^{65}R^{66}$; $-S(=O)-R^{69}$; $-S(=O)_2-R^{70}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist;

$C_{3-6}$-Cycloalkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist;

oder für einen Phenyl-Rest stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist;

$R^4$, $R^5$, $R^6$, $R^7$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$ und $R^{45}$, unabhängig voneinander, jeweils für H; F; Cl; Br; I; $-NO_2$;-CN; $-NH_2$; -OH; -SH; -C(=O)-OH; $-NH-R^{55}$; $-NR^{56}R^{57}$; $-C(=O)-R^{58}$; $-C(=O)-O-R^{59}$, $-O-R^{67}$; $-S-R^{68}$;

unsubstituiertes $C_{1-6}$-Alkyl; oder für einen Rest ausgewählt aus der Gruppe bestehend aus Phenyl, Benzyl und Phenethyl stehen, der unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist;

oder $R^4$ und $R^5$ oder $R^6$ und $R^7$ oder $R^{10}$ und $R^{11}$ oder $R^{12}$ und $R^{13}$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$ oder $R^{28}$ und $R^{29}$ oder $R^{32}$ und $R^{33}$ oder $R^{34}$ und $R^{35}$ oder $R^{36}$ und $R^{37}$ oder $R^{38}$ und $R^{39}$ oder $R^{40}$ und $R^{41}$ oder $R^{42}$ und $R^{43}$ oder $R^{44}$ und $R^{45}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^3$ und $R^4$ zusammen mit der sie verbindenden $-N-CR^5$-Gruppe einen Rest ausgewählt aus der Gruppe bestehend aus

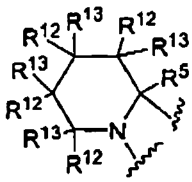 , 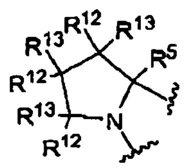 , 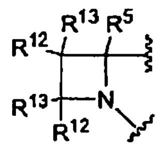 , 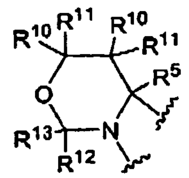 ,

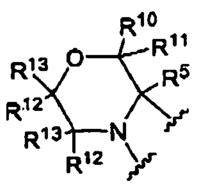 , 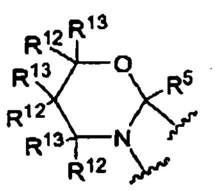 , 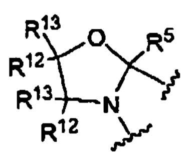 und 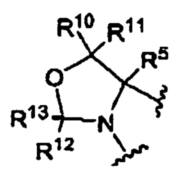

bilden;

oder $R^6$ und $R^8$ zusammen mit der sie verbindenden $-N-CR^7$-Gruppe einen Rest ausgewählt aus der Gruppe bestehend aus

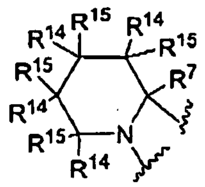 , 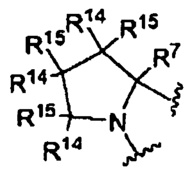 , 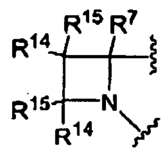 , 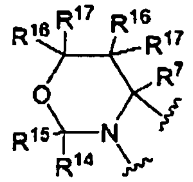 ,

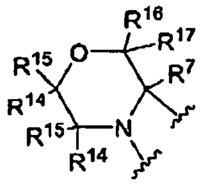 , 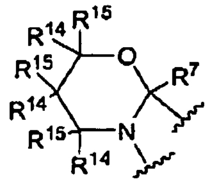 , 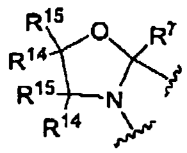 und 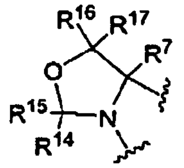 ,

bilden;

oder $R^3$ und $R^6$ zusammen mit der sie verbindenden $-N-CR^4R^5-CR^7$-Gruppe einen Rest ausgewählt aus der Gruppe bestehend aus

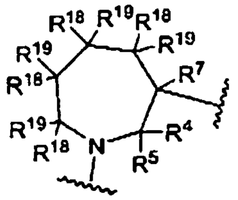 , 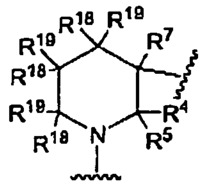 , 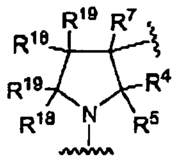 ,

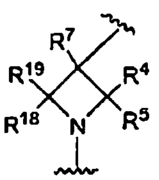 , 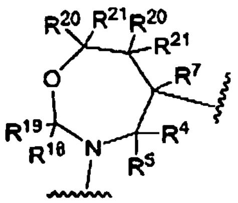 , 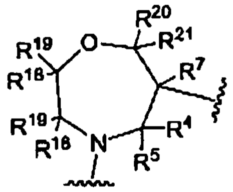 , 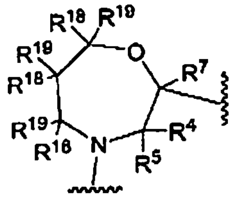 ,

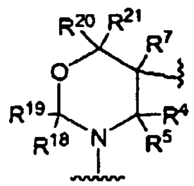 , 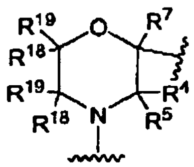 und 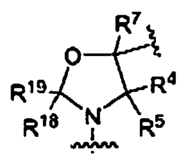

bilden;

oder $R^4$ und $R^8$ zusammen mit der sie verbindenden $-CR^5-CR^6CR^7-N$-Gruppe einen Rest ausgewählt aus der Gruppe bestehend aus

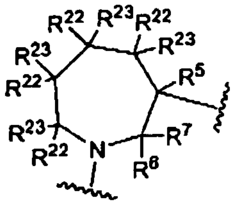,
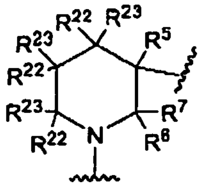,
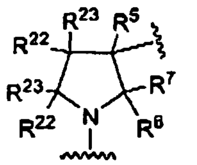,

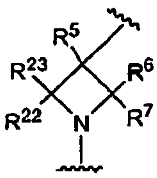,
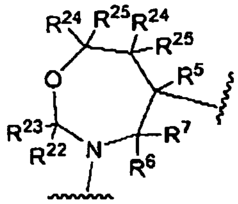,
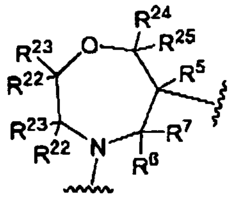,
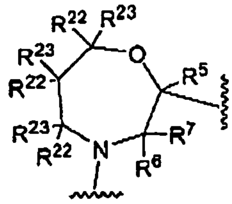,

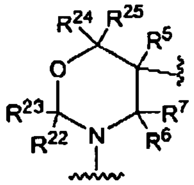,
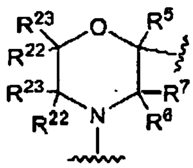
und
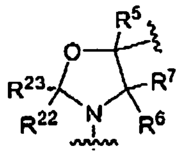

bilden;

oder $R^3$ und $R^8$ zusammen mit der sie verbindenden -N-$CR^4R^5$-$CR^6CR^7$-N-Gruppe einen Rest ausgewählt aus der Gruppe bestehend aus

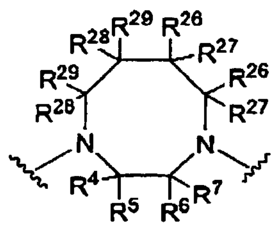
und
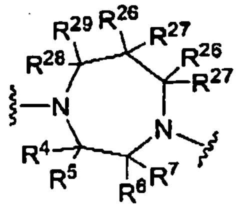

bilden;

oder $R^4$ und $R^6$ zusammen mit der sie verbindenden -$CR^5$-$CR^7$-Gruppe einen Rest ausgewählt aus der Gruppe bestehend aus

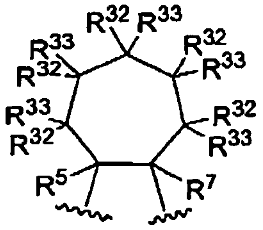,
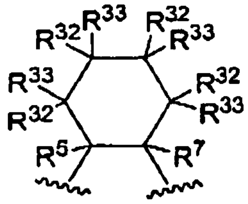
und
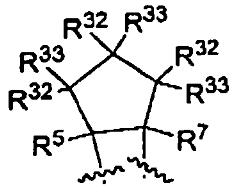,

bilden;

oder $R^3$ und $R^4$ zusammen mit der sie verbindenden -N-$CR^5$-Gruppe und $R^6$ und $R^6$ zusammen mit der sie verbindenden -N-$CR^7$-Gruppe einen Rest ausgewählt aus der Gruppe bestehend aus

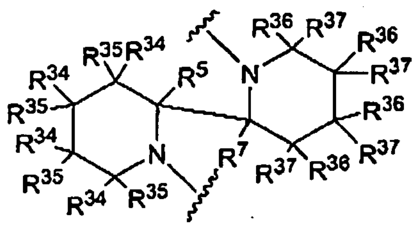
,
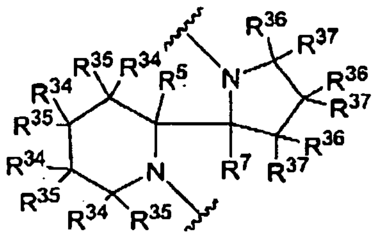
,

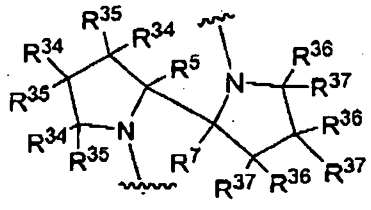
und
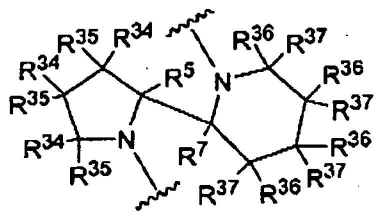

bilden;

oder $R^3$ und $R^6$ zusammen mit der sie verbindenden -N-$CR^4CR^s$-$CR^7$-Gruppe und $R^4$ und $R^8$ zusammen mit der sie verbindenden -$CR^5$-$CR^6R^7$-N-Gruppe einen Rest ausgewählt aus der Gruppe bestehend aus

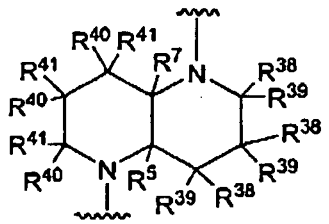
,
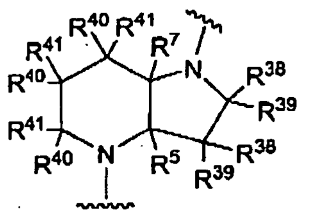
,

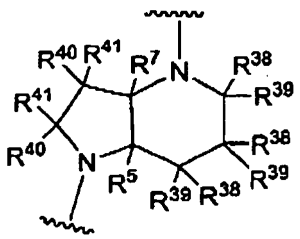
und
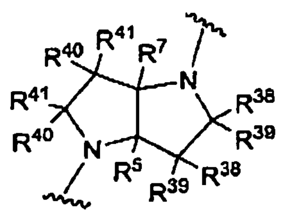

bilden;

oder $R^3$ und $R^8$ zusammen mit der sie verbindenden -N-$CR^4R^5$-$CR^6R^7$-N-Gruppe und $R^4$ und $R^6$ zusammen mit der sie verbindenden -$CR^5$-$CR^7$-Gruppe einen Rest ausgewählt aus der Gruppe bestehend aus

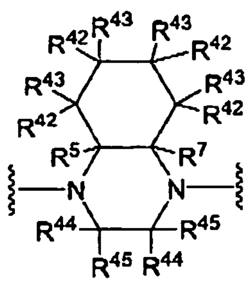
,
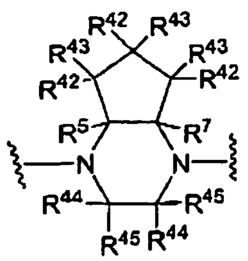
,

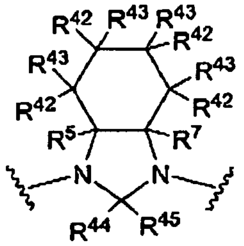

und

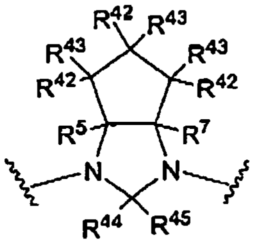

bilden;
$R^9$ für H; -C(=O)-$NH_2$; -C(=O)-NH-$R^{64}$; -C(=O)-$NR^{65}R^{66}$;
unsubstituiertes $C_{1-6}$-Alkyl; unsubstituiertes $C_{1-7}$-Cycloalkyl; unsubstituiertes $C_{5-6}$-Cycloalkenyl; unsubstituiertes 5- bis 7-gliedriges Heterocycloalkyl und unsubstituiertes 5- bis 7-gliedriges Heterocycloalkenyl;
oder für einen Rest ausgewählt aus der Gruppe bestehend aus Phenyl, Naphthyl, Anthracenyl, Furyl, Thienyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl, Imidazolyl, Indolyl, Benz[b]thiophenyl, Benzo[d]thiazolyl, Benzo[b]furanyl, Chinolinyl, Isochinolinyl und Chinazolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, -C=C-Si$(CH_3)_3$, -C≡C-Si$(C_2H_5)_3$, -$CH_2$-O-$CH_3$, -$CH_2$-O-$C_2H_5$, -OH, -O-$CH_3$, -O-$C_2H_5$, -O-$C_3H_7$, -S-$CH_3$, -S-$C_2H_5$, -S(=O)-$CH_3$, -S$(=O)_2$-$CH_3$, -S (=O)-$C_2H_5$, -S$(=O)_2$-$C_2H_5$, -$NH_2$,-N$(CH_3)_2$, -N$(C_2H_5)_2$, -NH-$CH_3$, -NH-$C_2H_5$, -$NO_2$, -$CF_3$, -$CH_2F$, -$CHF_2$, -O-$CF_3$, -S-$CF_3$, -SH, -NH-S$(=O)_2$-$CH_3$, - C(=O)-OH, -C(=O)-H; -C(=O)-$CH_3$, -C(=O)-$C_2H_5$, -C(=O)-$NH_2$, -C(=O)-N $(CH_3)_2$, -C(=O)-NH-$CH_3$, -NH-C(=O)-$CH_3$, -NH-C(=O)-$C_2H_5$, -C(=O)-O-$CH_3$, -C(=O)-O-$C_2H_5$, -C(=O)-O-C $(CH_3)_3$ und Phenyl substituiert ist;
und $R^{55}$, $R^{58}$, $R^{51}$, $R^{58}$, $R^{59}$, $R^{64}$, $R^{65}$, $R^{66}$, $R^{67}$, $R^{68}$, $R^{69}$ und $R^{70}$, unabhängig voneinander, jeweils für unsubstituiertes $C_{1-6}$-Alkyl; unsubstituiertes $C_{3-7}$-Cycloalkyl; unsubstituiertes $C_{5-6}$-Cycloalkenyl; unsubstituiertes 5- bis 7-gliedriges Heterocycloalkyl und unsubstituiertes 5- bis 7-gliedriges Heterocycloalkenyl; oder für einen Rest ausgewählt aus der Gruppe bestehend aus Phenyl, Benzyl, Naphthyl, Anthracenyl, Pyrrolyl, Indolyl, Furanyl, Benzo[b]furanyl, Thiophenyl, Benz[b]thiophenyl, Benzo[d]thiazolyl, Pyrazolyl, Imidazolyl, Thiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Chinolinyl, Isochinolinyl und Chinazolinyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, -O-$CH_3$, -O-$C_2H_5$, - O-$C_3H_7$, -$NH_2$, -N$(CH_3)_2$, -N $(C_2H_5)_2$, -NH-$CH_3$, -NH-$C_2H_5$, -$NO_2$, -$CF_3$, -O-$CF_3$, - S-$CF_3$, -SH, -NH-S$(=O)_2$-$CH_3$, -C(=O)-OH, -C(=O)-$CH_3$, -C(=O)-$C_2H_5$, -C(=O)-N$(CH_3)_2$, -C(=O)-NH-$CH_3$, -NH-C(=O)-$CH_3$, -NH-C(=O)-$C_2H_5$, -C(=O)-O-$CH_3$ und -C (=O)-O-$C_2H_5$ substituiert ist;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

**4.** Verbindungen gemäß einem oder mehreren der Ansprüche 1 bis 3, **dadurch gekennzeichnet, dass**

$R^1$ für H; F; Cl; Br, I; -$CF_3$; -$NO_2$; -CN; -C(=O)-OH; -C(=O)-O-$R^{59}$; -C(=O)-$NH_2$; -C(=O)-NH-$R^{64}$; -C(=O)-$NR^{65}R^{66}$; -O-$R^{67}$; -S-$R^{68}$; -S(=O)-$R^{69}$; -S$(=O)_2$-$R^{70}$; einen Alkyl-Rest ausgewählt aus der Gruppe bestehend aus Methyl, Ethyl, n-Propyl, Isopropyl, n-Butyl, 2-Butyl, Isobutyl und tert-Butyl; oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist, steht;
$R^2$ für H; F; Cl; Br; I; -$CF_3$; -$NO_2$; -CN; -C(=O)-OH; -C(=O)-O-$R^{59}$; -C(=O)-$NH_2$; -C(-O)-NH-$R^{64}$; -C(-O)-$NR^{65}R^{66}$; -O-$R^{67}$; -S-$R^{68}$; -S(=O)-$R^{69}$; -S$(=O)_2$-$R^{70}$; einen Alkyl-Rest ausgewählt aus der Gruppe bestehend aus Methyl, Ethyl, n-Propyl, Isopropyl, n-Butyl, 2-Butyl, Isobutyl und tert-Butyl; oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist, steht;

oder $R^1$ und $R^2$ zusammen mit den sie verbindenden Kohlenstoffatomen einen Phenylen-Rest bilden, der unsubstituiert oder mit 1, 2, 3 oder 4 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, $-O-CH_3$, $-O-C_2H_5$, $-O-C_3H_7$, $-CF_3$, $-CHF_2$, $-CH_2F$ und $-O-CF_3$ substituiert ist;

$R^3$ und $R^8$, unabhängig voneinander, jeweils für H; $-C(=O)-R^{58}$; $-C(=O)-O-R^{59}$; - $S(=O)-R^{69}$; $-S(=O)2-R^{70}$; einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; für einen Cycloalkylrest ausgewählt aus der Gruppe bestehend aus Cyclopropyl, Cyclobutyl, Cyclopentyl und Cyclohexyl; oder für einen Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist;

$R^4$, $R^5$, $R^6$, $R^7$, $R^{12}$, $R^{13}$, $R^{14}$, $R^{15}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{32}$, $R^{33}$, $R^{38}$, $R^{39}$, $R^{40}$, $R^{41}$, $R^{42}$, $R^{43}$, $R^{44}$ und $R^{45}$, unabhängig voneinander, jeweils für H; F; Cl; Br; I; $-NO_2$; -CN; $-NH_2$; -OH; -SH; $-NH-R^{55}$; - $NR^{56}R^{57}$; $-O-R^{67}$; $-S-R^{68}$; oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl stehen;

oder $R^4$ und $R^5$ oder $R^6$ und $R^7$ oder $R^{12}$ und $R^{13}$ oder $R^{14}$ und $R^{15}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$ oder $R^{28}$ und $R^{29}$ oder $R^{32}$ und $R^{33}$ oder oder $R^{38}$ und $R^{39}$ oder $R^{40}$ und $R^{41}$ oder $R^{42}$ und $R^{43}$ oder $R^{44}$ und $R^{45}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^3$ und $R^4$ zusammen mit der sie verbindenden $-N-CR^5$-Gruppe einen Rest ausgewählt aus der Gruppe bestehend aus

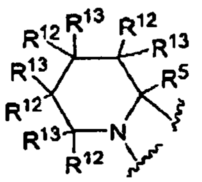 und 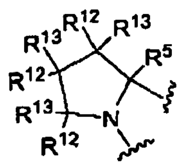

bilden;

oder $R^6$ und $R^8$ zusammen mit der sie verbindenden $-N-CR^7$-Gruppe einen Rest ausgewählt aus der Gruppe bestehend aus

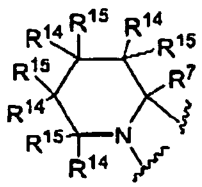 und 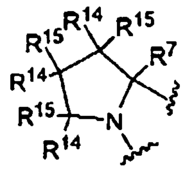

bilden;

oder $R^3$ und $R^6$ zusammen mit der sie verbindenden $-N-CR^4R^5-CR^7$-Gruppe einen Rest ausgewählt aus der Gruppe bestehend aus

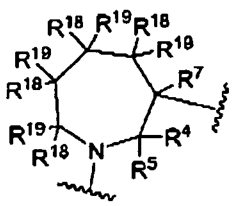 , 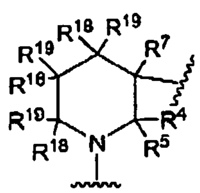 , 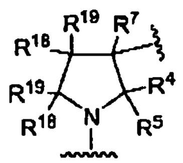 ,

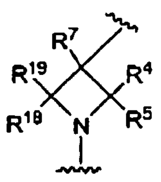 , 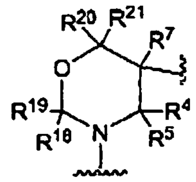 , 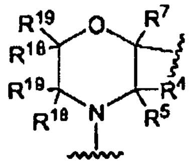 und 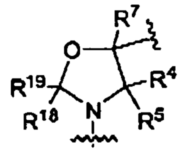

bilden;

oder $R^4$ und $R^8$ zusammen mit der sie verbindenden $-CR^5-CR^6CR^7-N-$Gruppe einen Rest ausgewählt aus der Gruppe bestehend aus

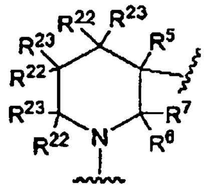 , 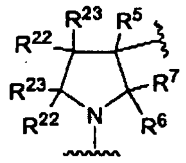 , 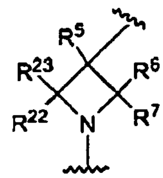 ,

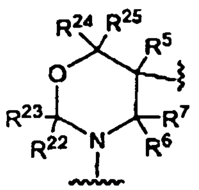 , 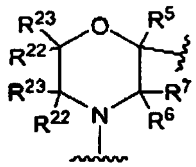 und 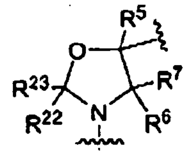

bilden;

oder $R^3$ und $R^8$ zusammen mit der sie verbindenden $-N-CR^4R^5-CR^6CR^7-N-$Gruppe den folgenden Rest

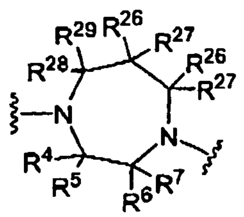

bilden;

oder $R^4$ und $R^6$ zusammen mit der sie verbindenden $-CR^5-CR^7-$Gruppe den folgenden Rest

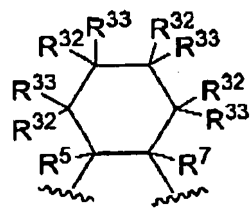

bilden;

oder $R^3$ und $R^6$ zusammen mit der sie verbindenden $-N-CR^4CR^5-CR^7-$Gruppe und $R^4$ und $R^8$ zusammen mit der sie verbindenden $-CR^5-CR^6R^7-N-$Gruppe den folgenden Rest

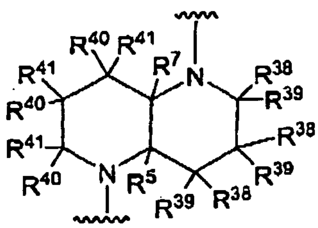

bilden;

oder $R^3$ und $R^8$ zusammen mit der sie verbindenden -N-$CR^4R^5$-$CR^6R^7$-N-Gruppe und $R^4$ und $R^6$ zusammen mit der sie verbindenden -$CR^5$-$CR^7$-Gruppe den folgenden Rest

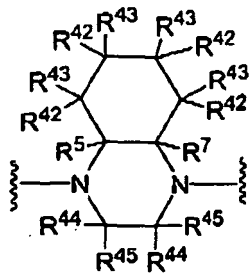

bilden;

$R^9$ für-C(=O)-NH-$R^{64}$; -C(=O)-$NR^{65}R^{66}$;

oder für einen Rest ausgewählt aus der Gruppe bestehend aus Phenyl, Naphthyl, Anthracenyl, Furyl, Thienyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl, Imidazolyl, Indolyl, Benz[b]thiophenyl, Benzo[d]thiazolyl, Benzo[b]furanyl, Chinolinyl, Isochinolinyl und Chinazolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, -C≡C-$Si(CH_3)_3$, -C≡C-$Si(C_2H_5)_3$, -$CH_2$-O-$CH_3$, -$CH_2$-O-$C_2H_5$, -OH, -O-$CH_3$, -O-$C_2H_5$, -O-$C_3H_7$, -S-$CH_3$, -S-$C_2H_5$, -S(=O)-$CH_3$, -$S(=O)_2$-$CH_3$, -S(=O)-$C_2H_5$, -$S(=O)_2$-$C_2H_5$, -$NH_2$, -$N(CH_3)_2$, -$N(C_2H_5)_2$, -NH-$CH_3$, -NH-$C_2H_5$, -$NO_2$, -$CF_3$, -$CH_2F$, -$CHF_2$, -O-$CF_3$, -S-$CF_3$, -SH, -NH-$S(=O)_2$-$CH_3$, - C(=O)-OH, -C(=O)-H; -C(=O)-$CH_3$, -C(=O)-$C_2H_5$, -C(=O)-$NH_2$, -C(=O)-$N(CH_3)_2$, -C(=O)-NH-$CH_3$, -NH-C(=O)-$CH_3$, -NH-C(=O)-$C_2H_5$, -C(=O)-O-$CH_3$ , -C(=O)-O-$C_2H_5$, -C(=O)-O-$C(CH_3)_3$ und Phenyl substituiert ist;

und $R^{55}$, $R^{56}$, $R^{57}$, $R^{58}$; $R^{59}$, $R^{64}$, $R^{65}$, $R^{66}$, $R^{67}$, $R^{68}$, $R^{69}$ und $R^{70}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; oder für einen Phenyl-, Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

**5.** Verbindungen gemäß einem oder mehreren der Ansprüche 1 bis 4, **dadurch gekennzeichnet, dass**

$R^1$ für H; F; Cl; Br; I; -$CF_3$; -$NO_2$; -CN; -C(=O)-OH; -C(=O)-O-$R^{59}$; -C(=O)-$NH_2$; (=O)-NH-$R^{64}$; -C(=O)-$NR^{65}R^{66}$; -O-$R^{67}$; -S-$R^{68}$; -S(=O)-$R^{69}$; -$S(=O)_2$-$R^{70}$; einen Alkyl-Rest ausgewählt aus der Gruppe bestehend aus Methyl, Ethyl, n-Propyl, Isopropyl, n-Butyl, 2-Butyl, Isobutyl und tert-Butyl; oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist, steht;

$R^2$ für H; F; Cl; Br; I; -$CF_3$; -$NO_2$; -CN; -C(=O)-OH; -C(=O)-O-$R^{59}$; -C(=O)-$NH_2$; -C(=O)-NH-$R^{64}$; -C(=O)-$NR^{65}R^{66}$; -O-$R^{67}$; -S-$R^{68}$; -S(=O)-$R^{69}$; -$S(=O)_2$-$R^{70}$; einen Alkyl-Rest ausgewählt aus der Gruppe bestehend aus Methyl,

Ethyl, n-Propyl, Isopropyl, n-Butyl, 2-Butyl, Isobutyl und tert-Butyl; oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist, steht;

oder $R^1$ und $R^2$ zusammen mit den sie verbindenden Kohlenstoffatomen einen Phenylen-Rest bilden, der unsubstituiert oder mit 1, 2, 3 oder 4 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, -O-$CH_3$, -O-$C_2H_5$, -O-$C_3H_7$, -$CF_3$, -$CHF_2$, -$CH_2F$ und -O-$CF_3$ substituiert ist;

$R^3$ und $R^6$, unabhängig voneinander, jeweils für H; -C(=O)-$R^{58}$; -C(=O)-O-$R^{59}$; - S(=O)-$R^{69}$; -S(=O)$_2$-$R^{70}$; einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; für einen Cycloalkylrest ausgewählt aus der Gruppe bestehend aus Cyclopropyl, Cyclobutyl, Cyclopentyl und Cyclohexyl; oder für einen Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist;

$R^4$, $R^5$, $R^6$ und $R^7$, unabhängig voneinander, jeweils für H; F; Cl; Br; I; -$NO_2$;-CN; -$NH_2$; -OH; -SH; -NH-R-$^{55}$; -$NR^{56}R^{57}$; -O-$R^{67}$; -S-$R^{68}$; oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl stehen;

oder $R^4$ und $R^5$ oder $R^6$ und $R^7$, unabhängig voneinander, zusammen jeweils für einen Rest ausgewählt aus der Gruppe bestehend aus einer Oxo-Gruppe (=O) und einer Thioxo-Gruppe (=S) stehen:

oder $R^3$ und $R^4$ zusammen mit der sie verbindenden -N-$CR^5$-Gruppe einen Rest ausgewählt aus der Gruppe bestehend aus

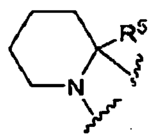 und 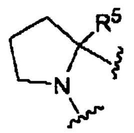

bilden;

oder $R^6$ und $R^8$ zusammen mit der sie verbindenden -N-$CR^7$-Gruppe einen Rest ausgewählt aus der Gruppe bestehend aus

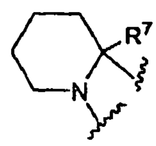 und 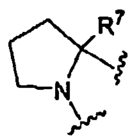

bilden;

oder $R^3$ und $R^6$ zusammen mit der sie verbindenden -N-$CR^4R^5$-$CR^7$-Gruppe einen Rest ausgewählt aus der Gruppe bestehend aus

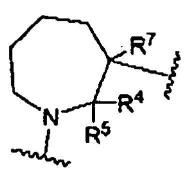 , 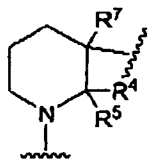 , 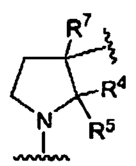 .

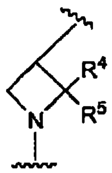, 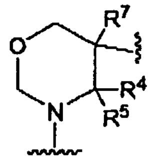, 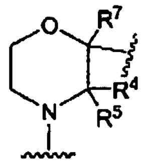 und 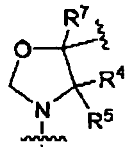

bilden;

oder $R^4$ und $R^8$ zusammen mit der sie verbindenden $-CR^5-CR^5CR^7-N-$Gruppe einen Rest ausgewählt aus der Gruppe bestehend aus

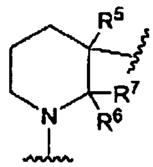, 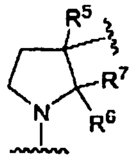, 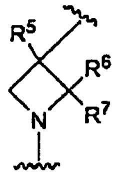,

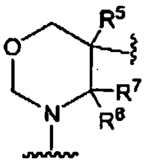, 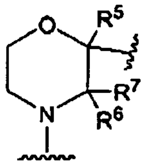 und 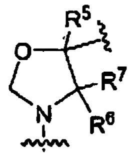

bilden;

oder $R^3$ und $R^8$ zusammen mit der sie verbindenden $-N-CR^4R^5-CR^6CR^7-N-$Gruppe den folgenden Rest

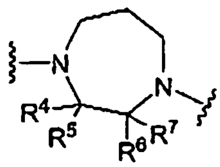

bilden;

oder $R^4$ und $R^6$ zusammen mit der sie verbindenden $-CR^5-CR^7-$Gruppe den folgenden Rest

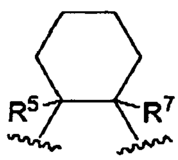

bilden;

oder $R^3$ und $R^6$ zusammen mit der sie verbindenden $-N-CR^4CR^5-CR^7-$Gruppe und $R^4$ und $R^8$ zusammen mit der sie verbindenden $-CR^5-CR^6R^7-N-$Gruppe den folgenden Rest

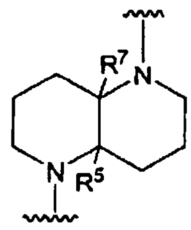

bilden;

oder $R^3$ und $R^8$ zusammen mit der sie verbindenden -N-$CR^4R^5$-$CR^6R^7$-N-Gruppe und $R^4$ und $R^6$ zusammen mit der sie verbindenden -$CR^5$-$CR^7$-Gruppe den folgenden Rest

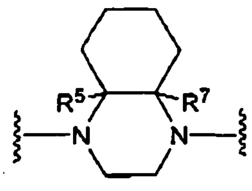

bilden;

$R^9$ für -C(=O)-NH-$R^{64}$; -C(=O)-$NR^{65}R^{66}$; oder für einen Rest ausgewählt aus der Gruppe bestehend aus Phenyl, Naphthyl, Furyl, Thienyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl, Imidazolyl, Indolyl, Benz[b]thiophenyl, Benzo[d]thiazolyl, Benzo[b]furanyl, Chinolinyl, Isochinolinyl und Chinazolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, -O-$CH_3$, -O-$C_2H_5$, - $NO_2$, -$CF_3$, -$CH_2F$, -$CHF_2$, -O-$CF_3$ und -S-$CF_3$ substituiert ist;

und $R^{55}$, $R^{58}$, $R^{57}$, $R^{58}$, $R^{59}$, $R^{64}$, $R^{65}$, $R^{66}$, $R^{67}$, $R^{68}$, $R^{69}$ und $R^{70}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; oder für einen Phenyl-, Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsvefiältnis, oder jeweils in Form entsprechender Salze.

**6.** Verbindungen gemäß einem oder mehreren der Ansprüche 1 bis 5, **dadurch gekennzeichnet, dass**

$R^1$ für H; F; Cl; Br; I; -$CF_3$; -$NO_2$; -CN; -C(=O)-O-$R^{59}$; einen Alkyl-Rest ausgewählt aus der Gruppe bestehend aus Methyl, Ethyl, n-Propyl, Isopropyl, n-Butyl, 2-Butyl, Isobutyl und tert-Butyl; oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl steht;

$R^2$ für H; F; Cl; Br; I; -$CF_3$; -$NO_2$; -CN; -C(=O)-O-$R^{59}$; einen Alkyl-Rest ausgewählt aus der Gruppe bestehend aus Methyl, Ethyl, n-Propyl, Isopropyl, n-Butyl, 2-Butyl, Isobutyl und tert-Butyl; oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl steht;

oder $R^1$ und $R^2$ zusammen mit den sie verbindenden Kohlenstoffatomen einen Phenylen-Rest bilden;

$R^3$ und $R^8$, unabhängig voneinander, jeweils für H; -C(=O)-$R^{58}$; -C(=O)-O-$R^{59}$; - einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; für einen Cycloalkylrest ausgewählt aus der Gruppe bestehend aus Cyclopropyl, Cyclobutyl, Cyclopentyl und Cyclohexyl; oder für einen Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist;

$R^4$, $R^5$, $R^6$ und $R^7$, unabhängig voneinander, jeweils für H; F; Cl; Br, I; -$NO_2$;-CN; -$NH_2$; -OH; -SH; -NH-$R^{55}$;

$-NR^{56}R^{57}$; $-O-R^{67}$; $-S-R^{68}$; oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl stehen;

oder $R^4$ und $R^5$ oder $R^6$ und $R^7$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^3$ und $R^6$ zusammen mit der sie verbindenden $-N-CR^4R^5-CR^7$-Gruppe einen Rest ausgewählt aus der Gruppe bestehend aus

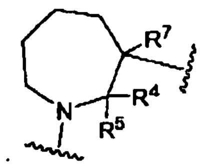 , 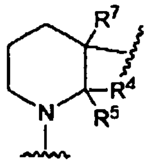 , 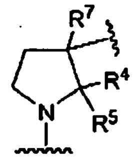 ,

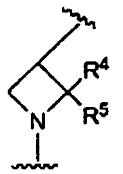 , 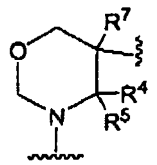 , 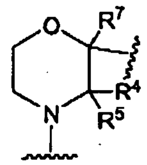 und 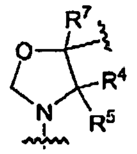

bilden;

oder $R^4$ und $R^8$ zusammen mit der sie verbindenden $-CR^5-CR^6CR^7-N$-Gruppe einen Rest ausgewählt aus der Gruppe bestehend aus

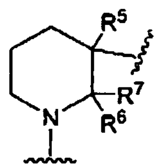 , 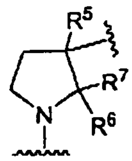 , 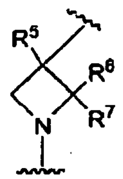 ,

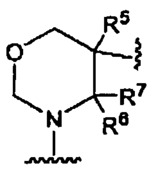 , 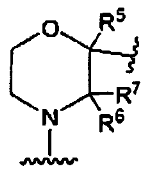 und 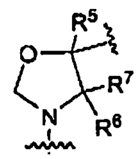

bilden;

oder $R^3$ und $R^8$ zusammen mit der sie verbindenden $-N-CR^4R^5-CR^6CR^7-N$-Gruppe den folgenden Rest

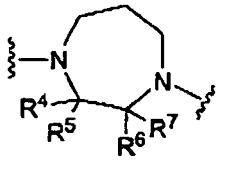

bilden;

oder $R^4$ und $R^6$ zusammen mit der sie verbindenden $-CR^5-CR^7$Gruppe den folgenden Rest

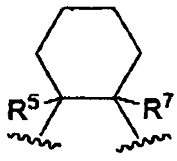

bilden;

$R^9$ für einen Rest ausgewählt aus der Gruppe bestehend aus Phenyl, Furyl, Thienyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl und Imidazolyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, $-O-CH_3$, $-O-C_2H_5$, $- NO_2$, $-CF_3$, $-CH_2F$, $-CHF_2$, $-O-CF_3$ und $-S-CF_3$ substituiert ist;

und $R^{55}$, $R^{58}$, $R^{57}$, $R^{58}$, $R^{59}$, $R^{67}$ und $R^{68}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl stehen;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

**7.** Verbindungen gemäß einem oder mehreren der Ansprüche 1 bis 6, **dadurch gekennzeichnet, dass**

$R^1$ für H, CN, Cl, Br, F, Methyl, Ethyl, n-Propyl, Isobutyl, n-Butyl, tert-Butyl,$-C(=O)-O-CH_3$, $-C(=O)-O-C_2H_5$ oder $-C(=O)-O-C(CH_3)_3$ steht;

$R^2$ für H, CN, Cl, Br, F, Methyl, Ethyl, n-Propyl, Isobutyl, n-Butyl, tert-Butyl,$-C(=O)-O-CH_3$, $-C(=O)-O-C_2H_5$ oder $-C(=O)-O-C(CH_3)_3$ steht;

oder $R^1$ und $R^2$ zusammen mit den sie verbindenden Kohlenstoffatomen einen Phenylen-Rest bilden:

$R^3$ und $R^8$, unabhängig voneinander, jeweils für H; für einen Alkyl-Rest ausgewählt aus der Gruppe bestehend aus Methyl, Ethyl, n-Propyl, Isopropyl, n-Butyl, 2-Butyl, Isobutyl und tert-Butyl; oder für Cyclopropyl; stehen;

$R^4$, $R^5$ und $R^7$, jeweils für H stehen;

$R^6$ für H oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl steht;

oder $R^4$ und $R^5$ oder $R^6$ und $R^7$, unabhängig voneinander, zusammen jeweils für eine Oxo-Gruppe (=O) stehen;

oder $R^3$ und $R^6$ zusammen mit der sie verbindenden $-N-CH_2-CH-$Gruppe einen Rest ausgewählt aus der Gruppe bestehend aus

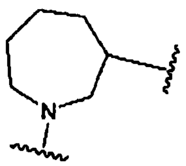 , 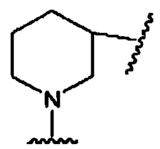 , 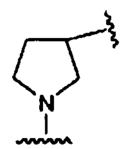 ,

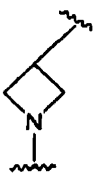 , 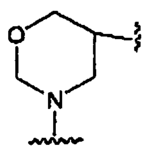 , 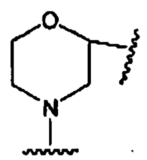 und 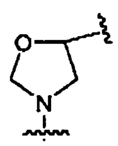

bilden;

oder $R^4$ und $R^8$ zusammen mit der sie verbindenden $-CH-CH_2-N-$Gruppe einen Rest ausgewählt aus der Gruppe bestehend aus

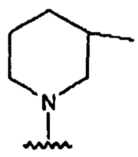 , 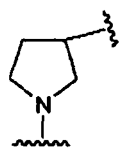 , 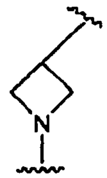 ,

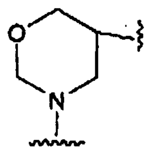 , 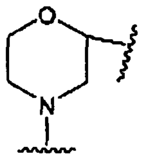 und 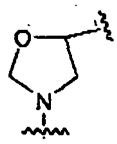

bilden;
oder $R^3$ und $R^8$ zusammen mit der sie verbindenden $-N-CH_2-CH_2-N-$Gruppe den folgenden Rest

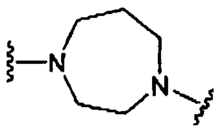

bilden;
oder $R^4$ und $R^6$ zusammen mit der sie verbindenden -CH-CH-Gruppe den folgenden Rest

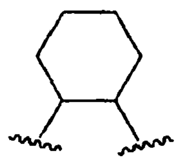

bilden;
und $R^9$ für einen Rest ausgewählt aus der Gruppe bestehend aus Pyrid-2-yl, Pyrid-3-yl, Pyrid-4-yl, Phenyl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Chlor-phenyl, 3-Chlor-phenyl, 3-Chlor-phenyl, 2-Trifluormethyl-phenyl, 3-Trifluormethyl-phenyl, 4-Trifluormethylphenyl, 2-Methoxy-phenyl, 3-Methoxy-phenyl, 4-Methoxy-phenyl, (2,4)-Difluor-phenyl, (2,4)-Dichlor-phenyl, (3,5)-Dichlor-phenyl, (3,5)-Difluor-phenyl, 2-Thiophenyl, 2-Chlor-5-trifluormethyl-phenyl, 3-Fluor-4-methyl-phenyl, 2-Fluor-3-methyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 3-Nitro-phenyl, 3-Ethenyl-phenyl, 3-Ethinyl-phenyl, 3-Allyl-phenyl, 3-Bromphenyl, 2-Trifluormethoxy-phenyl, 3-Trifuormethoxy-phenyl und 4-Trifluormethoxy-phenyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

**8.** Verbindungen gemäß einem oder mehreren der Ansprüche 1 bis 7 ausgewählt aus der Gruppe bestehend aus

[1] 3-Phenyl-N-(1-(thiazol-2-yl)pyrrolidin-3-yl)propiolamid,
[2] N-Methyl-3-phenyl-N-(1-(thiazol-2-yl)pyrrolidin-3-yl)propiolamid Hydrochlorid,
[3] 1 -Thiazol-2-yl-4-(3-phenyl-propiolyl)-1,4-diazepan,
[4] N-Methyl-N-(2-(methyl(thiazol-2-yl)amino)ethyl)-3-phenylpropiolamid,
[5] N-(2-((Thiazol-2-yl)amino)ethyl)-3-phenylpropiolamid,
[6] 3-Phenyl-N-(2-(thiazol-2-ylamino)cyclohexyl)propiolamid,

[7] N-Methyl-N-(2-(methyl(thiazol-2-yl)amino)ethyl)-3-(3-methyl-phenyl)-propiolamid,
[8] 3-Phenyl-N-(1 -(thiazol-2-yl)azetidin-3-yl)propiolamid,
[9] N-(2-(Methyl(thiazol-2-yl)amino)ethyl)-3-phenylpropiolamid,
[10] N-Methyl-N-(2-(thiazol-2-yl)amino)ethyl-3-phenylpropiolamid,
[11] 3-(Thiazol-2-yl-amino)-1-(3-phenyl-propiolyl)pyrrolidin,
[12] N-Methyl-N-(2-(methyl(thiazol-2-yl)amino)cyclohexyl)-3-phenylpropiolamid,
[13] N-Methyl-N-(2-(methyl(thiazol-2-yl)amino)ethyl)-3-(3-cyano-phenyl)-propiolamid,
[14] N-Methyl-N-(2-(methyl(thiazol-2-yl)amino)-2-oxoethyl)-3-phenylpropiolamid,
[15] 3-Phenyl-N-(1-(thiazol-2-yl)piperidin-3-yl)propiolamid,
[16] N-Methyl-N-(1-(thiazol-2-yl)piperidin-3-yl)-3-phenyl-propiolamid,
[17] N-Methyl-N-(2-(methyl(thiazol-2-yl)amino)ethyl)-3-(pyrid-2-yl)-propiolamid,
[18] N-Methyl-N-(2-(methyl(thiazol-2-yl)amino)ethyl)-3-(3-chlor-phenyl)-propiolamid,
[19] N-(2-(Methyl(thiazol-2-yl)amino)ethyl)-3-(3-chlor-phenyl)-propiolamid,
[20] N-Methyl-N-(1-(thiazol-2-yl)azetidin-3-yl)-3-phenyl-propiolamid,
[21] N-(2-(Methyl(thiazol-2-yl)amino)ethyl)-3-(tol-3-yl)-propiolamid,
[22] 3-Methyl(thiazol-2-yl)amino)-1-(3-phenyl-propiolyl)pyrrolidin,
[23] N-(2-(Methyl(thiazol-2-yl)-amino)ethyl)-3-(3-chlo-phenyl)-propiolamid Hydrochlorid,
[24] N-(2-(Benzo[d]thiazol-2-yl(methyl)amino)ethyl)-3-(3-chlor-phenyl)propiolamid,
[25] N-(2-Methyl(5-ethoxycarbonyl-thiazol-2-yl)amino)ethyl)-3-(3-chlor-phenyl)-propiolamid,
[26] N-(2-Methyl(4-ethoxycarbonyl-thiazol-2-yl)amino)ethyl)-3-(3-chlor phenyl)-propiolamid,
[27] 3-(Thiazol-2-yl-amino)-1-(3-phenyl-propiolyl)piperidin,
[28] 3-(Methyl-(thiazol-2-yl)-amino)-1-(3-phenyl-propiolyl)piperidin,
[29] N-(2-(Methyl-(4-methyl-thiazol-2-yl)amino)ethyl)-3-(3-chlor-phenyl)-propiolamid,
[30] 3-(3-Chlorphenyl)-N-(2-(methyl(5-methylthiazol-2-yl)amino)ethyl)propiolamid
[31] N-(2-((5-Bromthiazol-2-yl)(methyl)amino)ethyl)-3-(3-chlorphenyl)propiolamid,
[32] 3-(3-Chlorphenyl)-N-(1-(thiazol-2-yl)piperidin-3-yl)propiolamid,
[33] N-(2-((4-Bromthiazol-2-yl)(methyl)amino)ethyl)-3-(3-chlorphenyl)propiolamid,
[34] Methyl 2-((2-(3-(3-chlorphenyl)propiolamido)ethyl)(methyl)amino)thiazol-5-carboxylat, [35] 1-(3-(3-Chlorphenyl)propiolyl)-3-(methyl(thiazol-2-yl)amino)azetidin,
[36] 3-(3-Chlorphenyl)-N-methyl-N-(1-(thiazol-2-yl)piperidin-3-yl)propiolamid,
[37] 3-(3-Chlorphenyl)-N-(2-((4-chlorthiazol-2-yl)(methyl)amino)ethyl)propiolamid,
[38] 3-(3-Chlorphenyl)-N-(2-((5-chlorthiazol-2-yl)(methyl)amino)ethyl)propiolamid,
[39] 3-(3-Chlorphenyl)-N-(1-(thiazol-2-yl)azetidin-3-yl)propiolamid,
[40] 3-(3-Chlorphenyl)-N-methyl-N-(thiazol-2-yl)azetidin-3-yl)propiolamid,
[41] 3-(3-Chlorphenyl)-N-(2-(ethyl(thiazol-2-yl)amino)ethyl)propiolamid,
[42] 3-(3-Chlorphenyl)-N-(2-((5-cyanothiazol-2-yl)(methyl)amino)ethyl)propiolamid,
[43] 1-(3-(3-Chlorphenyl)propiolyl)-3-(methyl(5-fluor-thiazol-2-yl)amino)azetidin,
[44] 1-(3-(3-Chlorphenyl)propiolyl)-3-(methyl(5-fluor-thiazol-2-yl)amino)pyrrolidin,
[45] N-(1-(4-Methylthiazol-2-yl)pyrrolidin-3-yl)-3-phenylpropiolamid,
[46] 3-(3-Methoxyphenyl)-N-(1-(4-methylthiazol-2-yl)pyrrolidin-3-yl)propiolamid,
[47] 3-(2-Methoxyphenyl)-N-(1-(4-methylthiazol-2-yl)pyrrolidin-3-yl)propiolamid,
[48] 3-(4-Methoxyphenyl)-N-(1-(4-methylthiazol-2-yl)pyrrolidin-3-yl)propiolamid,
[49] 3-(4-Fluorphenyl)-N-(1-(4-methylthiazol-2-yl)pyrrolidin-3-yl)propiolamid,
[50] N-(1-(4-Methylthiazol-2-yl)pyrrolidin-3-yl)-3-p-tolylpropiolamid,
[51] N-(1-(4,5-Dimethylthiazol-2-yl)pyrrolidin-3-yl)-3-phenylpropiolamid,
[52] N-(1-(4,5-Dimethylthiazol-2-yl)pyrrolidin-3-yl)-3-(4-methoxyphenyl)propiolamid,
[53] N-(1-(4,5-Dimethylthiazol-2-yl)pyrrolidin-3-yl)-3-(4-fluorphenyl)propiolamid,
[54] N-(1-(4,5-Dimethylthiazol-2-yl)pyrrolidin-3-yl)-3-(2-methoxyphenyl)propiolamid,
[55] N-(1-(4-tert-Butylthiazol-2-yl)pyrrolidin-3-yl)-3-(4-methoxyphenyl)propiolamid,
[56] N-(1-(4-tert-Butylthiazol-2-yl)pyrrolidin-3-yl)-3-p-tolylpropiolamid,
[57] N-(1-(4-tert-Butylthiazol-2-yl)pyrrolidin-3-yl)-3-(4-fluorphenyl)propiolamid,
[58] N-(1-(4-tert-Butylthiazol-2-yl)pyrrolidin-3-yl)-3-o-tolylpropiolamid,
[59] N-(1-(4-tert-Butylthiazol-2-yl)pyrrolidin-3-yl)-3-(3-fluorphenyl)propiolamid,
[60] N-(1-(4-tert-Butylthiazol-2-yl)pyrrolidin-3-yl)-3-phenylpropiolamid,
[61] N-(1-(4-tert-Butylthiazol-2-yl)pyrrolidin-3-yl)-3-(2-fluorphenyl)propiolamid,
[62] N-(1-(4-tert-Butylthiazol-2-yl)pyrrolidin-3-yl)-3-(3-methoxyphenyl)propiolamid,
[63] N-(1-(4-tert-Butylthiazol-2-yl)pyrrolidin-3-yl)-3-(3-fluor-4-methylphenyl)propiolamid,
[64] N-(1-(4-tert-Butylthiazol-2-yl)pyrrolidin-3-yl)-3-(2-methoxyphenyl)propiolamid,

[65] N-(1-(4-Methylthiazol-2-yl)pyrrolidin-3-yl)-3-(4-(trifluormethyl)phenyl)propiolamid,
[66] N-(1-(4-Methylthiazol-2-yl)pyrrolidin-3-yl)-3-o-tolylpropiolamid,
[67] 3-(3-Fluorphenyl)-N-(1-(4-methylthiazol-2-yl)pyrrolidin-3-yl)propiolamid,
[68] 3-(3-Fluor-4-methylphenyl)-N-(1-(4-methylthiazol-2-yl)pyrrolidin-3-yl)propiolamid,
[69] 3-(2,4-Difluorphenyl)-N-(1-(4-methylthiazol-2-yl)pyrrolidin-3-yl)propiolamid,
[70] 3-(2-Fluorphenyl)-N-(1-(4-methylthiazol-2-yl)pyrrolidin-3-yl)propiolamid,
[71] N-(1-(4-Methylthiazol-2-yl)pyrrolidin-3-yl)-3-(3-(trifluormethyl)phenyl)propiolamid,
[72] N-(1-(4-Methylthiazol-2-yl)pyrrolidin-3-yl)-3-m-tolylpropiolamid,
[73] N-(1-(5-Methylthiazol-2-yl)pyrrolidin-3-yl)-3-p-tolylpropiolamid,
[74] N-(1-(5-Methylthiazol-2-yl)pyrrolidin-3-yl)-3-m-tolylpropiolamid,
[75] 3-(2-Methoxypheny)-N-(1-(5-methylthiazol-2-yl)pyrrolidin-3-yl)propiolamid,
[76] N-(1-(5-Methylthiazol-2-yl)pyrrolidin-3-yl)-3-phenylpropiolamid,
[77] 3-(2-Fluorphenyl)-N-(1-(5-methylthiazol-2-yl)pyrrolidin-3-yl)propiolamid,
[78] 3-(3-Methoxyphenyl)-N-(1-(5-methylthiazol-1-2-yl)pyrrolidin-3-yl)propiolamid,
[79] N-(1-(4,5-Dimethylthiazol-2-yl)pyrrolidin-3-yl)-3-(3-fluor-4-methylphenyl)propiolamid,
[80] N-(1-(4,5-Dimethylthiazol-2-yl)pyrrolidin-3-yl)-3-o-tolylpropiolamid,
[81] 3-(3-Fluorphenyl)-N-(1-(5-methylthiazol-2-yl)pyrrolidin-3-yl)propiolamid,
[82] N-(1-(4,5-Dimethylthiazol-2-yl)pyrrolidin-3-yl)-3-m-tolylpropiolamid,
[83] N-(1-(5-Methylthiazol-2-yl)pyrrolidin-3-yl)-3-o-tolylpropiolamid,
[84] N-(1-(4,5-Dimethylthiazol-2-yl)pyrrolidin-3-yl)-3-p-tolylpropiolamid,
[85] N-(1-(4,5-Dimethylthiazol-2-yl)pyrrolidin-3-yl)-3-(3-(trifluormethyl)phenyt)propiolamid,
[86] 3-(4-Methoxyphenyl)-N-(1-(5-methylthiazol-2-yl)pyrrolidin-3-yl)propiolamid,
[87] N-(1-(5-Methylthiazol-2-yl)pyrrolidin-3-yl)-3-(3-(trifluormethyl)phenyl)propiolamid,
[88] N-(1-(4,5-Dimethylthiazol-2-yl)pyrrolidin-3-yl)-3-(4-(trifluormethyl)phenyl)propiolamid,
[89] 3-(4-Fluorphenyl)-N-(1-(5-methylthiazol-2-yl)pyrrolidin-3-yl)propiolamid,
[90] N-(1-(4,5-Dimethylthiazol-2-yl)pyrrolidin-3-yl)-3-(3-methoxyphenyl)propiolamid,
[91] 3-(2,4-Difluorphenyl)-N-(1-(4,5-dimethylthiazol-2-yl)pyrrolidin-3-yl)propiolamid,
[92] N-(1-(4,5-Dimethylthiazol-2-yl)pyrrolidin-3-yl)-3-(2-fluorphenyl)propiolamid,
[93] N-(1-(5-Methylthiazol-2-yl)pyrrolidin-3-yl)-3-(4-(trifluormethyl)phenyl)propiolamid,
[94] 3-(3-Fluor-4-methylphenyl)-N-(1-(5-methylthiazol-2-yl)pyrrolidin-3-yl)propiolamid,
[95] 3-(2,4-Difluorphenyl)-N-(1-(5-methylthiazol-2-yl)pyrrolidin-3-yl)propiolamid,
[96] N-(1-(4-tert-Butylthiazol-2-yl)pyrrolidin-3-yl)-3-(2,4-difluorphenyl)propiolamid,
[97] N-(1-(4-tert-Butylthiazol-2-yl)pyrrolidin-3-yl)-3-(4-(trifluormethyl)phenyl)propiolamid,
[98] N-(1-(4-tert-Butylthiazol-2-yl)pyrrolidin-3-yl)-3-(3-(trifluormethyl)phenyl)propiolamid,
[99] N-(1-(4-tert-Nutylthiazol-2-yl)pyrrolidin-3-yl)-3-m-tolylpropiolamid,
[100] N-(1-(4-tert-Butylthiazol-2-yl)pyrrolidin-3-yl)-3-(3-chlorphenyl)propiolamid,
[101] N-(1-(4-tert-Butylthiazol-2-yl)pyrrolidin-3-yl)-3(thiophen-2-yl)propiolamid,
[102] N-(1-(4-tert-Butylthiazol-2-yl)pyrrolidin-3-yl)-3-(2,4-dichlorphenyl)propiolamid,
[103] N-(1-(4-tert-Butylthiazol-2-yl)pyrrolidin-3-yl)-3-(2-chlor-5-(trifluormethyl)phenyl)propiolamid,
[104] N-(1-(4-tert-Butylthiazol-2-yl)pyrrolidin-3-yl)-3-(3,5-dichlorphenyl)propiolamid,
[105] 3-(3-Chlorphenyl)-N-(1-(4-methylthiazol-2-yl)pyrrolidin-3-yl)propiolamid,
[106] N-(1-(4-Methylthiazol-2-yl)pyrrolidin-3-yl)-3-(thiophen-2-yl)propiolamid,
[107] 3-(2,4-Dichlorphenyl)-N-(1-(4-methylthiazol-2-yl)pyrrolidin-3-yl)propiolamid,
[108] 3-(2-Chlor-5-(trifluormethyl)phenyl)-N-(1-(4-methylthiazol-2-yl)pyrrolidin-3-yl)propiolamid,
[109] 3-(3,5-Dichlorphenyl)-N-(1-(4-methylthiazol-2-yl)pyrrolidin-3-yl)propiolamid,
[110] 3-(3-Chlorphenyl)-N-(1-(4,5-dimethylthiazol-2-yl)pyrmlidin-3. yl)propiolamid,
[111] 3-(2,4-Dichlorphenyl)-N-(1-(4,5-dimethylthiazol-2-yl)pyrrolidin-3-yl)propiolamid,
[112] 3-(3-Chlorphenyl)-N-(1-(5-methylthiazol-2-yl)pyrrolidin-3-yl)propiolamid,
[113] N-(1-(5-Methylthiazol-2-yl)pyrrolidin-3-yl)-3-(thiophen-2-yl)propiolamid,
[114] 3-(2,4-Dichlorphenyl)-N-(1-(5-methylthiazol-2-yl)pyrrolidin-3-yl)propiolamid,
[115] 3-(2-Chlor-5-(trifluormethyl)phenyl)-N-(1-(5-methylthiazol-2-yl)pyrrolidin-3-yl)propiolamid,
[116] 3-(3,5-Dichlorphenyl)-N-(1-(5-methylthiazol-2-yl)pyrrolidin-3-yl)propiolamid,
[117] N-(1-(4,5-Dimethylthiazol-2-yl)pyrrolidin-3-yl)-3-(thiophen-2-yl)propiolamid,
[118] 3-(2-Chlor-5-(trifluormethyl)phenyl)-N-(1-(4,5-dimethylthiazol-2-yl)pyrrolidin-3-yl)propiolamid,
[119] N-(1-(5-Methylthiazol-2-yl)pyrrolidin-3-yl)-3-(pyridin-2-yl)propiolamid,
[120] N-(1-(5-Methylthiazol-2-yl)pyrrolidin-3-yl)-3-(pyridin-3-yl)propiolamid,
[121] N-(1-(5-Methylthiazol-2-yl)pyrrolidin-3-yl)-3-(pyridin-4-yl)propiolamid,
[122] N)-(1-(4-tert-Butylthiazol-2-yl)pyrrolidin-3-yl)-3-(pyridin-2-yl)propiolamid,

[123] N-(1-(4-tert-Butylthiazol-2-yl)pyrrolidin-3-yl)-3-(pyridin-3-yl)propiolamid und
[126] 3-(3-Chlorphenyl)-N-(2-((5-fluorthiazol-2-yl)(methyl)amino)ethyl)propiolamid;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze.

9. Verfahren zur Herstellung von Verbindungen der allgemeinen Formel I gemäß einem oder mehreren der Ansprüche 1 bis 8, **dadurch gekennzeichnet, dass** wenigstens eine Verbindung der allgemeinen Formel II,

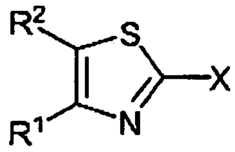

II,

worin die Reste $R^1$ und $R^2$ die Bedeutung gemäß einem oder mehreren der Ansprüche 1 bis 8 haben und X für eine Abgangsgruppe, vorzugsweise für einen Halogen-Rest oder einen Sulfonsäureester, besonders bevorzugt für einen Chlor- oder Brom-Rest, steht, mit wenigstens einer Verbindung der allgemeinen Formel III,

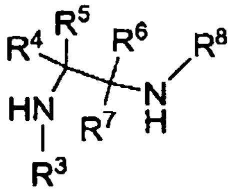

III,

worin $R^3$, $R^4$, $R^5$, $R^6$, $R^7$ und $R^8$ die Bedeutung gemäß einem oder mehreren der Ansprüche 1 bis 8 haben, ggf. in einem Reaktionsmedium, ggf. in Gegenwart wenigstens einer Base und/oder wenigstens einer metallorganischen Verbindung und/oder wenigstens eines Metallhydrid-Reagenzes oder In Gegenwart wenigstens eines Kupfersalzes und ggf. in Gegenwart wenigstens eines Metalls, vorzugsweise bei einer Temperatur von - 70 °C bis 300 °C, besonders bevorzugt von - 70 °C bis 150 °C, in wenigstens eine entsprechende Verbindung der allgemeinen Formel IV, ggf. in Form eines entsprechenden Salzes,

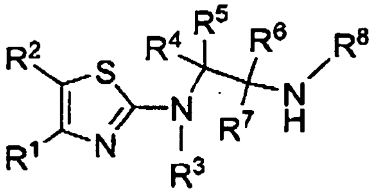

IV,

worin $R^1$, $R^2$, $R^3$, $R^4$, $R^5$, $R^6$, $R^7$ und $R^8$ die vorstehend genannte Bedeutung haben, überführt wird und diese ggf. gereinigt und/oder isoliert wird;
oder wenigstens eine Verbindung der allgemeinen Formel II mit wenigstens einer Verbindung der allgemeinen Formel V,

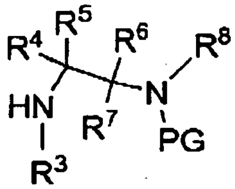

V,

worin $R^3$, $R^4$, $R^5$, $R^6$, $R^7$ und $R^8$ die Bedeutung gemäß einem oder mehreren der Ansprüche 1 bis 8 haben und PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht,
ggf. in einem Reaktionsmedium, ggf. in Gegenwart wenigstens einer Base und/oder wenigstens einer metallorganischen Verbindung und/oder wenigstens eines Metallhydrid-Reagenzes, vorzugsweise bei einer Temperatur von- 70°C bis 300 °C in wenigstens eine entsprechende Verbindung der allgemeinen Formel VI,

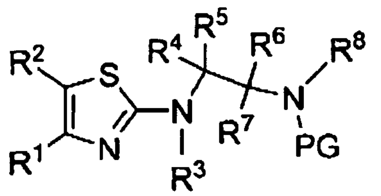

VI,

worin $R^1$, $R^2$, $R^3$, $R^4$, $R^5$, $R^6$, $R^7$, $R^8$ und PG die vorstehend genannte Bedeutung haben, überführt wird und diese ggf. gereinigt und/oder isoliert wird;
und ggf. wenigstens eine Verbindung der allgemeinen Formel VI, worin $R^1$, $R^2$, $R^4$, $R^5$, $R^6$, $R^7$, $R^8$ und PG die vorstehend genannte Bedeutung haben und $R^3$ für Wasserstoff steht, mit wenigstens einer Verbindung $R^3$-X, worin $R^3$ die vorstehend genannte Bedeutung mit Ausnahme von Wasserstoff hat und X für eine Abgangsgruppe, vorzugsweise für einen Halogen-Rest steht, ggf. in einem Reaktionsmedium, ggf. in Gegenwart wenigstens einer Base, bevorzugt in Gegenwart wenigstens eines Metallhydrid-Reagenzes, besonders bevorzugt in Gegenwart von Natriumhydrid, vorzugsweise bei einer Temperatur von- 70 °C bis 300°C in wenigstens eine entsprechende Verbindung der allgemeinen Formel VIa,

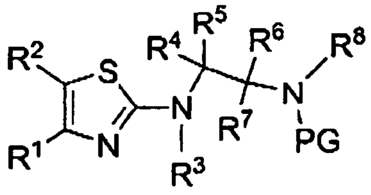

VIa,

worin $R^1$, $R^2$, $R^3$, $R^4$, $R^5$, $R^6$, $R^7$, $R^8$ und PG die vorstehend genannte Bedeutung haben und $R^3$ nicht für ein Wasserstoffatom steht, überführt wird und diese ggf. gereinigt und/oder isoliert wird;
oder wenigstens eine Verbindung der allgemeinen Formel XIII,

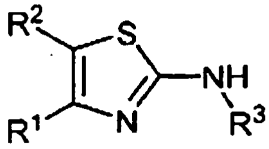

XIII,

worin $R^1$, $R^2$ und $R^3$ die Bedeutung gemäß einem oder mehreren der Ansprüche 1 bis 8 haben, mit wenigstens einer Verbindung der allgemeinen Formel XIV,

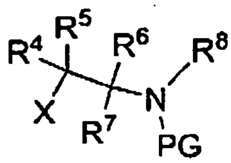

XIV,

worin $R^4$, $R^5$, $R^6$, $R^7$, $R^8$ und PG die vorstehend genannte Bedeutung haben und X für eine Abgangsgruppe, vorzugsweise für einen Halogen-Rest oder für einen Sulfonsäureester, besonders bevorzugt für einen Chlor- oder Brom-Rest steht, ggf. in einem Reaktionsmedium, ggf. in Gegenwart wenigstens einer Base, vorzugsweise in Gegenwart wenigstens einer Base ausgewählt aus der Gruppe bestehend aus Kalium-tert-butylat, Natriumhydroxid, Kaliumhydroxid, Dimethylamin und Triethylamin, besonders bevorzugt in Gegenwart von Diethylamin, oder ggf. in Gegenwart wenigstens einer metallorganischen Verbindung, bevorzugt in Gegenwart wenigstens einer metallorganischen Verbindung ausgewählt aus der Gruppe bestehend aus Methyllithium und Butyllithium oder ggf. in Gegenwart wenigstens einer Metallhydridverbindung, besonders bevorzugt in Gegenwart von Natriumhydrid, vorzugsweise bei einer Temperatur von- 70 °C bis 300 °C, besonders bevorzugt von - 70 °C bis 150 °C, in wenigstens eine entsprechende Verbindung der allgemeinen Formel VI überführt wird und diese ggf. gereinigt und/oder isoliert wird;
oder wenigstens eine Verbindung der allgemeinen Formel XIII,

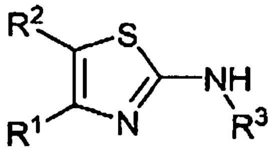

XIII,

worin $R^1$, $R^2$ und $R^3$ die Bedeutung gemäß einem oder mehreren der Ansprüche 1 bis 8 haben, mit wenigstens einer Verbindung der allgemeinen Formel XIVa,

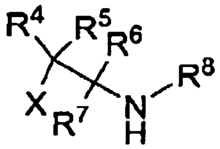

XIVa,

worin $R^4$, $R^5$, $R^6$, $R^7$ und $R^8$ die vorstehend genannte Bedeutung haben und X für eine Abgangsgruppe, vorzugsweise für einen Halogen-Rest oder für einen Sulfonsäureester, besonders bevorzugt für einen Chlor- oder Brom-Rest steht, ggf. in einem Reaktionsmedium, ggf. in Gegenwart wenigstens einer Base, vorzugsweise in Gegenwart wenigstens einer Base ausgewählt aus der Gruppe bestehend aus Kallum-tert-butylat, Natriumhydroxid, Kaliumhydroxid, Dimethylamin und Triethylamin, besonders bevorzugt in Gegenwart von Diethylamin, oder ggf. in Gegenwart wenigstens einer metallorganischen Verbindung, bevorzugt in Gegenwart wenigstens einer metallorganischen Verbindung ausgewählt aus der Gruppe bestehend aus Methyllithium und Butyllithium oder ggf. in Gegenwart wenigstens einer Metallhydridverbindung, besonders bevorzugt in Gegenwart von Natriumhydrid, vorzugsweise bei einer Temperatur von - 70 °C bis 300 °C, besonders bevorzugt von - 70 °C bis 150 °C, in wenigstens eine entsprechende Verbindung der allgemeinen Formel IV überführt wird und diese ggf. gereinigt und/oder isoliert wird;
oder wenigstens eine Verbindung der allgemeinen Formel VII,

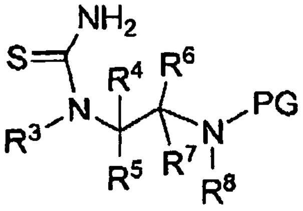

VII,

worin $R^3$, $R^4$, $R^5$, $R^6$, $R^7$ und $R^8$ die Bedeutung gemäß einem oder mehreren der Ansprüche 1 bis 8 haben und PG für eine Schutzgruppe, bevorzugt für eine Schutzgruppe ausgewählt aus der Gruppe bestehend aus tert-Butyloxycarbonyl, Benzyl, Benzyloxycarbonyl und 9-Fluorenylmethyloxycarbonyl steht, durch Umsetzung mit wenigstens einer Verbindung der allgemeinen Formel $R^1$-C(=O)-$CH_2$-X oder ($C_{1-5}$-Alkyl-O)$_2$-CH-$CH_2$-X, worin $R^1$ die Bedeutung gemäß einem oder mehreren der Ansprüche 1 bis 8 hat und X für eine Abgangsgruppe, vorzugsweise für einen Halogen-Rest, besonders bevorzugt für ein Brom-Atom steht, in einem Reaktionsmedium, ggf. in Gegenwart wenigstens einer organischen Base oder in Gegenwart wenigstens einer Säure, vorzugsweise in Gegenwart wenigstens einer Base ausgewählt aus der Gruppe bestehend aus Triethylamin, Diisopropylethylamin, N-Methylmorpholin, Dimethylaminopyridin und Pyridin oder in Gegenwart wenigstens einer Säure ausgewählt aus der Gruppe bestehend aus Essigsäure, Trifluoressigsäure und Salzsäure, vorzugsweise bei einer Temperatur zwischen - 70 °C bis 300 °C in wenigstens eine entsprechende Verbindung der allgemeinen Formel VI, ggf. in Form eines entsprechenden Salzes überführt wird, und diese ggf. gereinigt und/oder isoliert wird;
und wenigstens eine Verbindung der allgemeinen Formel VI oder der allgemeinen Formel VIa für den Fall, dass PG für eine tert-Butoxycarbonyl- oder 9-Fluorenylmethyloxycarbonyl-Gruppe steht, in einem Reaktionsmedium, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100°C oder für den Fall, dass PG für eine Benzyl-Gruppe oder Benzyloxycarbonyl-Gruppe steht, in einem Reaktionsmedium, in Gegenwart von Wasserstoff und in Gegenwart wenigstens eines Katalysators, vorzugsweise in Gegenwart von Palladium auf Kohle, vorzugsweise bei einer Temperatur zwischen -70 °C bis 100 °C in wenigstens eine entsprechende Verbindung der allgemeinen Formel IV, ggf. in Form eines entsprechenden Salzes überführt wird, und diese ggf. gereinigt und/oder isoliert wird;
und wenigstens eine Verbindung der allgemeinen Formel IV durch Umsetzung mit wenigstens einer Verbindung der allgemeinen Formel $R^9$-C≡C-C(=O)-OH, worin $R^9$ die Bedeutung gemäß einem oder mehreren der Ansprüche 1 bis 8 hat, in einem Reaktionsmedium, ggf. in Gegenwart wenigstens eines geeigneten Kopplungsmittels, das polymergebunden sein kann, ggf. in Gegenwart wenigstens einer Base, vorzugsweise bei einer Temperatur von - 70 °C bis 100°C, oder durch Umsetzung mit wenigstens einer Verbindung der allgemeinen Formel $R^9$-C≡C-C(=O)-X, worin $R^9$ die vorstehend genannte Bedeutung hat und X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel I, ggf. in Form eines entsprechenden Salzes überführt wird,

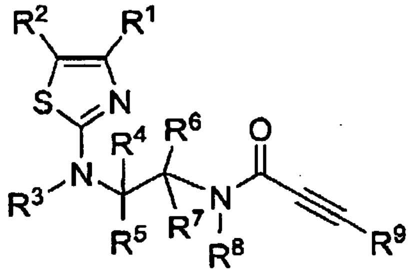

I,

worin $R^1$, $R^2$, $R^3$, $R^4$, $R^5$, $R^6$, $R^7$, $R^8$ und $R^9$ die vorstehend genannte Bedeutung haben, und diese ggf. gereinigt und/oder isoliert wird;
oder wenigstens eine Verbindung der allgemeinen Formel IV durch Umsetzung mit Propiolsäure [HC≡C-C(=O)-OH] in einem Reaktionsmedium, ggf. in Gegenwart wenigstens eines geeigneten Kopplungsmittels, das polymergebunden sein kann, ggf. in Gegenwart wenigstens einer Base, vorzugsweise bei einer Temperatur von - 70 °C bis 100 °C, oder durch Umsetzung mit wenigstens einer Verbindung der allgemeinen Formel HC≡C-C(=O)-X, worin X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel VIII, ggf. in Form eines entsprechenden Salzes überführt wird,

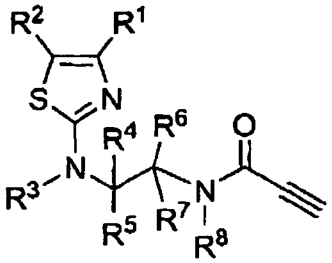

VIII,

worin $R^1$. $R^2$, $R^3$, $R^4$, $R^5$, $R^6$, $R^7$ und $R^8$ die vorstehend genannte Bedeutung haben, und diese ggf. gereinigt und/oder isoliert wird,
und wenigstens eine Verbindung der allgemeinen Formel VIII durch Umsetzung mit wenigstens einer Verbindung der allgemeinen Formel $R^9$-X, worin $R^9$ die Bedeutung gemäß einem oder mehreren der Ansprüche 1 bis 8 mit Ausnahme von Wasserstoff hat und X für eine Abgangsgruppe, vorzugsweise für einen Halogen-Rest oder einen Sulfonsäureester, besonders bevorzugt für Iod, Brom oder Triflat, steht, in einem Reaktionsmedium, ggf. in Gegenwart wenigstens eines Katalysators, bevorzugt in Gegenwart wenigstens eines Palladium-Katalysators ausgewählt aus der Gruppe bestehend aus Palladiumchlorid [$PdCl_2$], Palladiumacetat [$Pd(OAc)_2$]. Tetrakistriphenylphosphinpalladium [$Pd(PPh_3)_4$],
Bistriphenylphosphinpalladiumdichlorid [$Pd(PPh_3)_2Cl_2$] und Bistriphenylphosphinpalladiumacetat [$Pd(PPh_3)_2(OAc)_2$], ggf. in Gegenwart wenigstens eines Liganden, bevorzugt in Gegenwart wenigstens eines Liganden ausgewählt aus der Gruppe bestehend aus Triphenylphosphin, Triphenylarsin und Tri-2-furyl-phosphin, ggf. in Gegenwart wenigstens eines anorganischen Salzes, bevorzugt in Gegenwart wenigstens eines anorganischen Salzes ausgewählt aus der Gruppe bestehend aus Lithiumchlorid und Zinkchlorid, ggf. in Gegenwart wenigstens eines Kupfersalzes, bevorzugt in Gegenwart von Kupferiodid, ggf. in Gegenwart wenigstens einer organischen oder anorganischen Base, vorzugsweise in Gegenwart wenigstens einer Base ausgewählt aus der Gruppe bestehend aus Triethylamin, [1,4]-Diazabicyclo-[2.2.2]-octan, Diisopropylamin, Diisopropylethylamin, Kaliumcarbonat und Natriumhydrogencarbonat, vorzugsweise bei einer Temperatur zwischen -70°C bis 300 °C in wenigstens eine entsprechende Verbindung der allgemeinen Formel I, ggf. in Form eines entsprechenden Salzes überführt wird, und diese ggf. gereinigt und/oder isoliert wird.

**10.** Verfahren zur Herstellung von Verbindungen der allgemeinen Formel I gemäß einem oder mehreren der Ansprüche 1 bis 8, **dadurch gekennzeichnet, dass** wenigstens eine Verbindung der allgemeinen Formel III,

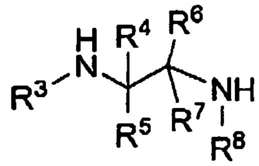

III,

worin $R^3$, $R^4$, $R^5$, $R^6$, $R^7$ und $R^8$ die Bedeutung gemäß einem oder mehreren der Ansprüche 1 bis 8 haben, durch Umsetzung mit wenigstens einer Verbindung der allgemeinen Formel $R^9$-C≡C-C(=O)-OH, worin $R^9$ die Bedeutung gemäß einem oder mehreren der Ansprüche 1 bis 8 hat, in einem Reaktionsmedium, ggf. in Gegenwart wenigstens eines geeigneten Kopplungsmittels, das polymergebunden sein kann, ggf. in Gegenwart wenigstens einer Base, vorzugsweise bei einer Temperatur von - 70°C bis 100 °C, oder durch Umsetzung mit wenigstens einer Verbindung der allgemeinen Formel $R^9$-C≡C-C(=O)-X, worin $R^9$ die vorstehend genannte Bedeutung hat und X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel IX, ggf. in Form eines entsprechenden Salzes,

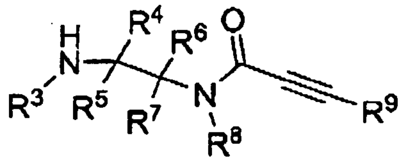

IX,

worin $R^3$, $R^4$, $R^5$. $R^6$, $R^7$. $R^8$ und $R^9$ die vorstehend genannte Bedeutung haben, überführt wird und diese ggf. gereinigt und/oder isoliert wird;
oder wenigstens eine Verbindung der allgemeinen Formel V,

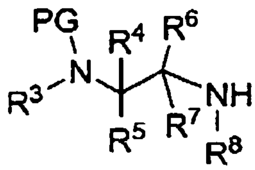

V,

worin $R^3$, $R^4$, $R^5$, $R^6$, $R^7$ und $R^8$ die Bedeutung gemäß einem oder mehreren der Ansprüche 1 bis 8 haben und PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, durch Umsetzung mit wenigstens einer Verbindung der allgemeinen Formel $R^9$-C≡C-C(=O)-OH, worin $R^9$ die Bedeutung gemäß einem oder mehreren der Ansprüche 1 bis 8 hat, in einem Reaktionsmedium, ggf. in Gegenwart wenigstens eines geeigneten Kopplungsmittels, das polymergebunden sein kann, ggf. in Gegenwart wenigstens einer Base, vorzugsweise bei einer Temperatur von - 70 °C bis 100 °C, oder durch Umsetzung mit wenigstens einer Ver-

bindung der allgemeinen Formel $R^9$-C≡C-C(=O)-X, worin $R^9$ die vorstehend genannte Bedeutung hat und X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel XI. ggf. in Form eines entsprechenden Salzes,

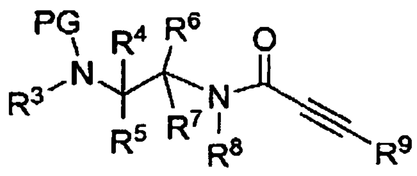

XI,

worin $R^3$, $R^4$, $R^5$, $R^6$ $R^7$, $R^8$, $R^9$ und PG die vorstehend genannte Bedeutung haben, überführt wird und diese ggf. gereinigt und/oder isoliert wird;
und wenigstens eine Verbindung der allgemeinen Formel XI für den Fall, dass PG für eine tert-Butoxycarbonyl- oder 9-Fluorenylmethyloxycarbonyl-Gruppe steht, in einem Reaktionsmedium, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100 °C oder für den Fall, dass PG für eine Benzyl- oder Benzyloxycarbonyl-Gruppe steht, in einem Reaktionsmedium, in Gegenwart von Wasserstoff und in Gegenwart wenigstens eines Katalysators, vorzugsweise in Gegenwart von Palladium auf Kohle, vorzugsweise bei einer Temperatur zwischen -70 °C bis 100°C in wenigstens eine entsprechende Verbindung der allgemeinen Formel IX, ggf. in Form eines entsprechenden Salzes überführt wird, und diese ggf. gereinigt und/oder isoliert wird;
und wenigstens eine Verbindung der allgemeinen Formel IX durch Umsetzung mit wenigstens einer Verbindung der allgemeinen Formel II,

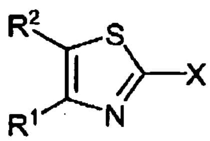

II,

worin die Reste $R^1$ und $R^2$ die Bedeutung gemäß einem oder mehreren der Ansprüche 1 bis 8 haben und X für eine Abgangsgruppe, vorzugsweise für einen Halogen-Rest oder einen Sulfonsäureester, besonders bevorzugt für einen Chlor- oder Brom-Rest, steht, in einem Reaktionsmedium, ggf. in Gegenwart wenigstens einer Base und/oder wenigstens einer metallorganischen Verbindung und/oder wenigstens eines Metallhydrid-Reagenzes, vorzugsweise bei einer Temperatur von - 70 °C bis 300 °C in wenigstens eine entsprechende Verbindung der allgemeinen Formel I, ggf. in Form eines entsprechenden Salzes überführt wird, worin $R^1$, $R^2$, $R^3$, $R^4$, $R^5$, $R^6$, $R^7$, $R^8$ und $R^9$ die vorstehend genannte Bedeutung haben, und diese ggf. gereinigt und/oder isoliert wird;
oder ggf. wenigstens eine Verbindung der allgemeinen Formel IX durch Umsetzung mit Kaliumthiocyanat und Ethylchloroformiat oder Ammoniumthiocyanat oder Trimethylsilylisothiocyanat oder Thiophosgen und Ammoniak oder Bromcyan und Schwefelwasserstoff in einem Reaktionsmedium, ggf. in Gegenwart wenigstens einer Säure, vorzugsweise in Gegenwart wenigstens einer Säure ausgewählt aus der Gruppe bestehend aus Salzsäure, Schwefelsäure, Essigsäure und Trifluoressigsäure, besonders bevorzugt in Gegenwart von Salzsäure, oder ggf. in Gegenwart wenigstens einer Base, vorzugsweise in Gegenwart wenigstens einer Base ausgewählt aus der Gruppe bestehend aus Kalium-tert-butylat, Natriumhydroxid, Kaliumhydroxid, Dimethylamin und Triethylamin, besonders bevorzugt in Gegenwart von Diethylamin, oder ggf. in Gegenwart wenigstens einer metallorganischen Verbindung, bevorzugt in Gegenwart wenigstens einer metallorganischen Verbindung ausgewählt aus der Gruppe bestehend aus Methyllithium und Butyllithium oder ggf. in Gegenwart wenigstens einer Metallhydridverbindung, besonders bevorzugt in Gegenwart von Natriumhydrid, vorzugsweise bei einer Temperatur von - 70 °C bis 250 °C, in wenigstens eine entsprechende Verbindung der allgemeinen Formel XII, ggf. in Form eines entsprechenden Salzes,

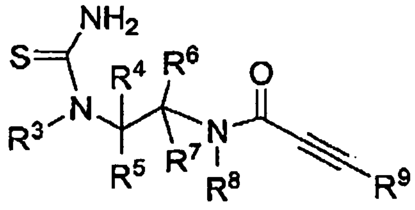

XII,

worin $R^3$, $R^4$, $R^5$, $R^6$, $R^7$, $R^8$ und $R^9$ die vorstehend genannte Bedeutung haben, überführt wird und diese ggf. gereinigt und/oder isoliert wird;
und wenigstens eine Verbindung der allgemeinen Formel XII durch Umsetzung mit wenigstens einer Verbindung der allgemeinen Formel $R^1$-C(=O)-$CH_2$-X oder ($C_{1-5}$-Alkyl-O)$_2$-CH-$CH_2$-X, worin $R^1$ die Bedeutung gemäß einem oder mehreren der Ansprüche 1 bis 8 hat und X für eine Abgangsgruppe, vorzugsweise für einen Halogen-Rest, besonders bevorzugt für ein Brom-Atom steht, in einem Reaktionsmedium, ggf. in Gegenwart wenigstens einer organischen Base oder in Gegenwart wenigstens einer Säure, vorzugsweise in Gegenwart wenigstens einer Base ausgewählt aus der Gruppe bestehend aus Triethylamin, Diisopropylethylamin, N-Methylmorpholin, Dimethylaminopyridin und Pyridin oder in Gegenwart wenigstens einer Säure ausgewählt aus der Gruppe bestehend aus Essigsäure, Trifluoressigsäure und Salzsäure, vorzugsweise bei einer Temperatur zwischen - 70 °C bis 300 °C in wenigstens eine entsprechende Verbindung der allgemeinen Formel I, ggf. in Form eines entsprechenden Salzes überführt wird, und diese ggf. gereinigt und/oder isoliert wird.

**11.** Arzneimittel enthaltend wenigstens eine Verbindung gemäß einem oder mehreren der Ansprüche 1 bis 8 und ggf. einen oder mehrere physiologisch verträgliche Hilfsstoff.

**12.** Verwendung wenigstens einer Verbindung gemäß einem oder mehreren der Ansprüche 1 bis 8 zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt des Aufmerksamkeit-Defizit-Syndroms (ADS); Angstzuständen; Panikattacken; Epilepsie; Husten; Harninkontinenz; Diarrhöe; Pruritus; Schizophrenie; cerebralen Ischämien; Muskelspasmen; Krämpfen; Lungenkrankheiten, vorzugsweise ausgewählt aus der Gruppe bestehend aus Asthma und Pseudokrupp; Regurgitation (Erbrechen); Schlaganfall; Dyskinesie: Retinopathie; Antriebslosigkeit; Kehlkopfentzündung (Laryngitis); Störungen der Nahrungsaufnahme, vorzugsweise ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-)abhängigkeit; Toleranzentwicklung gegenüber Medikamenten, insbesondere gegenüber natürlichen oder synthetischen Opioiden; Magen-Ösaphagus-Reflux-Syndrom; gastroösophageale Refluxkrankheit; Reizdarmsyndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

**13.** Verwendung gemäß Anspruch 12 zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Angstzuständen; Panikattacken; Alkoholabhängigkeit; Medikamentenabhängigkeit; Störungen der Nahrungsaufnahme, vorzugsweise ausgewählt aus der Gruppe bestehend aus Bulimie, Kachexie, Anorexie und Fettleibigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen-(insbesondere Nikotin- und/oder Kokain-)abhängigkeit; Toleranzentwicklung gegenüber Medikamenten und/oder Drogen, insbesondere gegenüber natürlichen oder synthetischen Opioiden; Magen-Ösaphagus-Reflux-Syndrom, gastroösophageale

Refluxkrankheit und Reizdarmsyndrom.

**14.** Verwendung gemäß Anspruch 12 oder 13 zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz und visceralem Schmerz.

**15.** Verwendung gemäß Anspruch 12 oder 13 zur Herstellung eines Arzneimittels zur Behandlung von Angstzuständen oder Panikattacken.

## Claims

**1.** Substituted thiazoles of the general formula I,

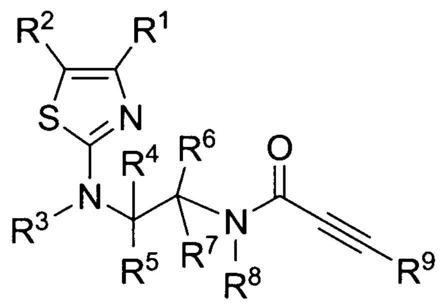

I,

in which

$R^1$ and $R^2$, mutually independently, in each case denote H; F; Cl; Br; I; $-NO_2$; -CN; $-NH_2$; -OH; -SH; -C(=O)-OH; -C(=O)-H; -NH-C(=O)-H; $-NH-R^{55}$; $-NR^{56}R^{57}$; $-C(=O)-R^{58}$; $-C(=O)-O-R^{59}$; $-O-C(=O)-R^{60}$; $-NH-C(=O)-R^{61}$; $-NR^{62}-C(=O)-R^{63}$; $-C(=O)-NH_2$; $-C(=O)-NH-R^{64}$; $-C(=O)-NR^{65}R^{66}$; $-O-R^{67}$; $-S-R^{68}$; $-S(=O)-R^{69}$; $-S(=O)_2-R^{70}$; $-NH-C(=O)-NH-R^{71}$; $-NH-C(=S)-NH-R^{72}$; -NH-S(=O)2-$R^{73}$; $-NR^{74}-S(=O)_2-R^{75}$; unsubstituted or at least monosubstituted alkyl, alkenyl or alkynyl; unsubstituted or at least monosubstituted heteroalkyl, heteroalkenyl or heteroalkynyl; unsubstituted or at least monosubstituted cycloalkyl or cycloalkenyl; unsubstituted or at least monosubstituted heterocycloalkyl or heterocycloalkenyl; unsubstituted or at least monosubstituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or at least monosubstituted -(heteroalkylene)-cycloalkyl, -(heteroalkenylene)-cycloalkyl, -(heteroalkylene)-cycloalkenyl or -(heteroalkenylene)-cycloalkenyl; unsubstituted or at least monosubstituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or at least monosubstituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or at least monosubstituted aryl; unsubstituted or at least monosubstituted heteroaryl; unsubstituted or at least monosubstituted -(alkylene)-aryl, -(alkenylene)-aryl, -(alkynylene)-aryl, -(heteroalkylene)-aryl or -(heteroalkenylene)-aryl; or unsubstituted or at least monosubstituted -(alkylene)-heteroaryl, -(alkenylene)-heteroaryl, -(alkynylene)-heteroaryl, -(heteroalkylene)-heteroaryl or -(heteroalkenylene)-heteroaryl;
or $R^1$ and $R^2$ together with the carbon atoms joining them form an unsubstituted or at least monosubstituted phenylene residue;
$R^3$, $R^8$ and $R^{54}$, mutually independently, in each case denote H; $-C(=O)-R^{58}$; $-C(=O)-O-R^{59}$; $-C(=O)-NH_2$; $-C(=O)-NH-R^{64}$; $-C(=O)-NR^{65}R^{66}$; $-S(=O)-R^{69}$; $-S(=O)_2-R^{70}$; unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or substituted -(heteroalkylene)-cycloalkyl,

-(heteroalkenylene)-cycloalkyl, -(heteroalkylene)-cycloalkenyl or -(heteroalkenylene)-cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or substituted aryl; unsubstituted or substituted heteroaryl; unsubstituted or substituted -(alkylene)-aryl, -(alkenylene)-aryl, -(alkynylene)-aryl, -(heteroalkylene)-aryl or -(heteroalkenylene)-aryl; or unsubstituted or substituted -(alkylene)-heteroaryl,- (alkenylene)-heteroaryl, -(alkynylene)-heteroaryl, -(heteroalkylene)-heteroaryl or -(heteroalkenylene)-heteroaryl;

$R^4$, $R^5$, $R^6$, $R^7$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$ and $R^{53}$, mutually independently, in each case denote H; F; Cl; Br; I; $-NO_2$; -CN; $-NH_2$; -OH; -SH; -C(=O)-OH; -C(=O)-H; -NH-C(=O)-H; -NH-$R^{55}$; -N$R^{56}R^{57}$; -C(=O)-$R^{58}$; -C(=O)-O-$R^{59}$; -O-C(=O)-$R^{60}$; -NH-C(=O)-$R^{61}$; -N$R^{62}$-C(=O)-$R^{63}$; -C(=O)-$NH_2$; -C(=O)-NH-$R^{64}$; -C(=O)-N$R^{65}R^{66}$; -O-$R^{67}$; -S-$R^{68}$; -S(=O)-$R^{69}$; $-S(=O)_2$-$R^{70}$; -NH-C(=O)-NH-$R^{71}$; -NH-C(=S)-NH-$R^{72}$; $-NH-S(=O)_2$-$R^{73}$; -N$R^{74}$-$S(=O)_2$-$R^{75}$; unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or substituted -(heteroalkylene)-cycloalkyl, -(heteroalkenylene)-cycloalkyl, -(heteroalkylene)-cycloalkenyl or -(heteroalkenylene)-cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or substituted aryl; unsubstituted or substituted heteroaryl; unsubstituted or substituted -(alkylene)-aryl, -(alkenylene)-aryl, -(alkynylene)-aryl, -(heteroalkylene)-aryl or -(heteroalkenylene)-aryl; or unsubstituted or substituted -(alkylene)-heteroaryl, -(alkenylene)-heteroaryl, -(alkynylene)-heteroaryl, -(heteroalkylene)-heteroaryl or -(heteroalkenylene)-heteroaryl;

or $R^4$ and $R^5$ or $R^6$ and $R^7$ or $R^{10}$ and $R^{11}$ or $R^{12}$ and $R^{13}$ or $R^{14}$ and $R^{15}$ or $R^{16}$ and $R^{17}$ or $R^{18}$ and $R^{19}$ or $R^{20}$ and $R^{21}$ or $R^{22}$ and $R^{23}$ or $R^{24}$ and $R^{25}$ or $R^{26}$ and $R^{27}$ or $R^{28}$ and $R^{29}$ or $R^{30}$ and $R^{31}$ or $R^{32}$ and $R^{33}$ or $R^{34}$ and $R^{35}$ or $R^{36}$ and $R^{37}$ or $R^{38}$ and $R^{39}$ or $R^{40}$ and $R^{41}$ or $R^{42}$ and $R^{43}$ or $R^{44}$ and $R^{45}$ or $R^{46}$ and $R^{47}$ or $R^{48}$ and $R^{49}$ or $R^{50}$ and $R^{51}$ or $R^{52}$ and $R^{53}$, mutually independently, together in each case denote a residue selected from the group consisting of an oxo group (=O) and a thioxo group (=S);

or $R^3$ and $R^4$ together with the -N-C$R^5$ group joining them form a residue of the general formula A,

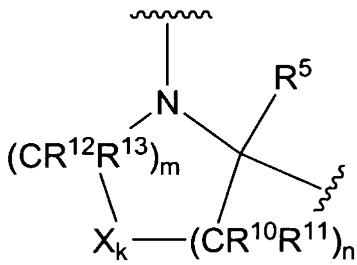

A,

or $R^6$ and $R^8$ together with the -N-C$R^7$ group joining them form a residue of the general formula B,

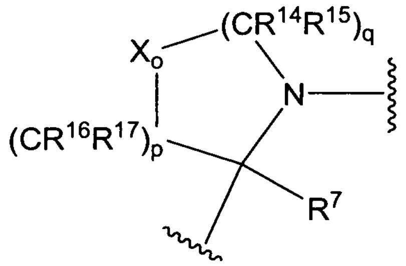

B,

m and q in each case denote 1, 2, 3, 4 or 5;
n and p in each case denote 0, 1, 2, 3 or 4;
k and o in each case denote 0 or 1;
wherein the sum of m, n and k or the sum of p, q and o is in each case equal to 1, 2, 3, 4, 5 or 6;
or $R^3$ and $R^6$ together with the -N-$CR^4R$ 5-$CR^7$ group joining them form a residue of the general formula C,

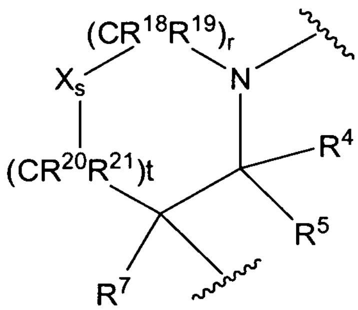

C,

or $R^4$ and $R^8$ together with the -$CR^5$-$CR^6R^7$-N group joining them form a residue of the general formula D,

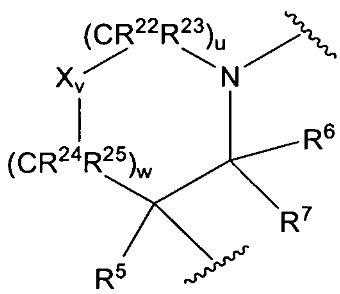

D,

r and u in each case denote 1, 2, 3 or 4;
t and w in each case denote 0, 1, 2 or 3;
s and v in each case denote 0 or 1;
wherein the sum of r, s and t or the sum of u, v and w is in each case equal to 1, 2, 3, 4 or 5;
or $R^3$ and $R^8$ together with the -N-$CR^4R^5$-$CR^6R^7$-N group joining them form a residue of the general formula E,

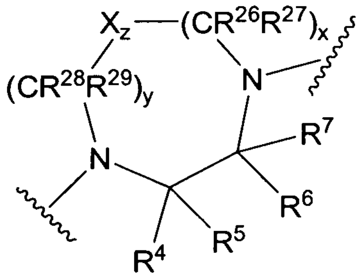

E,

x and y in each case denote 1 or 2;

z denotes 0 or 1; wherein the sum of x, y and z is equal to 3 or 4;
or $R^4$ and $R^6$ together with the $-CR^5-CR^7$ group joining them form a residue of the general formula F,

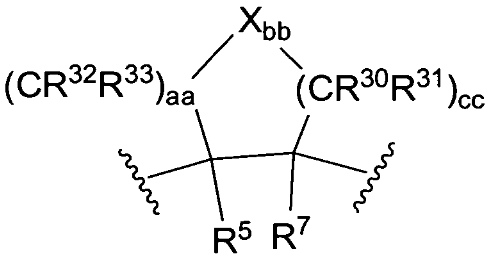

F,

aa and cc, mutually independently, in each case denote 1, 2, 3, 4 or 5; bb denotes 0 or 1;
wherein the sum of aa, bb and cc is equal to 1, 2, 3, 4, 5 or 6;
or $R^3$ and $R^4$ together with the $-N-CR^5$ group joining them and $R^6$ and $R^8$ together with the $-N-CR^7$ group joining them form a residue of the general formula G,

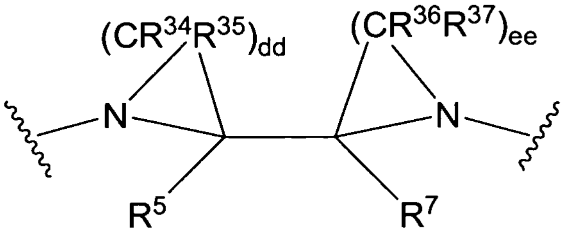

G,

dd and ee, mutually independently, in each case denote 1, 2, 3 or 4;
or $R^3$ and $R^6$ together with the $-N-CR^4CR^5-CR^7$ group joining them and $R^4$ and $R^8$ together with the $-CR^5-CR^6R^7-N$ group joining them form a residue of the general formula H,

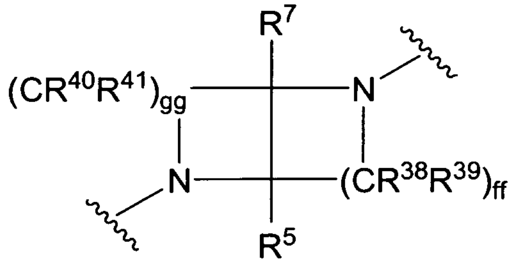

H,

ff and gg, mutually independently, in each case denote 1, 2 or 3;
or $R^3$ and $R^8$ together with the -N-$CR^4R^5$-$CR^6R^7$-N group joining them and $R^4$ and $R^6$ together with the -$CR^5$-$CR^7$ group joining them form a residue of the general formula K,

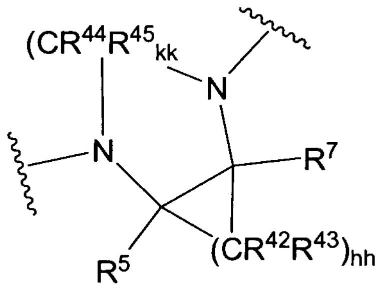

K

hh denotes 1, 2, 3 or 4;
kk denotes 1, 2, 3, 4, 5 or 6;
or $R^3$ and $R^8$ together with the -N-$CR^4R^5$-$CR^6R^7$-N group joining them form a bicyclic residue of the general formula L,

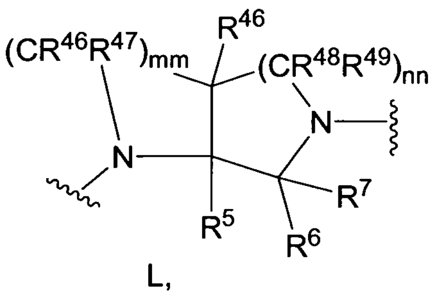

L,

mm denotes 1, 2 or 3;
nn denotes 1 or 2;
or $R^3$ and $R^8$ together with the -N-$CR^4R^5$-$CR^6R^7$-N group joining them form a bicyclic residue of the general formula M,

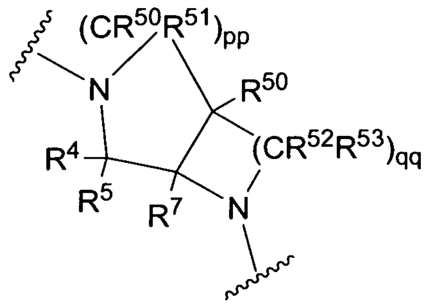

M,

pp denotes 1 or 2;
qq denotes 1, 2 or 3;
X denotes O, S or N-$R^{54}$;
$R^9$ denotes H; F; Cl; Br; I; -CN; -C(=O)-OH; -C(=O)-H; -C(=O)-$R^{58}$; -C(=O)-O-$R^{59}$; -C(=O)-$NH_2$; -C(=O)-NH-$R^{64}$; -C(=O)-$NR^{65}R^{66}$; unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or substituted -(heteroalkylene)-cycloalkyl, -(heteroalkenylene)-cycloalkyl, -(heteroalkylene)-cycloalkenyl or -(heteroalkenylene)-cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl; or -(heteroalkenylene)-heterocycloalkenyl; unsubstituted or substituted aryl; unsubstituted or substituted heteroaryl; unsubstituted or substituted -(alkylene)-aryl, -(alkenylene)-aryl, -(alkynylene)-aryl, -(heteroalkylene)-aryl or -(heteroalkenylene)-aryl; or unsubstituted or substituted -(alkylene)-heteroaryl, -(alkenylene)-heteroaryl, -(alkynylene)-heteroaryl, -(heteroalkylene)-heteroaryl or -(heteroalkenylene)-heteroaryl;
and $R^{55}$, $R^{56}$, $R^{57}$, $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$ and $R^{75}$, mutually independently, in each case denote unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or substituted -(heteroalkylene)-cycloalkyl, -(heteroalkenylene)-cycloalkyl, -(heteroalkylene)-cycloalkenyl or -(heteroalkenylene)-cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl; or -(heteroalkenylene)-heterocycloalkenyl; unsubstituted or substituted aryl; unsubstituted or substituted heteroaryl; unsubstituted or substituted -(alkylene)-aryl, -(alkenylene)-aryl, -(alkynylene)-aryl, -(heteroalkylene)-aryl or -(heteroalkenylene)-aryl; or unsubstituted or substituted -(alkylene)-heteroaryl, -(alkenylene)-heteroaryl, -(alkynylene)-heteroaryl, -(heteroalkylene)-heteroaryl or -(heteroalkenylene)-heteroaryl;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts.

**2.** Compounds according to claim 1, **characterised in that**

$R^1$ and $R^2$, mutually independently, in each case denote H; F; Cl; Br; I; -$NO_2$; -CN; -$NH_2$; -OH; -SH; -C(=O)-OH; -C(=O)-H; -NH-C(=O)-H; -NH-$R^{55}$; -$NR^{56}R^{57}$; -C(=O)-$R^{58}$; -C(=O)-O-$R^{59}$; -O-C(=O)-$R^{60}$; -NH-C(=O)-$R^{61}$; -$NR^{62}$-C(=O)-$R^{63}$; -C(=O)-$NH_2$; -C(=O)-NH-$R^{64}$; -C(=O)-$NR^{65}R^{66}$; -O-$R^{67}$; -S-$R^{68}$; -S(=O)-$R^{69}$; -S$(=O)_2$-$R^{70}$; -NH-C(=O)-NH-$R^{71}$; -NH-C(=S)-NH-$R^{72}$; -NH-S$(=O)_2$-$R^{73}$; -$NR^{74}$-S(=O)2-$R^{75}$; unsubstituted or at least monosubstituted alkyl, alkenyl or alkynyl; unsubstituted or at least monosubstituted heteroalkyl, heteroalkenyl or

heteroalkynyl; unsubstituted or at least monosubstituted cycloalkyl or cycloalkenyl; unsubstituted or at least monosubstituted heterocycloalkyl or heterocycloalkenyl; unsubstituted or at least monosubstituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or at least monosubstituted -(heteroalkylene)-cycloalkyl, -(heteroalkenylene)-cycloalkyl, -(heteroalkylene)-cycloalkenyl or -(heteroalkenylene)-cycloalkenyl; unsubstituted or at least monosubstituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or at least monosubstituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or at least monosubstituted aryl; unsubstituted or at least monosubstituted heteroaryl; unsubstituted or at least monosubstituted -(alkylene)-aryl, -(alkenylene)-aryl, -(alkynylene)-aryl, -(heteroalkylene)-aryl or -(heteroalkenylene)-aryl; or unsubstituted or at least monosubstituted -(alkylene)-heteroaryl, -(alkenylene)-heteroaryl, -(alkynylene)-heteroaryl, -(heteroalkylene)-heteroaryl or -(heteroalkenylene)-heteroaryl;

or $R^1$ and $R^2$ together with the carbon atoms joining them form an unsubstituted or at least monosubstituted phenylene residue;

$R^3$, $R^8$ and $R^{54}$, mutually independently, in each case denote H; -C(=O)-$R^{58}$; -C(=O)-O-$R^{59}$; -C(=O)-$NH_2$; -C(=O)-NH-$R^{64}$; -C(=O)-N$R^{65}R^{66}$; -S(=O)-$R^{69}$; -$S(=O)_2$-$R^{70}$; unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or substituted -(heteroalkylene)-cycloalkyl, -(heteroalkenylene)-cycloalkyl, -(heteroalkylene)-cycloalkenyl or -(heteroalkenylene)-cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or substituted aryl; unsubstituted or substituted heteroaryl; unsubstituted or substituted -(alkylene)-aryl, -(alkenylene)-aryl, -(alkynylene)-aryl, -(heteroalkylene)-aryl or -(heteroalkenylene)-aryl; or unsubstituted or substituted -(alkylene)-heteroaryl, -(alkenylene)-heteroaryl, -(alkynylene)-heteroaryl, -(heteroalkylene)-heteroaryl or -(heteroalkenylene)-heteroaryl;

$R^4$,$R^5$,$R^6$,$R^7R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$ and $R^{53}$, mutually independently, in each case denote H; F; Cl; Br; I; -$NO_2$; -CN; -$NH_2$; -OH; -SH; -C(=O)-OH; -C(=O)-H; -NH-C(=O)-H; -NH-$R^{55}$; -N$R^{56}R^{57}$; -C(=O)-$R^{58}$; -C(=O)-O-$R^{59}$; -O-C(=O)-$R^{60}$; -NH-C(=O)-$R^{61}$; -N$R^{62}$-C(=O)-$R^{63}$; -C(=O)-$NH_2$; -C(=O)-NH-$R^{64}$; -C(=O)-N$R^{65}R^{66}$; -O-$R^{67}$; -S-$R^{68}$; -S(=O)-$R^{69}$; -$S(=O)_2$-$R^{70}$; -NH-C(=O)-NH-$R^{71}$; -NH-C(=S)-NH-$R^{72}$; -NH-$S(=O)_2$-$R^{73}$; -N$R^{74}$-$S(=O)_2$-$R^{75}$; unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or substituted -(heteroalkylene)-cycloalkyl, -(heteroalkenylene)-cycloalkyl, -(heteroalkylene)-cycloalkenyl or -(heteroalkenylene)-cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or substituted aryl; unsubstituted or substituted heteroaryl; unsubstituted or substituted -(alkylene)-aryl, -(alkenylene)-aryl, -(alkynylene)-aryl, -(heteroalkylene)-aryl or -(heteroalkenylene)-aryl; or unsubstituted or substituted -(alkylene)-heteroaryl, -(alkenylene)-heteroaryl, -(alkynylene)-heteroaryl, -(heteroalkylene)-heteroaryl or -(heteroalkenylene)-heteroaryl;

or $R^4$ and $R^5$ or $R^6$ and $R^7$ or $R^{10}$ and $R^{11}$ or $R^{12}$ and $R^{13}$ or $R^{14}$ and $R^{15}$ or $R^{16}$ and $R^{17}$ or $R^{18}$ and $R^{19}$ or $R^{20}$ and $R^{21}$ or $R^{22}$ and $R^{23}$ or $R^{24}$ and $R^{25}$ or $R^{26}$ and $R^{27}$ or $R^{28}$ and $R^{29}$ or $R^{30}$ and $R^{31}$ or $R^{32}$ and $R^{33}$ or $R^{34}$ and $R^{35}$ or $R^{36}$ and $R^{37}$ or $R^{38}$ and $R^{39}$ or $R^{40}$ and $R^{41}$ or $R^{42}$ and $R^{43}$ or $R^{44}$ and $R^{45}$ or $R^{46}$ and $R^{47}$ or $R^{48}$ and $R^{49}$ or $R^{50}$ and $R^{51}$ or $R^{52}$ and $R^{53}$, mutually independently, together in each case denote a residue selected from the group consisting of an oxo group (=O) and a thioxo group (=S);

or $R^3$ and $R^4$ together with the -N-C$R^5$ group joining them form a residue of the general formula A,

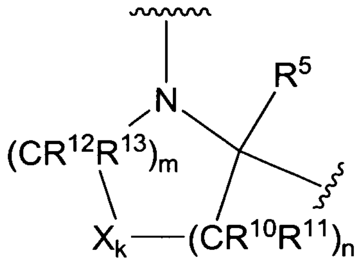

A,

or $R^6$ and $R^8$ together with the -N-$CR^7$ group joining them form a residue of the general formula B,

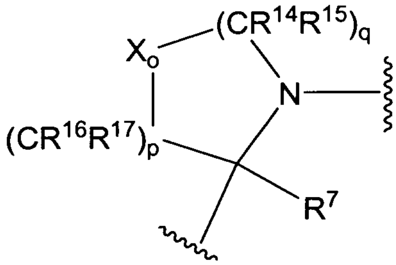

B,

m and q in each case denote 1, 2, 3, 4 or 5;

n and p in each case denote 0, 1, 2, 3 or 4;
k and o in each case denote 0 or 1;
wherein the sum of m, n and k or the sum of p, q and o is in each case equal to 1, 2, 3, 4, 5 or 6;
or $R^3$ and $R^6$ together with the -N-$CR^4R^5$-$CR^7$ group joining them form a residue of the general formula C,

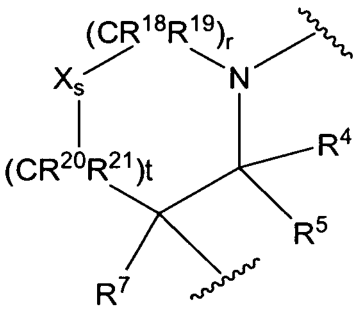

C,

or $R^4$ and $R^8$ together with the -$CR^5$-$CR^6R^7$-N group joining them form a residue of the general formula D,

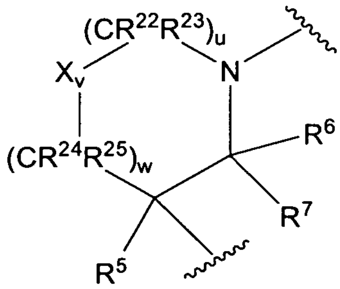

D,

r and u in each case denote 1, 2, 3 or 4;
t and w in each case denote 0, 1, 2 or 3;
s and v in each case denote 0 or 1;
wherein the sum of r, s and t or the sum of u, v and w is in each case equal to 1, 2, 3, 4 or 5;
or $R^3$ and $R^8$ together with the -N-$CR^4R^5$-$CR^6R^7$-N group joining them form a residue of the general formula E,

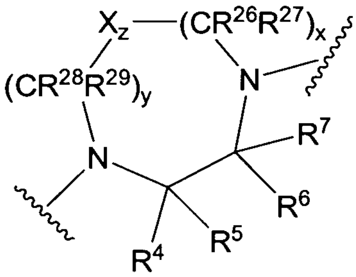

E,

x and y in each case denote 1 or 2;
z denotes 0 or 1;
wherein the sum of x, y and z is equal to 3 or 4;
or $R^4$ and $R^6$ together with the -$CR^5$-$CR^7$ group joining them form a residue of the general formula F,

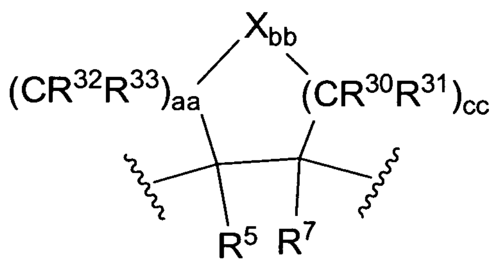

F,

aa and cc, mutually independently, in each case denote 1, 2, 3, 4 or 5; bb denotes 0 or 1;
wherein the sum of aa, bb and cc is equal to 1, 2, 3, 4, 5 or 6;
or $R^3$ and $R^4$ together with the -N-$CR^5$ group joining them and $R^6$ and $R^8$ together with the -N-$CR^7$ group joining them form a residue of the general formula G,

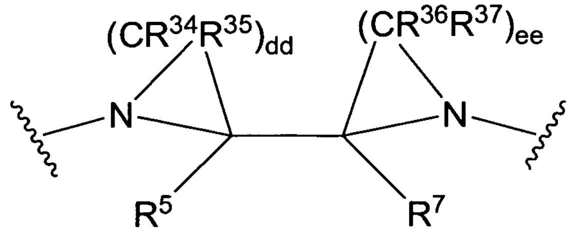

G,

dd and ee, mutually independently, in each case denote 1, 2, 3 or 4;
or $R^3$ and $R^6$ together with the -N-$CR^4CR^5$-$CR^7$ group joining them and $R^4$ and $R^8$ together with the -$CR^5$-$CR^6R^7$-N group joining them form a residue of the general formula H,

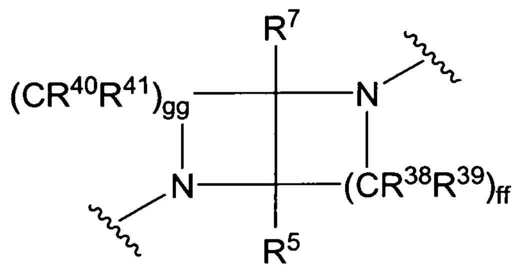

H,

ff and gg, mutually independently, in each case denote 1, 2 or 3;
or $R^3$ and $R^8$ together with the -N-$CR^4R^5$-$CR^6R^7$-N group joining them and $R^4$ and $R^6$ together with the -$CR^5$-$CR^7$ group joining them form a residue of the general formula K,

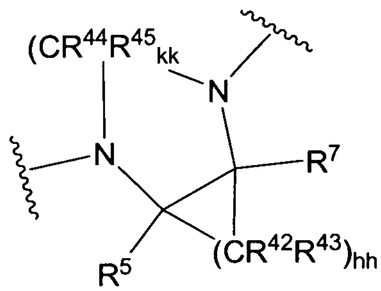

K,

hh denotes 1, 2, 3 or 4;
kk denotes 1, 2, 3, 4, 5 or 6;
or $R^3$ and $R^8$ together with the -N-$CR^4R^5$-$CR^6R^7$-N group joining them form a bicyclic residue of the general formula L,

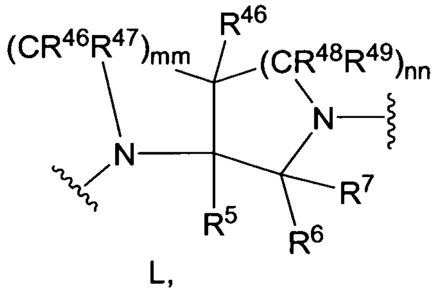

L,

mm denotes 1, 2 or 3;
nn denotes 1 or 2;
or $R^3$ and $R^8$ together with the -N-$CR^4R^5$-$CR^6R^7$-N group joining them form a bicyclic residue of the general formula M,

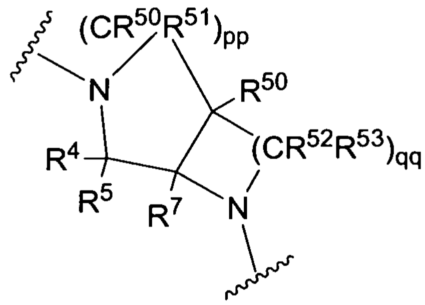

M,

pp denotes 1 or 2;
qq denotes 1, 2 or 3;
X denotes O, S or N-$R^{54}$;
$R^9$ denotes H; F; Cl; Br; I; -CN; -C(=O)-OH; -C(=O)-H; -C(=O)-$R^{58}$; -C(=O)-O-$R^{59}$; -C(=O)-$NH_2$; -C(=O)-NH-$R^{64}$; -C(=O)-$NR^{65}R^{66}$; unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or substituted -(heteroalkylene)-cycloalkyl, -(heteroalkenylene)-cycloalkyl, -(heteroalkylene)-cycloalkenyl or -(heteroalkenylene)-cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl; or -(heteroalkenylene)-heterocycloalkenyl; unsubstituted or substituted aryl; unsubstituted or substituted heteroaryl; unsubstituted or substituted -(alkylene)-aryl, -(alkenylene)-aryl, -(alkynylene)-aryl, -(heteroalkylene)-aryl or -(heteroalkenylene)-aryl; or unsubstituted or substituted -(alkylene)-heteroaryl, -(alkenylene)-heteroaryl, -(alkynylene)-heteroaryl, -(heteroalkylene)-heteroaryl or -(heteroalkenylene)-heteroaryl;
and $R^{55}$, $R^{56}$, $R^{57}$, $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$ and $R^{75}$, mutually independently, in each case denote unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or substituted -(heteroalkylene)-cycloalkyl, -(heteroalkenylene)-cycloalkyl, -(heteroalkylene)-cycloalkenyl or -(heteroalkenylene)-cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted

-(heteroalkylene)-heterocycloalkyl, -(heteroalkenylene)-heterocycloalkyl, -(heteroalkylene)-heterocycloalkenyl; or -(heteroalkenylene)-heterocycloalkenyl; unsubstituted or substituted aryl; unsubstituted or substituted heteroaryl; unsubstituted or substituted -(alkylene)-aryl, -(alkenylene)-aryl, -(alkynylene)-aryl, -(heteroalkylene)-aryl or -(heteroalkenylene)-aryl; or unsubstituted or substituted -(alkylene)-heteroaryl, -(alkenylene)-heteroaryl, -(alkynylene)-heteroaryl, -(heteroalkylene)-heteroaryl or -(heteroalkenylene)-heteroaryl;

wherein

the above-stated alkyl residues are in each case branched or straight-chain and comprise 1, 2, 3, 4, 5, 6, 7, 8, 9, 10, 11 or 12 carbon atoms as chain links;

the above-stated alkenyl residues are in each case branched or straight-chain and comprise 2, 3, 4, 5, 6, 7, 8, 9, 10, 11 or 12 carbon atoms as chain links;

the above-stated alkynyl residues are in each case branched or straight-chain and comprise 2, 3, 4, 5, 6, 7, 8, 9, 10, 11 or 12 carbon atoms as chain links;

the above-stated heteroalkyl residues, heteroalkenyl residues and heteroalkynyl residues are in each case 2-, 3-, 4-, 5-, 6-, 7-, 8-, 9-, 10-, 11- or 12-membered;

the above-stated heteroalkyl residues, heteroalkenyl residues and heteroalkynyl residues in each case optionally comprise 1, 2 or 3 heteroatom(s) mutually independently selected from the group consisting of oxygen, sulfur and nitrogen (NH) as chain link(s);

the above-stated alkyl residues, alkenyl residues, alkynyl residues, heteroalkyl residues, heteroalkenyl residues and heteroalkynyl residues may be substituted in each case with optionally 1, 2, 3, 4 or 5 substituents mutually independently selected from the group consisting of F, Cl, Br, I, $-NO_2$, -CN, -OH, -SH, $-NH_2$, $-N(C_{1-5}\text{-alkyl})_2$, $-N(C_{1-5}\text{-alkyl})$(phenyl), $-N(C_{1-5}\text{-alkyl})(CH_2\text{-phenyl})$, $-N(C_{1-5}\text{-alkyl})(CH_2\text{-}CH_2\text{-phenyl})$, -C(=O)-H, $-C(=O)-C_{1-5}\text{-alkyl}$, -C(=O)-phenyl, $-C(=S)-C_{1-5}\text{-alkyl}$, -C(=S)-phenyl, -C(=O)-OH, $-C(=O)-O-C_{1-5}\text{-alkyl}$, -C(=O)-O-phenyl, $-C(=O)-NH_2$, $-C(=O)-NH-C_{1-5}\text{-alkyl}$, $-C(=O)-N(C_{1-5}\text{-alkyl})_2$, $-S(=O)-C_{1-5}\text{-alkyl}$, -S(=O)-phenyl, $-S(=O)_2-C_{1-5}\text{-alkyl}$, $-S(=O)_2\text{-phenyl}$, $-S(=O)_2-NH_2$ and $-SO_3H$, wherein the phenyl residues may be substituted with 1, 2, 3, 4 or 5 substituents mutually independently selected from the group consisting of F, Cl, Br, I, -CN, $-CF_3$, -OH, $-NH_2$, $-O-CF_3$, -SH, $-O-CH_3$, $-O-C_2H_5$, $-O-C_3H_7$, methyl, ethyl, n-propyl, isopropyl, n-butyl, 2-butyl, isobutyl and *tert*.-butyl;

the above-stated cycloalkyl residues in each case comprise 3, 4, 5, 6, 7, 8 or 9 carbon atoms as ring members;

the above-stated cycloalkenyl residues in each case comprise 3, 4, 5, 6, 7, 8 or 9 carbon atoms as ring members;

the above-stated heterocycloalkyl residues are in each case 3-, 4-, 5-, 6-, 7-, 8- or 9-membered;

the above-stated heterocycloalkenyl residues are in each case 4-, 5-, 6-, 7-, 8- or 9-membered;

the above-stated heterocycloalkyl residues and heterocycloalkenyl residues in each case optionally comprise 1, 2 or 3 heteroatom(s) mutually independently selected from the group consisting of oxygen, sulfur and nitrogen (NH) as ring member(s);

the above-stated cycloalkyl residues, heterocycloalkyl residues, cycloalkenyl residues or heterocycloalkenyl residues may be substituted in each case with optionally 1, 2, 3, 4 or 5 substituents mutually independently selected from the group consisting of F, Cl, Br, I, -CN, $-CF_3$, -OH, $-NH_2$, $-O-CF_3$, -SH, $-O-C_{1-5}\text{-alkyl}$, -O-phenyl, $-O-CH_2\text{-phenyl}$, $-(CH_2)-O-C_{1-5}\text{-alkyl}$, $-S-C_{1-5}\text{-alkyl}$, -S-phenyl, $-S-CH_2\text{-phenyl}$, $-C_{1-5}$ alkyl, $-C_{2-5}$ alkenyl, $-C_{2-5}$ alkynyl, $-C=C-Si(CH_3)_3$, $-C\equiv C-Si(C_2H_5)_3$, $-C(=O)-O-C_{1-5}\text{-alkyl}$, $-C(=O)-CF_3$, $-S(=O)_2-C_{1-5}\text{-alkyl}$, $-S(=O)-C_{1-5}\text{-alkyl}$, $-S(=O)_2\text{-phenyl}$, oxo (=O), thioxo (=S), $-N(C_{1-5}\text{-alkyl})_2$, $-N(H)(C_{1-5}\text{-alkyl})$, $-NO_2$, $-S-CF_3$, -C(=O)-OH, $-NH-S(=O)_2-C_{1-5}\text{-alkyl}$, $-NH-C(=O)-C_{1-5}\text{-alkyl}$, -C(=O)-H, $-C(=O)-C_{1-5}\text{-alkyl}$, $-C(=O)-NH_2$, $-C(=O)-N(C_{1-5}\text{-alkyl})_2$, $-C(=O)-N(H)(-C_{1-5}\text{-alkyl})$ and phenyl, wherein the phenyl residues may in each case be unsubstituted or substituted with 1, 2, 3, 4 or 5 substituents mutually independently selected from the group consisting of F, Cl, Br, I, -CN, $-CF_3$, -OH, $-NH_2$, $-O-CF_3$, -SH, $-O-C_{1-5}\text{-alkyl}$, -0-phenyl, $-O-CH_2\text{-phenyl}$, $-(CH_2)-O-C_{1-5}\text{-alkyl}$, $-S-C_{1-5}\text{-alkyl}$, -S-phenyl, $-S-CH_2\text{-phenyl}$, $-C_{1-5}$ alkyl, $-C_{2-5}$ alkenyl, $-C_{2-5}$ alkynyl, $-C=C-Si(CH_3)_3$, $-C=C-Si(C_2H_5)_3$, $-C(=O)-O-C_{1-5}\text{-alkyl}$ and $-C(=O)-CF_3$, wherein the above-stated phenyl residues may preferably be substituted with 1, 2, 3, 4 or 5 substituents mutually independently selected from the group consisting of F, Cl, Br, I, -CN, $-CF_3$, -OH,- $NH_2$, $-O-CF_3$, -SH, $-O-CH_3$, $-O-C_2H_5$, $-O-C_3H_7$, methyl, ethyl, n-propyl, isopropyl, n-butyl, 2-butyl, isobutyl and tert.-butyl;

the above-stated alkylene residues are in each case branched or straight-chain and comprise 1, 2, 3, 4, 5, 6, 7, 8, 9, 10, 11 or 12 carbon atoms as chain links;

the above-stated alkenylene residues are in each case branched or straight-chain and comprise 2, 3, 4, 5, 6, 7, 8, 9, 10, 11 or 12 carbon atoms as chain links;

the above-stated alkynylene residues are in each case branched or straight-chain and comprise 2, 3, 4, 5, 6, 7, 8, 9, 10, 11 or 12 carbon atoms as chain links;

the above-stated heteroalkylene, heteroalkenylene and heteroalkynylene residues are in each case 2-, 3-, 4-, 5-, 6-, 7-, 8-, 9-, 10-, 11- or 12-membered;

the above-stated heteroalkylene, heteroalkenylene and heteroalkynylene groups may in each case optionally

comprise 1, 2 or 3 heteroatom(s) mutually independently selected from the group consisting of oxygen, sulfur and nitrogen (NH) as chain link(s);

the above-stated alkylene, alkenylene, alkynylene, heteroalkylene or heteroalkenylene group may in each case be unsubstituted or substituted with optionally 1, 2, 3, 4 or 5 substituents mutually independently selected from the group consisting of phenyl, F, Cl, Br, I, $-NO_2$, -CN, -OH, -O-phenyl, $-O-CH_2$-phenyl, -SH, -S-phenyl, $-S-CH_2$-phenyl, $NH_2$, $-N(C_{1-5}$-alkyl$)_2$, -NH-phenyl, $-N(C_{1-5}$-alkyl)(phenyl), $-N(C_{1-5}$-alkyl)($CH_2$-phenyl), $-N(C_{1-5}$-alkyl)($CH_2$-$CH_2$-phenyl), -C(=O)-H, $-C(=O)-C_{1-5}$-alkyl, -C(=O)-phenyl, $-C(=S)-C_{1-5}$-alkyl, -C(=S)-phenyl, -C(=O)-OH, $-C(=O)-O-C_{1-5}$-alkyl, -C(=O)-O-phenyl, $-C(=O)-NH_2$, $-C(=O)-NH-C_{1-5}$-alkyl, $-C(=O)-N(C_{1-5}$-alkyl$)_2$, $-S(=O)-C_{1-5}$-alkyl, -S(=O)-phenyl, $-S(=O)_2-C_{1-5}$-alkyl, $-S(=O)_2$-phenyl, $-S(=O)_2-NH_2$ and $-SO_3H$, wherein the phenyl residues may be substituted with 1, 2, 3, 4 or 5 substituents mutually independently selected from the group consisting of F, Cl, Br, I, -CN, $-NO_2$, -OH, -SH, $-NH_2$, -C(=O)-OH, $-C_{1-5}$ alkyl, $-(CH_2)-O-C_{1-5}$-alkyl, $-C_{2-5}$ alkenyl, $-C_{2-5}$ alkynyl, $-C=C-Si(CH_3)_3$, $-C=C-Si(C_2H_5)_3$, $-S-C_{1-5}$-alkyl, -S-phenyl, $-S-CH_2$-phenyl, $-O-C_{1-5}$-alkyl, -O-phenyl, $-O-CH_2$-phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ and $-S-CH_2F$;

the above-stated aryl residues are mono- or bicyclic and comprise 6, 10 or 14 carbon atoms;

the above-stated heteroaryl residues are mono-, di- or tricyclic and 5-, 6-, 7-, 8-, 9-, 10-, 11-, 12-, 13- or 14-membered;

the above-stated 5- to 14-membered heteroalkyl residues optionally comprise 1, 2, 3, 4 or 5 heteroatom(s) mutually independently selected from the group consisting of oxygen, sulfur and nitrogen (NH) as ring member(s);

and the above-stated phenylene residues, aryl residues or heteroaryl residues may optionally in each case be substituted with 1, 2, 3, 4 or 5 substituents mutually independently selected from the group consisting of F, Cl, Br,I, -CN, $-NO_2$, -OH, -SH, $-NH_2$, -C(=O)-OH, $-C_{1-5}$ alkyl, $-(CH_2)-O-C_{1-5}$-alkyl, $-C_{2-5}$ alkenyl, $-C_{2-5}$ alkynyl, $-C=C-Si(CH_3)_3$, $-C=-C-Si(C_2H_5)_3$, $-S-C_{1-5}$-alkyl, -S-phenyl, $-S-CH_2$-phenyl, $-O-C_{1-5}$-alkyl, -O-phenyl, $-O-CH_2$-phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-phenyl, $-S(=O)_2-C_{1-5}$-alkyl, $-S(=O)-C_{1-5}$-alkyl, $-NH-C_{1-5}$-alkyl, $N(C_{1-5}$alkyl$)_2$, $-C(=O)-O-C_{1-5}$-alkyl, -C(=O)-H, $-C(=O)-C_{1-5}$-alkyl, $-CH_2-O-C(=O)$-phenyl, -O-C(=O)-phenyl, $-NH-S(=O)_2-C_{1-5}$-alkyl, $-NH-C(=O)-C_{1-5}$-alkyl, $-C(=O)-NH_2$, $-C(=O)-NH-C_{1-5}$-alkyl, $-C(=O)-N(C_{1-5}$-alkyl$)_2$, pyrazolyl, phenyl, furyl (furanyl), thiazolyl, thiadiazolyl, thiophenyl (thienyl), benzyl and phenethyl, wherein the cyclic substituents or the cyclic residues of these substituents themselves may be substituted with optionally 1, 2, 3, 4 or 5 substituents mutually independently selected from the group consisting of F, Cl, Br, I, -CN, $-NO_2$, -OH, -SH, $-NH_2$, -C(=O)-OH, $-C_{1-5}$ alkyl, $-(CH_2)-O-C_{1-5}$-alkyl, $-C_{2-5}$ alkenyl, $-C_{2-5}$ alkynyl, $-C\equiv C-Si(CH_3)_3$, $-C\equiv C-Si(C_2H_5)_3$, $-S-C_{1-5}$-alkyl, -S-phenyl, $-S-CH_2$-phenyl, $-O-C_{1-5}$-alkyl, -O-phenyl, $-O-CH_2$-phenyl,- $CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ and $-S-CH_2F$;

in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

**3.** Compounds according to claim 1 or claim 2, **characterised in that**

$R^1$ denotes H; F; Cl; Br; I; $-NO_2$; -CN; $-NH_2$; -OH; -SH; -C(=O)-OH; -C(=O)-H; -NH-C(=O)-H; $-NH-R^{55}$; $-NR^{56}R^{57}$; $-C(=O)-R^{58}$; $-C(=O)-O-R^{59}$; $-C(=O)-NH_2$; $-C(=O)-NH-R^{64}$; $-C(=O)-NR^{65}R^{66}$; $-O-R^{67}$; $-S-R^{68}$; $-S(=O)-R^{69}$; $-S(=O)2-R^{70}$; $C_{1-6}$ alkyl, which is unsubstituted or substituted with optionally 1, 2, 3, 4 or 5 substituents mutually independently selected from the group consisting of unsubstituted $C_{1-6}$ alkyl or denotes a residue selected from the group consisting of (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, which is in each case unsubstituted or substituted with optionally 1, 2, 3, 4 or 5 substituents mutually independently selected from the group consisting of F, Cl, Br, I, -CN, methyl, ethyl, n-propyl, isopropyl, n-butyl, 2-butyl, isobutyl, *tert.*-butyl, -OH, $-O-CH_3$, $-O-C_2H_5$ and $-O-C_3H_7$;

$R^2$ denotes H; F; Cl; Br; I; $-CF_3$; $-NO_2$; -CN; $-NH_2$; -OH; -SH; -C(=O)-OH; -C(=O)-H; -NH-C(=O)-H; $-NH-R^{55}$; $-NR^{56}R^{57}$; $-C(=O)-R^{58}$; $-C(=O)-O-R^{59}$; $-C(=O)-NH_2$; $-C(=O)-NH-R^{64}$; $-C(=O)-NR^{65}R^{66}$; $-O-R^{67}$; $-S-R^{68}$; $-S(=O)-R^{69}$; $-S(=O)_2-R^{70}$; unsubstituted $C_{1-6}$ alkyl or denotes a residue selected from the group consisting of (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, which is in each case unsubstituted or substituted with optionally 1, 2, 3, 4 or 5 substituents mutually independently selected from the group consisting of F, Cl, Br, I, -CN, methyl, ethyl, n-propyl, isopropyl, n-butyl, 2-butyl, isobutyl, *tert.*-butyl, -OH, $-O-CH_3$, $-O-C_2H_5$ and $-O-C_3H_7$;

or $R^1$ and $R^2$ together with the carbon atoms joining them form a phenylene residue, which is unsubstituted or substituted with 1, 2, 3 or 4 substituents mutually independently selected from the group consisting of F, Cl, Br, I, -CN, methyl, ethyl, n-propyl, isopropyl, n-butyl, isobutyl, 2-butyl, *tert.*-butyl, -OH, -SH, $-NH_2$, -C(=O)-OH, -S-

$CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-CF_3$, $-CHF_2$, $-CH_2F$ and $-O-CF_3$;

$R^3$ and $R^8$, mutually independently, in each case denote H; -C(=O)-$R^{58;}$ -C(=O)-O-$R^{59}$; -C(=O)-$NH_2$; -C(=O)-NH-$R^{64}$; -C(=O)-$NR^{65}R^{66}$; -S(=O)-$R^{69}$; $-S(=O)_2-R^{70}$; $C_{1-6}$ alkyl, which is unsubstituted or substituted with optionally 1, 2, 3, 4 or 5 substituents mutually independently selected from the group consisting of F, Cl, Br, I, $-NO_2$, -CN, -OH, -SH and $-NH_2$;

$C_{3-6}$ cycloalkyl, which is unsubstituted or substituted with optionally 1, 2, 3, 4 or 5 substituents mutually independently selected from the group consisting of F, Cl, Br, I, $-NO_2$, -CN, -OH, -SH and $-NH_2$;

or denote a phenyl residue, which may in each case be attached via a $C_{1-3}$ alkylene, $C_{2-3}$ alkenylene or $C_{2-3}$ alkynylene group and/or is unsubstituted or substituted with 1, 2, 3, 4 or 5 substituents mutually independently selected from the group consisting of F, Cl, Br, I, -CN, methyl, ethyl, n-propyl, isopropyl, n-butyl, 2-butyl, isobutyl, *tert.*-butyl, -OH, $-O-CH_3$, $-O-C_2H_5$ and $-O-C_3H_7$;

$R^4$, $R^5$ $R^6$ $R^7$, $R^{10}$, $R^{11}$, $R^{12}$, $R^{13}$ $R^{14}$ $R^{15}$,$R^{16}$, $R^{17}$, $R^{18}$ $R^{19}$,$R^{20}$ $R^{21}$, $R^{22}$ $R^{23}$, $R^{21}$, $R^{15}$, $R^{26}$, $R^{27}$, $R^{28}$, $R^{29}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$ and $R^{45}$, mutually independently, in each case denote H; F; Cl; Br; I; $-NO_2$; -CN; $-NH_2$; -OH; -SH; -C(=O)-OH; -NH-R5$^5$; -NR $^{56}R^{57}$ ; -C(=O)-$R^{58}$; -C(=O)-O-$R^{59}$; -O-$R^{67}$; -S-$R^{68}$;

unsubstituted $C_{1-6}$ alkyl; or denote a residue selected from the group consisting of phenyl, benzyl and phenethyl, which is unsubstituted or substituted with optionally 1, 2, 3, 4 or 5 substituents mutually independently selected from the group consisting of F, Cl, Br, I, -CN, methyl, ethyl, n-propyl, isopropyl, n-butyl, 2-butyl, isobutyl, *tert.*-butyl, -OH, $-O-CH_3$, $-O-C_2H_5$ and $-O-C_3H_7$;

or $R^4$ and $R^5$ or $R^6$ and $R^7$ or $R^{10}$ and $R^{11}$ or $R^{12}$ and $R^{13}$ or $R^{14}$ and $R^{15}$ or $R^{16}$ and $R^{17}$ or R and $R^{19}$ or $R^{20}$ and $R^{21}$ or $R^{22}$ and $R^{23}$ or $R^{24}$ and $R^{25}$ or $R^{26}$ and $R^{27}$ or $R^{28}$ and $R^{29}$ or $R^{32}$ and $R^{33}$ or $R^{34}$ and $R^{35}$ or $R^{36}$ and $R^{37}$ or $R^{38}$ and $R^{39}$ or $R^{40}$ and $R^{41}$ or $R^{42}$ and $R^{43}$ or $R^{44}$ and $R^{45}$, mutually independently, in each case together denote a residue selected from the group consisting of an oxo group (=O) and a thioxo group (=S);

or $R^3$ and $R^4$ together with the -N-$CR^5$ group joining them form a residue selected from the group consisting of

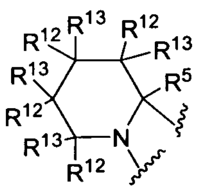 , 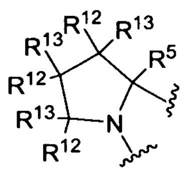 , 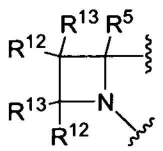 , 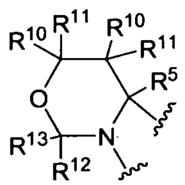 ,

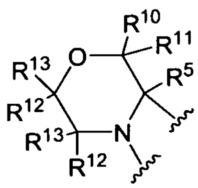 , 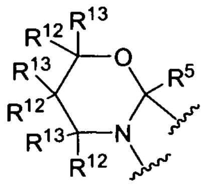 , 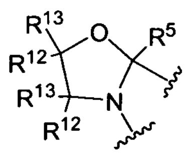 and 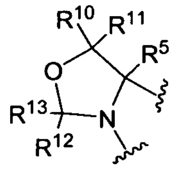

or $R^6$ and $R^8$ together with the -N-$CR^7$ group joining them form a residue selected from the group consisting of

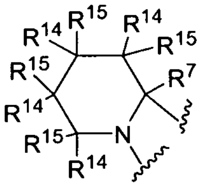 , 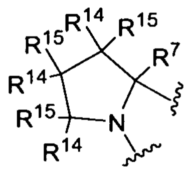 , 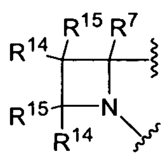 , 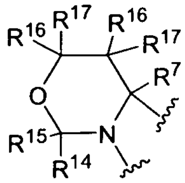 ,

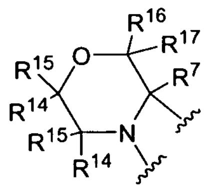,
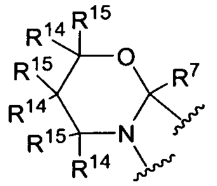,
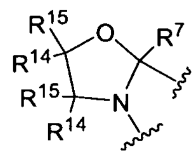 and 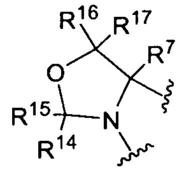,

or $R^3$ and $R^6$ together with the -N-$CR^4R^5$-$CR^7$ group joining them form a residue selected from the group consisting of

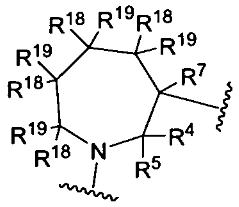,
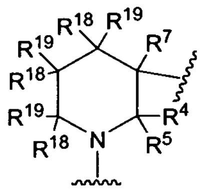,
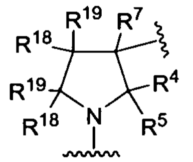,

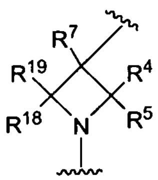,
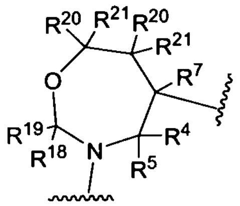,
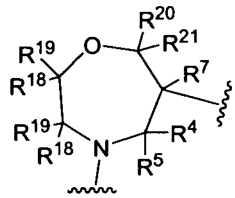,
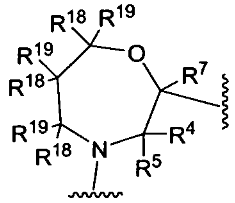,

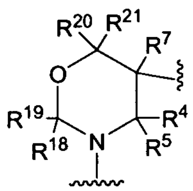,
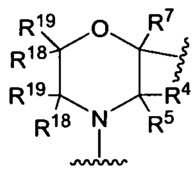 and 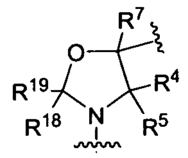

or $R^4$ and $R^8$ together with the -$CR^5$-$CR^6CR^7$-N group joining them form a residue selected from the group consisting of

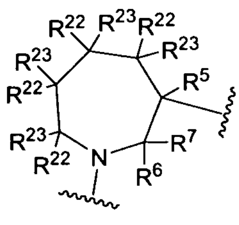,
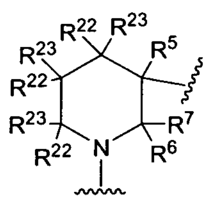,
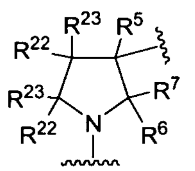,

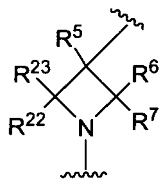, 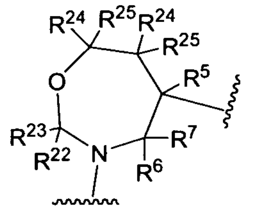, 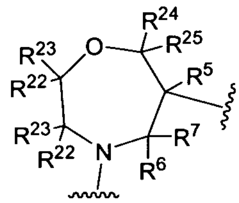, 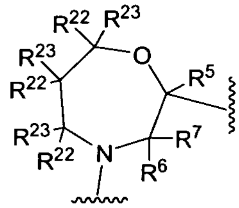,

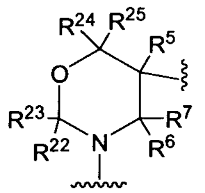, 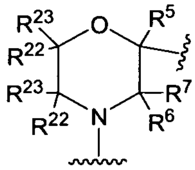 and 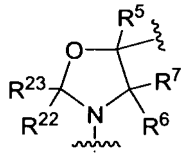

or $R^3$ and $R^8$ together with the -N-$CR^4R^5$-$CR^6CR^7$-N group joining them form a residue selected from the group consisting of

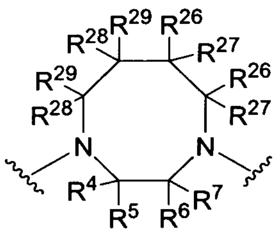 and 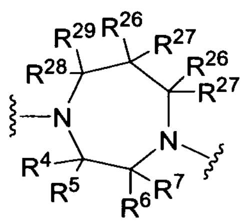

or $R^4$ and $R^6$ together with the -$CR^5$-$CR^7$ group joining them form a residue selected from the group consisting of

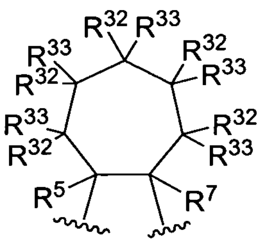, 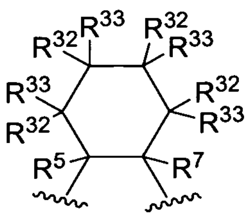 and 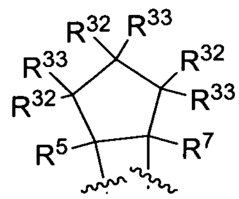,

or $R^3$ and $R^4$ together with the -N-$CR^5$ group joining them and $R^6$ and $R^8$ together with the -N-$CR^7$ group joining them form a residue selected from the group consisting of

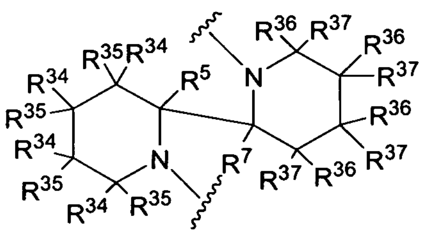, 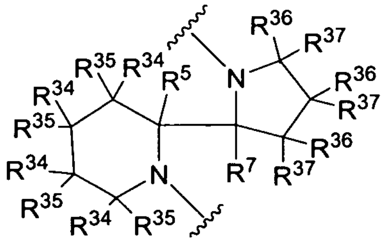,

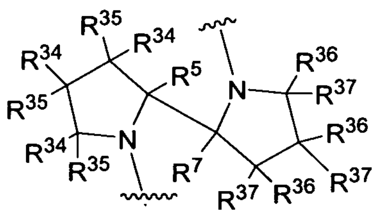 and 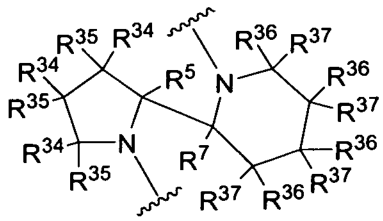

or $R^3$ and $R^6$ together with the -N-$CR^4CR^5$-$CR^7$ group joining them and $R^4$ and $R^8$ together with the -$CR^5CR^6R^7$-N group joining them form a residue selected from the group consisting of

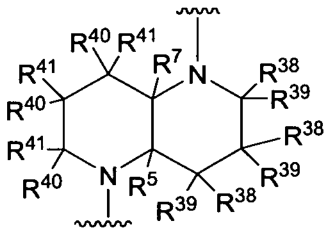 , 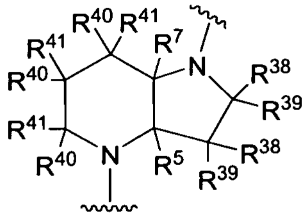 ,

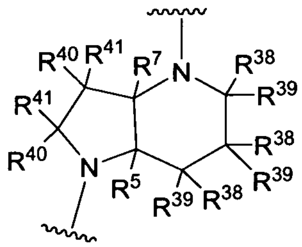 and 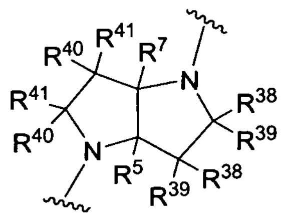

or $R^3$and $R^8$ together with the -N-$CR^4R^5$-$CR^6R^7$-N group joining them and $R^4$ and $R^6$ together with the -$CR^5$-$CR^7$group joining them form a residue selected from the group consisting of

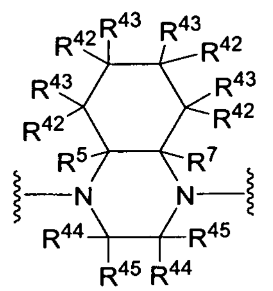 , 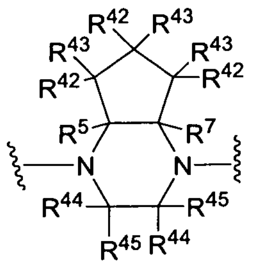 ,

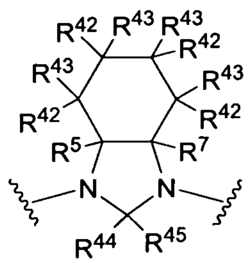 and 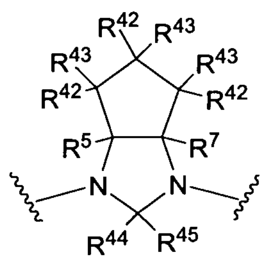

$R^9$ denotes H; -C(=O)-$NH_2$; -C(=O)-NH-$R^{64}$; -C(=O)-$NR^{65}R^{66}$; unsubstituted $C_{1-6}$ alkyl; unsubstituted $C_{3-7}$ cycloalkyl; unsubstituted $C_{5-6}$ cycloalkenyl; unsubstituted 5- to 7-membered heterocycloalkyl and unsubstituted 5- to 7-membered heterocycloalkenyl; or denotes a residue selected from the group consisting of phenyl, naphthyl, anthracenyl, furyl, thienyl, pyrazolyl, pyrazinyl, pyridazinyl, pyrimidinyl, pyridinyl, pyrrolyl, oxazolyl, isoxazolyl, thiazolyl, thiadiazolyl, oxadiazolyl, triazolyl, imidazolyl, indolyl, benzo[b]thiophenyl, benzo[d]thiazolyl, benzo[b]furanyl, quinolinyl, isoquinolinyl and quinazolinyl, which is in each case unsubstituted or substituted with optionally 1, 2, 3, 4 or 5 substituents mutually independently selected from the group consisting of F, Cl, Br, I, -CN, methyl, ethyl, n-propyl, isopropyl, n-butyl, 2-butyl, isobutyl, *tert*.-butyl, ethenyl, allyl, ethynyl, propynyl, -C=C-Si$(CH_3)_3$, -C=C-Si$(C_2H_5)_3$, -$CH_2$-O-$CH_3$, -$CH_2$-O-$C_2H_5$, -OH, -O-$CH_3$, -O-$C_2H_5$, -O-$C_3H_7$, -S-$CH_3$, -S-$C_2H_5$, -S(=O)-$CH_3$, -S(=O)$_2$-$CH_3$, -S(=O)-$C_2H_5$, -S(=O)$_2$-$C_2H_5$, -$NH_2$, -N$(CH_3)_2$, -N$(C_2H_5)_2$, -NH-$CH_3$, -NH-$C_2H_5$, -$NO_2$, -$CF_3$, -$CH_2F$, -$CHF_2$, -O-$CF_3$, -S-$CF_3$, -SH, -NH-S(=O)$_2$-$CH_3$, -C(=O)-OH, -C(=O)-H; -C(=O)-$CH_3$, -C(=O)-$C_2H_5$, -C(=O)-$NH_2$, -C(=O)-N$(CH_3)_2$, -C(=O)-NH-$CH_3$, -NH-C(=O)-$CH_3$, -NH-C(=O)-$C_2H_5$, -C(=O)-O-$CH_3$ , -C(=O)-O-$C_2H_5$, -C(=O)-O-C$(CH_3)_3$ and phenyl; and $R^{55}$, $R^{56}$ $R^{57}$, $R^{58}$, $R^{59}$ $R^{64}$, $R^{65}$, $R^{66}$ $R^{67}$ $R^{68}$, $R^{69}$ and $R^{70}$, mutually independently, in each case denote unsubstituted $C_{1-6}$ alkyl; unsubstituted $C_{3-7}$ cycloalkyl; unsubstituted $C_{5-6}$ cycloalkenyl; unsubstituted 5- to 7-membered heterocycloalkyl and unsubstituted 5- to 7-membered heterocycloalkenyl; or denote a residue selected from the group consisting of phenyl, benzyl, naphthyl, anthracenyl, pyrrolyl, indolyl, furanyl, benzo[b]furanyl, thiophenyl, benzo[b]thiophenyl, benzo[d]thiazolyl, pyrazolyl, imidazolyl, thiazolyl, thiadiazolyl, triazolyl, oxazolyl, oxadiazolyl, isoxazolyl, pyridinyl, pyridazinyl, pyrimidinyl, pyrazinyl, pyranyl, indazolyl, quinolinyl, isoquinolinyl and quinazolinyl, which is in each case unsubstituted or substituted with optionally 1, 2, 3, 4 or 5 substituents mutually independently selected from the group consisting of F, Cl, Br, I, -CN, methyl, ethyl, n-propyl, isopropyl, n-butyl, 2-butyl, isobutyl, *tert*.-butyl, -OH, -O-$CH_3$, -O-$C_2H_5$, -O-$C_3H_7$, -$NH_2$, -N$(CH_3)_2$, -N$(C_2H_5)_2$, -NH-$CH_3$, -NH-$C_2H_5$, -$NO_2$, -$CF_3$, -O-$CF_3$, -S-$CF_3$, -SH, -NH-S(=O)$_2$-$CH_3$, -C(=O)-OH, -C(=O)-$CH_3$, -C(=O)-$C_2H_5$, -C(=O)-N$(CH_3)_2$, -C(=O)-NH-$CH_3$, -NH-C(=O)-$CH_3$, -NH-C(=O)-$C_2H_5$, -C(=O)-O-$CH_3$ and -C(=O)-O-$C_2H_5$;

in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts.

**4.** Compounds according to one or more of claims 1 to 3, **characterised in that**

$R^1$ denotes H; F; Cl; Br; I; -$CF_3$; -$NO_2$; -CN; -C(=O)-OH; -C(=O)-O-$R^{59}$; -C(=O)-$NH_2$; -C(=O)-NH-$R^{64}$; -C(=O)-$NR^{65}R^{66}$; -O-$R^{67}$; -S-$R^{68}$; -S(=O)-$R^{69}$; -S(=O)$_2$-$R^{70}$; an alkyl residue selected from the group consisting of methyl, ethyl, n-propyl, isopropyl, n-butyl, 2-butyl, isobutyl and *tert*.-butyl; or denotes a residue selected from the group consisting of (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, which is in each case unsubstituted or substituted with optionally 1, 2, 3, 4 or 5 substituents mutually independently selected from the group consisting of F, Cl, Br, methyl, ethyl, n-propyl, isopropyl, n-butyl, 2-butyl, isobutyl, *tert*.-butyl, -OH, -O-$CH_3$, -O-$C_2H_5$ and -O-$C_3H_7$;

$R^2$ denotes H; F; Cl; Br; I; -$CF_3$; -$NO_2$; -CN; -C(=O)-OH; -C(=O)-O-$R^{59}$; -C(=O)-$NH_2$; -C(=O)-NH-$R^{64}$; -C(=O)-$NR^{65}R^{66}$; -O-$R^{67}$; -S-$R^{68}$; -S(=O)-$R^{69}$; -S(=O)$_2$-$R^{10}$; an alkyl residue selected from the group consisting of methyl, ethyl, n-propyl, isopropyl, n-butyl, 2-butyl, isobutyl and *tert*.-butyl; or denotes a residue selected from the group consisting of (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, which is in each case unsubstituted or substituted with optionally 1, 2, 3, 4 or 5 substituents mutually independently selected from the group consisting of F, Cl, Br, methyl, ethyl, n-propyl, isopropyl, n-butyl, 2-butyl, isobutyl, *tert*.-butyl, -OH, -O-$CH_3$, -O-$C_2H_5$ and -O-$C_3H_7$;

or $R^1$ and $R^2$ together with the carbon atoms joining them form a phenylene residue, which is unsubstituted or substituted with 1, 2, 3 or 4 substituents mutually independently selected from the group consisting of F, Cl, Br, I, -CN, methyl, ethyl, n-propyl, isopropyl, n-butyl, isobutyl, 2-butyl, *tert*.-butyl, -O-$CH_3$, -O-$C_2H_5$, -O-$C_3H_7$, -$CF_3$, -$CHF_2$, -$CH_2F$ and -O-$CF_3$;

$R^3$ and $R^8$, mutually independently, in each case denote H; -C(=O)-$R^{58}$; -C(=O)-O-$R^{59}$; -S(=O)-$R^{69}$; -S(=O)$_2$-$R^{70}$; an alkyl residue selected from the group consisting of methyl, ethyl, n-propyl, isopropyl, n-butyl, 2-butyl, isobutyl, *tert*.-butyl, n-pentyl and n-hexyl; denote a cycloalkyl residue selected from the group consisting of cyclopropyl, cyclobutyl, cyclopentyl and cyclohexyl; or denote a benzyl or phenethyl residue, which is unsubstituted or substituted with 1, 2, 3, 4 or 5 substituents mutually independently selected from the group consisting of F, Cl, Br, methyl, ethyl, n-propyl, isopropyl, n-butyl, 2-butyl, isobutyl, *tert*.-butyl, -OH, -O-$CH_3$, -O-$C_2H_5$ and -O-$C_3H_7$;

$R^4$, $R^5$, $R^6$, $R^7$, $R^{12}$, $R^{13}$, $R^{14}$, $R^{15}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{32}$, $R^{33}$, $R^{38}$, $R^{39}$, $R^{40}$, $R^{41}$, $R^{42}$, $R^{43}$, $R^{44}$ and $R^{45}$, mutually independently, in each case denote H; F; Cl; Br; I; -$NO_2$; -CN; -$NH_2$; -OH; -SH; -NH-$R^{55}$; -$NR^{56}R^{57}$; -O-$R^{67}$; -S-$R^{68}$; or denote an alkyl residue selected from the group consisting of methyl, ethyl, n-propyl, isopropyl, n-butyl, 2-butyl, isobutyl, *tert.*-butyl, n-pentyl and n-hexyl; or $R^4$ and $R^5$ or $R^6$ and $R^7$ or $R^{12}$ and $R^{13}$ or $R^{14}$ and $R^{15}$ or $R^{18}$ and $R^{19}$ or $R^{20}$and $R^{21}$ or $R^{22}$ and $R^{23}$ or $R^{24}$ and $R^{25}$ or $R^{26}$ and $R^{27}$ or $R^{28}$ and $R^{29}$ or $R^{32}$ and $R^{33}$ or $R^{38}$ and $R^{39}$ or $R^{40}$ and $R^{41}$ or $R^{42}$ and $R^{43}$ or $R^{44}$ and $R^{45}$, mutually independently, in each case together denote a residue selected from the group consisting of an oxo group (=O) and a thioxo group (=S);

or $R^3$ and $R^4$ together with the -N-CR$^5$ group joining them form a residue selected from the group consisting of

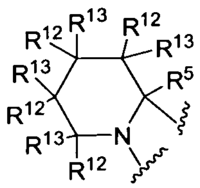

and

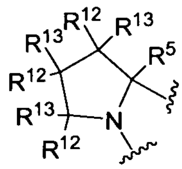

or $R^6$ and $R^8$ together with the -N-CR$^7$ group joining them form a residue selected from the group consisting of

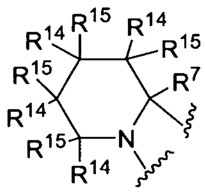

and

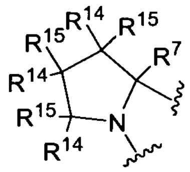

or $R^3$ and $R^6$ together with the -N-CR$^4$R$^5$-CR$^7$ group joining them form a residue selected from the group consisting of

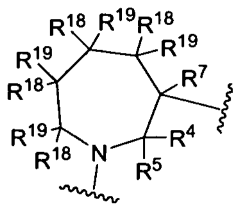

,

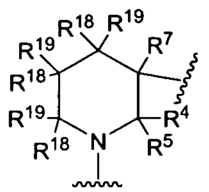

,

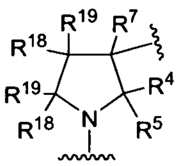

,

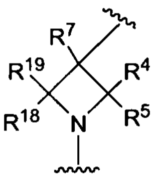

,

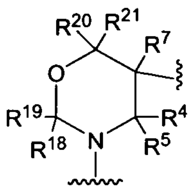

,

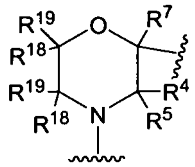

and

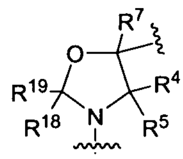

or $R^4$ and $R^8$ together with the -CR$^5$-CR$^6$CR$^7$-N group joining them form a residue selected from the group consisting of

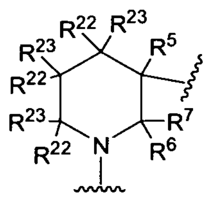

,

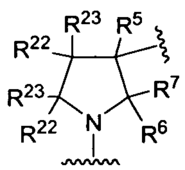

,

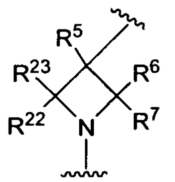

,

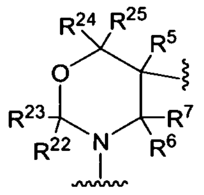,
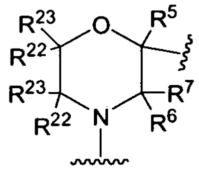
and
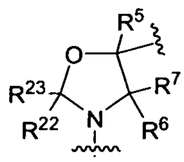

or $R^3$ and $R^8$ together with the $-N-CR^4R^5-CR^6CR^7-N$ group joining them form the following residue

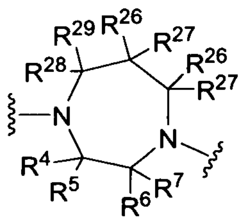

or $R^4$ and $R^6$ together with the $-CR^5-CR^7$group joining them form the following residue

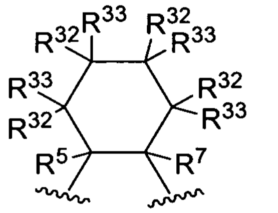

or $R^3$ and $R^6$ together with the $-N-CR^4CR^5-CR^7$ group joining them and $R^4$ and $R^8$ together with the $-CR^5-CR^6R^7-N$ group joining them form the following residue

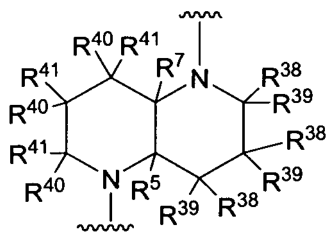

or $R^3$ and $R^8$ together with the $-N-CR^4R^5-CR^6R^7-N$ group joining them and $R^4$ and $R^6$ together with the $-CR^5-CR^7$ group joining them form the following residue

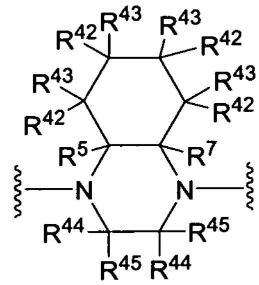

$R^9$ denotes -C(=O)-NH-$R^{64}$; -C(=O)-N$R^{65}R^{66}$;
or denotes a residue selected from the group consisting of phenyl, naphthyl, anthracenyl, furyl, thienyl, pyrazolyl, pyrazinyl, pyridazinyl, pyrimidinyl, pyridinyl, pyrrolyl, oxazolyl, isoxazolyl, thiazolyl, thiadiazolyl, oxadiazolyl, triazolyl, imidazolyl, indolyl, benzo[b]thiophenyl, benzo[d]thiazolyl, benzo[b]furanyl, quinolinyl, isoquinolinyl and quinazolinyl, which is in each case unsubstituted or substituted with optionally 1, 2, 3, 4 or 5 substituents mutually independently selected from the group consisting of F, Cl, Br, I, -CN, methyl, ethyl, n-propyl, isopropyl, n-butyl, 2-butyl, isobutyl, *tert.*-butyl, ethenyl, allyl, ethynyl, propynyl, -C=C-Si($CH_3$)$_3$, -C≡C-Si($C_2H_5$)$_3$, -$CH_2$-O-$CH_3$, -$CH_2$-O-$C_2H_5$, -OH, -O-$CH_3$, -O-$C_2H_5$, -O-$C_3H_7$, -S-$CH_3$, -S-$C_2H_5$, -S(=O)-$CH_3$, -S(=O)$_2$-$CH_3$, -S(=O)-$C_2H_5$,-S(=O)$_2$-$C_2H_5$, -$NH_2$, -N($CH_3$)$_2$, -N($C_2H_5$)$_2$, -NH-$CH_3$, -NH-$C_2H_5$, -$NO_2$, -$CF_3$, -$CH_2F$, -$CHF_2$, -O-$CF_3$, -S-$CF_3$, -SH, -NH-S(=O)$_2$-$CH_3$, -C(=O)-OH, -C(=O)-H; -C(=O)-$CH_3$, -C(=O)-$C_2H_5$, -C(=O)-$NH_2$, -C(=O)-N($CH_3$)$_2$, -C(=O)-NH-$CH_3$, -NH-C(=O)-$CH_3$, -NH-C(=O)-$C_2H_5$, -C(=O)-O-$CH_3$ , -C(=O)-O-$C_2H_5$, -C(=O)-O-C($CH_3$)$_3$ and phenyl;
and $R^{55}$, $R^{56}$, $R^{57}$, $R^{58}$ $R^{59}$ $R^{64}$ $R^{65}$ $R^{66}$ $R^{67}$ $R^{68}$ $R^{69}$ and $R^{70}$, mutually independently, in each case denote an alkyl residue selected from the group consisting of methyl, ethyl, n-propyl, isopropyl, n-butyl, 2-butyl, isobutyl, *tert.*-butyl, n-pentyl and n-hexyl; or denote a phenyl, benzyl or phenethyl residue, which is unsubstituted or substituted with 1, 2, 3, 4 or 5 substituents mutually independently selected from the group consisting of F, Cl, Br, methyl, ethyl, n-propyl" isopropyl, n-butyl, 2-butyl, isobutyl, *tert.*-butyl, -OH, -O-$CH_3$, -O-$C_2H_5$ and -O-$C_3H_7$;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts.

**5.** Compounds according to one or more of claims 1-4, **characterised in that**

$R^1$ denotes H; F; Cl; Br; I; -$CF_3$; -$NO_2$; -CN; -C(=O)-OH; -C(=O)-O-$R^{59}$; -C(=O)-$NH_2$; -C(=O)-NH-$R^{64}$; -C(=O)-N$R^{65}R^{66}$; -O-$R^{67}$; -S-$R^{68}$; -S(=O)-$R^{69}$; -S(=O)2-$R^{70}$; an alkyl residue selected from the group consisting of methyl, ethyl, n-propyl, isopropyl, n-butyl, 2-butyl, isobutyl and *tert.*-butyl; or denotes a residue selected from the group consisting of (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, which is in each case unsubstituted or substituted with optionally 1, 2, 3, 4 or 5 substituents mutually independently selected from the group consisting of F, Cl, Br, methyl, ethyl, n-propyl, isopropyl, n-butyl, 2-butyl, isobutyl, *tert.*-butyl, -OH, -O-$CH_3$, -O-$C_2H_5$ and -O-$C_3H_7$;
$R^2$ denotes H; F; Cl; Br; I; -$CF_3$; -$NO_2$; -CN; -C(=O)-OH; -C(=O)-O-$R^{59}$; -C(=O)-$NH_2$; -C(=O)-NH-$R^{64}$; -C(=O)-N$R^{65}R^{66}$; -O-$R^{67}$; -S-$R^{68}$; -S(=O)-$R^{69}$; -S(=O)$_2$-$R^{70}$; an alkyl residue selected from the group consisting of methyl, ethyl, n-propyl, isopropyl, n-butyl, 2-butyl, isobutyl and *tert.*-butyl; or denotes a residue selected from the group consisting of (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, which is in each case unsubstituted or substituted with optionally 1, 2, 3, 4 or 5 substituents mutually independently selected from the group consisting of F, Cl, Br, methyl, ethyl, n-propyl, isopropyl, n-butyl, 2-butyl, isobutyl, *tert.*-butyl, -OH, -O-$CH_3$, -O-$C_2H_5$ and -O-$C_3H_7$;
or $R^1$ and $R^2$ together with the carbon atoms joining them form a phenylene residue, which is unsubstituted or substituted with 1, 2, 3 or 4 substituents mutually independently selected from the group consisting of F, Cl, Br, I, -CN, methyl, ethyl, n-propyl, isopropyl, n-butyl, isobutyl, 2-butyl, *tert.*-butyl, -O-$CH_3$, -O-$C_2H_5$, -O-$C_3H_7$, -$CF_3$, -$CHF_2$, -$CH_2F$ and -O-$CF_3$;
$R^3$ and $R^8$, mutually independently, in each case denote H; -C(=O)-$R^{58}$; -C(=O)-O-$R^{59}$; -S(=O)-$R^{69}$; -S(=O)$_2$-$R^{70}$; an alkyl residue selected from the group consisting of methyl, ethyl, n-propyl, isopropyl, n-butyl, 2-butyl, isobutyl, *tert.*-butyl, n-pentyl and n-hexyl; denote a cycloalkyl residue selected from the group consisting of cyclopropyl, cyclobutyl, cyclopentyl and cyclohexyl; or denote a benzyl or phenethyl residue, which is unsubstituted or substituted with 1, 2, 3, 4 or 5 substituents mutually independently selected from the group consisting of F, Cl, Br, methyl, ethyl, n-propyl, isopropyl, n-butyl, 2-butyl, isobutyl, *tert.*-butyl, -OH, -O-$CH_3$, -O-$C_2H_5$ and -O-$C_3H_7$;

$R^4$, $R^5$, $R^6$ and $R^7$, mutually independently, in each case denote H; F; Cl; Br; I; $-NO_2$; -CN; $-NH_2$; -OH; -SH; $-NH-R^{55}$; $-NR^{56}R^{57}$ ; $-O-R^{67}$; $-S-R^{68}$; or denote an alkyl residue selected from the group consisting of methyl, ethyl, n-propyl, isopropyl, n-butyl, 2-butyl, isobutyl, *tert.*-butyl, n-pentyl and n-hexyl;
or $R^4$ and $R^5$ or $R^6$ and $R^7$ mutually independently, together in each case denote a residue selected from the group consisting of an oxo group (=O) and a thioxo group (=S);
or $R^3$ and $R^4$ together with the $-N-CR^5$ group joining them form a residue selected from the group consisting of

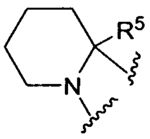 and 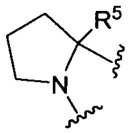

or $R^6$ and $R^8$ together with the $-N-CR^7$ group joining them form a residue selected from the group consisting of

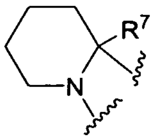 and 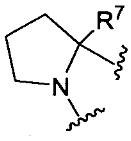

or $R^3$ and $R^6$ together with the $-N-CR^4R^5-CR^7$ group joining them form a residue selected from the group consisting of

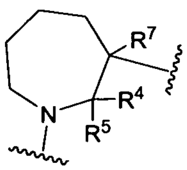 , 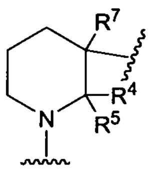 , 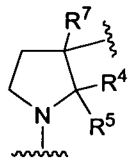 ,

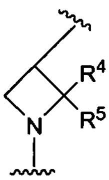 , 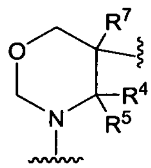 , 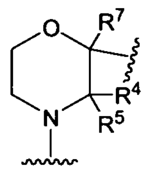 and 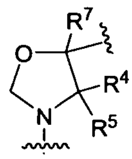

or $R^4$ and $R^8$ together with the $-CR^5-CR^6CR^7-N$ group joining them form a residue selected from the group consisting of

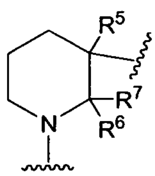 , 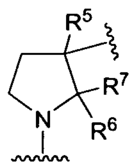 , 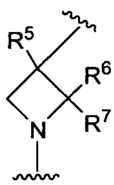 ,

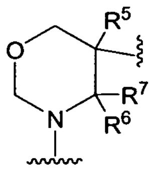 , 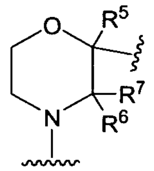 and 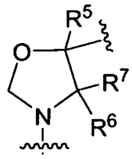

or $R^3$ and $R^8$ together with the -N-$CR^4R^5$-$CR^6CR^7$-N group joining them form the following residue

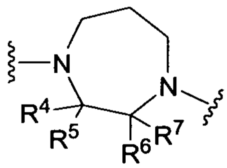

or $R^4$ and $R^6$ together with the -$CR^5$-$CR^7$group joining them form the following residue

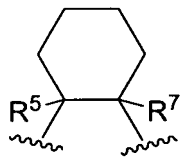

or $R^3$ and $R^6$ together with the -N-$CR^4CR^5$-CR group joining them and $R^4$ and $R^8$ together with the -$CR^5$-$CR^6R^7$-N group joining them form the following residue

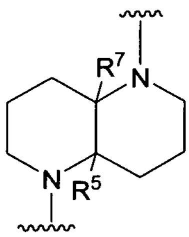

or $R^3$ and $R^8$ together with the -N-$CR^4R^5$-$CR^6R^7$-N group joining them and $R^4$ and $R^6$ together with the -$CR^5$-$CR^7$group joining them form the following residue

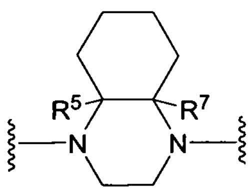

$R^9$ denotes -C(=O)-NH-$R^{64}$; -C(=O)-$NR^{65}R^{66}$; or denotes a residue selected from the group consisting of phenyl, naphthyl, furyl, thienyl, pyrazolyl, pyrazinyl, pyridazinyl, pyrimidinyl, pyridinyl, pyrrolyl, oxazolyl, isoxazolyl, thiazolyl, thiadiazolyl, oxadiazolyl, triazolyl, imidazolyl, indolyl, benzo[b]thiophenyl, benzo[d]thiazolyl, benzo[b]furanyl, quinolinyl, isoquinolinyl and quinazolinyl, which is in each case unsubstituted or substituted with optionally 1, 2, 3, 4 or 5 substituents mutually independently selected from the group consisting of F, Cl, Br, I, -CN,

methyl, ethyl, n-propyl, isopropyl, n-butyl, 2-butyl, isobutyl, *tert.*-butyl, ethenyl, allyl, ethynyl, propynyl, $-O-CH_3$, $-O-C_2H_5$, $-NO_2$, $-CF_3$, $-CH_2F$, $-CHF_2$, $-O-CF_3$ and $-S-CF_3$;
and $R^{55}$, $R^{56}$ $R^{57}$ $R^{58}$ $R^{59}$, $R^{64}$ $R^{65}$ $R^{66}$ $R^{67}$ $R^{68}$ $R^{69}$ and $R^{70}$, mutually independently, in each case denote an alkyl residue selected from the group consisting of methyl, ethyl, n-propyl, isopropyl, n-butyl, 2-butyl, isobutyl, *tert.*-butyl, n-pentyl and n-hexyl; or denote a phenyl, benzyl or phenethyl residue, which is unsubstituted or substituted with 1, 2, 3, 4 or 5 substituents mutually independently selected from the group consisting of F, Cl, Br, methyl, ethyl, n-propyl, isopropyl, n-butyl, 2-butyl, isobutyl, *tert.*-butyl, -OH, $-O-CH_3$, $-O-C_2H_5$ and $-O-C_3H_7$;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts.

**6.** Compounds according to one or more of claims 1 to 5, **characterised in that**

$R^1$ denotes H; F; Cl; Br; I; $-CF_3$; $-NO_2$; -CN; $-C(=O)-O-R^{59}$; an alkyl residue selected from the group consisting of methyl, ethyl, n-propyl, isopropyl, n-butyl, 2-butyl, isobutyl and *tert.*-butyl; or denotes a residue selected from the group consisting of (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl;
$R^2$ denotes H; F; Cl. Br; I; $-CF_3$; $-NO_2$; -CN; $-C(=O)-O-R^{59}$. an alkyl residue selected from the group consisting of methyl, ethyl, n-propyl, isopropyl, n-butyl, 2-butyl, isobutyl and *tert.*-butyl; or denotes a residue selected from the group consisting of (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl;
or $R^1$ and $R^2$ together with the carbon atoms joining them form a phenylene residue;
$R^3$ and $R^8$, mutually independently, in each case denote H; $-C(=O)-R^{58}$; $-C(=O)-O-R^{59}$; an alkyl residue selected from the group consisting of methyl, ethyl, n-propyl, isopropyl, n-butyl, 2-butyl, isobutyl, *tert.*-butyl, n-pentyl and n-hexyl; denote a cycloalkyl residue selected from the group consisting of cyclopropyl, cyclobutyl, cyclopentyl and cyclohexyl; or denote a benzyl or phenethyl residue, which is unsubstituted or substituted with 1, 2, 3, 4 or 5 substituents mutually independently selected from the group consisting of F, Cl, Br, methyl, ethyl, n-propyl, isopropyl, n-butyl, 2-butyl, isobutyl, *tert.*-butyl, -OH, $-O-CH_3$, $-O-C_2H_5$ and $-O-C_3H_7$;
$R^4$, $R^5$, $R^6$ and $R^7$, mutually independently, in each case denote H; F; Cl; Br; 1; $-NO_2$; -CN; $-NH_2$; -OH; -SH; $-NH-R^{55}$; $-NR^{56}R^{57}$ ; $-O-R^{67}$; $-S-R^{68}$; or denote an alkyl residue selected from the group consisting of methyl, ethyl, n-propyl, isopropyl, n-butyl, 2-butyl, isobutyl, *tert.*-butyl, n-pentyl and n-hexyl;
or $R^4$ and $R^5$ or $R^6$ and $R^7$, mutually independently, together in each case denote a residue selected from the group consisting of an oxo group (=O) and a thioxo group (=S);
or $R^3$ and $R^6$ together with the $-N-CR^4R^5-CR^7$ group joining them form a residue selected from the group consisting of

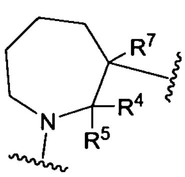 , 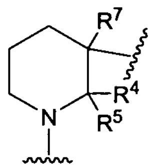 , 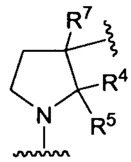 ,

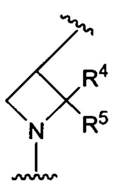 , 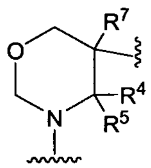 , 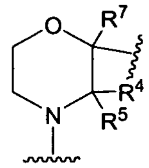 and 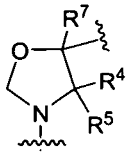

or $R^4$ and $R^8$ together with the $-CR^5-CR^6CR^7-N$ group joining them form a residue selected from the group consisting of

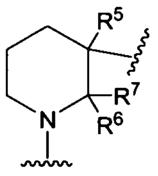 , 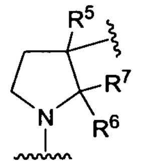 , 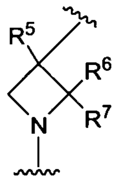 ,

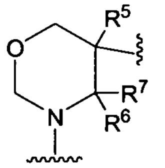 , 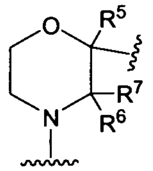 and 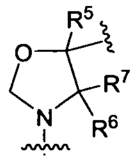

or $R^3$ and $R^8$ together with the $-N-CR^4R^5-CR^6CR^7-N$ group joining them form the following residue

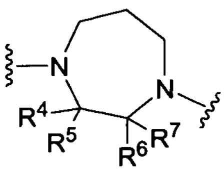

or $R^4$ and $R^6$ together with the $-CR^5-CR^7$ group joining them form the following residue

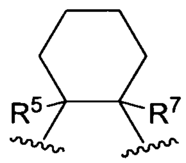

$R^9$ denotes a residue selected from the group consisting of phenyl, furyl, thienyl, pyrazolyl, pyrazinyl, pyridazinyl, pyrimidinyl, pyridinyl, pyrrolyl, oxazolyl, isoxazolyl, thiazolyl, thiadiazolyl, oxadiazolyl, triazolyl and imidazolyl, which is in each case unsubstituted or substituted with optionally 1, 2, 3, 4 or 5 substituents mutually independently selected from the group consisting of F, Cl, Br, I, -CN, methyl, ethyl, n-propyl, isopropyl, n-butyl, 2-butyl, isobutyl, *tert.*-butyl, ethenyl, allyl, ethynyl, propynyl, $-O-CH_3$, $-O-C_2H_5$, $-NO_2$, $-CF_3$, $-CH_2F$, $-CHF_2$, $-O-CF_3$ and $-S-CF_3$;
and $R^{55}$, $R^{56}$ , $R^{57}$ , $R^{58}$, $R^{59}$, $R^{67}$ and $R^{68}$, mutually independently, in each case denote an alkyl residue selected from the group consisting of methyl, ethyl, n-propyl, isopropyl, n-butyl, 2-butyl, isobutyl, *tert.*-butyl, n-pentyl and n-hexyl;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts.

**7.** Compounds according to one or more of claims 1 to 6, **characterised in that**

$R^1$ denotes H, CN, Cl, Br, F, methyl, ethyl, n-propyl, isobutyl, n-butyl, *tert.*-butyl, $-C(=O)-O-CH_3$, $-C(=O)-O-C_2H_5$ or $-C(=O)-O-C(CH_3)_3$;
$R^2$ denotes H, CN, Cl, Br, F, methyl, ethyl, n-propyl, isobutyl, n-butyl, *tert.*-butyl, $-C(=O)-O-CH_3$, $-C(=O)-O-C_2H_5$ or $-C(=O)-O-C(CH_3)_3$;
or $R^1$ and $R^2$ together with the carbon atoms joining them form a phenylene residue;
$R^3$ and $R^8$, mutually independently, in each case denote a residue selected from the group consisting of methyl, ethyl, n-propyl, isopropyl, n-butyl, 2-butyl, isobutyl and *tert.*-butyl; or denote cyclopropyl;
$R^4$, $R^5$ and $R^7$ in each case denote H;

$R^6$ denotes H or denotes an alkyl residue selected from the group consisting of methyl, ethyl, n-propyl, isopropyl, n-butyl, 2-butyl, isobutyl, *tert.*-butyl, n-pentyl and n-hexyl;
or $R^4$ and $R^5$ or $R^6$ and $R^7$, mutually independently, together in each case denote an oxo group (=O);
or $R^3$ and $R^6$ together with the $-N-CH_2-CH$ group joining them form a residue selected from the group consisting of

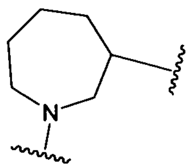 , 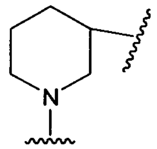 , 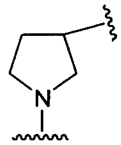 ,

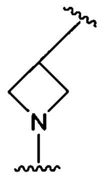 , 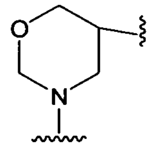 , 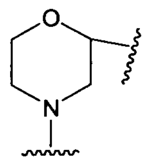 and 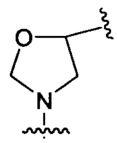

or $R^4$ and $R^8$ together with the $-CH-CH_2-N$ group joining them form a residue selected from the group consisting of

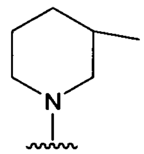 , 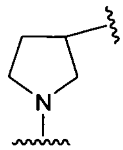 , 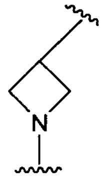 ,

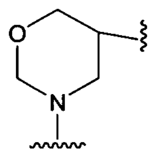 , 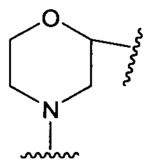 and 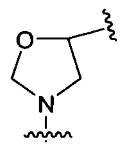

or $R^3$ and $R^8$ together with the $-N-CH_2-CH_2-N$ group joining them form the following residue

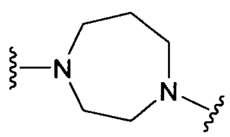

or $R^4$ and $R^6$ together with the -CH-CH group joining them form the following residue

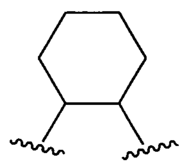

and $R^9$ denotes a residue selected from the group consisting of pyrid-2-yl, pyrid-3-yl, pyrid-4-yl, phenyl, 2-methylphenyl, 3-methylphenyl, 4-methylphenyl, 2-fluorophenyl, 3-fluorophenyl, 4-fluorophenyl, 2-cyanophenyl, 3-cyanophenyl, 4-cyanophenyl, 2-chlorophenyl, 3-chlorophenyl, 3-chlorophenyl, 2-trifluoromethylphenyl, 3-trifluoromethylphenyl, 4-trifluoromethylphenyl, 2-methoxyphenyl, 3-methoxyphenyl, 4-methoxyphenyl, (2,4)-difluorophenyl, (2,4)-dichlorophenyl, (3,5)-dichlorophenyl, (3,5)-difluorophenyl, 2-thiophenyl, 2-chloro-5-trifluoromethylphenyl, 3-fluoro-4-methylphenyl, 2-fluoro-3-methylphenyl, 2-difluoromethylphenyl, 3-difluoromethylphenyl, 4-difluoromethylphenyl, 2-fluoromethylphenyl, 3-fluoromethylphenyl, 4-fluoromethylphenyl, 3-nitrophenyl, 3-ethenylphenyl, 3-ethynylphenyl, 3-allylphenyl, 3-bromophenyl, 2-trifluoromethoxyphenyl, 3-trifuoromethoxyphenyl and 4-trifluoromethoxyphenyl;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts.

**8.** Compounds according to one or more of claims 1 to 7 selected from the group consisting of

[1] 3-phenyl-N-(1-(thiazol-2-yl)pyrrolidin-3-yl)propiolamide,
[2] N-methyl-3-phenyl-N-(1-(thiazol-2-yl)pyrrolidin-3-yl)propiolamide hydrochloride,
[3] 1-thiazol-2-yl-4-(3-phenylpropiolyl)-1,4-diazepane,
[4] N-Methyl-N-(2-(methyl(thiazol-2-yl)amino)ethyl)-3-phenylpropiolamide,
[5] N-(2-((thiazol-2-yl)amino)ethyl)-3-phenylpropiolamide,
[6] 3-phenyl-N-(2-(thiazol-2-ylamino)cyclohexyl)propiolamide,
[7] N-methyl-N-(2-(methyl(thiazol-2-yl)amino)ethyl)-3-(3-methylphenyl)-propiolamide,
[8] 3-phenyl-N-(1-(thiazol-2-yl)azetidin-3-yl)propiolamide,
[9] N-(2-(methyl(thiazol-2-yl)amino)ethyl)-3-phenylpropiolamide,
[10] N-methyl-N-(2-(thiazol-2-yl)amino)ethyl-3-phenylpropiolamide,
[11] 3-(thiazol-2-yl-amino)-1-(3-phenylpropiolyl)pyrrolidine,
[12] N-methyl-N-(2-(methyl(thiazol-2-yl)amino)cyclohexyl)-3-phenylpropiolamide,
[13] N-methyl-N-(2-(methyl(thiazol-2-yl)amino)ethyl)-3-(3-cyanophenyl)-propiolamide,
[14] N-methyl-N-(2-(methyl(thiazol-2-yl)amino)-2-oxoethyl)-3-phenylpropiolamide,
[15] 3-phenyl-N-(1-(thiazol-2-yl)piperidin-3-yl)propiolamide,
[16] N-methyl-N-(1-(thiazol-2-yl)piperidin-3-yl)-3-phenylpropiolamide,
[17] N-methyl-N-(2-(methyl(thiazol-2-yl)amino)ethyl)-3-(pyrid-2-yl)-propiolamide,
[18] N-methyl-N-(2-(methyl(thiazol-2-yl)amino)ethyl)-3-(3-chlorophenyl)-propiolamide,
[19] N-(2-(methyl(thiazol-2-yl)amino)ethyl)-3-(3-chlorophenyl)-propiolamide,
[20] N-methyl-N-(1-(thiazol-2-yl)azetidin-3-yl)-3-phenylpropiolamide,
[21] N-(2-(methyl(thiazol-2-yl)amino)ethyl)-3-(tol-3-yl)-propiolamide,
[22] 3-methyl(thiazol-2-yl)amino)-1-(3-phenylpropiolyl)pyrrolidine,
[23] N-(2-(methyl(thiazol-2-yl)-amino)ethyl)-3-(3-chlorophenyl)-propiolamide hydrochloride,
[24] N-(2-(benzo[d]thiazol-2-yl(methyl)amino)ethyl)-3-(3-chlorophenyl)-propiolamide,
[25] N-(2-methyl(5-ethoxycarbonylthiazol-2-yl)amino)ethyl)-3-(3-chlorophenyl)-propiolamide,
[26] N-(2-methyl(4-ethoxycarbonylthiazol-2-yl)amino)ethyl)-3-(3-chlorophenyl)-propiolamide,
[27] 3-(thiazol-2-yl-amino)-1-(3-phenylpropiolyl)piperidine,
[28] 3-(methyl-(thiazol-2-yl)-amino)-1-(3-phenylpropiolyl)piperidine,
[29] N-(2-(methyl-(4-methylthiazol-2-yl)amino)ethyl)-3-(3-chlorophenyl)-propiolamide,
[30] 3-(3-chlorophenyl)-N-(2-(methyl(5-methylthiazol-2-yl)amino)ethyl)-propiolamide
[31] N-(2-((5-bromothiazol-2-yl)(methyl)amino)ethyl)-3-(3-chlorophenyl)-propiolamide,
[32] 3-(3-chlorophenyl)-N-(1-(thiazol-2-yl)piperidin-3-yl)propiolamide,
[33] N-(2-((4-bromothiazol-2-yl)(methyl)amino)ethyl)-3-(3-chlorophenyl)-propiolamide,
[34] methyl2-((2-(3-(3-chlorophenyl)propiolamido)ethyl)(methyl)amino)thiazole-5-carboxylate,
[35] 1-(3-(3-chlorophenyl)propiolyl)-3-(methyl(thiazol-2-yl)amino)azetidine,
[36] 3-(3-chloropheny)-N-methyl-N-(1-(thiazol-2-yl)piperidin-3-yl)propiolamide,
[37] 3-(3-chlorophenyl)-N-(2-((4-chlorothiazol-2-yl)(methyl)amino)ethyl)-propiolamide,
[38] 3-(3-chlorophenyl)-N-(2-((5-chlorothiazol-2-yl)(methyl)amino)ethyl)-propiolamide,
[39] 3-(3-chlorophenyl)-N-(1-(thiazol-2-yl)azetidin-3-yl)propiolamide,
[40] 3-(3-chlorophenyl)-N-methyl-N-(1-(thiazol-2-yl)azetidin-3-yl)propiolamide,
[41] 3-(3-chlorophenyl)-N-(2-(ethyl(thiazol-2-yl)amino)ethyl)propiolamide,
[42] 3-(3-chlorophenyl)-N-(2-((5-cyanothiazol-2-yl)(methyl)amino)ethyl)-propiolamide,
[43] 1-(3-(3-chlorophenyl)propiolyl)-3-(methyl(5-fluorothiazol-2-yl)amino)azetidine,

[44] 1-(3-(3-chlorophenyl)propiolyl)-3-(methyl(5-fluorothiazol-2-yl)amino)pyrrolidine,
[45] N-(1-(4-methylthiazol-2-yl)pyrrolidin-3-yl)-3-phenylpropiolamide,
[46] 3-(3-methoxyphenyl)-N-(1-(4-methylthiazol-2-yl)pyrrolidin-3-yl)-propiolamide,
[47] 3-(2-methoxyphenyl)-N-(1-(4-methylthiazol-2-yl)pyrrolidin-3-yl)-propiolamide,
[48] 3-(4-methoxyphenyl)-N-(1-(4-methylthiazol-2-yl)pyrrolidin-3-yl)-propiolamide,
[49] 3-(4-fluorophenyl)-N-(1-(4-methylthiazol-2-yl)pyrrolidin-3-yl)propiolamide,
[50] N-(1-(4-methylthiazol-2-yl)pyrrolidin-3-yl)-3-p-tolylpropiolamide,
[51] N-(1-(4,5-dimethylthiazol-2-yl)pyrrolidin-3-yl)-3-phenylpropiolamide,
[52] N-(1-(4,5-dimethylthiazol-2-yl)pyrrolidin-3-yl)-3-(4-methoxyphenyl)-propiolamide,
[53] N-(1-(4,5-dimethylthiazol-2-yl)pyrrolidin-3-yl)-3-(4-fluorophenyl)-propiolamide,
[54] N-(1-(4,5-dimethylthiazol-2-yl)pyrrolidin-3-yl)-3-(2-methoxyphenyl)-propiolamide,
[55] N-(1-(4-*tert*.-butylthiazol-2-yl)pyrrolidin-3-yl)-3-(4-methoxyphenyl)-propiolamide,
[56] N-(1-(4-*tert*.-butylthiazol-2-yl)pyrrolidin-3-yl)-3-p-tolylpropiolamide,
[57] N-(1-(4-*tert*.-butylthiazol-2-yl)pyrrolidin-3-yl)-3-(4-fluorophenyl)-propiolamide,
[58] N-(1-(4-*tert*.-butylthiazol-2-yl)pyrrolidin-3-yl)-3-o-tolylpropiolamide,
[59] N-(1-(4-*tert*.-butylthiazol-2-yl)pyrrolidin-3-yl)-3-(3-fluorophenyl)-propiolamide,
[60] N-(1-(4-*tert*.-butylthiazol-2-yl)pyrrolidin-3-yl)-3-phenylpropiolamide,
[61] N-(1-(4-*tert*.-butylthiazol-2-yl)pyrrolidin-3-yl)-3-(2-fluorophenyl)-propiolamide,
[62] N-(1-(4-*tert*.-butylthiazol-2-yl)pyrrolidin-3-yl)-3-(3-methoxyphenyl)-propiolamide,
[63] N-(1-(4-*tert*.-butylthiazol-2-yl)pyrrolidin-3-yl)-3-(3-fluoro-4-methylphenyl)-propiolamide,
[64] N-(1-(4-*tert*.-butylthiazol-2-yl)pyrrolidin-3-yl)-3-(2-methoxyphenyl)-propiolamide,
[65] N-(1-(4-methylthiazol-2-yl)pyrrolidin-3-yl)-3-(4-(trifluoromethyl)phenyl)-propiolamide,
[66] N-(1-(4-methylthiazol-2-yl)pyrrolidin-3-yl)-3-o-tolylpropiolamide,
[67] 3-(3-fluorophenyl)-N-(1-(4-methylthiazol-2-yl)pyrrolidin-3-yl)propiolamide,
[68] 3-(3-fluoro-4-methylphenyl)-N-(1-(4-methylthiazol-2-yl)pyrrolidin-3-yl)-propiolamide,
[69] 3-(2,4-difluorophenyl)-N-(1-(4-methylthiazol-2-yl)pyrrolidin-3-yl)-propiolamide,
[70] 3-(2-fluorophenyl)-N-(1-(4-methylthiazol-2-yl)pyrrolidin-3-yl)propiolamide,
[71] N-(1-(4-methylthiazol-2-yl)pyrrolidin-3-yl)-3-(3-(trifluoromethyl)phenyl)-propiolamide,
[72] N-(1-(4-methylthiazol-2-yl)pyrrolidin-3-yl)-3-m-tolylpropiolamide,
[73] N-(1-(5-methylthiazol-2-yl)pyrrolidin-3-yl)-3-p-tolylpropiolamide,
[74] N-(1-(5-methylthiazol-2-yl)pyrrolidin-3-yl)-3-m-tolylpropiolamide,
[75] 3-(2-methoxyphenyl)-N-(1-(5-methylthiazol-2-yl)pyrrolidin-3-yl)-propiolamide,
[76] N-(1-(5-methylthiazol-2-yl)pyrrolidin-3-yl)-3-phenylpropiolamide,
[77] 3-(2-fluorophenyl)-N-(1-(5-methylthiazol-2-yl)pyrrolidin-3-yl)propiolamide,
[78] 3-(3-methoxyphenyl)-N-(1-(5-methylthiazol-2-yl)pyrrolidin-3-yl)-propiolamide,
[79] N-(1-(4,5-dimethylthiazol-2-yl)pyrrolidin-3-yl)-3-(3-fluoro-4-methylphenyl)-propiolamide,
[80] N-(1-(4,5-dimethylthiazol-2-yl)pyrrolidin-3-yl)-3-o-tolylpropiolamide,
[81] 3-(3-fluorophenyl)-N-(1-(5-methylthiazol-2-yl)pyrrolidin-3-yl)propiolamide,
[82] N-(1-(4,5-dimethylthiazol-2-yl)pyrrolidin-3-yl)-3-m-tolylpropiolamide,
[83] N-(1-(5-methylthiazol-2-yl)pyrrolidin-3-yl)-3-o-tolylpropiolamide,
[84] N-(1-(4,5-dimethylthiazol-2-yl)pyrrolidin-3-yl)-3-p-tolylpropiolamide,
[85] N-(1-(4,5-dimethylthiazol-2-yl)pyrrolidin-3-yl)-3-(3-(trifluoromethyl)phenyl)-propiolamide,
[86] 3-(4-methoxyphenyl)-N-(1-(5-methylthiazol-2-yl)pyrrolidin-3-yl)-propiolamide,
[87] N-(1-(5-methylthiazol-2-yl)pyrrolidin-3-yl)-3-(3-(trifluoromethyl)phenyl)-propiolamide,
[88] N-(1-(4,5-dimethylthiazol-2-yl)pyrrolidin-3-yl)-3-(4-(trifluoromethyl)phenyl)-propiolamide,
[89] 3-(4-fluorophenyl)-N-(1-(5-methylthiazol-2-yl)pyrrolidin-3-yl)propiolamide,
[90] N-(1-(4,5-dimethylthiazol-2-yl)pyrrolidin-3-yl)-3-(3-methoxyphenyl)-propiolamide,
[91] 3-(2,4-difluorophenyl)-N-(1-(4,5-dimethylthiazol-2-yl)pyrrolidin-3-yl)-propiolamide,
[92] N-(1-(4,5-dimethylthiazol-2-yl)pyrrolidin-3-yl)-3-(2-fluorophenyl)-propiolamide,
[93] N-(1-(5-methylthiazol-2-yl)pyrrolidin-3-yl)-3-(4-(trifluoromethyl)phenyl)-propiolamide,
[94] 3-(3-fluoro-4-methylphenyl)-N-(1-(5-methylthiazol-2-yl)pyrrolidin-3-yl)-propiolamide,
[95] 3-(2,4-difluorophenyl)-N-(1-(5-methylthiazol-2-yl)pyrrolidin-3-yl)-propiolamide,
[96] N-(1-(4-*tert*.-butylthiazol-2-yl)pyrrolidin-3-yl)-3-(2,4-difluorophenyl)-propiolamide,
[97] N-(1-(4-*tert*.-butylthiazol-2-yl)pyrrolidin-3-yl)-3-(4-(trifluoromethyl)phenyl)-propiolamide,
[98] N-(1-(4-*tert*.-butylthiazol-2-yl)pyrrolidin-3-yl)-3-(3-(trifluoromethyl)phenyl)-propiolamide,
[99] N-(1-(4-*tert*.-butylthiazol-2-yl)pyrrolidin-3-yl)-3-m-tolylpropiolamide,
[100] N-(1-(4-*tert*.-butylthiazol-2-yl)pyrrolidin-3-yl)-3-(3-chlorophenyl)-propiolamide,
[101] N-(1-(4-*tert*.-butylthiazol-2-yl)pyrrolidin-3-yl)-3-(thiophen-2-yl)propiolamide,

[102] N-(1-(4-*tert*.-butylthiazol-2-yl)pyrrolidin-3-yl)-3-(2,4-dichlorophenyl)-propiolamide,
[103] N-(1-(4-*tert*.-butylthiazol-2-yl)pyrrolidin-3-yl)-3-(2-chloro-5-(trifluoromethyl)-phenyl)propiolamide,
[104] N-(1-(4-*tert*.-butylthiazol-2-yl)pyrrolidin-3-yl)-3-(3,5-dichlorophenyl)-propiolamide,
[105] 3-(3-chlorophenyl)-N-(1-(4-methylthiazol-2-yl)pyrrolidin-3-yl)propiolamide,
[106] N-(1-(4-methylthiazol-2-yl)pyrrolidin-3-yl)-3-(thiophen-2-yl)propiolamide,
[107] 3-(2,4-dichlorophenyl)-N-(1-(4-methylthiazol-2-yl)pyrrolidin-3-yl)-propiolamide,
[108] 3-(2-chloro-5-(trifluoromethyl)phenyl)-N-(1-(4-methylthiazol-2-yl)pyrrolidin-3-yl)propiolamide,
[109] 3-(3,5-dichlorophenyl)-N-(1-(4-methylthiazol-2-yl)pyrrolidin-3-yl)-propiolamide,
[110] 3-(3-chlorophenyl)-N-(1-(4,5-dimethylthiazol-2-yl)pyrrolidin-3-yl)-propiolamide,
[111] 3-(2,4-dichlorophenyl)-N-(1-(4,5-dimethylthiazol-2-yl)pyrrolidin-3-yl)-propiolamide,
[112] 3-(3-chlorophenyl)-N-(1-(5-methylthiazol-2-yl)pyrrolidin-3-yl)propiolamide,
[113] N-(1-(5-methylthiazol-2-yl)pyrrolidin-3-yl)-3-(thiophen-2-yl)propiolamide,
[114] 3-(2,4-dichlorophenyl)-N-(1-(5-methylthiazol-2-yl)pyrrolidin-3-yl)-propiolamide,
[115] 3-(2-chloro-5-(trifluoromethyl)phenyl)-N-(1-(5-methylthiazol-2-yl)pyrrolidin-3-yl)propiolamide,
[116] 3-(3,5-dichlorophenyl)-N-(1-(5-methylthiazol-2-yl)pyrrolidin-3-yl)-propiolamide,
[117] N-(1-(4,5-dimethylthiazol-2-yl)pyrrolidin-3-yl)-3-(thiophen-2-yl)propiolamide,
[118] 3-(2-chloro-5-(trifluoromethyl)phenyl)-N-(1-(4,5-dimethylthiazol-2-yl)pyrrolidin-3-yl)propiolamide,
[119] N-(1-(5-methylthiazol-2-yl)pyrrolidin-3-yl)-3-(pyridin-2-yl)propiolamide,
[120] N-(1-(5-methylthiazol-2-yl)pyrrolidin-3-yl)-3-(pyridin-3-yl)propiolamide,
[121] N-(1-(5-methylthiazol-2-yl)pyrrolidin-3-yl)-3-(pyridin-4-yl)propiolamide,
[122] N-(1-(4-*tert*.-butylthiazol-2-yl)pyrrolidin-3-yl)-3-(pyridin-2-yl)propiolamide,
[123] N-(1-(4-*tert*.-butylthiazol-2-yl)pyrrolidin-3-yl)-3-(pyridin-3-yl)propiolamide, and
[126] 3-(3-chlorophenyl)-N-(2-((5-fluorothiazol-2-yl)(methyl)amino)ethyl)-propiolamide;

in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts.

**9.** A method for producing compounds of the general formula I according to one or more of claims 1 to 8, **characterised in that** at least one compound of the general formula II,

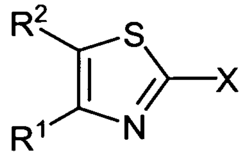

II,

in which the residues $R^1$ and $R^2$ have the meaning according to one or more of claims 1 to 8 and X denotes a leaving group, preferably a halogen residue or a sulfonic acid ester, particularly preferably a chlorine or bromine residue, is converted with at least one compound of the general formula III,

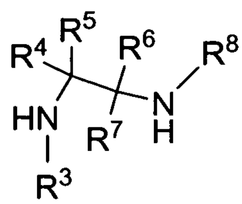

III,

in which $R^3$, $R^4$, $R^5$, $R^6$, $R^7$ and $R^8$ have the meaning according to one or more of claims 1 to 8, optionally in a reaction medium, optionally in the presence of at least one base and/or at least one organometallic compound

and/or at least one metal hydride reagent or in the presence of at least one copper salt and optionally in the presence of at least one metal, preferably at a temperature of -70°C to 300°C, particularly preferably of -70°C to 150°C, into at least one corresponding compound of the general formula IV, optionally in the form of a corresponding salt,

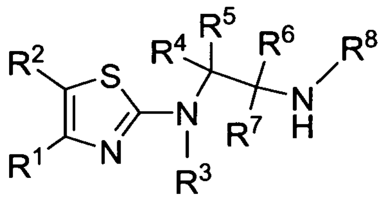

IV,

in which $R^1$, $R^2$, $R^3$, $R^4$, $R^5$, $R^6$, $R^7$ and $R^8$ have the above-stated meaning, and the latter is optionally purified and/or isolated;
or at least one compound of the general formula II is converted with at least one compound of the general formula V,

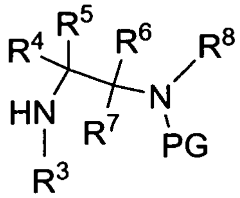

V,

in which $R^3$, $R^4$, $R^5$, $R^6$, $R^7$ and $R^8$ have the meaning according to one or more of claims 1 to 8 and PG denotes a protective group, preferably a protective group selected from the group consisting of *tert.*-butyloxycarbonyl, benzyl, benzyloxycarbonyl and 9-fluorenylmethyloxycarbonyl,
optionally in a reaction medium, optionally in the presence of at least one base and/or at least one organometallic compound and/or at least one metal hydride reagent, preferably at a temperature of -70°C to 300°C into at least one corresponding compound of the general formula VI,

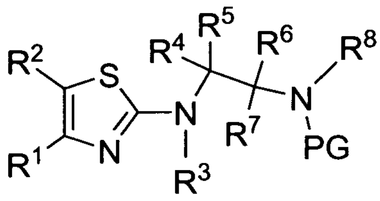

VI,

in which $R^1$, $R^2$, $R^3$, $R^4$, $R^5$, $R^6$, $R^7$, $R^8$ and PG have the above-stated meaning, and the latter is optionally purified and/or isolated;
and optionally at least one compound of the general formula VI, in which $R^1$, $R^2$, $R^4$, $R^5$, $R^6$, $R^7$, $R^8$ and PG have the above-stated meaning and $R^3$ denotes hydrogen, is converted with at least one compound $R^3$-X, in which $R^3$ has above-stated meaning with the exception of hydrogen and X denotes a leaving group, preferably a halogen residue, optionally in a reaction medium, optionally in the presence of at least one base, preferably

in the presence of at least one metal hydride reagent, particularly preferably in the presence of sodium hydride, preferably at a temperature of -70°C to 300°C into at least one corresponding compound of the general formula VIa,

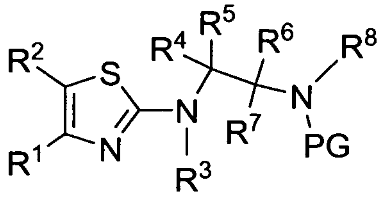

VIa,

in which $R^1$, $R^2$, $R^3$, $R^4$, $R^5$, $R^6$, $R^7$, $R^8$ and PG have the above-stated meaning and $R^3$ does not denote a hydrogen atom, and the latter is optionally purified and/or isolated;
or at least one compound of the general formula XIII,

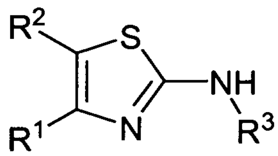

XIII,

in which $R^1$, $R^2$ and $R^3$ have the meaning according to one or more of claims 1 to 8, is converted with at least one compound of the general formula XIV,

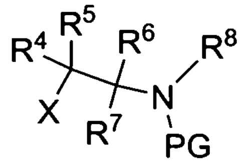

XIV,

in which $R^4$, $R^5$, $R^6$, $R^7$, $R^8$ and PG have the above-stated meaning and X denotes a leaving group, preferably a halogen residue or denotes a sulfonic acid ester, particularly preferably a chlorine or bromine residue, optionally in a reaction medium, optionally in the presence of at least one base, preferably in the presence of at least one base selected from the group consisting of potassium *tert.*-butylate, sodium hydroxide, potassium hydroxide, dimethylamine and triethylamine, particularly preferably in the presence of diethylamine, or optionally in the presence of at least one organometallic compound, preferably in the presence of at least one organometallic compound selected from the group consisting of methyllithium and butyllithium or optionally in the presence of at least one metal hydride compound, particularly preferably in the presence of sodium hydride, preferably at a temperature of -70°C to 300°C, particularly preferably of -70°C to 150°C, into at least one corresponding compound of the general formula VI and the latter is optionally purified and/or isolated;
or at least one compound of the general formula XIII,

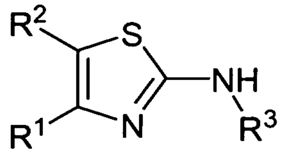

XIII,

in which $R^1$ , $R^2$ and $R^3$ have the meaning according to one or more of claims 1 to 8 is converted with at least one compound of the general formula XIVa,

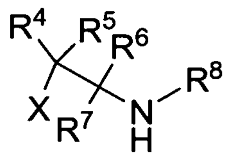

XIVa,

in which $R^4$, $R^5$, $R^6$, $R^7$ and $R^8$ have the above-stated meaning and X denotes a leaving group, preferably a halogen residue or denotes a sulfonic acid ester, particularly preferably a chlorine or bromine residue, optionally in a reaction medium, optionally in the presence of at least one base, preferably in the presence of at least one base selected from the group consisting of potassium *tert.*-butylate, sodium hydroxide, potassium hydroxide, dimethylamine and triethylamine, particularly preferably in the presence of diethylamine, or optionally in the presence of at least one organometallic compound, preferably in the presence of at least one organometallic compound selected from the group consisting of methyllithium and butyllithium or optionally in the presence of at least one metal hydride compound, particularly preferably in the presence of sodium hydride, preferably at a temperature of -70°C to 300°C, particularly preferably of -70°C to 150°C, into at least one corresponding compound of the general formula IV and the latter is optionally purified and/or isolated;
or at least one compound of the general formula VII,

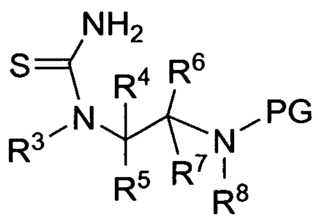

VII,

in which $R^3$, $R^4$, $R^5$, $R^6$, $R^7$ and $R^8$ have the meaning according to one or more of claims 1 to 8 and PG denotes a protective group, preferably a protective group selected from the group consisting of *tert.*-butyloxycarbonyl, benzyl, benzyloxycarbonyl and 9-fluorenylmethyloxycarbonyl, is converted by reaction with at least one compound of the general formula $R^1$-C(=O)-$CH_2$-X or ($C_{1-5}$-alkyl-O)$_2$-CH-$CH_2$-X, in which $R^1$ has the meaning according to one or more of claims 1 to 8 and X denotes a leaving group, preferably a halogen residue, particularly preferably a bromine atom, in a reaction medium, optionally in the presence of at least one organic base or in the presence of at least one acid, preferably in the presence of at least one base selected from the group consisting of triethylamine, diisopropylethylamine, N-methylmorpholine, dimethylaminopyridine and pyridine or in the presence of at least one acid selected from the group consisting of acetic acid, trifluoroacetic acid and hydrochloric acid, preferably at a temperature of between -70°C to 300°C into at least one corresponding compound of the general formula VI, optionally in the form of a corresponding salt, and the latter is optionally purified and/or isolated;

and at least one compound of the general formula VI or of the general formula VIa, in the event that PG denotes a *tert*.-butoxycarbonyl residue or 9-fluorenylmethyloxycarbonyl group, is converted in a reaction medium, in the presence of at least one acid, preferably in the presence of at least one acid selected from the group consisting of hydrochloric acid and trifluoroacetic acid, preferably at a temperature between -70°C to 100°C or, in the event that PG denotes a benzyl group or benzyloxycarbonyl group, in a reaction medium, in the presence of hydrogen and in the presence at least one catalyst, preferably in the presence of palladium on carbon, preferably at a temperature between -70°C to 100°C into at least one corresponding compound of the general formula IV, optionally in the form of a corresponding salt, and the latter is optionally purified and/or isolated;
and at least one compound of the general formula IV is converted by reaction with at least one compound of the general formula $R^9$-C≡-C-C(=O)-OH, in which $R^9$ has the meaning according to one or more of claims 1 to 8, in a reaction medium, optionally in the presence of at least one suitable coupling agent, which may be polymer-bound, optionally in the presence of at least one base, preferably at a temperature of -70°C to 100°C, or by reaction with at least one compound of the general formula $R^9$-C≡C-C(=O)-X, in which $R^9$ has the above-stated meaning and X denotes a leaving group, preferably a halogen residue, particularly preferably a chlorine or bromine residue, in a reaction medium, optionally in the presence of at least one base, preferably at a temperature of -70°C to 100°C, into at least one corresponding compound of the general formula I, optionally in the form of a corresponding salt,

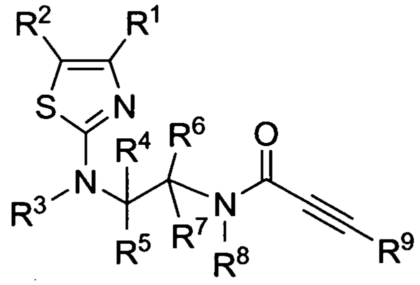

I,

in which $R^1$, $R^2$, $R^3$, $R^4$, $R^5$, $R^6$, $R^7$, $R^8$ and $R^9$ have the above-stated meaning, and the latter is optionally purified and/or isolated;
or at least one compound of the general formula IV is converted by reaction with propiolic acid [HC≡C-C(=O)-OH,] in a reaction medium, optionally in the presence of at least one suitable coupling agent, which may be polymer-bound, optionally in the presence of at least one base, preferably at a temperature of -70°C to 100°C, or by reaction with at least one compound of the general formula HC≡C-C(=O)-X, in which X denotes a leaving group, preferably a halogen residue, particularly preferably a chlorine or bromine residue, in a reaction medium, optionally in the presence of at least one base, preferably at a temperature of -70°C to 100°C, into at least one corresponding compound of the general formula VIII, optionally in the form of a corresponding salt,

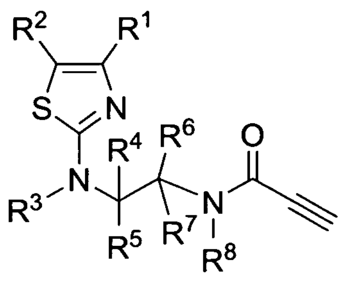

VIII,

in which $R^1$, $R^2$, $R^3$, $R^4$, $R^5$, $R^6$, $R^7$ and $R^8$ have the above-stated meaning, and the latter is optionally purified and/or isolated,
and at least one compound of the general formula VIII is converted by reaction with at least one compound of

the general formula $R^9$-X, in which $R^9$ has the meaning according to one or more of claims 1 to 8 with the exception of hydrogen and X denotes a leaving group, preferably a halogen residue or a sulfonic acid ester, particularly preferably iodine, bromine or triflate, in a reaction medium, optionally in the presence of at least one catalyst, preferably in the presence of at least one palladium catalyst selected from the group consisting of palladium chloride [$PdCl_2$], palladium acetate [$Pd(OAc)_2$], tetrakistriphenylphosphinepalladium [$Pd(PPh_3)_4$], bis-triphenylphosphinepalladium dichloride [$Pd(PPh_3)_2Cl_2$] and bistriphenylphosphinepalladium acetate [$Pd(PPh_2)_3(OAc)_2$], optionally in the presence of at least one ligand, preferably in the presence of at least one ligand selected from the group consisting of triphenylphosphine, triphenylarsine and tri-2-furylphosphine, optionally in the presence of at least one inorganic salt, preferably in the presence of at least one inorganic salt selected from the group consisting of lithium chloride and zinc chloride, optionally in the presence of at least one copper salt, preferably in the presence of copper iodide, optionally in the presence of at least one organic or inorganic base, preferably in the presence of at least one base selected from the group consisting of triethylamine, [1,4]-diazabicyclo-[2.2.2]-octane, diisopropylamine, diisopropylethylamine, potassium carbonate and sodium hydrogencarbonate, preferably at a temperature of between -70°C and 300°C, into at least one corresponding compound of the general formula I, optionally in the form of a corresponding salt, and the latter is optionally purified and/or isolated.

10. A method for producing compounds of the general formula I according to one or more of claims 1 to 8, **characterised in that** at least one compound of the general formula III,

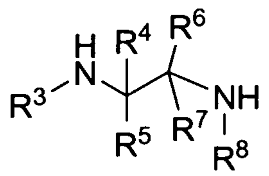

III,

in which $R^3$, $R^4$, $R^5$, $R^6$, $R^7$ and $R^8$ have the meaning according to one or more of claims 1 to 8,
is converted by reaction with at least one compound of the general formula $R^9$-C≡C-C(=O)-OH, in which $R^9$ has the meaning according to one or more of claims 1 to 8, in a reaction medium, optionally in the presence of at least one suitable coupling agent, which may be polymer-bound, optionally in the presence of at least one base, preferably at a temperature of -70°C to 100°C, or by reaction with at least one compound of the general formula $R^9$-C≡C-C(=O)-X, in which $R^9$ has the above-stated meaning and X denotes a leaving group, preferably a halogen residue, particularly preferably a chlorine or bromine residue, in a reaction medium, optionally in the presence of at least one base, preferably at a temperature of -70°C to 100°C, into at least one corresponding compound of the general formula IX, optionally in the form of a corresponding salt,

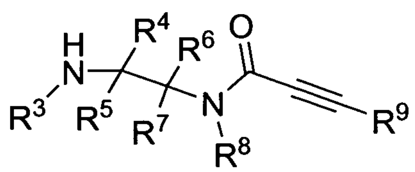

IX,

in which $R^3$, $R^4$, $R^5$, $R^6$, $R^7$, $R^8$ and $R^9$ have the above-stated meaning, and the latter is optionally purified and/or isolated;
or at least one compound of the general formula V,

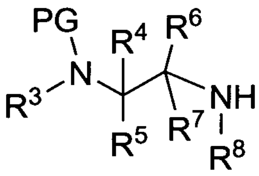

V,

in which $R^3$, $R^4$, $R^5$, $R^6$, $R^7$ and $R^8$ have the meaning according to one or more of claims 1 to 8 and PG denotes a protective group, preferably a protective group selected from the group consisting of *tert.*-butyloxycarbonyl, benzyl, benzyloxycarbonyl and 9-fluorenylmethyloxycarbonyl,
is converted by reaction with at least one compound of the general formula $R^9$-C≡C-C(=O)-OH, in which $R^9$ has the meaning according to one or more of claims 1 to 8, in a reaction medium, optionally in the presence of at least one suitable coupling agent, which may be polymer-bound, optionally in the presence of at least one base, preferably at a temperature of -70°C to 100°C, or by reaction with at least one compound of the general formula $R^9$-C≡C-C(=O)-X, in which $R^9$ has the above-stated meaning and X denotes a leaving group, preferably a halogen residue, particularly preferably a chlorine or bromine residue, in a reaction medium, optionally in the presence of at least one base, preferably at a temperature of -70°C to 100°C, into at least one corresponding compound of the general formula XI, optionally in the form of a corresponding salt,

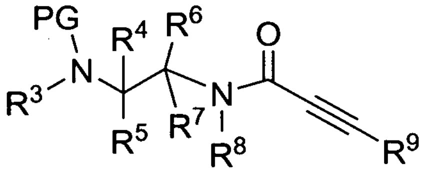

XI,

in which $R^3$, $R^4$, $R^5$, $R^6$, $R^7$, $R^8$, $R^9$ and PG have the above-stated meaning, and the latter is optionally purified and/or isolated;
and at least one compound of the general formula XI, in the event that PG denotes a tert.-butoxycarbonyl or 9-fluorenylmethyloxycarbonyl group, is converted in a reaction medium, in the presence of at least one acid, preferably in the presence of at least one acid selected from the group consisting of hydrochloric acid and trifluoroacetic acid, preferably at a temperature of between -70°C to 100°C or, in the event that PG denotes a benzyl or benzyloxycarbonyl group, in a reaction medium, in the presence of hydrogen and in the presence of at least one catalyst, preferably in the presence of palladium on carbon, preferably at a temperature of between -70°C to 100°C into at least one corresponding compound of the general formula IX, optionally in the form of a corresponding salt, and the latter is optionally purified and/or isolated;
and at least one compound of the general formula IX is converted by reaction with at least one compound of the general formula II,

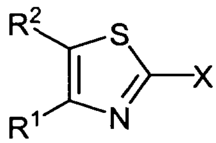

II,

in which the residues $R^1$ and $R^2$ have the meaning according to one or more of claims 1 to 8 and X denotes a

leaving group, preferably a halogen residue or a sulfonic acid ester, particularly preferably a chlorine or bromine residue, in a reaction medium, optionally in the presence of at least one base and/or at least one organometallic compound and/or at least one metal hydride reagent, preferably at a temperature of -70°C to 300°C into at least one corresponding compound of the general formula I, optionally in the form of a corresponding salt, in which $R^1$, $R^2$, $R^3$, $R^4$, $R^5$, $R^6$, $R^7$, $R^8$ and $R^9$ have the above-stated meaning, and the latter is optionally purified and/or isolated;

or optionally at least one compound of the general formula IX is converted by reaction with potassium thiocyanate and ethyl chloroformate or ammonium thiocyanate or trimethylsilyl isothiocyanate or thiophosgene and ammonia or cyanogen bromide and hydrogen sulfide in a reaction medium, optionally in the presence of at least one acid, preferably in the presence of at least one acid selected from the group consisting of hydrochloric acid, sulfuric acid, acetic acid and trifluoroacetic acid, particularly preferably in the presence of hydrochloric acid, or optionally in the presence of at least one base, preferably in the presence of at least one base selected from the group consisting of potassium *tert.*-butylate, sodium hydroxide, potassium hydroxide, dimethylamine and triethylamine, particularly preferably in the presence of diethylamine, or optionally in the presence of at least one organometallic compound, preferably in the presence of at least one organometallic compound selected from the group consisting of methyllithium and butyllithium or optionally in the presence of at least one metal hydride compound, particularly preferably in the presence of sodium hydride, preferably at a temperature of -70°C to 250°C, into at least one corresponding compound of the general formula XII, optionally in the form of a corresponding salt,

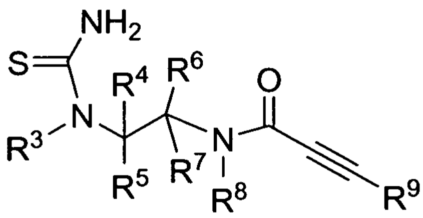

XII,

in which $R^3$, $R^4$, $R^5$, $R^6$, $R^7$, $R^8$ and $R^9$ have the above-stated meaning, and the latter is optionally purified and/or isolated;

and at least one compound of the general formula XII is converted by reaction with at least one compound of the general formula $R^1$-C(=O)-$CH_2$-X or ($C_{1-5}$-alkyl-O)$_2$-CH-$CH_2$-X, in which $R^1$ has the meaning according to one or more of claims 1 to 8 and X denotes a leaving group, preferably a halogen residue, particularly preferably a bromine atom, in a reaction medium, optionally in the presence of at least one organic base or in the presence of at least one acid, preferably in the presence of at least one base selected from the group consisting of triethylamine, diisopropylethylamine, N-methylmorpholine, dimethylaminopyridine and pyridine or in the presence of at least one acid selected from the group consisting of acetic acid, trifluoroacetic acid and hydrochloric acid, preferably at a temperature of between -70°C to 300°C into at least one corresponding compound of the general formula I, optionally in the form of a corresponding salt, and the latter is optionally purified and/or isolated.

**11.** A medicament containing at least one compound according to one or more of claims 1 to 8 and optionally one or more physiologically acceptable auxiliary substances.

**12.** Use of at least one compound according to one or more of claims 1 to 8 for the production of a medicament for the treatment and/or prevention of pain, preferably of pain selected from the group consisting of acute pain, chronic pain, neuropathic pain and visceral pain; migraine; depression; neurodegenerative diseases, preferably selected from the group consisting of multiple sclerosis, Alzheimer's disease, Parkinson's disease and Huntington's chorea; cognitive diseases, preferably cognitive deficiency states, particularly preferably attention deficit syndrome (ADS); anxiety states; panic attacks; epilepsy; coughing; urinary incontinence; diarrhoea; pruritus; schizophrenia; cerebral ischaemic episodes; muscle spasms; cramps; pulmonary diseases, preferably selected from the group comprising asthma and pseudocroup; regurgitation (vomiting); stroke; dyskinesia; retinopathy; lack of drive; laryngitis; disorders of food intake, preferably selected from the group consisting of bulimia, cachexia, anorexia and obesity; dependency on alcohol; dependency on medicines; dependency on drugs, preferably nicotine and/or cocaine dependency; alcohol abuse; abuse of medicines; drug abuse; preferably nicotine and/or cocaine abuse; withdrawal symptoms associated with dependency on alcohol, medicines and/or drugs (in particular nicotine and/or cocaine dependency); development of tolerance towards medicines, in particular towards natural or synthetic opioids; gastro-oesophageal reflux syndrome; gastro-oesophageal reflux disease; irritable bowel syndrome; for diuresis; for antinatriuresis; for influencing the cardiovascular system; for increasing vigilance; for increasing libido; for modulating locomotor activity or for local anaesthesia.

**13.** Use according to claim 12 for producing a medicament for the treatment and/or prevention of pain, preferably of pain selected from the group consisting of acute pain, chronic pain, neuropathic pain and visceral pain; anxiety states; panic attacks; dependency on alcohol; dependency on medicines; disorders of food intake, preferably selected from the group consisting of bulimia, cachexia, anorexia and obesity; dependency on drugs, preferably nicotine and/or cocaine dependency; alcohol abuse; abuse of medicines; drug abuse; preferably nicotine and/or cocaine abuse; withdrawal symptoms associated with dependency on alcohol, medicines and/or drugs (in particular nicotine and/or cocaine dependency); development of tolerance towards medicines and/or drugs, preferably towards natural or synthetic opioids; gastro-oesophageal reflux syndrome, gastro-oesophageal reflux disease and irritable bowel syndrome.

**14.** Use according to claim 12 or claim 13 for producing a medicament for the treatment of pain, preferably of pain selected from the group consisting of acute pain, chronic pain, neuropathic pain and visceral pain.

**15.** Use according to claim 12 or claim 13 for producing a medicament for the treatment of anxiety states or panic attacks.

**Revendications**

**1.** Thiazoles substitués de formule générale I,

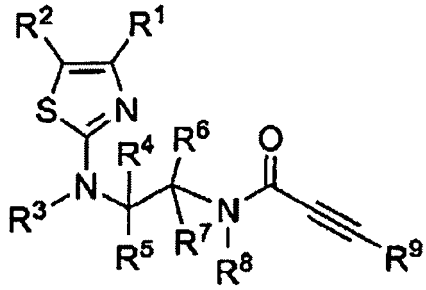

I,

où

$R^1$ et $R^2$, représentent, indépendamment l'un de l'autre, à chaque fois H ; F ; Cl ; Br ; I ; $-NO_2$ ; -CN ; $-NH_2$ ; -OH ; -SH ; -C(=O)-OH ; -C(=O)-H ; -NH- C (=O) -H ; $-NH-R^{55}$ ; $-NR^{56}R^{57}$ ; $-C(=O)-R^{58}$ ; $-C(=O)-O-R^{59}$ ; -O-C (=O) $-R^{60}$ $-NH-C(=O)-R^{61}$ ; $-NR^{62}-C(=O)-R^{63}$ ; $-C(=O)-NH_2$ ; $-C(=O)-NH-R^{64}$ ; $-C(=O)-NR^{65}R^{66}$ ; $-O-R^{67}$ ; $-S-R^{68}$ ; $-S(=O)-R^{69}$ ; $-S(=O)_2-R^{70}$ ; $-NH-C(=O)-NH-R^{71}$ ; $-NH-C(=S)-NH-R^{72}$ ; $-NH-S(=O)_2-R^{73}$ ; $-NR^{74}-S(=O)_2-R^{75}$ ; alkyle, alcényle ou alcynyle non substitué ou au moins monosubstitué ; hétéroalkyle, hétéroalcényle ou hétéroalcynyle non substitué ou au moins monosubstitué ; cycloalkyle ou cycloalcényle non substitué ou au moins monosubstitué ; hétérocycloalkyle ou hétérocycloalcényle non substitué ou au moins monosubstitué ; -(alkylène)- cycloalkyle, -(alcénylène)-cycloalkyle, -(alcynylène)-cycloalkyle, -(alkylène)- cycloalcényle, -(alcénylène)-cycloalcényle ou -(alcynylène)-cycloalcényle non substitué ou au moins monosubstitué ; -(hétéroalkylène)- cycloalkyle, -(hétéroalcénylène)-cycloalkyle, -(hétéroalkylène)-cycloalcényle ou -(hétéroalcénylène)-cycloalcényle non substitué ou au moins monosubstitué ; -(alkylène)- hétérocycloalkyle, -(alcénylène)- hétérocycloalkyle, -(alcynylène)- hétérocycloalkyle, -(alkylène)- hétérocycloalcényle, -(alcénylène)- hétérocycloalcényle ou -(alcynylène)- hétérocycloalcényle non substitué ou au moins monosubstitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou au moins monosubstitué ; aryle non substitué ou au moins monosubstitué ; hétéroaryle non substitué ou au moins monosubstitué ; -(alkylène)-aryle, -(alcénylène)-aryle, -(alcynylène)-aryle, -(hétéroalkylène)-aryle ou -(hétéroalcénylène)-aryle non substitué ou au moins monosubstitué ; ou -(alkylène)-hétéroaryle, -(alcénylène)-hétéroaryle, -(alcynylène)- hétéroaryle, -(hétéroalkylène)-hétéroaryle ou -(hétéroalcénylène)-hétéroaryle non substitué ou au moins monosubstitué ;

ou $R^1$ et $R^2$ forment, ensemble avec les atomes de carbone qui les relient un radical phénylène non substitué ou au moins monosubstitué ;

$R^3$, $R^8$ et $R^{54}$, représentent, indépendamment l'un de l'autre, à chaque fois H ; -C(=O)-$R^{58}$ ; -C(=O)-O- $R^{59}$ ; -C(=O)-$NH_2$ ; -C(=O)-NH-$R^{64}$ ; -C(=O)-N$R^{65}R^{66}$ ; -S(=O)-$R^{69}$ ; -S $(=O)_2$-$R^{70}$ ; alkyle, alcényle ou alcynyle non substitué ou substitué ; hétéroalkyle, hétéroalcényle ou hétéroalcynyle non substitué ou substitué ; cycloalkyle ou cycloalcényle non substitué ou substitué ; hétérocycloalkyle ou hétérocycloalcényle non substitué ou substitué ; -(alkylène)-cycloalkyle, -(alcénylène)-cycloalkyle, -(alcynylène)- cycloalkyle, -(alkylène)-cycloalcényle, -(alcénylène)-cycloalcényle ou -(alcynylène)- cycloalcényle non substitué ou substitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou substitué ; -(alkylène)-hétérocycloalkyle, -(alcénylène)-hétérocycloalkyle, -(alcynylène)- hétérocycloalkyle, -(alkylène)- hétérocycloalcényle, -(alcénylène)- hétérocycloalcényle ou -(alcynylène)- hétérocycloalcényle non substitué ou substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou substitué ; aryle non substitué ou substitué ; hétéroaryle non substitué ou substitué ; -(alkylène)-aryle, -(alcénylène)- aryle, -(alcynylène)-aryle, -(hétéroalkylène)- aryle ou -(hétéroalcénylène)-aryle non substitué ou substitué ; ou -(alkylène)-hétéroaryle, -(alcénylène)-hétéroaryle, -(alcynylène)- hétéroaryle, -(hétéroalkylène)-hétéroaryle ou -(hétéroalcénylène)-hétéroaryle non substitué ou substitué ;

$R^4$, $R^5$, $R^6$, $R^7$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$ et $R^{53}$, représentent, indépendamment l'un de l'autre, à chaque fois H ; F ; Cl ; Br ; I ; -$NO_2$ ; -CN ; -$NH_2$ ; -OH ; -SH ; -C(=O)-OH ; -C(=O)-H ; -NH- C(=)-H ; -NH-$R^{55}$ ; -N$R^{56}R^{17}$ ; -C(=O) -$R^{58}$ ; -C(=O)-O- $R^{59}$ ; -O-C (=O)-$R^{60}$ ; -NH-C(=O)-$R^{61}$ ; -N$R^{62}$-C(=O) -$R^{63}$ ; -C(=O) -$NH_2$ ; -C (=O) -NH-$R^{64}$ ; -C(=O) -N$R^{65}R^{66}$ ; -O- $R^{67}$ ; -S-$R^{68}$ ; -S(=O)-$R^{69}$ ; -$S(=O)_2$-$R^{70}$ ; -NH-C(=O)- NH-$R^{71}$ ; -NH-C(=S) -NH-$R^{72}$ ; -NH-$S(=O)_2$-$R^{73}$ ; -N$R^{74}$- S $(=O)_2$-$R^{75}$ ; alkyle, alcényle ou alcynyle non substitué ou substitué ; hétéroalkyle, hétéroalcényle ou hétéroalcynyle non substitué ou substitué ; cycloalkyle ou cycloalcényle non substitué ou substitué ; hétérocycloalkyle ou hétérocycloalcényle non substitué ou substitué ; -(alkylène)-cycloalkyle, -(alcénylène)- cycloalkyle, -(alcynylène)-cycloalkyle, -(alkylène)-cycloalcényle, -(alcénylène)- cycloalcényle ou -(alcynylène)-cycloalcényle non substitué ou substitué ; -(hétéroalkylène)- cycloalkyle, -(hétéroalcénylène)-cycloalkyle, -(hétéroalkylène)-cycloalcényle ou -(hétéroalcénylène)-cycloalcényle non substitué ou substitué ; -(alkylène)-hétérocycloalkyle, -(alcénylène)-hétérocycloalkyle, -(alcynylène)- hétérocycloalkyle, -(alkylène)- hétérocycloalcényle, -(alcénylène)-hétérocycloalcényle ou -(alcynylène)- hétérocycloalcényle non substitué ou substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou substitué ; aryle non substitué ou substitué ; hétéroaryle non substitué ou substitué ; -(alkylène)-aryle, -(alcénylène)- aryle, -(alcynylène)-aryle, -(hétéroalkylène)- aryle ou -(hétéroalcénylène)-aryle non substitué ou substitué ; ou -(alkylène)-hétéroaryle, -(alcénylène)-hétéroaryle, -(alcynylène)- hétéroaryle, -(hétéroalkylène)-hétéroaryle ou -(hétéroalcénylène)-hétéroaryle non substitué ou substitué ;

ou $R^4$ et $R^5$ ou $R^6$ et $R^7$ ou $R^{10}$ et $R^{11}$ ou $R^{12}$ et $R^{13}$ ou $R^{14}$ et $R^{15}$ ou $R^{16}$ et $R^{17}$ ou $R^{18}$ et $R^{19}$ ou $R^{20}$ et $R^{21}$ ou $R^{22}$ et $R^{23}$ ou $R^{24}$ et $R^{25}$ ou $R^{26}$ et $R^{27}$ ou $R^{28}$ et $R^{29}$ ou $R^{30}$ et $R^{31}$ ou $R^{32}$ et $R^{33}$ ou $R^{34}$ et $R^{35}$ ou $R^{36}$ et $R^{37}$ ou $R^{38}$ et $R^{39}$ ou $R^{40}$ et $R^{41}$ ou $R^{42}$ et $R^{43}$ ou $R^{44}$ et $R^{45}$ ou $R^{46}$ et $R^{47}$ ou $R^{48}$ et $R^{49}$ ou $R^{50}$ et $R^{51}$ ou $R^{52}$ et $R^{53}$, représentent, indépendamment l'un de l'autre, ensemble à chaque fois un radical choisi dans le groupe constitué par un groupe oxo (=O) et un groupe thioxo (=S) ;

ou $R^3$ et $R^4$ forment ensemble avec le groupe -N-C$R^5$- qui les relie un radical de formule générale A,

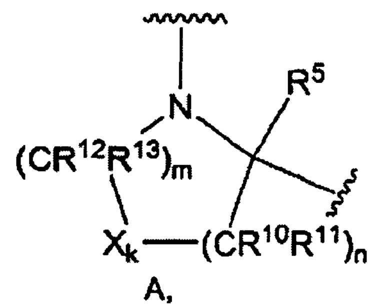

A,

ou $R^6$ et $R^8$ forment ensemble avec le groupe -N-C$R^7$- qui les relie un radical de formule générale B,

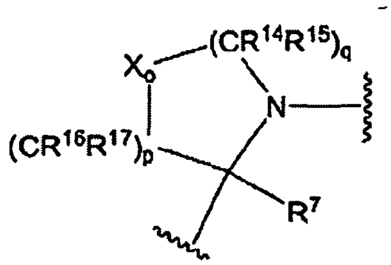

B,

m et q représentent à chaque fois 1, 2, 3, 4 ou 5 ;
n et p représentent à chaque fois 0, 1, 2, 3 où 4 ;
k et o représentent à chaque fois 0 ou 1 ;

où la somme de m, n et k ou la somme de p, q et o est à chaque fois égale à 1, 2, 3, 4, 5 ou 6 ;

ou $R^3$ et $R^6$ forment ensemble avec le groupe -N-$CR^4R^5$-$CR^7$- qui les relie un radical de formule générale C,

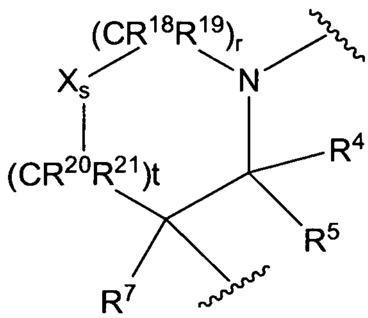

C,

ou $R^4$ et $R^8$ forment ensemble avec le groupe -$CR^5$-$CR^6R^7$-N- qui les relie un radical de formule générale D,

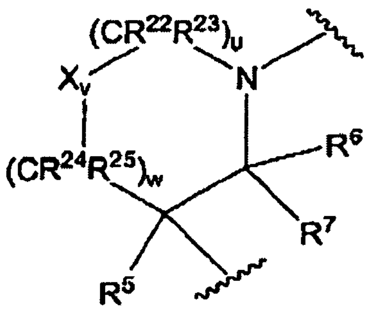

D,

r et u représentent à chaque fois 0, 1, 2, 3 ou 4 ;
t et w représentent à chaque fois 0, 1, 2 ou 3 ;
s et v représentent à chaque fois 0 ou 1 ;

où la somme de r, s et t ou la somme de u, v et w est à chaque fois égale à 1, 2, 3, 4 ou 5 ;

ou $R^3$ et $R^8$ forment ensemble avec le groupe -N-$CR^4R^5$- $CR^6R^7$-N- qui les relie un radical de formule générale E,

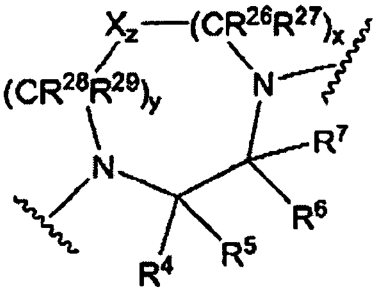

E,

x et y représentent à chaque fois 1 ou 2 ;
z représente 0 ou 1 ;

où la somme de x, y et z est égale à 3 ou 4 ;

ou $R^4$ et $R^6$ forment ensemble avec le groupe -$CR^5$-$CR^7$- qui les relie un radical de formule générale F,

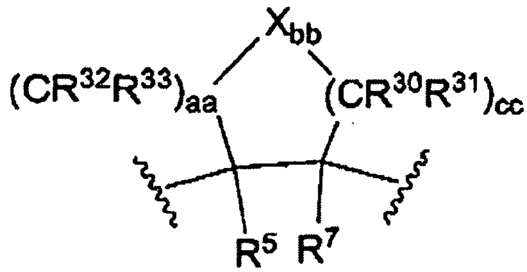

F,

aa et cc, représentent, indépendamment l'un de l'autre, à chaque fois 1, 2, 3, 4 ou 5 ;
bb représente 0 ou 1 ;

où la somme de aa, bb et cc est égale à 1, 2, 3, 4, 5 ou 6 ;

ou $R^3$ et $R^4$ ensemble avec le groupe -N-$CR^5$- qui les relie et $R^6$ et $R^8$ ensemble avec le groupe -N-$CR^7$- qui les relie forment un radical de formule générale G,

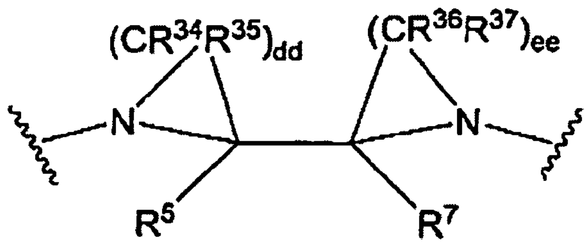

G,

dd et ee, représentent, indépendamment l'un de l'autre, à chaque fois 1, 2, 3 ou 4 ;
ou $R^3$ et $R^6$ ensemble avec le groupe -N-$CR^4CR^5$-$CR^7$- qui les relie et $R^4$ et $R^8$ ensemble avec le groupe -$CR^5$-$CR^6R^7$-N- qui les relie forment un radical de formule générale H,

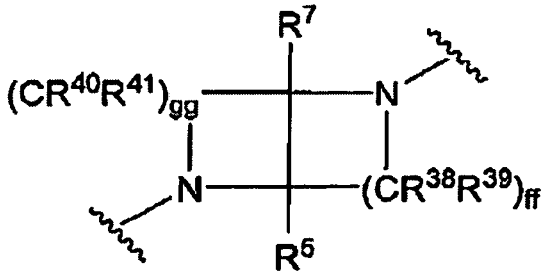

H,

ff et gg, représentent, indépendamment l'un de l'autre, à chaque fois 1, 2 ou 3 ;
ou $R^3$ et $R^8$ ensemble avec le groupe -N-$CR^4R^5$-$CR^6R^7$-N- qui les relie et $R^4$ et $R^6$ ensemble avec le groupe -$CR^5$- $CR^7$- qui les relie forment un radical de formule générale K,

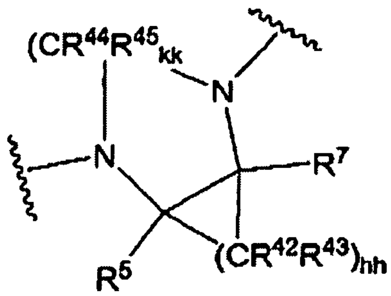

K,

hh représente 1, 2, 3 ou 4 ;
kk représente 1, 2, 3, 4, 5 ou 6 ;
ou $R^3$ et $R^8$ ensemble avec le groupe -N-$CR^4R^5$-$CR^6R^7$-N- qui les relie forment un radical bicyclique de formule générale L,

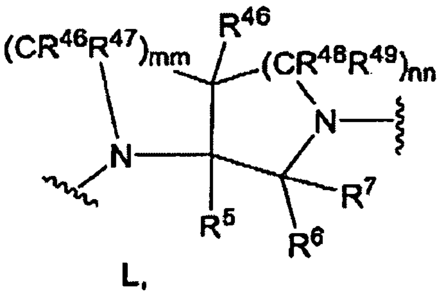

L,

mm représente 1, 2 ou 3 ;
nn représente 1 ou 2 ;
ou $R^3$ et $R^8$ ensemble avec le groupe -N-$CR^4R^5$-$CR^6R^7$-N- qui les relie forment un radical bicyclique de formule générale M,

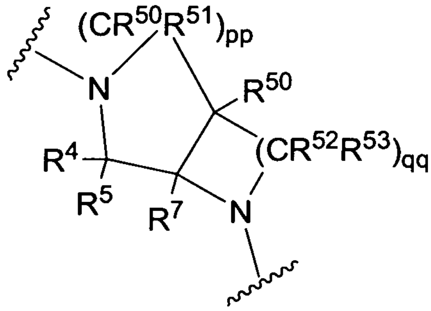

M,

pp représente 1 ou 2 ;
qq représente 1, 2 ou 3 ;
X représente O, S ou N-$R^{54}$ ;
$R^9$ représente H ; F ; Cl ; Br ; I ; -CN ; -C(=O) -OH ; -C(=O) -H ; -C(=O)-$R^{58}$ ; -C(-=O)-O-$R^{59}$ ; -C(=O) -$HN_2$ ; -C (=O) -NH-$R^{64}$ ; -C(=O) -N$R^{65}R^{66}$ ; alkyle, alcényle ou alcynyle non substitué ou substitué ; hétéroalkyle, hétéroalcényle ou hétéroalcynyle non substitué ou substitué ; cycloalkyle ou cycloalcényle non substitué ou substitué ; hétérocycloalkyle ou hétérocycloalcényle non substitué ou substitué ; -(alkylène)-cycloalkyle, -(alcénylène)-cycloalkyle, -(alcynylène)- cycloalkyle, -(alkylène)-cycloalcényle, -(alcénylène)-cycloalcényle ou -(alcynylène)- cycloalcényle non substitué ou substitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou substitué ; -(alkylène)-hétérocycloalkyle, -(alcénylène)-hétérocycloalkyle, -(alcynylène)- hétérocycloalkyle, -(alkylène)- hétérocycloalcényle, -(alcénylène)- hétérocycloalcényle ou -(alcynylène)- hétérocycloalcényle non substitué ou substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou substitué ; aryle non substitué ou substitué ; hétéroaryle non substitué ou substitué ; -(alkylène)-aryle, -(alcénylène)- aryle, -(alcynylène)-aryle, -(hétéroalkylène)- aryle ou -(hétéroalcénylène)-aryle non substitué ou substitué ; ou -(alkylène)-hétéroaryle, -(alcénylène)-hétéroaryle, -(alcynylène)- hétéroaryle, -(hétéroalkylène)-hétéroaryle ou- (hétéroalcénylène)-hétéroaryle non substitué ou substitué ;
et $R^{55}$, $R^{56}$, $R^{57}$, $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$ et $R^{75}$, représentent, indépendamment l'un de l'autre, à chaque fois alkyle, alcényle ou alcynyle non substitué ou substitué ; hétéroalkyle, hétéroalcényle ou hétéroalcynyle non substitué ou substitué ; cycloalkyle ou cycloalcényle non substitué ou substitué ; hétérocycloalkyle ou hétérocycloalcényle non substitué ou substitué ; -(alkylène)-cycloalkyle, -(alcénylène)- cycloalkyle, -(alcynylène)-cycloalkyle, -(alkylène)-cycloalcényle, -(alcénylène)- cycloalcényle ou -(alcynylène)-cycloalcényle non substitué ou substitué ; -(hétéroalkylène)- cycloalkyle, -(hétéroalcénylène)-cycloalkyle, -(hétéroalkylène)-cycloalcényle ou -(hétéroalcénylène)-cycloalcényle non substitué ou substitué ; -(alkylène)-hétérocycloalkyle, -(alcénylène)-hétérocycloalkyle, -(alcynylène)- hétérocycloalkyle, -(alkylène)- hétérocycloalcényle, -(alcénylène)- hétérocycloalcényle ou -(alcynylène)- hétérocycloalcényle non substitué ou substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou substitué ; aryle non substitué ou substitué ; hétéroaryle non substitué ou substitué ; -(alkylène)-aryle, -(alcénylène)- aryle, -(alcynylène)-aryle, -(hétéroalkylène)- aryle ou -(hétéroalcénylène)-aryle non substitué ou substitué ; ou -(alkylène)-hétéroaryle, -(alcénylène)-hétéroaryle, -(alcynylène)- hétéroaryle, -(hétéroalkylène)-hétéroaryle ou -(hétéroalcénylène)-hétéroaryle non substitué ou substitué ;

le cas échéant à chaque fois sous forme d'un de leurs stéréo-isomères purs, en particulier leurs énantiomères ou diastéréo-isomères, leurs mélanges racémiques ou sous forme d'un mélange de stéréo-isomères, en particulier des énantiomères et/ou des diastéréo-isomères, dans un rapport de mélange quelconque ou à chaque fois sous forme de sels correspondants.

**2.** Composés selon la revendication 1, **caractérisés en ce que**

$R^1$ et $R^2$, représentent, indépendamment l'un de l'autre, à chaque fois H ; F ; Cl ; Br ; I ; -$NO_2$ ; -CN ; -$NH_2$ ; -OH ; -SH ; -C(=O) -OH ; -C(=O) -H ; -NH- C(=O)-H ; -NH-$R^{55}$ ; -N$R^{56}R^{57}$ ; -C(=O)-$R^{58}$ ; -C(=O)-O- $R^{59}$ ; -O-C (=O)-$R^{60}$ ; -NH-C(=O)-$R^{61}$ ; -N$R^{62}$-C(=O)-$R^{63}$ ; -C(=O) -$N_H2$ ; -C(=O) -NH-$R^{64}$ ; -C(=O) -N$R^{65}R^{66}$ ; -O- $R^{67}$ ;

-S-$R^{68}$ ; -S(=O) -$R^{69}$ ; -S(=O)$_2$-$R^{70}$ ; -NH-C(=O)- NH-$R^{71}$ ; -NH-C(=S) -NH-$R^{72}$ ; -NH-S (=O)$_2$-$R^{73}$ ; -N$R^{74}$- S (=O)$_2$-$R^{75}$ ; alkyle, alcényle ou alcynyle non substitué ou au moins monosubstitué ; hétéroalkyle, hétéroalcényle ou hétéroalcynyle non substitué ou au moins monosubstitué ; cycloalkyle ou cycloalcényle non substitué ou au moins monosubstitué ; hétérocycloalkyle ou hétérocycloalcényle non substitué ou au moins monosubstitué ; -(alkylène)-cycloalkyle, -(alcénylène)-cycloalkyle, -(alcynylène)- cycloalkyle, -(alkylène)-cycloalcényle, -(alcénylène)-cycloalcényle ou -(alcynylène)- cycloalcényle non substitué ou au moins monosubstitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou au moins monosubstitué ; -(alkylène)- hétérocycloalkyle, -(alcénylène)- hétérocycloalkyle, -(alcynylène)- hétérocycloalkyle, -(alkylène)- hétérocycloalcényle, -(alcénylène)- hétérocycloalcényle ou -(alcynylène)- hétérocycloalcényle non substitué ou au moins monosubstitué ; -(hétéroalkylène)- hétérocycloalkyle, -(hétéroalcénylène)- hétérocycloalkyle, -(hétéroalkylène)- hétérocycloalcényle ou -(hétéroalcénylène)- hétérocycloalcényle non substitué ou au moins monosubstitué ; aryle non substitué ou au moins monosubstitué ; hétéroaryle non substitué ou au moins monosubstitué ; -(alkylène)-aryle, -(alcénylène)-aryle, -(alcynylène)-aryle, -(hétéroalkylène)-aryle ou -(hétéroalcénylène)- aryle non substitué ou au moins monosubstitué ; ou -(alkylène)-hétéroaryle, -(alcénylène)- hétéroaryle, -(alcynylène)-hétéroaryle, -(hétéroalkylène)-hétéroaryle ou -(hétéroalcénylène)-hétéroaryle non substitué ou au moins monosubstitué ;

ou $R^1$ et $R^2$ forment, ensemble avec les atomes de carbone qui les relient un radical phénylène non substitué ou au moins monosubstitué ;

$R^3$, $R^8$ et $R^{54}$, représentent, indépendamment l'un de l'autre, à chaque fois H ; -C(=O) -$R^{58}$ ; -C(=O)-O- $R^{59}$ ; -C(=O) -$NH_2$ ; -C(=O) -NH-$R^{64}$ ; -C(=O) -N$R^{65}R^{66}$ ; -S(=O) -$R^{69}$ ; -S(=O)$_2$-$R^{70}$ ; alkyle, alcényle ou alcynyle non substitué ou substitué ; hétéroalkyle, hétéroalcényle ou hétéroalcynyle non substitué ou substitué ; cycloalkyle ou cycloalcényle non substitué ou substitué ; hétérocycloalkyle ou hétérocycloalcényle non substitué ou substitué ; -(alkylène)-cycloalkyle, -(alcénylène)-cycloalkyle, -(alcynylène)- cycloalkyle, -(alkylène)-cycloalcényle, -(alcénylène)-cycloalcényle ou -(alcynylène)- cycloalcényle non substitué ou substitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou substitué ; -(alkylène)-hétérocycloalkyle, -(alcénylène)-hétérocycloalkyle, -(alcynylène)- hétérocycloalkyle, -(alkylène)- hétérocycloalcényle, -(alcénylène)- hétérocycloalcényle ou -(alcynylène)- hétérocycloalcényle non substitué ou substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou substitué ; aryle non substitué ou substitué ; hétéroaryle non substitué ou substitué ; -(alkylène)-aryle, -(alcénylène)- aryle, -(alcynylène)-aryle, -(hétéroalkylène)- aryle ou -(hétéroalcénylène)-aryle non substitué ou substitué ; ou -(alkylène)-hétéroaryle, -(alcénylène)-hétéroaryle, -(alcynylène)- hétéroaryle, -(hétéroalkylène)-hétéroaryle ou -(hétéroalcénylène)-hétéroaryle non substitué ou substitué ;

$R^4$, $R^5$, $R^6$, $R^7$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$ et $R^{53}$, représentent, indépendamment l'un de l'autre, à chaque fois H ; F ; Cl ; Br ; I ; -$NO_2$ ; -CN ; -$NH_2$ ; -OH ; -SH ; -C(=O) -OH ; -C(=O) -H ; -NH-C(=O) -H ; -NH-$R^{55}$ ; -N$R^{56}R^{57}$ ; -C(=O) -$R^{58}$ ; -C(=O) -O-$R^{59}$ ; -O-C(=O)-$R^{60}$ ; -NH-C(=O)-$R^{61}$ ; -N$R^{62}$- C(=O) -$R^{63}$ ; -C(=O) -$NH_2$ ; -C(=O)-NH-$R^{64}$ ; -C(=O)- N$R^{65}R^{66}$ ; -O-$R^{67}$ ; -S-$R^{68}$ ; -S(=O) -$R^{69}$ ; -S(=O)$_2$-$R^{70}$ ; -NH-C(=O)-NH-$R^{71}$ ; -NH-C(=S) -NH-$R^{72}$ ; -NH-S(=O)$_2$- $R^{73}$ ; -N$R^{74}$-S (=O)$_2$-$R^{75}$ ; alkyle, alcényle ou alcynyle substitué ou non substitué ; hétéroalkyle, hétéroalcényle ou hétéroalcynyle substitué ou non substitué ; cycloalkyle ou cycloalcényle substitué ou non substitué ; hétérocycloalkyle ou hétérocycloalcényle substitué ou non substitué ; -(alkylène)-cycloalkyle, -(alcénylène)- cycloalkyle, -(alcynylène)-cycloalkyle, -(alkylène)-cycloalcényle, -(alcénylène)- cycloalcényle ou -(alcynylène)-cycloalcényle substitué ou non substitué ; -(hétéroalkylène)- cycloalkyle, -(hétéroalcénylène)-cycloalkyle, -(hétéroalkylène)-cycloalcényle ou -(hétéroalcénylène)-cycloalcényle substitué ou non substitué ; -(alkylène)-hétérocycloalkyle, -(alcénylène)-hétérocycloalkyle, -(alcynylène)- hétérocycloalkyle, -(alkylène)- hétérocycloalcényle, -(alcénylène)- hétérocycloalcényle ou -(alcynylène)- hétérocycloalcényle substitué ou non substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle substitué ou non substitué ; aryle substitué ou non substitué ; hétéroaryle substitué ou non substitué ; -(alkylène)-aryle, -(alcénylène)- aryle, -(alcynylène)-aryle, -(hétéroalkylène)- aryle ou -(hétéroalcénylène)-aryle substitué ou non substitué ; ou -(alkylène)-hétéroaryle, -(alcénylène)-hétéroaryle, -(alcynylène)- hétéroaryle, -(hétéroalkylène)-hétéroaryle ou -(hétéroalcénylène)-hétéroaryle substitué ou non substitué ;

ou $R^4$ et $R^5$ ou $R^6$ et $R^7$ ou $R^{10}$ et $R^{11}$ ou $R^{12}$ et $R^{13}$ ou $R^{14}$ et $R^{15}$ ou $R^{16}$ et $R^{17}$ ou $R^{18}$ et $R^{19}$ ou $R^{20}$ et $R^{21}$ ou $R^{22}$ et $R^{23}$ ou $R^{24}$ et $R^{25}$ ou $R^{26}$ et $R^{27}$ ou $R^{28}$ et $R^{29}$ ou $R^{30}$ et $R^{31}$ ou $R^{32}$ et $R^{33}$ ou $R^{34}$ et $R^{35}$ ou $R^{36}$ et $R^{37}$ ou $R^{38}$ et $R^{39}$ ou $R^{40}$ et $R^{41}$ ou $R^{42}$ et $R^{43}$ ou $R^{44}$ et $R^{45}$ ou $R^{46}$ et $R^{47}$ ou $R^{48}$ et $R^{49}$ ou $R^{50}$ et $R^{51}$ ou $R^{52}$ et $R^{53}$, représentent, indépendamment l'un de l'autre, ensemble à chaque fois un radical choisi dans le groupe constitué par un groupe oxo (=O) et un groupe thioxo (=S) ;

ou $R^3$ et $R^4$ forment ensemble avec le groupe -N-$CR^5$- qui les relie un radical de formule générale A,

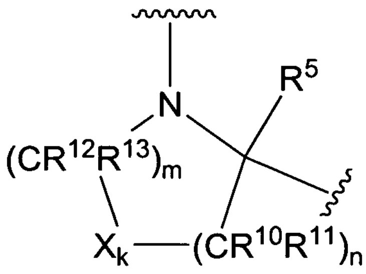

A,

ou $R^6$ et $R^8$ forment ensemble avec le groupe -N-$CR^7$- qui les relie un radical de formule générale B,

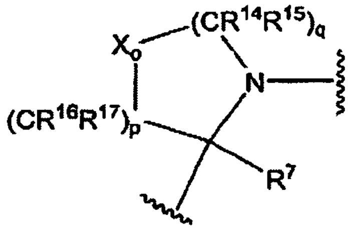

B,

m et q représentent à chaque fois 1, 2, 3, 4 ou 5 ;
n et p représentent à chaque fois 0, 1, 2, 3 ou 4 ;
k et o représentent à chaque fois 0 ou 1 ;

où la somme de m, n et k ou la somme de p, q et o est à chaque fois égale à 1, 2, 3, 4, 5 ou 6 ;

ou $R^3$ et $R^6$ forment ensemble avec le groupe -N-$CR^4R^5$-$CR^7$- qui les relie un radical de formule générale C,

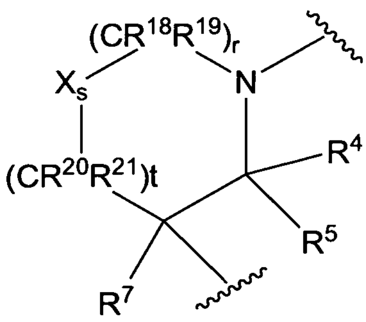

C,

ou $R^4$ et $R^8$ forment ensemble avec le groupe -$CR^5$-$CR^6R^7$-N- qui les relie un radical de formule générale D,

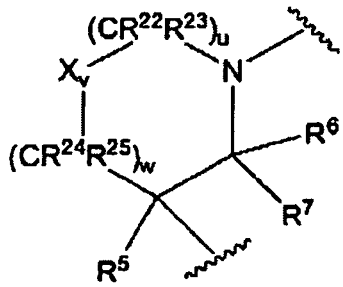

D,

r et u représentent à chaque fois 0, 1, 2, 3 ou 4 ;
t et w représentent à chaque fois 0, 1, 2 ou 3 ;
s et v représentent à chaque fois 0 ou 1 ;

où la somme de r, s et t ou la somme de u, v et w est à chaque fois égale à 1, 2, 3, 4 ou 5 ;

ou $R^3$ et $R^8$ forment ensemble avec le groupe $-N-CR^4R^5-CR^6R^7-N-$ qui les relie un radical de formule générale E,

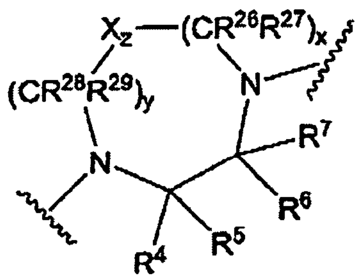

E,

x et y représentent à chaque fois 1 ou 2 ;
z représente 0 ou 1 ;

où la somme de x, y et z est égale à 3 ou 4 ;

ou $R^4$ et $R^6$ forment ensemble avec le groupe $-CR^5-CR^7-$ qui les relie un radical de formule générale F,

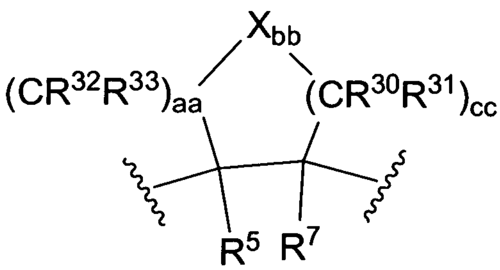

F

aa et cc, représentent, indépendamment l'un de l'autre, à chaque fois 1, 2, 3, 4 ou 5 ;
bb représente 0 ou 1 ;

où la ou 6 ;

somme de aa, bb et cc est égale à 1, 2, 3, 4, 5

ou $R^3$ et $R^4$ ensemble avec le groupe -N-CR$^5$- qui les relie et $R^6$ et $R^8$ ensemble avec le groupe -N-CR$^7$- qui les relie forment un radical de formule générale G,

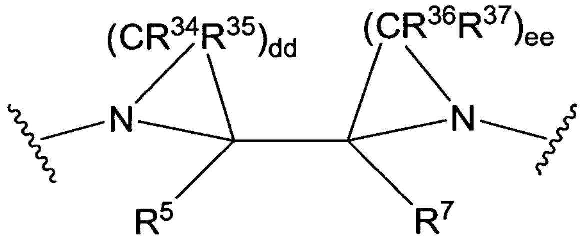

G

dd et ee, représentent, indépendamment l'un de l'autre, à chaque fois 1, 2, 3 ou 4 ;
ou $R^3$ et $R^6$ ensemble avec le groupe -N-CR$^4$CR$^5$-CR$^7$- qui les relie et $R^4$ et $R^8$ ensemble avec le groupe -CR$^5$-CR$^6$R$^7$-N- qui les relie forment un radical de formule générale H,

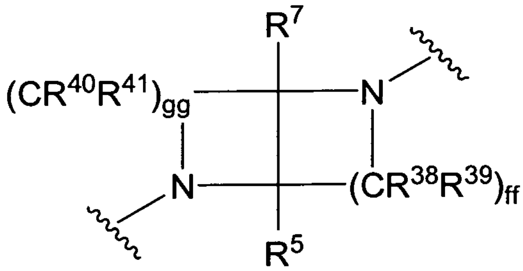

H

ff et gg, représentent, indépendamment l'un de l'autre, à chaque fois 1, 2 ou 3 ;
ou $R^3$ et $R^8$ ensemble avec le groupe -N-CR$^4$R$^5$-CR$^6$R$^7$-N- qui les relie et $R^9$ et $R^6$ ensemble avec le groupe -CR$^5$- CR$^7$- qui les relie forment un radical de formule générale K,

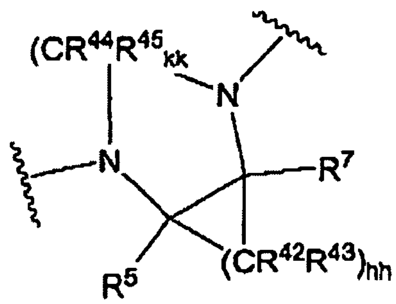

K,

hh représente 1, 2, 3 ou 4 ;
kk représente 1, 2, 3, 4, 5 ou 6 ;
ou $R^3$ et $R^8$ ensemble avec le groupe -N-CR$^4$R$^5$-CR$^6$R$^7$-N- qui les relie forment un radical bicyclique de formule générale L,

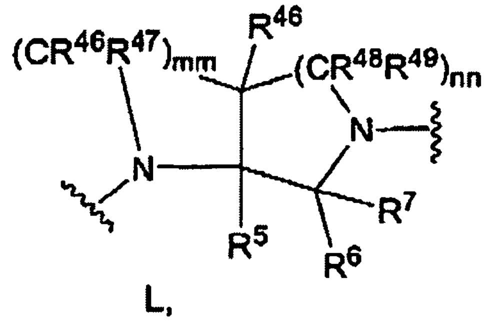

L,

mm représente 1, 2 ou 3 ;
nn représente 1 ou 2 ;
ou $R^3$ et $R^8$ ensemble avec le groupe $-N-CR^4R^5-CR^6R^7-N-$ qui les relie forment un radical bicyclique de formule générale M,

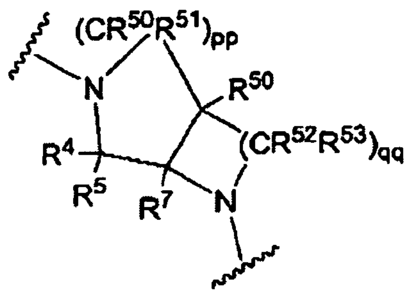

M,

pp représente 1 ou 2 ;
qq représente 1, 2 ou 3 ;
X représente O, S ou $N-R^{54}$;
$R^9$ représente H ; F ; Cl ; Br ; I ; -CN ; -C (=O) -OH ; -C (=O) -H ; -C (=O) $-R^{58}$ ; -C (=O) $-O-R^{59}$ ; -C (=O) $-NH_2$ ; - C (=O) $-NH-R^{64}$ ; -C (=O) $-NR^{65}R^{66}$ ; alkyle, alcényle ou alcynyle non substitué ou substitué ; hétéroalkyle, hétéroalcényle ou hétéroalcynyle non substitué ou substitué ; cycloalkyle ou cycloalcényle non substitué ou substitué ; hétérocycloalkyle ou hétérocycloalcényle non substitué ou substitué ; -(alkylène)-cycloalkyle, -(alcénylène)-cycloalkyle, -(alcynylène)- cycloalkyle, -(alkylène)-cycloalcényle, -(alcénylène)-cycloalcényle ou -(alcynylène)- cycloalcényle non substitué ou substitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou substitué ; -(alkylène)-hétérocycloalkyle, -(alcénylène)-hétérocycloalkyle, -(alcynylène)- hétérocycloalkyle, -(alkylène)- hétérocycloalcényle, -(alcénylène)- hétérocycloalcényle ou -(alcynylène)- hétérocycloalcényle non substitué ou substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou substitué ; aryle non substitué ou substitué ; hétéroaryle non substitué ou substitué ; -(alkylène)-aryle, -(alcénylène)- aryle, -(alcynylène)-aryle, -(hétéroalkylène)- aryle ou -(hétéroalcénylène)-aryle non substitué ou substitué ; ou -(alkylène)-hétéroaryle, -(alcénylène)-hétéroaryle, -(alcynylène)- hétéroaryle, -(hétéroalkylène)-hétéroaryle ou -(hétéroalcénylène)-hétéroaryle non substitué ou substitué ;
et $R^{55}$, $R^{56}$, $R^{57}$, $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$ et $R^{75}$, représentent, indépendamment l'un de l'autre, à chaque fois alkyle, alcényle ou alcynyle non substitué ou substitué ; hétéroalkyle, hétéroalcényle ou hétéroalcynyle non substitué ou substitué ; cycloalkyle ou cycloalcényle non substitué ou substitué ; hétérocycloalkyle ou hétérocycloalcényle non substitué ou substitué ; -(alkylène)-cycloalkyle, -(alcénylène)- cycloalkyle, -(alcynylène)-cycloalkyle, -(alkylène)-cycloalcényle, -(alcénylène)- cycloalcényle ou -(alcynylène)-cycloalcényle non substitué ou substitué ; -(hétéroalkylène)- cycloalkyle, -(hétéroalcénylène)-cycloalkyle, -(hétéroalkylène)-cycloalcényle ou -(hétéroalcénylène)-cycloalcényle non substitué ou substitué ; -(alkylène)-hétérocycloalkyle, -(alcénylène)-hétérocycloalkyle, -(alcynylène)- hétérocycloalkyle, -(alkylène)- hétérocycloalcényle, -(alcénylène)- hétérocycloalcényle ou -(alcynylène)- hétérocycloalcényle non substitué ou substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou substitué ; aryle non substitué ou substitué ; hétéroaryle non substitué ou substitué ; -(alkylène)-aryle, -(alcénylène)- aryle, -(alcynylène)-aryle, -(hétéroalkylène)- aryle ou -(hétéroalcénylène)-aryle non substitué ou substitué ; ou -(alkylène)-hétéroaryle, -(alcénylène)-hétéroaryle, -(alcynylène)- hétéroaryle, -(hétéroalkylène)-hétéroaryle ou -(hétéroalcénylène)-hétéroaryle non substitué ou substitué ;

où

- les radicaux alkyle susmentionnés sont à chaque fois ramifiés ou linéaires et présentent 1, 2, 3, 4, 5, 6, 7, 8, 9, 10, 11 ou 12 atomes de carbone comme éléments de chaîne ;
- les radicaux alcényle susmentionnés sont à chaque fois ramifiés ou linéaires et présentent 2, 3, 4, 5, 6, 7, 8, 9, 10, 11 ou 12 atomes de carbone comme éléments de chaîne ;
- les radicaux alcynyle susmentionnés sont à chaque fois ramifiés ou linéaires et présentent 2, 3, 4, 5, 6, 7, 8, 9, 10, 11 ou 12 atomes de carbone comme éléments de chaîne ;
- les radicaux hétéroalkyle, hétéroalcényle et hétéroalcynyle susmentionnés sont à chaque fois à 2, 3, 4, 5, 6, 7, 8, 9, 10, 11 ou 12 chaînons ;
- les radicaux hétéroalkyle, hétéroalcényle et hétéroalcynyle susmentionnés présentent à chaque fois le cas échéant 1, 2 ou 3 hétéroatome(s), choisis indépendamment l'un de l'autre dans le groupe constitué par oxygène, soufre et azote comme élément(s) de chaîne ;
- les radicaux alkyle, alcényle, alcynyle, hétéroalkyle, hétéroalcényle et hétéroalcynyle susmentionnés peuvent à chaque fois être substitués par le cas échant 1, 2, 3, 4 ou 5 substituants choisis indépendamment l'un de l'autre dans le groupe constitué par F, Cl, Br, I, $-NO_2$, -CN, -OH, -SH, $-NH_2$, $-N(C_{1-5}$-alkyle$)_2$, $-N(C_{1-5}$-alkyl) (phényle), -N ($C_{1-5}$-alkyl) ($CH_2$-phényle), -N ($C_{1-5}$-alkyl) ($CH_2$-$CH_2$-phényle), -C(=O)-H, -C(=O)-$C_{1-5}$-alkyle, -C (=O)-phényle, -C (=S) -$C_{1-5}$-alkyle, -C(=S)-phényle, -C(=O)-OH, -C(=O)-O-$C_{1-5}$-alkyle, -C(=O)-O-phényle, -C (=O)-$NH_2$, -C(=O)-NH-$C_{1-5}$-alkyle, -C(=O)-N($C_{1-5}$-alkyle$)_2$, -S(=O)-$C_{1-5}$-alkyle, -S(=O)-phényle, -S $(=O)_2$-$C_{1-5}$-alkyle, -S$(=O)_2$-phényle, -S$(=O)_2$-$NH_2$ et -$SO_3H$, où les radicaux phényle peuvent être substitués par 1, 2, 3, 4 ou 5 substituants choisis indépendamment l'un de l'autre dans le groupe constitué par F, Cl, Br, I, -CN, -$CF_3$, -OH, -$NH_2$, -O-$CF_3$, -SH, -O-$CH_3$, -O-$C_2H_5$, -O-$C_3H_7$, méthyle, éthyle, n-propyle, isopropyle, n-butyle, 2-butyle, isobutyle et tert-butyle ;- les radicaux cycloalkyle susmentionnés présentent à chaque fois 3, 4, 5, 6, 7, 8 ou 9 atomes de carbone comme éléments de cycle ;
- les radicaux cycloalcényle susmentionnés présentent à chaque fois 3, 4, 5, 6, 7, 8 ou 9 atomes de carbone comme éléments de cycle ;
- les radicaux hétérocycloalkyle susmentionnés sont à chaque fois à 3, 4, 5, 6, 7, 8 ou 9 chaînons ;
- les radicaux hétérocycloalcényle susmentionnés sont à chaque fois à 4, 5, 6, 7, 8 ou 9 chaînons ;
- les radicaux hétérocycloalkyle et hétérocycloalcényle susmentionnés présentent à chaque fois le cas échéant 1, 2 ou 3 hétéroatome(s) choisi(s) indépendamment l'un de l'autre dans le groupe constitué par oxygène, soufre et azote (NH) comme élément(s) de cycle ;
- les radicaux cycloalkyle, hétérocycloalkyle, cycloalcényle ou hétérocycloalcényle susmentionnés peuvent à chaque fois être substitués par le cas échéant 1, 2, 3, 4 ou 5 substituants choisis indépendamment l'un de l'autre dans le groupe constitué par F, Cl, Br, I, -CN, -$CF_3$, -OH, -$NH_2$, -O-$CF_3$, -SH, -O-$C_{1-5}$-alkyle, -O-phényle, -O-$CH_2$-phényle, - ($CH_2$) -O-$C_{1-5}$-alkyle, -S-$C_{1-5}$-alkyle, -S-phényle, -S-$CH_2$-phényle, -$C_{1-5}$-alkyle, -$C_{2-5}$-alcényle, -$C_{2-5}$-alcynyle, -C≡C-Si $(CH_3)_3$, -C≡C-Si$(C_2H_5)_3$, -C(=O)-O-$C_{1-5}$-alkyle, -C(=O)-$CF_3$, -S(=O)2-$C_{1-5}$-alkyle, -S(=O)-$C_{1-5}$-alkyle, -S$(=O)_2$-phényle, oxo (=O), thioxo (=S), -N ($C_{1-5}$-alkyle$)_2$, -N (H) ($C_{1-5}$-alkyle), -$NO_2$, -S-$CF_3$, -C(=O)-OH, -NH-S$(=O)_2$-$C_{1-5}$-alkyle, -NH-C (=O) -$C_{1-5}$-alkyle, -C(=O)-H, -C(=O)-$C_{1-5}$-alkyle, -C(=O)-$NH_2$, -C (=O) -N ($C_{1-5}$-alkyle$)_2$, -C(=O)-N(H) ($C_{1-5}$-alkyle) et phényle, où les radicaux phényle peuvent à chaque fois être non substitués ou substitués par 1, 2, 3, 4 ou 5 substituants choisis indépendamment l'un de l'autre dans le groupe constitué par F, Cl, Br, I, -CN, -$CF_3$, -OH, -$NH_2$, -O-$CF_3$, -SH, -O-$C_{1-5}$-alkyle, -O-phényle, -O-$CH_2$-phényle, - ($CH_2$) -O-$C_{1-5}$-alkyle,- S-$C_{1-5}$-alkyle, -S-phényle, -S-$CH_2$-phényle, -$C_{1-5}$-alkyle, -$C_{2-5}$-alcényle, -$C_{2-5}$-alcynyle, -C≡C-Si$(CH_3)_3$, -C≡C-Si$(C_2H_5)_3$, -C (=O) -O-$C_{1-5}$-alkyle et -C(=O)-$CF_3$, où les radicaux phényle susmentionnés peuvent de préférence être substitués par 1, 2, 3, 4 ou 5 substituants choisis indépendamment l'un de l'autre dans le groupe constitué par F, Cl, Br, I, -CN, -$CF_3$, -OH, -$NH_2$, -O-$CF_3$, -SH, -O-$CH_3$, -O-$C_2H_5$, -O-$C_3H_7$, méthyle, éthyle, n-propyle, isopropyle, n-butyle, 2-butyle, isobutyle et tert-butyle ;
- les radicaux alkylène susmentionnés sont à chaque fois ramifiés ou linéaires et présentent 1, 2, 3, 4, 5, 6, 7, 8, 9, 10, 11 ou 12 atomes de carbone comme éléments de chaîne ;
- les radicaux alcénylène susmentionnés sont à chaque fois ramifiés ou linéaires et présentent 2, 3, 4, 5, 6, 7, 8, 9, 10, 11 ou 12 atomes de carbone comme éléments de chaîne ;

- les radicaux alcynylène susmentionnés sont à chaque fois ramifiés ou linéaires et présentent 2, 3, 4, 5, 6, 7, 8, 9, 10, 11 ou 12 atomes de carbone comme éléments de chaîne ;
- les radicaux hétéroalkylène, hétéroalcénylène et hétéroalcynylène susmentionnés sont à chaque fois à 2, 3, 4, 5, 6, 7, 8, 9, 10, 11 ou 12 chaînons ;
- les groupes hétéroalkylène, hétéroalcénylène et hétéroalcynylène susmentionnés présentent à chaque fois le cas échéant 1, 2 ou 3 hétéroatome(s), choisis indépendamment l'un de l'autre dans le groupe constitué par oxygène, soufre et azote (NH) comme élément(s) de chaîne ;
- les groupes alkylène, alcénylène, alcynylène, hétéroalkylène, hétéroalcénylène et hétéroalcynylène susmentionnés peuvent à chaque fois être non substitués ou substitués par le cas échéant 1, 2, 3, 4 ou 5 substituants choisis indépendamment l'un de l'autre dans le groupe constitué par phényle, F, Cl, Br, I, $-NO_2$, -CN, -OH, -O-phényle, $-O-CH_2$-phényle, -SH, -S-phényle,- $S-CH_2$-phényle, $NH_2$, $-N(C_{1-5}$-alkyle$)_2$, -NH-phényle, $-N(C_{1-5}$-alkyl) (phényle) , $-N(C_{1-5}$-alkyl) $(CH_2$-phényle) , -N $(C_{1}$-$_5$-alkyl) $(CH_2-CH_2$-phényle) , -C (=O) -H, -C (=O) - $C_{1-5}$-alkyle, -C(=O)-phényle, -C (=S) $-C_{1-5}$-alkyle, -C(=S)-phényle, -C(=O)-OH, $-C(=O)-O-C_{1-5}$-alkyle, -C(=O)-O-phényle, $-C(=O)-NH_2$, $-C(=O)-NH-C_{1-5}$-alkyle, -C (=O) -N $(C_{1-5}$-alkyle$)_2$, -S (=O) $-C_{1-5}$-alkyle, -S(=O)-phényle, -S $(=O)_2-C_{1-5}$-alkyle, -S $(=O)_2$phényle, $-S(=O)_2-NH_2$ et $-SO_3H$, où les radicaux phényle peuvent être substitués par 1, 2, 3, 4 ou 5 substituants choisis indépendamment l'un de l'autre dans le groupe constitué par F, Cl, Br, I, -CN, $-NO_2$, -OH, -SH, $-NH_2$, -C(=O)-OH, $-C_{1-5}$-alkyle, - $(CH_2)$ $-O-C_{1-5}$-alkyle, $-C_{2-5}$-alcényle, $-C_{2-5}$-alcynyle, $-C\equiv C-Si (CH_3)_3$, $-C\equiv C-Si (C_2H_5)_3$, $-S-C_{1-5}$-alkyle, -S-phényle, $-S-CH_2$-phényle, $-O-C_{1-5}$-alkyle, -O-phényle, $-O-CH_2$-phényle, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ et $-S-CH_2F$ ;
- les radicaux aryle susmentionnés sont monocycliques ou bicycliques et présentent 6, 10 ou 14 atomes de carbone ;
- les radicaux hétéroaryle susmentionnés sont monocycliques, bicycliques ou tricycliques et à 5, 6, 7, 8, 9, 10, 11, 12, 13 ou 14 chaînons ;
- les radicaux hétéroaryle à 5 jusqu'à 14 chaînons susmentionnés présentent le cas échéant 1, 2, 3, 4 ou 5 hétéroatome(s), choisis indépendamment l'un de l'autre dans le groupe constitué par oxygène, soufre et azote (NH) comme élément (s) de chaîne ;
- et les radicaux phénylène, aryle et hétéroaryle susmentionnés peuvent à chaque fois être substitués par le cas échéant 1, 2, 3, 4 ou 5 substituants choisis indépendamment l'un de l'autre dans le groupe constitué par F, Cl, Br, I, -CN, $-NO_2$, -OH, -SH, $-NH_2$, -C(=O)-OH, $-C_{1-5}$-alkyle, $-(CH_2)-O-C_{1-5}$-alkyle, $-C_{2-5}$-alcényle, $-C_{2-5}$-alcynyle, $-C\equiv C-Si(CH_3)_3$, $-C\equiv C-Si(C_2H_5)_3$, $-S-C_{1-5}$-alkyle, -S-phényle, $-S-CH_2$-phényle, $-O-C_{1-5}$-alkyle, -O-phényle, $-O-CH_2$-phényle, $-CF_3$, $-CHF_2$, $-CH_2F$, $-O-CF_3$, $-O-CHF_2$,- $O-CH_2F$, $-C(=O)-CF_3$, $-S-CF_3$, $-S-CHF_2$, $-S-CH_2F$, -S $(=O)_2$-phényle, -S $(=O)_2-C_{1-5}$-alkyle, -S (=O) $-C_{1-5}$-alkyle, $-NH-C_{1-5}$-alkyle, $N(C_{1-5}$alkyle$)_2$, -C (=O)-$OC_{1-5}$-alkyle, -C(=O)-H, -C (=O) $-C_{1-5}$-alkyle, $-CH_2$-OC(=O)-phényle, -O-C(=O)-phényle, -NH-S $(=O)_2-C_{1-5}$-alkyle, $-NH-C(=O)-C_{1-5}$-alkyle, $-C(=O)-NH_2$, $-C(=O)-NH-C_{1-5}$-alkyle, -C (=O) -N $(C_{1-5}$-alkyle$)_2$, pyrazolyle, phényle, furyle (furannyle), thiazolyle, thiadiazolyle, thiophényle (thiényle), benzyle et phénéthyle, où les substituants cycliques ou, selon le cas, les radicaux cycliques de ces substituants eux-mêmes peuvent être substitués par le cas échéant 1, 2, 3, 4 ou 5, substituants choisis indépendamment l'un de l'autre dans le groupe constitué par F, Cl, Br, I, -CN, $-NO_2$, -OH, -SH, $-NH_2$, -C(=O)-OH, $-C_{1-5}$-alkyle, $-(CH_2)-O-C_{1-5}$-alkyle, $-C_{2-5}$-alcényle, $-C_{2-5}$-alcynyle, $-C\equiv C-Si (CH_3)_3$, $-C\equiv C-Si (C_2H_5)_3$, $-S-C_{1-5}$-alkyle, -S-phényle, $-S-CH_2$-phényle, $-O-C_{1-5}$-alkyle, -O-phényle, $-O-CH_2$-phényle, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ et $-S-CH_2F$ ;

le cas échéant à chaque fois sous forme d'un de leurs stéréo-isomères purs, en particulier leurs énantiomères ou diastéréo-isomères, leurs mélanges racémiques ou sous forme d'un mélange de stéréo-isomères, en particulier des énantiomères et/ou des diastéréo-isomères, dans un rapport de mélange quelconque ou à chaque fois sous forme de sels correspondants.

**3.** Composés selon la revendication 1 ou 2, **caractérisés en ce que**

$R^1$ représente H ; F ; Cl ; Br ; I ; $-CF_3$ ; $-NO_2$ ; -CN ; $-NH_2$ ; -OH ; -SH ; -C(=O)-OH; -C(=O)-H; -NH-C(=O)-H ; $-NH-R^{55}$ ; $-NR^{56}R^{57}$ ; $-C(=O)-R^{58}$ ; $-C(=O)-O-R^{59}$ ; $-C(=O)-NH_2$ ; $-C(=O)-NH-R^{61}$; -C(=O)- $NR^{65}R^{66}$ ; $-O-R^{67}$ ; $-S-R^{68}$ ; $-S(=O)-R^{69}$ ; $-S(=O)_2-R^{70}$ ; $C_{1-6}$-alkyle non substitué ou représente un radical choisi dans le groupe constitué par (1,3)- dioxolan-2-yle, phényle, benzyle, phénéthyle, oxadiazolyle, 2-pyridyle, 3-pyridyle, 4-pyridyle, 2-thiényle, 3-thiényle, 2-furyle et 3-furyle, qui est à chaque fois non substitué ou substitué par le cas échéant 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert-butyle, -OH, $-O-CH_3$, $-O-C_2H_5$ et -O- $C_3H_7$ ;

$R^2$ représente H ; F ; Cl ; Br ; I ; $-CF_3$ ; $-NO_2$ ; -CN ; $-NH_2$ ; -OH ; -SH ; -C(=O)-OH ; -C(=O)-H ; -NH-C(=O)-H ; $-NH-R^{55}$ ; $-NR^{56}R^{57}$; $-C(=O)-R^{58}$ ; $-C(=O)-O-R^{59}$ ; $-C(=O)-NH_2$ ; $-C(=O)-NH-R^{64}$ ; -C(=O)- $NR^{65}R^{66}$ ; $-O-R^{67}$ ; $-S-R^{68}$ ; $-S(=O)-R^{69}$ ; -S $(=O)_2-R^{70}$ ; $C_{1-6}$-alkyle non substitué ou représente un radical choisi dans le groupe constitué par (1,3)- dioxolan-2-yle, phényle, benzyle, phénéthyle, oxadiazolyle, 2-pyridyle, 3-pyridyle, 4-pyridyle, 2-thiényle, 3-thiényle, 2-furyle et 3-furyle, qui est à chaque fois non substitué ou substitué par le cas échéant 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert-butyle, -OH, $-O-CH_3$, $-O-C_2H_5$ et -O- $C_3H_7$ ;

ou $R^1$ et $R^2$ ensemble avec les atomes de carbone qui les relient forment un radical phénylène, qui est non substitué ou substitué par 1, 2, 3 ou 4 substituants choisis indépendamment l'un de l'autre dans le groupe constitué par F, Cl, Br, I, -CN, méthyle, éthyle, n-propyle, isopropyle, n- butyle, isobutyle, 2-butyle, tert-butyle, -OH, -SH, $-NH_2$, -C(=O)-OH, $-S-CH_3$, $-S-C_2H_5$, $-S(=O)-CH_3$, -S $(=O)_2-CH_3$, -S (=O) $-C_2H_5$, -S $(=O)_2-C_2H_5$, $-O-CH_3$, -O- $C_2H_5$, $-O-C_3H_7$, $-CF_3$, $-CHF_2$, $-CH_2F$ et $-O-CF_3$ ;

$R^3$ et $R^8$, représentent, indépendamment l'un de l'autre, à chaque fois H ; $-C(=O)-R^{58}$ ; $-C(=O)-O-R^{59}$ ;- C $(=O)-NH_2$; $-C(=O)-NH-R^{64}$ ; $-C(=O)-NR^{65}R^{66}$ ; -S(=O)- $R^{69}$ ; $-S(=O)_2-R^{70}$ ; $C_{1-6}$-alkyle, qui est non substitué ou substitué par le cas échéant 1, 2, 3, 4 ou 5 substituants choisis indépendamment l'un de l'autre dans le groupe constitué par F, Cl, Br, I, $-NO_2$, -CN, -OH, -SH et $-NH_2$ ; $C_{3-6}$-cycloalkyle, qui est non substitué ou substitué par le cas échéant 1, 2, 3, 4 ou 5 substituants choisis indépendamment l'un de l'autre dans le groupe constitué par F, Cl, Br, I, $-NO_2$, -CN, -OH, -SH et $-NH_2$ ; ou un radical phényle, qui peut à chaque fois être lié via un groupe $C_{1-3}$-alkylène, $C_{2-3}$-alcénylène ou $C_{2-3}$-alcynylène et/ou qui est non substitué ou substitué par 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert-butyle, -OH, $-O-CH_3$, $-O-C_2H_5$ et -O- $C_3H_7$ ;

$R^4$, $R^5$, $R^6$, $R^7$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$ et $R^{45}$ représentent, indépendamment l'un de l'autre à chaque fois H ; F ; Cl ; Br ; I ; $-NO_2$ ; -CN ; $-NH_2$ ; -OH ; -SH ; -C(=O)-OH ; $-NH-R^{55}$ ; $-NR^{56}R^{57}$ ; -C $(=O)-R^{58}$ ; $-C(=O)-O-R^{59}$ ; $-O-R^{67}$ ; -S- $R^{68}$ ; $C_{1-6}$-alkyle non substitué ; ou représentent un radical choisi dans le groupe constitué par phényle, benzyle et phénéthyle, qui est non substitué ou substitué par le cas échéant 1, 2, 3, 4 ou 5 substituants choisis indépendamment l'un de l'autre dans le groupe constitué par F, Cl, Br, I, -CN, méthyle, éthyle, n-propyle, isopropyle, n- butyle, 2-butyle, isobutyle, tert-butyle, -OH, -O- $CH_3$, $-O-C_2H_5$ et $-O-C_3H_7$ ;

ou $R^4$ et $R^5$ ou $R^6$ et $R^7$ou $R^{10}$ et $R^{11}$ ou $R^{12}$ et $R^{13}$ ou $R^{14}$ et $R^{15}$ ou $R^{16}$ et $R^{17}$ ou $R^{18}$ et $R^{19}$ ou $R^{20}$ et $R^{21}$ ou $R^{22}$ et $R^{23}$ ou $R^{24}$ et $R^{25}$ ou $R^{26}$ et $R^{27}$ ou $R^{28}$ et $R^{29}$ ou $R^{32}$ et $R^{33}$

ou $R^{34}$ et $R^{35}$ ou $R^{36}$ et $R^{37}$ ou $R^{38}$ et $R^{39}$ ou $R^{40}$ et $R^{41}$ ou $R^{42}$ et $R^{43}$ ou $R^{44}$ et $R^{45}$, représentent, indépendamment l'un de l'autre, ensemble à chaque fois un radical choisi dans le groupe constitué par un groupe oxo (=O) et un groupe thioxo (=S) ;

ou $R^3$ et $R^4$ forment ensemble avec le groupe $-N-CR^5-$ qui les relie un radical choisi dans le groupe constitué par

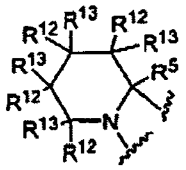 , 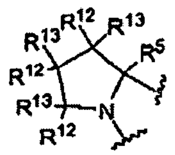 , 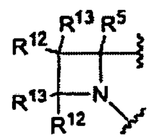 , 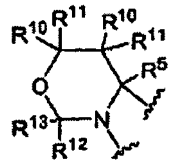 ,

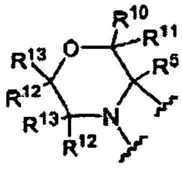 , 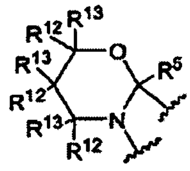 , 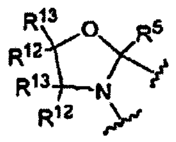 et 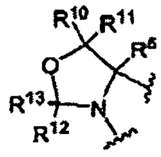

ou $R^6$ et $R^8$ forment ensemble avec le groupe $-N-CR^7-$ qui les relie un radical choisi dans le groupe constitué par

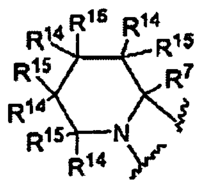, 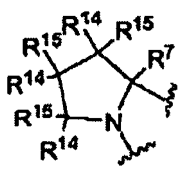, 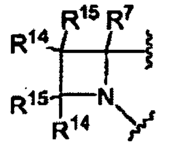, 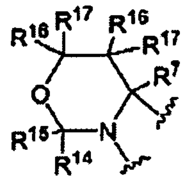

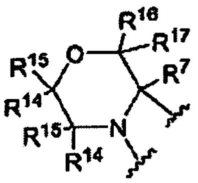, 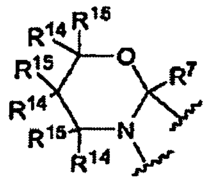, 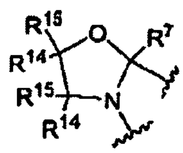 et 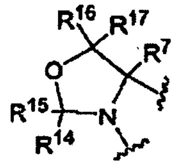

ou $R^3$ et $R^6$ forment ensemble avec le groupe -N-$CR^4R^5$-$CR^7$- qui les relie un radical choisi dans le groupe constitué par

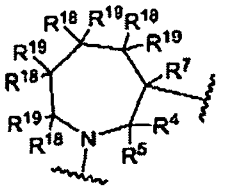, 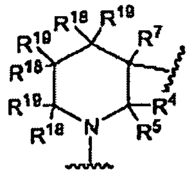, 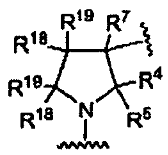,

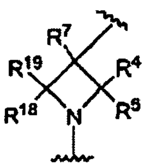, 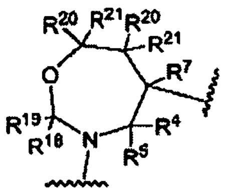, 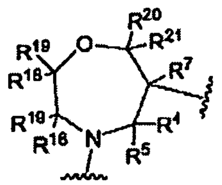, 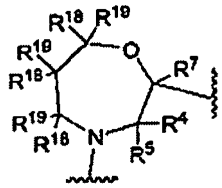,

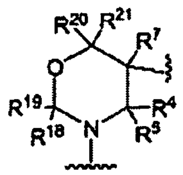, 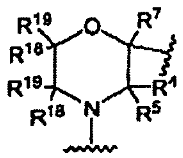 et 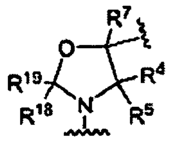

ou $R^4$ et $R^8$ forment ensemble avec le groupe -$CR^5$-$CR^6CR^7$-N- qui les relie un radical choisi dans le groupe constitué par

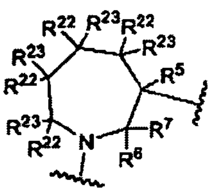, 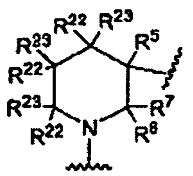, 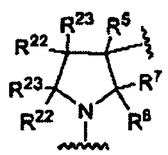,

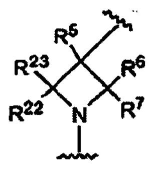 , 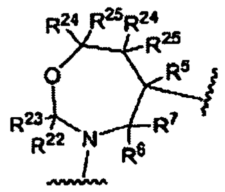 , 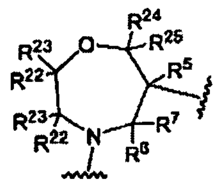 , 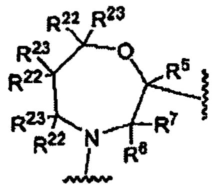 ,

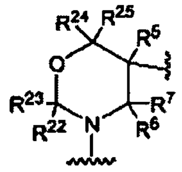 , 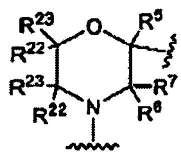 et 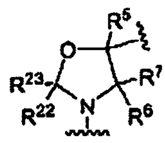

ou $R^3$ et $R^8$ forment ensemble avec le groupe $-N-CR^4R^5-CR^6CR^7-N-$ qui les relie un radical choisi dans le groupe constitué par

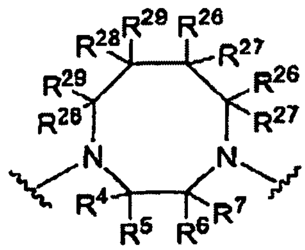 et 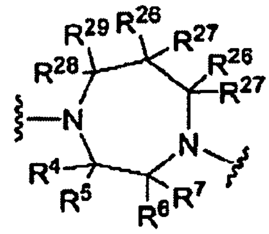

ou $R^4$ et $R^6$ forment ensemble avec le groupe $-CR^5-CR^7-$ qui les relie un radical choisi dans le groupe constitué par

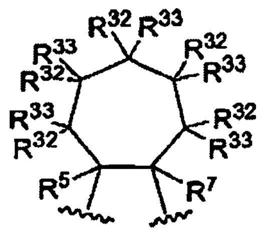 , 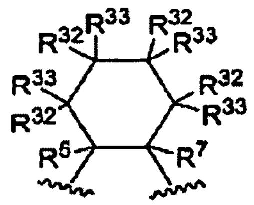 et 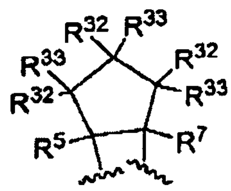

ou $R^3$ et $R^4$ ensemble avec le groupe $-N-CR^5-$ qui les relie et $R^6$ et $R^8$ ensemble avec le groupe $-N-CR^7-$ qui les relie forment un radical choisi dans le groupe constitué par

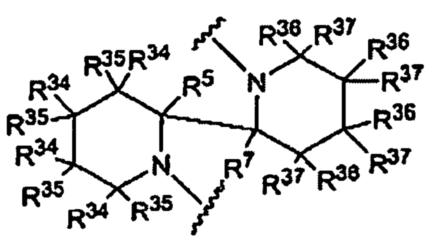 , 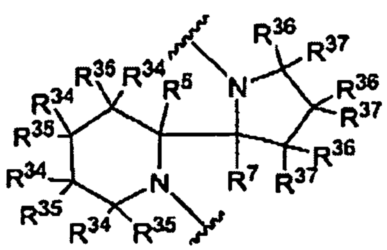 .

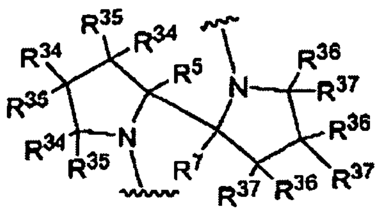

et

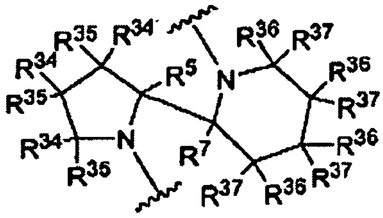

ou $R^3$ et $R^6$ ensemble avec le groupe -N-$CR^4CR^5$-$CR^7$- qui les relie et $R^9$ et $R^8$ ensemble avec le groupe -$CR^5$-$CR^6R^7$-N- qui les relie forment un radical choisi dans le groupe constitué par

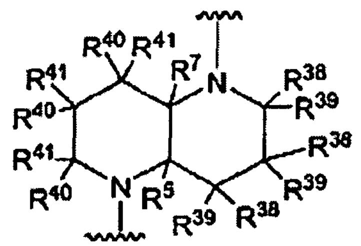

,

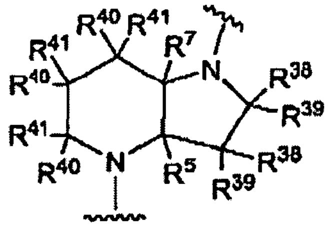

,

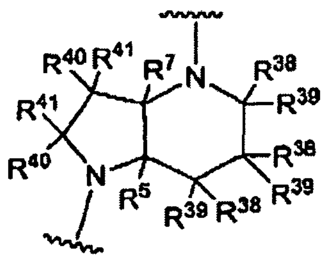

et

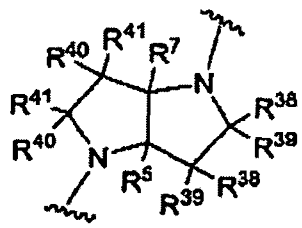

ou $R^3$ et $R^8$ ensemble avec le groupe -N-$CR^4R^5$-$CR^6R^7$- qui les relie et $R^4$ et $R^6$ ensemble avec le groupe -$CR^5$-$CR^7$- qui les relie forment un radical choisi dans le groupe constitué par

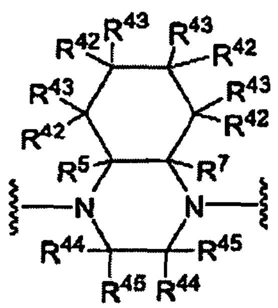

,

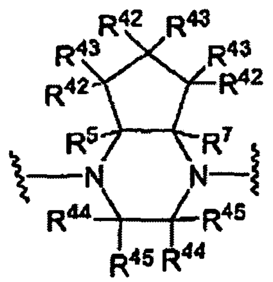

,

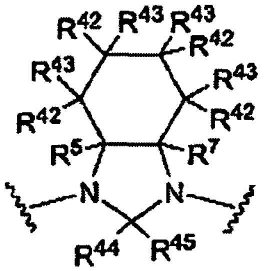

et

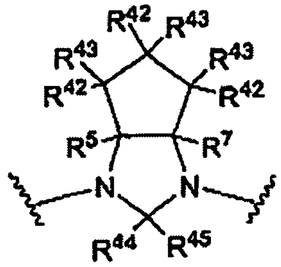

$R^9$ représente H ; -C(=O)-$NH_2$ ; -C(=O)-NH-$R^{64}$ ; -C(=O)- $NR^{65}R^{66}$ ; $C_{1-6}$-alkyle non substitué ; $C_{3-7}$-cycloalkyle non substitué ; $C_{5-6}$-cycloalcényle non substitué ; hétérocycloalkyle non substitué de 5 à 7 chaînons et hétérocycloalcényle non substitué de 5 à 7 chaînons ; ou un radical choisi dans le groupe constitué par phényle, naphtyle, anthracényle, furyle, thiényle, pyrazolyle, pyrazinyle, pyridazinyle, pyrimidinyle, pyridinyle, pyrrolyle, oxazolyle, isoxazolyle, thiazolyle, thiadiazolyle, oxadiazolyle, triazolyle, imidazolyle, indolyle, benzo[b]thiophényle, benzo[d]thiazolyle, benzo[b]furannyle, quinoléinyle, isoquinoléinyle et quinazolinyle, qui est à chaque fois non substitué ou substitué par le cas échéant 1, 2, 3, 4 ou 5 substituants choisis indépendamment l' un de l'autre dans le groupe constitué par F, Cl, Br, I, -CN, méthyle, éthyle, n-propyle, isopropyle, n- butyle, 2-butyle, isobutyle, tert-butyle, éthényle, allyle, éthynyle, propynyle, -C≡C- $Si(CH_3)_3$, -C≡C-Si $(C_2H_5)_3$, -$CH_2$-O-$CH_3$, -$CH_2$-O-$C_2H_5$, -OH, -O-$CH_3$, -O-$C_2H_5$, -O-$C_3H_7$, -S-$CH_3$, -S-$C_2H_5$, -S(=O)-$CH_3$, -S $(=O)_2$-$CH_3$, -S (=O) -$C_2H_5$, -S $(=O)_2$-$C_2H_5$, -$NH_2$, -$N(CH_3)_2$, -$N(C_2H_5)_2$, -NH-$CH_3$, -NH-$C_2H_5$, -$NO_2$, -$CF_3$, -$CH_2F$, -$CHF_2$, -O-$CF_3$, -S-$CF_3$, -SH, -NH-$S(=O)_2$- $CH_3$, -C(=O)-OH, -C(=O)-H ; -C(=O)-$CH_3$, -C(=O)-$C_2H_5$, -C(=O)-$NH_2$, -C(=O)-$N(CH_3)_2$, -C (=O)-NH-$CH_3$, -NH- C(=O)-$CH_3$, -NH-C(=O)-$C_2H_5$, -C(=O)-O-$CH_3$, -C(-O)-O- $C_2H_5$, -C(=O)-O-$C(CH_3)_3$ et phényle ;

et $R^{55}$,$R^{56}$, $R^{57}$, $R^{58}$, $R^{59}$, $R^{64}$, $R^{65}$, $R^{66}$, $R^{67}$, $R^{68}$, $R^{69}$ et $R^{70}$, représentent, indépendamment l'un de l'autre, à chaque fois $C_{1-6}$-alkyle non substitué ; $C_{3-7}$- cycloalkyle non substitué ; $C_{5-6}$-cycloalcényle non substitué ; hétérocycloalkyle non substitué de 5 à 7 chaînons et hétérocycloalcényle non substitué de 5 à 7 chaînons ; ou représentent un radical choisi dans le groupe constitué par phényle, benzyle, naphtyle, anthracényle, pyrrolyle, indolyle, furannyle, benzo[b]furannyle, thiophényle, benzo[b]thiophényle, benzo[d]thiazolyle, pyrazolyle, imidazolyle, thiazolyle, thiadiazolyle, triazolyle, oxazolyle, oxadiazolyle, isoxazolyle, pyridinyle, pyridazinyle, pyrimidinyle, pyrazinyle, pyrannyle, indazolyle, quinoléinyle, isoquinoléinyle et quinazolinyle, qui est à chaque fois non substitué ou substitué par le cas échéant 1, 2, 3, 4 ou 5 substituants choisis indépendamment l'un de l'autre dans le groupe constitué par F, Cl, Br, I, -CN, méthyle, éthyle, n-propyle, isopropyle, n- butyle, 2-butyle, isobutyle, tert-butyle, -OH, -O- $CH_3$, -O-$C_2H_5$, -O-$C_3H_7$, -$NH_2$, -$N(CH_3)_2$, -$N(C_2H_5)_2$, -NH- $CH_3$, -NH-$C_2H_5$, -$NO_2$, -$CF_3$, -O-$CF_3$; -S-$CF_3$, -SH, -NH- S(=O) 2-$CH_3$, -C(=O)-OH, -C(-O)-$CH_3$, -C(=O)-$C_2H_5$, -C(=O)-$N(CH_3)_2$, -C(=O)-NH-$CH_3$, -NH-C(=O)-$CH_3$, -NH- C(=O)-$C_2H_5$, -C(=O)-O-$CH_3$ et -C(=O)-O-$C_2H_5$ ;

le cas échéant à chaque fois sous forme d'un de leurs stéréo-isomères purs, en particulier leurs énantiomères ou diastéréo-isomères, leurs mélanges racémiques ou sous forme d'un mélange de stéréo-isomères, en particulier des énantiomères et/ou des diastéréo-isomères, dans un rapport de mélange quelconque ou à chaque fois sous forme de sels correspondants.

**4.** Composés selon l'une ou plusieurs des revendications 1 à 3, **caractérisés en ce que**

$R^1$ représente H ; F ; Cl ; Br ; I ; -$CF_3$ ; -$NO_2$ ; -CN ; -C(=O)-OH ; -C(=O)-O-$R^{59}$; -C(=O)-$NH_2$ ; -C(=O)-NH-$R^{64}$; -C(=O)-$NR^{65}R^{66}$; -O-$R^{67}$ ; -S-$R^{68}$ ; -S(=O)-$R^{69}$ ; -S $(=O)_2$-$R^{70}$ ; un radical alkyle choisi dans le groupe constitué par méthyle, éthyle, n- propyle, isopropyle, n-butyle, 2-butyle, isobutyle et tert-butyle ; ou représente un radical choisi dans le groupe constitué par (1,3)-dioxolan-2-yle, phényle, benzyle, phénéthyle, oxadiazolyle, 2- pyridyle, 3-pyridyle, 4-pyridyle, 2-thiényle, 3- thiényle, 2-furyle et 3-furyle, qui est à chaque fois non substitué ou substitué par le cas échéant 1, 2, 3, 4 ou 5 substituants choisis indépendamment l'un de l'autre dans le groupe constitué par F, Cl, Br, méthyle, éthyle, n-propyle, isopropyle, n-butyle, 2-butyle, isobutyle, tert-butyle, -OH, -O-$CH_3$, -O-$C_2H_5$ et -0-$C_3H_7$ ;

$R^2$ représente H ; F ; Cl ; Br ; I ; -$CF_3$ ; -$NO_2$ ; -CN ; -C(=O)-OH ; -C(=O)-O-$R^{59}$ ; -C(=O)-$NH_2$ ; -C(=O)-NH-$R^{64}$ ; -C(=O)-$NR^{65}R^{66}$ ; -O-$R^{67}$ ; -S-$R^{68}$ ; -S(=O)-$R^{69}$ ; -$S(=O)_2$-$R^{70}$ ; un radical alkyle choisi dans le groupe constitué par méthyle, éthyle, n-propyle, isopropyle, n-butyle, 2-butyle, isobutyle et tert-butyle ; ou représente un radical

choisi dans le groupe constitué par (1,3)-dioxolan-2-yle, phényle, benzyle, phénéthyle, oxadiazolyle, 2-pyridyle, 3-pyridyle, 4-pyridyle, 2-thiényle, 3-thiényle, 2-furyle et 3-furyle, qui est à chaque fois non substitué ou substitué par le cas échéant 1, 2, 3, 4 ou 5 substituants choisis indépendamment l'un de l'autre dans le groupe constitué par F, Cl, Br, méthyle, éthyle, n-propyle, isopropyle, n-butyle, 2-butyle, isobutyle, tert-butyle, -OH, $-O-CH_3$, $-O-C_2H_5$ et $-0-C_3H_7$ ;

ou $R^1$ et $R^2$ ensemble avec les atomes de carbone qui les relient forment un radical phénylène, qui est non substitué ou substitué par 1, 2, 3 ou 4 substituants choisis indépendamment l'un de l'autre dans le groupe constitué par F, Cl, Br, I, -CN, méthyle, éthyle, n-propyle, isopropyle, n-butyle, isobutyle, 2-butyle, tert-butyle, $-O-CH_3$, $-O-C_2H_5$, $-O-C_3H_7$, $-CF_3$, $-CHF_2$, $-CH_2F$ et $-O-CF_3$ ;

$R^3$ et $R^8$, représentent, indépendamment l'un de l'autre, à chaque fois H ; $-C(=O)-R^{58}$; $-C(=O)-O-R^{59}$ ; $-S(=O)-R^{69}$ ; $-S(=O)_2-R^{70}$ ; un radical alkyle choisi dans le groupe constitué par méthyle, éthyle, n-propyle, isopropyle, n-butyle, 2-butyle, isobutyle, tert-butyle, n-pentyle et n-hexyle ; un radical cycloalkyle choisi dans le groupe constitué par cyclopropyle, cyclobutyle, cyclopentyle et cyclohexyle ; ou un radical benzyle ou phénéthyle, qui est non substitué ou substitué par 1, 2, 3, 4 ou 5 substituants choisis indépendamment l'un de l'autre dans le groupe constitué par F, Cl, Br, méthyle, éthyle, n-propyle, isopropyle, n-butyle, 2-butyle, isobutyle, tert-butyle, -OH, $-O-CH_3$, $-O-C_2H_5$ et $-0-C_3H_7$ ;

$R^4$ , $R^5$ , $R^6$ , $R^7$ , $R^{12}$ , $R^{13}$ , $R^{14}$ , $R^{15}$ , $R^{18}$ , $R^{19}$ , $R^{20}$ , $R^{21}$ , $R^{22}$ , $R^{23}$ , $R^{24}$ , $R^{25}$ , $R^{26}$ , $R^{27}$ , $R^{28}$ , $R^{29}$ , $R^{32}$ , $R^{33}$ , $R^{38}$ , $R^{39}$ , $R^{40}$, $R^{41}$, $R^{42}$, $R^{43}$, $R^{44}$ et $R^{45}$, représentent, indépendamment l'un de l'autre, à chaque fois H ; F ; Cl ; Br ; I ; $-NO_2$ ; -CN ; $-NH_2$ ; -OH ; -SH ; $-NH-R^{55}$ ; $-NR^{56}R^{57}$ ; $-O-R^{67}$ ; $-S-R^{68}$ ; ou représentent un radical alkyle choisi dans le groupe constitué par méthyle, éthyle, n-propyle, isopropyle, n-butyle, 2-butyle, isobutyle, tert-butyle, n-pentyle et n-hexyle ;

ou $R^4$ et $R^5$ ou $R^6$ et $R^7$ ou $R^{12}$ et $R^{13}$ ou $R^{14}$ et $R^{15}$ ou $R^{18}$ et $R^{19}$ ou $R^{20}$ et $R^{21}$ ou $R^{22}$ et $R^{23}$ ou $R^{24}$ et $R^{25}$ ou $R^{26}$ et $R^{27}$ ou $R^{28}$ et $R^{29}$ ou $R^{32}$ et $R^{33}$ ou $R^{38}$ et $R^{39}$ ou $R^{40}$ et $R^{41}$ ou $R^{42}$ et $R^{43}$ ou $R^{44}$ et $R^{45}$, représentent, indépendamment l'un de l'autre, ensemble à chaque fois un radical choisi dans le groupe constitué par un groupe oxo (=O) et un groupe thioxo (=S) ;

ou $R^3$ et $R^9$ forment ensemble avec le groupe $-N-CR^5$ qui les relie un radical choisi dans le groupe constitué par

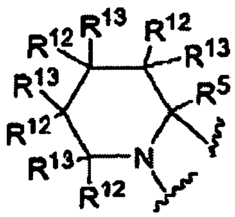

et

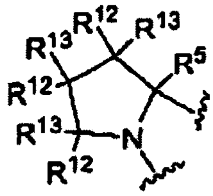

ou $R^6$ et $R^8$ forment ensemble avec le groupe $-N-CR^7$-qui les relie un radical choisi dans le groupe constitué par

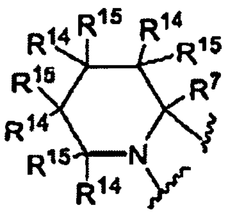

et

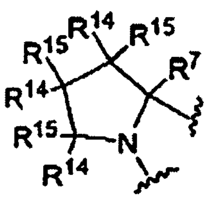

ou $R^3$ et $R^6$ forment ensemble avec le groupe $-N-CR^4R^5-CR^7-$ qui les relie un radical choisi dans le groupe constitué par

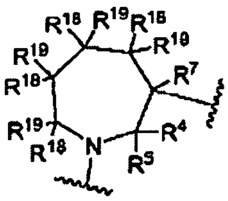, 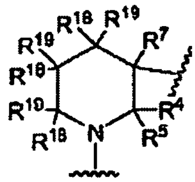, 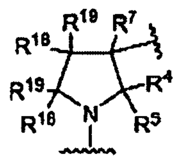,

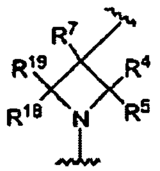, 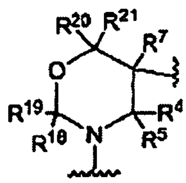, 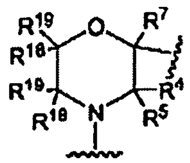 et 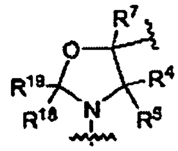

ou $R^4$ et $R^8$ forment ensemble avec le groupe $-CR^5-CR^6CR^7-N-$ qui les relie un radical choisi dans le groupe constitué par

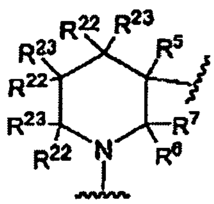, 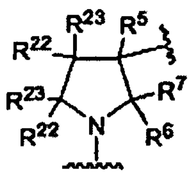, 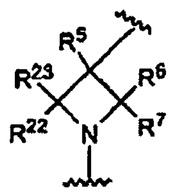,

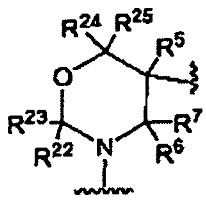, 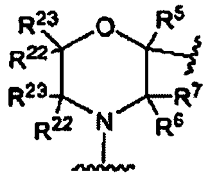 et 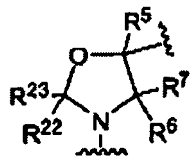

ou $R^3$ et $R^8$ forment ensemble avec le groupe $-N-CR^4R^5-CR^6CR^7-N-$ qui les relie le radical suivant

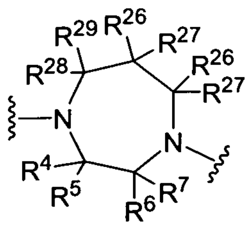

ou $R^4$ et $R^6$ forment ensemble avec le groupe $-CR^5-CR^7-$ qui les relie le radical suivant

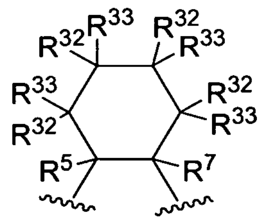

ou $R^3$ et $R^6$ ensemble avec le groupe $-N-CR^4CR^5-CR^7-$ qui les relie et $R^4$ et $R^8$ ensemble avec le groupe $-CR^5-CR^6R^7-N-$ qui les relie forment le radical suivant,

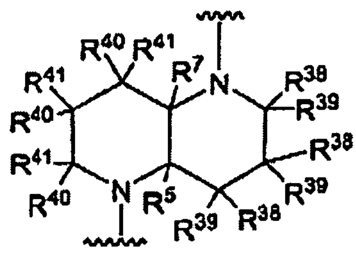

ou $R^3$ et $R^8$ ensemble avec le groupe $-N-CR^4R^5-CR^6R^7-N-$ qui les relie et $R^4$ et $R^6$ ensemble avec le groupe $-CR^5-CR^7-$ qui les relie forment le radical suivant,

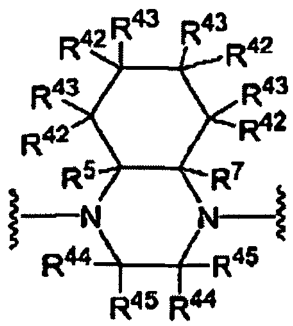

$R^9$ représente -C(=O) -NH-$R^{64}$ ; -C(=O) -N$R^{65}R^{66}$ ; ou un radical choisi dans le groupe constitué par phényle, naphtyle, anthracényle, furyle, thiényle, pyrazolyle, pyrazinyle, pyridazinyle, pyrimidinyle, pyridinyle, pyrrolyle, oxazolyle, isoxazolyle, thiazolyle, thiadiazolyle, oxadiazolyle, triazolyle, imidazolyle, indolyle, benzo[b]thiophényle, benzo[d]thiazolyle, benzo[b]furannyle, quinoléinyle, isoquinoléinyle et quinazolinyle, qui est à chaque fois non substitué ou substitué par le cas échéant 1, 2, 3, 4 ou 5 substituants choisis indépendamment l'un de l'autre dans le groupe constitué par F, Cl, Br, I, -CN, méthyle, éthyle, n-propyle, isopropyle, n- butyle, 2-butyle, isobutyle, tert-butyle, éthényle, allyle, éthynyle, propynyle, -C≡C- $Si(CH_3)_3$, -C≡C-$Si(C_2H_5)_3$, -$CH_2$-O-$CH_3$, -$CH_2$-O-$C_2H_5$, -OH, -O-$CH_3$, -O-$C_2H_5$, -O-$C_3H_7$, -S-$CH_3$, -S-$C_2H_5$, -S(=O)-$CH_3$, -S (=O)$_2$-$CH_3$, -S (=O) -$C_2H_5$, -S (=O)$_2$-$C_2H_5$, -$NH_2$, -N$(CH_3)_2$, -N$(C_2H_5)_2$, -NH-$CH_3$, -NH-$C_2H_5$, -$NO_2$, -$CF_3$, -$CH_2$F, -$CHF_2$, -O-$CF_3$, -S-$CF_3$, -SH, -NH-S(=O)$_2$-$CH_3$, -C(=O) -OH, -C(=O)-H ; -C(=O)-$CH_3$, -C(=O)-$C_2H_5$, -C(=O)-$NH_2$, -C(=O)-N$(CH_3)_2$, -C(=O)-NH-$CH_3$, -NH-C(=O)-$CH_3$, -NH-C(=O)-$C_2H_5$, -C(=O)-O-$CH_3$, -C(=O)-O- $C_2H_5$, -C(=O)-O-C$(CH_3)_3$ et phényle ;

et $R^{55}$, $R^{56}$, $R^{57}$ , $R^{58}$, $R^{59}$, $R^{64}$, $R^{65}$ , $R^{66}$, $R^{67}$, $R^{68}$, $R^{69}$ et $R^{70}$, représentent, indépendamment l'un de l'autre, à chaque fois un radical alkyle choisi dans le groupe constitué par méthyle, éthyle, n-propyle, isopropyle, n-butyle, 2-butyle, isobutyle, tert- butyle, n-pentyle et n-hexyle ; ou un radical phényle, benzyle ou phénéthyle, qui est non substitué ou substitué par 1, 2, 3, 4 ou 5 substituants choisis indépendamment l'un de l'autre dans le groupe constitué par F, Cl, Br, méthyle, éthyle, n-propyle, isopropyle, n-butyle, 2-butyle, isobutyle, tert-butyle, -OH, -O-$CH_3$, -O- $C_2H_5$ et -O-$C_3H_7$ ;

le cas échéant à chaque fois sous forme d'un de leurs stéréo-isomères purs, en particulier leurs énantiomères ou

diastéréo-isomères, leurs mélanges racémiques ou sous forme d'un mélange de stéréo-isomères, en particulier des énantiomères et/ou des diastéréo-isomères, dans un rapport de mélange quelconque ou à chaque fois sous forme de sels correspondants.

**5.** Composés selon l'une ou plusieurs des revendications 1 à 4, **caractérisés en ce que**

$R^1$ représente H ; F ; Cl ; Br ; I ; $-CF_3$ ; $-NO_2$ ; -CN ; -C(=O)-OH ; -C(=O)-O-$R^{59}$ ; -C(=O)-$NH_2$ ; -C(=O)-NH-$R^{64}$; -C(=O)-$NR^{65}R^{66}$ ; -O-$R^{67}$ ; -S-$R^{68}$ ; -S(=O)-$R^{69}$ ; -S $(=O)_2$-$R^{70}$ ; un radical alkyle choisi dans le groupe constitué par méthyle, éthyle, n- propyle, isopropyle, n-butyle, 2-butyle, isobutyle et tert-butyle ; ou représente un radical choisi dans le groupe constitué par (1,3)-dioxolan-2-yle, phényle, benzyle, phénéthyle, oxadiazolyle, 2- pyridyle, 3-pyridyle, 4-pyridyle, 2-thiényle, 3- thiényle, 2-furyle et 3-furyle, qui est à chaque fois non substitué ou substitué par le cas échéant 1, 2, 3, 4 ou 5 substituants choisis indépendamment l'un de l'autre dans le groupe constitué par F, Cl, Br, méthyle, éthyle, n- propyle, isopropyle, n-butyle, 2-butyle, isobutyle, tert-butyle, -OH, -O-$CH_3$, -O-$C_2H_5$ et -O- $C_3H_7$ ;
$R^2$ représente H ; F ; Cl ; Br ; I ; $-CF_3$ ; $-NO_2$ ; -CN ; -C(=O)-OH ; -C(=O)-O-$R^{59}$ ; -C(=O)-$NH_2$ ; -C(=O)-NH-$R^{64}$ ; -C(=O)-$NR^{65}R^{66}$ ; -O-$R^{67}$ ; -S-$R^{68}$ ; -S(=O)-$R^{69}$ ; -S $(=O)_2$-$R^{70}$ ; un radical alkyle choisi dans le groupe constitué par méthyle, éthyle, n- propyle, isopropyle, n-butyle, 2-butyle, isobutyle et tert-butyle ; ou représente un radical choisi dans le groupe constitué par (1,3)-dioxolan-2-yle, phényle, benzyle, phénéthyle, oxadiazolyle, 2- pyridyle, 3-pyridyle, 4-pyridyle, 2-thiényle, 3- thiényle, 2-furyle et 3-furyle, qui est à chaque fois non substitué ou substitué par le cas échéant 1, 2, 3, 4 ou 5 substituants choisis indépendamment l'un de l'autre dans le groupe constitué par F, Cl, Br, méthyle, éthyle, n- propyle, isopropyle, n-butyle, 2-butyle, isobutyle, tert-butyle, -OH, -O-$CH_3$, -O-$C_2H_5$ et -O- $C_3H_7$ ; ou $R^1$ et $R^2$ ensemble avec les atomes de carbone qui les relient forment un radical phénylène, qui est non substitué ou substitué par 1, 2, 3 ou 4 substituants choisis indépendamment l'un de l'autre dans le groupe constitué par F, Cl, Br, I, -CN, méthyle, éthyle, n-propyle, isopropyle, n- butyle, isobutyle, 2-butyle, tert-butyle, -O-$CH_3$, -O-$C_2H_5$, -O-$C_3H_7$, $-CF_3$, $-CHF_2$, $-CH_2F$ et -O-$CH_3$ ;
$R^3$ et $R^8$, représentent, indépendamment l'un de l'autre, à chaque fois H ; -C(=O)-$R^{58}$ ; -C(=O)-O-$R^{59}$ ; -S (=O)-$R^{69}$ ; $-S(=O)_2$-$R^{70}$ ; un radical alkyle choisi dans le groupe constitué par méthyle, éthyle, n- propyle, isopropyle, n-butyle, 2-butyle, isobutyle, tert-butyle, n-pentyle et n-hexyle ; un radical cycloalkyle choisi dans le groupe constitué par cyclopropyle, cyclobutyle, cyclopentyle et cyclohexyle ; ou un radical benzyle ou phénéthyle, qui est non substitué ou substitué par 1, 2, 3, 4 ou 5 substituants choisis indépendamment l'un de l'autre dans le groupe constitué par F, Cl, Br, méthyle, éthyle, n- propyle, isopropyle, n-butyle, 2-butyle, isobutyle, tert-butyle, -OH, -O-$CH_3$, -O-$C_2H_5$ et -O- $C_3H_7$ ;
$R^4$, $R^5$, $R^6$ et $R^7$, représentent, indépendamment l'un de l'autre, à chaque fois H ; F ; Cl ; Br ; I ; $-NO_2$ ; -CN ; $-NH_2$ ; -OH ; -SH ; -NH-$R^{55}$ ; -$NR^{56}R^{57}$ ; -O-$R^{67}$ ; -S-$R^{68}$ ; ou représentent un radical alkyle choisi dans le groupe constitué par méthyle, éthyle, n-propyle, isopropyle, n-butyle, 2-butyle, isobutyle, tert-butyle, n-pentyle et n-hexyle ;

ou $R^4$ et $R^5$ ou $R^6$ et $R^7$, représentent, indépendamment l'un de l'autre, ensemble à chaque fois un radical choisi dans le groupe constitué par un groupe oxo (=O) et un groupe thioxo (=S) ;
ou $R^3$ et $R^4$ forment ensemble avec le groupe -N-$CR^5$- qui les relie un radical choisi dans le groupe constitué par

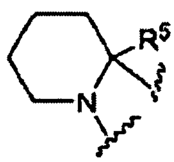

et

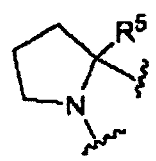

ou $R^6$ et $R^8$ forment ensemble avec le groupe -N-$CR^7$- qui les relie un radical choisi dans le groupe constitué par

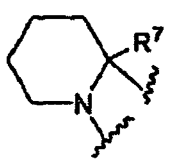

et

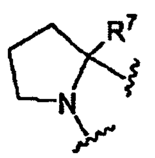

ou $R^3$ et $R^6$ forment ensemble avec le groupe -N-$CR^4R^5$-$CR^7$- qui les relie un radical choisi dans le groupe constitué par

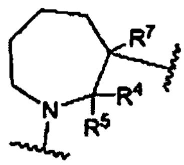 , 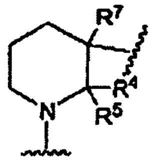 , 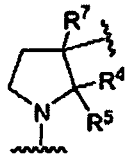 ,

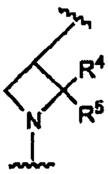 , 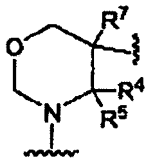 , 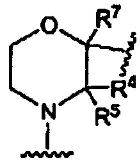 et 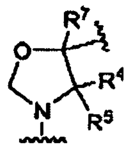

ou $R^4$ et $R^8$ forment ensemble avec le groupe -$CR^5$-$CR^6CR^7$-N- qui les relie un radical choisi dans le groupe constitué par

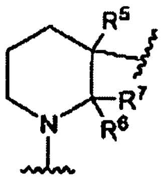 , 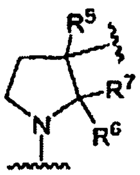 , 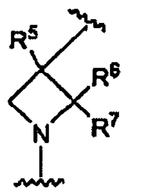 ,

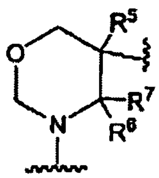 , 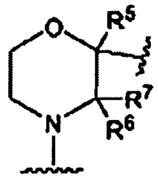 et 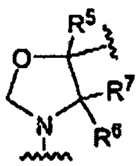

ou $R^3$ et $R^8$ forment ensemble avec le groupe -N-$CR^4R^5$-$CR^6CR^7$-N- qui les relie le radical suivant

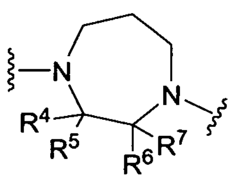

ou $R^4$ et $R^6$ forment ensemble avec le groupe -$CR^5$-$CR^7$- qui les relie le radical suivant

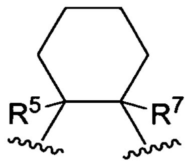

ou $R^3$ et $R^6$ ensemble avec le groupe -N-$CR^4CR^5$-$CR^7$- qui les relie et $R^4$ et $R^8$ ensemble avec le groupe -$CR^5$-$CR^6R^7$-N- qui les relie forment le radical suivant,

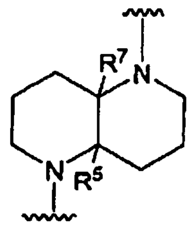

ou $R^3$ et $R^8$ ensemble avec le groupe -N-$CR^4R^5$-$CR^6R^7$-N- qui les relie et $R^4$ et $R^6$ ensemble avec le groupe -$CR^5$-$CR^7$- qui les relie forment le radical suivant,

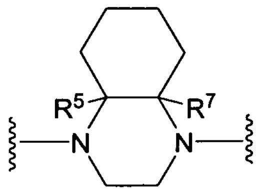

$R^9$ représente -C(=O)-NH-$R^{64}$ ; -C(=O)-$NR^{65}R^{66}$ ; ou un radical choisi dans le groupe constitué par phényle, naphtyle, furyle, thiényle, pyrazolyle, pyrazinyle, pyridazinyle, pyrimidinyle, pyridinyle, pyrrolyle, oxazolyle, isoxazolyle, thiazolyle, thiadiazolyle, oxadiazolyle, triazolyle, imidazolyle, indolyle, benzo[b]thiophényle, benzo[d]thiazolyle, benzo[b]furannyle, quinoléinyle, isoquinoléinyle et quinazolinyle, qui est à chaque fois non substitué ou substitué par le cas échéant 1, 2, 3, 4 ou 5 substituants choisis indépendamment l'un de l'autre dans le groupe constitué par F, Cl, Br, I, -CN, méthyle, éthyle, n-propyle, isopropyle, n- butyle, 2-butyle, isobutyle, tert-butyle, éthényle, allyle, éthynyle, propynyle, -O-$CH_3$, -O- $C_2H_5$, -$NO_2$, -$CF_3$, -$CH_2F$, -$CHF_2$, -O-$CF_3$ et -S-$CF_3$ ; et $R^{55}$, $R^{56}$, $R^{57}$, $R^{58}$, $R^{59}$, $R^{64}$, $R^{65}$, $R^{66}$, $R^{67}$, $R^{68}$, $R^{69}$ et $R^{70}$, représentent, indépendamment l'un de l'autre, à chaque fois un radical alkyle choisi dans le groupe constitué par méthyle, éthyle, n-propyle, isopropyle, n-butyle, 2-butyle, isobutyle, tert- butyle, n-pentyle et n-hexyle ; ou un radical phényle, benzyle ou phénéthyle, qui est non substitué ou substitué par 1, 2, 3, 4 ou 5 substituants choisis indépendamment l'un de l'autre dans le groupe constitué par F, Cl, Br, méthyle, éthyle, n-propyle, isopropyle, n-butyle, 2-butyle, isobutyle, tert-butyle, -OH, -O-$CH_3$, -O- $C_2H_5$ et -O-$C_3H_7$ ;

le cas échéant à chaque fois sous forme d'un de leurs stéréo-isomères purs, en particulier leurs énantiomères ou diastéréo-isomères, leurs mélanges racémiques ou sous forme d'un mélange de stéréo-isomères, en particulier des énantiomères et/ou des diastéréo-isomères, dans un rapport de mélange quelconque ou à chaque fois sous forme de sels correspondants.

**6.** Composés selon l'une ou plusieurs des revendications 1 à 5, **caractérisés en ce que**

$R^1$ représente H ; F ; Cl ; Br ; I ; -$CF_3$ ; -$NO_2$ ; -CN ; -C(=O)-O-$R^{59}$ ; un radical alkyle choisi dans le groupe constitué par méthyle, éthyle, n- propyle, isopropyle, n-butyle, 2-butyle, isobutyle et tert-butyle ; ou représente un radical choisi dans le groupe constitué par (1,3)-dioxolan-2-yle, phényle, benzyle, phénéthyle, oxadiazolyle,

2- pyridyle, 3-pyridyle, 4-pyridyle, 2-thiényle, 3- thiényle, 2-furyle et 3-furyle ; $R^2$ représente H ; F ; Cl ; Br ; I ; $-CF_3$ ; $-NO_2$ ; -CN ; -C (=O)-O-$R^{59}$ ; un radical alkyle choisi dans le groupe constitué par méthyle, éthyle, n- propyle, isopropyle, n-butyle, 2-butyle, isobutyle et tert-butyle ; ou représente un radical choisi dans le groupe constitué par (1,3)-dioxolan-2-yle, phényle, benzyle, phénéthyle, oxadiazolyle, 2- pyridyle, 3-pyridyle, 4-pyridyle, 2-thiényle, 3- thiényle, 2-furyle et 3-furyle ; ou $R^1$ et $R^2$ forment, ensemble avec les atomes de carbone qui les relient un radical phénylène ; $R^3$ et $R^8$, représentent, indépendamment l'un de l'autre, à chaque fois H ; -C(=O)-$R^{58}$ ; -C(=O)-O- $R^{59}$ ; un radical alkyle choisi dans le groupe constitué par méthyle, éthyle, n-propyle, isopropyle, n-butyle, 2-butyle, isobutyle, tert- butyle, n-pentyle et n-hexyle ; un radical cycloalkyle choisi dans le groupe constitué par cyclopropyle, cyclobutyle, cyclopentyle et cyclohexyle ; ou un radical benzyle ou phénéthyle, qui est non substitué ou substitué par 1, 2, 3, 4 ou 5 substituants choisis indépendamment l'un de l'autre dans le groupe constitué par F, Cl, Br, méthyle, éthyle, n-propyle, isopropyle, n-butyle, 2-butyle, isobutyle, tert-butyle, -OH, $-O-CH_3$, -O- $C_2H_5$ et $-O-C_3H_7$ ;

$R^4$, $R^5$, $R^6$ et $R^7$, représentent, indépendamment l'un de l'autre, à chaque fois H ; F ; Cl ; Br ; I ; $-NO_2$; -CN ; $-NH_2$ ; -OH ; -SH ; -NH-$R^{55}$ ; -$NR^{56}R^{57}$; -O-$R^{67}$ ; -S-$R^{68}$ ; ou représentent un radical alkyle choisi dans le groupe constitué par méthyle, éthyle, n-propyle, isopropyle, n-butyle, 2-butyle, isobutyle, tert-butyle, n-pentyle et n-hexyle ;

ou $R^4$ et $R^5$ ou $R^6$ et $R^7$, représentent, indépendamment l'un de l'autre, ensemble à chaque fois un radical choisi dans le groupe constitué par un groupe oxo (=O) et un groupe thioxo (=S) ;

ou $R^3$ et $R^6$ forment ensemble avec le groupe -N-$CR^4R^5$-$CR^7$ qui les relie un radical choisi dans le groupe constitué par

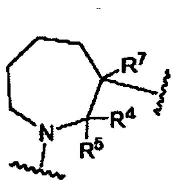 , 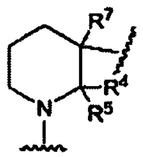 , 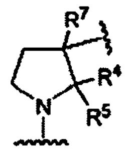 ,

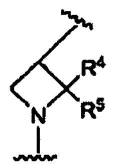 , 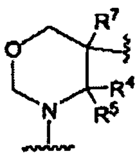 , 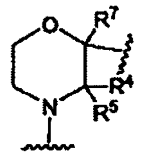 et 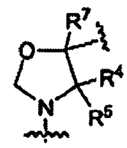

ou $R^4$ et $R^8$ forment ensemble avec le groupe -$CR^5$-$CR^6CR^7$-N- qui les relie un radical choisi dans le groupe constitué par

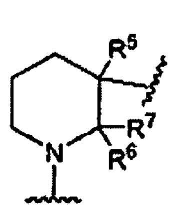 , 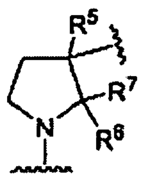 , 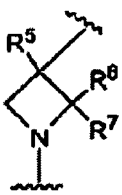 ,

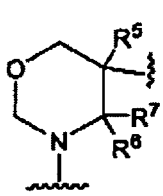 , 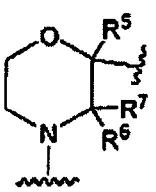 et 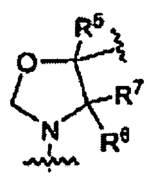

ou $R^3$ et $R^8$ forment ensemble avec le groupe $-N-CR^4R^5-CR^6CR^7-N-$ qui les relie le radical suivant

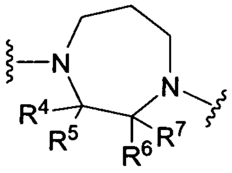

ou $R^4$ et $R^6$ forment ensemble avec le groupe $-CR^5-CR^7-$ qui les relie le radical suivant

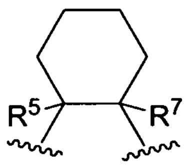

$R^9$ représente un radical choisi dans le groupe constitué par phényle, furyle, thiényle, pyrazolyle, pyrazinyle, pyridazinyle, pyrimidinyle, pyridinyle, pyrrolyle, oxazolyle, isoxazolyle, thiazolyle, thiadiazolyle, oxadiazolyle, triazolyle et imidazolyle, qui est à chaque fois non substitué ou substitué par le cas échéant 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert-butyle, éthényle, allyle, éthynyle, propynyle, $-O-CH_3$, $-O-C_2H_5$, $-NO_2$, $-CF_3$, $-CH_2F$, $-CHF_2$, $-O-CF_3$ et $-S-CF_3$ ; et $R^{55}$, $R^{56}$, $R^{57}$, $R^{58}$, $R^{59}$, $R^{67}$ et $R^{68}$, représentent, indépendamment l'un de l'autre, à chaque fois un radical alkyle choisi dans le groupe constitué par méthyle, éthyle, n-propyle, isopropyle, n-butyle, 2-butyle, isobutyle, tert-butyle, n-pentyle et n- hexyle ;

le cas échéant à chaque fois sous forme d'un de leurs stéréo-isomères purs, en particulier leurs énantiomères ou diastéréo-isomères, leurs mélanges racémiques ou sous forme d'un mélange de stéréo-isomères, en particulier des énantiomères et/ou des diastéréo-isomères, dans un rapport de mélange quelconque ou à chaque fois sous forme de sels correspondants.

**7.** Composés selon l'une ou plusieurs des revendications 1 à 6, **caractérisés en ce que**

$R^1$ représente H, CN, Cl, Br, F, méthyle, éthyle, n- propyle, isobutyle, n-butyle, tert-butyle, $-C(=O)-O-CH_3$, $-C(=O)-O-C_2H_5$ ou $-C(=O)-O-C(CH_3)_3$ ;
$R^2$ représente H, CN, Cl, Br, F, méthyle, éthyle, n- propyle, isobutyle, n-butyle, tert-butyle, $-C(=O)-O-CH_3$, $-C(=O)-O-C_2H_5$ ou $-C(=O)-O-C(CH_3)_3$ ; ou $R^1$ et $R^2$ forment, ensemble avec les atomes de carbone qui les relient un radical phénylène ;
$R^3$ et $R^8$, représentent, indépendamment l'un de l'autre, à chaque fois H ; un radical alkyle choisi dans le groupe constitué par méthyle, éthyle, n-propyle, isopropyle, n-butyle, 2-butyle, isobutyle, tert- butyle ; ou cyclopropyle ;
$R^4$, $R^5$ et $R^7$, représentent à chaque fois H ;
$R^6$ représente H ou représente un radical alkyle choisi dans le groupe constitué par méthyle, éthyle, n-propyle, isopropyle, n-butyle, 2-butyle, isobutyle, tert-butyle, n-pentyle et n-hexyle ; ou $R^4$ et $R^5$ ou $R^6$ et $R^7$, représentent, indépendamment l'un de l'autre, ensemble à chaque fois un groupe oxo (=O) ;

ou $R^3$ et $R^6$ forment ensemble avec le groupe $-N-CH_2-CH-$ qui les relie un radical choisi dans le groupe constitué par

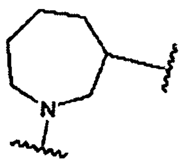 , 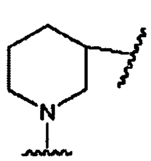 , 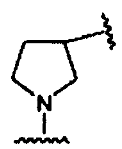 ,

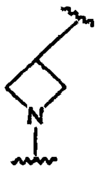 , 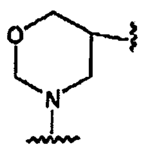 , 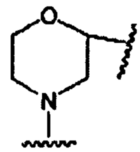 et 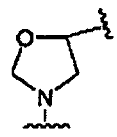

ou $R^4$ et $R^8$ forment ensemble avec le groupe $-CH-CH_2-N-$qui les relie un radical choisi dans le groupe constitué par

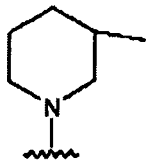 , 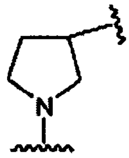 , 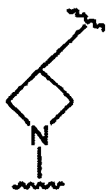 ,

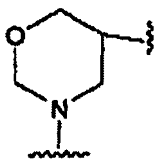 , 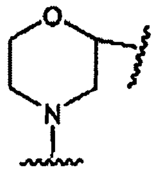 et 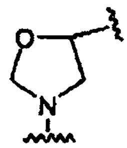

ou $R^3$ et $R^8$ forment ensemble avec le groupe $-N-CH_2-CH_2-N-$qui les relie le radical suivant

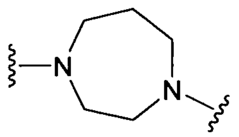

ou $R^4$ et $R^6$ forment ensemble avec le groupe -CH-CH- qui les relie le radical suivant

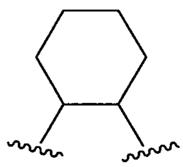

et $R^9$ représente un radical choisi dans le groupe constitué par pyrid-2-yle, pyrid-3-yle, pyrid-4-yle, phényle, 2-méthylphényle, 3-méthylphényle, 4-méthylphényle, 2-fluorophényle, 3-fluorophényle, 4-fluorophényle, 2-cyanophényle, 3-cyanophényle, 4-cyanophényle, 2-chlorophényle, 3-chlorophényle, 3-chlorophényle, 2-trifluorométhylphényle, 3-trifluorométhylphényle, 4-trifluorométhylphényle, 2-méthoxyphényle, 3-méthoxyphényle, 4-méthoxyphényle, (2,4)-difluorophényle, (2,4)-dichlorophényle, (3,5)-dichlorophényle, (3,5)-difluorophényle, 2-thiophényle, 2-chloro-5-trifluorométhylphényle, 3-fluoro-4-méthylphényle, 2-fluoro-3-méthylphényle, 2-difluorométhylphényle, 3-difluorométhylphényle, 4-difluorométhylphényle, 2-fluorométhylphényle, 3-fluorométhylphényle, 4-fluorométhylphényle, 3-nitrophényle, 3-éthénylphényle, 3-éthynylphényle, 3-allylphényle, 3-bromophényle, 2-trifluorométhoxyphényle, 3-trifluorométhoxyphényle et 4-trifluorométhoxyphényle ;
le cas échéant à chaque fois sous forme d'un de leurs stéréo-isomères purs, en particulier leurs énantiomères ou diastéréo-isomères, leurs mélanges racémiques ou sous forme d'un mélange de stéréo-isomères, en particulier des énantiomères et/ou des diastéréo-isomères, dans un rapport de mélange quelconque ou à chaque fois sous

forme de sels correspondants.

**8.** Composés selon l'une ou plusieurs des revendications 1 à 7 choisis dans le groupe constitué par

1. 3-phényl-N-(1-(thiazol-2-yl)pyrrolidin-3-yl)propiolamide,
2. chlorhydrate de N-méthyl-3-phényl-N-(1-(thiazol-2-yl)pyrrolidin-3-yl)propiolamide,
3. 1-thiazol-2-yl-4-(3-phénylpropiolyl)-1,4-diazépane,
4. N-méthyl-N-(2-(méthyl(thiazol-2-yl)amino)éthyl)-3-phénylpropiolamide,
5. N-(2-((thiazol-2-yl)amino)éthyl)-3-phénylpropiolamide,
6. 3-phényl-N-(2-(thiazol-2-ylamino)cyclohexyl)propiolamide,
7. N-méthyl-N-(2-(méthyl(thiazol-2-yl)amino)éthyl)-3-(3-méthylphényl)-propiolamide,
8. 3-phényl-N-(1-(thiazol-2-yl)azétidin-3-yl)propiolamide,
9. N-(2-(méthyl(thiazol-2-yl)amino)éthyl)-3-phénylpropiolamide,
10. N-méthyl-N-(2-(thiazol-2-yl)amino)éthyl-3-phénylpropiolamide,
11. 3-(thiazol-2-ylamino)-1-(3-phénylpropiolyl)pyrrolidine,
12. N-méthyl-N-(2-(méthyl(thiazol-2-yl)amino)cyclohexyl)-3-phénylpropiolamide,
13. N-méthyl-N-(2-(méthyl(thiazol-2-yl)amino)éthyl)-3-(3-cyanophényl)-propiolamide,
14. N-méthyl-N-(2-(méthyl(thiazol-2-yl)amino)-2-oxoéthyl)-3-phénylpropiolamide,
15. 3-phényl-N-(1-(thiazol-2-yl)pipéridin-3-yl)propiolamide,
16. N-méthyl-N-(1-(thiazol-2-yl)pipéridin-3-yl)-3-phénylpropiolamide,
17. N-méthyl-N-(2-(méthyl(thiazol-2-yl)amino)éthyl)-3-(pyrid-2-yl)-propiolamide,
18. N-méthyl-N-(2-(méthyl(thiazol-2-yl)amino)éthyl)-3-(3-chlorophényl)-propiolamide,
19. N-(2-(méthyl(thiazol-2-yl)amino)éthyl)-3-(3-chlorophényl)-propiolamide,
20. N-méthyl-N-(1-(thiazol-2-yl)azétidin-3-yl)-3-phénylpropiolamide,
21. N-(2-(méthyl(thiazol-2-yl)amino)éthyl)-3-(tolu-3-yl)-propiolamide,
22. (3-méthyl(thiazol-2-yl)amino)-1-(3-phénylpropiolyl)pyrrolidine,
23. chlorhydrate de N-(2-(méthyl(thiazol-2-yl)-amino)éthyl)-3-(3-chlorophényl)-propiolamide,
24. N-(2-(benzo[d]thiazol-2-yl(méthyl)amino)éthyl)-3-(3-chlorophényl)propiolamide,
25. N-(2-(méthyl(5-éthoxycarbonylthiazol-2-yl)amino)éthyl)-3-(3-chlorophényl)-propiolamide,
26. N-(2-(méthyl(4-éthoxycarbonylthiazol-2-yl)amino)éthyl)-3-(3-chlorophényl)-propiolamide,
27. 3-(thiazol-2-yl-amino)-1-(3-phénylpropiolyl)pipéridine,
28. 3-(méthyl-(thiazol-2-yl)-amino)-1-(3-phénylpropiolyl)pipéridine,
29. N-(2-(méthyl-(4-méthylthiazol-2-yl)amino)éthyl)-3-(3-chlorophényl)-propiolamide,
30. 3-(3-chlorophényl)-N-(2-(méthyl(5-méthylthiazol-2-yl)amino)éthyl)propiolamide
31. N-(2-((5-bromothiazol-2-yl)(méthyl)amino)éthyl)-3-(3-chlorophényl)propiolamide,
32. 3-(3-chlorophényl)-N-(1-(thiazol-2-yl)pipéridin-3-yl)propiolamide,
33. N-(2-((4-bromothiazol-2-yl)(méthyl)amino)éthyl)-3-(3-chlorophényl)propiolamide,
34. 2-((2-(3-(3-chlorophényl)propiolamido)éthyl)(méthyl)amino)-thiazol-5-carboxylate de méthyle,
35. 1-(3-(3-chlorophényl)propiolyl)-3-(méthyl(thiazol-2-yl)amino)azétidine,
36. 3-(3-chlorophényl)-N-méthyl-N-(1-(thiazol-2-yl)pipéridin-3-yl)propiolamide,
37. 3-(3-chlorophényl)-N-(2-((4-chlorothiazol-2-yl)(méthyl)amino)éthyl)propiolamide,
38. 3-(3-chlorophényl)-N-(2-((5-chlorothiazol-2-yl) (méthyl)amino)éthyl)propiolamide,
39. 3-(3-chlorophényl)-N-(1-(thiazol-2-yl)azétidin-3-yl)propiolamide,
40. 3-(3-chlorophényl)-N-méthyl-N-(1-(thiazol-2-yl)azétidin-3-yl)propiolamide,
41. 3-(3-chlorophényl)-N-(2-(éthyl(thiazol-2-yl)amino)éthyl)propiolamide,
42. 3-(3-chlorophényl)-N-(2-((5-cyanothiazol-2-yl) (méthyl)amino)éthyl)propiolamide,
43. 1-(3-(3-chlorophényl)propiolyl)-3-(méthyl(5-fluorothiazol-2-yl)amino)azétidine,
44. 1-(3-(3-chlorophényl)propiolyl)-3-(méthyl(5-fluorothiazol-2-yl)amino)pyrrolidine,
45. N-(1-(4-méthylthiazol-2-yl)pyrrolidin-3-yl)-3-phénylpropiolamide,
46. 3-(3-méthoxyphényl)-N-(1-(4-méthylthiazol-2-yl)pyrrolidin-3-yl)propiolamide,
47. 3-(2-méthoxyphényl)-N-(1-(4-méthylthiazol-2-yl)pyrrolidin-3-yl)propiolamide,
48. 3-(4-méthoxyphényl)-N-(1-(4-méthylthiazol-2-yl)pyrrolidin-3-yl)propiolamide,
49. 3-(4-fluorophényl)-N-(1-(4-méthylthiazol-2-yl)pyrrolidin-3-yl)propiolamide,
50. N-(1-(4-méthylthiazol-2-yl)pyrrolidin-3-yl)-3-p-toluylpropiolamide,
51. N-(1-(4,5-diméthylthiazol-2-yl)pyrrolidin-3-yl)-3-phénylpropiolamide,
52. N-(1-(4,5-diméthylthiazol-2-yl)pyrrolidin-3-yl)-3-(4-méthoxyphényl)propiolamide,
53. N-(1-(4,5-diméthylthiazol-2-yl)pyrrolidin-3-yl)-3-(4-fluorophényl)propiolamide,
54. N-(1-(4,5-diméthylthiazol-2-yl)pyrrolidin-3-yl)-3-(2-méthoxyphényl)propiolamide,

55. N-(1-(4-tert-butylthiazol-2-yl)pyrrolidin-3-yl)-3-(4-méthoxyphényl)propiolamide,
56. N-(1-(4-tert-butylthiazol-2-yl)pyrrolidin-3-yl)-3-p-toluylpropiolamide,
57. N-(1-(4-tert-butylthiazol-2-yl)pyrrolidin-3-yl)-3-(4-fluorophényl)propiolamide,
58. N-(1-(4-tert-butylthiazol-2-yl)pyrrolidin-3-yl)-3-o-toluylpropiolamide,
59. N-(1-(4-tert-butylthiazol-2-yl)pyrrolidin-3-yl)-3-(3-fluorophényl)propiolamide,
60. N-(1-(4-tert-butylthiazol-2-yl)pyrrolidin-3-yl)-3-phénylpropiolamide,
61. N-(1-(4-tert-butylthiazol-2-yl)pyrrolidin-3-yl)-3-(2-fluorophényl)propiolamide,
62. N-(1-(4-tert-butylthiazol-2-yl)pyrrolidin-3-yl)-3-(3-méthoxyphényl)propiolamide,
63. N-(1-(4-tert-butylthiazol-2-yl)pyrrolidin-3-yl)-3-(3-fluoro-4-méthylphényl)propiolamide,
64. N-(1-(4-tert-butylthiazol-2-yl)pyrrolidin-3-yl)-3-(2-méthoxyphényl)propiolamide,
65. N-(1-(4-méthylthiazol-2-yl)pyrrolidin-3-yl)-3-(4-(trifluorométhyl)phényl)propiolamide,
66. N-(1-(4-méthylthiazol-2-yl)pyrrolidin-3-yl)-3-o-toluylpropiolamide,
67. 3-(3-fluorophényl)-N-(1-(4-méthylthiazol-2-yl)pyrrolidin-3-yl)propiolamide,
68. 3-(3-fluoro-4-méthylphényl)-N-(1-(4-méthylthiazol-2-yl)pyrrolidin-3-yl)propiolamide,
69. 3-(2,4-difluorophényl)-N-(1-(4-méthylthiazol-2-yl)pyrrolidin-3-yl)propiolamide,
70. 3-(2-fluorophényl)-N-(1-(4-méthylthiazol-2-yl)pyrrolidin-3-yl)propiolamide,
71. N-(1-(4-méthylthiazol-2-yl)pyrrolidin-3-yl)-3-(3-(trifluorométhyl)phényl)propiolamide,
72. N-(1-(4-méthylthiazol-2-yl)pyrrolidin-3-yl)-3-m-toluylpropiolamide,
73. N-(1-(5-méthylthiazol-2-yl)pyrrolidin-3-yl)-3-p-toluylpropiolamide,
74. N-(1-(5-méthylthiazol-2-yl)pyrrolidin-3-yl)-3-m-toluylpropiolamide,
75. 3-(2-méthoxyphényl)-N-(1-(5-méthylthiazol-2-yl)pyrrolidin-3-yl)propiolamide,
76. N-(1-(5-méthylthiazol-2-yl)pyrrolidin-3-yl)-3-phénylpropiolamide,
77. 3-(2-fluorophényl)-N-(1-(5-méthylthiazol-2-yl)pyrrolidin-3-yl)propiolamide,
78. 3-(3-méthoxyphényl)-N-(1-(5-méthylthiazol-2-yl)pyrrolidin-3-yl)propiolamide,
79. N-(1-(4,5-diméthylthiazol-2-yl)pyrrolidin-3-yl)-3-(3-fluoro-4-méthylphényl)propiolamide,
80. N-(1-(4,5-diméthylthiazol-2-yl)pyrrolidin-3-yl)-3-o-toluylpropiolamide,
81. 3-(3-fluorophényl)-N-(1-(5-méthylthiazol-2-yl)pyrrolidin-3-yl)propiolamide,
82. N-(1-(4,5-diméthylthiazol-2-yl)pyrrolidin-3-yl)-3-m-toluylpropiolamide,
83. N-(1-(5-méthylthiazol-2-yl)pyrrolidin-3-yl)-3-o-toluylpropiolamide,
84. N-(1-(4,5-diméthylthiazol-2-yl)pyrrolidin-3-yl)-3-p-toluylpropiolamide,
85. N-(1-(4,5-diméthylthiazol-2-yl)pyrrolidin-3-yl)-3-(3-(trifluorométhyl)phényl)propiolamide,
86. 3-(4-méthoxyphényl)-N-(1-(5-méthylthiazol-2-yl)pyrrolidin-3-yl)propiolamide,
87. N-(1-(5-méthylthiazol-2-yl)pyrrolidin-3-yl)-3-(3-(trifluorométhyl)phényl)propiolamide,
88. N-(1-(4,5-diméthylthiazol-2-yl)pyrrolidin-3-yl)-3-(4-(trifluorométhyl)phényl)propiolamide,
89. 3-(4-fluorophényl)-N-(1-(5-méthylthiazol-2-yl)pyrrolidin-3-yl)propiolamide,
90. N-(1-(4,5-diméthylthiazol-2-yl)pyrrolidin-3-yl)-3-(3-méthoxyphényl)propiolamide,
91. 3-(2,4-difluorophényl)-N-(1-(4,5-diméthylthiazol-2-yl)pyrrolidin-3-yl)propiolamide,
92. N-(1-(4,5-diméthylthiazol-2-yl)pyrrolidin-3-yl)-3-(2-fluorophényl)propiolamide,
93. N-(1-(5-méthylthiazol-2-yl)pyrrolidin-3-yl)-3-(4-(trifluorométhyl)phényl)propiolamide,
94. 3-(3-fluoro-4-méthylphényl)-N-(1-(5-méthylthiazol-2-yl)pyrrolidin-3-yl)propiolamide,
95. 3-(2,4-difluorophényl)-N-(1-(5-méthylthiazol-2-yl)pyrrolidin-3-yl)propiolamide,
96. N-(1-(4-tert-butylthiazol-2-yl)pyrrolidin-3-yl)-3-(2,4-difluorophényl)propiolamide,
97. N-(1-(4-tert-butylthiazol-2-yl)pyrrolidin-3-yl)-3-(4-(trifluorométhyl)phényl)propiolamide,
98. N-(1-(4-tert-butylthiazol-2-yl)pyrrolidin-3-yl)-3-(3-(trifluorométhyl)phényl)propiolamide,
99. N-(1-(4-tert-butylthiazol-2-yl)pyrrolidin-3-yl)-3-m-toluylpropiolamide,
100. N-(1-(4-tert-butylthiazol-2-yl)pyrrolidin-3-yl)-3-(3-chlorophényl)propiolamide,
101. N-(1-(4-tert-butylthiazol-2-yl)pyrrolidin-3-yl)-3-(thiophén-2-yl)propiolamide,
102. N-(1-(4-tert-butylthiazol-2-yl)pyrrolidin-3-yl)-3-(2,4-dichlorophényl)propiolamide,
103. N-(1-(4-tert-butylthiazol-2-yl)pyrrolidin-3-yl)-3-(2-chloro-5-(trifluorométhyl)phényl)propiolamide,
104. N-(1-(4-tert-butylthiazol-2-yl)pyrrolidin-3-yl)-3-(3,5-dichlorophényl)propiolamide,
105. 3-(3-chlorophényl)-N-(1-(4-méthylthiazol-2-yl)pyrrolidin-3-yl)propiolamide,
106. N-(1-(4-méthylthiazol-2-yl)pyrrolidin-3-yl)-3-(thiophén-2-yl)propiolamide,
107. 3-(2,4-dichlorophényl)-N-(1-(4-méthylthiazol-2-yl)pyrrolidin-3-yl)propiolamide,
108. 3-(2-chloro-5-(trifluorométhyl)phényl)-N-(1-(4-méthylthiazol-2-yl)pyrrolidin-3-yl)propiolamide,
109. 3-(3,5-dichlorophényl)-N-(1-(4-méthylthiazol-2-yl)pyrrolidin-3-yl)propiolamide,
110. 3-(3-chlorophényl)-N-(1-(4,5-diméthylthiazol-2-yl)pyrrolidin-3-yl)propiolamide,
111. 3-(2,4-dichlorophényl)-N-(1-(4,5-diméthylthiazol-2-yl)pyrrolidin-3-yl)propiolamide,
112. 3-(3-chlorophényl)-N-(1-(5-méthylthiazol-2-yl)pyrrolidin-3-yl)propiolamide,

113. N-(1-(5-méthylthiazol-2-yl)pyrrolidin-3-yl)-3-(thiophén-2-yl)propiolamide,
114. 3-(2,4-dichlorophényl)-N-(1-(5-méthylthiazol-2-yl)pyrrolidin-3-yl)propiolamide,
115. 3-(2-chloro-5-(trifluorométhyl)phényl)-N-(1-(5-méthylthiazol-2-yl)pyrrolidin-3-yl)propiolamide,
116. 3-(3,5-dichlorophényl)-N-(1-(5-méthylthiazol-2-yl)pyrrolidin-3-yl)propiolamide,
117. N-(1-(4,5-diméthylthiazol-2-yl)pyrrolidin-3-yl)-3-(thiophén-2-yl)propiolamide,
118. 3-(2-chloro-5-(trifluorométhyl)phényl)-N-(1-(4,5-diméthylthiazol-2-yl)pyrrolidin-3-yl)propiolamide,
119. N-(1-(5-méthylthiazol-2-yl)pyrrolidin-3-yl)-3-(pyridin-2-yl)propiolamide,
120. N-(1-(5-méthylthiazol-2-yl)pyrrolidin-3-yl)-3-(pyridin-3-yl)propiolamide,
121. N-(1-(5-méthylthiazol-2-yl)pyrrolidin-3-yl)-3-(pyridin-4-yl)propiolamide,
122. N-(1-(4-tert-butylthiazol-2-yl)pyrrolidin-3-yl)-3-(pyridin-2-yl)propiolamide,
123. N-(1-(4-tert-butylthiazol-2-yl)pyrrolidin-3-yl)-3-(pyridin-3-yl)propiolamide et
126. 3-(3-chlorophényl)-N-(2-((5-fluorothiazol-2-yl) (méthyl)amino)éthyl)propiolamide ;

le cas échéant à chaque fois sous forme d'un de leurs stéréo-isomères purs, en particulier leurs énantiomères ou diastéréo-isomères, leurs mélanges racémiques ou sous forme d'un mélange de stéréo-isomères, en particulier des énantiomères et/ou des diastéréo-isomères, dans un rapport de mélange quelconque ou à chaque fois sous forme de sels correspondants.

**9.** Procédé pour la préparation de composés de formule générale I selon l'une ou plusieurs des revendications 1 à 8, **caractérisé**
**en ce qu'**on transforme au moins un composé de formule générale II,

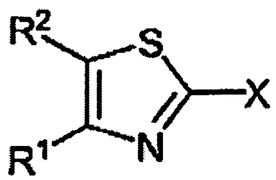

II,

où les radicaux $R^1$ et $R^2$ présentent la signification selon l'une ou plusieurs des revendications 1 à 8 et X représente un groupe partant, de préférence un radical halogène ou un ester d'acide sulfonique, de manière particulièrement préférée un radical chloro ou bromo, avec au moins un composé de formule générale III,

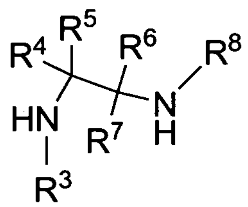

III

où $R^3$, $R^4$, $R^5$, $R^6$, $R^7$ et $R^8$ présentent la signification selon l'une ou plusieurs des revendications 1 à 8, le cas échéant dans un mélange réactionnel, le cas échéant en présence d'au moins une base et/ou d'au moins un composé métallo-organique et/ou d'au moins un réactif de type hydrure de métal ou en présence d'au moins un sel de cuivre et le cas échéant en présence d'au moins un métal, de préférence à une température de -70°C à 300°C, de manière particulièrement préférée de -70°C à 150°C, en au moins un composé correspondant de formule générale IV, le cas échéant sous forme d'un sel correspondant,

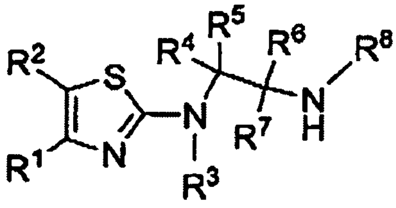

IV.

où $R^1$, $R^2$, $R^3$, $R^4$, $R^5$, $R^6$, $R^7$ et $R^8$ ont la signification susmentionnée et ce composé est le cas échéant purifié et/ou isolé ;
ou on transforme au moins un composé de formule générale II avec au moins un composé de formule générale V,

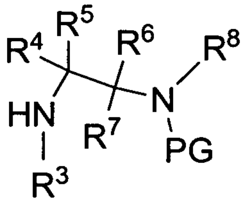

où $R^3$, $R^4$, $R^5$, $R^6$, $R^7$ et $R^8$ ont la signification selon l'une ou plusieurs des revendications 1 à 8 et PG représente un groupe de protection, de préférence un groupe de protection constitué par tert-butyloxycarbonyle, benzyle, benzyloxycarbonyle et 9-fluorénylméthyloxycarbonyle,
le cas échéant dans un mélange réactionnel, le cas échéant en présence d'au moins une base et/ou d'au moins un composé métallo-organique et/ou d'au moins un réactif de type hydrure de métal, de préférence à une température de -70°C à 300°C, en au moins un composé correspondant de formule générale VI,

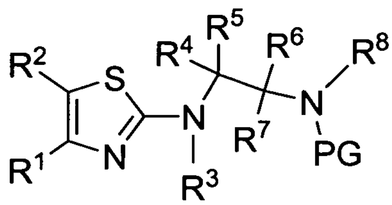

VI

où $R^1$, $R^2$, $R^3$, $R^4$, $R^5$, $R^6$, $R^7$, $R^8$ et PG ont la signification susmentionnée et ce composé est le cas échéant purifié et/ou isolé ;
et on transforme le cas échéant au moins un composé de formule générale VI, où $R^1$, $R^2$, $R^4$, $R^5$, $R^6$, $R^7$, $R^8$ et PG ont la signification susmentionnée et $R^3$ représente hydrogène, avec au moins un composé $R^3$-X, où $R^3$ présente la signification susmentionnée à l'exception d'hydrogène et X représente un groupe partant, de préférence un radical halogène, le cas échéant dans un mélange réactionnel, le cas échéant en présence d'au moins une base, de préférence en présence d'au moins un réactif de type hydrure de métal, de manière particulièrement préférée en présence d'hydrure de sodium, de préférence à une température de -70°C à 300°C en au moins un composé correspondant de formule générale VIa,

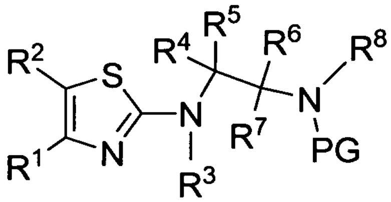

VIa

où $R^1$, $R^2$, $R^3$, $R^9$, $R^5$, $R^6$, $R^7$, $R^8$ et PG ont la signification susmentionnée et $R^3$ ne représente pas un atome d'hydrogène et ce composé est le cas échéant purifié et/ou isolé ;
ou on transforme au moins un composé de formule générale XIII,

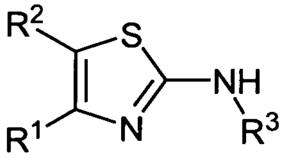

XIII

où $R^1$, $R^2$ et $R^3$ ont la signification selon l'une ou plusieurs des revendications 1 à 8, avec au moins un composé de formule générale XIV,

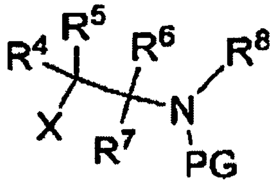

**XIV.**

où $R^4$, $R^5$, $R^6$, $R^7$, $R^8$ et PG ont la signification susmentionnée et X représente un groupe partant, de préférence un radical halogène ou un ester d'acide sulfonique, de manière particulièrement préférée un radical chloro ou bromo, le cas échéant dans un mélange réactionnel, le cas échéant en présence d'au moins une base, de préférence en présence d'au moins une base choisie dans le groupe constitué par le tert-butylate de potassium, l'hydroxyde de sodium, l'hydroxyde de potassium, la diméthylamine et la triéthylamine, de manière particulièrement préférée en présence de diéthylamine, ou le cas échéant en présence d'au moins un composé métallo-organique, de préférence en présence d'au moins un composé métallo-organique choisi dans le groupe constitué par le méthyllithium et le butyllithium ou le cas échéant en présence d'au moins un composé de type hydrure de métal, de manière particulièrement préférée en présence d'hydrure de sodium, de préférence à une température de -70°C à 300°C, de manière particulièrement préférée de -70°C à 150°C, en au moins un composé correspondant de formule générale VI et celui-ci est le cas échéant purifié et/ou isolé ;
ou on transforme au moins un composé de formule générale XIII,

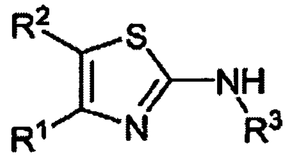

XIII,

où $R^1$, $R^2$ et $R^3$ ont la signification selon l'une ou plusieurs des revendications 1 à 8, avec au moins un composé de formule générale XIVa,

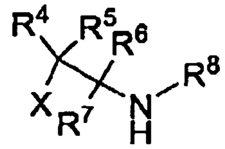

XIVa,

où $R^4$, $R^5$, $R^6$, $R^7$ et $R^8$ ont la signification susmentionnée et X représente un groupe partant, de préférence un radical halogène ou un ester d'acide sulfonique, de manière particulièrement préférée un radical chloro ou bromo, le cas échéant dans un mélange réactionnel, le cas échéant en présence d'au moins une base, de préférence en présence d'au moins une base choisie dans le groupe constitué par le tert-butylate de potassium, l'hydroxyde de sodium, l'hydroxyde de potassium, la diméthylamine et la triéthylamine, de manière particulièrement préférée en présence de diéthylamine, ou le cas échéant en présence d'au moins un composé métallo-organique, de préférence en présence d'au moins un composé métallo-organique choisi dans le groupe constitué par le méthyllithium et le butyllithium ou le cas échéant en présence d'au moins un composé de type hydrure de métal, de manière particulièrement préférée en présence d'hydrure de sodium, de préférence à une température de -70°C à 300°C, de manière particulièrement préférée de -70°C à 150°C, en au moins un composé correspondant de formule générale IV et celui-ci est le cas échéant purifié et/ou isolé ;
ou on transforme au moins un composé de formule générale VII,

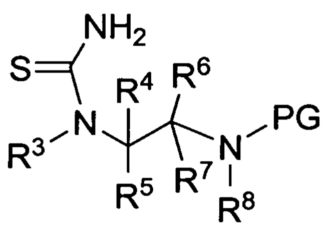

VII

où $R^3$, $R^4$, $R^5$, $R^6$, $R^7$ et $R^8$ ont la signification selon l'une ou plusieurs des revendications 1 à 8 et PG représente un groupe de protection, de préférence un groupe de protection choisi dans le groupe constitué par tert-butyloxycarbonyle, benzyle, benzyloxycarbonyle et 9-fluorénylméthyloxycarbonyle, par transformation avec au moins un composé de formule générale $R^1$-C(=O)-$CH_2$-X ou ($C_{1-5}$-alkyl-O)$_2$-CH-$CH_2$-X, où $R^1$ a la signification selon l'une ou plusieurs des revendications 1 à 8 et X représente un groupe partant, de préférence un radical halogène, de manière particulièrement préférée un atome de brome, dans un mélange réactionnel, le cas échéant en présence d'au moins une base organique ou en présence d'au moins un acide, de préférence en présence d'au moins une base choisie dans le groupe constitué par la triéthylamine, la diisopropyléthylamine, la N-méthylmorpholine, la diméthylaminopyridine et la pyridine ou en présence d'au moins un acide choisi dans le groupe constitué par l'acide acétique, l'acide trifluoroacétique et l'acide chlorhydrique, de préférence à une température entre -70°C à 300°C en au moins un composé correspondant de formule générale VI, le cas échéant sous forme d'un sel correspondant et celui-ci est le cas échéant purifié et/ou isolé ;

et on transforme au moins un composé de formule générale VI ou de formule générale VIa pour le cas où PG représente un groupe tert-butoxycarbonyle ou 9-fluorénylméthyloxycarbonyle, dans un mélange réactionnel, en présence d'au moins un acide, de préférence en présence d'au moins un acide choisi dans le groupe constitué par l'acide chlorhydrique et l'acide trifluoroacétique, de préférence à une température entre -70°C à 100°C ou pour le cas où PG représente un groupe benzyle ou benzyloxycarbonyle, dans un mélange réactionnel, en présence d'hydrogène et en présence d'au moins un catalyseur, de préférence en présence de palladium sur carbone, de préférence à une température entre -70°C à 100°C en au moins un composé correspondant de formule générale IV, le cas échéant sous forme d'un sel correspondant et celui-ci est le cas échéant purifié et/ou isolé ;
et on transforme au moins un composé de formule générale IV par transformation avec au moins un composé de formule générale $R^9$-C=C-C(=O)-OH, où $R^9$ présente la signification selon l'une ou plusieurs des revendications 1 à 8, dans un mélange réactionnel, le cas échéant en présence d'au moins un agent de couplage approprié, qui peut être lié à un polymère, le cas échéant en présence d'au moins une base, de préférence à une température de -70°C à 100°C, ou par transformation avec au moins un composé de formule générale $R^9$-C=C-C(=O)-X, où $R^9$ présente la signification susmentionnée et X représente un groupe partant, de préférence un radical halogène, de manière particulièrement préférée un radical chloro ou bromo, dans un mélange réactionnel, le cas échéant en présence d'au moins une base, de préférence à une température de -70°C à 100°C, en au moins un composé correspondant de formule générale I, le cas échéant sous forme d'un sel correspondant,

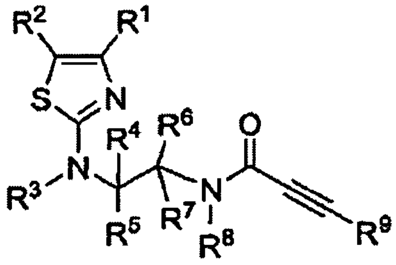

I,

où $R^1$, $R^2$, $R^3$, $R^4$, $R^5$, $R^6$, $R^7$, $R^8$ et $R^9$ ont la signification susmentionnée et ce composé est le cas échéant purifié et/ou isolé ;
ou on transforme au moins un composé de formule générale IV par transformation avec de l'acide propiolique [HC≡C-C(=O)-OH], dans un mélange réactionnel, le cas échéant en présence d'au moins un agent de couplage approprié, qui peut être lié à un polymère, le cas échéant en présence d'au moins une base, de préférence à une température de -70°C à 100°C, ou par transformation avec au moins un composé de formule générale HC≡C-C(=O)-X, où X représente un groupe partant, de préférence un radical halogène, de manière particulièrement préférée un radical chloro ou bromo, dans un mélange réactionnel, le cas échéant en présence d'au moins une base, de préférence à une température de -70°C à 100°C, en au moins un composé correspondant de formule générale VIII, le cas échéant sous forme d'un sel correspondant,

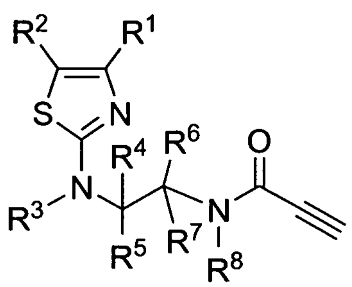

VIII

où $R^1$, $R^2$, $R^3$, $R^4$, $R^5$, $R^6$, $R^7$ et $R^8$ ont la signification susmentionnée et ce composé est le cas échéant purifié et/ou isolé ;

et on transforme au moins un composé de formule générale VIII par transformation avec au moins un composé de formule générale $R^9$-X, où $R^9$ a la signification selon l'une ou plusieurs des revendications 1 à 8 à l'exception d'hydrogène et X représente un groupe partant, de préférence un radical halogène ou un ester d'acide sulfonique, de manière particulièrement préférée l'iode, le brome ou le triflate, dans un mélange réactionnel, le cas échéant en présence d'au moins un catalyseur, de préférence en présence d'au moins un catalyseur du palladium choisi dans le groupe constitué par le chlorure de palladium [$PdCl_2$], l'acétate de palladium [$Pd(OAc)_2$], le tétrakistriphénylphosphine-palladium [$Pd(PPh_3)_4$], le dichlorure de bistriphénylphosphine-palladium [$Pd(PPh_3)_2Cl_2$] et l'acétate de bistriphénylphosphine-palladium [$Pd(PPh_3)_2(OAc)_2$], le cas échéant en présence d'au moins un ligand, de préférence en présence d'au moins un ligand choisi dans le groupe constitué par la triphénylphosphine, la triphénylarsine et la tri-2-furylphosphine, le cas échéant en présence d'au moins un sel inorganique, de préférence en présence d'au moins un sel inorganique choisi dans le groupe constitué par le chlorure de lithium et le chlorure de zinc, le cas échéant en présence d'au moins un sel de cuivre, de préférence en présence d'iodure de cuivre, le cas échéant en présence d'au moins une base organique ou inorganique, de préférence en présence d'au moins une base, choisie dans le groupe constitué par la triéthylamine, le [1,4]-diazabicyclo-[2,2,2]-octane, la diisopropylamine, la diisopropyléthylamine, le carbonate de potassium et l'hydrogénocarbonate de sodium, de préférence à une température entre -70°C à 300°C en au moins un composé correspondant de formule générale I, le cas échéant sous forme d'un sel correspondant et ce composé est le cas échéant purifié et/ou isolé.

**10.** Procédé pour la préparation de composés de formule générale I selon l'une ou plusieurs des revendications 1 à 8, **caractérisé**
**en ce qu'**on transforme au moins un composé de formule générale III,

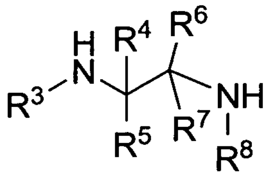

III

où $R^3$, $R^4$, $R^5$, $R^6$, $R^7$ et $R^8$ ont la signification selon l'une ou plusieurs des revendications 1 à 8, par transformation avec au moins un composé de formule générale $R^9$-C≡C-C(=O)-OH, où $R^9$ présente la signification selon l'une ou plusieurs des revendications 1 à 8, dans un mélange réactionnel, le cas échéant en présence d'au moins un agent de couplage approprié, qui peut être lié à un polymère, le cas échéant en présence d'au moins une base, de préférence à une température de -70°C à 100°C, ou par transformation avec au moins un composé de formule générale $R^9$-C≡C-C(=O)-X, où $R^9$ présente la signification susmentionnée et X représente un groupe partant, de préférence un radical halogène, de manière particulièrement préférée un radical chloro ou bromo, dans un mélange réactionnel, le cas échéant en présence d'au moins une base, de préférence à une température de -70°C à 100°C, en au moins un composé correspondant de formule générale IX, le cas échéant sous forme d'un sel correspondant,

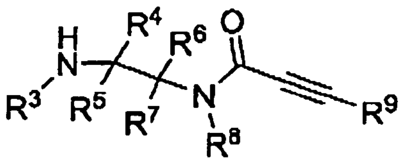

IX,

où $R^3$, $R^4$, $R^5$, $R^6$, $R^7$, $R^8$ et $R^9$ ont la signification susmentionnée et ce composé est le cas échéant purifié et/ou isolé ;
ou on transforme au moins un composé de formule générale V,

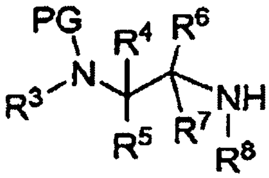

V.

où $R^3$, $R^4$, $R^5$, $R^6$, $R^7$ et $R^8$ ont la signification selon l'une ou plusieurs des revendications 1 à 8 et PG représente un groupe de protection, de préférence un groupe de protection choisi dans le groupe constitué par tert-butyloxycarbonyle, benzyle, benzyloxycarbonyle et 9-fluorénylméthyloxycarbonyle, par transformation avec au moins un composé de formule générale $R^9$-C≡C-C(=O)-OH, où $R^9$ a la signification selon l'une ou plusieurs des revendications 1 à 8, dans un mélange réactionnel, le cas échéant en présence d'au moins un agent de couplage approprié qui peut être lié à un polymère, le cas échéant en présence d'au moins une base, de préférence à une température de -70°C à 100°C, ou par transformation avec au moins un composé de formule générale $R^9$-C≡C-C (=O) -X, où $R^9$ a la signification susmentionnée et X représente un groupe partant, de préférence un radical halogène, de manière particulièrement préférée un radical chloro ou bromo, dans un mélange réactionnel, le cas échéant en présence d'au moins une base, de préférence à une température de -70°C à 100°C, en au moins un composé correspondant de formule générale XI, le cas échéant sous forme d'un sel correspondant,

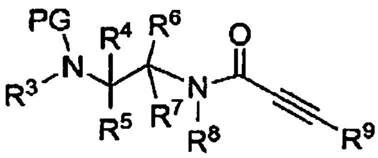

XI,

où $R^3$, $R^4$, $R^5$, $R^6$, $R^7$, $R^8$, $R^9$ et PG ont la signification susmentionnée et ce composé est le cas échéant purifié et/ou isolé ;

et on transforme au moins un composé de formule générale XI pour le cas où PG représente un groupe tert-butoxycarbonyle ou 9-fluorénylméthyloxycarbonyle, dans un mélange réactionnel, en présence d'au moins un acide, de préférence en présence d'au moins un acide choisi dans le groupe constitué par l'acide chlorhydrique et l'acide trifluoroacétique, de préférence à une température entre -70°C à 100°C ou pour le cas où PG représente un groupe benzyle ou benzyloxycarbonyle, dans un mélange réactionnel, en présence d'hydrogène et en présence d'au moins un catalyseur, de préférence en présence de palladium sur carbone, de préférence à une température entre -70°C à 100°C en au moins un composé correspondant de formule générale IX, le cas échéant sous forme d'un sel correspondant et celui-ci est le cas échéant purifié et/ou isolé ;

et on transforme au moins un composé de formule générale IX par transformation avec au moins un composé de formule générale II,

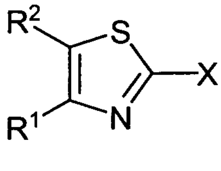

II,

où les radicaux $R^1$ et $R^2$ ont la signification selon l'une ou plusieurs des revendications 1 à 8 et X représente un groupe partant, de préférence un radical halogène ou un ester d'acide sulfonique, de manière particulièrement

préférée un radical chloro ou bromo, dans un mélange réactionnel, le cas échéant en présence d'au moins une base et/ou d'au moins un composé métallo-organique et/ou d'au moins un réactif de type hydrure de métal, de préférence à une température de -70°C à 300°C en au moins un composé correspondant de formule générale I, le cas échéant sous forme d'un sel correspondant, où $R^1$, $R^2$, $R^3$, $R^4$, $R^5$, $R^6$, $R^7$, $R^8$ et $R^9$ ont la signification susmentionnée et ce composé est le cas échéant purifié et/ou isolé ;
ou on transforme le cas échéant au moins un composé de formule générale IX par transformation avec du thiocyanate de potassium et du chloroformiate d'éthyle ou du thiocyanate d'ammonium ou de l'isothiocyanate de triméthylsilyle ou du thiophosgène et de l'ammoniac ou du bromocyan et du sulfure d'hydrogène dans un mélange réactionnel, le cas échéant en présence d'au moins un acide, de préférence en présence d'au moins un acide choisi dans le groupe constitué par l'acide chlorhydrique, l'acide sulfurique, l'acide acétique et l'acide trifluoroacétique, de manière particulièrement préférée en présence d'acide chlorhydrique ou le cas échéant en présence d'au moins une base, de préférence en présence d'au moins une base choisie dans le groupe constitué par le tert-butylate de potassium, l'hydroxyde de sodium, l'hydroxyde de potassium, la diméthylamine et la triéthylamine, de manière particulièrement préférée en présence de diéthylamine, ou le cas échéant en présence d'au moins un composé métallo-organique, de préférence en présence d'au moins un composé métallo-organique choisi dans le groupe constitué par le méthyllithium et le butyllithium ou le cas échéant en présence d'au moins un composé de type hydrure de métal, de manière particulièrement préférée en présence d'hydrure de sodium, de préférence à une température de -70°C à 250°C, en au moins un composé correspondant de formule générale XII, le cas échéant sous forme d'un sel correspondant,

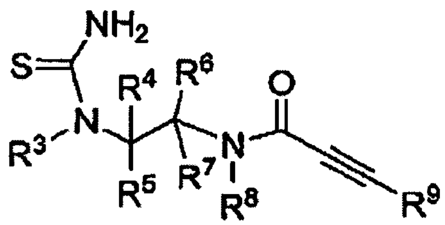

XII,

où $R^3$, $R^4$, $R^5$, $R^6$, $R^7$, $R^8$ et $R^9$ ont la signification susmentionnée et ce composé est le cas échéant purifié et/ou isolé ;
et on transforme au moins un composé de formule générale XII par transformation avec au moins un composé de formule générale $R^1$-C (=O) -$CH_2$-X ou ($C_{1-5}$-alkyl-O)$_2$-CH-$CH_2$-X, où $R^1$ a la signification selon l'une ou plusieurs des revendications 1 à 8 et X représente un groupe partant, de préférence un radical halogène, de manière particulièrement préférée un atome de brome, dans un mélange réactionnel, le cas échéant en présence d'au moins une base organique ou en présence d'au moins un acide, de préférence en présence d'au moins une base choisie dans le groupe constitué par la triéthylamine, la diisopropyléthylamine, la N-méthylmorpholine, la diméthylaminopyridine et la pyridine ou en présence d'au moins un acide choisi dans le groupe constitué par l'acide acétique, l'acide trifluoroacétique et l'acide chlorhydrique, de préférence à une température entre -70°C à 300°C en au moins un composé correspondant de formule générale I, le cas échéant sous forme d'un sel correspondant et ce composé est le cas échéant purifié et/ou isolé.

**11.** Médicament contenant au moins un composé selon l'une ou plusieurs des revendications 1 à 8 et le cas échéant un ou plusieurs adjuvants physiologiquement acceptables.

**12.** Utilisation d'au moins un composé selon l'une ou plusieurs des revendications 1 à 8 pour la préparation d'un médicament destiné au traitement et/ou à la prophylaxie de la douleur, de préférence la douleur choisie dans le groupe constitué par la douleur aiguë, la douleur chronique, la douleur neuropathique et la douleur viscérale ; de la migraine ; des dépressions ; des maladies de neurodégénérescence, de préférence choisies dans le groupe constitué par la sclérose en plaques, la maladie d'Alzheimer, la maladie de Parkinson et la maladie de Huntington ; des maladies cognitives, de préférence les états de carence cognitive, de manière particulièrement préférée le syndrome de déficit d'attention (ADS) ; des états d'anxiété ; des attaques de panique ; de l'épilepsie ; de la toux ; de l'incontinence urinaire ; de la diarrhée ; du prurit ; de la schizophrénie ; des ischémies cérébrales ; des spasmes musculaires ; des crampes ; des maladies pulmonaires, de préférence choisies dans le groupe constitué par l'asthme et la laryngite striduleuse ; de la régurgitation (vomissements) ; de l'attaque ; de la dyskinésie ; de la rétinopathie ; du manque d'entrain ; de l'inflammation du larynx (laryngite) ; des troubles de l'absorption des aliments, de préférence choisis dans le groupe constitué par la boulimie, la cachexie, l'anorexie et l'obésité ; de la dépendance de l'alcool ;

de la dépendance des médicaments ; de la dépendance des drogues, de préférence la dépendance de la nicotine et/ou de la cocaïne ; de l'usage abusif d'alcool ; de l'usage abusif de médicaments ; de l'usage abusif de drogues ; de préférence de l'usage abusif de la nicotine et/ou de la cocaïne ; des phénomènes de sevrage lors de la dépendance de l'alcool, des médicaments et/ou des drogues (en particulier de la nicotine et/ou de la cocaïne) ; du développement de la tolérance aux médicaments, en particulier aux opioïdes naturels ou synthétiques ; du syndrome du reflux gastro-oesophagique ; de la maladie du reflux gastro-oesophagique ; du syndrome de l'intestin irritable ; à la diurèse ; à l'antinatriurèse ; à influencer le système cardiovasculaire ; à l'augmentation de la vigilance ; à l'augmentation de la libido ; à la modulation de l'activité des mouvements ou à l'anesthésie locale.

**13.** Utilisation selon la revendication 12 pour la préparation d'un médicament destiné au traitement et/ou à la prophylaxie de la douleur, de préférence la douleur choisie dans le groupe constitué par la douleur aiguë, la douleur chronique, la douleur neuropathique et la douleur viscérale ; des états d'anxiété ; des attaques de panique ; de la dépendance de l'alcool ; de la dépendance des médicaments ; des troubles de l'absorption des aliments, de préférence choisis dans le groupe constitué par la boulimie, la cachexie, l'anorexie et l'obésité ; de la dépendance des drogues, de préférence la dépendance de la nicotine et/ou de la cocaïne ; de l'usage abusif d'alcool ; de l'usage abusif de médicaments ; de l'usage abusif de drogues ; de préférence de l'usage abusif de la nicotine et/ou de la cocaïne ; des phénomènes de sevrage lors de la dépendance de l'alcool, des médicaments et/ou des drogues (en particulier de la nicotine et/ou de la cocaïne) ; du développement de la tolérance aux médicaments, en particulier aux opioïdes naturels ou synthétiques ; du syndrome du reflux gastro-oesophagique ; de la maladie du reflux gastro-oesophagique et du syndrome de l'intestin irritable.

**14.** Utilisation selon la revendication 12 ou 13 pour la préparation d'un médicament destiné au traitement de la douleur, de préférence de la douleur choisie dans le groupe constitué par la douleur aiguë, la douleur chronique, la douleur neuropathique et la douleur viscérale.

**15.** Utilisation selon la revendication 12 ou 13 pour la préparation d'un médicament destiné au traitement des états d'anxiété ou de panique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- *Journal of Medicinal Chemistry,* 1972, vol. 15 (3), 295-301
- *Journal of Medicinal Chemistry,* 1998, vol. 41 (25), 5027-5054
- **T. W. Greene et al.** Monografien Protective Groups in Organic Synthesis. Wiley, 1999
- **P. J. Kocienski.** Protecting Groups. Georg Thieme Verlag, 2004
- Remington's Pharmaceutical Sciences. Mack Publishing Company, 1985
- **Dubuisson, D. ; Dennis, S.G.** *Pain,* 1977, vol. 4, 161-174